# EUROPEAN PATENT SPECIFICATION

(11) **EP 4 096 667 B1**
(45) Date of publication and mention of the grant of the patent: **29.07.2026**
(21) Application number: 21748194.4
(22) Date of filing: 29.01.2021
(51) Int. Cl.: A61K 31/495, A61K 45/06, C07D 235/14, C07D 405/14, C07D 405/12, C07D 417/10, C07D 417/14, C07D 471/04, C07D 487/10, C07D 513/04, C07D 519/00, A61P 35/00

(54) **MODULATORS OF A BAF-COMPLEX**
MODULATOREN EINES BAF-KOMPLEXES
MODULATEURS D'UN COMPLEXE BAF

(30) Priority: 29.01.2020 US 202062967208 P; 29.01.2020 US 202062967206 P
(43) Date of publication of application: 07.12.2022
(73) Proprietor: Foghorn Therapeutics Inc., Cambridge, MA 02139 (US)
(72) Inventor: WILSON, Kevin, J., Cambridge, MA 02139 (US); SCHILLER, Shawn, E.R., Cambridge, MA 02139 (US); NEGRETTI, Solymar, Cambridge, MA 02139 (US)
(74) Representative: Eli Lilly and Company Limited
(86) International application number: PCT/US2021/015943
(87) International publication number: WO 2021/155320

(56) References cited:
- WO-A1-2017/087608
- WO-A1-2019/152437
- US-A1- 2013 190 290
- US-A1- 2014 288 123
- JULIEN P. N. PAPILLON ET AL: "Discovery of Orally Active Inhibitors of Brahma Homolog (BRM)/SMARCA2 ATPase Activity for the Treatment of Brahma Related Gene 1 (BRG1)/SMARCA4-Mutant Cancers", JOURNAL OF MEDICINAL CHEMISTRY, vol. 61, no. 22, 19 October 2018 (2018-10-19), US, pages 10155 - 10172, XP055619669, ISSN: 0022-2623, DOI: 10.1021/acs.jmedchem.8b01318
- DATABASE PubCHEM Compound U.S. National Library of Medicine; 7 December 2019 (2019-12-07), "N-[(4-Ethylsulfonylphenyl)methyl]-4,4-dimethyl-2-[(5-methylpyrazin-2-yl)methyl]-1,3-dihydroisoquinoline-6-carboxamide | C27H32N4O3S", XP055845259, Database accession no. CID 145315170
- ZEYNEP ATES-ALAGOZ, TULAY COBAN,ERDEM BUYUKBINGOL,: "Synthesis and Antioxidant Activity of New Tetrahydro-Naphthalene-Indole Derivatives as Retinoid and Melatonin Analogs", ARCHIV DER PHARMAZIE - CHEMISTRY IN LIFE SCIENCES, vol. 339, no. 4, 6 April 2006 (2006-04-06), pages 193 - 200, XP055845262

## Description

### Background

The invention relates to compounds useful for modulating BRG1- or BRM-associated factors (BAF) complexes. In particular, the invention relates to compounds useful for treatment of disorders associated with BAF complex function.

Chromatin regulation is essential for gene expression, and ATP-dependent chromatin remodeling is a mechanism by which such gene expression occurs. The human Switch/Sucrose Non-Fermentable (SWI/SNF) chromatin remodeling complex, also known as BAF complex, has two SWI2-like ATPases known as BRG1 (Brahma-related gene-1) and BRM (Brahma). The transcription activator BRG1 is also known as ATP-dependent chromatin remodeler SMARCA4, is encoded by the SMARCA4 gene on chromosome 19. BRG1 is overexpressed in some cancer tumors and is needed for cancer cell proliferation. BRM, also known as probable global transcription activator SNF2L2 and/or ATP-dependent chromatin remodeler SMARCA2, is encoded by the SMARCA2 gene on chromosome 9 and has been shown to be essential for tumor cell growth in cells characterized by loss of BRG1 function mutations. Deactivation of BRG and/or BRM results in downstream effects in cells, including cell cycle arrest and tumor suppression. WO 2019/152437 is directed to compounds useful for modulating BRG1- or BRM associated factors, and J. Med. Chem. Vol. 61, no. 22, pages 10155-10172 is directed to inhibitors of BRM.

### Summary

The present invention features compounds useful for modulating a BAF complex. In some embodiments, the compounds are useful for the treatment of disorders associated with an alteration in a BAF complex, e.g., a disorder associated with an alteration in one or both of the BRG1 and BRM proteins. The compounds of the invention, alone or in combination with other pharmaceutically active agents, can be used for treating such disorders.

In an aspect, the invention features a compound having the structure: 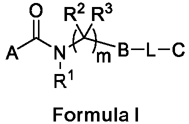 wherein
A is 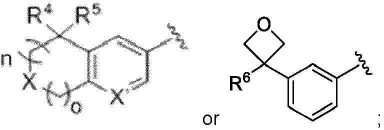
m is 0, 1, 2, or 3;
n is 1 or 2;
o is 0 or 1;
X is O, CH₂, or NR⁷;
X' is N, CH, or CR^{X}, wherein R^{X} is a halogen;
B is an optionally substituted 6-membered monocyclic heteroarylene or an optionally substituted 9- or 10-membered bicyclic heteroarylene;
L is a covalent bond, optionally substituted C₁-C₃ alkylene, C₂ alkynylene, optionally substituted C₂ alkenylene, optionally substituted C₂-C₃ heteroalkylene, optionally substituted C₃-C₅ cycloalkylene, or optionally substituted 4- to 10-membered heterocyclylene;
C is optionally substituted 3- to 10-membered cycloalkyl; optionally substituted 6- to 10-membered aryl; optionally substituted 5- to 10-membered heteroaryl; or optionally substituted 5- to 10-membered heterocyclyl;
R¹ and R⁷ are, independently, hydrogen or optionally substituted C₁-C₆ alkyl;
each R² and R³ is independently, hydrogen, optionally substituted C₁-C₆ alkyl; or optionally substituted C₁-C₆ heteroalkyl;
R⁴ is cyano, fluoro, hydroxy, or -CH₂OH;
R⁵ is C₁-C₃ alkyl optionally substituted with one, two, three, four, five, six, or seven fluoro groups; and
R⁶ is C₁-C₃ alkyl optionally substituted with one, two, three, four, five, six, or seven fluoro groups,
or a pharmaceutically acceptable salt thereof.

In some embodiments, R¹ is hydrogen. In some embodiments, m is 1. In some embodiments, R³ is hydrogen. In some embodiments, R² is hydrogen. In some embodiments, R² is optionally substituted C₁-C₆ alkyl (e.g., methyl). In some embodiments, R² is optionally substituted C₁-C₆ heteroalkyl (e.g., -CH₂OCH₃).

In some embodiments, A is 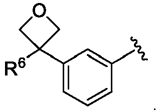 In some embodiments, R⁶ is -CHF₂.

In some embodiments, A is 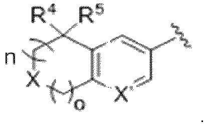 In some embodiments, R⁵ is methyl or - CHF₂. In some embodiments, X is O. In some embodiments, X is CH₂. In some embodiments, X is NR⁷. In some embodiments, X' is N. In some embodiments, X' is CH. In some embodiments, X' is CR^{X} (e.g., R^{X} is fluoro). In some embodiments, R⁷ is hydrogen. In some embodiments, R⁷ is methyl. In some embodiments, o is 0. In some embodiments, o is 1. In some embodiments, n is 1. In some embodiments, n is 2. In some embodiments, R⁴ is cyano. In some embodiments, R⁴ is fluoro. In some embodiments, R⁴ is hydroxy. In some embodiments, R⁴ is -CH₂OH. In some embodiments, A is

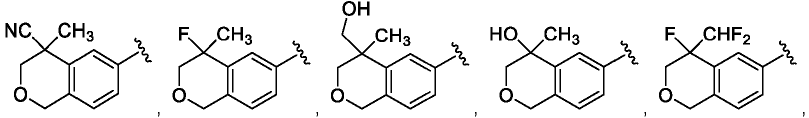

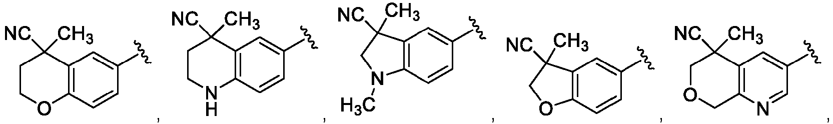

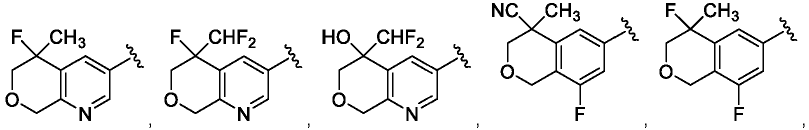

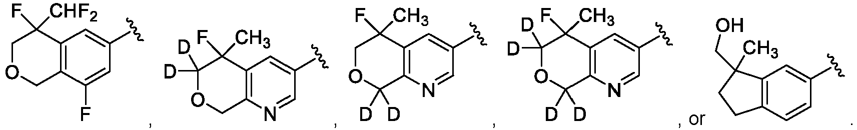

In some embodiments, C is optionally substituted 3- to 10-membered cycloalkyl (e.g., cyclopropyl).

In some embodiments, C is optionally substituted 6- to 10-membered aryl (e.g., phenyl, 3-difluoromethyl-phenyl, 4-dilfluoromethylphenyl, 2-methoxy-phenyl, 3-methoxy-phenyl, 4-methoxy-phenyl, 3,5-difluorophenyl, 3-difluoromethylphenyl, 2-methoxy-5-methyl-phenyl, 2-methoxy-4-chloro-phenyl, 3-cyano-phenyl, 3-fluoro-5-methoxy-phenyl, 3-fluoro-5-azitidinyl-phenyl, 3-azetidyl-phenyl, 4-azetidyl-2-methoxy-phenyl, 4-morpholin-4-yl-2-methoxy-phenyl, 4-tert-butyl-2-methoxy-phenyl, 4-azetidyl-phenyl, 2-methoxy-4-oxetan-3-yl-phenyl, 2-azetidin-2-one-1-yl-phenyl, 3-(3-difluoromethyloxetan-3-yl)-phenyl, 3-(3-methoxyoxetan-3-yl)-phenyl, 3-(N-methyl-N-acetamido)-phenyl, 3-cyclopropylphenyl, 3-(N-methylazetidin-3-yl)-phenyl, 3-(N-methyl-N'-piperazinyl)-phenyl, 3-fluoro-5-azetidyl-phenyl, 3-methyl ester-phenyl,

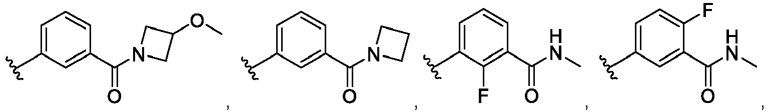

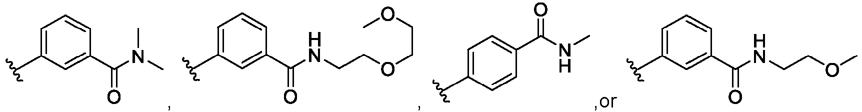

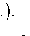

In some embodiments, C is optionally substituted 5- to 10-membered heteroaryl (e.g., 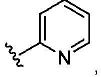 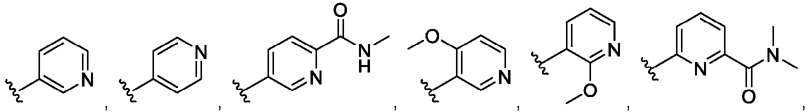 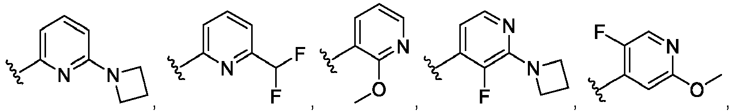 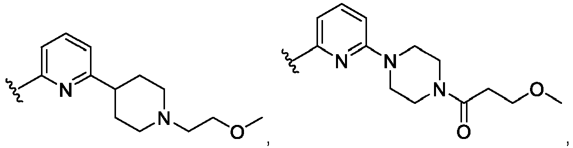 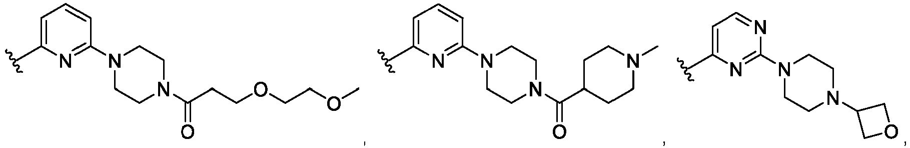 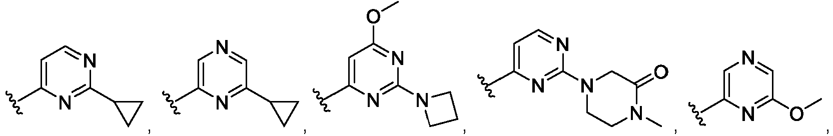 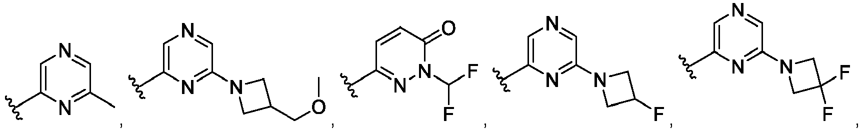 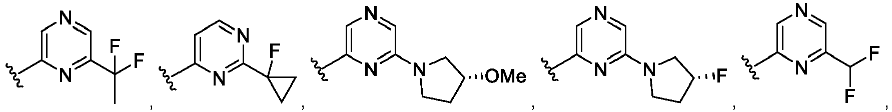 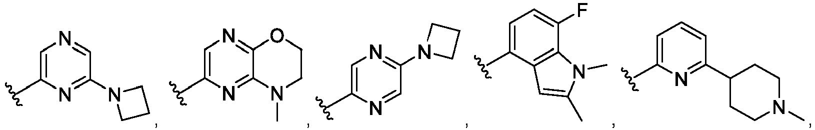 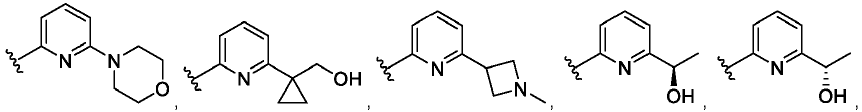 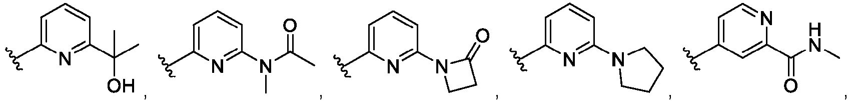 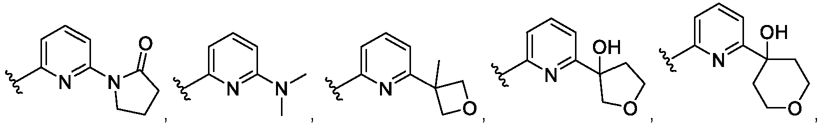 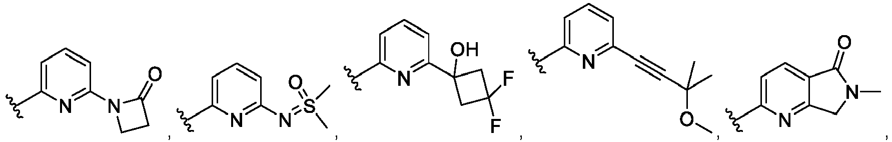 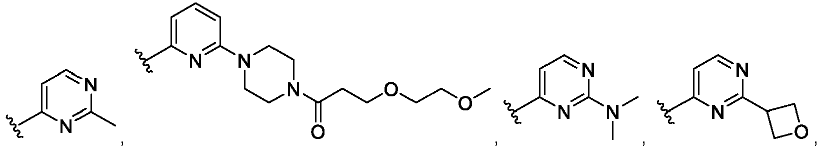 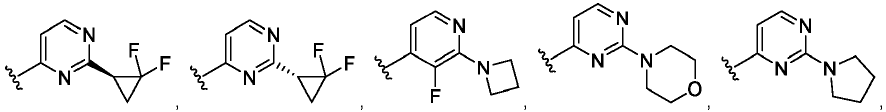 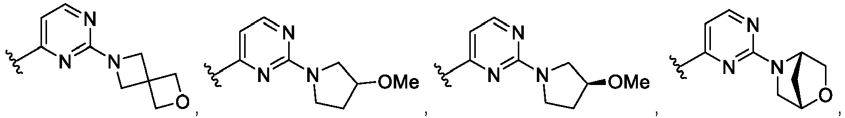 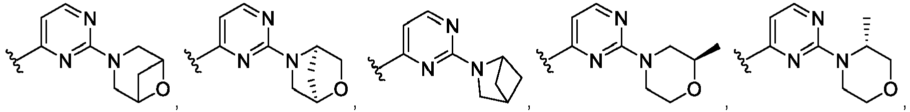 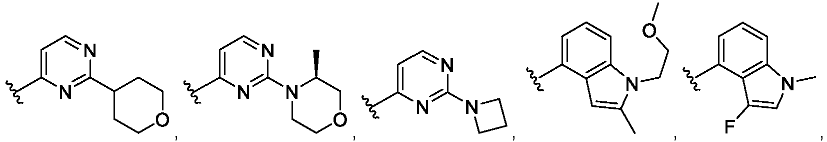 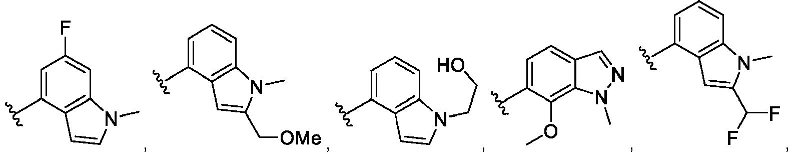 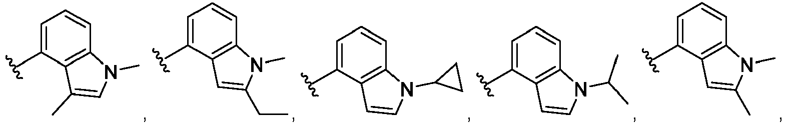 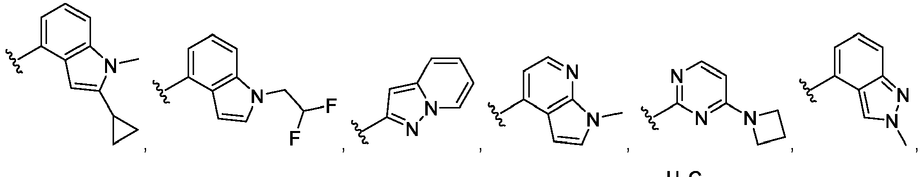 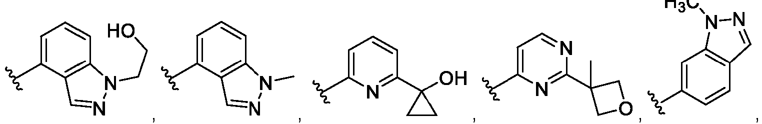 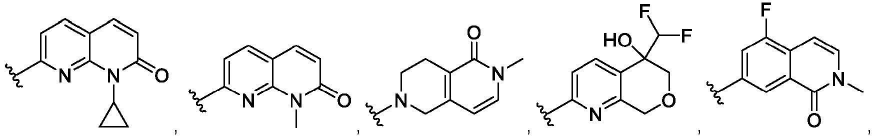 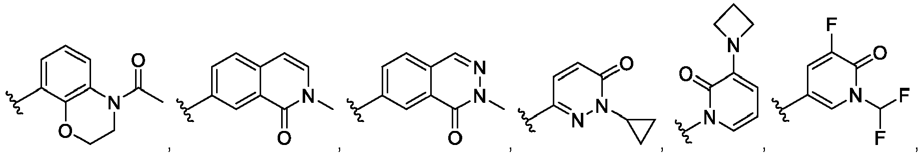 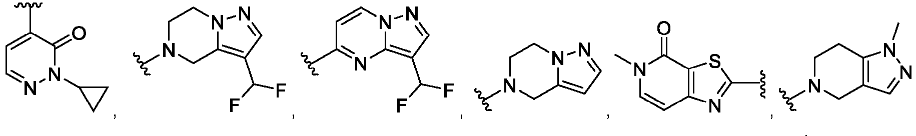 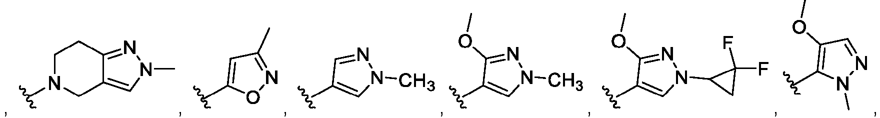 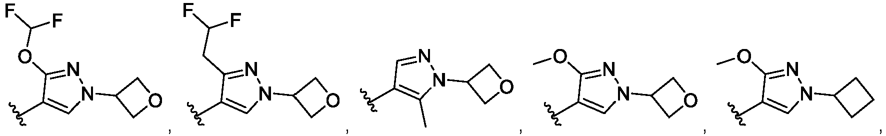 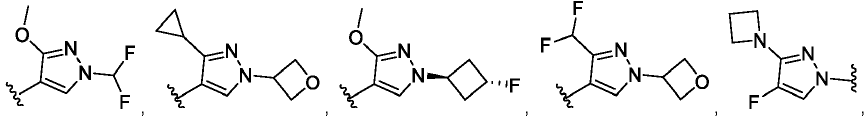 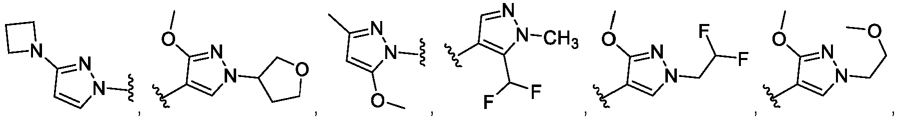 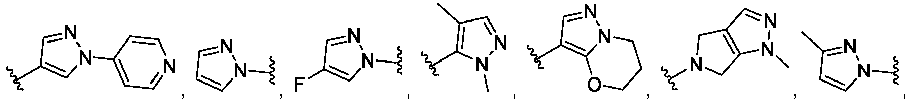 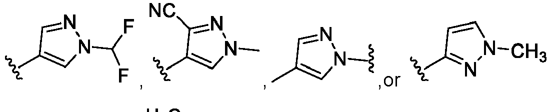 ). In some embodiments, C is

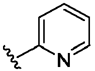

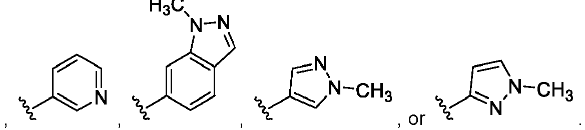

In some embodiments, C is optionally substituted 5- to 10-membered heterocyclyl (e.g., 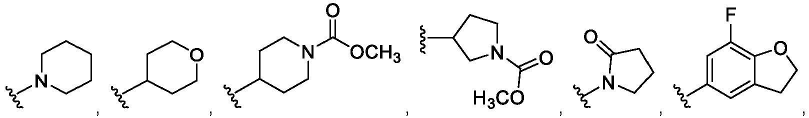 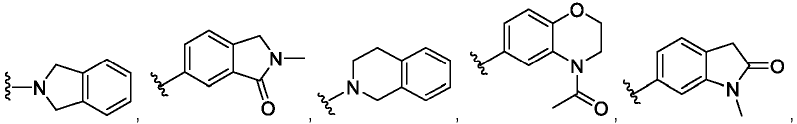 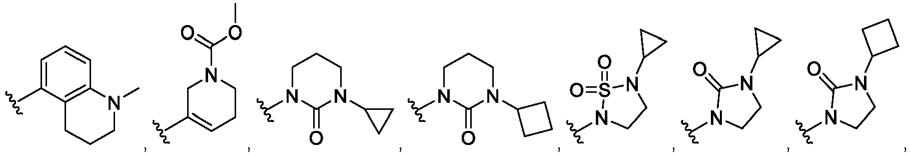 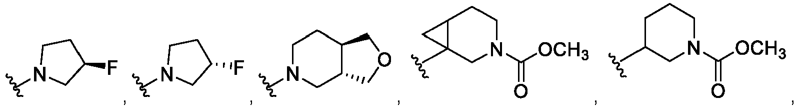 or N-pyrrolidinyl). In some embodiments, C is

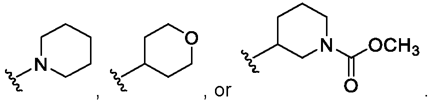

In some embodiments, B is an optionally substituted 6-membered monocyclic heteroarylene. In some embodiments, B has the structure: 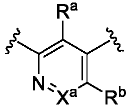
wherein R^{a} is hydrogen or fluoro;
R^{b} is hydrogen, fluoro, or C₁-C₃ alkyl; and
X^{a} is N or CH.

In some embodiments, R^{a} is hydrogen. In some embodiments, R^{a} is fluoro. In some embodiments, R^{b} is hydrogen. In some embodiments, R^{b} is fluoro. In some embodiments, C₁-C₃ alkyl (e.g., methyl). In some embodiments, X^{a} is N. In some embodiments, X^{a} is CH. In some embodiments, B is 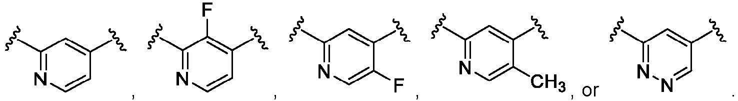 In some embodiments, B is

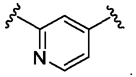

In some embodiments, B is an optionally substituted 9- or 10-membered bicyclic heteroarylene. In some embodiments, B has the structure: 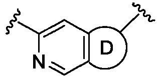 wherein D is an optionally substituted 5- or 6-membered heteroaryl or optionally substituted 5- or 6-membered heterocyclyl (e.g., 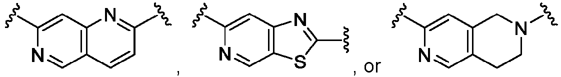 ).

In some embodiments, L is optionally substituted C₁-C₃ alkylene, C₂ alkynylene, optionally substituted C₂ alkenylene, optionally substituted C₂-C₃ heteroalkylene, optionally substituted C₃-C₅ cycloalkylene, or optionally substituted 4- to 10-membered heterocyclylene. In some embodiments, L is covalent a bond, e.g., the compound of Formula I is a compound of Formula la: 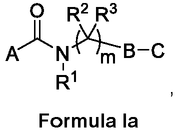 or a pharmaceutically acceptable salt thereof, where all variables are as described herein.

In some embodiments, L is optionally substituted C₁-C₃ alkylene (e.g., ethylene). In some embodiments, C₂ alkynylene. In some embodiments, L is optionally substituted C₂ alkenylene (e.g., 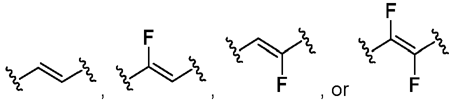 ). In some embodiments, L is optionally substituted C₂-C₃ heteroalkylene (e.g., 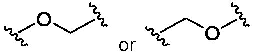 ). In some embodiments, L is optionally substituted C₃-C₅ cycloalkylene (e.g., 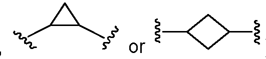 ). In some embodiments, L is optionally substituted 4- to 10-membered heterocyclylene (e.g.,

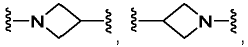

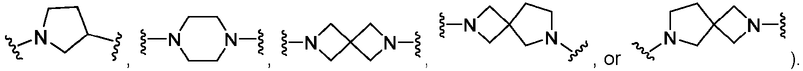

In some embodiments, the compound is any one of compounds 1-48 in Table 1.

**Table 1. Compounds of the invention**

| **#** | **Compound** | **#** | **Compound** |
|---|---|---|---|
| 1 | 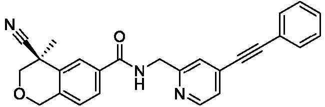 | 25 | 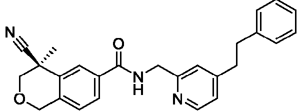 |
| 2 | 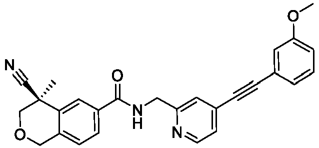 | 26 | 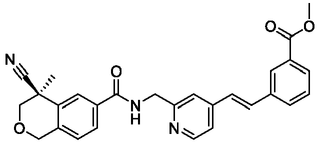 |
| 3 | 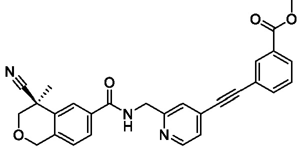 | 27 | 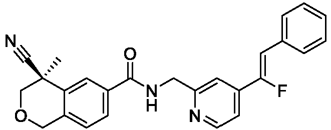 |
| 4 | 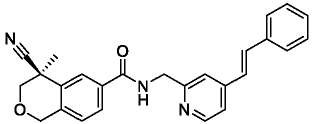 | 28 | 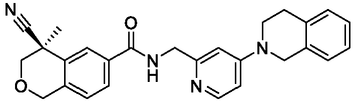 |
| 5 | 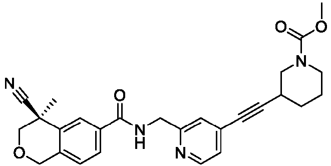 | 29 | 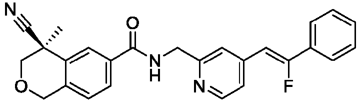 |
| 6 | 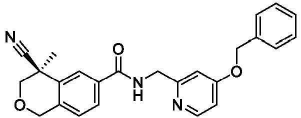 | 30 | 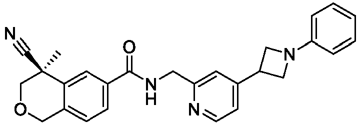 |
| 7 | 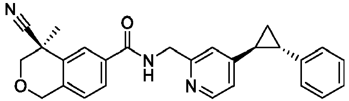 | 31 | 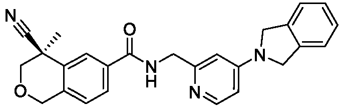 |
| 8 | 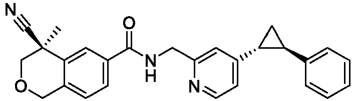 | 32 | 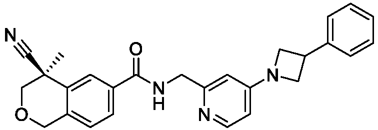 |
| 9 | 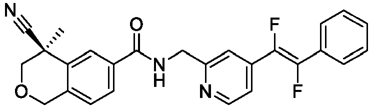 | 33 | 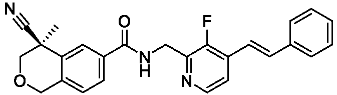 |
| 10 | 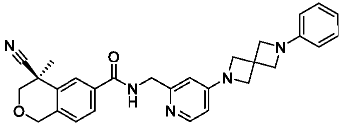 | 34 | 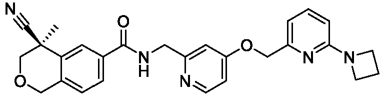 |
| 11 | 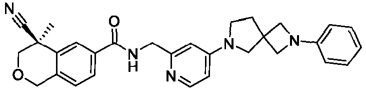 | 35 | 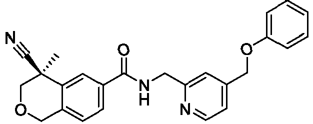 |
| 12 | 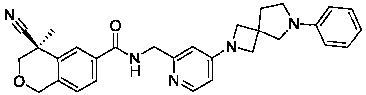 | 36 | 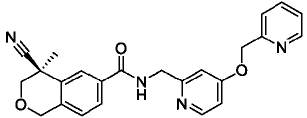 |
| 13 | 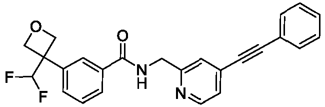 | 37 | 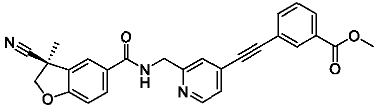 |
| 14 | 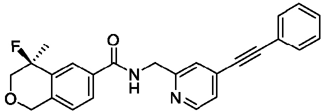 | 38 | 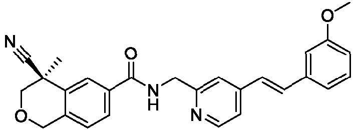 |
| 15 | 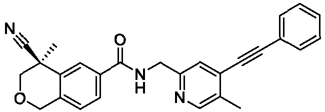 | 39 | 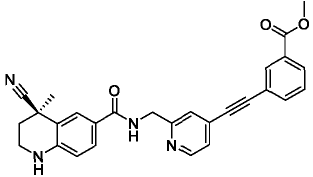 |
| 16 | 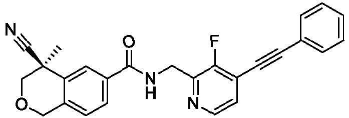 | 40 | 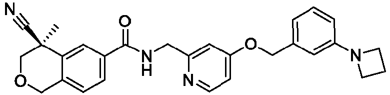 |
| 17 | 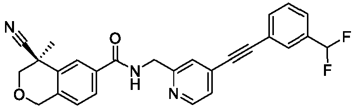 | 41 | 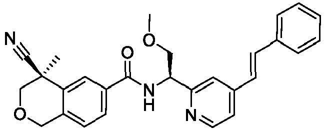 |
| 18 | 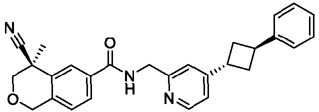 | 42 | 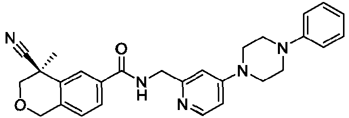 |
| 19 | 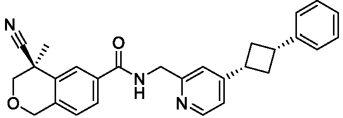 | 43 | 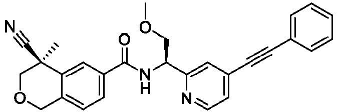 |
| 20 | 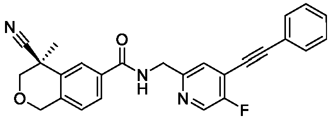 | 44 | 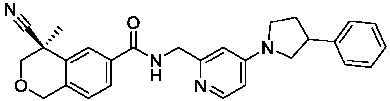 |
| 21 | 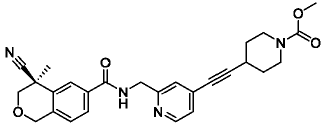 | 45 | 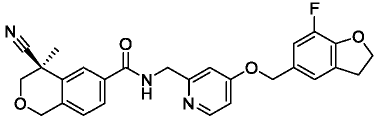 |
| 22 | 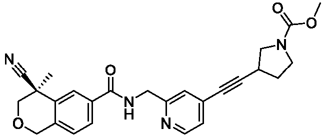 | 46 | 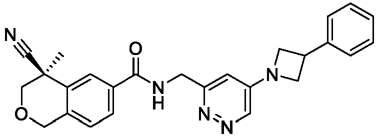 |
| 23 | 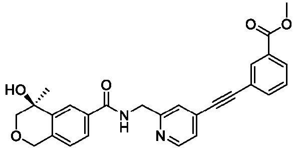 | 47 | 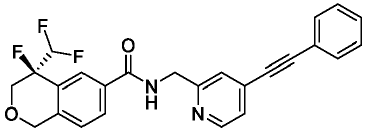 |
| 24 | 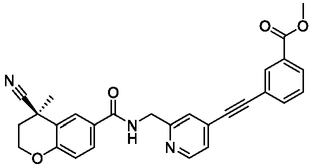 | 48 | 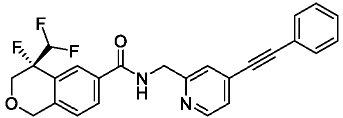 |

In some embodiments, the compound is any one of compounds 49-84 in Table 2.

**Table 2. Compounds of the invention**

| **#** | **Compound** | **#** | **Compound** |
|---|---|---|---|
| 49 | 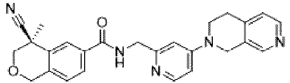 | 67 | 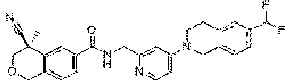 |
| 50 | 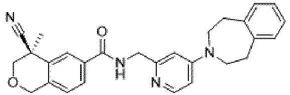 | 68 | 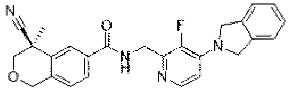 |
| 51 | 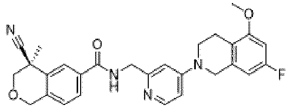 | 69 | 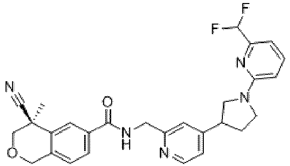 |
| 52 | 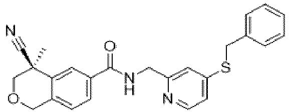 | 70 | 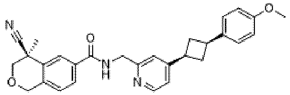 |
| 53 | 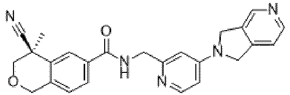 | 71 | 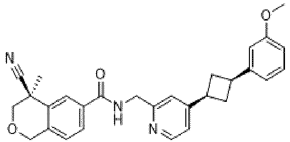 |
| 54 | 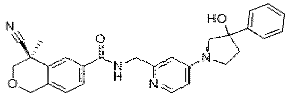 | 72 | 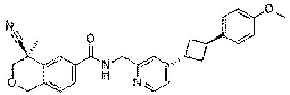 |
| 55 | 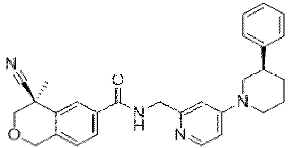 | 73 | 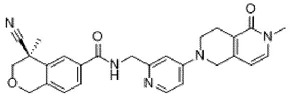 |
| 56 | 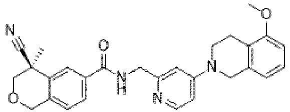 | 74 | 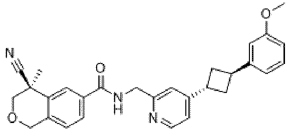 |
| 57 | 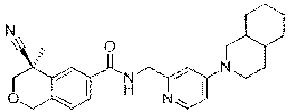 | 75 | 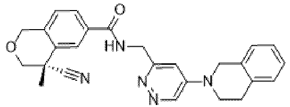 |
| 58 | 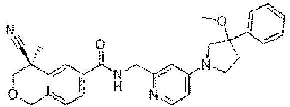 | 76 | 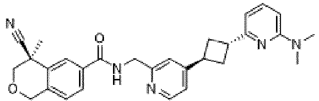 |
| 59 | 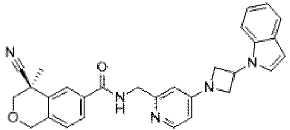 | 77 | 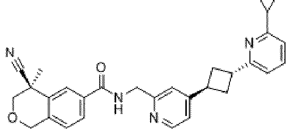 |
| 60 | 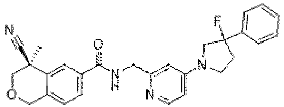 | 78 | 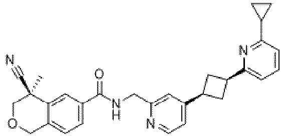 |
| 61 | 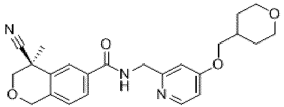 | 79 | 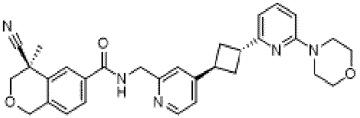 |
| 62 | 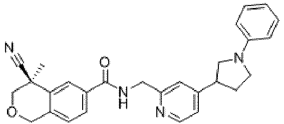 | 80 | 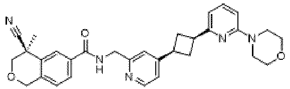 |
| 63 | 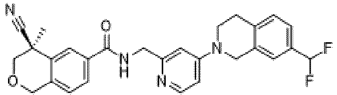 | 81 | 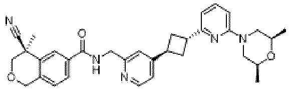 |
| 64 | 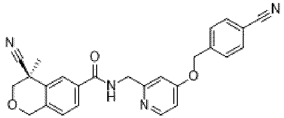 | 82 | 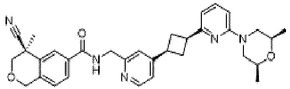 |
| 65 | 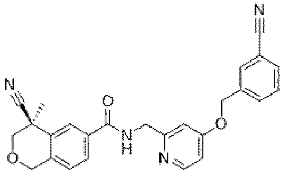 | 83 | 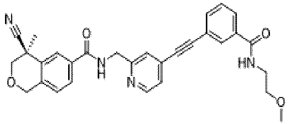 |
| 66 | 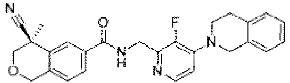 | 84 | 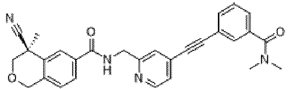 |

In some embodiments, the compound is any one of compounds 85-126 in Table 3.

**Table 3. Compounds of the invention**

| # | Compound | # | Compound |
|---|---|---|---|
| 85 | 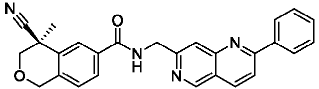 | 106 | 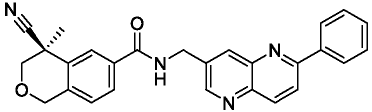 |
| 86 | 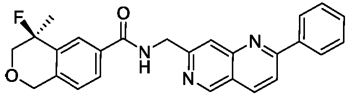 | 107 | 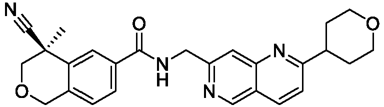 |
| 87 | 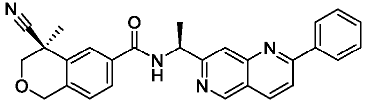 | 108 | 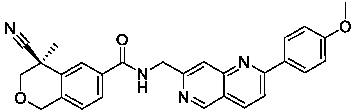 |
| 88 | 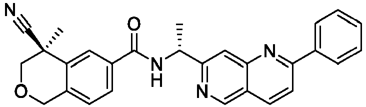 | 109 | 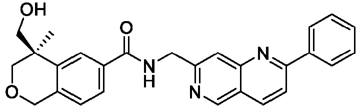 |
| 89 | 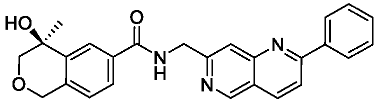 | 110 | 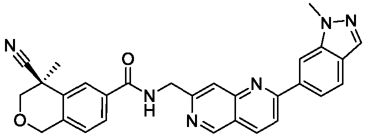 |
| 90 | 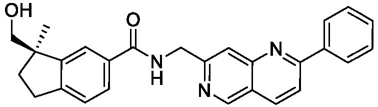 | 111 | 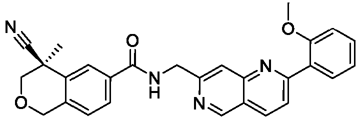 |
| 91 | 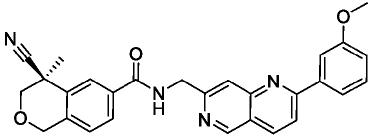 | 112 | 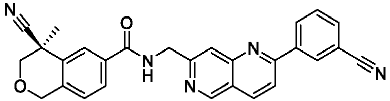 |
| 92 | 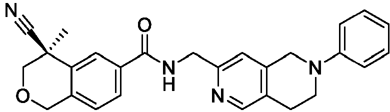 | 113 | 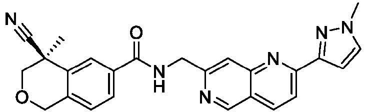 |
| 93 | 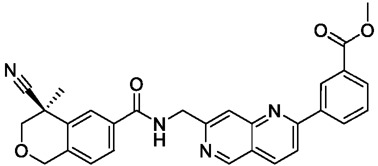 | 114 | 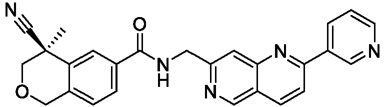 |
| 94 | 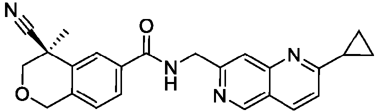 | 115 | 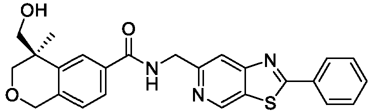 |
| 95 | 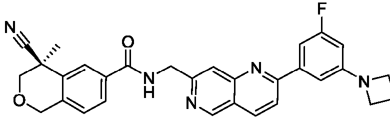 | 116 | 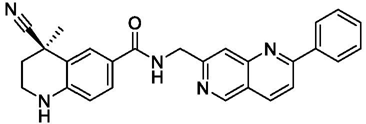 |
| 96 | 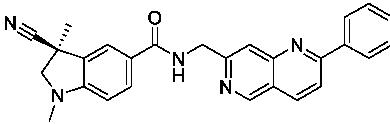 | 117 | 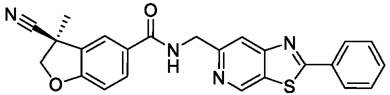 |
| 97 | 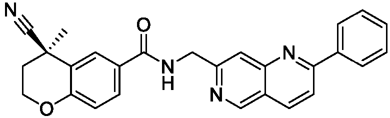 | 118 | 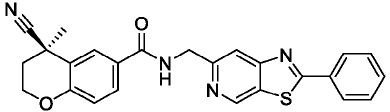 |
| 98 | 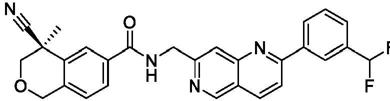 | 119 | 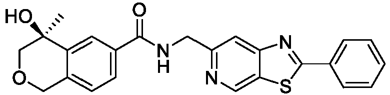 |
| 99 | 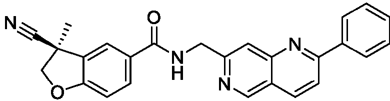 | 120 | 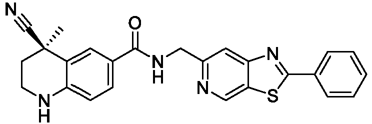 |
| 100 | 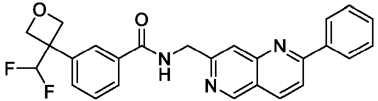 | 121 | 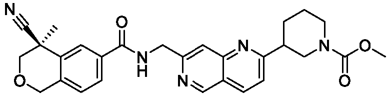 |
| 101 | 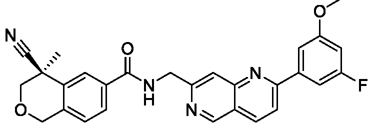 | 122 | 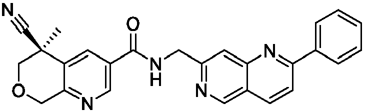 |
| 102 | 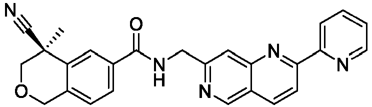 | 123 | 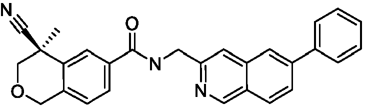 |
| 103 | 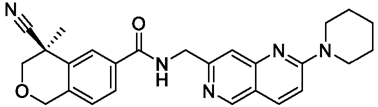 | 124 | 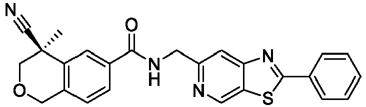 |
| 104 | 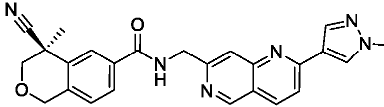 | 125 | 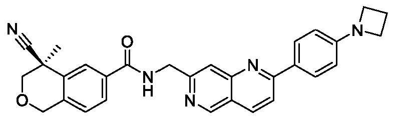 |
| 105 | 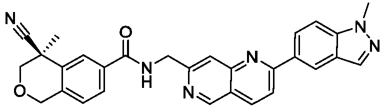 | 126 | 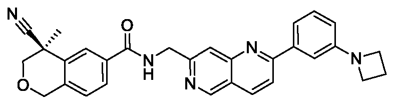 |

In some embodiments, the compound is any one of compounds 127-525 in Table 3a.

**Table 3a. Compounds of the invention**

| **#** | **Compound** | **#** | **Compound** |
|---|---|---|---|
| 127 | 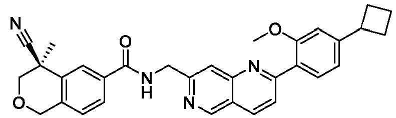 | 327 | 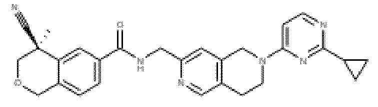 |
| 128 | 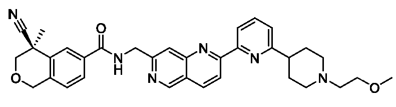 | 328 | 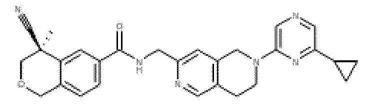 |
| 129 | 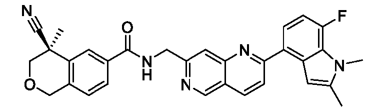 | 329 | 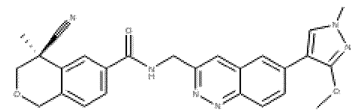 |
| 130 | 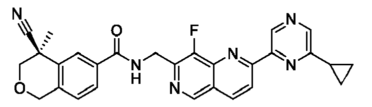 | 330 | 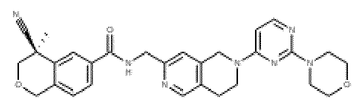 |
| 131 | 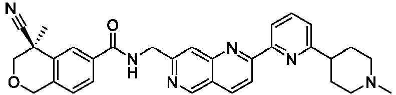 | 331 | 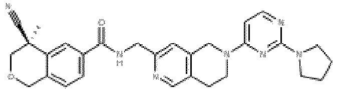 |
| 132 | 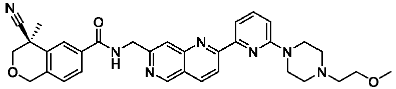 | 332 | 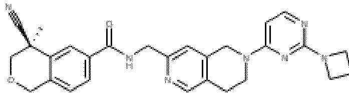 |
| 133 | 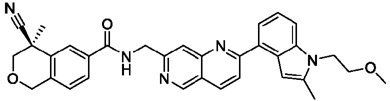 | 333 | 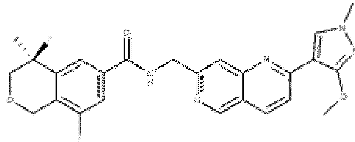 |
| 134 | 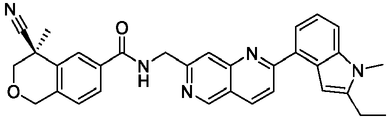 | 334 | 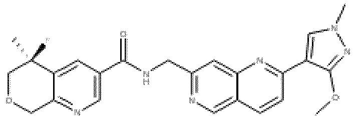 |
| 135 | 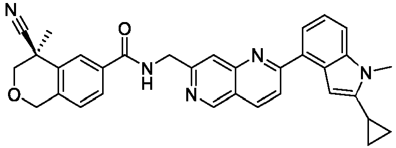 | 335 | 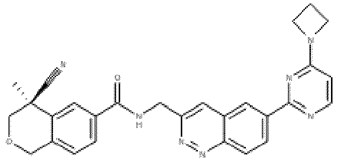 |
| 136 | 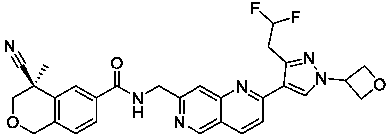 | 336 | 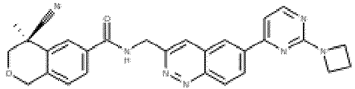 |
| 137 | 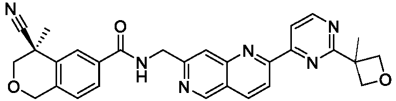 | 337 | 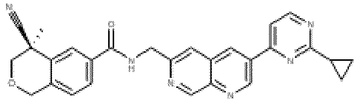 |
| 138 | 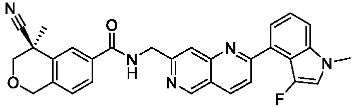 | 338 | 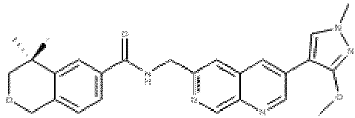 |
| 139 | 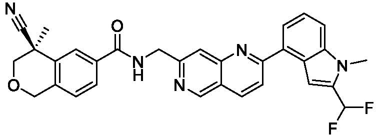 | 339 | 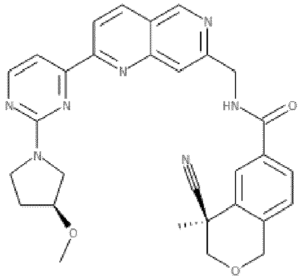 |
| 140 | 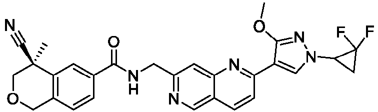 | 340 | 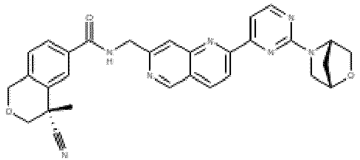 |
| 141 | 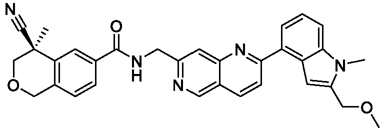 | 341 | 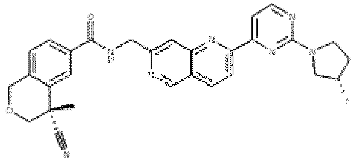 |
| 142 | 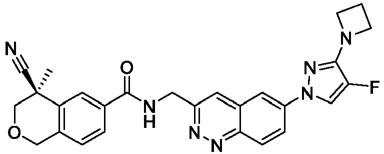 | 342 | 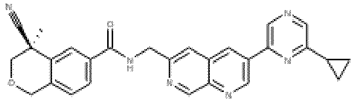 |
| 143 | 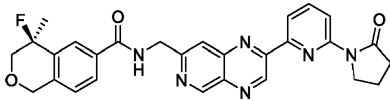 | 343 | 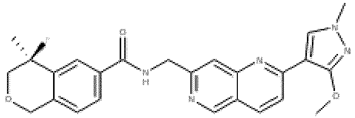 |
| 144 | 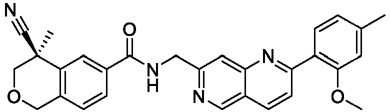 | 344 | 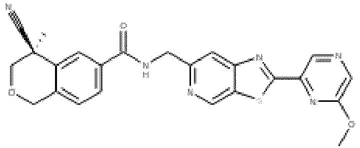 |
| 145 | 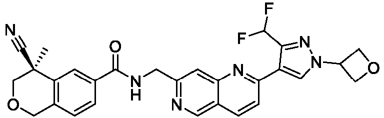 | 345 | 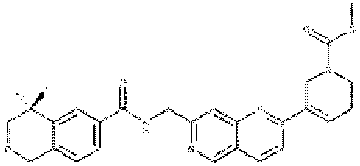 |
| 146 | 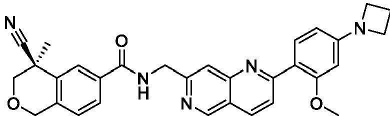 | 346 | 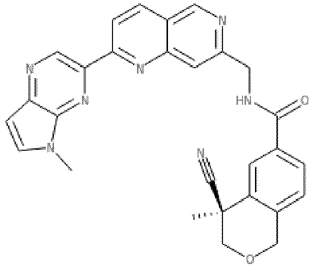 |
| 147 | 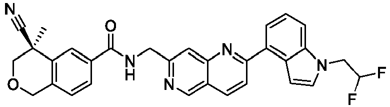 | 347 | 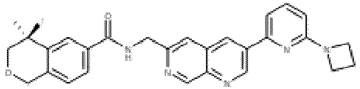 |
| 148 | 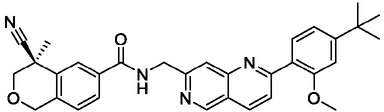 | 348 | 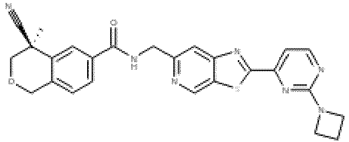 |
| 149 | 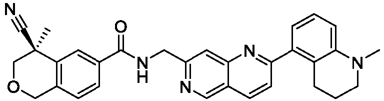 | 349 | 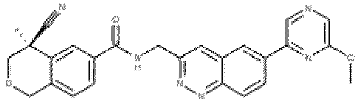 |
| 150 | 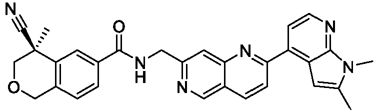 | 350 | 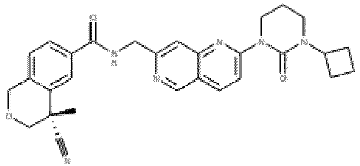 |
| 151 | 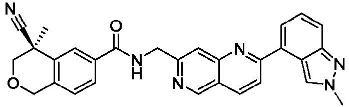 | 351 | 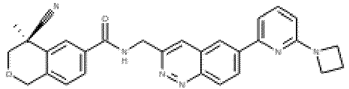 |
| 152 | 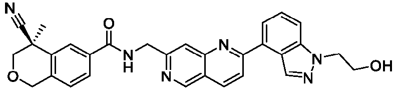 | 352 | 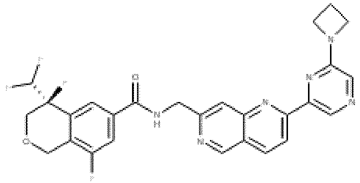 |
| 153 | 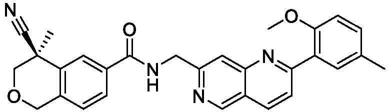 | 353 | 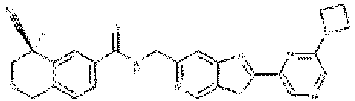 |
| 154 | 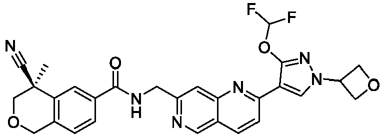 | 354 | 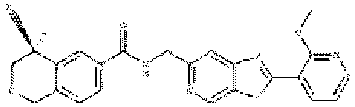 |
| 155 | 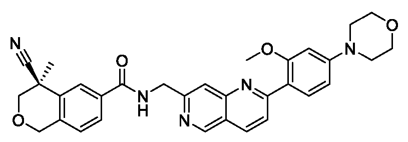 | 355 | 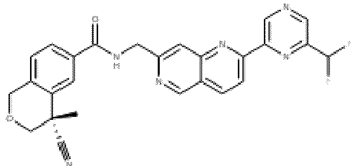 |
| 156 | 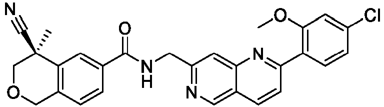 | 356 | 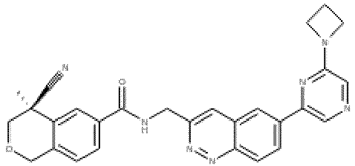 |
| 157 | 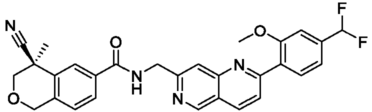 | 357 | 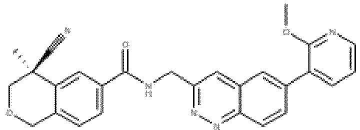 |
| 158 | 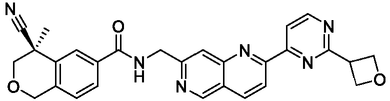 | 358 | 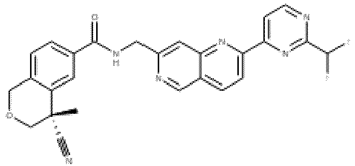 |
| 159 | 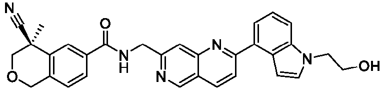 | 359 | 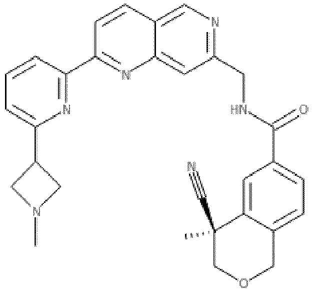 |
| 160 | 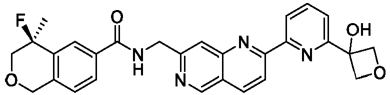 | 360 | 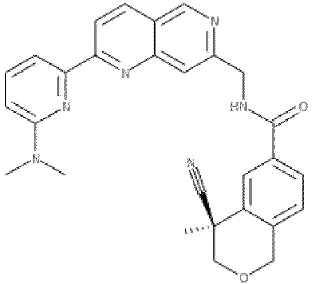 |
| 161 | 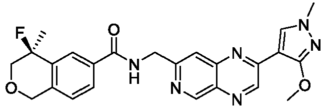 | 361 | 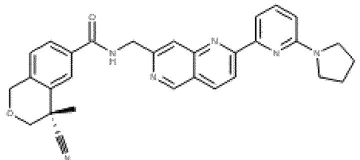 |
| 162 | 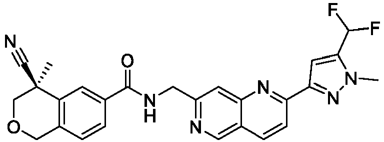 | 362 | 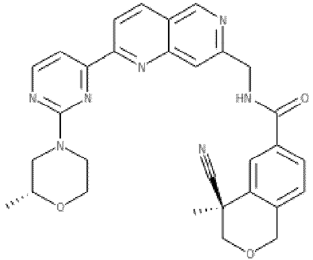 |
| 163 | 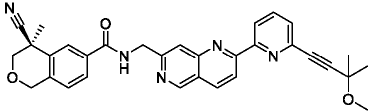 | 363 | 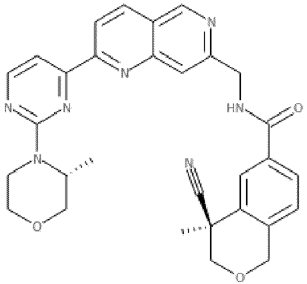 |
| 164 | 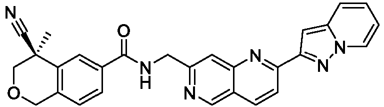 | 364 | 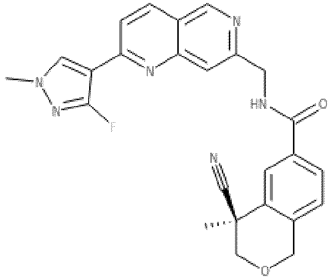 |
| 165 | 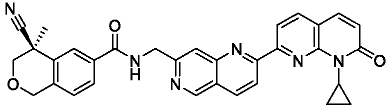 | 365 | 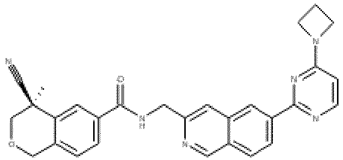 |
| 166 | 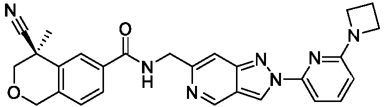 | 366 | 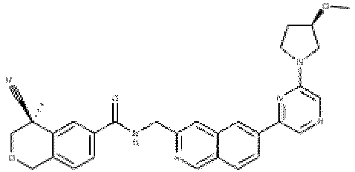 |
| 167 | 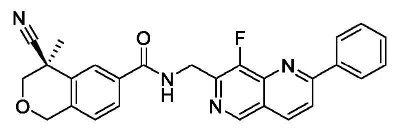 | 367 | 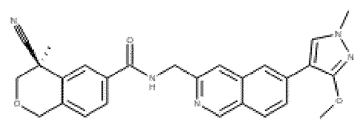 |
| 168 | 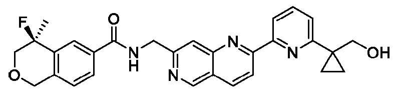 | 368 | 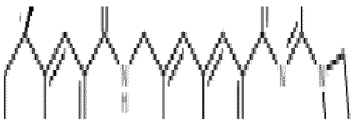 |
| 169 | 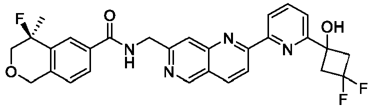 | 369 | 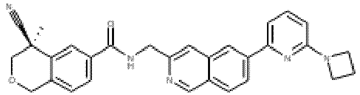 |
| 170 | 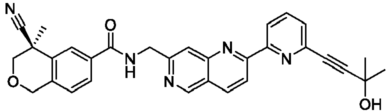 | 370 | 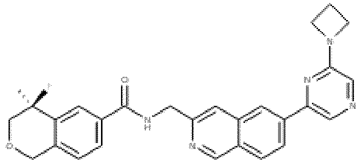 |
| 171 | 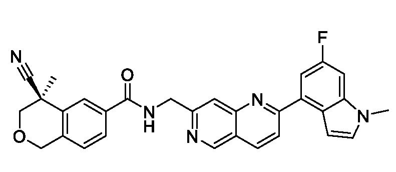 | 371 | 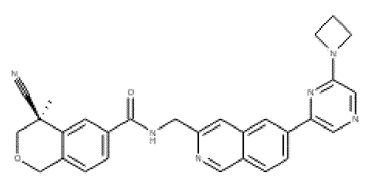 |
| 172 | 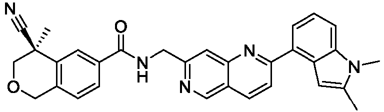 | 372 | 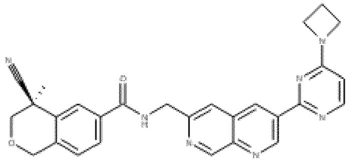 |
| 173 | 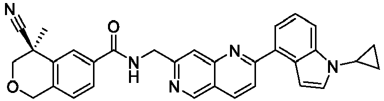 | 373 | 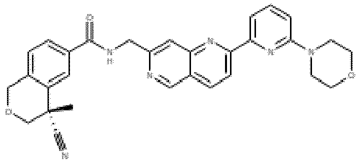 |
| 174 | 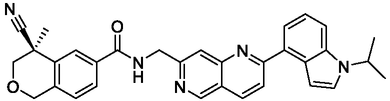 | 374 | 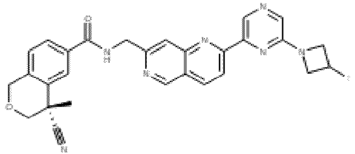 |
| 175 | 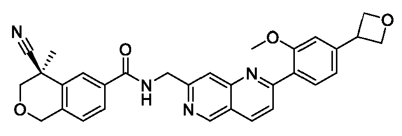 | 375 | 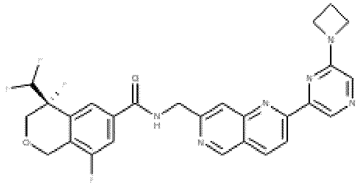 |
| 176 | 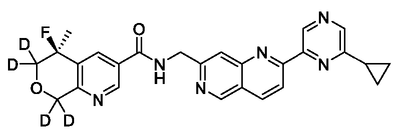 | 376 | 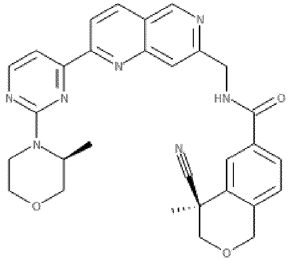 |
| 177 | 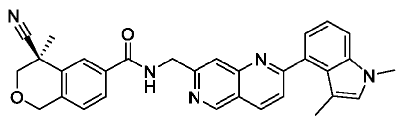 | 377 | 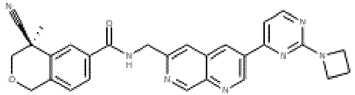 |
| 178 | 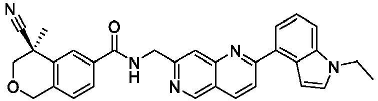 | 378 | 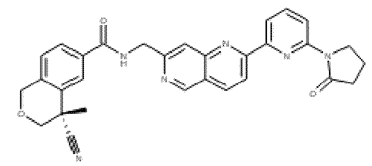 |
| 179 | 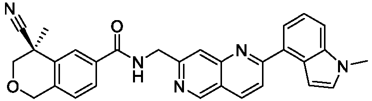 | 379 | 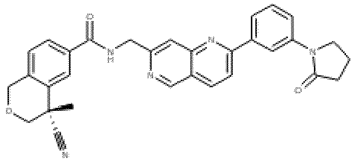 |
| 180 | 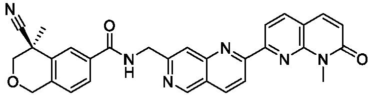 | 380 | 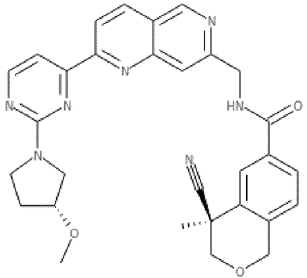 |
| 181 | 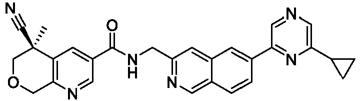 | 381 | 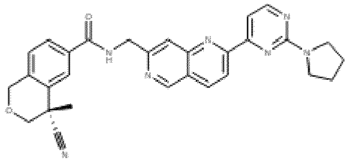 |
| 182 | 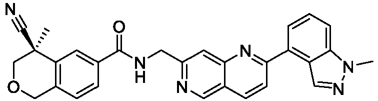 | 382 | 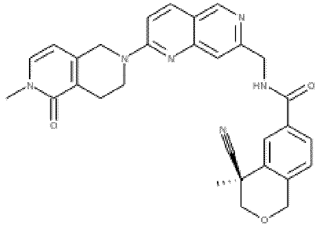 |
| 183 | 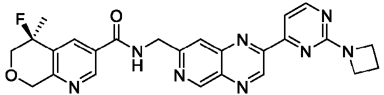 | 383 | 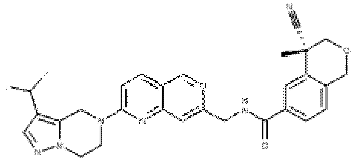 |
| 184 | 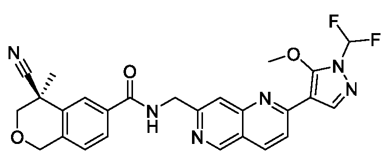 | 384 | 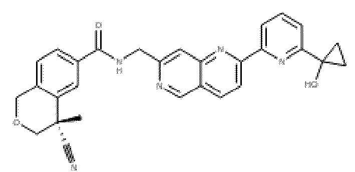 |
| 185 | 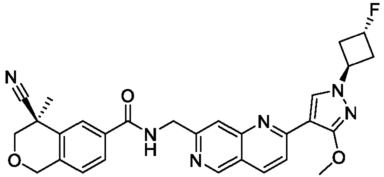 | 385 | 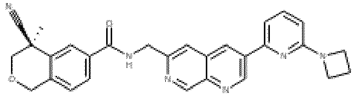 |
| 186 | 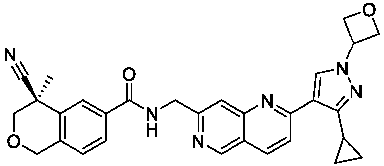 | 386 | 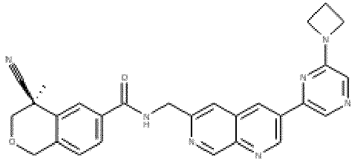 |
| 187 | 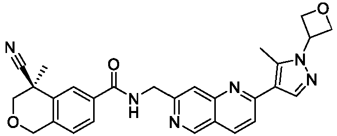 | 387 | 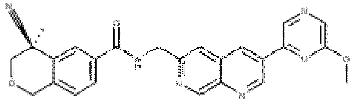 |
| 188 | 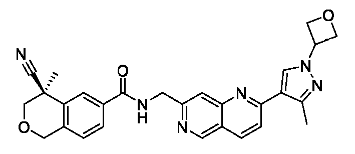 | 388 | 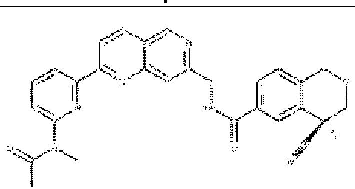 |
| 189 | 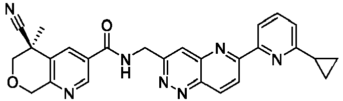 | 389 | 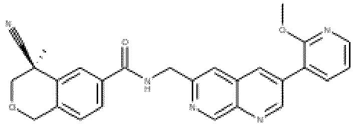 |
| 190 | 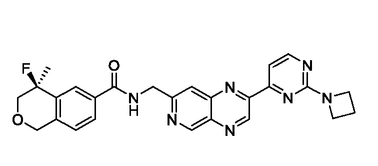 | 390 | 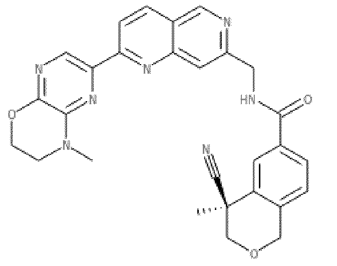 |
| 191 | 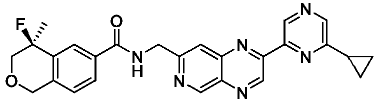 | 391 | 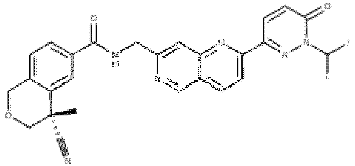 |
| 192 | 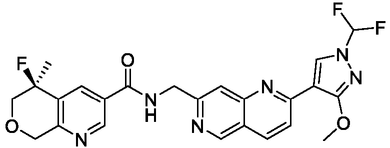 | 392 | 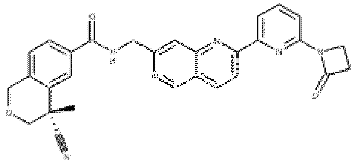 |
| 193 | 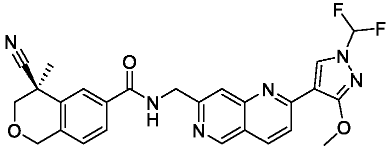 | 393 | 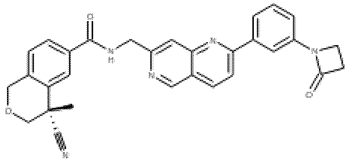 |
| 194 | 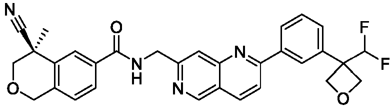 | 394 | 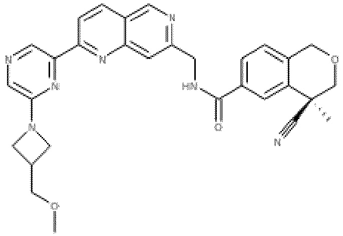 |
| 195 | 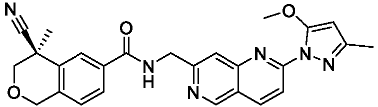 | 395 | 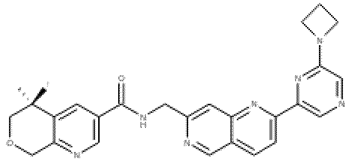 |
| 196 | 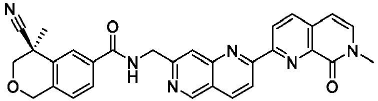 | 396 | 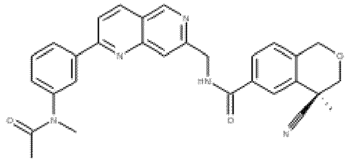 |
| 197 | 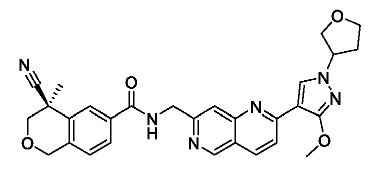 | 397 | 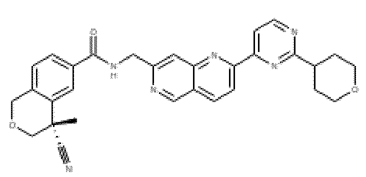 |
| 198 | 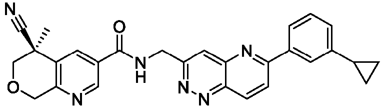 | 398 | 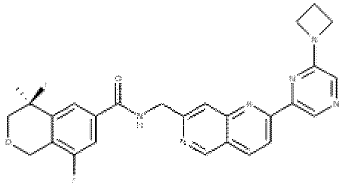 |
| 199 | 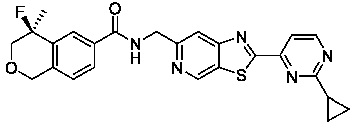 | 399 | 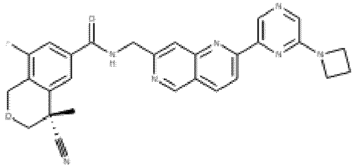 |
| 200 | 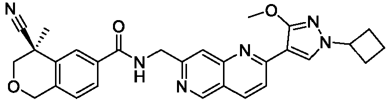 | 400 | 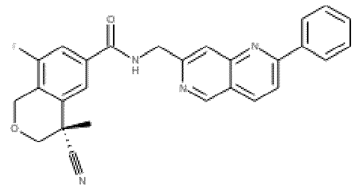 |
| 201 | 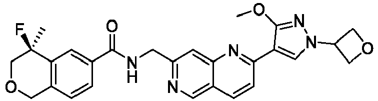 | 401 | 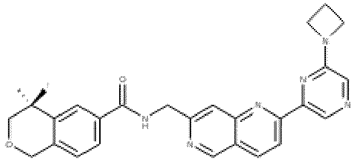 |
| 202 | 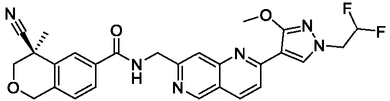 | 402 | 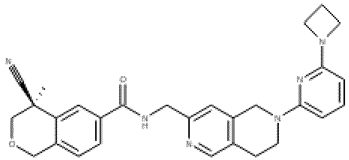 |
| 203 | 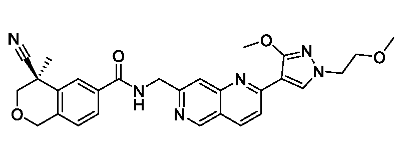 | 403 | 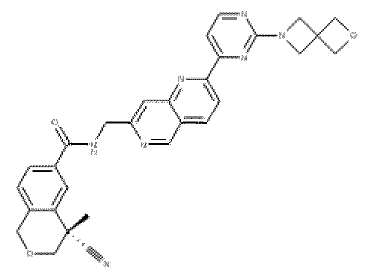 |
| 204 | 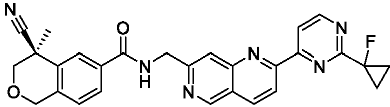 | 404 | 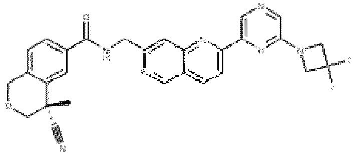 |
| 205 | 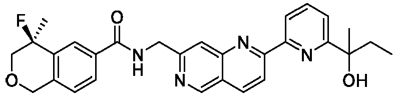 | 405 | 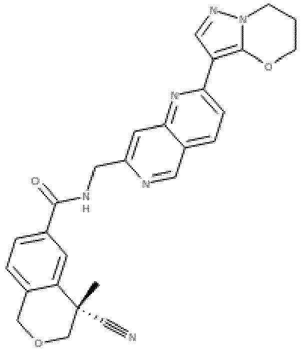 |
| 206 | 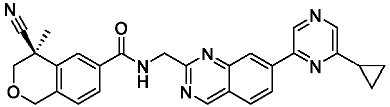 | 406 | 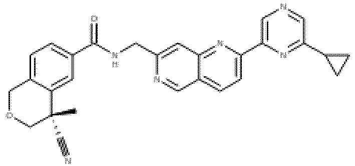 |
| 207 | 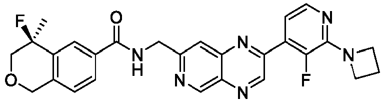 | 407 | 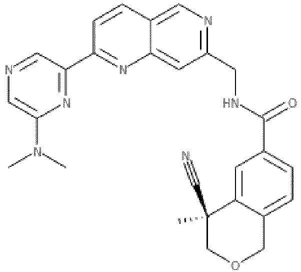 |
| 208 | 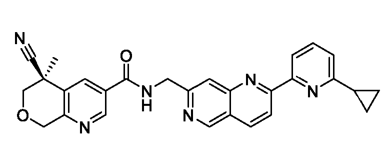 | 408 | 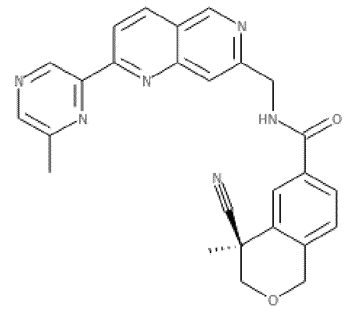 |
| 209 | 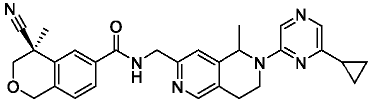 | 409 | 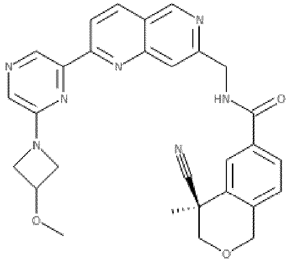 |
| 210 | 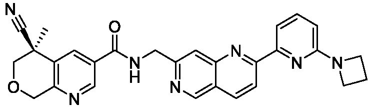 | 410 | 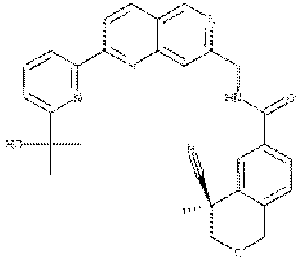 |
| 211 | 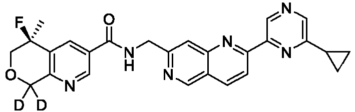 | 411 | 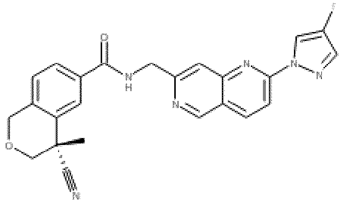 |
| 212 | 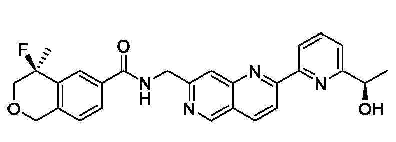 | 412 | 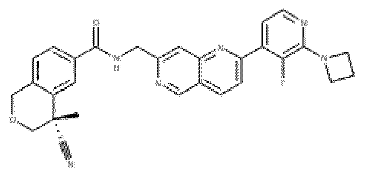 |
| 213 | 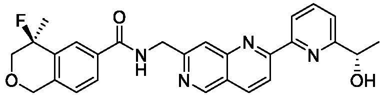 | 413 | 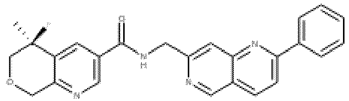 |
| 214 | 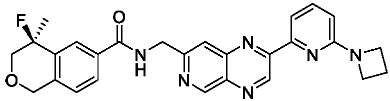 | 414 | 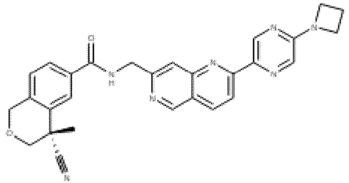 |
| 215 | 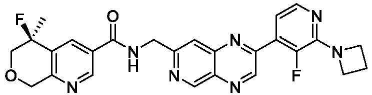 | 415 | 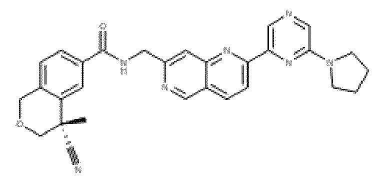 |
| 216 | 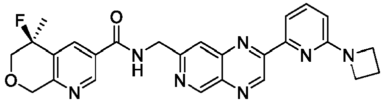 | 416 | 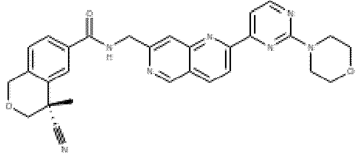 |
| 217 | 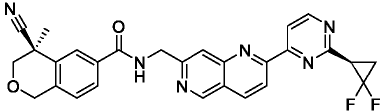 | 417 | 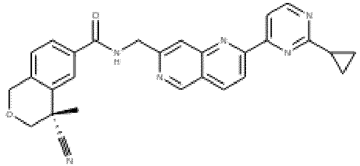 |
| 218 | 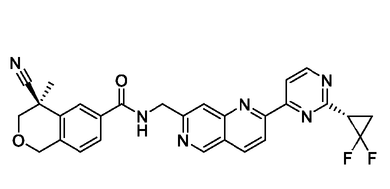 | 418 | 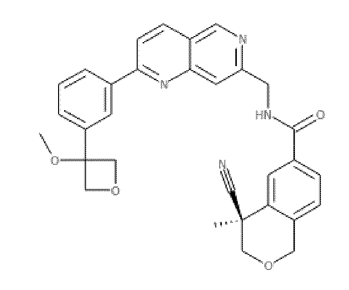 |
| 219 | 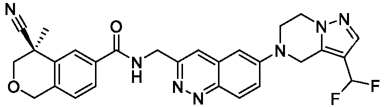 | 419 | 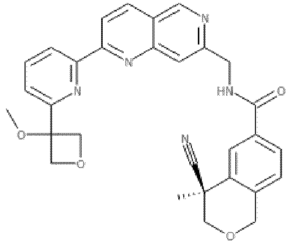 |
| 220 | 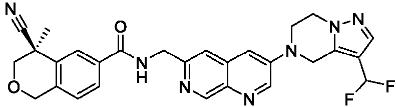 | 420 | 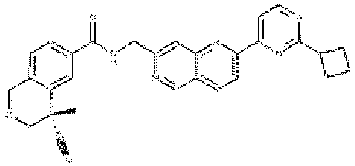 |
| 221 | 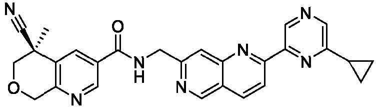 | 421 | 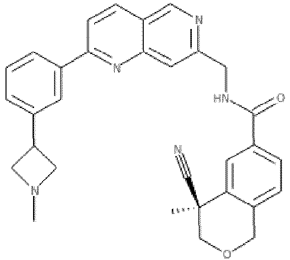 |
| 222 | 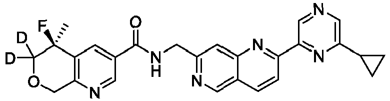 | 422 | 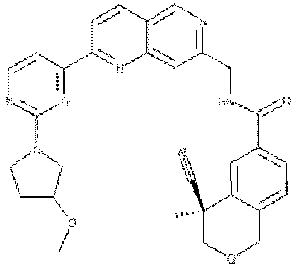 |
| 223 | 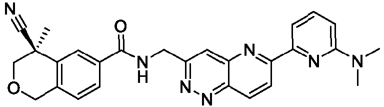 | 423 | 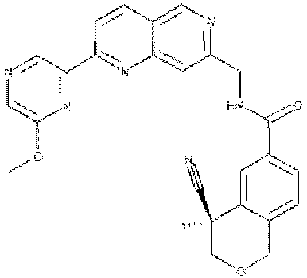 |
| 224 | 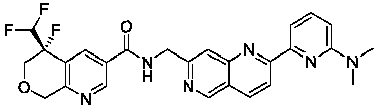 | 424 | 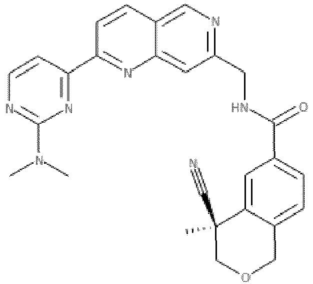 |
| 225 | 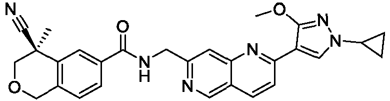 | 425 | 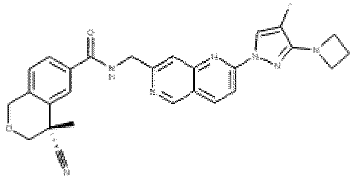 |
| 226 | 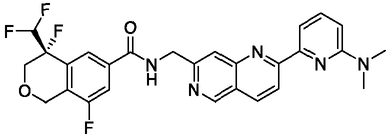 | 426 | 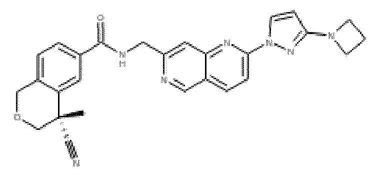 |
| 227 | 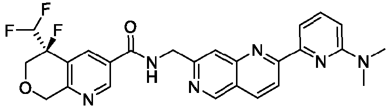 | 427 | 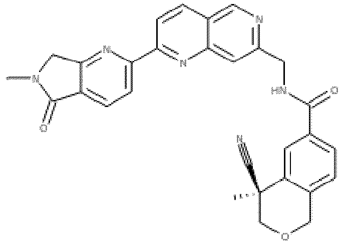 |
| 228 | 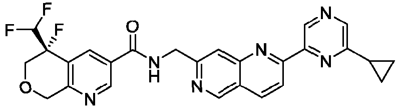 | 428 | 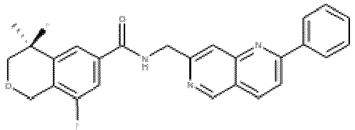 |
| 229 | 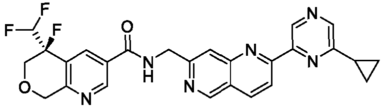 | 429 | 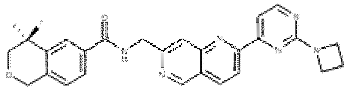 |
| 230 | 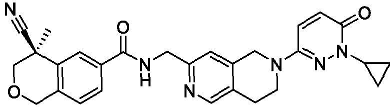 | 430 | 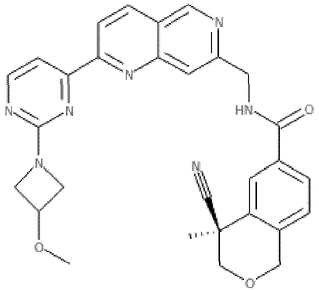 |
| 231 | 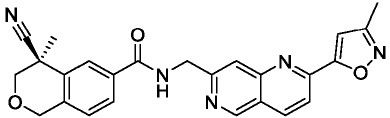 | 431 | 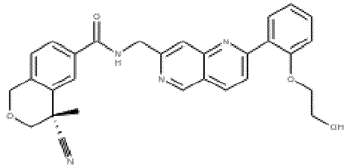 |
| 232 | 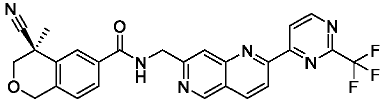 | 432 | 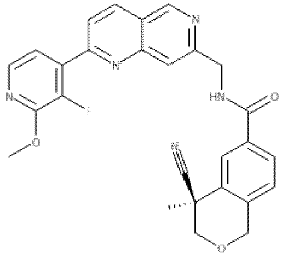 |
| 233 | 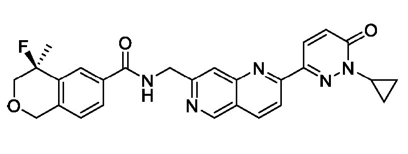 | 433 | 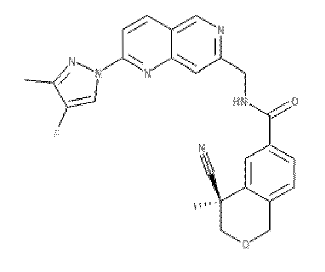 |
| 234 | 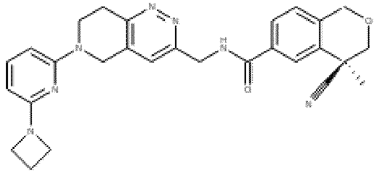 | 434 | 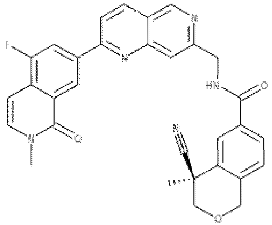 |
| 235 | 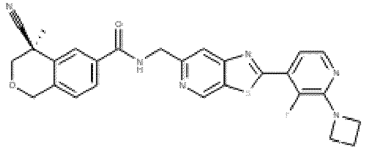 | 435 | 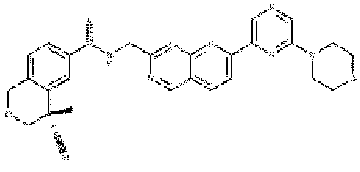 |
| 236 | 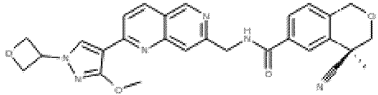 | 436 | 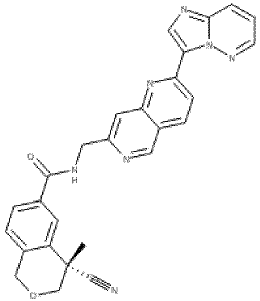 |
| 237 | 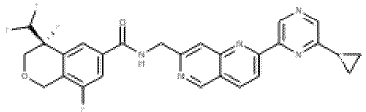 | 437 | 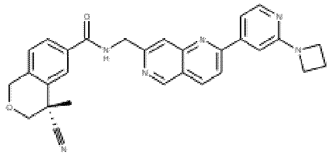 |
| 238 | 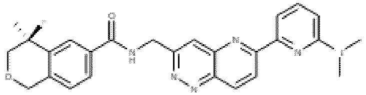 | 438 | 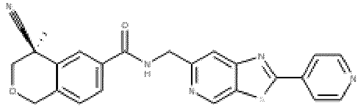 |
| 239 | 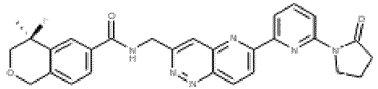 | 439 | 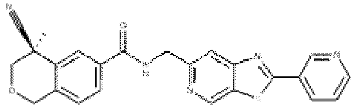 |
| 240 | 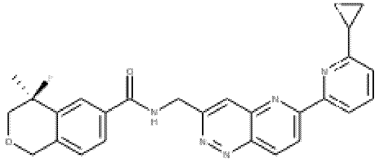 | 440 | 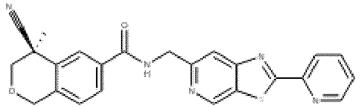 |
| 241 | 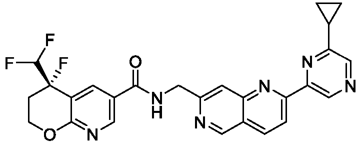 | 441 | 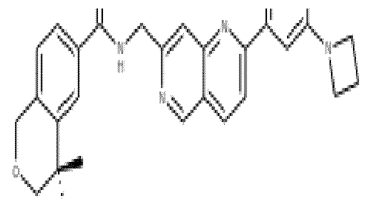 |
| 242 | 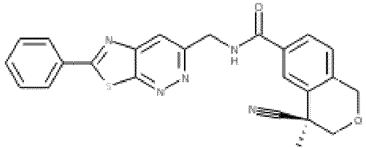 | 442 | 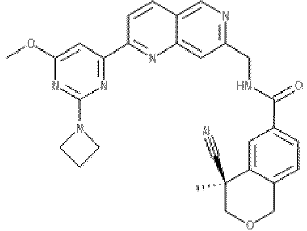 |
| 243 | 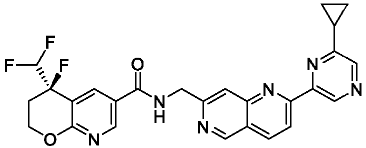 | 443 | 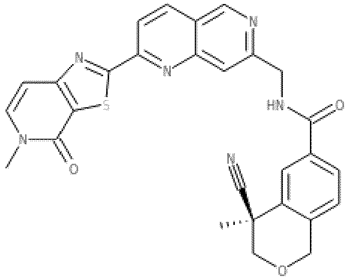 |
| 244 | 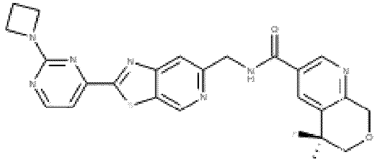 | 444 | 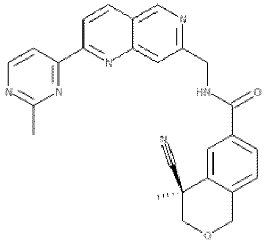 |
| 245 | 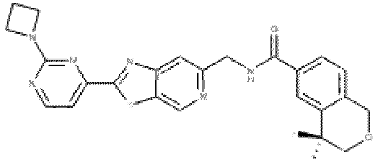 | 445 | 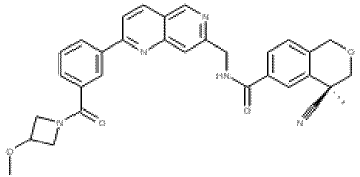 |
| 246 | 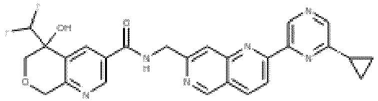 | 446 | 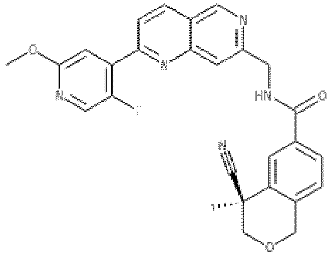 |
| 247 | 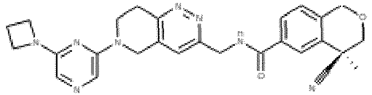 | 447 | 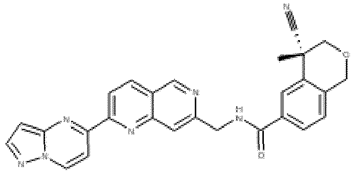 |
| 248 | 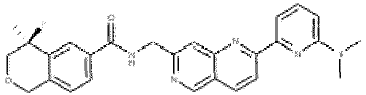 | 448 | 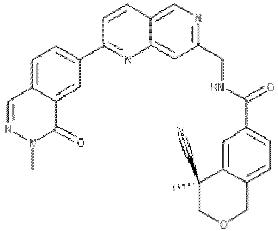 |
| 249 | 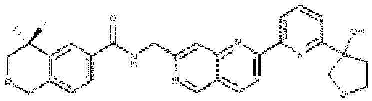 | 449 | 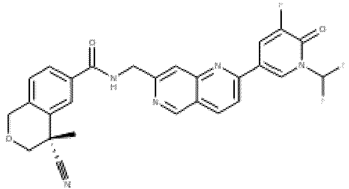 |
| 250 | 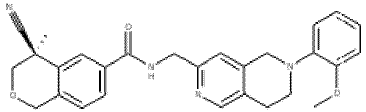 | 450 | 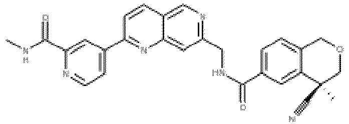 |
| 251 | 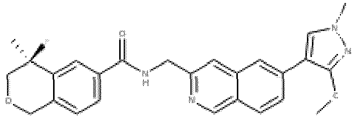 | 451 | 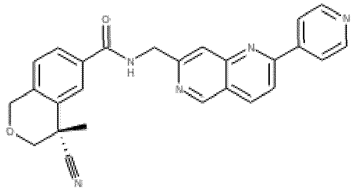 |
| 252 | 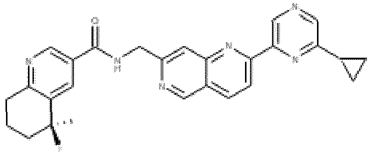 | 452 | 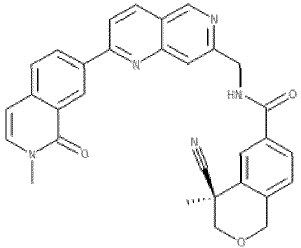 |
| 253 | 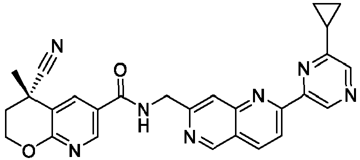 | 453 | 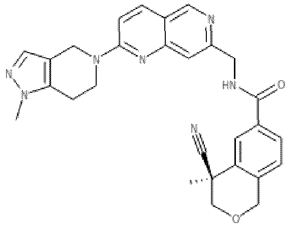 |
| 254 | 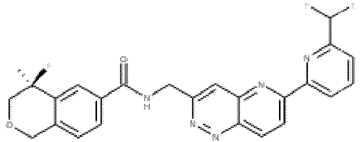 | 454 | 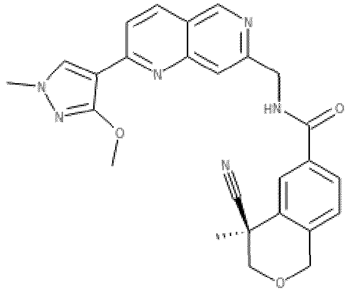 |
| 255 | 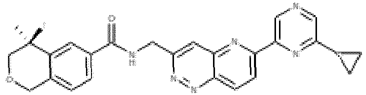 | 455 | 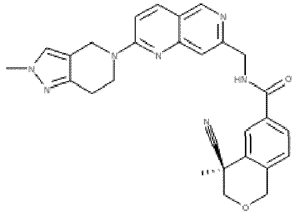 |
| 256 | 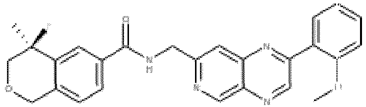 | 456 | 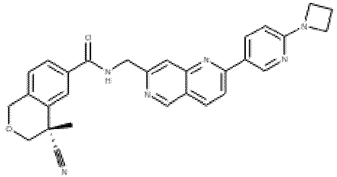 |
| 257 | 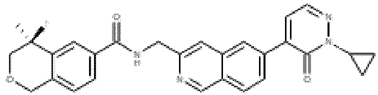 | 457 | 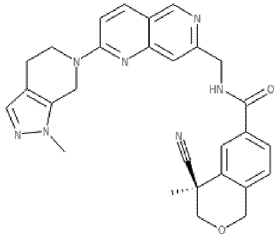 |
| 258 | 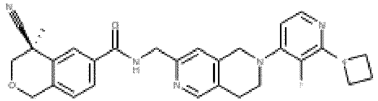 | 458 | 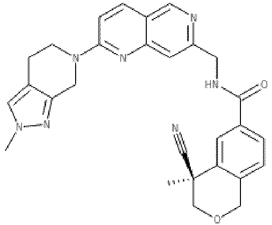 |
| 259 | 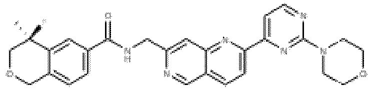 | 459 | 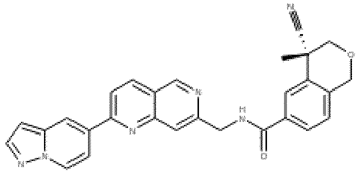 |
| 260 | 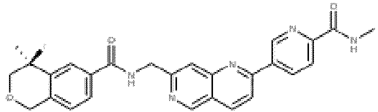 | 460 | 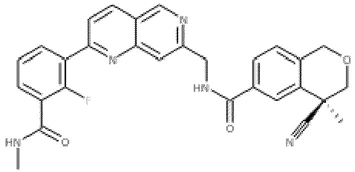 |
| 261 | 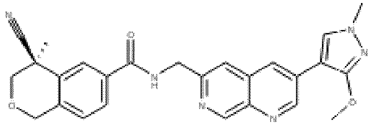 | 461 | 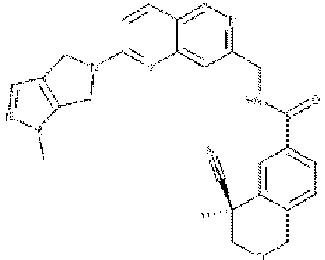 |
| 262 | 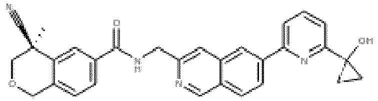 | 462 | 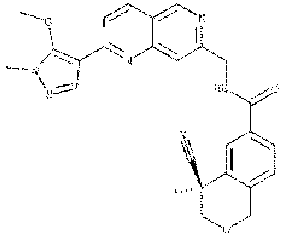 |
| 263 | 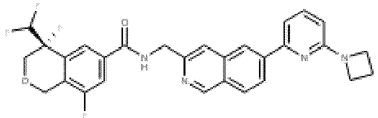 | 463 | 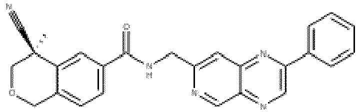 |
| 264 | 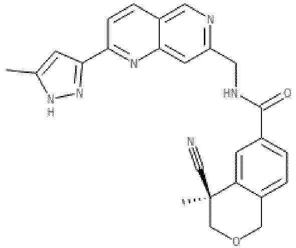 | 464 | 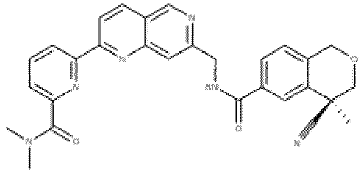 |
| 265 | 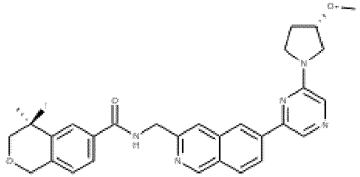 | 465 | 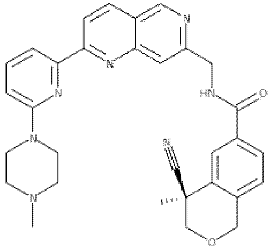 |
| 266 | 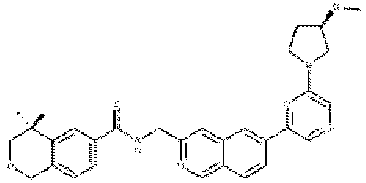 | 466 | 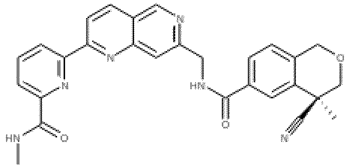 |
| 267 | 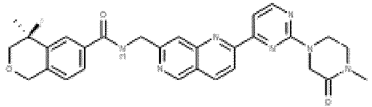 | 467 | 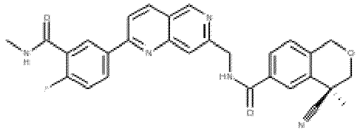 |
| 268 | 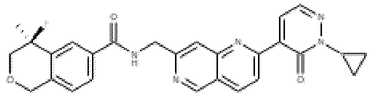 | 468 | 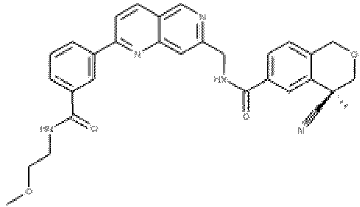 |
| 269 | 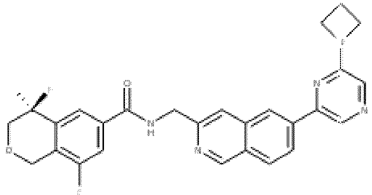 | 469 | 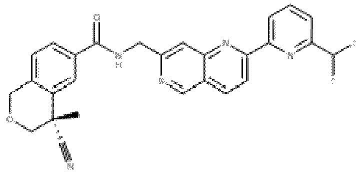 |
| 270 | 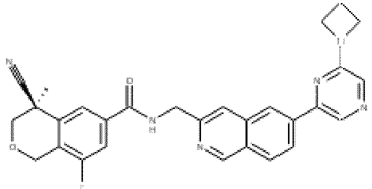 | 470 | 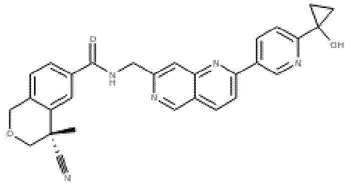 |
| 271 | 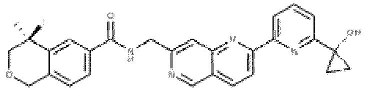 | 471 | 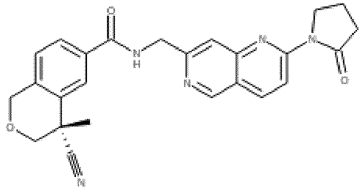 |
| 272 | 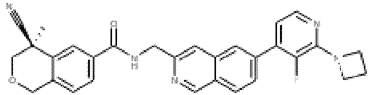 | 472 | 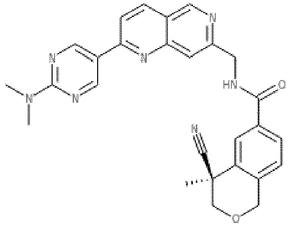 |
| 273 | 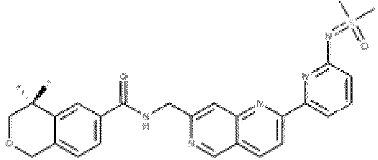 | 473 | 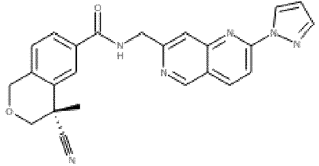 |
| 274 | 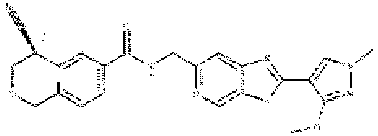 | 474 | 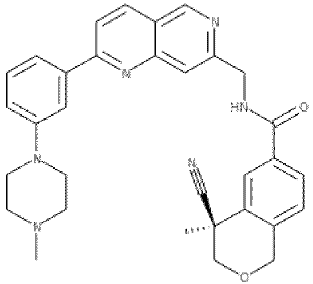 |
| 275 | 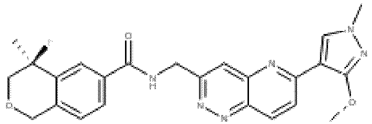 | 475 | 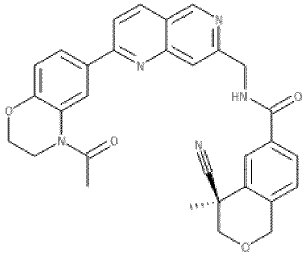 |
| 276 | 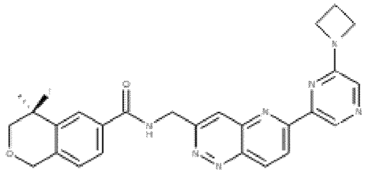 | 476 | 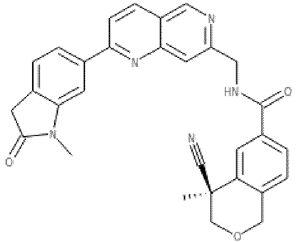 |
| 277 | 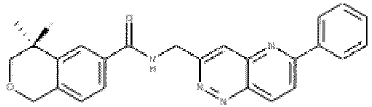 | 477 | 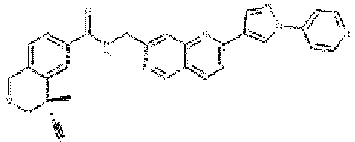 |
| 278 | 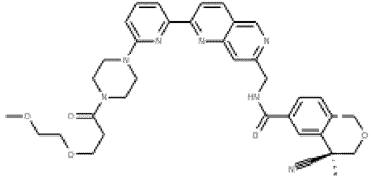 | 478 | 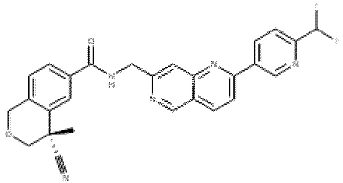 |
| 279 | 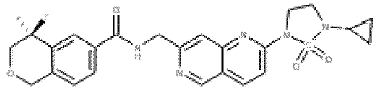 | 479 | 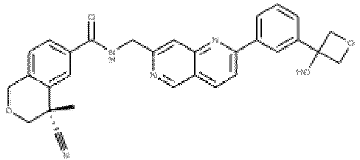 |
| 280 | 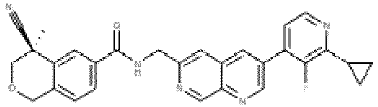 | 480 | 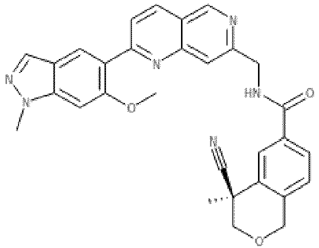 |
| 281 | 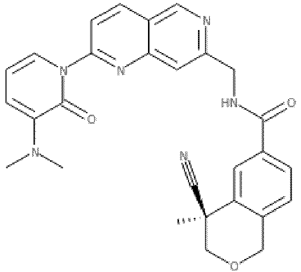 | 481 | 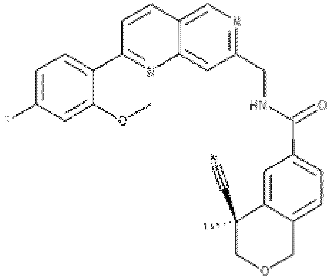 |
| 282 | 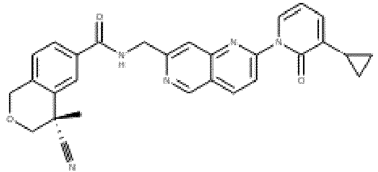 | 482 | 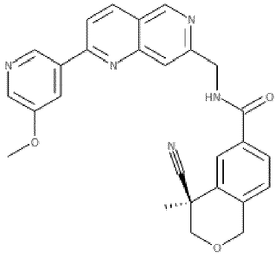 |
| 283 | 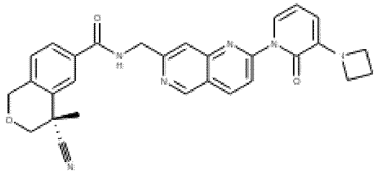 | 483 | 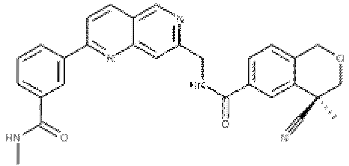 |
| 284 | 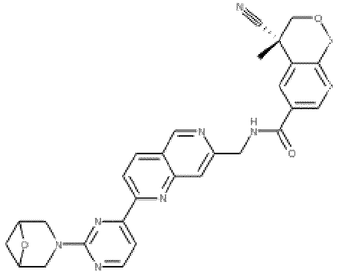 | 484 | 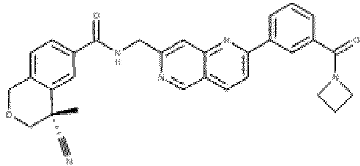 |
| 285 | 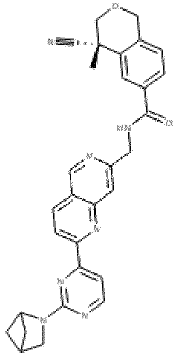 | 485 | 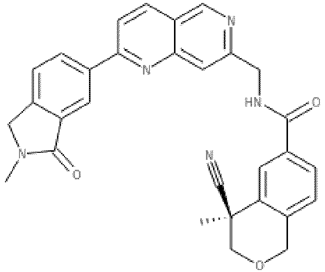 |
| 286 | 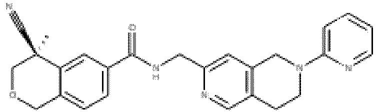 | 486 | 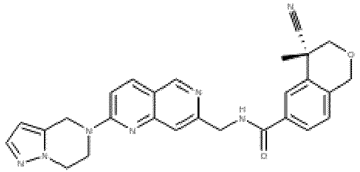 |
| 287 | 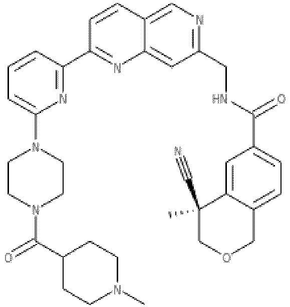 | 487 | 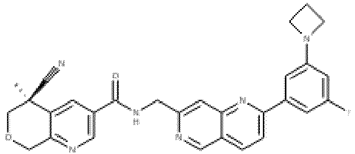 |
| 288 | 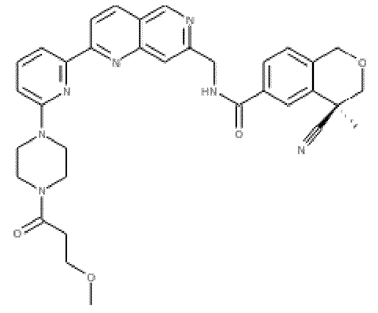 | 488 | 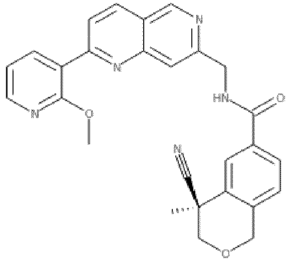 |
| 289 | 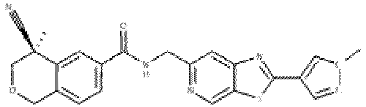 | 489 | 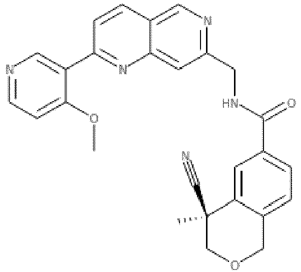 |
| 290 | 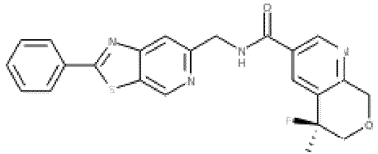 | 490 | 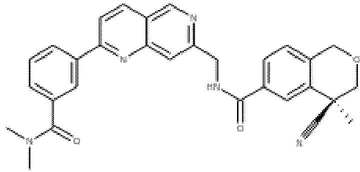 |
| 291 | 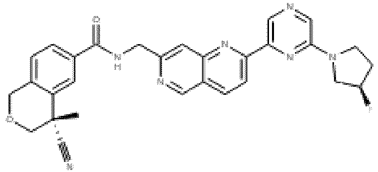 | 491 | 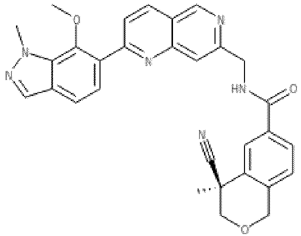 |
| 292 | 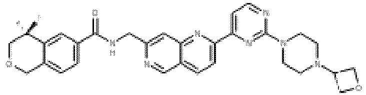 | 492 | 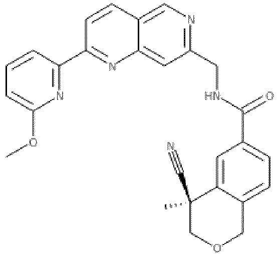 |
| 293 | 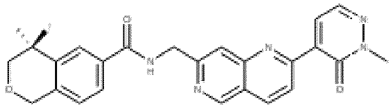 | 493 | 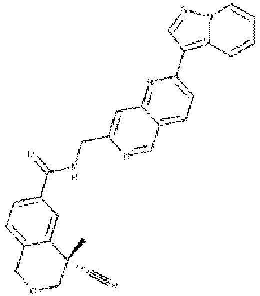 |
| 294 | 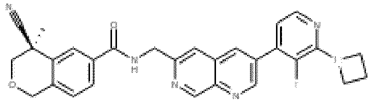 | 494 | 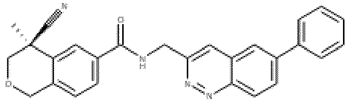 |
| 295 | 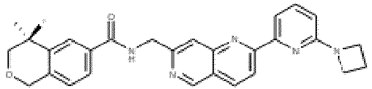 | 495 | 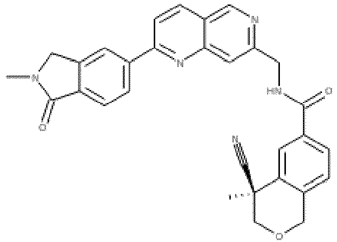 |
| 296 | 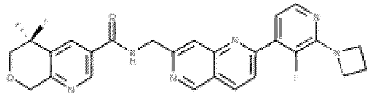 | 496 | 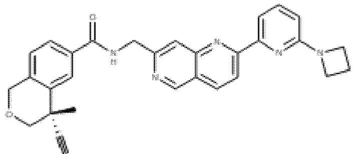 |
| 297 | 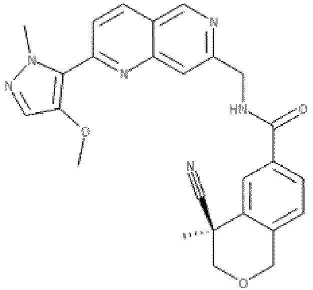 | 497 | 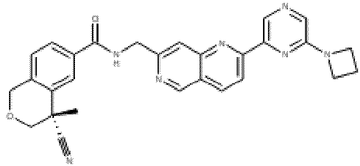 |
| 298 | 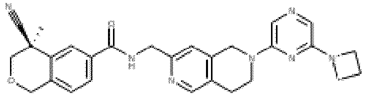 | 498 | 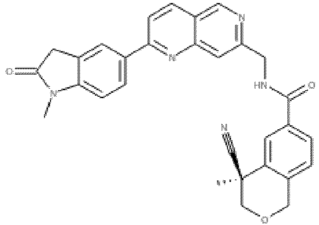 |
| 299 | 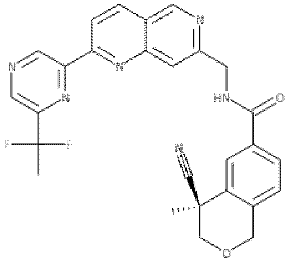 | 499 | 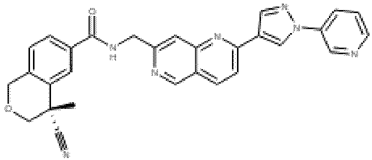 |
| 300 | 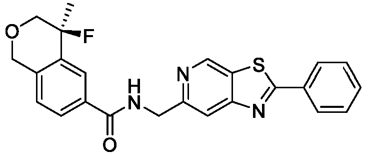 | 500 | 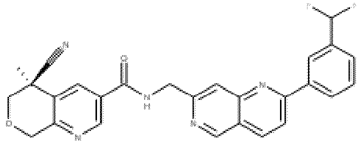 |
| 301 | 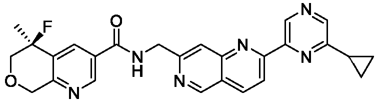 | 501 | 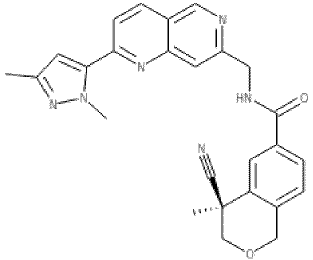 |
| 302 | 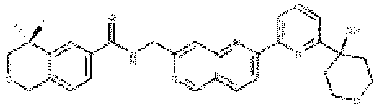 | 502 | 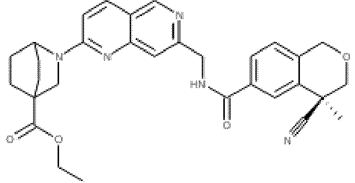 |
| 303 | 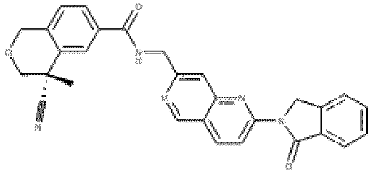 | 503 | 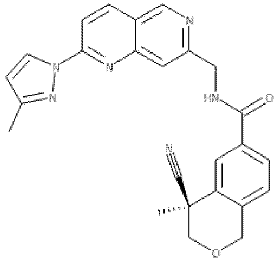 |
| 304 | 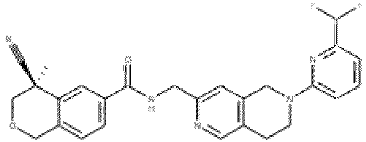 | 504 | 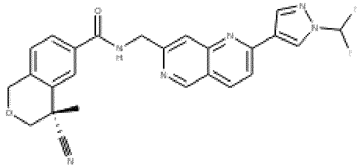 |
| 305 | 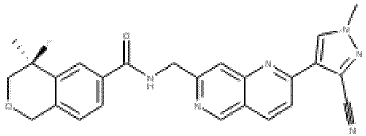 | 505 | 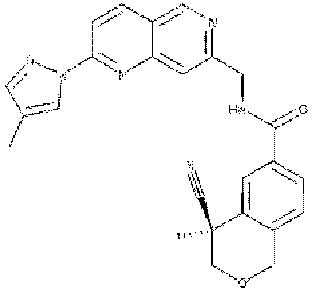 |
| 306 | 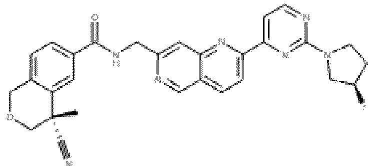 | 506 | 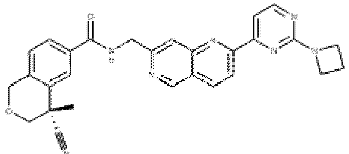 |
| 307 | 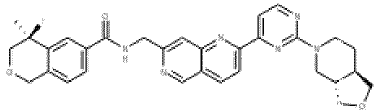 | 507 | 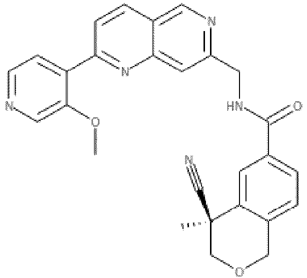 |
| 308 | 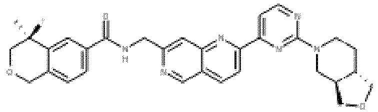 | 508 | 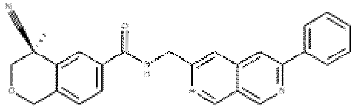 |
| 309 | 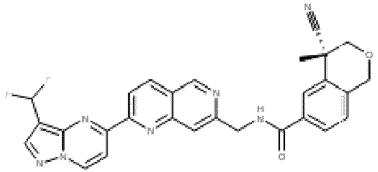 | 509 | 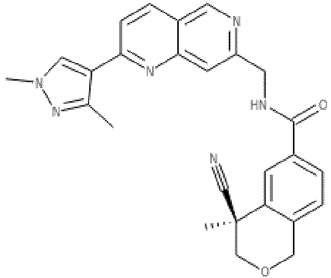 |
| 310 | 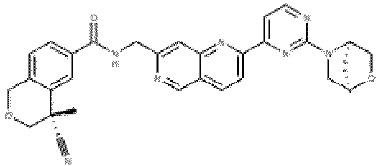 | 510 | 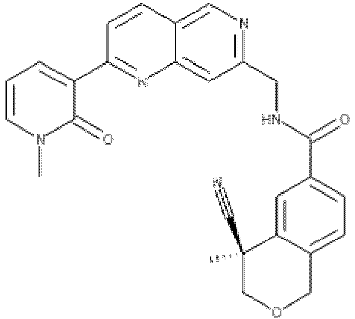 |
| 311 | 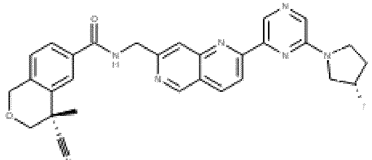 | 511 | 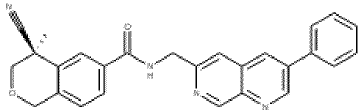 |
| 312 | 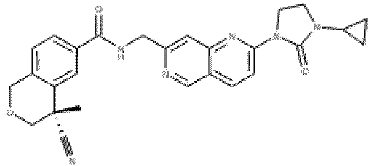 | 512 | 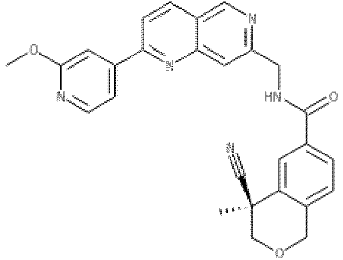 |
| 313 | 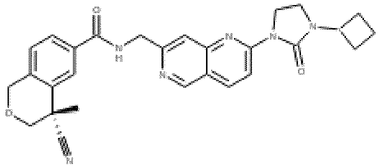 | 513 | 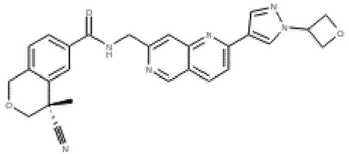 |
| 314 | 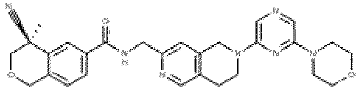 | 514 | 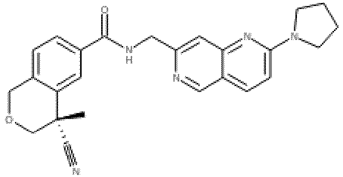 |
| 315 | 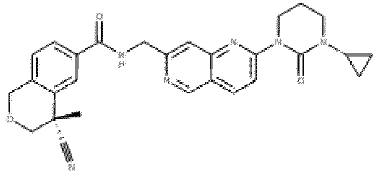 | 515 | 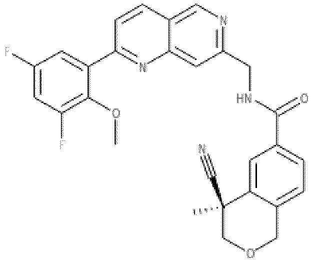 |
| 316 | 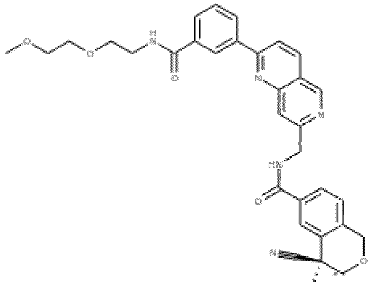 | 516 | 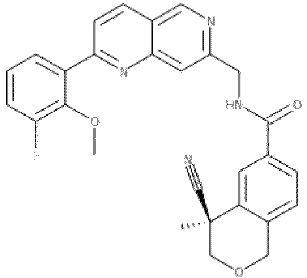 |
| 317 | 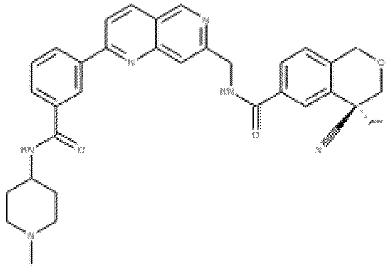 | 517 | 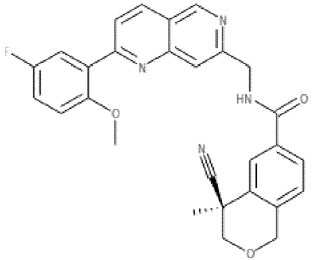 |
| 318 | 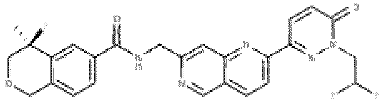 | 518 | 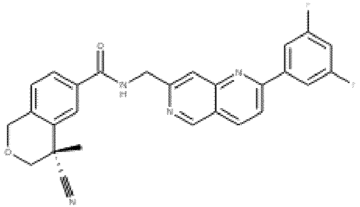 |
| 319 | 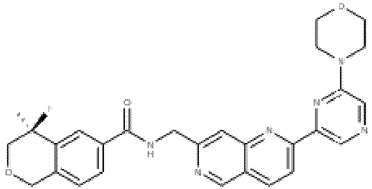 | 519 | 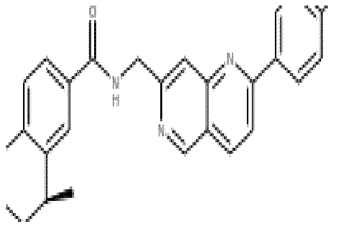 |
| 320 | 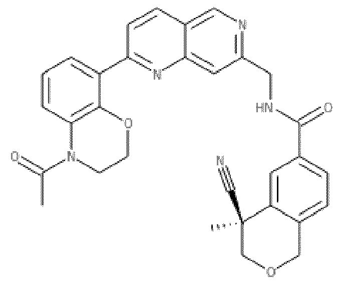 | 520 | 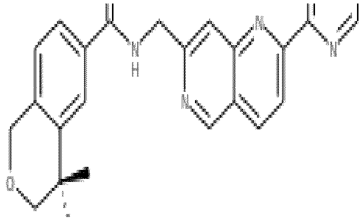 |
| 321 | 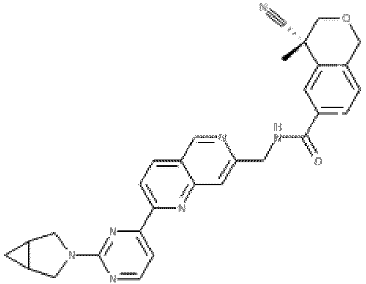 | 521 | 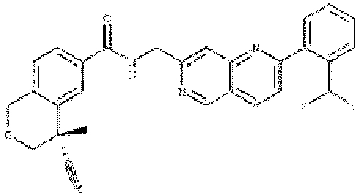 |
| 322 | 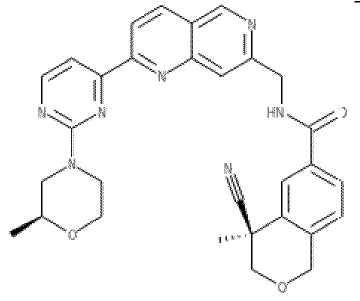 | 522 | 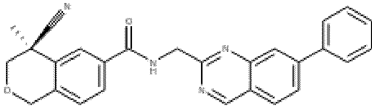 |
| 323 | 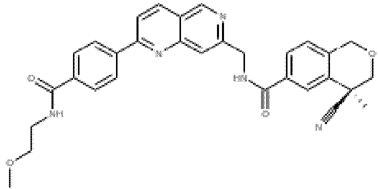 | 523 | 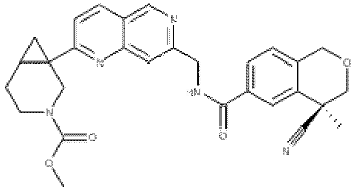 |
| 324 | 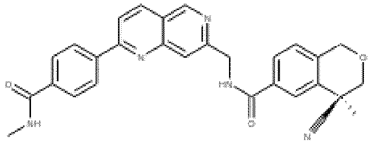 | 524 | 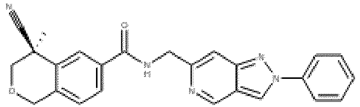 |
| 325 | 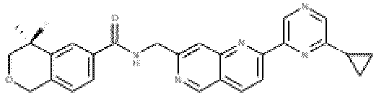 | 525 | 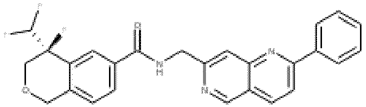 |
| 326 | 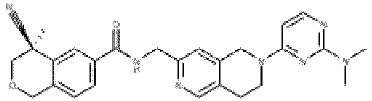 | | |

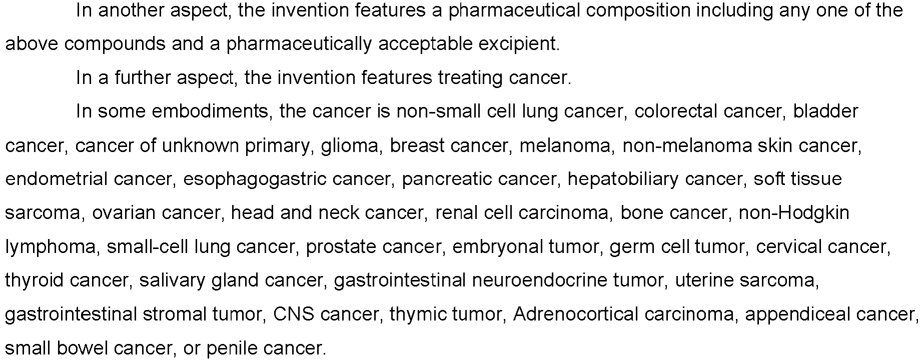

In some embodiments, the cancer is non-small cell lung cancer, colorectal cancer, bladder cancer, cancer of unknown primary, glioma, breast cancer, melanoma, non-melanoma skin cancer, endometrial cancer, or penile cancer.

In some embodiments, the cancer is a drug resistant cancer or has failed to respond to a prior therapy (e.g., vemurafenib, dacarbazine, a CTLA4 inhibitor, a PD1 inhibitor, interferon therapy, a BRAF inhibitor, a MEK inhibitor, radiotherapy, temozolimide, irinotecan, a CAR-T therapy, herceptin, perjeta, tamoxifen, xeloda, docetaxol, platinum agents such as carboplatin, taxanes such as paclitaxel and docetaxel, ALK inhibitors, MET inihibitors, alimta, abraxane, Adriamycin^{®}, gemcitabine, avastin, halaven, neratinib, a PARP inhibitor, ARN810, an mTOR inhibitor, topotecan, gemzar, a VEGFR2 inhibitor, a folate receptor antagonist, demcizumab, fosbretabulin, or a PDL1 inhibitor).

In some embodiments, the cancer has or has been determined to have BRG1 mutations. In some embodiments, the BRG1 mutations are homozygous. In some embodiments, the cancer does not have, or has been determined not to have, an epidermal growth factor receptor (EGFR) mutation. In some embodiments, the cancer does not have, or has been determined not to have, an anaplastic lymphoma kinase (ALK) driver mutation. In some embodiments, the cancer has, or has been determined to have, a KRAS mutation. In some embodiments, the BRG1 mutation is in the ATPase catalytic domain of the protein. In some embodiments, the BRG1 mutation is a deletion at the C-terminus of BRG1.

In another aspect, the disclosure provides for treating a viral infection. In some embodiments, the viral infection is an infection with a virus of the Retroviridae family such as the lentiviruses (e.g., Human immunodeficiency virus (HIV) and deltaretroviruses (e.g., human T cell leukemia virus I (HTLV-I), human T cell leukemia virus II (HTLV-II)), Hepadnaviridae family (e.g., hepatitis B virus (HBV)), Flaviviridae family (e.g., hepatitis C virus (HCV)), Adenoviridae family (e.g., Human Adenovirus), Herpesviridae family (e.g., Human cytomegalovirus (HCMV), Epstein-Barr virus, herpes simplex virus 1 (HSV-1), herpes simplex virus 2 (HSV-2), human herpesvirus 6 (HHV-6), Herpesvitus K*, CMV, varicella-zoster virus), Papillomaviridae family (e.g., Human Papillomavirus (HPV, HPV E1)), Parvoviridae family (e.g., Parvovirus B19), Polyomaviridae family (e.g., JC virus and BK virus), Paramyxoviridae family (e.g., Measles virus), or Togaviridae family (e.g., Rubella virus).

In another aspect, the invention features treating melanoma, prostate cancer, breast cancer, bone cancer, renal cell carcinoma, or a hematologic cancer.

In another aspect, the invention features reducing tumor growth of melanoma, prostate cancer, breast cancer, bone cancer, renal cell carcinoma, or a hematologic cancer.

In another aspect, the invention features suppressing metastatic progression of melanoma, prostate cancer, breast cancer, bone cancer, renal cell carcinoma, or a hematologic cancer.

In another aspect, the invention features suppressing metastatic colonization of melanoma, prostate cancer, breast cancer, bone cancer, renal cell carcinoma, or a hematologic cancer.

In another aspect, the invention features reducing the level and/or activity of BRG1 and/or BRM in a melanoma, prostate cancer, breast cancer, bone cancer, renal cell carcinoma, or hematologic cancer cell.

In some embodiments of any of the above aspects, the melanoma, prostate cancer, breast cancer, bone cancer, renal cell carcinoma, or hematologic cell is in a subject.

In some embodiments, the subject has cancer. In some embodiments, the cancer expresses BRG1 and/or BRM protein and/or the cell or subject has been identified as expressing BRG1 and/or BRM. In some embodiments, the cancer expresses BRG1 protein and/or the cell or subject has been identified as expressing BRG1. In some embodiments, the cancer expresses BRM protein and/or the cell or subject has been identified as expressing BRM. In some embodiments, the cancer is melanoma (e.g., uveal melanoma, mucosal melanoma, or cutaneous melanoma). In some embodiments, the cancer is prostate cancer. In some embodiments, the cancer is a hematologic cancer, e.g., multiple myeloma, large cell lymphoma, acute T-cell leukemia, acute myeloid leukemia, myelodysplastic syndrome, immunoglobulin A lambda myeloma, diffuse mixed histiocytic and lymphocytic lymphoma, B-cell lymphoma, acute lymphoblastic leukemia (e.g., T-cell acute lymphoblastic leukemia or B-cell acute lymphoblastic leukemia), diffuse large cell lymphoma, or non-Hodgkin's lymphoma. In some embodiments, the cancer is breast cancer (e.g., an ER positive breast cancer, an ER negative breast cancer, triple positive breast cancer, or triple negative breast cancer). In some embodiments, the cancer is a bone cancer (e.g., Ewing's sarcoma). In some embodiments, the cancer is a renal cell carcinoma (e.g., a Microphthalmia Transcription Factor (MITF) family translocation renal cell carcinoma (tRCC)). In some embodiments, the cancer is metastatic (e.g., the cancer has spread to the liver). The metastatic cancer can include cells exhibiting migration and/or invasion of migrating cells and/or include cells exhibiting endothelial recruitment and/or angiogenesis. In other embodiments, the migrating cancer is a cell migration cancer. In still other embodiments, the cell migration cancer is a non-metastatic cell migration cancer. The metastatic cancer can be a cancer spread via seeding the surface of the peritoneal, pleural, pericardial, or subarachnoid spaces. Alternatively, the metastatic cancer can be a cancer spread via the lymphatic system, or a cancer spread hematogenously. In some embodiments, the effective amount of a compound of the invention is an amount effective to inhibit metastatic colonization of the cancer to the liver.

In some embodiments the cancer harbors a mutation in GNAQ. In some embodiments the cancer harbors a mutation in GNA11. In some embodiments the cancer harbors a mutation in PLCB4. In some embodiments the cancer harbors a mutation in CYSLTR2. In some embodiments the cancer harbors a mutation in BAP1. In some embodiments the cancer harbors a mutation in SF3B1. In some embodiments the cancer harbors a mutation in EIF1AX. In some embodiments the cancer harbors a TFE3 translocation. In some embodiments the cancer harbors a TFEB translocation. In some embodiments the cancer harbors a MITF translocation. In some embodiments the cancer harbors an EZH2 mutation. In some embodiments the cancer harbors a SUZ12 mutation. In some embodiments the cancer harbors an EED mutation.

Some embodiments further include an anticancer therapy, e.g., a chemotherapeutic or cytotoxic agent, immunotherapy, surgery, radiotherapy, thermotherapy, or photocoagulation, or a combination thereof. In some embodiments, the anticancer therapy is a chemotherapeutic or cytotoxic agent, e.g., an antimetabolite, antimitotic, antitumor antibiotic, asparagine-specific enzyme, bisphosphonates, antineoplastic, alkylating agent, DNA-Repair enzyme inhibitor, histone deacetylase inhibitor, corticosteroid, demethylating agent, immunomodulatory, janus-associated kinase inhibitor, phosphinositide 3-kinase inhibitor, proteasome inhibitor, or tyrosine kinase inhibitor, or a combination thereof.

In some embodiments, the compound of the invention is used in combination with another anti-cancer therapy used for the treatment of uveal melanoma such as surgery, a MEK inhibitor, and/or a PKC inhibitor, or a combination thereof. For example, some embodiments, further comprises performing surgery prior to, subsequent to, or at the same time as administration of the compound of the invention. Some embodiments, the further comprises administration of a MEK inhibitor and/or a PKC inhibitor prior to, subsequent to, or at the same time as administration of the compound of the invention.

In some embodiments, the anticancer therapy and the compound of the invention are administered within 28 days of each other and each in an amount that together are effective to treat the subject.

In some embodiments, the cancer has and/or has been identified as having a BRG1 loss of function mutation. In some embodiments, the subject or cancer has and/or has been identified as having a BRM loss of function mutation.

In some embodiments, the cancer is resistant to one or more chemotherapeutic or cytotoxic agents (e.g., the cancer has been determined to be resistant to chemotherapeutic or cytotoxic agents such as by genetic markers, or is likely to be resistant, to chemotherapeutic or cytotoxic agents such as a cancer that has failed to respond to a chemotherapeutic or cytotoxic agent). In some embodiments, the cancer has failed to respond to one or more chemotherapeutic or cytotoxic agents. In some embodiments, the cancer is resistant or has failed to respond to dacarbazine, temozolomide, cisplatin, treosulfan, fotemustine, IMCgp100, a CTLA-4 inhibitor (e.g., ipilimumab), a PD-1 inhibitor (e.g., Nivolumab or pembrolizumab), a PD-L1 inhibitor (e.g., atezolizumab, avelumab, or durvalumab), a mitogen-activated protein kinase (MEK) inhibitor (e.g., selumetinib, binimetinib, or tametinib), and/or a protein kinase C (PKC) inhibitor (e.g., sotrastaurin or IDE196).

In some embodiments, the cancer is resistant to or failed to respond to a previously administered therapeutic used for the treatment of uveal melanoma such as a MEK inhibitor or PKC inhibitor. For example, in some embodiments, the cancer is resistant to or failed to respond to a mitogen-activated protein kinase (MEK) inhibitor (e.g., selumetinib, binimetinib, or tametinib), and/or a protein kinase C (PKC) inhibitor (e.g., sotrastaurin or IDE196).

### Chemical Terms

The terminology employed herein is for the purpose of describing particular embodiments.

For any of the following chemical definitions, a number following an atomic symbol indicates that total number of atoms of that element that are present in a particular chemical moiety. As will be understood, other atoms, such as H atoms, or substituent groups, as described herein, may be present, as necessary, to satisfy the valences of the atoms. For example, an unsubstituted C₂ alkyl group has the formula -CH₂CH₃. When used with the groups defined herein, a reference to the number of carbon atoms includes the divalent carbon in acetal and ketal groups but does not include the carbonyl carbon in acyl, ester, carbonate, or carbamate groups. A reference to the number of oxygen, nitrogen, or sulfur atoms in a heteroaryl group only includes those atoms that form a part of a heterocyclic ring.

The term "acyl," as used herein, represents a H or an alkyl group that is attached to a parent molecular group through a carbonyl group, as defined herein, and is exemplified by formyl (i.e., a carboxyaldehyde group), acetyl, trifluoroacetyl, propionyl, and butanoyl. Exemplary unsubstituted acyl groups include from 1 to 6, from 1 to 11, or from 1 to 21 carbons.

The term "alkyl," as used herein, refers to a branched or straight-chain monovalent saturated aliphatic hydrocarbon radical of 1 to 20 carbon atoms (e.g., 1 to 16 carbon atoms, 1 to 10 carbon atoms, 1 to 6 carbon atoms, or 1 to 3 carbon atoms).

An alkylene is a divalent alkyl group. The term "alkenyl," as used herein, alone or in combination with other groups, refers to a straight chain or branched hydrocarbon residue having a carbon-carbon double bond and having 2 to 20 carbon atoms (e.g., 2 to 16 carbon atoms, 2 to 10 carbon atoms, 2 to 6 carbon atoms, or 2 carbon atoms).

The term "alkylsulfinimide," as used herein, refers to a group of formula -N=S(O)R₂, where each R is independently an alkyl.

The term "alkynyl," as used herein, alone or in combination with other groups, refers to a straight chain or branched hydrocarbon residue having a carbon-carbon triple bond and having 2 to 20 carbon atoms (e.g., 2 to 16 carbon atoms, 2 to 10 carbon atoms, 2 to 6 carbon atoms, or 2 carbon atoms).

The term "amino," as used herein, represents -N(R^{N1})₂, wherein each R^{N1} is, independently, H, OH, NO₂, N(R^{N2})₂, SO₂O_{R}^{N2}, SO_{2R}^{N2}, SOR^{N2}, an N-protecting group, alkyl, alkoxy, aryl, arylalkyl, cycloalkyl, acyl (e.g., acetyl, trifluoroacetyl, or others described herein), wherein each of these recited R^{N1} groups can be optionally substituted; or two R^{N1} combine to form an alkylene or heteroalkylene, and wherein each R^{N2} is, independently, H, alkyl, or aryl. The amino groups of the invention can be an unsubstituted amino (i.e., -NH₂) or a substituted amino (i.e., -N(R^{N1})₂).

The term "aryl," as used herein, refers to an aromatic mono- or polycarbocyclic radical of 6 to 12 carbon atoms having at least one aromatic ring. Examples of such groups include, but are not limited to, phenyl, naphthyl, 1,2,3,4-tetrahydronaphthyl, 1,2-dihydronaphthyl, indanyl, and 1H-indenyl.

The term "arylalkyl," as used herein, represents an alkyl group substituted with an aryl group. Exemplary unsubstituted arylalkyl groups are from 7 to 30 carbons (e.g., from 7 to 16 or from 7 to 20 carbons, such as C₁-C₆ alkyl C₆-C₁₀ aryl, C₁-C₁₀ alkyl C₆-C₁₀ aryl, or C₁-C₂₀ alkyl C₆-C₁₀ aryl), such as, benzyl and phenethyl. In some embodiments, the alkyl and the aryl each can be further substituted with 1, 2, 3, or 4 substituent groups as defined herein for the respective groups.

The term "azido," as used herein, represents a -N₃ group.

The term "bridged polycycloalkyl," as used herein, refers to a bridged polycyclic group of 5 to 20 carbons, containing from 1 to 3 bridges.

The term "cyano," as used herein, represents a -CN group.

The term "carbocyclyl," as used herein, refers to a non-aromatic C₃-C₁₂ monocyclic, bicyclic, or tricyclic structure in which the rings are formed by carbon atoms. Carbocyclyl structures include cycloalkyl groups and unsaturated carbocyclyl radicals.

The term "cycloalkyl," as used herein, refers to a saturated, non-aromatic, and monovalent mono- or polycarbocyclic radical of 3 to 10, preferably 3 to 6 carbon atoms. This term is further exemplified by radicals such as cyclopropyl, cyclobutyl, cyclopentyl, cyclohexyl, cycloheptyl, norbornyl, and adamantyl.

The term "halo," as used herein, means a fluorine (fluoro), chlorine (chloro), bromine (bromo), or iodine (iodo) radical.

The term "heteroalkyl," as used herein, refers to an alkyl group, as defined herein, in which one or more of the constituent carbon atoms have been replaced by nitrogen, oxygen, or sulfur. In some embodiments, the heteroalkyl group can be further substituted with 1, 2, 3, or 4 substituent groups as described herein for alkyl groups. Examples of heteroalkyl groups are an "alkoxy" which, as used herein, refers alkyl-O- (e.g., methoxy and ethoxy). A heteroalkylene is a divalent heteroalkyl group. The term "heteroalkenyl," as used herein, refers to an alkenyl group, as defined herein, in which one or more of the constituent carbon atoms have been replaced by nitrogen, oxygen, or sulfur. In some embodiments, the heteroalkenyl group can be further substituted with 1, 2, 3, or 4 substituent groups as described herein for alkenyl groups. Examples of heteroalkenyl groups are an "alkenoxy" which, as used herein, refers alkenyl-O-. A heteroalkenylene is a divalent heteroalkenyl group. The term "heteroalkynyl," as used herein, refers to an alkynyl group, as defined herein, in which one or more of the constituent carbon atoms have been replaced by nitrogen, oxygen, or sulfur. In some embodiments, the heteroalkynyl group can be further substituted with 1, 2, 3, or 4 substituent groups as described herein for alkynyl groups. Examples of heteroalkynyl groups are an "alkynoxy" which, as used herein, refers alkynyl-O-. A heteroalkynylene is a divalent heteroalkynyl group.

The term "heteroaryl," as used herein, refers to an aromatic mono- or polycyclic radical of 5 to 12 atoms having at least one aromatic ring containing 1, 2, or 3 ring atoms selected from nitrogen, oxygen, and sulfur, with the remaining ring atoms being carbon. One or two ring carbon atoms of the heteroaryl group may be replaced with a carbonyl group. Examples of heteroaryl groups are pyridyl, pyrazoyl, benzooxazolyl, benzoimidazolyl, benzothiazolyl, imidazolyl, oxaxolyl, and thiazolyl. Heteroarylene is a divalent heteroaryl.

The term "heteroarylalkyl," as used herein, represents an alkyl group substituted with a heteroaryl group. Exemplary unsubstituted heteroarylalkyl groups are from 7 to 30 carbons (e.g., from 7 to 16 or from 7 to 20 carbons, such as C₁-C₆ alkyl C₂-C₉ heteroaryl, C₁-C₁₀ alkyl C₂-C₉ heteroaryl, or C₁-C₂₀ alkyl C₂-C₉ heteroaryl). In some embodiments, the alkyl and the heteroaryl each can be further substituted with 1, 2, 3, or 4 substituent groups as defined herein for the respective groups.

The term "heterocyclyl," as used herein, refers a mono- or polycyclic radical having 3 to 12 atoms having at least one non-aromatic ring containing 1, 2, 3, or 4 ring atoms selected from N, O and S and no aromatic rings containing any N, O or S ring atoms. Examples of heterocyclyl groups include, but are not limited to, morpholinyl, thiomorpholinyl, piperazinyl, piperidinyl, pyranyl, pyrrolidinyl, tetrahydropyranyl, tetrahydrofuranyl, and 1,3-dioxanyl. Heterocyclylene is a divalent heterocyclyl.

The term "heterocyclylalkyl," as used herein, represents an alkyl group substituted with a heterocyclyl group. Exemplary unsubstituted heterocyclylalkyl groups are from 7 to 30 carbons (e.g., from 7 to 16 or from 7 to 20 carbons, such as C₁-C₆ alkyl C₂-C₉ heterocyclyl, C₁-C₁₀ alkyl C₂-C₉ heterocyclyl, or C₁-C₂₀ alkyl C₂-C₉ heterocyclyl). In some embodiments, the alkyl and the heterocyclyl each can be further substituted with 1, 2, 3, or 4 substituent groups as defined herein for the respective groups

The term "hydroxyalkyl," as used herein, represents alkyl group substituted with an -OH group.

The term "hydroxyl," as used herein, represents an -OH group.

The term "*N*-protecting group," as used herein, represents those groups intended to protect an amino group against undesirable reactions during synthetic procedures. Commonly used *N*-protecting groups are disclosed in Greene, "Protective Groups in Organic Synthesis," 3rd Edition (John Wiley & Sons, New York, 1999). *N*-protecting groups include, but are not limited to, acyl, aryloyl, or carbamyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and chiral auxiliaries such as protected or unprotected D, L, or D, L-amino acids such as alanine, leucine, and phenylalanine; sulfonyl-containing groups such as benzenesulfonyl, and p-toluenesulfonyl; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4- 20 dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxy carbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, and phenylthiocarbonyl, arylalkyl groups such as benzyl, triphenylmethyl, and benzyloxymethyl, and silyl groups, such as trimethylsilyl. Preferred *N*-protecting groups are alloc, formyl, acetyl, benzoyl, pivaloyl, t-butylacetyl, alanyl, phenylsulfonyl, benzyl, t-butyloxycarbonyl (Boc), and benzyloxycarbonyl (Cbz).

The term "nitro," as used herein, represents an -NO₂ group.

The term "oxo," as used herein, represents an =O group.

The term "thiol," as used herein, represents an -SH group.

The alkyl, alkenyl, alkynyl, heteroalkyl, heteroalkenyl, heteroalkynyl, carbocyclyl (e.g., cycloalkyl), aryl, heteroaryl, and heterocyclyl groups may be substituted or unsubstituted. When substituted, there will generally be 1 to 4 substituents present, unless otherwise specified. Substituents include, for example: alkyl (e.g., unsubstituted and substituted, where the substituents include any group described herein, e.g., aryl, halo, hydroxy), alkynyl (e.g., unsubstituted and substituted, where the substituents include any group described herein, e.g., aryl, halo, hydroxy, or alkoxy), aryl (e.g., substituted and unsubstituted phenyl), carbocyclyl (e.g., substituted and unsubstituted cycloalkyl), halo (e.g., fluoro), hydroxyl, heteroalkyl (e.g., substituted and unsubstituted methoxy, ethoxy, or thioalkoxy), heteroaryl, heterocyclyl, amino (e.g., NH₂ or mono- or dialkyl amino), alkylsulfinimide, azido, cyano, nitro, oxo, or thiol. In some embodiments (e.g., for the compounds of Formula la), substituents include, for example: alkyl (e.g., unsubstituted and substituted, where the substituents include any group described herein, e.g., aryl, halo, hydroxy), aryl (e.g., substituted and unsubstituted phenyl), carbocyclyl (e.g., substituted and unsubstituted cycloalkyl), halo (e.g., fluoro), hydroxyl, heteroalkyl (e.g., substituted and unsubstituted methoxy, ethoxy, or thioalkoxy), heteroaryl, heterocyclyl, amino (e.g., NH₂ or mono- or dialkyl amino), azido, cyano, nitro, or thiol. Aryl, carbocyclyl (e.g., cycloalkyl), heteroaryl, and heterocyclyl groups may also be substituted with alkyl (unsubstituted and substituted such as arylalkyl (e.g., substituted and unsubstituted benzyl)).

Compounds of the invention can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates, or mixtures of diastereoisomeric racemates. The optically active forms can be obtained for example by resolution of the racemates, by asymmetric synthesis or asymmetric chromatography (chromatography with a chiral adsorbents or eluant). That is, certain of the disclosed compounds may exist in various stereoisomeric forms. Stereoisomers are compounds that differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that are not related as mirror images, most commonly because they contain two or more asymmetrically substituted carbon atoms and represent the configuration of substituents around one or more chiral carbon atoms. Enantiomers of a compound can be prepared, for example, by separating an enantiomer from a racemate using one or more well-known techniques and methods, such as, for example, chiral chromatography and separation methods based thereon. The appropriate technique and/or method for separating an enantiomer of a compound described herein from a racemic mixture can be readily determined by those of skill in the art. "Racemate" or "racemic mixture" means a compound containing two enantiomers, wherein such mixtures exhibit no optical activity; i.e., they do not rotate the plane of polarized light. "Geometric isomer" means isomers that differ in the orientation of substituent atoms in relationship to a carbon-carbon double bond, to a cycloalkyl ring, or to a bridged bicyclic system. Atoms (other than H) on each side of a carbon- carbon double bond may be in an E (substituents are on opposite sides of the carbon- carbon double bond) or Z (substituents are oriented on the same side) configuration. "R," "S," "S*," "R*," "E," "Z," "cis," and "trans," indicate configurations relative to the core molecule. Certain of the disclosed compounds may exist in atropisomeric forms. Atropisomers are stereoisomers resulting from hindered rotation about single bonds where the steric strain barrier to rotation is high enough to allow for the isolation of the conformers. The compounds of the invention may be prepared as individual isomers by either isomer-specific synthesis or resolved from an isomeric mixture. Conventional resolution techniques include forming the salt of a free base of each isomer of an isomeric pair using an optically active acid (followed by fractional crystallization and regeneration of the free base), forming the salt of the acid form of each isomer of an isomeric pair using an optically active amine (followed by fractional crystallization and regeneration of the free acid), forming an ester or amide of each of the isomers of an isomeric pair using an optically pure acid, amine or alcohol (followed by chromatographic separation and removal of the chiral auxiliary), or resolving an isomeric mixture of either a starting material or a final product using various well known chromatographic methods. When the stereochemistry of a disclosed compound is named or depicted by structure, the named or depicted stereoisomer is at least 60%, 70%, 80%, 90%, 99%, or 99.9% by weight relative to the other stereoisomers. When a single enantiomer is named or depicted by structure, the depicted or named enantiomer is at least 60%, 70%, 80%, 90%, 99%, or 99.9% by weight optically pure. When a single diastereomer is named or depicted by structure, the depicted or named diastereomer is at least 60%, 70%, 80%, 90%, 99%, or 99.9% by weight pure. Percent optical purity is the ratio of the weight of the enantiomer or over the weight of the enantiomer plus the weight of its optical isomer. Diastereomeric purity by weight is the ratio of the weight of one diastereomer or over the weight of all the diastereomers. When the stereochemistry of a disclosed compound is named or depicted by structure, the named or depicted stereoisomer is at least 60%, 70%, 80%, 90%, 99%, or 99.9% by mole fraction pure relative to the other stereoisomers. When a single enantiomer is named or depicted by structure, the depicted or named enantiomer is at least 60%, 70%, 80%, 90%, 99%, or 99.9% by mole fraction pure. When a single diastereomer is named or depicted by structure, the depicted or named diastereomer is at least 60%, 70%, 80%, 90%, 99%, or 99.9% by mole fraction pure. Percent purity by mole fraction is the ratio of the moles of the enantiomer or over the moles of the enantiomer plus the moles of its optical isomer. Similarly, percent purity by moles fraction is the ratio of the moles of the diastereomer or over the moles of the diastereomer plus the moles of its isomer. When a disclosed compound is named or depicted by structure without indicating the stereochemistry, and the compound has at least one chiral center, it is to be understood that the name or structure encompasses either enantiomer of the compound free from the corresponding optical isomer, a racemic mixture of the compound, or mixtures enriched in one enantiomer relative to its corresponding optical isomer. When a disclosed compound is named or depicted by structure without indicating the stereochemistry and has two or more chiral centers, it is to be understood that the name or structure encompasses a diastereomer free of other diastereomers, a number of diastereomers free from other diastereomeric pairs, mixtures of diastereomers, mixtures of diastereomeric pairs, mixtures of diastereomers in which one diastereomer is enriched relative to the other diastereomer(s), or mixtures of diastereomers in which one or more diastereomer is enriched relative to the other diastereomers. The invention embraces all of these forms.

Compounds of the present disclosure also include all of the isotopes of the atoms occurring in the intermediate or final compounds. "Isotopes" refers to atoms having the same atomic number but different mass numbers resulting from a different number of neutrons in the nuclei. For example, isotopes of hydrogen include tritium and deuterium.

Unless otherwise stated, structures depicted herein are also meant to include compounds that differ only in the presence of one or more isotopically enriched atoms. Exemplary isotopes that can be incorporated into compounds of the present invention include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Isotopically-labeled compounds (e.g., those labeled with ³H and ¹⁴C) can be useful in compound or substrate tissue distribution assays. Tritiated (i.e., ³H) and carbon-14 (i.e., ¹⁴C) isotopes can be useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements). In some embodiments, one or more hydrogen atoms are replaced by ²H or ³H, or one or more carbon atoms are replaced by ¹³C- or ¹⁴C-enriched carbon. Positron emitting isotopes such as ¹⁵O, ¹³N, ¹¹C, and ¹⁸F are useful for positron emission tomography (PET) studies to examine substrate receptor occupancy. Preparations of isotopically labelled compounds are known to those of skill in the art. For example, isotopically labeled compounds can generally be prepared by following procedures analogous to those disclosed for compounds of the present invention described herein, by substituting an isotopically labeled reagent for a non-isotopically labeled reagent.

Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used.

### Definitions

In this application, unless otherwise clear from context, (i) the term "a" may be understood to mean "at least one"; (ii) the term "or" may be understood to mean "and/or"; and (iii) the terms "comprising" and "including" may be understood to encompass itemized components or steps whether presented by themselves or together with one or more additional components or steps.

As used herein, the terms "about" and "approximately" refer to a value that is within 10% above or below the value being described. For example, the term "about 5 nM" indicates a range of from 4.5 to 5.5 nM.

As used herein, the term "administration" refers to the administration of a composition (e.g., a compound or a preparation that includes a compound as described herein) to a subject or system. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intratumoral, intravenous, intraventricular, mucosal, nasal, oral, rectal, subcutaneous, sublingual, topical, tracheal (including by intratracheal instillation), transdermal, vaginal, and vitreal.

As used herein, the term "BAF complex" refers to the BRG1- or HBRM-associated factors complex in a human cell.

As used herein, the term "BAF complex-related disorder" refers to a disorder that is caused or affected by the level of activity of a BAF complex.

As used herein, the term "BRG1 loss of function mutation" refers to a mutation in BRG1 that leads to the protein having diminished activity (e.g., at least 1% reduction in BRG1 activity, for example 2%, 5%, 10%, 25%, 50%, or 100% reduction in BRG1 activity). Exemplary BRG1 loss of function mutations include, but are not limited to, a homozygous BRG1 mutation and a deletion at the C-terminus of BRG1.

As used herein, the term "BRG1 loss of function disorder" refers to a disorder (e.g., cancer) that exhibits a reduction in BRG1 activity (e.g., at least 1% reduction in BRG1 activity, for example 2%, 5%, 10%, 25%, 50%, or 100% reduction in BRG1 activity).

The term "cancer" refers to a condition caused by the proliferation of malignant neoplastic cells, such as tumors, neoplasms, carcinomas, sarcomas, leukemias, and lymphomas.

As used herein, a "combination therapy" or "administered in combination" means that two (or more) different agents or treatments are administered to a subject as part of a defined treatment regimen for a particular disease or condition. The treatment regimen defines the doses and periodicity of administration of each agent such that the effects of the separate agents on the subject overlap. In some embodiments, the delivery of the two or more agents is simultaneous or concurrent and the agents may be co-formulated. In some embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally.

By "determining the level" of a protein or RNA is meant the detection of a protein or an RNA, by methods known in the art, either directly or indirectly. "Directly determining" means performing a process (e.g., performing an assay or test on a sample or "analyzing a sample" as that term is defined herein) to obtain the physical entity or value. "Indirectly determining" refers to receiving the physical entity or value from another party or source (e.g., a third party laboratory that directly acquired the physical entity or value). Methods to measure protein level generally include, but are not limited to, western blotting, immunoblotting, enzyme-linked immunosorbent assay (ELISA), radioimmunoassay (RIA), immunoprecipitation, immunofluorescence, surface plasmon resonance, chemiluminescence, fluorescent polarization, phosphorescence, immunohistochemical analysis, matrix-assisted laser desorption/ionization time-of-flight (MALDI-TOF) mass spectrometry, liquid chromatography (LC)-mass spectrometry, microcytometry, microscopy, fluorescence activated cell sorting (FACS), and flow cytometry, as well as assays based on a property of a protein including, but not limited to, enzymatic activity or interaction with other protein partners. Methods to measure RNA levels are known in the art and include, but are not limited to, quantitative polymerase chain reaction (qPCR) and Northern blot analyses.

By a "decreased level" or an "increased level" of a protein or RNA is meant a decrease or increase, respectively, in a protein or RNA level, as compared to a reference (e.g., a decrease or an increase by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 300%, about 400%, about 500%, or more; a decrease or an increase of more than about 10%, about 15%, about 20%, about 50%, about 75%, about 100%, or about 200%, as compared to a reference; a decrease or an increase by less than about 0.01-fold, about 0.02-fold, about 0.1-fold, about 0.3-fold, about 0.5-fold, about 0.8-fold, or less; or an increase by more than about 1.2-fold, about 1.4-fold, about 1.5-fold, about 1.8-fold, about 2.0-fold, about 3.0-fold, about 3.5-fold, about 4.5-fold, about 5.0-fold, about 10-fold, about 15-fold, about 20-fold, about 30-fold, about 40-fold, about 50-fold, about 100-fold, about 1000-fold, or more). A level of a protein may be expressed in mass/vol (e.g., g/dL, mg/mL, µg/mL, ng/mL) or percentage relative to total protein in a sample.

By "decreasing the activity of a BAF complex" is meant decreasing the level of an activity related to a BAF complex, or a related downstream effect. An example of decreasing an activity of a BAF complex is Sox2 activation. The activity level of a BAF complex may be measured using any method known in the art, e.g., the methods described in Kadoch et al. Cell, 2013, 153, 71-85.

As used herein, the term "inhibiting BRM" refers to blocking or reducing the level or activity of the ATPase catalytic binding domain or the bromodomain of the protein. BRM inhibition may be determined using methods known in the art, e.g., a BRM ATPase assay, a Nano DSF assay, or a BRM Luciferase cell assay.

As used herein, the term "LXS196," also known as IDE196, refers to the PKC inhibitor having the structure: 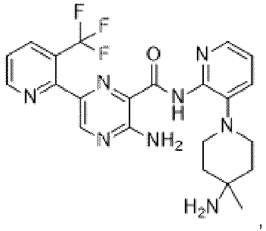 or a pharmaceutically acceptable salt thereof.

The term "pharmaceutical composition," as used herein, represents a composition containing a compound described herein formulated with a pharmaceutically acceptable excipient and appropriate for administration to a mammal, for example a human. Typically, a pharmaceutical composition is manufactured or sold with the approval of a governmental regulatory agency as part of a therapeutic regimen for the treatment of disease in a mammal. Pharmaceutical compositions can be formulated, for example, for oral administration in unit dosage form (e.g., a tablet, capsule, caplet, gelcap, or syrup); for topical administration (e.g., as a cream, gel, lotion, or ointment); for intravenous administration (e.g., as a sterile solution free of particulate emboli and in a solvent system suitable for intravenous use); or in any other pharmaceutically acceptable formulation.

A "pharmaceutically acceptable excipient," as used herein, refers to any ingredient other than the compounds described herein (for example, a vehicle capable of suspending or dissolving the active compound) and having the properties of being substantially nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration.

As used herein, the term "pharmaceutically acceptable salt" means any pharmaceutically acceptable salt of a compound, for example, any compound of **Formula I.** Pharmaceutically acceptable salts of any of the compounds described herein may include those that are within the scope of sound medical judgment, suitable for use in contact with the tissues of humans and animals without undue toxicity, irritation, allergic response and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting a free base group with a suitable organic acid.

The compounds of the invention may have ionizable groups so as to be capable of preparation as pharmaceutically acceptable salts. These salts may be acid addition salts involving inorganic or organic acids or the salts may, in the case of acidic forms of the compounds of the invention be prepared from inorganic or organic bases. Frequently, the compounds are prepared or used as pharmaceutically acceptable salts prepared as addition products of pharmaceutically acceptable acids or bases. Suitable pharmaceutically acceptable acids and bases and methods for preparation of the appropriate salts are well-known in the art. Salts may be prepared from pharmaceutically acceptable non-toxic acids and bases including inorganic and organic acids and bases.

By a "reference" is meant any useful reference used to compare protein or RNA levels. The reference can be any sample, standard, standard curve, or level that is used for comparison purposes. The reference can be a normal reference sample or a reference standard or level. A "reference sample" can be, for example, a control, e.g., a predetermined negative control value such as a "normal control" or a prior sample taken from the same subject; a sample from a normal healthy subject, such as a normal cell or normal tissue; a sample (e.g., a cell or tissue) from a subject not having a disease; a sample from a subject that is diagnosed with a disease, but not yet treated with a compound of the invention; a sample from a subject that has been treated by a compound of the invention; or a sample of a purified protein or RNA (e.g., any described herein) at a known normal concentration. By "reference standard or level" is meant a value or number derived from a reference sample. A "normal control value" is a pre-determined value indicative of non-disease state, e.g., a value expected in a healthy control subject. Typically, a normal control value is expressed as a range ("between X and Y"), a high threshold ("no higher than X"), or a low threshold ("no lower than X"). A subject having a measured value within the normal control value for a particular biomarker is typically referred to as "within normal limits" for that biomarker. A normal reference standard or level can be a value or number derived from a normal subject not having a disease or disorder (e.g., cancer); a subject that has been treated with a compound of the invention. In preferred embodiments, the reference sample, standard, or level is matched to the sample subject sample by at least one of the following criteria: age, weight, sex, disease stage, and overall health. A standard curve of levels of a purified protein or RNA, e.g., any described herein, within the normal reference range can also be used as a reference.

As used herein, the term "subject" refers to any organism to which a composition in accordance with the invention may be administered, e.g., for experimental, diagnostic, prophylactic, and/or therapeutic purposes. Typical subjects include any animal (e.g., mammals such as mice, rats, rabbits, non-human primates, and humans). A subject may seek or be in need of treatment, require treatment, be receiving treatment, be receiving treatment in the future, or be a human or animal who is under care by a trained professional for a particular disease or condition.

As used herein, the terms "treat," "treated," or "treating" mean therapeutic treatment or any measures whose object is to slow down (lessen) an undesired physiological condition, disorder, or disease, or obtain beneficial or desired clinical results. Beneficial or desired clinical results include, but are not limited to, alleviation of symptoms; diminishment of the extent of a condition, disorder, or disease; stabilized (i.e., not worsening) state of condition, disorder, or disease; delay in onset or slowing of condition, disorder, or disease progression; amelioration of the condition, disorder, or disease state or remission (whether partial or total); an amelioration of at least one measurable physical parameter, not necessarily discernible by the patient; or enhancement or improvement of condition, disorder, or disease. Treatment includes eliciting a clinically significant response without excessive levels of side effects. Treatment also includes prolonging survival as compared to expected survival if not receiving treatment. Compounds of the invention may also be used to "prophylactically treat" or "prevent" a disorder, for example, in a subject at increased risk of developing the disorder.

As used herein, the terms "variant" and "derivative" are used interchangeably and refer to naturally-occurring, synthetic, and semi-synthetic analogues of a compound, peptide, protein, or other substance described herein. A variant or derivative of a compound, peptide, protein, or other substance described herein may retain or improve upon the biological activity of the original material.

The details of one or more embodiments of the invention are set forth in the description below. Other features, objects, and advantages of the invention will be apparent from the description and from the claims.

### Brief Description of the Drawings

**FIG. 1** is a graph illustrating inhibition of cell proliferation of several cancer cell lines by a BRG1/BRM inhibitor (Compound A).
**FIG. 2A** is a graph illustrating inhibition of cell proliferation of uveal melanoma cell line 92-1 by a BRG1/BRM inhibitor (Compound A), a MEK inhibitor (Selumetinib), and a PKC inhibitor (LXS196).
**FIG. 2B** is a graph illustrating inhibition of cell proliferation of uveal melanoma cell line MP41 by a BRG1/BRM inhibitor (Compound A), a MEK inhibitor (Selumetinib), and a PKC inhibitor (LXS196).
**FIG. 3** is a graph illustrating inhibition of cell proliferation of several cancer cell lines by a BRG1/BRM inhibitor (Compound B).
**FIG. 4** is a graph illustrating the area under the curves (AUCs) calculated from dose-response curves for cancer cell lines treated with a BRG1/BRM inhibitor.
**FIG. 5** is a graph illustrating inhibition of cell proliferation of uveal melanoma and non-small cell lung cancer cell lines by a BRG1/BRM inhibitor (Compound B).
**FIG. 6A** is a graph illustrating inhibition of cell proliferation of uveal melanoma cell line 92-1 by a BRG1/BRM inhibitor (Compound B), a MEK inhibitor (Selumetinib), and a PKC inhibitor (LXS196).
**FIG. 6B** is a graph illustrating inhibition of cell proliferation of uveal melanoma cell line MP41 by a BRG1/BRM inhibitor (Compound B), a MEK inhibitor (Selumetinib), and a PKC inhibitor (LXS196).
**FIG. 7A** is a graph illustrating inhibition of cell proliferation of parental and PKC-inhibitor refractory uveal melanoma cell lines by a PKC inhibitor (LXS196).
**FIG. 7B** is a graph illustrating inhibition of cell proliferation of parental and PKC-inhibitor refractory uveal melanoma cell lines by a BRG1/BRM inhibitor (Compound B).
**FIG. 8A** is a graph illustrating inhibition of tumor growth in mice engrafted with uveal melanoma cell lines by a BRG1/BRM inhibitor (Compound C).
**FIG. 8B** is an illustration of the size of tumors from mice engrafted with uveal melanoma cell lines and dosed with a BRG1/BRM inhibitor (Compound C).
**FIG. 8C** is a graph illustrating body weight change of mice engrafted with uveal melanoma cell lines and dosed with a BRG1/BRM inhibitor (Compound C).

### Detailed Description

The present disclosure features compounds useful for the inhibition of BRG1 and/or BRM. These compounds may be used to modulate the activity of a BAF complex, for example, for the treatment of a BAF-related disorder, such as cancer. Exemplary compounds described herein include compounds having a structure according to **Formula I:** 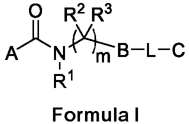 wherein
A is 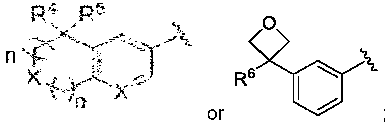
m is 0, 1, 2, or 3;
n is 1 or 2;
o is 0 or 1;
X is O, CH₂, or NR⁷;
X' is N, CH, or CR^{X}, wherein R^{X} is a halogen;
B is an optionally substituted 6-membered bicyclic heteroarylene or an optionally substituted 9- or 10-membered bicyclic heteroarylene;
L is a covalent bond, optionally substituted C₁-C₃ alkylene, C₂ alkynylene, optionally substituted C₂ alkenylene, optionally substituted C₂-C₃ heteroalkylene, optionally substituted C₃-C₅ cycloalkylene, or optionally substituted 4- to 10-membered heterocyclylene;
C is optionally substituted 3- to 10-membered cycloalkyl; optionally substituted 6- to 10-membered aryl; optionally substituted 5- to 10-membered heteroaryl; or optionally substituted 5- to 10-membered heterocyclyl;
R¹ and R⁷ are, independently, hydrogen or optionally substituted C₁-C₆ alkyl;
each R² and R³ is independently, hydrogen, optionally substituted C₁-C₆ alkyl; or optionally substituted C₁-C₆ heteroalkyl;
R⁴ is cyano, fluoro, hydroxy, or -CH₂OH;
R⁵ is C₁-C₃ alkyl optionally substituted with one, two, three, four, five, six, or seven fluoro groups; and
R⁶ is C₁-C₃ alkyl optionally substituted with one, two, three, four, five, six, or seven fluoro groups,
or a pharmaceutically acceptable salt thereof.

Preferably, when B is optionally substituted 6-membered bicyclic heteroarylene, L is a covalent bond, optionally substituted C₁-C₃ alkylene, C₂ alkynylene, optionally substituted C₂ alkenylene, optionally substituted C₂-C₃ heteroalkylene, optionally substituted C₃-C₅ cycloalkylene, or optionally substituted 4- to 10-membered heterocyclylene. Also preferably, when B is an optionally substituted 9- or 10-membered bicyclic heteroarylene, L is a covalent bond, e.g., the compound of Formula I is a compound of Formula Ia: 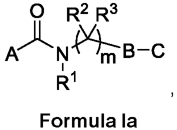 or a pharmaceutically acceptable salt thereof, where all variables are as described herein.

In some embodiments, the compound, or pharmaceutically acceptable salt thereof, has the structure of any one of compounds 1-48 in Table 1, compounds 49-84 in Table 2, or compounds 85-126 in Table 3.

Other embodiments, as well as exemplary methods for the synthesis of production of these compounds, are described herein.

### Pharmaceutical Uses

The compounds described herein are useful and, while not bound by theory, are believed to exert their ability to modulate the level, status, and/or activity of a BAF complex, i.e., by inhibiting the activity of the BRG1 and/or BRM proteins within the BAF complex in a mammal. BAF complex-related disorders include, but are not limited to, BRG1 loss of function mutation-related disorders.

An aspect of the present invention relates to treating disorders related to BRG1 loss of function mutations such as cancer (e.g., non-small cell lung cancer, colorectal cancer, bladder cancer, cancer of unknown primary, glioma, breast cancer, melanoma, non-melanoma skin cancer, endometrial cancer, or penile cancer). In some embodiments, the compound is administered in an amount and for a time effective to result in one or more (e.g., two or more, three or more, four or more) of: (a) reduced tumor size, (b) reduced rate of tumor growth, (c) increased tumor cell death (d) reduced tumor progression, (e) reduced number of metastases, (f) reduced rate of metastasis, (g) decreased tumor recurrence (h) increased survival of subject, (i) increased progression free survival of subject.

Treating cancer can result in a reduction in size or volume of a tumor. For example, after treatment, tumor size is reduced by 5% or greater (e.g., 10%, 20%, 30%, 40%, 50%, 60%, 70%, 80%, 90%, or greater) relative to its size prior to treatment. Size of a tumor may be measured by any reproducible means of measurement. For example, the size of a tumor may be measured as a diameter of the tumor.

Treating cancer may further result in a decrease in number of tumors. For example, after treatment, tumor number is reduced by 5% or greater (e.g., 10%, 20%, 30%, 40%, 50%, 60%, 70%, 80%, 90%, or greater) relative to number prior to treatment. Number of tumors may be measured by any reproducible means of measurement, e.g., the number of tumors may be measured by counting tumors visible to the naked eye or at a specified magnification (e.g., 2x, 3x, 4x, 5x, 10x, or 50x).

Treating cancer can result in a decrease in number of metastatic nodules in other tissues or organs distant from the primary tumor site. For example, after treatment, the number of metastatic nodules is reduced by 5% or greater (e.g., 10%, 20%, 30%, 40%, 50%, 60%, 70%, 80%, 90% or greater) relative to number prior to treatment. The number of metastatic nodules may be measured by any reproducible means of measurement. For example, the number of metastatic nodules may be measured by counting metastatic nodules visible to the naked eye or at a specified magnification (e.g., 2x, 10x, or 50x).

Treating cancer can result in an increase in average survival time of a population of subjects treated according to the present invention in comparison to a population of untreated subjects. For example, the average survival time is increased by more than 30 days (more than 60 days, 90 days, or 120 days). An increase in average survival time of a population may be measured by any reproducible means. An increase in average survival time of a population may be measured, for example, by calculating for a population the average length of survival following initiation of treatment with the compound of the invention. An increase in average survival time of a population may also be measured, for example, by calculating for a population the average length of survival following completion of a first round of treatment with a pharmaceutically acceptable salt of the invention.

Treating cancer can also result in a decrease in the mortality rate of a population of treated subjects in comparison to an untreated population. For example, the mortality rate is decreased by more than 2% (e.g., more than 5%, 10%, or 25%). A decrease in the mortality rate of a population of treated subjects may be measured by any reproducible means, for example, by calculating for a population the average number of disease-related deaths per unit time following initiation of treatment with a pharmaceutically acceptable salt of the invention. A decrease in the mortality rate of a population may also be measured, for example, by calculating for a population the average number of disease-related deaths per unit time following completion of a first round of treatment with a pharmaceutically acceptable salt of the invention.

Exemplary cancers that may be treated by the invention include, but are not limited to, non-small cell lung cancer, small-cell lung cancer, colorectal cancer, bladder cancer, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer and penile cancer.

### Combination Formulations and Uses Thereof

The compounds of the invention can be combined with one or more therapeutic agents. In particular, the therapeutic agent can be one that treats or prophylactically treats any cancer described herein.

### Combination Therapies

A compound of the invention can be used alone or in combination with an additional therapeutic agent, e.g., other agents that treat cancer or symptoms associated therewith, or in combination with other types of treatment to treat cancer. In combination treatments, the dosages of one or more of the therapeutic compounds may be reduced from standard dosages when administered alone. For example, doses may be determined empirically from drug combinations and permutations or may be deduced by isobolographic analysis (e.g., Black et al., Neurology 65:S3-S6, 2005). In this case, dosages of the compounds when combined should provide a therapeutic effect.

In some embodiments, the second therapeutic agent is a chemotherapeutic agent (e.g., a cytotoxic agent or other chemical compound useful in the treatment of cancer). These include alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Also included is 5-fluorouracil (5-FU), leucovorin (LV), irenotecan, oxaliplatin, capecitabine, paclitaxel and doxetaxel. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall (see, e.g., Agnew, Chem. Intl. Ed Engl. 33:183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, Adriamycin^{®} (doxorubicin, including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5- FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., Taxol^{®} paclitaxel (Bristol-Myers Squibb Oncology, Princeton, N.J.), ABraxane^{®}, cremophor-free, albumin-engineered nanoparticle formulation of paclitaxel (American Pharmaceutical Partners, Schaumberg, Ill.), and Taxotere^{®} doxetaxel (Rhone-Poulenc Rorer, Antony, France); chloranbucil; Gemzar^{®} gemcitabine; 6-thioguanine; mercaptopurine; methotrexate; platinum coordination complexes such as cisplatin, oxaliplatin and carboplatin; vinblastine; platinum; etoposide (VP-16); ifosfamide; mitoxantrone; vincristine; Navelbine^{®} vinorelbine; novantrone; teniposide; edatrexate; daunomycin; aminopterin; xeloda; ibandronate; irinotecan (e.g., CPT-11); topoisomerase inhibitor RFS 2000; difluoromethylornithine (DMFO); retinoids such as retinoic acid; capecitabine; and pharmaceutically acceptable salts, acids or derivatives of any of the above. Two or more chemotherapeutic agents can be used in a cocktail to be administered in combination with the first therapeutic agent described herein. Suitable dosing regimens of combination chemotherapies are known in the art and described in, for example, Saltz et al. (1999) Proc ASCO 18:233a and Douillard et al. (2000) Lancet 355:1041-7.

In some embodiments, the second therapeutic agent is a therapeutic agent which is a biologic such a cytokine (e.g., interferon or an interleukin (e.g., IL-2)) used in cancer treatment. In some embodiments the biologic is an anti-angiogenic agent, such as an anti-VEGF agent, e.g., bevacizumab (Avastin^{®}). In some embodiments the biologic is an immunoglobulin-based biologic, e.g., a monoclonal antibody (e.g., a humanized antibody, a fully human antibody, an Fc fusion protein or a functional fragment thereof) that agonizes a target to stimulate an anti-cancer response or antagonizes an antigen important for cancer. Such agents include Rituxan (Rituximab); Zenapax (Daclizumab); Simulect (Basiliximab); Synagis (Palivizumab); Remicade (Infliximab); Herceptin (Trastuzumab); Mylotarg (Gemtuzumab ozogamicin); Campath (Alemtuzumab); Zevalin (Ibritumomab tiuxetan); Humira (Adalimumab); Xolair (Omalizumab); Bexxar (Tositumomab-I-131); Raptiva (Efalizumab); Erbitux (Cetuximab); Avastin (Bevacizumab); Tysabri (Natalizumab); Actemra (Tocilizumab); Vectibix (Panitumumab); Lucentis (Ranibizumab); Soliris (Eculizumab); Cimzia (Certolizumab pegol); Simponi (Golimumab); Ilaris (Canakinumab); Stelara (Ustekinumab); Arzerra (Ofatumumab); Prolia (Denosumab); Numax (Motavizumab); ABThrax (Raxibacumab); Benlysta (Belimumab); Yervoy (Ipilimumab); Adcetris (Brentuximab Vedotin); Perjeta (Pertuzumab); Kadcyla (Ado-trastuzumab emtansine); and Gazyva (Obinutuzumab). Also included are antibody-drug conjugates.

The second agent may be a therapeutic agent which is a non-drug treatment. For example, the second therapeutic agent is radiation therapy, cryotherapy, hyperthermia and/or surgical excision of tumor tissue.

The second agent may be a checkpoint inhibitor. In one embodiment, the inhibitor of checkpoint is an inhibitory antibody (e.g., a monospecific antibody such as a monoclonal antibody). The antibody may be, e.g., humanized or fully human. In some embodiments, the inhibitor of checkpoint is a fusion protein, e.g., an Fc-receptor fusion protein. In some embodiments, the inhibitor of checkpoint is an agent, such as an antibody, that interacts with a checkpoint protein. In some embodiments, the inhibitor of checkpoint is an agent, such as an antibody, that interacts with the ligand of a checkpoint protein. In some embodiments, the inhibitor of checkpoint is an inhibitor (e.g., an inhibitory antibody or small molecule inhibitor) of CTLA-4 (e.g., an anti-CTLA4 antibody such as ipilimumab/Yervoy or tremelimumab). In some embodiments, the inhibitor of checkpoint is an inhibitor (e.g., an inhibitory antibody or small molecule inhibitor) of PD-1 (e.g., nivolumab/Opdivo^{®}; pembrolizumab/Keytruda^{®}; pidilizumab/CT-011). In some embodiments, the inhibitor of checkpoint is an inhibitor (e.g., an inhibitory antibody or small molecule inhibitor) of PDL1 (e.g., MPDL3280A/RG7446; MEDI4736; MSB0010718C; BMS 936559). In some embodiments, the inhibitor of checkpoint is an inhibitor (e.g., an inhibitory antibody or Fc fusion or small molecule inhibitor) of PDL2 (e.g., a PDL2/lg fusion protein such as AMP 224). In some embodiments, the inhibitor of checkpoint is an inhibitor (e.g., an inhibitory antibody or small molecule inhibitor) of B7-H3 (e.g., MGA271), B7-H4, BTLA, HVEM, TIM3, GAL9, LAG3, VISTA, KIR, 2B4, CD160, CGEN-15049, CHK1, CHK2, A2aR, B-7 family ligands, or a combination thereof.

In any of the combination embodiments described herein, the first and second therapeutic agents are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours up to 24 hours or up to 1-7, 1-14, 1-21 or 1-30 days before or after the second therapeutic agent.

### Pharmaceutical Compositions

The compounds of the invention are preferably formulated into pharmaceutical compositions for administration to a mammal, preferably, a human, in a biologically compatible form suitable for administration in vivo. Accordingly, in an aspect, the present invention provides a pharmaceutical composition comprising a compound of the invention in admixture with a suitable diluent, carrier, or excipient.

The compounds of the invention may be used in the form of the free base, in the form of salts, solvates, and as prodrugs. All forms are within the scope of the invention. In accordance with the invention, the described compounds or salts, solvates, or prodrugs thereof may be administered to a patient in a variety of forms depending on the selected route of administration, as will be understood by those skilled in the art. The compounds of the invention may be administered, for example, by oral, parenteral, buccal, sublingual, nasal, rectal, patch, pump, or transdermal administration and the pharmaceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

A compound of the invention may be orally administered, for example, with an inert diluent or with an assimilable edible carrier, or it may be enclosed in hard- or soft-shell gelatin capsules, or it may be compressed into tablets, or it may be incorporated directly with the food of the diet. For oral therapeutic administration, a compound of the invention may be incorporated with an excipient and used in the form of ingestible tablets, buccal tablets, troches, capsules, elixirs, suspensions, syrups, and wafers. A compound of the invention may also be administered parenterally. Solutions of a compound of the invention can be prepared in water suitably mixed with a surfactant. Under ordinary conditions of storage and use, these preparations may contain a preservative to prevent the growth of microorganisms. Conventional procedures and ingredients for the selection and preparation of suitable formulations are described, for example, in Remington's Pharmaceutical Sciences (2003, 20th ed.) and in The United States Pharmacopeia: The National Formulary (USP 24 NF19), published in 1999. The pharmaceutical forms suitable for injectable use include sterile aqueous solutions or dispersions and sterile powders for the extemporaneous preparation of sterile injectable solutions or dispersions. In all cases the form must be sterile and must be fluid to the extent that may be easily administered via syringe. Compositions for nasal administration may conveniently be formulated as aerosols, drops, gels, and powders. Aerosol formulations typically include a solution or fine suspension of the active substance in a physiologically acceptable aqueous or non-aqueous solvent and are usually presented in single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal after use. Where the dosage form comprises an aerosol dispenser, it will contain a propellant, which can be a compressed gas, such as compressed air or an organic propellant. The aerosol dosage forms can also take the form of a pump-atomizer. Compositions suitable for buccal or sublingual administration include tablets, lozenges, and pastilles, where the active ingredient is formulated with a carrier. Compositions for rectal administration are conveniently in the form of suppositories containing a conventional suppository base. A compound described herein may be administered intratumorally, for example, as an intratumoral injection. Intratumoral injection is injection directly into the tumor vasculature and is specifically contemplated for discrete, solid, accessible tumors. Local, regional, or systemic administration also may be appropriate. A compound described herein may advantageously be contacted by administering an injection or multiple injections to the tumor, spaced for example, at approximately, 1 cm intervals. In the case of surgical intervention, the present invention may be used preoperatively, such as to render an inoperable tumor subject to resection. Continuous administration also may be applied where appropriate, for example, by implanting a catheter into a tumor or into tumor vasculature.

The compounds of the invention may be administered to an animal, e.g., a human, alone or in combination with pharmaceutically acceptable carriers, as noted herein, the proportion of which is determined by the solubility and chemical nature of the compound, chosen route of administration, and standard pharmaceutical practice.

### Dosages

The dosage of the compounds of the invention, and/or compositions comprising a compound of the invention, can vary depending on many factors, such as the pharmacodynamic properties of the compound; the mode of administration; the age, health, and weight of the recipient; the nature and extent of the symptoms; the frequency of the treatment, and the type of concurrent treatment, if any; and the clearance rate of the compound in the animal to be treated. One of skill in the art can determine the appropriate dosage based on the above factors. The compounds of the invention may be administered initially in a suitable dosage that may be adjusted as required, depending on the clinical response. In general, satisfactory results may be obtained when the compounds of the invention are administered to a human at a daily dosage of, for example, between 0.05 mg and 3000 mg. Dose ranges include, for example, between 10-1000 mg.

Alternatively, the dosage amount can be calculated using the body weight of the patient. For example, the dose of a compound, or pharmaceutical composition thereof, administered to a patient may range from 0.1-100 mg/kg.

### Examples

**Definitions used in the following Schemes and elsewhere herein are:**
- MeCN or ACN: acetonitrile
- AIBN: azobisisobutyronitrile
- Boc: tert-butoxycarbonyl
- n-BuLi: n-butyllithium
- t-BuOK: potassium tert-butoxide
- t-BuONa: sodium tert-butoxide
- t-BuXPhos Pd G3: [(2-Di-tert-butylphosphino-2',4',6'-triisopropyl-1,1'-biphenyl)-2-(2'-amino-1,1'-biphenyl)] palladium(II) methanesulfonate
- tBuphos Pd G3: [(2-Di-tert-butylphosphino-2',4',6'-triisopropyl-1,1'-biphenyl)-2-(2'-amino-1,1'-biphenyl)] palladium(II) methanesulfonate
- DAST: diethylaminosulfur trifluoride
- DCE: dichloroethane
- DCM: dichloromethane
- DCPP-2HBF₄: 1,3-bis(dicyclohexylphosphino)propane bis(tetrafluoroborate)
- DEA: N,N-diethylamine
- DMP: Dess-Martin periodinane, 1,1,1-Tris(acetyloxy)-1,1-dihydro-1,2-
- DIAD: diisopropyl azodicarboxylate
- DIBAL-H: diisobutylaluminum hydride
- DIEA or DIPEA: N,N-diisopropylethylamine
- DMA: dimethylacetamide
- DMAP: 4-(dimethylamino)pyridine
- DME: 1,2-dimethoxyethane
- DMF: N,N-dimethylformamide
- DMSO: dimethylsulfoxide
- dppf: bis(diphenylphosphino)ferrocene
- dtbpf: 1,1'-bis(di-tert-butylphosphino)ferrocene
- EDCI: 1-ethyl-(3-dimethylaminopropyl)carbodiimide hydrochloride
- ESI: electrospray ionization
- Et₃N or TEA: triethylamine
- EA: ethyl acetate
- EtOH: ethyl alcohol
- FA: formic acid
- FCC: flash column chromatography
- g: grams
- HATU: 2-(3H-[1,2,3]triazolo[4,5-b]pyridin-3-yl)-I, 1,3 ,3-tetramethylisouronium
- HCl: hydrochloric acid
- HOAc: acetic acid
- HOBt: hydroxybenzotriazole
- HPLC: high performance liquid chromatography
- IPA: isopropyl alcohol
- L: liter
- LCMS: liquid chromatography / mass spectrometry
- LiHMDS: lithium bis(trimethylsilyl)amide
- m-CPBA: 3-chloroperoxybenzoic acid
- MeCN: acetonitrile
- Mel: methyl iodide
- MeOH: methyl alcohol
- mL: milliliter
- mmol: millimole
- mg: milligrams
- MHz: megahertz
- MS: mass spectrometry
- MTBE: methyl tert-butyl ether
- m/z: mass/charge ratio
- NBS: N-bromosuccinimide
- NIS: N-iodosuccinimide
- nm: nanometer
- NMR: nuclear magnetic resonance
- PdCl2(dtbpf): [1,1'-Bis(di-tert-butylphosphino)ferrocene]dichloropalladium(II)
- PE: petroleum ether
- PhMe: toluene
- ppm: parts per million
- rt: room temperature
- RT: retention time
- SFC: supercritical fluid chromatography
- SPhos Pd G3: (2-dicyclohexylphosphino-2',6'-dimethoxybiphenyl) [2-(2'-amino-1,1'-biphenyl)]palladium(II) methanesulfonate
- TBS: tert-butyldimethylsilyl
- TBSCl: tert-butyldimethylsilyl chloride
- TBDMS: tert-butyldimethylsilyl chloride
- TCCA or TCICA: trichloroisocyanuric acid
- TFA: trifluoroacetic acid
- TFAA: trifluoroacetic anhydride
- THF: tetrahydrofuran
- TMSCN: trimethylsilyl cyanide
- TosMIC: toluenesulfonylmethyl isocyanide
- Ziram: zinc dimethyldithiocarbamate

### Materials

Unless otherwise noted, all materials were obtained from commercial suppliers and were used without further purification. All reactions involving air- or moisture-sensitive reagents were performed under a nitrogen atmosphere.

### Example 1. Preparation of Intermediates

### Intermediates 1 and 2: (4S)-4-Cyano-4-methyl-isochromane-6-carboxylic acid and (4R)-4-Cyano-4-methyl-isochromane-6-carboxylic acid

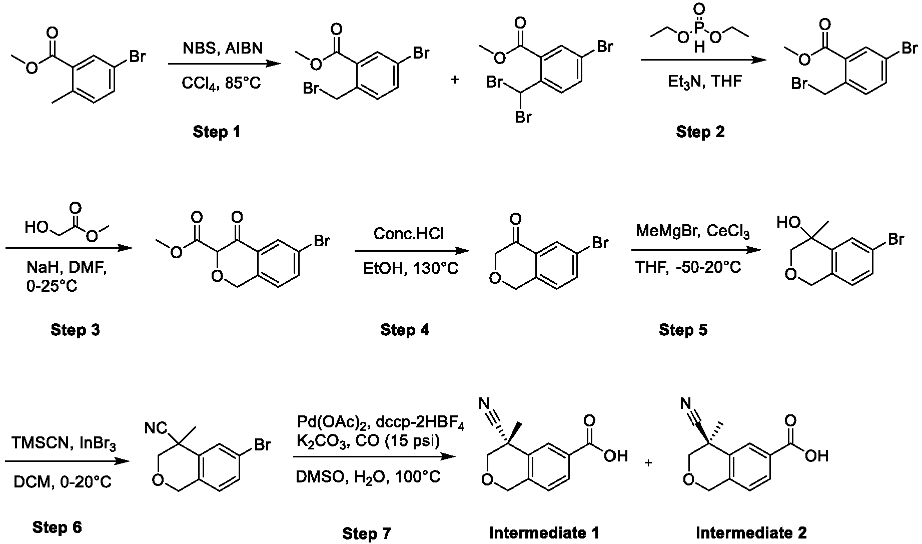

### Step 1. Methyl 5-bromo-2-(bromomethyl)benzoate and methyl 5-bromo-2-(dibromomethyl)benzoate

N-Bromosuccinimide (130.5 g, 733 mmol) and AIBN (11.47 g, 69.8 mmol) was added to a mixture of methyl 5-bromo-2-methyl-benzoate (160 g, 698 mmol) in CCl₄ (1.6 L). The mixture was stirred at 80°C for 8 hrs. The mixture was cooled to 25°C and filtered. The filtrate was concentrated in vacuo affording a mixture of the title compounds (215 g, crude) as a yellow oil, which was used to next step directly.

### Step 2. Methyl 5-bromo-2-(bromomethyl)benzoate

Diisopropylethyl amine (48.6 mL, 279 mmol) and 1-ethoxyphosphonoyloxyethane (36.0 mL, 279 mmol) were added to a mixture of methyl 5-bromo-2-(bromomethyl)benzoate and methyl 5-bromo-2-(dibromomethyl)benzoate (215 g, crude) in THF (1 L) at 0 °C. The mixture was stirred at 25°C for 8 hrs. The mixture was diluted with water (1 L) and extracted with EA (1 L x 3). The combined organic phase was washed with brine (1 L), dried over Na₂SO₄, filtered, and concentrated in vacuo. The crude product was dissolved with PE:EA = 10:1 (1 L). The mixture was filtered through a short silica gel column and washed with PE:EA = 10:1 (2 L). The filtrate was concentrated under vacuum affording the title compound (210 g, 682 mmol) as yellow oil.
¹H NMR (400MHz, DMSO-d6) δ = 7.99 (d, J = 2.0 Hz, 1H), 7.82-7.79 (m, 1H), 7.56 (d, J = 8.4 Hz, 1H), 4.97 (s, 2H), 3.88 (s, 3H) ppm.

### Step 3. Methyl 6-bromo-4-oxo-isochromane-3-carboxylate

Sodium hydride (39 g, 974 mmol, 60% purity) was added to a mixture of methyl 5-bromo-2-(bromomethyl)benzoate (150 g, 487 mmol) and methyl 2-hydroxyacetate (75 mL, 974 mmol) in DMF (1.5 L) at 0 °C. The mixture was stirred at 25°C for 1hr. The mixture was poured into saturated NH₄Cl (aq., 6 L), and then extracted with MTBE (3 L x 2). The combined organic layers were dried over Na₂SO₄, filtered, and concentrated under reduced pressure affording the title compound (140 g, crude) as a brown oil, which was used to next step directly.

### Step 4. 6-Bromoisochroman-4-one

A mixture of methyl 6-bromo-4-oxo-isochromane-3-carboxylate (140 g, 487 mmol) in EtOH (500 mL) and HCl (12 M, I L) was stirred at 120°C for 1hr. The reaction mixture was cooled to 25 °C and filtered. The filter cake was saved. The filtrate was diluted with water (2 L) and extracted with MTBE (1.5 L x 2). The combined organic layers were dried over Na₂SO₄, filtered, and concentrated under reduced pressure to give brown oil. The brown oil was combined with the filter cake and then triturated with EtOH (200 mL) at 0°C for 0.5 hrs. The solid was collected by filtration, washed with EtOH (50 mL x 2) and dried in vacuo affording the title compound (50 g, 242 mmol) as a yellow solid.
¹H NMR (400MHz, CDCl₃) δ = 8.17 (d, J = 20 Hz, 1H), 7.69-7.67 (m, 1H), 7.13 (d, J = 8.4 Hz, 1H), 4.85 (s, 2H), 4.37 (s, 2H) ppm.

### Step 5. 6-Bromo-4-methyl-isochroman-4-ol

Methylmagnesium bromide (3 M in THF, 440 mL) was added dropwise to a mixture of 6-bromoisochroman-4-one (100 g, 440 mmol) and CeCl₃ (54.3 g, 220 mmol, dried in a muffle furnace at 300°C for 3 hrs.) in THF (2 L) at -50 °C. The mixture was warmed to 20°C and stirred for 1 hr. The reaction mixture was poured into water (2 L) and then filtered through diatomite. The diatomite was then washed with EA (2L). The filtrate was separated and the aqueous layer was extracted with EA (1 L x 2). The combined organic layers were washed with brine (2 L), dried over Na₂SO₄, filtered, and concentrated under reduced pressure to give a residue. The crude residue was purified by FCC (Eluent: PE:EA = 50:1 to 3:1) affording the title compound (105 g, 432 mmol) as light yellow oil.
¹H NMR (400MHz, DMSO-d6) δ = 7.67 (d, J = 2.0 Hz, 1H), 7.38-7.35 (m, 1H), 6.99 (d, J = 8.4 Hz, 1H), 5.35 (s, 1H), 4.71 - 4.58 (m, 2H), 3.68 - 3.59 (m, 1H), 3.58 - 3.50 (m, 1H), 1.38 (s, 3H) ppm.

### Step 6. 6-Bromo-4-methyl-isochromane-4-carbonitrile

TMSCN (102.9 mL, 823 mmol) was added to a solution of 6-bromo-4-methyl-isochroman-4-ol (100 g, 411 mmol) in DCM (2 L) at 0 °C. InBr₃ (29.2 g, 82.3 mmol) was then added, and the mixture was warmed and stirred at 25 °C for 1 hr. The mixture was poured into water (2 L) and extracted with DCM (1 L x 2). The combined organic layers were washed with brine (1 L), dried over Na₂SO₄, filtered, and concentrated under reduced pressure. The crude residue was purified by FCC (Eluent: PE:EA = 20:1 to 5:1) affording the title compound (55 g, 218 mmol) as a white solid.
¹H NMR (400MHz, DMSO-d6) δ = 7.82 (d, J = 2.0 Hz, 1H), 7.55-7.52 (m, 1H), 7.13 (d, J = 8.3 Hz, 1H), 4.83 - 4.68(m, 2H), 4.17 (d, J = 11.5 Hz, 1H), 3.79 (d, J = 11.5 Hz, 1H), 1.65 (s, 3H) ppm.

### Step 7. (4S)-4-Cyano-4-methyl-isochromane-6-carboxylic acid and (4R)-4-Cyano-4-methyl-isochromane-6-carboxylic acid

Palladium acetate (490 mg, 2.18 mmol) and K₂CO₃ (9.05 g, 65.4 mmol) were added to a mixture 6-bromo-4-methyl-isochromane-4-carbonitrile (11 g, 43.6 mmol) and DCPP-2HBF₄ (2.67 g, 4.36 mmol) in DMSO (110 mL) and H₂O (5.5 mL). The mixture was degassed and purged with CO (g) three times, and then the mixture was stirred at 100°C for 4 hrs under an atmosphere of CO (15 psi). The aqueous layer was then extracted with EA (1 L x 2). The aqueous phase was acidified with HCl (2N) until a pH = 3 was achieved. The aqueous layer was then extracted with EA(1 L x 3). The combined organic phase was washed with brine (1 L), dried over Na₂SO₄, filtered, and concentrated under vacuum. The crude product was triturated with EA (100 mL) at 20°C for 15 min, then the solid was collected by filtration, washed with EA (50 mL), and dried affording the title compounds (30 g, 138 mmol) as a white solid. The mixture of stereoisomers was purified by SFC separation (column: DAICEL CHIRALPAK AD-H (250mm x 30mm, 5µm); mobile phase: [0.1%NH₃₋H₂O MEOH]; B%: 20%-20%, 3.52min; 514minmin). Peak 1 was concentrated under reduced pressure to give an oil. The oil was dissolved in water (200 mL). The mixture was acidified with HCl (2N) to pH = 3 and extracted with EA (100 mL x 3). The combined organic phase washed with brine (100 mL), dried over Na₂SO₄, filtered, and concentrated under vacuum affording Intermediate 1 (4.15 g, 19.1 mmol) as an off-white solid. Peak 2 was concentrated under reduced pressure to give an oil. The oil was dissolved in water (200 mL). The mixture was acidified with HCl (2N) to pH = 3 and extracted with EA (100 mL x 3). The combined organic phase washed with brine (100 mL), dried over Na₂SO₄, filtered, and concentrated under vacuum affording Intermediate 2 (4.1 g, 18.9 mmol) as an off- white solid.
**Intermediate 1:** Chiral SFC: AD-3_5CM_MEOH(DEA)_5_40_3ML_AT35.M; RT = 0.89 min
LCMS (ESI) m/z: [M+H]⁺ = 218.3.
¹HNMR (400MHz, DMSO-d6) δ = 13.17 (br s, 1H), 8.07 (d, J = 1.6 Hz, 1H), 7.89-7.87 (m, 1H), 7.29 (d, J = 8.0 Hz, 1H), 4.95 - 4.79 (m, 2H), 4.19 (d, J = 11.6 Hz, 1H), 3.88 (d, J = 11.6 Hz, 1H), 1.67 (s, 3H) ppm. **Intermediate 2:** Chiral SFC: AD-3_5CM_MEOH(DEA)_5_40_3ML_AT35.M; RT = 1.03 min
LCMS (ESI) m/z: [M+H]⁺ = 218.3.
¹H NMR (400MHz, DMSO-d6) δ = 13.17 (br s, 1H), 8.07 (d, J = 1.6 Hz, 1H), 7.89-7.87 (m, 1H), 7.29 (d, J = 8.0 Hz, 1H), 4.95 - 4.79 (m, 2H), 4.19 (d, J = 11.6 Hz, 1H), 3.88 (d, J = 11.6 Hz, 1H), 1.67 (s, 3H) ppm.

### Intermediates 3 and 4: (4R)-4-Hydroxy-4-methylisochromane-6-carboxylic acid and (4S)-4-Hydroxy-4-methylisochromane-6-carboxylic acid

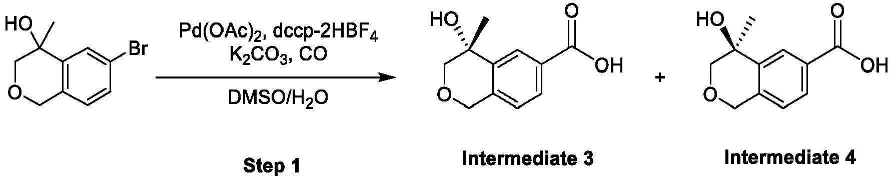

### (4R)-4-Hydroxy-4-methylisochromane-6-carboxylic acid and (4S)-4-Hydroxy-4-methylisochromane-6-carboxylic acid

A mixture of 6-bromo-4-methyl-isochroman-4-ol (1.00 g, 4.11 mmol), dccp2BF₄ (252 mg, 0.41 mmol), K₂CO₃ (853 mg, 6.17 mmol), Pd(OAc)₂ (92.4 mg, 0.41 mmol) and H₂O (1.48 mL, 82.3 mmol) in DMSO (10 mL) was degassed and purged with CO (g) three times. The mixture was stirred at 100°C for 14 hrs under a CO atmosphere. The reaction mixture was filtered to remove the black solid and then diluted with water (60 mL) and extracted with EA (60 mL x 2). The oganic layer was discarded and the aqueous phase was adjusted to pH = 6 with aqueous HCl. The aqueous solution was then extracted with EA (100 mL x 3). The combined organic layer was washed with brine (200 mL x 2), dried over anhydrous Na₂SO₄, filtered, and concentrated under reduced pressure affording a mixture of the title compounds (550 mg, 2.43 mmol) as a light yellow solid. The stereoisomers were separated by chiral SFC (column: DAICEL CHIRALPAK AD (250mm*30mm, 10µm); mobile phase: [0.1%NH₃•H₂O IPA]; B%: 40%-40%,4.0 min; 60 minmin). The desired fractions were collected and concentrated under reduced pressure. The product was diluted with water, adjusted to pH = 4 with aq. HCl, then extracted with EA (80 mL x 3). The combined organic layers were dried over anhydrous Na₂SO₄, filtered, and concentrated under reduced pressure affording Intermediate 3 (260 mg, 1.18 mmol) as a yellow solid and Intermediate 4 (240 mg, 1.04 mmol) as a yellow solid.
**Intermediate 3:** Chiral SFC: AD-3_5CM_IPA (DEA)_5_40_3ML_AT35.M; RT = 1.537min.
LCMS (ESI) m/z: [M-18+H]+ = 191.1.
¹H NMR (400 MHz, DMSO-d6) δ = 13.19 - 12.40 (m, 1H), 8.15 (d, J = 1.6 Hz, 1H), 7.75 - 7.73 (m, 1H), 7.13 (d, J = 8.0 Hz, 1H), 5.34 (s, 1H), 4.82 - 4.68 (m, 2H), 3.71 - 3.65 (m, 1H), 3.61 - 3.54 (m, 1H), 1.40 (s, 3H) ppm.
**Intermediate 4:** Chiral SFC: AD-3_5CM_IPA (DEA)_5_40_3ML_AT35.M; RT = 1.926 min.
LCMS (ESI) m/z: [M-17+H]+ = 191.1.
¹H NMR (400 MHz, DMSO-d6) δ = 12.86 (br s, 1H), 8.15 (d, J = 1.6 Hz, 1H), 7.75 - 7.73 (m, 1H), 7.13 (d, J = 8.0 Hz, 1H), 5.34 (s, 1H), 4.84 - 4.67 (m, 2H), 3.73 - 3.62 (m, 1H), 3.62 - 3.53 (m, 1H), 1.40 (s, 3H) ppm.

### Intermediates 5 and 6: (S)-4-Cyano-4-methylchroman-6-carboxylic acid and (R)-4-Cyano-4-methylchroman-6-carboxylic acid

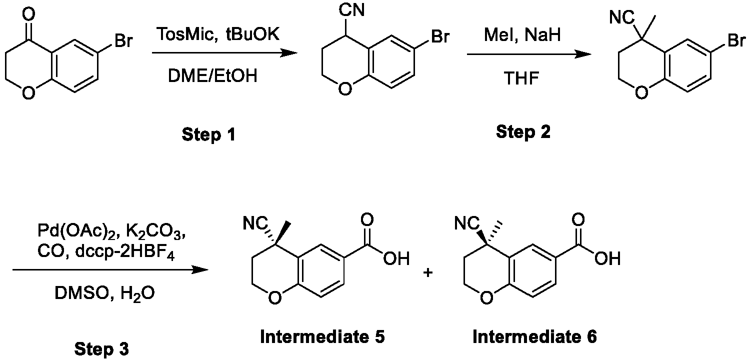

### Step 1. 6-Bromochromane-4-carbonitrile

Potassium tert-butoxide (54.4 g, 484 mmol) was added to a solution of 6-bromochroman-4-one (50 g, 220 mmol) and 1-(isocyanomethylsulfonyl)-4-methyl-benzene (64.5 g, 330 mmol) in DME (2.5 L) and EtOH (100 mL) at 0 °C. The reaction mixture was warmed and stirred at 25°C for 16 hrs. The reaction mixture was poured into water (2 L) and adjusted to pH=6-7 with NH₄Cl (56.9 g). The organic solvents were removed in vacuo. The aqueous phase was extracted with EA (1.5 L x 2). The combined organic layers were dried over anhydrous Na₂SO₄ and concentrated in vacuum. The residue was purified by FCC (Eluent: PE:EA = 20:1). The purified residue was combined with another batch that was run under the same conditions and triturated with MTBE (150 mL). The solid was filtered and concentrated in vacuo affording the title compound (40 g, 151 mmol) as a white solid.
¹H NMR (400 MHz, CDCl₃) δ = 7.43 - 7.42 (m, 1H), 7.33 - 7.31 (m, 1H), 6.76 (d, J = 8.8 Hz, 1H), 4.36 - 4.30 (m, 1H), 4.26 - 4.21 (m, 1H), 4.01 - 3.98 (m, 1H), 2.35 - 2.31 (m, 2H) ppm.

### Step 2. 6-Bromo-4-methyl-chromane-4-carbonitrile

Sodium hydride (8.40 g, 210 mmol, 60% purity) was added to a solution of 6-bromochromane-4-carbonitrile (25 g, 105 mmol) in THF (250 mL) at 0 °C. The reaction mixture was stirred at 0 °C for 0.5 hr, then Mel (32.7 mL, 525 mmol) was added. The reaction mixture was stirred at 25°C for 2 hrs. The reaction mixture was diluted with saturated NH₄Cl (aq., 800 mL) and extracted with EA (800 mL x 3). The combined organic layer was dried over anhydrous Na₂SO₄, filtered, and concentrated in vacuo. The crude residue was purified by FCC (Eluent: PE/EA = 1:0 to 5:1) affording the title compound (18 g, 69.2 mmol) as a light yellow solid.
¹H NMR (400 MHz, DMSO-d6) δ = 7.69 (d, J = 2.4 Hz, 1H), 7.41 - 7.38 (m, 1H), 6.83 (d, J = 8.8 Hz, 1H), 4.33 - 4.25 (m, 1H), 4.24 - 4.15 (m, 1H), 2.41 - 2.35 (m, 1H), 2.17 - 2.11 (m, 1H), 1.75 (s, 3H) ppm.

### Step 3. (S)-4-Cyano-4-methylchroman-6-carboxylic acid and (R)-4-Cyano-4-methylchroman-6-carboxylic acid

A mixture of 6-bromo-4-methyl-chromane-4-carbonitrile (8.5 g, 33.7 mmol), dccp-2HBF₄ (2.06 g, 3.37 mmol), K₂CO₃ (6.99 g, 50.6 mmol), Pd(OAc)₂ (757 mg, 3.37 mmol), and H₂O (1.22 mL, 67.4 mmol) in DMSO (85 mL) was degassed and purged with CO (g) three times. The mixture was then stirred at 100°C for 14 hrs under a CO (15 psi) atmosphere. The reaction mixture combined with a 2^{nd} batch and filtered to remove the black solid. The filtrate was diluted with water (60 mL) and extracted with EA (60 mL x 2). The organic layer was discarded and the pH of aqueous phase was adjusted to pH=4 with aq. HCl resulting in a precipitate. The mixture was filtered and the white solid was washed with water (40 mL x 2), filtered, and dried in vacuo affording a mixture of the title compounds (12 g, 51.2 mmol) as a white solid. Intermediates 5 and 6 were separated by chiral SFC (column: DAICEL CHIRALPAK AD-H(250mm*30mm,5µm);mobile phase:[0.1%NH₃-H₂O MEOH];B%: 30%-30%,4.7 min: 600 min.). The fraction was concentrated under reduced pressure to get Intermediate 5 (5.3 g, 24.3 mmol) as an off-white solid and Intermediate 6 (5.2 g, 23.6 mmol) as an off-white solid.
**Intermediate 5:** Chiral SFC: AD-3_5CM_MEOH(DEA)_5_40_3ML_AT35.M, RT = 0.958min
LCMS (ESI) m/z: [M+H]+= 218.2.
¹H NMR (400 MHz, DMSO-d6) δ= 800 (d, J = 2.0 Hz, 1H), 7.79 - 7.76 (m, 1H), 6.88 (d, J = 8.4 Hz, 1H), 4.37 - 4.22 (m, 2H), 2.45 - 2.39 (m, 1H), 2.22 - 1.75 (m, 1H), 1.76 (s, 3H) ppm.
**Intermediate 6:** Chiral SFC: AD-3_5CM_MEOH (DEA)_5_40_3ML_AT35.M, RT = 1.294
LCMS (ESI) m/z: [M+H]+= 218.2.
¹H NMR (400 MHz, DMSO-d6) δ= 8.00 (d, J = 2.0 Hz, 1H), 7.79 - 7.76 (m, 1H), 6.89 (d, J = 8.4 Hz, 1H), 4.37 - 4.23 (m, 2H), 2.50 - 2.41 (m, 1H), 2.22 - 2.18 (m, 1H), 1.76 (s, 3H) ppm.

### Intermediates 7 and 8: (4R)-4-Fluoro-4-methyl-isochromane-6-carboxylic acid and (4S)-4-Fluoro-4-methyl-isochromane-6-carboxylic acid

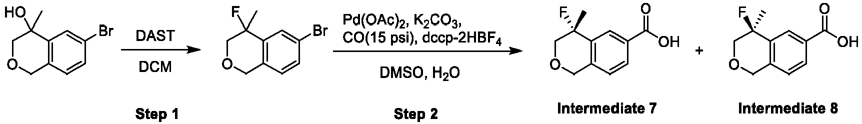

### Step 1. 6-Bromo-4-fluoro-4-methyl-isochromane

DAST (52.2 mL, 395 mmol,) was added dropwise to a mixture of Intermediate 8 (32 g, 132 mmol) in DCM (320 mL) at 0 °C. The mixture was warmed and stirred at 25 °C for 20 min. The mixture was poured into ice water (600 mL) and extracted with DCM (250 mL x 3). The combined organic layers were dried over anhydrous Na₂SO₄, filtered, and concentrated in vacuo. The residue was purified by FCC (Eluent: PE:EA = 30:1) affording the title compound (24.5 g, 99.96 mmol) as a yellow oil.
LCMS (ESI) m/z: [M-18+H]+ = 226.9.
¹H NMR (400 MHz, CDCl₃) δ = 7.72 (s, 1H), 7.45 - 7.42 (m, 1H), 6.93 (d, J = 8.0 Hz, 1H), 4.84 - 4.74 (m, 1H), 4.73 - 4.64 (m, 1H), 4.06 - 4.00 (m, 1H), 3.86 - 3.78 (m, 1H), 1.76 - 1.64 (m, 3H) ppm.

### Step 2. (4R)-4-Fluoro-4-methyl-isochromane-6-carboxylic acid and (4S)-4-Fluoro-4-methyl-isochromane-6-carboxylic acid

Palladium acetate (687 mg, 3.06 mmol), dccp-2HBF₄ (1.87 g, 3.06 mmol), K₂CO₃ (6.34 g, 45.9 mmol), and H₂O (7.50 mL) were added to a solution of 6-bromo-4-fluoro-4-methyl-isochromane (7.5 g, 30.6 mmol) in DMSO (75 mL) at 25 °C. The mixture was purged with CO (g) three times. The mixture was heated and stirred at 100°C for 4 hrs under an atmosphere of CO (15 psi). The reaction mixture was poured into H₂O (1.2 L) and extracted with EA (400 mL x 2). The aqueous phase was acidified with 1N HCl to pH=5 and extracted with EA (300 mL x 3). The combined organic layers were washed with brine (300 mL x 2), dried over anhydrous Na₂SO₄, filtered, and concentrated in vacuo affording a mixture of the title compounds (2.6 g, 12.2 mmol) as a white solid.
¹H NMR (400 MHz, DMSO-d6) δ = 13.42 - 12.67 (m, 1H), 8.13 (s, 1H), 7.92 - 7.88 (m, 1H), 7.29 (d, J = 8.0 Hz, 1H), 4.91 - 4.82 (m, 1H), 4.77 - 4.68 (m, 1H), 4.10 - 4.01 (m, 1H), 3.89 - 3.76 (m, 1H), 1.70 - 1.58 (m, 3H) ppm.

Intermediates 7 and 8 were separated by SFC (Column: DAICEL CHIRALPAK IG (250mm*30mm, 10µm); mobile phase: [0.1%NH₃₋H₂O MeOH];B%: 20%-20%, 3.4min; 2300minmin) to give two peaks. Peak 1 was concentrated under reduced pressure to remove 95% MeOH and poured into H₂O (500 mL). The mixture was acidified with HCl (1N) to pH=5 and extracted with EA (150 mL x 3). The combined organic layers were washed with brine (100 mL), dried over anhydrous Na₂SO₄, filtered, and concentrated under reduced pressure to afford Intermediate 7 (1.06 g, 5.04 mmol) as a white solid. Peak 2 was concentrated under reduced pressure and poured into H₂O (500 mL). The mixture was acidified with HCl (1N) to pH=5 and extracted with EA (150 mL x 3). The combined organic layers were washed with brine (100 mL), dried over anhydrous Na₂SO₄, filtered, and concentrated under reduced pressure to afford Intermediate 8 (1.07 g, 5.09 mmol) as a white solid.
**Intermediate 7:** Chiral SFC: AD-3_5CM_MEOH (DEA)_5_40_3ML_AT35.M, RT = 0.819 min.
¹H NMR (400 MHz, DMSO-d6) δ= 13.08 (br s, 1H), 8.13 (s, 1H), 7.92 - 7.88 (m, 1H), 7.29 (d, J = 8.0 Hz, 1H), 4.90 - 4.81 (m, 1H), 4.78 - 4.68 (m, 1H), 4.05 - 4.01 (m, 1H), 3.87 - 3.78 (m, 1H), 1.72 - 1.58 (m, 3H) ppm.
**Intermediate 8:** Chiral SFC: AD-3_5CM_MEOH (DEA)_5_40_3ML_AT35.M, RT = 0.903 min.
¹H NMR (400 MHz, DMSO-d6) δ= 13.31 - 12.88 (m, 1H), 8.13 (s, 1H), 7.92 - 7.88 (m, 1H), 7.29 (d, J = 8.0 Hz, 1H), 4.90 - 4.81 (m, 1H), 4.78 - 4.68 (m, 1H), 4.08 - 4.01 (m, 1H), 3.89 - 3.77 (m, 1H), 1.72 - 1.58 (m, 3H) ppm.

### Intermediates 9 and 10: (R)-1-(tert-Butoxycarbonyl)-4-cyano-4-methyl-1,2,3,4-tetrahydroquinoline-6-carboxylic acid and (S)-1-(tert-Butoxycarbonyl)-4-cyano-4-methyl-1,2,3,4-tetrahydroquinoline-6-carboxylic acid

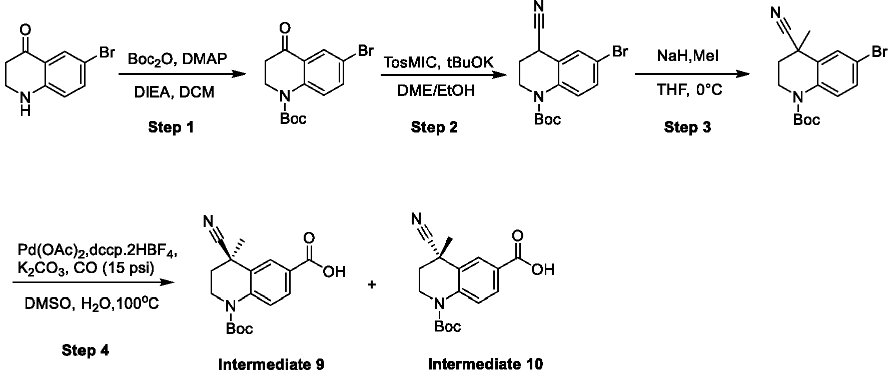

### Step 1. tert-Butyl 6-bromo-4-oxo-3,4-dihydroquinoline-1(2H)-carboxylate

A mixture of 6-bromo-2,3-dihydro-1H-quinolin-4-one (2 g, 8.85 mmol), Boc₂O (3.86 g, 17.7 mmol), DMAP (108 mg, 0.88 mmol), and DIEA (3.1 mL, 17.7 mmol) in DCM (20 mL) was stirred at 60°C for 12 hrs. The mixture was concentrated under reduced pressure and then poured into water (5 mL). The aqueous solution was then extracted with EA (5mL x 3). The combined organic layers were dried over anhydrous Na₂SO₄, filtered, and concentrated under reduced pressure. The residue was purified by FCC (Eluent: 0 to 12% EA in PE) affording the title compound (1.75 g, 5.37 mmol) as a yellow oil.
LCMS (ESI) m/z: [M+H]⁺ = 326.0.
¹H NMR (400 MHz, DMSO-d6) δ = 7.89 - 7.88 (m, 1H), 7.75 (d, J = 1.2 Hz, 2H), 4.11 - 4.07 (m, 2H), 2.79 - 2.75 (m, 2H), 1.51 (s, 9H) ppm.

### Step 2. tert-Butyl 6-bromo-4-cyano-3,4-dihydroquinoline-1(2H)-carboxylate

Potassium tert-butoxide (1.38 g, 12.26 mmol) was added to a mixture of tert-Butyl 6-bromo-4-oxo-3,4-dihydroquinoline-1(2H)-carboxylate (2 g, 6.13 mmol) and 1-(isocyanomethylsulfonyl)-4-methylbenzene (1.80 g, 9.20 mmol) in DME (100 mL) and EtOH (4 mL) at 0 °C. The mixture was stirred at 25 °C for 12 hrs. The reaction mixture was poured into water (5 mL) and extracted with EA (5 mL x 3). The combined organic layers were dried over anhydrous Na₂SO₄, filtered, and concentrated under reduced pressure. The residue was purified by FCC (Eluent: 0 to 20% EA in PE) affording the title compound (900 mg, 2.67 mmol) as a yellow oil.
LCMS (ESI) m/z: [M-55]+ = 281.0.
¹H NMR (400 MHz, DMSO-d6) δ = 7.67 (d, J = 8.8 Hz, 1H), 7.55 (d, J = 2.4 Hz, 1H), 7.51 - 7.47 (m, 1H), 4.48 - 4.44 (m, 1H), 3.74 - 3.69 (m, 2H), 2.21 - 2.15 (m, 2H), 1.48 (s, 9H) ppm.

### Step 3. tert-Butyl 6-bromo-4-cyano-4-methyl-3,4-dihydroquinoline-1(2H)-carboxylate

Sodium hydride (89 mg, 2.22 mmol, 60% purity) was added slowly to a mixture of tert-butyl 6-bromo-4-cyano-3,4-dihydroquinoline-1(2H)-carboxylate (500 mg, 1.48 mmol) in THF (5 mL) at 0 °C. The mixture was stirred at 0 °C for 0.5 hr, then Mel (0.14 mL, 2.22 mmol) was added. The reaction mixture was warmed to 25°C and stirred for 2 hrs. The reaction mixture was quenched with saturated NH₄Cl (20 mL) and extracted with EA (10 mL x 3). The combined organic layers were dried over anhydrous Na₂SO₄, filtered, and concentrated under reduced pressure. The resulting oil was purified by FCC (Eluent: 0 to 50% EA in PE) affording the title compound (370 mg, 1.01 mmol) as yellow oil.
LCMS (ESI) m/z: [M-55]+=295.0
¹H NMR (400 MHz, CDCl₃) δ = 7.66 (d, J = 8.8 Hz, 1H), 7.56 (d, J = 2.0 Hz, 1H), 7.38 - 7.36 (m, 1H), 3.89 - 3.80 (m, 2H), 2.40 - 2.34 (m, 1H), 2.04 - 2.00 (m, 1H), 1.73 (s, 3H), 1.53 (s, 9H) ppm.

### Step 4. (R)-1-(tert-Butoxycarbonyl)-4-cyano-4-methyl-1,2,3,4-tetrahydroquinoline-6-carboxylic acid & (S)-1-(tert-Butoxycarbonyl)-4-cyano-4-methyl-1,2,3,4-tetrahydroquinoline-6-carboxylic acid

Palladium acetate (11.8 mg, 0.053 mmol) and dccp2HBF₄ (64.5 mg, 0.11 mmol) were added to a mixture of tert-butyl 6-bromo-4-cyano-4-methyl-3,4-dihydroquinoline-1(2H)-carboxylate (370 mg, 1.05 mmol) and K₂CO₃ (218 mg, 1.58 mmol) in DMSO (3 mL) and H₂O (1 mL) at 25 °C. The mixture was degassed and purged with CO (g, 15 psi) three times, and then the mixture was warmed stirred at 100 °C under CO (g) for 12 hrs. The reaction mixture was filtered and the filtrate was purified by reversed-phase HPLC affording a mixture of the title compounds (190 mg, 0.60 mmol) as a white solid. The stereoisomers were separated via chiral SFC (DAICEL CHIRALPAK AD(250mm*30mm,10µm); mobile phase: [0.1%NH₃₋H₂O MEOH]; B%: 20%- 20%,1.75min; 75minmin) to afford Intermediate 9 (80 mg, 0.25 mmol) as light yellow solid and Intermediate 10 (90 mg, 0.28 mmol) as light yellow solid.
**Intermediate 9:** LCMS (ESI) m/a: [M-55]+=261.1
¹H NMR (400 MHz, DMSO-d6) δ = 7.96 (d, J = 1.6 Hz, 1H), 7.80 - 7.78 (m, 1H), 7.69 (d, J = 8.8 Hz, 1H), 3.79 - 3.74 (m, 2H), 2.36 - 2.32 (m, 1H), 2.07 - 2.05 (m, 1H), 1.71 (s, 3H), 1.47 (s, 9H) ppm.
Chiral SFC: AD-3_5CM_MEOH(DEA)_5_40_3ML_AT35.M, Rt =0.766 min, ee value =98.82 %.
**Intermediate 10:** LCMS (ESI) m/a: [M-55]+=261.1
¹H NMR (400 MHz, DMSO-d6) δ = 7.96 (br s, 1H), 7.81 - 7.78 (m, 1H), 7.71 (br d, J = 8.8 Hz, 1H), 3.80 - 3.74 (m, 2H), 2.36 - 2.31 (m, 1H), 2.07 - 2.04 (m, 1H), 1.71 (s, 3H), 1.48 (s, 9H) ppm.
Chiral SFC: AD-3_5CM_MEOH(DEA)_5_40_3ML_AT35.M. Rt =0.916 min, ee value =93.82 %.

### Intermediate 11: tert-Butyl N-[1-(4-bromo-2-pyridyl)-2-methoxy-ethyl]carbamate

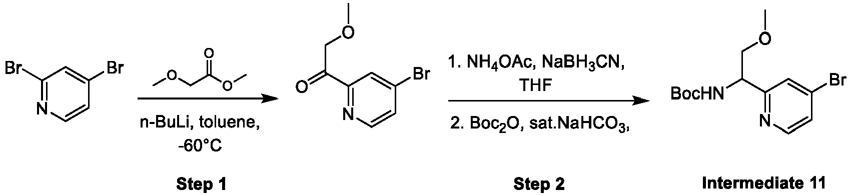

### Step 1. 1-(4-Bromo-2-pyridyl)-2-methoxy-ethanone

n-Butyl lithium (2.5 M, 16.9 mL) was added to a solution of 2,4-dibromopyridine (10.0 g, 42.2 mmol) in toluene (400 mL) at -60 °C. The mixture was stirred at -60°C for 1 hr, and then methyl 2-methoxyacetate (7.53 mL, 76.0 mmol) was added to the mixture and the mixture was stirred at -60 °C for 1 hr. The reaction mixture was quenched by addition water (1 L), and then extracted with EA (1 L x 2). The combined organic layers were washed with brine (1 L), dried over anhydrous Na₂SO₄, filtered, and concentrated under reduced pressure. The crude product was purified by FCC (Eluent: PE:EA = 3:1) affording the title compound (5 g, 21.7 mmol) as a brown solid.
¹H NMR (400 MHz, CDCl₃) δ= 8.45 (d, J = 5.2 Hz, 1H), 8.23 (d, J = 2.0 Hz, 1H), 7.68 - 7.66 (m, 1H), 5.01 (s, 2H), 3.54 (s, 3H) ppm.

### Step 2. tert-Butyl N-[1-(4-bromo-2-pyridyl)-2-methoxy-ethyl]carbamate

Ammonium acetate (13.4 g, 174 mmol) and NaBH₃CN (10.93 g, 174 mmol) were added to a solution of 1-(4-bromo-2-pyridyl)-2-methoxy-ethanone (4 g, 17.4 mmol) in THF (120 mL) at 20 °C. The mixture was stirred at 20 °C for 2 hr. Boc₂O (22.8 g, 104 mmol) and saturated NaHCO₃ (60 mL) were added to the reaction mixture and the mixture was stirred at 20 °C for 2 hrs. The reaction mixture was diluted with water (300 mL) and extracted with EA (300 mL x 2). The combined organic layers were washed with brine (300 mL), dried over anhydrous Na₂SO₄, filtered, and concentrated under reduced pressure. The crude product was purified by FCC (Eluent: PE:EA = 3:1) and concentrated under reduced pressure to give a white solid. The white solid was then re-purified by RPC. The fractions containing the title compound were collected and concentrated to remove the MeCN. The aqueous layer was basified with sodium carbonate (solid) until pH = 9.0 was achieved. The aqueous solution was extracted with EA (100 mL x 2). The combined organic layers were dried with anhydrous Na₂SO₄ and concentrated in vacuo affording the title compound (1.5 g, 4.19 mmol) as brown oil.
LCMS (ESI) m/z: [Br81M+H]+ = 333.1.
¹H NMR (400MHz, DMSO-d6) δ = 8.41 (d, J = 5.2 Hz, 1H), 7.62 (d, J = 1.2 Hz, 1H), 7.58 - 7.56 (m, 1H), 7.37 (br d, J = 8.8 Hz, 1H), 4.81 - 4.76 (m, 1H), 3.56 - 3.54 (m, 2H), 3.23 (s, 3H), 1.40 - 1.37 (m, 9H) ppm.

### Intermediates 12 and 13: (S)-4-(Difluoromethyl)-4-fluoroisochroman-6-carboxylic acid and (R)-4-(Difluoromethyl)-4-fluoroisochroman-6-carboxylic acid

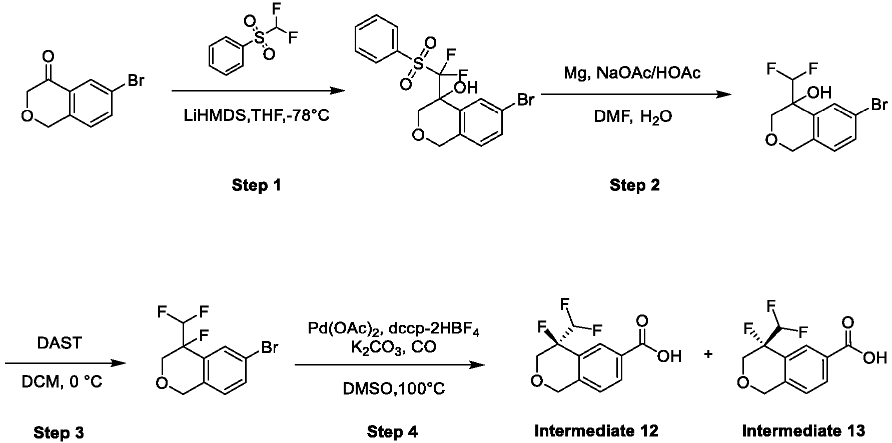

### Step 1. 6-Bromo-4-(difluoro(phenylsulfonyl)methyl)isochroman-4-ol

LiHMDS (1 M, 26.4 mL) was added to a solution of 6-bromoisochroman-4-one (3 g, 13.2 mmol), difluoromethylsulfonylbenzene (2.8 mL, 19.8 mmol) in THF (30 mL) at -78 °C. The mixture was stirred at -78 °C for 2 hr. The reaction mixture was poured into saturated aq. NH₄Cl (50 mL) and extracted with EA (50 mL x 2). The organic layer was washed with brine (50 mL) and dried over anhydrous Na₂SO₄, filtered and concentrated in vacuo. The residue was purified by FCC (Eluent: PE:EA = 10:1 to 3:1) affording the title compound (3 g, 6.70 mmol) as yellow solid .
LCMS (ESI) m/z: [M+H2O]+ = 435.8, 437.8.
¹H NMR (400MHz, CDCl₃) δ = 8.04 (d, J = 7.6 Hz, 2H), 7.90 - 7.89 (m, 1H), 7.85 - 7.76 (m, 1H), 7.71 - 7.62 (m,2H), 7.49 - 7.47 (m, 1H), 6.97 (d, J = 8.4 Hz, 1H), 5.00 (d, J = 12.4 Hz, 1H), 4.93 - 4.73 (m, 2H), 3.93 - 3.88 (m, 1H), 3.83 (s,1H).

### Step 2. 6-Bromo-4-(difluoromethyl)isochroman-4-ol

Magnesium (2.61 g, 107 mmol) was added to a solution of 6-bromo-4-(difluoro(phenylsulfonyl)methyl)isochroman-4-ol (3 g, 7.16 mmol) and NaOAc/HOAc (aq., 8 M, 44.7 mL) in DMF (100 mL). The mixture was stirred at 25 °C for 2 hrs. The reaction mixture was poured into water (50 mL), and then extracted with EA (50 mL x 3). The combined organic layer was washed with brine (100 mL), dried over anhydrous Na₂SO₄, filtered, and concentrated in vacuo. The residue was purified by FCC (Eluent: PE:EA = 10:1 to 3:1) affording the title compound (1.5 g, 5.37 mmol) as colorless oil. LCMS (ESI) m/z: [79BrM-H₂O]+ = 260.9.
¹H NMR (400MHz, CDCl₃) δ = 7.83 - 7.60 (m, 1H), 7.51 - 7.33 (m, 1H), 7.11 - 6.93 (m, 1H), 6.25 - 5.88 (m, 1H), 4.92 - 4.70 (m, 2H), 4.26 - 4.17 (m, 1H), 3.87 - 3.66 (m, 1H), 2.75 - 2.58 (m, 1H).

### Step 3. 6-Bromo-4-(difluoromethyl)-4-fluoroisochroman

DAST (1.33 mL, 10.0 mmol) was added to a solution of 6-bromo-4-(difluoromethyl)isochroman-4-ol (1.4 g, 5.02 mmol) in DCM (20 mL) at 0 °C. The mixture was stirred at 0 °C for 2 hr. The reaction mixture was poured into water (50 mL) and extracted with DCM (20 mL x 2). The organic layer was washed with brine (20 mL) and dried over anhydrous Na₂SO₄, filtered, and concentrated in vacuo. The residue was purified by FCC (Eluent: PE:EA = 10:1 to 5:1) affording the title compound (900 mg, 2.88 mmol) as colorless oil.
¹H NMR (400MHz, CDCl₃) δ = 7.73 - 7.51 (m, 1H), 7.48 - 7.28 (m, 1H), 7.08 - 6.89 (m, 1H), 6.20 - 5.80 (m, 1H), 4.82 - 4.61 (m, 2H), 4.33 - 4.18 (m, 1H), 4.01 - 3.82 (m, 1H).

### Step 4. (S)-4-(Difluoromethyl)-4-fluoroisochroman-6-carboxylic acid and (R)-4-(Difluoromethyl)-4-fluoroisochroman-6-carboxylic acid

Palladium acetate (34.0 mg, 0.15 mmol), K₂CO₃ (627 mg, 4.54 mmol) and dccp-2HBF₄ (185 mg, 0.30 mmol) were added to a solution of 6-bromo-4-(difluoromethyl)-4-fluoroisochroman (850 mg, 3.02 mmol), in H₂O (0.11 mL) and DMSO (10 mL). The mixture was stirred at 100 °C for 16 hrs under CO (g, 15 psi). The reaction mixture was poured into water (20 mL) and extracted with EA (20 mL x 2). The organic layer was washed with brine (20 mL), dried over anhydrous Na₂SO₄, and concentrated in vacuo. The crude product was purified by reverse phase column chromatography affording a mixture of the title compounds (450 mg, 1.64 mmol) as colorless oil. The mixture of stereoisomers was purified by SFC separation (column: DAICEL CHIRALPAK IG (250mm*30mm,10µm);mobile phase: [0.1%NH₃₋H₂O MEOH];B%: 20%- 20%,3min;36minmin) to give intermediate 12 (220 mg, 0.89 mmol) as a colorless oil and intermediate 13 (220 mg, 0.89 mmol) as a colorless oil.
**Intermediate 12:** Chiral SFC: Chiralpak IG-3 50×4.6mm I.D., RT = 0.928 min.
LCMS (ESI) m/z: [M+H2O]+ = 247.2.
¹H NMR (400MHz, CDCl₃) δ = 8.36 (s, 1H), 8.14 (d, J = 8.0 Hz, 1H), 7.26 (d, J = 8.4 Hz, 1H), 6.38 - 5.96 (m, 1H), 5.01 - 4.76 (m, 2H), 4.40 - 4.35 (m, 1H), 4.18 - 4.01 (m, 1H).
**Intermediate 13:** Chiral SFC: Chiralpak IG-3 50×4.6mm I.D., RT = 1.115 min.
LCMS (ESI) m/z: [M+H2O]+ = 247.2.
¹H NMR (400MHz, CDCl₃) δ = 8.36 (s, 1H), 8.14 (d, J = 8.0 Hz, 1H), 7.26 (d, J = 8.4 Hz, 1H), 6.38 - 5.96 (m, 1H), 5.01 - 4.76 (m, 2H), 4.40 - 4.35 (m, 1H), 4.18 - 4.01 (m, 1H).

### Intermediates 14 and 15: (R)-3-Cyano-3-methyl-2,3-dihydrobenzofuran-5-carboxylic acid & (S)-3-Cyano-3-methyl-2,3-dihydrobenzofuran-5-carboxylic acid

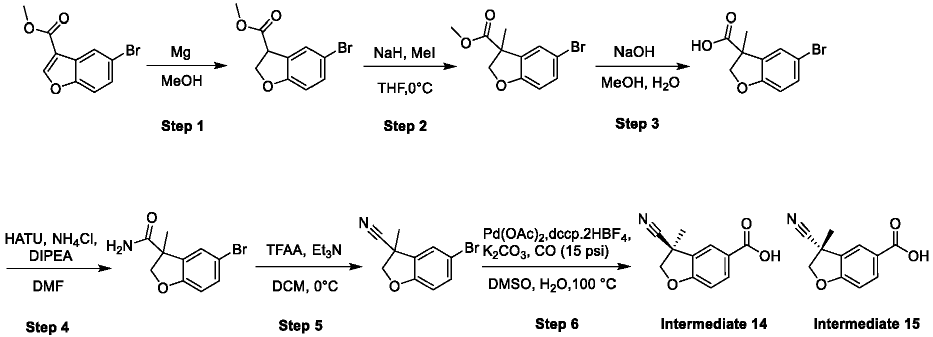

### Step 1. Methyl 5-bromo-2,3-dihydrobenzofuran-3-carboxylate

Magnesium (9.53 g, 392 mmol) was added to a solution of methyl 5-bromobenzofuran-3-carboxylate (10 g, 39.2 mmol) in MeOH (400 mL). The reaction solution was stirred at 25 °C for 6 hrs. 4N HCl (200 mL) was carefully added to the solution. After complete dissolution of the magnesium metal, the solution was concentrated under reduced pressure to remove MeOH. The resulting mixture was partitioned between EA (200 mL x 3) and water (100 mL). The organic phase was separated, dried over anhydrous MgSO₄, filtered, and concentrated in vacuo. The residue was purified by reversed phase chromatography and concentrated under reduced pressure to remove MeCN. The aqueous layer was then extracted with EA (50 mL x 3). The combined organic layers were washed with brine (50 mL x 2), dried over anhydrous Na₂SO₄, filtered, and concentrated under reduced pressure affording the title compound (6 g, 23.3 mmol) as a red oil.
¹H NMR (400 MHz, CDCl₃) δ = 7.41 - 7.40 (m, 1H), 7.25 - 7.16 (m, 1H), 6.62 (d, J = 8.8 Hz, 1H), 4.89 - 4.85 (m, 1H), 4.61 - 4.60 (m, 1H), 4.28 - 4.24 (m, 1H), 3.73 (s, 3H) ppm.

### Step 2. Methyl 5-bromo-3-methyl-2,3-dihydrobenzofuran-3-carboxylate

Sodium hydride (350 mg, 8.75 mmol, 60% purity) was slowly added to a mixture of methyl 5-bromo-2,3-dihydrobenzofuran-3-carboxylate (1.5 g, 5.83 mmol) in THF (15 mL) at 0 °C. The mixture was stirred at 0°C for 0.5 hr, then Mel (0.54 mL, 8.75 mmol) was added drop-wise at 0 °C. The reaction mixture was warmed up to 25 °C and stirred for 2 hrs. The reaction mixture was quenched with saturated aqueous NH₄Cl (30 mL) and extracted with EA (10 mL x 3). The combined organic phase was concentrated to afford yellow oil. The oil was purified by FCC (Eluent: 0 to 40% EA/PE) affording the title compound (670 mg, 2.43 mmol) as yellow oil.
LCMS (ESI) m/z: [79BrM+H]+=271.0.
¹H NMR (400 MHz, CDCl₃) δ = 7.43 (d, J = 2.0 Hz, 1H), 7.29 - 7.27 (m, 1H), 6.71 (d, J = 8.4 Hz, 1H), 5.08 (d, J = 9.2 Hz, 1H), 4.28 (d, J = 9.2 Hz, 1H), 3.76 (s, 3H), 1.61 (s, 3H) ppm.

### Step 3. 5-Bromo-3-methyl-2,3-dihydrobenzofuran-3-carboxylic acid

A mixture of methyl 5-bromo-3-methyl-2,3-dihydrobenzofuran-3-carboxylate (650 mg, 2.40 mmol) and NaOH (240 mg, 5.99 mmol) in MeOH (5 mL) and H₂O (2 mL) was stirred at 25 °C for 1 hour. The reaction mixture was diluted with water (10 mL). HCl (1 M) was added to adjust pH to 4, and then the solution was extracted with EA (10 mL x 3). The combined organic layer was concentrated affording the title compound (600 mg) as a white solid.
¹H NMR (400 MHz, DMSO-d6) δ = 7.44 (d, J = 2.0 Hz, 1H), 7.34 - 7.31 (m, 1H), 6.80 (d, J = 8.4 Hz, 1H), 4.94 (d, J = 9.2 Hz, 1H), 4.30 (d, J = 9.2 Hz, 1H), 1.53 (s, 3H) ppm.

### Step 4. 5-Bromo-3-methyl-2,3-dihydrobenzofuran-3-carboxamide

Ammonium chloride (1.25 g, 23.3 mmol) was added to a mixture of 5-bromo-3-methyl-2,3-dihydrobenzofuran-3-carboxylic acid (600 mg, 2.33 mmol), DIPEA (2.0 mL, 11.7 mmol), and HATU (1.33 g, 3.50 mmol) in DMF (6 mL) at 25 °C. The mixture was stirred for 12 hrs. The reaction mixture was diluted with water (20 mL) and extracted with EA (10 mL x 3). The combined organic layer was washed with brine (10 mL x 3), dried over anhydrous Na₂SO₄, filtered, and concentrated in vacuo affording the title compound (580 mg) as a yellow solid.
LCMS (ESI) m/z: [79BrM+H]+=257.0.
¹H NMR (400 MHz, DMSO-d6) δ = 7.60 (d, J = 2.0 Hz, 1H), 7.38 - 7.24 (m, 3H), 6.76 (d, J = 8.4 Hz, 1H), 5.03 (d, J = 8.8 Hz, 1H), 4.20 (d, J = 9.2 Hz, 1H), 1.52 (s, 3H) ppm.

### Step 5. 5-Bromo-3-methyl-2,3-dihydrobenzofuran-3-carbonitrile

Triethylamine (0.95 mL, 6.79 mmol) was added to a mixture of 5-bromo-3-methyl-2,3-dihydrobenzofuran-3-carboxamide (580 mg, 2.26 mmol) in DCM (6 mL) at 0 °C. TFAA (0.79 mL, 5.66 mmol) was then added at 0 °C. The mixture was stirred for 10 minutes. The reaction mixture was diluted with water (20 mL) and extracted with DCM (10 mL x 2). The combined organic phase was concentrated to afford yellow oil. The oil was purified by FCC (Eluent: 0 to 20% EA in PE) affording the title compound (500 mg, 1.89 mmol) as yellow oil.
¹H NMR (400 MHz, DMSO-d6) δ = 7.80 (d, J = 2.0 Hz, 1H), 7.47 - 7.44 (m, 1H), 6.93 (d, J = 8.8 Hz, 1H), 4.96 (d, J = 9.4 Hz, 1H), 4.46 (d, J = 9.6 Hz, 1H), 1.74 (s, 3H) ppm.

### Step 6. (R)-3-Cyano-3-methyl-2,3-dihydrobenzofuran-5-carboxylic acid and (S)-3-Cyano-3-methyl-2,3-dihydrobenzofuran-5-carboxylic acid

Palladium acetate (18.9 mg, 0.084 mmol) and dccp2HBF₄ (103 mg, 0.17 mmol) were added to a mixture of 5-bromo-3-methyl-2,3-dihydrobenzofuran-3-carbonitrile (400 mg, 1.68 mmol) and K₂CO₃ (348 mg, 2.52 mmol) in DMSO (3.2 mL) and H₂O (0.8 mL) at 25 °C. The mixture was degassed and purged with CO (g) three times. The mixture was stirred at 100 °C under a CO atmosphere (15 psi) for 12 hrs. The reaction mixture was filtered and the filtrate was purified by reversed-phase HPLC. The desired fractions were concentrated to remove MeCN and lyophilized affording the mixture of the title compounds (210 mg, 1.02 mmol) as a white solid. The stereoisomers were separated by chiral SFC (DAICEL CHIRALPAK IG (250mm*30mm,10µm);mobile phase: [0.1%NH₃₋H₂O MEOH];B%: 20%-20%,3.9min). The eluent was concentrated to afford Intermediate 14 (90 mg, 0.44 mmol) as a red solid and Intermediate 15 (100 mg, 0.49mol) as a light yellow solid.
**Intermediate 14:** Chiral SFC: AD-3_5CM_ETOH(DEA)_5_40_3ML_AT35.M, RT = 0.924 min.
LCMS (ESI) m/z: [M+H]+=204.1.
¹HNMR (400 MHz, DMSO-d6) δ = 8.02 (s, 1H), 7.90 - 7.88 (m, 1H), 6.98 (d, J = 8.4 Hz, 1H), 4.99 (d, J = 9.6 Hz, 1H), 4.52 (d, J = 9.6 Hz, 1H), 1.75 (s, 3H) ppm.
**Intermediate 15:** Chiral SFC: AD-3_5CM_ETOH(DEA)_5_40_3ML_AT35.M, RT = 1.078 min.
LCMS (ESI) m/z: [M+H]+=204.
¹H NMR (400 MHz, DMSO-d6) δ = 8.02 (d, J = 1.6 Hz, 1H), 7.89 - 7.87 (m, 1H), 6.97 (d, J = 8.4 Hz, 1H), 4.99 (d, J = 9.6 Hz, 1H), 4.52 (d, J = 9.2 Hz, 1H), 1.75 (s, 3H) ppm.

### Intermediate 16: 3-[3-(difluoromethyl)oxetan-3-yl]benzoic acid

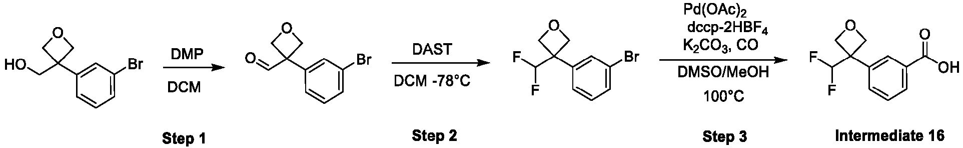

### Step 1. 3-(3-Bromophenyl)oxetane-3-carbaldehyde

DMP (1.66 mL, 5.35 mmol) was added to a solution of [3-(3-bromophenyl)oxetan-3-yl]methanol (1 g, 4.11 mmol) in DCM (10 mL) at 0 °C. The mixture was stirred for 1 hr at 25 °C. The mixture was filtered and concentrated. The residue was purified by FCC (Eluent: PE:EA = 100:1 to 1:100) affording the title compound (850 mg, 3.53 mmol) as colorless oil.
LCMS (ESI) m/z: [79BrM+H]+ = 259.0.
¹H NMR (400 MHz, CDCl₃) δ = 9.71 (s, 1H), 7.46 - 7.40 (m, 1H), 7.24 (t, J = 8.0 Hz, 1H),7.18 (t, J = 2.0 Hz, 1H), 6.99 - 6.92 (m, 1H), 5.08 (d, J = 6.4 Hz, 2H), 4.91 (d, J = 6.4 Hz, 2H) ppm.

### Step 2. 3-(3-Bromophenyl)-3-(difluoromethyl)oxetane

DAST (1.15 mL, 8.71 mmol) was added to a solution of 3-(3-bromophenyl)oxetane-3-carbaldehyde (700 mg, 2.90 mmol) in DCM (7 mL) at -78 °C. The mixture was warmed and stirred for 1 hr at 0 °C. The mixture was poured into saturated aqueous solution of NaHCO₃ (5 mL). The organic layer was collected and concentrated in vacuo. The residue was purified by FCC (Eluent: PE:EA = 100:1 to 1:1) affording the title compound (500 mg, 1.90 mmol) as brown oil.
¹H NMR (400 MHz, CDCl₃) δ = 7.59 - 7.45 (m, 1H), 7.34 - 7.29 (m, 1H), 7.26 (s, 1H), 7.11 -6.94 (m, 1H), 6.43 - 5.96 (m, 1H), 4.99 (s, 4H) ppm.

### Step 3. 3-[3-(Difluoromethyl)oxetan-3-yl]benzoic acid

Palladium acetate (6.40 mg, 0.029 mmol), dccp2HBF₄ (34.9 mg, 0.057 mmol), and K₂CO₃ (118 mg, 0.86 mmol was added to a solution of 3-(3-bromophenyl)-3-(difluoromethyl)oxetane (150 mg, 0.57 mmol) in DMSO (5 mL) and H₂O (2.5 mL) at 25 °C. The mixture was degassed and purged with CO (g) three times, and then the mixture was stirred at 100 °C for 16 hrs under a CO atmosphere (15 psi). The mixture was extracted with EA (5 ml). The aqueous layer was adjusted pH to 5 - 6 with aqueous HCl (1 M) and extracted with EA (5 ml x 3). The organic layers were combined, dried over anhydrous Na₂SO₄, filtered, and concentrated in vacuo affording the title compound (120 mg, 0.53 mmol) as colorless oil. LCMS (ESI) m/z: [M+H]⁺ = 229.1.

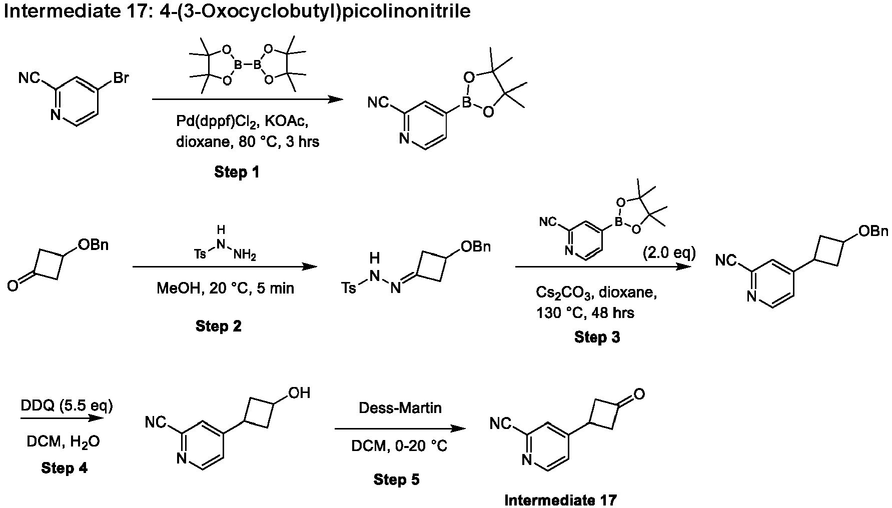

### Step 1: 4-(4,4,5,5-Tetramethyl-1,3,2-dioxaborolan-2-yl)picolinonitrile

To a solution of 4-bromopyridine-2-carbonitrile (25 g, 136.61 mmol) and 4,4,5,5-tetramethyl-2-(4,4,5,5-tetramethyl-1,3,2-dioxaborolan-2-yl)-1,3,2-dioxaborolane (41.63 g, 163.93 mmol) in dioxane (250 mL) was added KOAc (40.22 g, 409.82 mmol) and Pd(dppf)Cl₂.CH₂Cl₂ (3 g, 3.67 mmol). The mixture was stirred at 80 °C for 3 hrs. The reaction mixture was poured into water (1000 mL), the solution was extracted with EA (1000 mL * 2), the combined organic layer was washed with brine (2000 mL), dried over Na₂SO₄, filtered and concentrated. The residue was purified by column chromatography (SiO₂, Petroleum ether/Ethyl acetate=10:1-1:1) to give the title compound (24 g, 104.32 mmol, 76.36% yield) as a white solid.
LCMS (ESI) m/z: [M+H]⁺ = 231.1
¹H NMR (400 MHz, DMSO-d₆) δ = 8.79 (d, *J* = 4.4 Hz, 1H), 8.04 (s, 1H), 7.87 (d, *J* = 4.8 Hz, 1H), 1.31 (s, 12H) ppm

### Step 2: N'-(3-(Benzyloxy)cyclobutylidene)-4-methylbenzenesulfonohydrazide

To a solution of 3-benzyloxycyclobutanone (15 g, 85.13 mmol) in MeOH (150 mL) was added 4-methylbenzenesulfonohydrazide (15.85 g, 85.13 mmol). The mixture was stirred at 20 °C for 5 min. The reaction mixture was filtered, the filter cake was washed MeOH (30 mL) and the filtrate dried in vacuo to give the title compound (23 g, 66.78 mmol, 78.45% yield) as a white solid.

### Step 3: 4-(3-(Benzyloxy)cyclobutyl)picolinonitrile

To a solution of *N*'-(3-(benzyloxy)cyclobutylidene)-4-methylbenzenesulfonohydrazide (23 g, 66.78 mmol) and 4-(4,4,5,5-tetramethyl-1,3,2-dioxaborolan-2-yl)picolinonitrile (23.05 g, 100.17 mmol) in dioxane (450 mL) was added Cs₂CO₃ (65.27 g, 200.33 mmol). The mixture was refluxed at 130 °C for 48 hr. The reaction mixture was diluted with water (800 mL) and extracted with EA (300 mL * 3). The combined organic layers were washed with dried over Na₂SO₄, filtered and concentrated under reduced pressure. The residue was purified by column chromatography (SiO₂, DCM/EA=1/0 to 10/1) to give the title compound (3.7 g, 13.29 mmol, 19.90% yield) as yellow oil.
LCMS (ESI) m/z: [M+H]⁺ = 265.1

### Step 4: 4-(3-Hydroxycyclobutyl)picolinonitrile

To a solution of 4-(3-(benzyloxy)cyclobutyl)picolinonitrile (3.7 g, 14.00 mmol) in DCM (120 mL) and H₂O (12 mL) was added DDQ (17.48 g, 76.99 mmol). The mixture was stirred at 30 °C for 16 hrs under N₂. The reaction mixture was poured into sat. NaSO₃ solution (500 mL) and extracted with DCM (500 mL * 3). The combined organic layer was washed with brine (500 mL), dried over Na₂SO₄, filtered and concentrated. The residue was purified by column chromatography (SiO₂, Petroleum ether/Ethyl acetate=5:1-0:1) to give the title compound (1.4 g, 8.04 mmol, 57.41% yield) as a yellow oil.

### Step 5: 4-(3-Oxocyclobutyl)picolinonitrile

To a solution of 4-(3-hydroxycyclobutyl)picolinonitrile (1.4 g, 5.44 mmol) in DCM (15 mL) was added Dess-Martin (6.92 g, 16.31 mmol). The mixture was stirred at 30 °C for 2 hrs. The reaction mixture was poured into Na₂SO₃ solution (100 mL) and extracted with EA (100 mL). The combined organic layer was washed with brine (200 mL), dried over Na₂SO₄, filtered and concentrated. The residue was purified by column chromatography (SiO₂, Petroleum ether/Ethyl acetate=5:1-0:1) to give the title compound (900 mg, 4.98 mmol, 91.62% yield) as an off-white solid.
LCMS (ESI) m/z: [M+H]⁺ = 173.1

### Intermediate 18: 2-Methyl-5,6,7,8-tetrahydro-2,6-naphthyridin-1(2H)-one

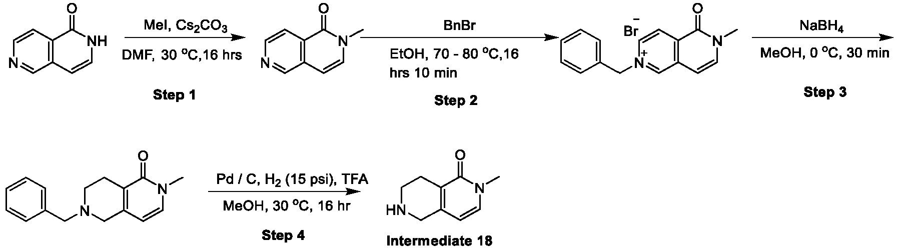

### Step 1: 2-Methyl-2,6-naphthyridin-1(2H)-one

To a mixture of 2,6-naphthyridin-1(2H)-one (200 mg, 1.37 mmol) in DMF (10 mL) was added Cs₂CO₃ (668.82 mg, 2.05 mmol) and Mel (291.36 mg, 2.05 mmol, 127.79 uL) at 30 °C. The mixture was stirred at 30 °C for 16 hrs. The mixture was poured into H₂O (10 mL) and extracted with DCM (10 mL*3). The combined organic layers were dried over anhydrous Na₂SO₄, filtered and concentrated under reduced pressure. The residue was purified by prep-TLC (DCM / MeOH = 20 / 1, Rf = 0.4) to afford the title compound (160 mg, 946.72 umol, 69.18% yield) as a yellow solid.
LCMS (ESI) m/z: [M+H]⁺ = 161.1.
¹H NMR (400 MHz, CDCl₃) δ = 8.98 (s, 1H), 8.69 (d, *J* = 5.2 Hz, 1H), 8.19 (d, *J* = 5.2 Hz, 1H), 7.20 (d, *J* = 7.2 Hz, 1H), 6.58 (d, *J* = 7.2 Hz, 1H), 3.65 (s, 3H) ppm.

### Step 2: 2-Benzyl-6-methyl-5-oxo-5,6-dihydro-2,6-naphthyridin-2-ium bromide

A mixture of 2-methyl-2,6-naphthyridin-1(2*H*)-one (50 mg, 312.16 umol) in EtOH (0.5 mL) was stirred at 70 °C for 10 min. Then benzyl bromide (720.00 mg, 4.21 mmol, 0.5 mL) was added. The mixture was stirred at 80 °C for 16 hrs. The mixture was treated with MTBE (10 mL) and filtered. The filter cake was dried under reduced pressure to afford the title compound (130 mg, crude) as a yellow solid. ¹H NMR (400 MHz, DMSO-d6) δ = 9.87 (s, 1H), 8.98 - 8.97 (m, 1H), 8.68 (d, *J* = 6.4 Hz, 1H), 7.96 (d, *J* = 7.2 Hz, 1H), 7.59 - 7.42 (m, 5H), 6.96 (d, *J* = 7.6 Hz, 1H), 5.96 (s, 2H), 3.60 (s, 3H) ppm.

### Step 3: 6-Benzyl-2-methyl-5,6,7,8-tetrahydro-2,6-naphthyridin-1(2H)-one

To a mixture of 2-benzyl-6-methyl-5-oxo-5,6-dihydro-2,6-naphthyridin-2-ium bromide (70 mg, 211.35 umol) in MeOH (1 mL) was added NaBH₄ (39.98 mg, 1.06 mmol) in several portions at 0 °C. The mixture was stirred at 0 °C for 30 min. The mixture was poured into 1M HCl aqueous solution (5 mL) and basified with sat. NaHCO₃ to pH=8. The mixture was extracted with EA (10 mL *3). The combined organic layers were dried over anhydrous Na₂SO₄, filtered and concentrated under reduced pressure to afford the title compound (35 mg, 137.62 umol, 65.1% yield) as a yellow solid.
LCMS (ESI) m/z: [M+H]⁺ = 255.3.
¹H NMR (400 MHz, CDCl₃) δ = 7.40 - 7.28 (m, 5H), 7.07 (d, *J* = 7.2 Hz, 1H), 5.84 (d, *J* = 6.8 Hz, 1H), 3.69 (s, 2H), 3.52 (s, 3H), 3.39 (s, 2H), 2.79 - 2.64 (m, 4H) ppm.

### Step 4: 2-Methyl-2,6-naphthyridin-1(2H)-one

To a mixture of 6-benzyl-2-methyl-5,6,7,8-tetrahydro-2,6-naphthyridin-1(2H)-one (35 mg, 137.62 umol) in MeOH (1 mL) was added TFA (31.38 mg, 275.24 umol, 20.38 uL) and Pd / C (20 mg, 10% purity). The mixture was purged with H₂ 3 times and stirred at 30 °C for 16 hrs under H₂ (15 psi). The mixture was filtered and the mother liquor was concentrated under reduced pressure to afford the title compound (30 mg, 107.83 umol, 78.4% yield, TFA salt) as a yellow oil.
LCMS (ESI) m/z: [M+H]⁺ = 165.1.

### Intermediate 19: 7-Fluoro-5-methoxy-1,2,3,4-tetrahydroisoquinoline hydrochloride

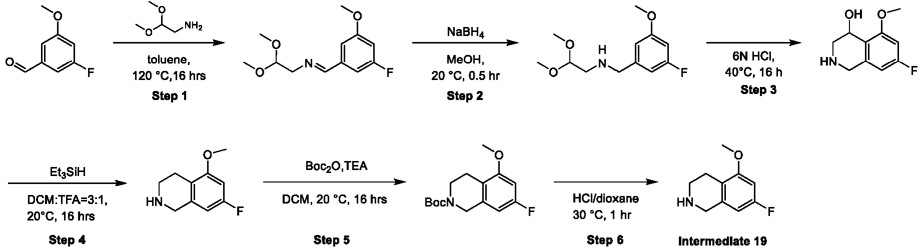

### Step 1: (E)-N-(2,2-Dimethoxyethyl)-1-(3-fluoro-5-methoxyphenyl)methanimine

To a solution of 3-fluoro-5-methoxy-benzaldehyde (2 g, 12.98 mmol) in toluene (20 mL) was added 2,2-dimethoxyethanamine (1.50 g, 14.27 mmol, 1.56 mL). The mixture was stirred at 120 °C for 16 hrs. The reaction mixture was concentrated to give the title compound (3 g, crude) as a yellow oil.

### Step 2: N-(3-Fluoro-5-methoxybenzyl)-2,2-dimethoxyethan-1-amine

To a solution of (*E*)-*N*-(2,2-dimethoxyethyl)-1-(3-fluoro-5-methoxyphenyl)methanimine (3 g, 12.43 mmol) in MeOH (30 mL) was added NaBH₄ (564.53 mg, 14.92 mmol) at 0 °C. The mixture was stirred at 30 °C for 30 min. The reaction mixture was poured into water (100 mL) and the mixture was extracted with EA (100 mL*3). The combined organic layer was washed with brine (200 mL), dried over Na₂SO₄, filtered and concentrated to give the title compound (2.2 g, 9.04 mmol, 72.73% yield) as a yellow oil. ¹H NMR (400 MHz, DMSO-d₆) δ=6.66 - 6.63 (m, 2H), 6.51 - 6.48 (m, 1H), 4.49 - 4.46 (m, 1H), 3.79 (s, 3H), 3.76 (s, 2H), 3.47 - 3.37 (m, 6H), 2.73 (d, *J* = 5.4 Hz, 2H) ppm.

### Step 3: 7-Fluoro-5-methoxy-1,2,3,4-tetrahydroisoquinolin-4-ol

A mixture of *N*-(3-fluoro-5-methoxybenzyl)-2,2-dimethoxyethan-1-amine (1 g, 4.11 mmol) in HCl (10 mL) was stirred at 40 °C for12 hrs. The reaction mixture was concentrated to give the title compound (810 mg, crude) as a yellow oil.

### Step 4: 7-Fluoro-5-methoxy-1,2,3,4-tetrahydroisoquinoline

To a solution of 7-fluoro-5-methoxy-1,2,3,4-tetrahydroisoquinolin-4-ol (810 mg, 4.11 mmol) in DCM (8 mL) was added Et₃SiH (1.43 g, 12.32 mmol, 1.97 mL) and TFA (5.39 g, 47.27 mmol, 3.5 mL). The mixture was stirred at 30 °C for 16 hrs. The reaction mixture was concentrated to give the title compound (744 mg, crude) as a yellow oil.
LCMS (ESI) m/z: [M+H]⁺ = 182.3

### Step 5: tert-Butyl 7-fluoro-5-methoxy-3,4-dihydroisoquinoline-2(1H)-carboxylate

To a solution of 7-fluoro-5-methoxy-1,2,3,4-tetrahydroisoquinoline (744 mg, 4.11 mmol) in DCM (10 mL) was added Et₃N (2.08 g, 20.53 mmol, 2.86 mL) and Boc₂O (1.08 g, 4.93 mmol, 1.13 mL). The mixture was stirred at 30 °C for 2 hrs. The reaction mixture was poured into water (20 mL) and extracted with EA (20 mL*3). The combined organic layer was washed with brine (50 mL), dried over Na₂SO₄, filtered and concentrated. The residue was purified by column chromatography (SiO₂, Petroleum ether/Ethyl acetate=5:1-1:1) to give the title compound (150 mg, 494.22 umol, 12.04% yield) as a white solid.
LCMS (ESI) m/z: [M+H]⁺ = 226.2
¹H NMR (400 MHz, DMSO-d₆) δ= 6.45 - 6.42 (m, 2H), 4.54 - 4.51 (m, 2H), 3.80 (s, 3H), 3.63 - 3.60 (m, 2H), 2.69 - 2.66 (m, 2H), 1.49 (s, 9H) ppm.

### Step 6: 7-Fluoro-5-methoxy-1,2,3,4-tetrahydroisoquinoline hydrochloride

A mixture of *tert*-butyl 7-fluoro-5-methoxy-3,4-dihydroisoquinoline-2(1*H*)-carboxylate (150 mg, 533.20 umol) in HCl/dioxane (2 mL) was stirred at 30 °C for 1 hr. The reaction mixture was concentrated and the residue was triturated with MTBE (10 mL) to give the title compound (60 mg, 263.71 umol, 49.46% yield) as a white solid.
LCMS (ESI) m/z: [M+H]⁺ = 182.1
¹H NMR (400 MHz, DMSO-d₆) δ= 6.53 - 6.43 (m, 2H), 4.28 (s, 2H), 3.81 (s, 3H), 3.43 (s, 2H), 3.00 (s, 2H) ppm

### Intermediate 20: 3-Methoxy-3-phenylpyrrolidine hydrochloride

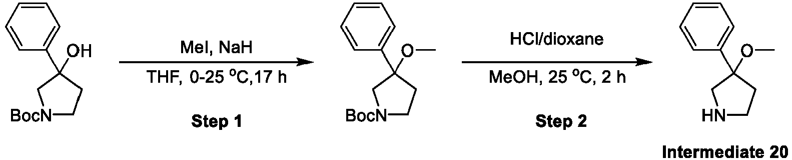

### Step 1: tert-Butyl 3-methoxy-3-phenylpyrrolidine-1-carboxylate

To a solution of *tert*-butyl 3-hydroxy-3-phenylpyrrolidine-1-carboxylate (200 mg, 759.50 umol) in DMF (3 mL) was added NaH (42.53 mg, 1.06 mmol, 60% purity) at 0 °C. The mixture was stirred at 0 °C for 1 hr. Mel (161.70 mg, 1.14 mmol, 70.92 uL) was added to the mixture and stirring at 25 °C continued for 16 hrs. The mixture was poured into water (50 mL) and extracted with EA (30 mL *3). The combined organic layers were washed with brine (30 mL), dried over Na₂SO₄, filtered and concentrated. The residue was purified by flash silica gel chromatography (ISCO^{®}; 12 g SepaFlash^{®} Silica Flash Column, Eluent of 0-60% Ethyl acetate/Petroleum ether gradient @ 60 mL/min) to give the title compound (180 mg, 648.98 umol, 85.45% yield) as a colorless oil.
¹HNMR (400 MHz, CDCl₃) δ = 7.43 - 7.29 (m, 5H), 3.97 - 3.78 (m, 1H), 3.64 - 3.45 (m, 3H), 3.02 (d, *J* = 5.6 Hz, 3H), 2.48 - 2.36 (m, 1H), 2.24 - 2.09 (m, 1H), 1.48 (d, *J* = 4.8 Hz, 9H) ppm.

### Step 2: 3-Methoxy-3-phenylpyrrolidine hydrochloride

To a solution of *tert*-butyl 3-methoxy-3-phenylpyrrolidine-1-carboxylate (170 mg, 612.93 umol) in MeOH (2 mL) was added HCl/dioxane (4 M, 2 mL). The mixture was stirred at 25 °C for 2 hrs. The reaction was concentrated under reduced pressure to to give the title compound (120 mg, crude, HCl) as a white solid.
¹HNMR (400 MHz, DMSO-d₆) δ = 9.89 (br s, 1H), 9.48 (br s, 1H), 7.47 - 7.34 (m, 4H), 3.80 - 3.67 (m, 1H), 3.40 - 3.14 (m, 3H), 2.92 (s, 3H), 2.63 - 2.53 (m, 1H), 2.24 - 2.10 (m, 1H) ppm.

### Intermediate 21: 1-(Azetidin-3-yl)-1H-indole

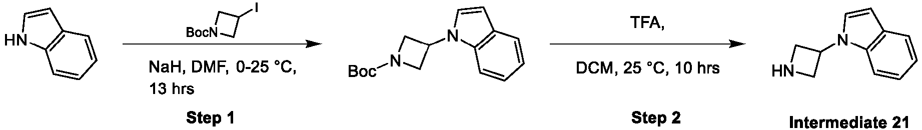

### Step 1: tert-Butyl 3-(1H-indol-1-yl)azetidine-1-carboxylate

To a solution of indole (500 mg, 4.27 mmol) in DMF (5 mL) was added NaH (256.06 mg, 6.40 mmol, 60% purity) in portions at 0 °C. The reaction mixture was stirred at 0 °C for 0.5 hr. *tert*-Butyl 3-iodoazetidine-1-carboxylate (1.33 g, 4.69 mmol) was then added dropwise. The reaction solution was stirred at 25 °C for 12.5 hrs. The reaction mixture was quenched by saturated aqueous NH₄Cl (10 mL), diluted with H₂O (10 mL) and extracted with EA (10 mL*3). The combined organic layers were washed with brine (10 mL*3), dried over Na₂SO₄, filtered and concentrated under reduced pressure. The residue was purified by reversed phase chromatography (FA condition). The fraction was concentrated under reduced pressure to remove MeCN and extracted with EA (25 mL * 3). The combined organic layers were washed with brine (20 mL), dried over Na₂SO₄, filtered and concentrated under reduced pressure to give the title compound (520 mg, 1.77 mmol, 41.41 % yield) as a red oil.
LCMS (ESI) m/z: [M+H]⁺=273.1
¹H NMR (400 MHz, CDCl₃-d) δ = 7.66 (d, *J* = 7.6 Hz, 1H), 7.39 (d, *J* = 8.4 Hz, 1H), 7.32 (d, *J* = 3.2 Hz, 1H), 7.26 - 7.22 (m, 1H), 7.19 - 7.11 (m, 1H), 6.60 (d, *J* = 2.8 Hz, 1H), 5.22 - 5.18 (m, 1H), 4.52 - 4.48 (m, 2H), 4.36 - 4.32 (m, 2H), 1.50 (s, 9H)

### Step 2: 1-(Azetidin-3-yl)-1H-indole

To a solution of tert-butyl 3-(1*H*-indol-1-yl)azetidine-1-carboxylate (220 mg, 807.81 umol) in DCM (3 mL) was added TFA (92.11 mg, 807.81 umol, 59.81 uL). The reaction mixture was stirred at 25 °C for 10 hrs. The reaction mixture was then concentrated under reduced pressure and the residue purified by reversed phase chromatography (FA condition). The fraction was concentrated under reduced pressure to remove MeCN and lyophilized to give the title compound (110 mg, 504.01 umol, 62.39 % yield, FA) as a red solid.
LCMS (ESI) m/z: [M+H]⁺=173.1

### Intermediate 22: 7-(Difluoromethyl)-1,2,3,4-tetrahydroisoquinoline hydrochloride

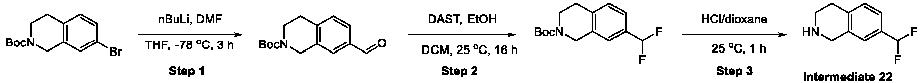

### Step 1: tert-Butyl 7-formyl-3,4-dihydroisoquinoline-2(1H)-carboxylate

To a solution of tert-butyl 7-bromo-3,4-dihydroisoquinoline-2(1*H*)-carboxylate (1 g, 3.20 mmol) in THF (40 mL) at -78 °C under N₂ was added n-BuLi (2.5 M, 2.56 mL) dropwise. The reaction was stirred at -78 °C for 0.5 hr. DMF (468.25 mg, 6.41 mmol, 492.89 uL) was then added dropwise at -78 °C and stirred at -78 °C for 2.5 hrs. The reaction mixture was quenched with aq. NH₄Cl (60 mL) and extracted with EA (50 mL*3). The combined organic layer was dried over anhydrous Na₂SO₄, filtered and concentrated under reduced pressure. The residue purified by flash silica gel chromatography (SiO₂, Petroleum ether/Ethyl acetate=1/0~1/1) to give the title compound (280 mg, 1.01 mmol, 31.46% yield) as a colorless oil.
LCMS (ESI) m/z: [M+H-56]⁺= 206.1
¹H NMR (400 MHz, DMSO-d₆) δ = 9.95 (s, 1H), 7.76 - 7.68 (m, 2H), 7.39 (d, *J* = 8.0 Hz, 1H), 4.59 (s, 2H), 3.58 - 3.55 (m, 2H), 2.88 - 2.85 (m, 2H), 1.46 - 1.41 (m, 9H) ppm.

### Step 2: tert-Butyl 7-(difluoromethyl)-3,4-dihydroisoquinoline-2(1H)-carboxylate

To a solution of tert-butyl 7-formyl-3,4-dihydroisoquinoline-2(1H)-carboxylate (150 mg, 574.02 umol) in DCM (1.5 mL) was added DAST (157.29 mg, 975.83 umol, 128.93 uL) and EtOH (5.29 mg, 114.80 umol, 6.71 uL). The mixture was stirred at 25 °C for 14 hrs. Additional DAST (92.53 mg, 574.02 umol, 75.84 uL) was added at 0 °C and the mixture stirred at 25 °C for 2 hrs. The reaction mixture was diluted with H₂O (20 mL) and extracted with EA (20 mL*3). The combined organic layer was dried over anhydrous Na₂SO₄, filtered and concentrated under reduced pressure. The residue was purified by flash silica gel chromatography (SiO₂, Petroleum ether/Ethyl acetate=1/0 to 1/1) to give the title compound (120 mg, 388.11 umol, 67.61% yield) as a colorless oil.
LCMS (ESI) m/z: [M+H-56]⁺= 228.1
¹H NMR (400 MHz, DMSO-d₆) δ = 7.40 - 7.34 (m, 2H), 7.32 - 7.28 (m, 1H), 7.12 - 6.82 (m, 1H), 4.54 (s, 2H), 3.57 - 3.52 (m, 2H), 2.83 - 2.80 (m, 2H), 1.43 (s, 9H) ppm.

### Step 3: 7-(Difluoromethyl)-1,2,3,4-tetrahydroisoquinoline hydrochloride

To a solution of tert-butyl 7-(difluoromethyl)-3,4-dihydroisoquinoline-2(1H)-carboxylate (120 mg, 423.56 umol) in dioxane (1 mL) was added HCl/dioxane (4 M, 756.34 uL). The reaction mixture was stirred at 25 °C for 1 hr. The reaction mixture was concentrated under reduced pressure to give the title compound (90 mg, 378.26 umol, 89.30% yield) as white solid.
LCMS (ESI) m/z: [M+H]⁺ = 184.0.

### Intermediate 23: 7-Bromo-1,5-naphthyridin-2(1H)-one

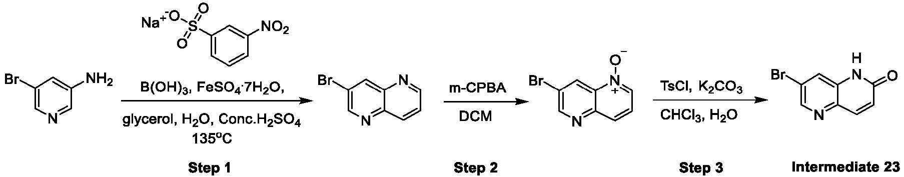

### Step 1. 3-Bromo-1,5-naphthyridine

Boric acid (5.72 g, 92.5 mmol), FeSO_{4·}7H₂O (2.09 g, 7.51 mmol), and sodium 3-nitrobenzenesulfonate hydrate (28.1 g, 116 mmol) were added to a solution of concentrated H₂SO₄ (50 mL) and the mixture was stirred at 30 °C for 0.5 hr. Glycerol (20.3 mL, 272 mmol), 5-bromopyridin-3-amine (10.0 g, 57.8 mmol), and H₂O (50 mL) were added to the reaction solution, and the mixture was stirred at 135°C for 18 hrs. The mixture was cooled to 30°C, and the pH was adjusted to pH=14 with NaOH. The aqueous layer was extracted with EA (300 mL x 3), and the combined organic layer was washed with brine (500 mL), dried over anhydrous Na₂SO₄, filtered, and concentrated in vacuo. The residue was purified by FCC (Eluent: PE:EA = 10:1 to 2:1) affording the title compuond (4.50 g, 19.9 mmol) as a yellow solid.
LCMS (ESI) m/z: [Br79M+H]+ = 209.0
¹H NMR (400 MHz, DMSO-d6) δ= 8.98 - 8.97 (m, 2H), 8.58 (d, J 1.6 Hz, 1H), 8.40 - 8.37 (m, 1H), 7.67 - 7.64 (m, 1H).

### Step 2. 7-Bromo-1,5-naphthyridine 1-oxide

m-CPBA (5.24 g, 25.8 mmol, 85% purity) was added to a solution of 3-bromo-1,5-naphthyridine (4.50 g, 21.5 mmol) in DCM (45.0 mL). The mixture was stirred at 30 °C for 16 hr. The mixture was poured into saturated Na₂SO₃ (100 mL) and extracted with DCM (100 mL x 3). The combined organic layer was washed with brine (100 mL), dried over anhydrous Na₂SO₄, filtered, and concentrated. The crude product was triturated with MTBE (50 mL), and the solid was filtered. The solid was collected and dried in vacuo affording the title compound (3.3 g, 14.66 mmol) as a yellow solid.
¹H NMR (400 MHz, DMSO-d6) δ= 9.23 - 9.22 (m, 1H), 9.03 (d, J = 2.0 Hz, 1H), 8.55 (d, J = 6.4 Hz, 1H), 7.99 (d, J =8.8 Hz, 1H), 7.57 - 7.53 (m, 1H).

### Step 3. 7-Bromo-1,5-naphthyridin-2(1H)-one

Potassium carbonate (6.89 g, 49.86 mmol) in water (25 mL) was added to a solution of 7-bromo-1,5-naphthyridine 1-oxide (3.30 g, 14.7 mmol) and TosCl (3.35 g, 17.6 mmol) in CHCl₃ (60 mL). The mixture was stirred at 30 °C for 16 hrs. The reaction mixture was filtered, and the filter cake was dried in vacuo affording the title compound (2.5 g, 10.0 mmol) as a yellow solid.
LCMS (ESI) m/z: [Br79M+H]+ = 225.1
¹H NMR (400 MHz, DMSO-d6) δ = 12.21 - 11.78 (m, 1H), 8.54 (d, J = 2.0 Hz, 1H), 7.91 (d, J = 10.4 Hz, 1H), 7.85 (d, J = 1.6 Hz, 1H), 6.78 - 6.74 (m, 1H).

### Intermediate 24: tert-Butyl N-[1-(2-phenyl-1,6-naphthyridin-7-yl)ethyl]carbamate

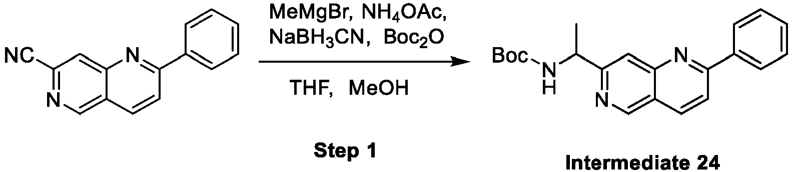

### tert-Butyl N-[1-(2-phenyl-1,6-naphthyridin-7-yl)ethyl]carbamate

Methylmagnesium bromide (3 M, 1.73 mL) was added to a solution of 2-phenyl-1,6-naphthyridine-7-carbonitrile (300 mg, 1.30 mmol) in THF (6 mL) at 0 °C. The mixture was warmed and stirred at 25°C for 1 hr. The reaction mixture was quenched by addition dry MeOH (3 mL), then NH₄OAc (1 g, 13.0 mmol) and NaBH₃CN (815 mg, 13.0 mmol) were added and the mixture was stirred at 25 °C for 1 hr. Boc₂O (1.70 g, 7.78 mmol) was added and the reaction mixture was stirred at 25°C for 16 hrs. The reaction mixture was quenched with water (100 mL) and extracted with EA (100 mL x 2). The combined organic layers were washed with brine (100 mL), dried over anhydrous Na₂SO₄, filtered, and concentrated under in vacuo. The crude product was purified by FCC (Eluent: PE:EA = 1:1) affording the title compound (250 mg, 0.68 mmol) as light yellow oil.
LCMS (ESI) m/z: [M+H]⁺ = 350.1.
¹H NMR (400 MHz, DMSO-d6) δ = 9.34 (s, 1H), 8.63 (d, J = 8.8 Hz, 1H), 8.33 - 8.24 (m, 3H), 7.84 (s, 1H), 7.62 - 7.53 (m, 4H), 4.87 (d, J = 7.2 Hz, 1H), 1.47 - 1.37 (m, 12H) ppm.

### Intermediates 25 and 26: (3S)-3-(Hydroxymethyl)-3-methyl-indane-5-carboxylic acid and (3R)-3-(Hydroxymethyl)-3-methyl-indane-5-carboxylic acid

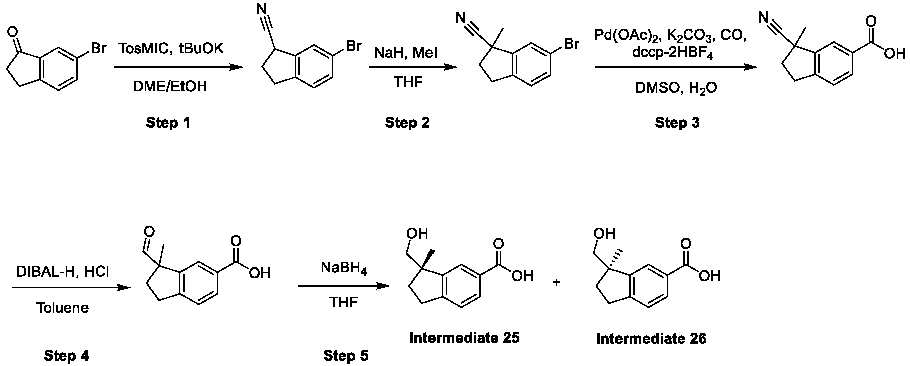

### Step 1. 6-Bromoindane-1-carbonitrile

Potassium tert-butoxide (53.2 g, 474 mmol) was added to a solution of 6-bromoindan-1-one (50 g, 237 mmol) and 1-(isocyanomethylsulfonyl)-4-methyl-benzene (69.4 g, 356 mmol) in DME (2.5 L) and EtOH (100 mL) at 0 °C. The reaction mixture was stirred at 25°C for 16 hrs. The reaction mixture was poured into water (2 L) and extracted with EA (2 L x 2). The combined organic layer was concentrated in vacuo. The crude product was diluted with EA (500 mL), washed with brine (500 mL), dried over anhydrous Na₂SO₄, and concentrated in vacuo. The residue was purified by FCC (Eluent: PE:EA = 20:1). The desired fractions were collected and concentrated in vacuo. The solid was then triturated with MTBE (100 mL) affording the title compound (29 g, 130 mmol) as a white solid.
¹H NMR (400 MHz, CHLOROFORM-d) δ = 7.58 (s, 1H), 7.42 - 7.40 (m, 1H), 7.16 (d, J = 8.4 Hz, 1H), 4.12 - 4.08 (m, 1H), 3.10 - 300 (m, 1H), 2.97 - 2.86 (m, 1H), 2.65 - 2.55 (m, 1H), 2.43 - 2.37 (m, 1H) ppm.

### Step 2. 6-Bromo-1-methyl-indane-1-carbonitrile

Sodium hydride (10.8 g, 270 mmol, 60% purity) was added to a solution of 6-bromoindane-1-carbonitrile (30 g, 135 mmol) in THF (300 mL) at 0 °C. The reaction mixture was warmed and stirred at 10°C for 0.5 hr. CH₃l (42.1 mL, 675.43 mmol) was added at 0 °C. The mixture was warmed and stirred at 25 °C for 2 hrs. The reaction was diluted with NH₄Cl (aq., 800 mL) and extracted with EA (500 mL x 3). The combined organic layers were dried over anhydrous Na₂SO₄, filtered, and concentrated under reduced pressure. The crude residue was purified by FCC (Eluent: PE:EA = 1:0 to 5:1) affording the title compound (27 g, 114 mmol) as a yellow solid.
¹H NMR (400 MHz, DMSO-d6) δ = 7.70 (d, J = 2.0 Hz, 1H), 7.50 - 7.48 (m, 1H), 7.29 (d, J = 8.0 Hz, 1H), 2.97 - 2.93 (m, 2H), 2.60 - 2.53 (m, 1H), 2.18 (d, J = 12.8 Hz, 1H), 1.64 (s, 3H) ppm.

### Step 3. 3-Cyano-3-methyl-indane-5-carboxylic acid

A mixture of 6-bromo-1-methyl-indane-1-carbonitrile (6.5 g, 27.5 mmol), dccp2HBF4 (1.69 g, 2.75 mmol), postassium carbonate (5.71 g, 41.3 mmol), Pd(OAc)₂ (618 mg, 2.75 mmol), and water (0.99 mL) in DMSO (60 mL) was degassed and purged with CO (g) three times. The mixture was stirred at 100°C for 14 hr under a CO (15 psi) atmosphere. The reaction mixture was filtered to remove the black solid, and then diluted with water (60 mL) and extracted with EA (80 mL x 3). The organic layer was discarded and the aqueous phase was adjusted to pH=4 with aq. HCl. The aqueous solution was then extracted with EA (100 mL x 3). The combined organic layers were washed with brine (500 mL x 2) then dried over anhydrous Na₂SO₄, filtered, and concentrated under reduced pressure affording the title compound (4.5 g, 20.7 mmol) as a brown solid.
LCMS (ESI) m/z: [M+H]+= 202.1.
¹H NMR (400 MHz, DMSO-d6) δ = 13.04 (s, 1H), 7.96 (s, 1H), 7.91 - 7.88 (m, 1H), 7.45 (d, J = 8.0 Hz, 1H), 3.07 - 3.04 (m, 2H), 2.64 - 2.53 (m, 1H), 2.28 - 2.19 (m, 1H), 1.65 (s, 3H) ppm.

### Step 4. 3-Formyl-3-methyl-indane-5-carboxylic acid

DIBAL-H (1 M, 159.03 mL) was added to a solution of 3-cyano-3-methyl-indane-5-carboxylic acid (16 g, 79.5 mmol) in toluene (160 mL) at -70 °C. The mixture was stirred at -70°C for 2 hrs. The reaction was quenched via the addition of HCl (2 M, 323 mL). The reaction mixture was warmed and stirred at 20°C for 1 h. The mixture was diluted with water (2 L) and extracted with EA (1.5 L x 3). The combined organic layer was dried over anhydrous Na₂SO₄, filtered, and concentrated affording the title compound (14 g, 58.2 mmol) as a white solid.
LCMS (ESI) m/z: [M+H]+= 205.1.
¹H NMR (400 MHz, DMSO-d6) δ = 12.91 (br s, 1H), 9.58 (s, 1H), 7.85 - 7.83 (m, 1H), 7.73 (s, 1H), 7.40 (d, J = 8.0 Hz, 1H), 3.06 - 2.90 (m, 2H), 2.61 - 2.54 (m, 1H), 1.98 - 1.87 (m, 1H), 1.39 (s, 3H) ppm.

### Step 5. (3S)-3-(Hydroxymethyl)-3-methyl-indane-5-carboxylic acid and (3R)-3-(Hydroxymethyl)-3-methyl-indane-5-carboxylic acid

Sodium borohydride (2.49 g, 65.8 mmol) was added to a solution of 3-formyl-3-methyl-indane-5-carboxylic acid (14 g, 58.2 mmol) in THF (150 mL) at 0 °C. The mixture was warmed and stirred at 25°C for 3 hrs. The reaction was diluted with water (200 mL) and extracted with EA (80 mL x 2). The organic layer was discarded and the aqueous phase was adjusted to pH=4 with aq. HCl. The aqueous solution was then extracted with EA (300 mL x 3). The organic layer was dried over anhydrous Na₂SO₄, filtered, and concentrated in vacuo. The residue was washed with DCM (50 mL x 3), filtered, and concentrated to get a white solid. The filtrate was concentrated under reduced pressure and the resulting residue was purified by FCC (Eluent: PE:EA = 1:0 to 1:5). The desired fractions were collected and concentrated under reduced pressure to get off-white solid. The solid was then washed with MTBE (20 mL x 2). This solid was combined with the filtered solid and dried in vacuo affording a mixture of the title compounds (9.5 g, 44.3 mmol) as a white solid. The stereoisomers were separated by chiral SFC (column: DAICEL CHIRALPAK AD-H(250mm*30mm,5µm); mobile phase: [0.1%NH₃₋H₂O MEOH]; B%: 40%-40%,5 min; 540 min) affording the pure title compounds.
**Intermediate 25:** (4.0 g, 19.40 mmol, white solid)
Chiral SFC: AD-3_5CM_MEOH (DEA)_5_40_3ML_AT35.M, Rt= 1.575 min, ee%= 100%.
LCMS (ESI) m/z: [M+H]+= 207.2.
¹H NMR (400 MHz, DMSO-d6) δ= 7.74 - 7.68 (m, 2H), 7.17 (d, J = 8.4 Hz, 1H), 3.39 - 3.31 (m, 2H), 2.87 - 2.80 (m, 2H), 2.15 - 2.12 (m, 1H), 1.73 - 1.65 (m, 1H), 1.20 (s, 3H) ppm.
**Intermediate 26:** ((4.3 g, 20.44 mmol, light yellow solid)
Chiral SFC: AD-3_5CM_MEOH (DEA)_5_40_3ML_AT35.M, Rt= 1.799 min, ee%= 97.93%.
LCMS (ESI) m/z: [M+H]+= 207.2.
¹H NMR (400 MHz, DMSO-d6) δ= 7.73 - 7.64 (m, 2H), 7.16 (d, J = 8.4 Hz, 1H), 3.44 - 3.25 (m, 2H), 2.88 - 2.78 (m, 2H), 2.15 - 2.12 (m, 1H), 1.73 - 1.65 (m, 1H), 1.20 (s, 3H) ppm.

### Intermediates 27 and 28: (S)-3-cyano-1,3-dimethylindoline-5-carboxylic acid and (R)-3-cyano-1,3-dimethylindoline-5-carboxylic acid

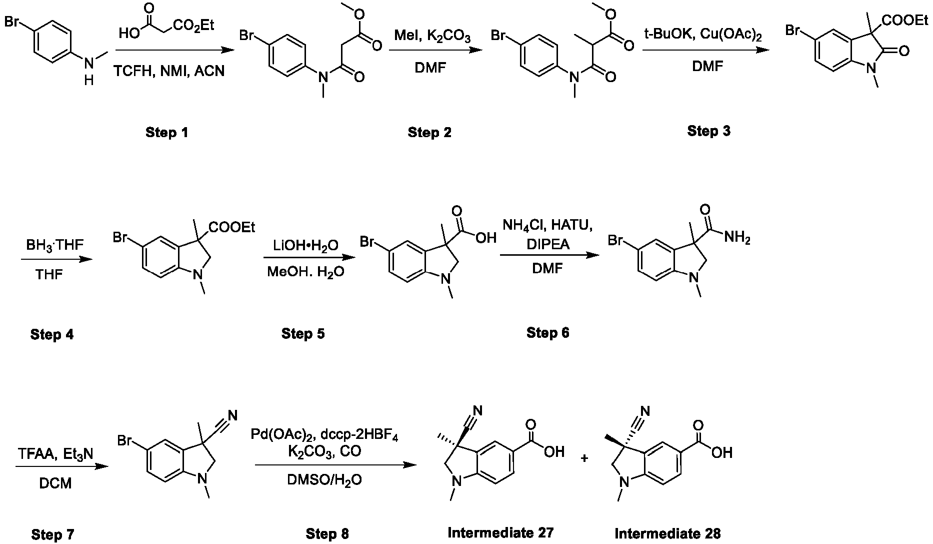

### Step 1. Methyl 3-((4-bromophenyl)(methyl)amino)-3-oxopropanoate

TCFH (22.6 g, 80.6 mmol) and NMI (12.5 mL, 161.2 mmol) were added to a mixture of 4-bromo-N-methyl-aniline (10 g, 53.8 mmol) and 3-ethoxy-3-oxo-propanoic acid (8.52 g, 64.5 mmol) in MeCN (150 mL) at 20 °C. The mixture was stirred at 20 °C for 15 hrs. The mixture was concentrated and diluted with water (10 mL). The aqueous solution was then extracted with DCM (15 mL x 3). The combined organic phase was dried over anhydrous Na₂SO₄, filtered, and concentrated in vacuo. The residue was purified by FCC (Eluent: PE:EA = 5:1 to 3:1) affording the title compound (13 g, 43.3 mmol) as a white solid. LCMS (ESI) m/z: [M+H]⁺ = 286.0.
¹H NMR (400MHz, CDCl₃) δ = 7.56 (d, J=8.4 Hz, 2H), 7.14 (d, J=8.4 Hz, 2H), 4.13 (d, J=7.2 Hz, 2H), 3.29 (s, 3H), 3.21 (s, 2H), 1.29 - 1.15 (m, 3H) ppm.

### Step 2. Methyl 3-((4-bromophenyl)(methyl)amino)-2-methyl-3-oxopropanoate

Potassium carbonate (4.14 g, 30.0 mmol), and Mel (3.7 mL, 59.9 mmol) were added to a mixture of methyl 3-((4-bromophenyl)(methyl)amino)-3-oxopropanoate (6 g, 20.0 mmol) in DMF (60 mL) at 0°C. The reaction mixture was stirred at 20 °C for 15 hrs. The reaction was diluted with water (100 mL) and extracted with EA (60 mL x 3). The combined organic phase was washed with brine (50 mL), dried over anhydrous Na₂SO₄, filtered, and concentrated in vacuo. The residue was purified by FCC (Eluent: PE:EA = 5:1 to 3:1) affording the title compound (4 g, 12.7 mmol) as a yellow solid.
LCMS (ESI) m/z: [M+H]⁺ = 300.0.
¹H NMR (400MHz, CDCl₃) δ = 7.57 (d, J=8.4 Hz, 2H), 7.18 - 7.11 (m, 2H), 4.19 - 4.03 (m, 2H), 3.40 - 3.28 (m, 1H), 3.28 (s, 3H), 1.31 (d, J=7.2 Hz, 3H), 1.27 - 1.21 (m, 3H) ppm.

### Step 3. Ethyl 5-bromo-1,3-dimethyl-2-oxoindoline-3-carboxylate

t-BuOK (2.86 g, 25.5 mmol) and copper(ll)acetate monohydrate (5.08 g, 25.5 mmol) were added to a mixture of methyl 3-((4-bromophenyl)(methyl)amino)-2-methyl-3-oxopropanoate (4 g, 12.7 mmol) in DMF (120 mL) at 20 °C. The mixture was stirred at 100°C for 1 hr. The mixture was filtered off, the filtrate was diluted with water (150 mL), and then extracted with EA (50 mL x 3). The combined organic phase was washed with brine (50 mL), dried over anhydrous Na₂SO₄, filtered, and concentrated in vacuo. The residue was purified by FCC (Eluent: PE:EA = 5:1 to 3:1) affording the title compound (2 g, 5.77 mmol) as a yellow solid.
LCMS (ESI) m/z: [M+H]⁺ = 312.0.
¹H NMR (400MHz, CDCl₃) δ = 7.46 - 7.44 (m, 1H), 7.38 (d, J=2.0 Hz, 1H), 6.75 (d, J=8.4 Hz, 1H), 4.23 - 4.09 (m, 2H), 3.26 - 3.19 (m, 3H), 1.66 (s, 3H), 1.21 - 1.16 (m, 3H) ppm.

### Step 4. Ethyl 5-bromo-1,3-dimethylindoline-3-carboxylate

BH₃·THF (1 M, 9.61 mL) was added to a solution of ethyl 5-bromo-1,3-dimethyl-2-oxoindoline-3-carboxylate (2 g, 6.41 mmol) in THF (20 mL) at 0 °C. The mixture was stirred at 60°C for 2 hrs. The mixture was slowly quenched with MeOH (30 mL) at 0 °C and then concentrated. The crude product was purified by FCC (Eluent: PE:EA = 10:1 to 3:1) affording the title compound (300 mg, 0.86 mmol) as colorless oil.
LCMS (ESI) m/z: [M+H]⁺ = 298.0.
¹H NMR (400MHz, CDCl₃) δ = 7.34 (d, J=2.0 Hz, 1H), 7.23 - 7.20 (m, 1H), 6.35 (d, J=8.4 Hz, 1H), 4.26 - 4.15 (m, 2H), 3.89 (d, J=9.2 Hz, 1H), 3.19 - 3.12 (m, 1H), 2.78 - 2.72 (m, 3H), 1.54 (s, 3H), 1.33 - 1.25 (m, 3H) ppm.

### Step 5. 5-Bromo-1,3-dimethylindoline-3-carboxylic acid

LiOH·H₂O (126.66 mg, 3.02 mmol) was added to a mixture of ethyl 5-bromo-1,3-dimethylindoline-3-carboxylate (300 mg, 1.01 mmol) in MeOH (3 mL) and water (1 mL) at 20 °C. The mixture was stirred at 20°C for 15 hrs. MeOH was removed under vacuum and the solution was diluted with water (1 mL). The pH was adjusted to 3 with 1N HCl and the solution was extracted with EA (5 mL x 3). The combined organic phase was washed with brine (10 mL), dried over Na₂SO₄, filtered, and concentrated to affording the title compound (220 mg, 0.70 mmol) as a white solid.
LCMS (ESI) m/z: [M+H]⁺ = 270.0.

### Step 6. 5-Bromo-1,3-dimethylindoline-3-carboxamide

Ammonium chloride (436 mg, 8.14 mmol), HATU (465 mg, 1.22 mmol), and DIPEA (0.56 mL, 4.07 mmol) were added to a mixture of 5-bromo-1,3-dimethylindoline-3-carboxylic acid (220 mg, 0.81 mmol) in DMF (4 mL) at 20 °C. The mixture was stirred for 15 hrs. Water (5 mL) was added and the mixture was extracted with EA (5 mL x 3). The combined organic layer was washed with brine (10 mL), dried over anhydrous Na₂SO₄, filtered, and concentrated in vacuo. The residue was purified by FCC (Eluent: PE:EA = 1:1) affording the title compound (210 mg, 0.47 mmol) as colorless oil.
LCMS (ESI) m/z: [M+H]⁺ = 269.0.
¹H NMR (400MHz, CDCl₃) δ = 7.29 (br d, J=2.0 Hz, 1H), 7.22 (d, J=2.0 Hz, 1H), 6.44 (d, J=8.4 Hz, 1H), 3.84 (d, J=9.2 Hz, 1H), 3.10 (d, J=9.2 Hz, 1H), 2.76 (s, 3H), 1.57 (s, 3H) ppm.

### Step 7. 5-Bromo-1,3-dimethylindoline-3-carbonitrile

Triethylamine (0.33 mL, 2.34 mmol) was added to a mixture of 5-bromo-1,3-dimethylindoline-3-carboxamide (210 mg, 0.78 mmol) in DCM (3 mL) at 0 °C. TFAA (0.27 mL, 1.95 mmol) was added slowly and the resulting mixture was stirred at 20 °C for 4 hrs. The mixture was diluted with water (5 mL) and extracted with DCM (5 mL x 3). The combined organic phase was washed with saturated NaHCO₃ (aq.), dried over anhydrous Na₂SO₄, filtered, and concentrated in vacuo. The residue was purified by FCC (Eluent: PE:EA = 10:1 to 5:1) affording the title compound (100 mg, 0.39mol) as colorless oil.
LCMS (ESI) m/z: [M+H]⁺ = 251.1.
¹H NMR (400MHz, CDCl₃) δ = 7.33 (d, J=2.0 Hz), 7.31 - 7.28 (m, 1H), 6.42 (d, J=8.4 Hz, 1H), 3.70 (d, J=9.2 Hz, 1H), 3.32 (d, J=9.2 Hz, 1H), 2.77 (s, 3H), 1.68 (s, 3H) ppm.

### Step 8. 3-Cyano-1,3-dimethylindoline-5-carboxylic acid

Palladium acetate (2.2 mg, 0.010 mol), dccp2HBF₄ (12.2 mg, 0.020 mmol), and potassium carbonate (41.3 mg, 0.30 mmol) were added to a solution of 5-bromo-1,3-dimethylindoline-3-carbonitrile (50 mg, 0.20 mmol) in DMSO (1 mL) and water (0.007 mL). The mixture was degassed and flushed with CO (g) three times. The mixture was then stirred under a CO atmosphere (15 psi) at 100 °C for 15 hrs. Water (3 mL) was added to the mixture and the mixture was extracted with EA (3 mL x 3). The combined organic phase was washed with brine (5 mL), dried over Na₂SO₄, filtered, and concentrated in vacuo. The crude product was purified by reversed-phase HPLC. The desired fractions were collected and extracted with DCM (5 mL x 3). The combined organic layer was washed with brine (5 mL), dried over Na₂SO₄, filtered, and concentrated affording a mixture of the title compounds (40 mg) as a white solid. The stereoisomers were separated by SFC condition (column: DAICEL CHIRALPAK IC(250mm*30mm,5µm);mobile phase: [0.1%NH₃₋H₂O IPA]; B%: 20%-20%,5 min;85 minmin) affording intermediate 27 (20 mg, 0.09 mmol) as a white solid and Intermediate 28 (15 mg, 0.07 mmol) as a white solid.
**Intermediate 27:** Chiral SFC: IC-3-IPA (DEA)-5-40-3mL-35T.Icm; RT =1.517 min.
LCMS (ESI) m/z: [M+H]⁺ = 217.1.
¹H NMR (400MHz, DMSO-d6) δ = 12.39 (br s, 1H), 7.85 - 7.76 (m, 2H), 6.66 (d, J=8.4 Hz, 1H), 3.87 (d, J=10.0 Hz, 1H28 3.44 (d, J=10.0 Hz, 1H), 2.83 (s, 3H), 1.68 (s, 3H) ppm.
**Intermediate 28:** Chiral SFC: IC-3-IPA (DEA)-5-40-3mL-35T.Icm; RT =1.608 min.
LCMS (ESI) m/z: [M+H]⁺ = 217.1.
¹HNMR (400MHz, DMSO-d6) δ = 12.39 (br s, 1H), 7.85 - 7.76 (m, 2H), 6.66 (d, J=8.4 Hz, 1H), 3.87 (d, J=10.0 Hz, 1H), 3.44 (d, J=10.0 Hz, 1H), 2.83 (s, 3H), 1.68 (s, 3H) ppm.

### Intermediates 29 and 30: (R)-5-Cyano-5-methyl-6,8-dihydro-5H-pyrano[3,4-b]pyridine-3-carboxylic acid and (S)-5-Cyano-5-methyl-6,8-dihydro-5H-pyrano[3,4-b]pyridine-3-carboxylic acid

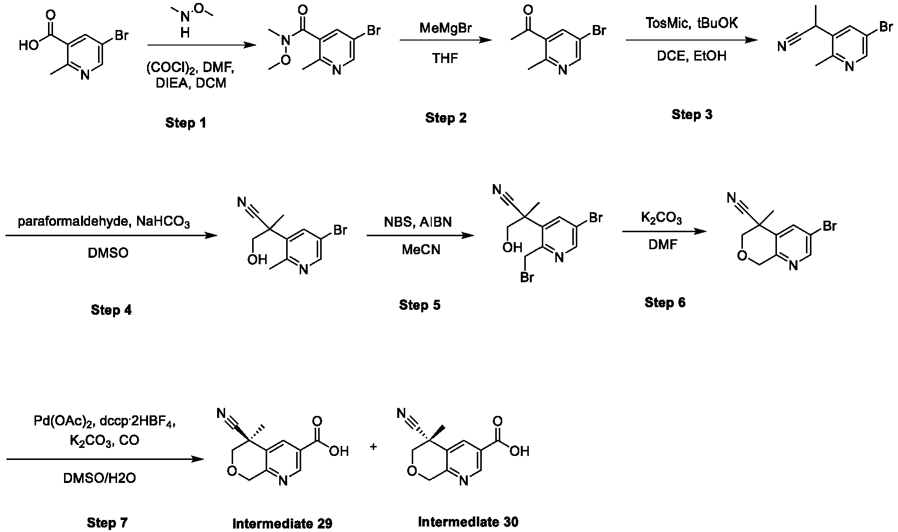

### Step 1. 5-Bromo-N-methoxy-N,2-dimethylnicotinamide

Oxalyl chloride (12.2 mL, 139 mmol) was added to a solution of 5-bromo-2-methyl-pyridine-3-carboxylic acid (10 g, 46.29 mmol) and DMF (0.036 mL, 0.46 mmol) in DCM (100 mL). The reaction mixture was stirred at 25 °C for 1 hr. The reaction mixture was concentrated in vacuo and the residue was dissolved in DCM (100 mL). The organic solution was added to a solution of N-methoxymethanamine (6.77 g, 69.4 mmol, HCl salt) and DIEA (40.3 mL, 231 mmol) in DCM (50 mL). The reaction mixture was stirred at 25 °C for 14 hrs. The reaction mixture was poured into water (500 mL) and extracted with DCM (500 mL x 2). The combined organic layer was dried over anhydrous Na₂SO₄, filtered, and concentrated in vacuo. The residue was purified by FCC (Eluent: PE:EA = 1:0 to 1:1) affording the title compound (11.5 g, 44.4 mmol) as yellow solid.
LCMS (ESI) m/z: [79BrM+H]+ = 259.0.

### Step 2. 1-(5-Bromo-2-methylpyridin-3-yl)ethenone

Methylmagnesium bromide (3 M, 21.2 mL) was added to a solution of 5-bromo-N-methoxy-N,2-dimethylnicotinamide (11 g, 42.4 mmol) in THF (100 mL) at 0 °C. The reaction mixture was stirred at 25 °C for 1 hr. The reaction mixture was poured into saturated aq. NH₄Cl (400 mL) and extracted with EA (400 mL x 2). The combined organic layer was dried over anhydrous Na₂SO₄, filtered, and concentrated in vacuo. The residue was purified by FCC (Eluent: PE:EA = 1:0 to 5:1) affording the title compound (9 g, 41.5 mmol) as yellow oil.
LCMS (ESI) m/z: [79BrM+H]+ = 214.0.
¹H NMR (400 MHz, CDCl₃) δ = 8.66 (d, J = 2.4 Hz, 1H), 8.06 (d, J = 2.0 Hz, 1H), 2.70 (s, 3H), 2.60 (s, 3H) ppm.

### Step 3. 2-(5-Bromo-2-methylpyridin-3-yl)propanenitrile

tBuOK (9.23 g, 82.2 mmol) was added to a solution of 1-(5-bromo-2-methylpyridin-3-yl)ethenone (8 g, 37.4 mmol) and 1-(isocyanomethylsulfonyl)-4-methylbenzene (11.0 g, 56.1 mmol) in DME (400 mL) and EtOH (16 mL) at 0 °C. The reaction mixture was stirred at 25 °C for 14 hrs. The reaction mixture was poured into saturated aq. NH₄Cl (400 mL). The mixture was concentrated in vacuo to remove DME and EtOH. The aqueous phase was extracted with EA (400 mL x 2). The combined organic layer was dried over anhydrous Na₂SO₄, filtered, and concentrated in vacuo. The residue was purified by FCC (Eluent: PE:EA = 1:0 to 5:1) affording the title compound (7 g, 31.1 mmol) as colorless oil.
LCMS (ESI)3m/z: [79BrM+H]+ = 225.1.
¹H NMR (400 MHz, CDCl₃) δ = 8.55 (d, J = 2.4 Hz, 1H), 7.89 (d, J = 2.0 Hz, 1H), 4.03 - 3.98 (m, 1H), 2.57 (s, 3H), 1.66 (d, J = 7.2 Hz, 3H) ppm.

### Step 4. 2-(5-Bromo-2-methylpyridin-3-yl)-3-hydroxy-2-methylpropanenitrile

Paraformaldehyde (1.12 g, 37.3 mmol) was added to a solution of 2-(5-bromo-2-methylpyridin-3-yl)propanenitrile (7 g, 31.1 mmol) and NaHCO₃ (261 mg, 3.11 mmol) in DMSO (60 mL). The reaction mixture was stirred at 25 °C for 14 hrs. The reaction mixture was poured into water (400 mL) and extracted with EA (400 mL x 2). The combined organic layer was washed with brine (300 mL), dried over anhydrous Na₂SO₄, filtered, and concentrated in vacuo. The residue was purified by FCC (Eluent: PE:EA = 1:0 to 1:1) affording the title compound (7.5 g, 29.4 mmol) as white solid.
LCMS (ESI) m/z: [79BrM+H]+ = 255.1.
¹H NMR (400 MHz, CDCl₃) δ = 8.54 (d, J = 2.0 Hz, 1H), 7.79 (d, J = 2.0 Hz, 1H), 4.14 - 4.09 (m, 1H), 3.96 - 3.92 (m, 1H), 2.80 (s, 3H), 2.69 - 2.66 (m, 1H), 1.81 (s, 3H) ppm.

### Step 5. 2-(5-Bromo-2-(bromomethyl)pyridin-3-yl)-3-hydroxy-2-methylpropanenitrile

NBS (1.40 g, 7.84 mmol) and AIBN (64.4 mg, 0.39 mmol) were added to a solution of 2-(5-bromo-2-methylpyridin-3-yl)-3-hydroxy-2-methylpropanenitrile (1 g, 3.92 mmol) in MeCN (10 mL). The reaction mixture was stirred at 80°C for 4 hrs. The reaction mixture was concentrated. The residue was purified by FCC (Eluent: PE:EA = 1:0 to 1:1) affording the title compound (0.25 g, 0.76 mmol) as brown solid.
LCMS (ESI) m/z: [79BrM+H]+ = 335.0.
¹H NMR (400 MHz, CDCl₃) δ = 8.71 (d, J = 2.0 Hz, 1H), 7.89 (d, J = 2.0 Hz, 1H), 5.00 - 4.85 (m, 2H), 4.21 - 4.06 (m, 2H), 2.24 - 2.21 (m, 1H), 1.89 (s, 3H) ppm.

### Step 6. 3-Bromo-5-methyl-6,8-dihydro-5H-pyrano[3,4-b]pyridine-5-carbonitrile

Potassium carbonate (1.61 g, 11.7 mmol) was added to a solution of 2-(5-bromo-2-(bromomethyl)pyridin-3-yl)-3-hydroxy-2-methylpropanenitrile (1.3 g, 3.89 mmol) in DMF (65 mL). The reaction mixture was stirred at 25 °C for 12 hrs. The reaction mixture was poured into water (300 mL) and extracted with EA (300 mL x 2). The combined organic layer was washed with water (300 mL), dried over anhydrous Na₂SO₄, filtered, and concentrated in vacuo. The residue was purified by FCC (Eluent: PE:EA = 1:0 to 1:1) affording the title compound (350 mg, 1.27 mmol) as white solid.
LCMS (ESI) m/z: [79BrM+H]+ = 253.0.
¹HNMR (400 MHz, CDCl₃) δ = 8.59 (d, J = 2.4 Hz, 1H), 7.95 (d, J = 2.4 Hz, 1H), 4.85 - 4.76 (m, 2H), 4.12 - 3.94 (m, 2H), 1.75 (s, 3H) ppm.

### Step 7. (R)-5-Cyano-5-methyl-6,8-dihydro-5H-pyrano[3,4-b]pyridine-3-carboxylic acid and (S)-5-Cyano-5-methyl-6,8-dihydro-5H-pyrano[3,4-b]pyridine-3-carboxylic acid

A mixture of 3-bromo-5-methyl-6,8-dihydro-5H-pyrano[3,4-b]pyridine-5-carbonitrile (270 mg, 1.07 mmol), dccp2HBF₄ (65.3 mg, 0.11 mmol), Pd(OAc)₂ (23.9 mg, 0.11 mmol), H₂O (0.038 mL), and K₂CO₃ (221 mg, 1.60 mmol) in DMSO (3 mL) was degassed and purged with CO (g) three times. The mixture was stirred at 100°C for 16 hrs under a CO atmosphere (15 psi). The reaction mixture was filtered, poured into water (80 mL), and extracted with EA (30 mL x 2). The EA phase was discarded. The aqueous phase was adjusted to pH = 5 with aq. HCl (1 M). The aqueous phase was extracted with EA (30 mL x 3). The combined organic layers were washed with brine (30 mL), dried over anhydrous Na₂SO₄, filtered, and concentrated in vacuo affording a mixture of the title compounds (130 mg, 0.57 mmol) as yellow oil. The stereoisomers were separated by chiral SFC (column: DAICEL CHIRALPAK AD (250mm*30mm,10µm); mobile phase: [0.1% NH₃•H₂O MEOH]; B%: 30% - 30%, 4.2min; 55minmin) affording Intermediate 29 (55 mg, 0.24 mmol) as a yellow solid and Intermediate 30 (50 mg, 0.22 mmol) as a yellow solid.
**Intermediate 29:** Chiral SFC: AD-3-MeOH(DEA)-5-40-3mL-35T.Icm; RT = 1.055min.
LCMS (ESI) m/z: [M+H]+ = 219.0.
¹H NMR (400 MHz, DMSO-d6) δ= 8.99 (d, J = 2.0 Hz, 1H), 8.43 (d, J = 2.0 Hz, 1H), 4.96 - 4.75 (m, 2H), 4.26 (d, J = 11.8 Hz, 1H), 3.92 (d, J = 11.8 Hz, 1H), 1.70 (s, 3H).
**Intermediate 30:** Chiral SFC: AD-3-MeOH(DEA)-5-40-3mL-35T.Icm; RT = 1.666 min.
LCMS (ESI) m/z: [M+H]+ = 219.0.
¹H NMR (400 MHz, DMSO-d6) δ= 8.99 (d, J = 2.0 Hz, 1H), 8.43 (d, J = 2.0 Hz, 1H), 4.96 - 4.75 (m, 2H), 4.26 (d, J = 11.8 Hz, 1H), 3.92 (d, J = 11.8 Hz, 1H), 1.70 (s, 3H).

### Intermediates 31 and 32: (4S)-4-(hydroxymethyl)-4-methyl-isochromane-6-carboxylic acid and (4R)-4-(hydroxymethyl)-4-methyl-isochromane-6-carboxylic acid

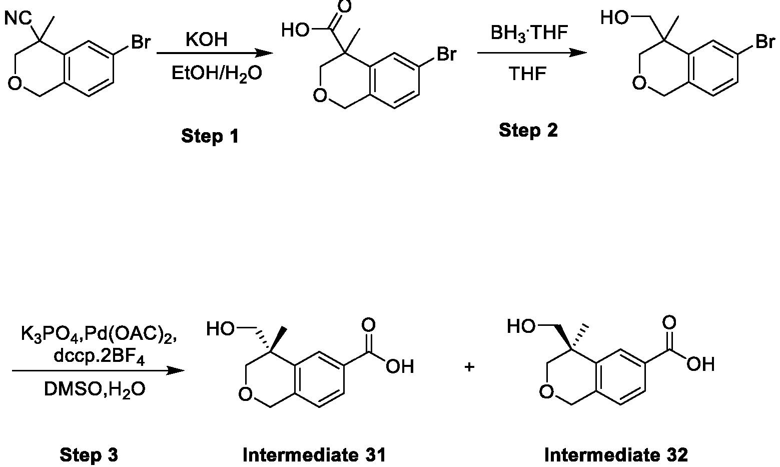

### Step 1. 6-Bromo-4-methyl-isochromane-4-carboxylic acid

Potassium hydroxide (aq., 9M, 5.29 mL) was added to a solution of 6-bromo-4-methyl-isochromane-4-carbonitrile (1.2 g, 4.76 mmol) in EtOH (12 mL). The mixture was stirred at 90°C for 5 hrs. The reaction mixture was diluted with H₂O (20 mL) and extracted with EA (30 mL x 3). The combined organic layers were discarded and the aqueous phase was adjusted pH = 4 with 1N HCl. The aqueous layer was extracted with EA (30 mL x 3). The combined organic layers were washed with brine (60 mL), dried over Na₂SO₄, filtered, and concentrated in vacuo affording the title compound (800 mg, 2.95 mmol) as yellow oil.
¹H NMR (400 MHz, DMSO-d6) δ = 13.11 - 12.45 (m, 1H), 7.52 (d, J = 2.0 Hz, 1H), 7.40 (d, J = 8.4 Hz, 1H), 7.03 (d, J =8.4 Hz, 1H), 4.75 - 4.62 (m, 2H), 4.16 (d, J = 11.2 Hz, 1H), 3.59 (d, J = 11.2 Hz, 1H), 1.41 (s, 3H).

### Step 2. (6-Bromo-4-methyl-isochroman-4-yl)methanol

6-bromo-4-methyl-isochromane-4-carboxylic acid (800 mg, 2.95 mmol) was dissolved in BH₃·THF (1 M, 8.85) at 0 °C and the resulting mixture was stirred at 25 °C for 2 hrs. The reaction mixture was quenched by addition of 1N HCl (25 mL) at 20 °C and stirred for 30 min. Then the mixture was diluted with water (20 mL) and extracted with EA (50 mL x 2). The combined organic layers were washed with brine (50 mL), dried over Na₂SO₄, filtered, and concentrated in vacuo. The residue was purified by FCC (Eluent: DCM:MeOH = 100:1 to 10:1) affording the title compound (400 mg, 1.56 mmol) as colorless oil. ¹H NMR (400 MHz, CDCl₃) δ = 7.47 (d, J = 2.0, 1H), 7.33 (d, J = 8.4 Hz, 1H), 6.89 (d, J = 8.4 Hz,
1H),4.79 - 4.68 (m, 2H), 4.05 (d, J = 11.6 z, 1H), 3.82 - 3.75 (m, 1H), 3.71 - 3.63 (m, 1H), 3.53 (d, J = 11.6 Hz, 1H), 1.21 (s, 3H).

### Step 3. 4-(Hydroxymethyl)-4-methyl-isochromane-6-carboxylic acid

Water (1 mL), K₂CO₃ (185 mg, 1.34 mmol), Pd(OAc)₂ (20.1 mg, 0.089 mmol), and dccp·2HBF₄ (110 mg, 0.18 mmol) were added to a solution of (6-bromo-4-methyl-isochroman-4-yl)methanol (230 mg, 0.89 mmol) in DMSO (5 mL). The reaction mixture was purged with CO (g) three times and the resulting mixture was stirred at 100 °C for 16 hrs under a CO atmosphere (15 psi). The reaction mixture was diluted with H₂O (30 mL) and extracted with EA (50 mL x 3). The combined organic layers were discarded and the aqueous layer was adjusted to pH = 4 with HCl (1 N). The aqueous layer was extracted with EA (30 mL x 3). The combined organic layers were washed with brine (60 mL), dried over Na₂SO₄, filtered, and concentrated in vacuo affording a mixture of the title compounds (150 mg, 0.62 mmol) as a yellow solid. The stereoisomers were separated by chiral SFC (column:DAICEL CHIRALPAK IG (250mm x 30mm,10µm);mobile phase: [0.1%NH₃-H₂O IPA];B%: 25%-25%,6.65min;66min). The mobile phase was concentrated under reduced pressure to remove most of the MeOH. Water (40 mL) was added and each desired compound mixture was lyophilized affording Intermediate 31 (44 mg, 0.20 mmol) as an off-white solid and Intermediate 32 (48 mg, 0.21 mmol) as an off-white solid.

### Intermediate 31:

¹H NMR (400 MHz, DMSO-d6) δ = 13.02 - 12.77 (m, 1H), 7.90 (s, 1H), 7.72 (d, J = 1.2 Hz, 1H), 7.14 (d, J = 8.0 Hz, 1H),4.88 - 4.87 (m, 1H), 4.74 (d, J = 4.0 Hz, 2H), 3.90 (d, J = 11.2 Hz, 1H), 3.58 - 3.50 (m, 1H), 3.42 - 3.35 (m, 2H), 1.14 (s,3H).

### Intermediate 32:

¹H NMR (400 MHz, DMSO-d6) δ = 13.02 - 12.77 (m, 1H), 7.90 (s, 1H), 7.72 (d, J = 1.2 Hz, 1H), 7.14 (d, J = 8.0 Hz, 1H),4.88 - 4.87 (m, 1H), 4.74 (d, J = 4.0 Hz, 2H), 3.90 (d, J = 11.2 Hz, 1H), 3.58 - 3.50 (m, 1H), 3.42 - 3.35 (m, 2H), 1.14 (s,3H).

### Intermediate 33: 2-Methyl-5,6,7,8-tetrahydro-2,6-naphthyridin-1(2H)-one

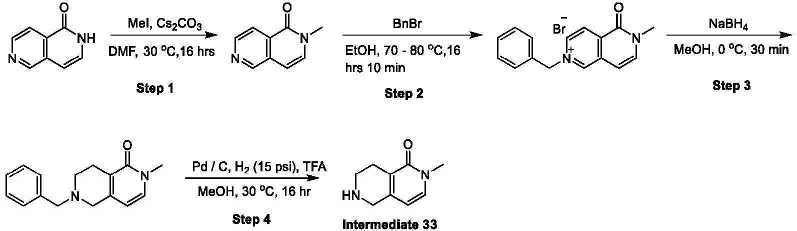

### Step 1: 2-Methyl-2,6-naphthyridin-1(2H)-one

To a mixture of 2,6-naphthyridin-1(2H)-one (200 mg, 1.37 mmol) in DMF (10 mL) was added Cs₂CO₃ (668.82 mg, 2.05 mmol) and Mel (291.36 mg, 2.05 mmol, 127.79 uL) at 30 °C. The mixture was stirred at 30 °C for 16 hrs. The mixture was poured into H₂O (10 mL) and extracted with DCM (10 mL*3). The combined organic layers were dried over anhydrous Na₂SO₄, filtered and concentrated under reduced pressure. The residue was purified by prep-TLC (DCM / MeOH = 20 / 1, Rf = 0.4) to afford the title compound (160 mg, 946.72 umol, 69.18% yield) as a yellow solid.
LCMS (ESI) m/z: [M+H]⁺ = 161.1.
¹H NMR (400 MHz, CDCl₃) δ = 8.98 (s, 1H), 8.69 (d, *J* = 5.2 Hz, 1H), 8.19 (d, *J* = 5.2 Hz, 1H), 7.20 (d, *J* = 7.2 Hz, 1H), 6.58 (d, *J* = 7.2 Hz, 1H), 3.65 (s, 3H) ppm.

### Step 2: 2-Benzyl-6-methyl-5-oxo-5,6-dihydro-2,6-naphthyridin-2-ium bromide

A mixture of 2-methyl-2,6-naphthyridin-1(2*H*)-one (50 mg, 312.16 umol) in EtOH (0.5 mL) was stirred at 70 °C for 10 min. Then benzyl bromide (720.00 mg, 4.21 mmol, 0.5 mL) was added. The mixture was stirred at 80 °C for 16 hrs. The mixture was treated with MTBE (10 mL) and filtered. The filter cake was dried under reduced pressure to afford the title compound (130 mg, crude) as a yellow solid. ¹H NMR (400 MHz, DMSO-d6) δ = 9.87 (s, 1H), 8.98 - 8.97 (m, 1H), 8.68 (d, *J* = 6.4 Hz, 1H), 7.96 (d, *J* = 7.2 Hz, 1H), 7.59 - 7.42 (m, 5H), 6.96 (d, *J* = 7.6 Hz, 1H), 5.96 (s, 2H), 3.60 (s, 3H) ppm.

### Step 3: 6-Benzyl-2-methyl-5,6,7,8-tetrahydro-2,6-naphthyridin-1(2H)-one

To a mixture of 2-benzyl-6-methyl-5-oxo-5,6-dihydro-2,6-naphthyridin-2-ium bromide (70 mg, 211.35 umol) in MeOH (1 mL) was added NaBH₄ (39.98 mg, 1.06 mmol) in several portions at 0 °C. The mixture was stirred at 0 °C for 30 min. The mixture was poured into 1M HCl aqueous solution (5 mL) and basified with sat. NaHCO₃ to pH=8. The mixture was extracted with EA (10 mL *3). The combined organic layers were dried over anhydrous Na₂SO₄, filtered and concentrated under reduced pressure to afford the title compound (35 mg, 137.62 umol, 65.1% yield) as a yellow solid.
LCMS (ESI) m/z: [M+H]⁺ = 255.3.
¹H NMR (400 MHz, CDCl₃) δ = 7.40 - 7.28 (m, 5H), 7.07 (d, *J* = 7.2 Hz, 1H), 5.84 (d, *J* = 6.8 Hz, 1H), 3.69 (s, 2H), 3.52 (s, 3H), 3.39 (s, 2H), 2.79 - 2.64 (m, 4H) ppm.

### Step 4: 2-Methyl-2,6-naphthyridin-1 (2H)-one

To a mixture of 6-benzyl-2-methyl-5,6,7,8-tetrahydro-2,6-naphthyridin-1(2*H*)-one (35 mg, 137.62 umol) in MeOH (1 mL) was added TFA (31.38 mg, 275.24 umol, 20.38 uL) and Pd / C (20 mg, 10% purity). The mixture was purged with H₂ 3 times and stirred at 30 °C for 16 hrs under H₂ (15 psi). The mixture was filtered and the mother liquor was concentrated under reduced pressure to afford the title compound (30 mg, 107.83 umol, 78.4% yield, TFA salt) as a yellow oil.
LCMS (ESI) m/z: [M+H]⁺ = 165.1.

### Intermediate 34: 1-Bromo-4-cyclobutyl-2-methoxybenzene

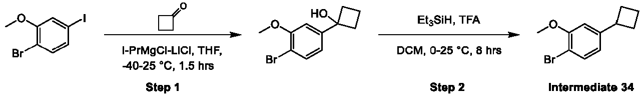

### Step 1: 1-(4-Bromo-3-methoxyphenyl)cyclobutan-1-ol

To a mixture of 1-bromo-4-iodo-2-methoxy-benzene (2.4 g, 7.67 mmol, 5.41 mL) in THF (25 mL) was added i-PrMgCI-LiCl (1.3 M, 6.19 mL) dropwise at -40 °C. The mixture was stirred at -40 °C for 0.5 hr, then cyclobutanone (591.30 mg, 8.44 mmol) was added at -40 °C slowly. The resulting mixture was stirred at 25 °C for 1 hr. The mixture was poured into sat.NH₄Cl (200 mL) and extracted with EA (150 mL * 2). The combined organic layers were washed with brine (250 mL), dried over Na₂SO₄, filtered and concentrated under reduced pressure. The residue was purified by column chromatography (SiO₂, Petroleum ether/Ethyl acetate=20/1 to 1/1) to give the title compound (1.9 g, 6.65 mmol, 86.71% yield ) as a colorless oil.
LCMS (ESI) m/z: [M+H]⁺=211.0.
1H NMR (400 MHz, CDCl₃) δ = 7.52 (d, *J* = 8.0 Hz, 1H), 7.08 (d, *J* = 2.0 Hz, 1H), 6.98-6.96 (m, 1H),4.14-4.12 (m, 1H), 3.93 (s, 3H), 2.61 - 2.48 (m, 2H), 2.43 - 2.30 (m, 2H), 2.12 - 1.97 (m, 3H), 1.80 - 1.68 (m, 1H) ppm

### Step 2: 1-Bromo-4-cyclobutyl-2-methoxybenzene

To a mixture of 1-(4-bromo-3-methoxyphenyl)cyclobutan-1-ol (400 mg, 1.56 mmol) in DCM (20 mL) was added Et₃SiH (1.81 g, 15.56 mmol) and TFA (1.77 g, 15.56 mmol) at 0 °C, then the mixture was stirred at 25 °C for 8 hrs. The mixture was diluted with DCM (150 mL), then washed with sat. NaHCO₃ (100 mL * 2). The organic phase was dried over Na₂SO₄, filtered and concentrated under vacuum. The residue was purified by column chromatography (SiO₂, Petroleum ether) to give the title compound (150 mg, 622.09 umol, 39.99% yield) as a colorless oil.
¹H NMR (400 MHz, CDCl₃) δ = 7.44 (d, *J* = 8.0 Hz, 1H), 6.78 - 6.68 (m, 2H), 3.91 (s, 3H), 3.55 - 3.47 (m, 1H), 2.39 - 2.32 (m, 2H), 2.15 - 2.07(m, 2H), 2.06-1.98 (m, 1H), 1.90 - 1.85 (m, 1H) ppm

### Intermediate 35: 4-Bromo-7-fluoro-1,2-dimethyl-1H-indole

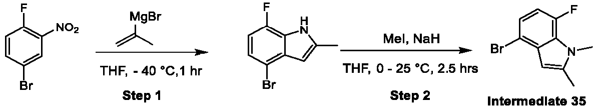

### Step 1: 4-Bromo-7-fluoro-2-methyl-1H-indole

To a mixture of 4-bromo-1-fluoro-2-nitro-benzene (2 g, 9.09 mmol, 1.12 mL) in THF (20 mL) was added bromo(isopropenyl)magnesium (0.5 M, 72.73 mL) dropwisely at - 40 °C under N₂. The mixture was stirred at - 40 °C for 1 hr. The mixture was poured into sat. NH₄Cl (100 mL) and extracted with ethyl acetate (50 mL*2). The combined organic phase was washed with brine (50 mL*1), dried with anhydrous Na₂SO₄, filtered and concentrated under vacuum. The residue was purified by silica gel chromatography (Petroleum ether / Ethyl acetate = 20 / 1) to give the title compound (500 mg, 2.19 mmol, 24.12% yield) as a yellow oil.

### Step 2: 4-Bromo-7-fluoro-1,2-dimethyl-1H-indole

To a solution of 4-bromo-7-fluoro-2-methyl-1H-indole (80 mg, 350.78 umol) in THF (1 mL) was added NaH (28.06 mg, 701.57 umol, 60% purity) at 0 °C and stirred at 25 °C for 0.5 hr. Mel (149.37 mg, 1.05 mmol) was then added to the mixture at 0 °C. The mixture was stirred at 25 °C for 2 hrs. The reaction mixture was diluted with sat. NH₄Cl (10 mL) and extracted with EA (10 mL*2). The combined organic layers were dried over anhydrous Na₂SO₄, filtered and concentrated. The residue was purified by silica gel chromatography (Petroleum ether / Ethyl acetate = 3 / 1) to give the title compound (60 mg, 247.84 umol, 70.65% yield) as a white solid.
¹H NMR (400 MHz, CDCl₃) δ = 7.08 - 7.06 (m 1H), 6.71 - 6.66 (m, 1H), 6.30 - 6.29 (m, 1H), 3.87 (d, *J* = 1.2 Hz, 3H), 2.41(s, 3H) ppm.

### Intermediate 36: 4-Bromo-2-ethyl-1-methyl-1H-indole

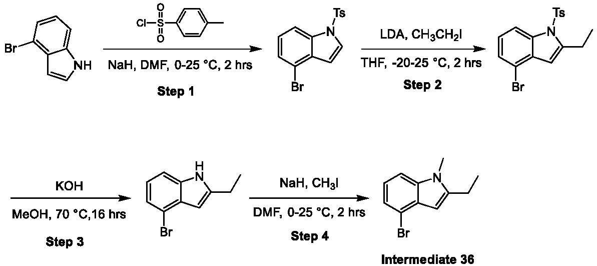

### Step 1: 4-Bromo-1-tosyl-1H-indole

To a solution of 4-bromo-1*H*-indole (1 g, 5.10 mmol, 641.03 uL) in DMF (10 mL) was added NaH (244.82 mg, 6.12 mmol, 60% purity) at 0 °C and the mixture was stirred at 0 °C for 1 hr under N₂ atmosphere. 4-Methylbenzenesulfonyl chloride (1.16 g, 6.08 mmol) was then added and the mixture was stirred at 25 °C for 1 hr. The reaction mixture was poured into NH₄Cl (30 mL) and extracted with EA (40 mL *3). The combined organic layers were washed with brine (30 mL*3), dried over Na₂SO₄, filtered and concentrated under reduced pressure. The residue was purified by column chromatography (SiO₂, Petroleum ether/Ethyl acetate=50/1 to 3/1) to give the title compound (1.6 g, 4.02 mmol, 78.73% yield) as an off-white solid.
¹H NMR (400 MHz, DMSO-d6) δ = 7.99 - 7.93 (m, 2H), 7.89 (d, *J* = 8.4 Hz, 2H), 7.49 (d, *J* = 7.6 Hz, 1H), 7.39 (d, *J* = 8.4 Hz, 2H), 7.29 - 7.27 (m, 1H), 6.77 (d, *J* = 3.6 Hz, 1H), 2.31 (s, 3H) ppm.

### Step 2: 4-Bromo-2-ethyl-1-tosyl-1H-indole

To a solution of 4-bromo-1-tosyl-1H-indole (1.2 g, 3.43 mmol) in THF (12 mL) was added LDA (2 M, 2.06 mL) at -20 °C under N₂, and the mixture was stirred at -20 °C for 30 min under N₂ atmosphere. lodoethane (801.58 mg, 5.14 mmol) was then added and the mixture was stirred at 25 °C for 2 hrs. The reaction mixture was diluted with H₂O (30 mL) and extracted with EA (40 mL * 3). The combined organic layers were washed with brine (30 mL*3), dried over Na₂SO₄, filtered and concentrated under reduced pressure. The residue was purified by column chromatography (SiO₂, Petroleum ether/Ethyl acetate=100/1 to 10/1) to give the title compound (220 mg, 581.58 umol, 16.97% yield) as a white solid. ¹H NMR (400 MHz, CDCl₃) δ = 8.15 (d, *J* = 8.4 Hz, 1H), 7.63 (d, *J* = 8.4 Hz, 2H), 7.38 (d, *J* = 7.6 Hz, 1H), 7.22 (d, *J* = 8.4 Hz, 2H), 7.15 - 7.10 (m, 1H), 6.47 (s, 1H), 3.06 - 3.01 (m, 2H), 2.36 (s, 3H), 1.39 - 1.35 (m, 3H) ppm.

### Step 3: 4-Bromo-2-ethyl-1H-indole

To a solution of 4-bromo-2-ethyl-1-tosyl-1*H*-indole (220.00 mg, 581.58 umol) in MeOH (60 mL) was added KOH (1.63 g, 29.08 mmol). The mixture was stirred at 70 °C for 16 hrs. The reaction mixture was treated with 2 N HCl (20 mL) and was extracted with EA (30 mL *3). The combined organic layers were washed with brine ( 30 mL*3), dried over Na₂SO₄, filtered and concentrated under reduced pressure. The residue was purified by column chromatography (SiO₂, Petroleum ether/Ethyl acetate=100/1 to 10/1) to give the title compound (100 mg, 446.24 umol, 76.73% yield) as a colorless oil. ¹H NMR (400 MHz, CHLOROFORM-d) δ = 8.09 - 7.95 (m, 1H), 7.24 (d, *J* = 8.0 Hz, 2H), 7.03 - 6.92 (m, 1H), 6.32 (s, 1H), 2.84 - 2.79 (m, 2H), 1.40 - 1.36 (m, 3H) ppm.

### Step 4: 4-Bromo-2-ethyl-1-methyl-1H-indole

To a solution of 4-bromo-2-ethyl-1*H*-indole (150 mg, 669.35 umol) in DMF (2 mL) was added NaH (40.16 mg, 1.00 mmol, 60 % purity) at 0 °C under N₂. The resulting mixture was stirred at 0 °C for 15 min under N₂ atmosphere. Iodomethane (114.01 mg, 803.22 umol) was then added and the resulting mixture was stirred at 25 °C for 2 hrs. The reaction mixture was poured into NH₄Cl (30 mL) and extracted with EA (40 mL * 3). The combined organic layers were washed with brine ( 30 mL*3), dried over Na₂SO₄, filtered and concentrated under reduced pressure. The residue was purified by column chromatography (SiO₂, Petroleum ether/Ethyl acetate=100/1 to 10/1) to give the title compound (150 mg, 629.93 umol, 94.11% yield) as colorless oil.

### Intermediate 37: 4-Chloro-2-cyclopropyl-1-methyl-1H-indole

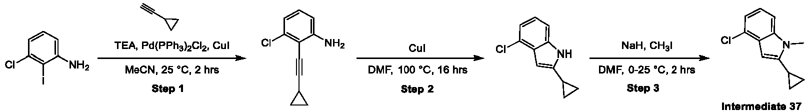

### Step 1: 3-Chloro-2-(cyclopropylethynyl)aniline

To a mixture of 3-chloro-2-iodo-aniline (2 g, 7.89 mmol) and ethynylcyclopropane (521.57 mg, 7.89 mmol) in MeCN (40 mL) was added TEA (7.98 g, 78.91 mmol), Pd(PPh₃)₂Cl₂ (553.84 mg, 789.06 umol) and Cul (150.28 mg, 789.06 umol). The mixture was degassed and purged with N₂ 3 times and the mixture was stirred at 25 °C for 2 hrs under N₂ atmosphere. The reaction mixture was diluted with H₂O (20 mL) and extracted with EA (30 mL * 3). The combined organic layers were washed with brine (30 mL*3), dried over Na₂SO₄, filtered and concentrated under reduced pressure. The residue was purified by column chromatography (SiO₂, Petroleum ether/Ethyl acetate=50/1 to 5/1) to give the title compound (1.3 g, 6.78 mmol, 85.96% yield) as a yellow oil.
¹H NMR (400 MHz, DMSO-d6) δ = 6.97 - 6.95 (m, 1H), 6.59 (d, *J* = 8.0 Hz, 2H), 5.50 (s, 2H), 1.63 - 1.61 (m, 1H), 0.95 - 0.86 (m, 2H), 0.83 - 0.76 (m, 2H) ppm.

### Step 2: 4-Chloro-2-cyclopropyl-1H-indole

To a solution of 3-chloro-2-(cyclopropylethynyl)aniline (1 g, 5.22 mmol) in DMF (10 mL) was added Cul (2.98 g, 15.65 mmol). The mixture was stirred at 100 °C for 16 hrs. The reaction mixture was diluted with H₂O (20 mL) and extracted with EA (30 mL * 3). The combined organic layers were washed with brine (30 mL*3), dried over Na₂SO₄, filtered and concentrated under reduced pressure. The residue was purified by column chromatography (SiO₂, Petroleum ether/Ethyl acetate=100/1 to 10/1) to give the title compound (600 mg, 2.71 mmol, 52.01 % yield) as a yellow oil.
LCMS (ESI) m/z: [M+H]+ = 191.9.
¹H NMR (400 MHz, CHLOROFORM-d) δ = 8.37 - 7.67 (m, 1H), 7.18 (d, *J* = 7.6 Hz, 1H), 7.11 - 6.98 (m, 2H), 6.25 (s, 1H), 2.03 - 1.87 (m, 1H), 1.08 - 0.95 (m, 2H), 0.89 - 0.72 (m, 2H) ppm.

### Step 3: 4-Chloro-2-cyclopropyl-1-methyl-1H-indole

To a solution of 4-chloro-2-cyclopropyl-1*H*-indole (180 mg, 939.18 umol) in DMF (2 mL) was added NaH (56.35 mg, 1.41 mmol, 60% purity) at 0 °C under N₂ and the mixture was stirred at 0 °C for 15 min under N₂ atmosphere. Iodomethane (199.96 mg, 1.41 mmol) was then added and the mixture was stirred at 25 °C for 2 hrs. The reaction mixture was poured into NH₄Cl (30 mL) and extracted with EA (40 mL * 3). The combined organic layers were washed with brine (30 mL*3), dried over Na₂SO₄, filtered and concentrated under reduced pressure. The residue was purified by column chromatography (SiO₂, Petroleum ether/Ethyl acetate=100/1 to 10/1) to give the title compound (190 mg, 864.18 umol, 92.01% yield) as a colorless oil.
LCMS (ESI) m/z: [M+H]+ = 206.0.

### Intermediate 38: 4-Bromo-3-(difluoromethyl)-1-(oxetan-3-yl)-1H-pyrazole

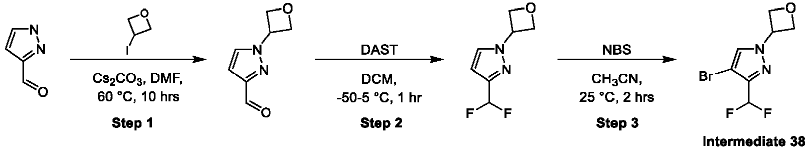

### Step 1: 1-(Oxetan-3-yl)-1H-pyrazole-3-carbaldehyde

To a mixture of 1*H*-pyrazole-3-carbaldehyde (10 g, 104.07 mmol) and Cs₂CO₃ (67.82 g, 208.14 mmol) in DMF (200 mL) was added 3-iodooxetane (28.72 g, 156.11 mmol), then the mixture was stirred at 60 °C for 10 hrs. The reaction mixture was quenched by the addition of water (1000 mL) and the resulting mixture was extracted with EA (400 mL * 3). The combined organic layers were washed with brine (200 mL), dried over Na₂SO₄, filtered and concentrated under reduced pressure. The residue was purified by column chromatography (SiO₂, Petroleum ether/Ethyl acetate=10/1 to 0/1) to give the title compound (3.1 g, 20.37 mmol, 19.58% yield) as a yellow solid.
¹H NMR (400 MHz, CDCl₃) δ = 10.01 (s, 1H), 7.63 - 7.62 (m, 1H), 6.86 (d, *J* = 2.4 Hz, 1H), 5.57 - 5.50 (m, 1H), 5.09 (d, *J* = 6.4 Hz, 4H) ppm.

### Step 2: 3-(Difluoromethyl)-1-(oxetan-3-yl)-1H-pyrazole

To a mixture of 1-(oxetan-3-yl)-1*H*-pyrazole-3-carbaldehyde (600 mg, 3.94 mmol) in DCM (12 mL) was added DAST (1.91 g, 11.83 mmol, 1.56 mL) at -50 °C, then the mixture was stirred at 5 °C for 1 hr. The mixture was poured into sat. NaHCO₃ (100 mL) and extracted with EA (50 mL * 2). The combined organic layers were washed with brine (40 mL), dried over Na₂SO₄, filtered and concentrated under vacuum. The residue was purified by reversed phase HPLC (0.1% FA condition). The product fraction was extracted with EA (150 mL * 2) and the combined organic layers were washed with brine (150 mL), dried over Na₂SO₄, filtered and concentrated under vacuum to give the title compound (170 mg, 898.09 umol, 22.77% yield) as a colorless oil.
LCMS (ESI) m/z: [M+H]⁺ = 175.1

### Step 3: 4-Bromo-3-(difluoromethyl)-1-(oxetan-3-yl)-1H-pyrazole

To a mixture of 3-(difluoromethyl)-1-(oxetan-3-yl)-1*H*-pyrazole (170 mg, 976.18 umol) in MeCN (4 mL) was added NBS (191.12 mg, 1.07 mmol) and the mixture was stirred at 25 °C for 2 hrs. The mixture was concentrated under vacuum and the residue was purified by column chromatography (SiO₂, Petroleum ether/Ethyl acetate=20/1 to 5/1) to give the title compound (200 mg, 735.05 umol, 75.30% yield) as a colorless oil.
LCMS (ESI) m/z: [⁸¹BrM+H]⁺ = 255.0
¹H NMR (400 MHz, CDCl₃) δ= 7.68 (s, 1H), 6.84 - 6.58 (m, 1H), 5.46-5.51 (m, 1H), 5.07 - 4.99 (m, 4H) ppm

### Intermediate 39: 4-Bromo-3-(2,2-difluoroethyl)-1-(oxetan-3-yl)-1H-pyrazole

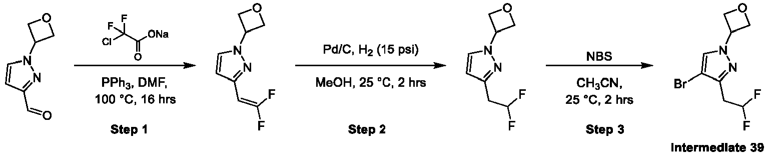

### Step 1: 3-(2,2-Difluorovinyl)-1-(oxetan-3-yl)-1H-pyrazole

To a mixture of 1-(oxetan-3-yl)-1*H*-pyrazole-3-carbaldehyde (1 g, 6.57 mmol) and sodium 2-chloro-2,2-difluoroacetate (1.50 g, 9.86 mmol) in DMF (10 mL) was added PPh₃ (2.59 g, 9.86 mmol) under N₂, then the mixture was stirred at 100 °C for 2 hrs. The reaction mixture was diluted with water (250 mL) and extracted with EA (150 mL * 2). The combined organic layers were washed with brine (150 mL * 2), dried over Na₂SO₄, filtered and concentrated under reduced pressure. The residue was purified by column chromatography (SiO₂, Petroleum ether/Ethyl acetate=50/1 to 3/1) to give the title compound (700 mg, 3.12 mmol, 47.49% yield) as a colorless oil.
LCMS (ESI) m/z: [M+H]⁺ = 187.1

### Step 2: 3-(2,2-Difluoroethyl)-1-(oxetan-3-yl)-1H-pyrazole

A mixture of 3-(2,2-difluorovinyl)-1-(oxetan-3-yl)-1*H*-pyrazole (250 mg, 1.34 mmol) in MeOH (10 mL) and EA (10 mL) was added Pd/C (100 mg, 1.34 mmol, 10% purity) under H₂ (15 psi), then the mixture was stirred at 25 °C for 2 hrs. The mixture was filtered and the filtrate concentrated under vacuum to give the title compound (230 mg, crude) as colorless oil, used directly in the next step.
LCMS (ESI) m/z: [M+H]⁺ = 189.1

### Step 3: 4-Bromo-3-(2,2-difluoroethyl)-1-(oxetan-3-yl)-1H-pyrazole

A mixture of 3-(2,2-difluoroethyl)-1-(oxetan-3-yl)-1*H*-pyrazole (210 mg, 1.12 mmol) and NBS (218.49 mg, 1.23 mmol) in MeCN (4 mL) was stirred at 25 °C for 2 hrs. The mixture was concentrated under vacuum and the residue was purified by column chromatography (SiO2, Petroleum ether/Ethyl acetate=20/1 to 3/1) to give the title compound (150 mg, 544.80 umol, 48.82% yield) as a colorless oil. LCMS (ESI) m/z: [⁸¹BrM+H]⁺ = 269.0
¹H NMR (400 MHz, CDCl₃) δ= 7.61 (s, 1H), 6.280 - 5.97 (m, 1H), 5.40 - 5.36 (m, 1H), 5. 50 - 4.98 (m, 4H), 3.27-3.17 (m, 2H) ppm

### Intermediate 40: 4-Bromo-3-fluoro-1-methyl-1H-indole

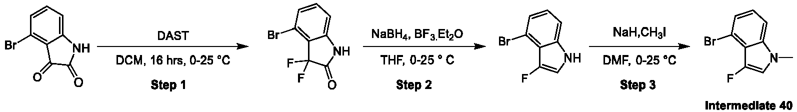

### Step 1: 4-Bromo-3,3-difluoroindolin-2-one

To a solution of 4-bromoindoline-2,3-dione (1 g, 4.42 mmol) in DCM (10 mL) was added DAST (1.78 g, 11.06 mmol) at 0 °C under N₂. The mixture was stirred at 25 °C for 16 hrs. The reaction mixture was poured into sat. NaHCO₃ (30 mL) and extracted with DCM (30 mL * 3). The combined organic layers were washed with brine (30 mL*2), dried over Na₂SO₄, filtered and concentrated under reduced pressure. The residue was purified by column chromatography (SiO₂, Petroleum ether/Ethyl acetate=50/1 to 3/1) to give the title compound (1 g, 4.03 mmol, 91.13% yield) as a brown solid.
¹H NMR (400 MHz, DMSO-d6) δ = 11.41 (d, *J* = 0.8 Hz, 1H), 7.50 - 7.40 (m, 1H), 7.34 (d, *J* = 8.1 Hz, 1H), 6.99 (d, *J* = 7.6 Hz, 1H) ppm.

### Step 2: 4-Bromo-3-fluoro-1H-indole

To a mixture of NaBH₄ (91.52 mg, 2.42 mmol) in THF (2 mL) was added BF₃·Et₂O (343.34 mg, 2.42 mmol) at 0 °C under N₂ and the mixture was stirred at 0 °C for 1 hr. This mixture was added to a solution of 4-bromo-3,3-difluoroindolin-2-one (200 mg, 806.37 umol) in THF (2 mL) at 0 °C under N₂ and the resulting mixture was stirred at 25 °C for 16 hrs. After 16 hrs, to a solution of NaBH₄ (152.54 mg, 4.03 mmol) in THF (2 mL) was added BF₃·Et₂O (572.23 mg, 4.03 mmol) at 0 °C and the mixture was stirred at 0 °C for 1 hr. This mixture was then added to the former reaction mixture at 0 °C. The resulting mixture was stirred at 25 °C for another 4 hrs. The reaction mixture was quenched by addition of 2N HCl, diluted with H₂O (30 mL) and extracted with EA (40 mL *3). The combined organic layers were washed with brine (30 mL), dried over Na₂SO₄, filtered and concentrated under reduced pressure. The residue was purified by column chromatography (SiO₂, Petroleum ether/Ethyl acetate=50/1 to 5/1) to give the title compound (100 mg, 420.07 umol, 52.09% yield) as a colorless oil.
¹H NMR (400 MHz, DMSO-d6) δ = 11.18 (s, 1H), 7.46 - 7.45 (m, 1H), 7.42 - 7.35 (m, 1H), 7.22 (d, *J* = 7.6 Hz, 1H), 7.10 - 7.01 (m, 1H) ppm.

### Step 3: 4-Bromo-3-fluoro-1-methyl-1H-indole

To a solution of 4-bromo-3-fluoro-1H-indole (100 mg, 467.22 umol) in DMF (1 mL) was added NaH (28.03 mg, 700.82 umol, 60% purity) at 0 °C under N₂, and the mixture was stirred at 0 °C for 15 min. lodomethane (79.58 mg, 560.66 umol) was then added and the resulting mixture was stirred at 25 °C for 2 hrs. The reaction mixture was poured into NH₄Cl (30 mL) and extracted with EA (40 mL *3). The combined organic layers were washed with brine (30 mL*3), dried over Na₂SO₄, filtered and concentrated under reduced pressure. The residue was purified by column chromatography (SiO₂, Petroleum ether/Ethyl acetate=100/1 to 10/1) to give the title compound (60 mg, 263.09 umol, 56.31% yield) as a colorless oil.
¹H NMR (400 MHz, DMSO-d6) δ = 7.56 - 7.44 (m, 2H), 7.25 (d, *J* =7.6 Hz, 1H), 7.16 - 7.06 (m, 1H), 3.74 (s, 3H) ppm.

### Intermediate 41: 4-Bromo-2-(difluoromethyl)-1-methyl-1H-indole

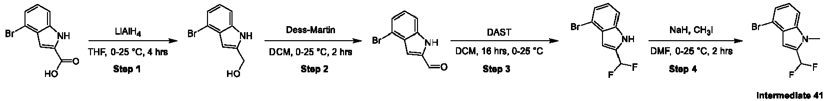

### Step 1: (4-Bromo-1H-indol-2-yl)methanol

To a solution of 4-bromo-1*H*-indole-2-carboxylic acid (2 g, 8.33 mmol) in THF (20 mL) was added LiAlH₄ (948.65 mg, 24.99 mmol) at 0 °C under N₂. The mixture was stirred at 25 °C for 4 hrs. The reaction mixture was quenched by addition of H₂O, the mixture was adjusted to pH=2 with HCl (2 N) and extracted with EA (30 mL * 3). The combined organic layers were washed with brine (60 mL*2), dried over Na₂SO₄, filtered and concentrated under reduced pressure. The residue was purified by column chromatography (SiO₂, DCM/MeOH=100:1-10:1) to give the title compound (1 g, 4.24 mmol, 50.86% yield) as a white solid.
LCMS (ESI) m/z: [M+H]+ = 235.0.
¹H NMR (400 MHz, DMSO-d6) δ= 11.40 (s, 1H), 7.34 (d, *J* = 8.4 Hz, 1H), 7.16 - 7.15 (m, 1H), 6.98 - 6.94 (m, 1H), 6.25 (d, *J* = 1.2 Hz, 1H), 5.34 - 5.31 (m, 1H), 4.61 (d, *J* = 5.6 Hz, 2H) ppm.

### Step 2: 4-Bromo-1H-indole-2-carbaldehyde

To a solution of (4-bromo-1*H*-indol-2-yl)methanol (300 mg, 1.33 mmol) in DCM (3 mL) was added Dess-Martin (675.42 mg, 1.59 mmol) at 0 °C. The mixture was stirred at 25 °C for 2 hrs. The reaction mixture was poured into sat. NaHCO₃ (30mL) and extracted with DCM (30 mL * 3). The combined organic layers were washed with brine (30 mL*2), dried over Na₂SO₄, filtered and concentrated under reduced pressure to give the title compound (297 mg, crude) as a brown solid, which was used directly in the next step.

### Step 3: 4-Bromo-2-(difluoromethyl)-1H-indole

To a solution of 4-bromo-1*H*-indole-2-carbaldehyde (290 mg, 1.29 mmol) in DCM (3 mL) was added DAST (625.90 mg, 3.88 mmol) at 0 °C. The mixture was stirred at 25 °C for 2 hrs. The reaction mixture was poured into sat. NaHCO₃ (30mL) and extracted with DCM (30 mL * 3). The combined organic layers were washed with brine (30 mL*2), dried over Na₂SO₄, filtered and concentrated under reduced pressure. The residue was purified by column chromatography (SiO₂, Petroleum ether/Ethyl acetate=50/1 to 3/1) to give the title compound (110 mg, 408.13 umol, 31.53% yield) as a brown solid.
¹H NMR (400 MHz, CDCl₃) δ= 8.56 (s, 1H), 7.40 - 7.35 (m, 2H), 7.20 - 7.12 (m, 1H), 7.05 - 6.68 (m, 2H) ppm.

### Step 4: 4-Bromo-2-(difluoromethyl)-1-methyl-1H-indole

To a solution of 4-bromo-2-(difluoromethyl)-1*H*-indole (110 mg, 447.06 umol) in DMF (1 mL) was added NaH (26.82 mg, 670.59 umol, 60% purity) at 0 °C. The mixture was degassed and purged with N₂ for 3 times and stirred at 0 °C for 15 min under N₂ atmosphere. lodomethane (69.80 mg, 491.77 umol) was then added and the resulting mixture was stirred at 25 °C for 2 hrs. The reaction mixture was poured into NH₄Cl (30 mL) and extracted with EA (40 mL * 3). The combined organic layers were washed with brine (30 mL*3), dried over Na₂SO₄, filtered and concentrated under reduced pressure. The residue was purified by column chromatography (SiO₂, Petroleum ether/Ethyl acetate=100/1 to 10/1) to give the title compound (110 mg, 422.95 umol, 94.61% yield) as a colorless oil.
LCMS (ESI) m/z: [M+H]+ = 261.9.
¹H NMR (400 MHz, DMSO-d6) δ = 7.63 (d, *J* = 8.4 Hz, 1H), 7.54 - 7.20 (m, 4H), 6.80 (s, 1H), 3.86 (s, 3H) ppm.

### Intermediate 42: 4-Bromo-1-(2,2-difluorocyclopropyl)-3-methoxy-1H-pyrazole

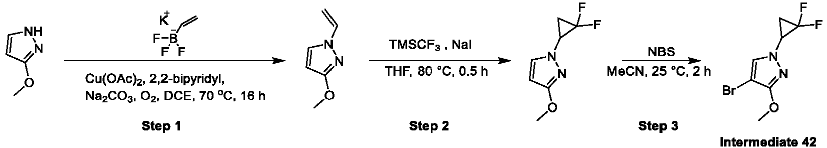

### Step 1: 3-Methoxy-1-vinyl-1H-pyrazole

A mixture of 3-methoxy-1*H*-pyrazole (500 mg, 5.10 mmol), potassium trifluoro(vinyl)borate (1.02 g, 7.65 mmol), Cu(OAc)₂ (1.02 g, 5.61 mmol), Na₂CO₃ (1.19 g, 11.21 mmol) and 2,2'-bipyridyl (875.60 mg, 5.61 mmol) in DCE (10 mL) was degassed and purged with O₂ for 3 times. The mixture was stirred at 70 °C for 16 hrs under O₂ atmosphere. The mixture was poured into water (100 mL) and extracted with EA (50 mL*3). The combined organic layers were washed with brine (30 mL), dried over Na₂SO₄, filtered and concentrated. The residue was purified by flash silica gel chromatography (ISCO^{®}; 25 g SepaFlash^{®} Silica Flash Column, Eluent of 0~20% EA/Petroleum ether gradient @50 mL/min) to give the title compound (400 mg, 3.22 mmol, 63.22% yield) as a yellow oil.
LCMS (ESI) m/z: [M+H]⁺ = 125.0
¹H NMR (400 MHz, CDCl₃-d) δ = 7.33 (d, *J* = 2.4 Hz, 1H), 6.94 - 6.72 (m, 1H), 5.78 (d, *J* = 2.4 Hz, 1H), 5.44 - 5.32 (m, 1H), 4.67 (d, *J* = 8.8 Hz, 1H), 3.93 (s, 3H) ppm.

### Step 2: 1-(2,2-Difluorocyclopropyl)-3-methoxy-1H-pyrazole

To a mixture of 3-methoxy-1-vinyl-1*H*-pyrazole (400 mg, 3.22 mmol) in THF (5 mL) was added Nal (169.04 mg, 1.13 mmol). TMSCF₃ (1.60 g, 11.28 mmol) was then added slowly at 80 °C under N₂. The reaction was stirred at 80 °C for 30 min. The solvent was removed under vacuum and the residue purified by flash silica gel chromatography (ISCO^{®}; 25 g SepaFlash^{®} Silica Flash Column, Eluent of 0~20% EA/Petroleum ether gradient @ 50 mL/min) to give the title compound (200 mg, 1.15 mmol, 35.64% yield) as a yellow oil.
LCMS (ESI) m/z: [M+H]⁺ = 175.0
¹HNMR (400 MHz, CDCl₃-d) δ = 7.32 - 7.13 (m, 1H), 5.62 (d, *J* = 2.4 Hz, 1H), 3.91 - 3.83 (m, 1H), 3.81 (s, 3H), 2.08 - 1.99 (m, 1H), 1.97 - 1.88 (m, 1H) ppm.

### Step 3: 4-Bromo-1-(2,2-difluorocyclopropyl)-3-methoxy-1H-pyrazole

To a mixture of 1-(2,2-difluorocyclopropyl)-3-methoxy-1*H*-pyrazole (100 mg, 574.22 umol) in MeCN (5 mL) was added NBS (112.42 mg, 631.65 umol). The reaction was stirred at 25 °C for 2 hrs. The solvent was removed under vacuum and the residue purified by flash silica gel chromatography (ISCO^{®}; 20 g SepaFlash^{®} Silica Flash Column, Eluent of 0~20% EA/Petroleum ether gradient @ 50mL/min) to give the title compound (120 mg, 474.23 umol, 82.59% yield) as a yellow oil.
LCMS (ESI) m/z: [⁷⁹BrM+H]⁺ = 253.0
¹H NMR (400 MHz, CDCl₃-d) δ = 7.34 (s, 1H), 3.95 (s, 3H), 3.94 - 3.88 (m, 1H), 2.15 - 1.97 (m, 2H) ppm.

### Intermediate 43: 1-(4-Bromo-3-methoxyphenyl)azetidine

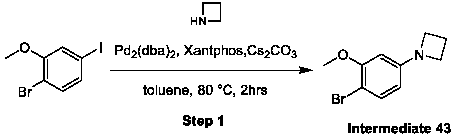

### Step 1: 1-(4-Bromo-3-methoxyphenyl)azetidine

To a mixture of 1-bromo-4-iodo-2-methoxy-benzene (500 mg, 1.60 mmol) and azetidine (91.35 mg, 1.60 mmol) in toluene (5 mL) was added Pd₂(dba)₃ (146.52 mg, 160.00 umol), Xantphos (185.16 mg, 320.00 umol) and Cs₂CO₃ (1.56 g, 4.80 mmol) under N₂. The mixture was stirred at 80 °C for 16 hrs. The reaction mixture was added slowly into water (50 mL) and extracted with EA (50 mL*2). The combined organic phase was washed with brine (100 mL), dried over anhydrous Na₂SO₄, filtered and concentrated under vacuum. The residue was purified by column chromatography (SiO₂, PE/EA=10:1-1:1) to give the title compound (150 mg, 619.55 umol, 38.72% yield) as a yellow solid.
LCMS (ESI) m/z= [M+H]⁺ = 242.0.
¹H NMR (400 MHz, DMSO-d₆) δ = 7.25 (d, *J* = 8.4 Hz, 1H), 6.06 (d, *J* = 2.4 Hz, 1H), 5.93-5.90 (m, 1H), 3.81 - 3.78 (m, 7H), 2.32-2.24 (m, 2H) ppm.

### Intermediate 44: 1-Bromo-4-(tert-butyl)-2-methoxybenzene

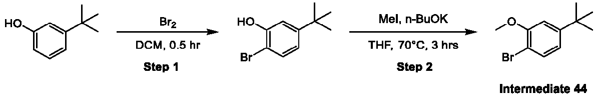

### Step 1: 2-Bromo-5-(tert-butyl)phenol

To a solution of 3-*tert*-butylphenol (5 g, 33.29 mmol) in DCM (50 mL) was added a solution of Br₂ (5.59 g, 34.95 mmol, 1.80 mL) in DCM (25 mL) over 30 mins at 0 °C. The mixture was poured into sat. Na₂SO₃ (200 mL) and extracted with DCM (100 mL * 2). The combined organic layers were dried over Na₂SO₄, filtered and concentrated under vacuum to give the title compound (8 g, crude) as a colorless oil. ¹H NMR (400 MHz, DMSO-d₆) δ = 9.98 (s, 1H), 7.35 (d, J = 8.4 Hz, 1H), 6.97 (d, J = 2.4 Hz, 1H), 6.77-6.74 (m, 1H), 1.22 (s, 9H) ppm.

### Step 2: 1-Bromo-4-(tert-butyl)-2-methoxybenzene

To a solution of 2-bromo-5-(*tert*-butyl)phenol (1 g, 4.36 mmol) in THF (5 mL) was added t-BuOK (494.66 mg, 4.41mmol) followed by Mel (631.90 mg, 4.45 mmol). The mixture was stirred at 70 °C for 3 hrs. The mixture was poured into water (100 mL) and extracted with EA (100 mL * 2). The combined organic layers were washed with brine (100 mL), dried over Na₂SO₄, filtered and concentrated under vacuum. The residue was purified by column chromatography (SiO₂, Petroleum ether/Ethyl acetate=1/0 to 10/1) to give the title compound (750 mg, 3.08 mmol, 70.67% yield) as a white solid.
¹H NMR (400 MHz, DMSO-d₆) δ = 7.45 (d, *J* = 8.4 Hz, 1H), 7.07 - 7.04 (m, 1H), 6.91-6.88 (m, 1H), 3.85 (s, 3H), 1.28 (s, 9H) ppm

### Intermediate 45: 4-Bromo-3-(difluoromethoxy)-1-(oxetan-3-yl)-1H-pyrazole

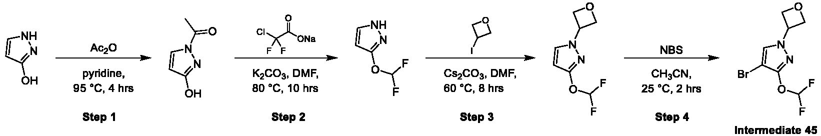

### Step 1: 1-(3-Hydroxy-1H-pyrazol-1-yl)ethan-1-one

To a solution of 1*H*-pyrazol-3-ol (3 g, 35.68 mmol) in pyridine (15 mL) was added a solution of Ac₂O (3.82 g, 37.47 mmol, 3.51 mL) in pyridine (6 mL) at 95 °C over 30 min, then the mixture was stirred at 95°C for 3.5 hrs. The reaction mixture was concentrated under reduced pressure and the residue was triturated in MeOH (20 mL) to give the title compound (3.8 g, 30.13 mmol, 84.45% yield) as a yellow solid. ¹H NMR (400 MHz, DMSO-d₆) δ= 10.97 (s, 1H), 8.12 (d, *J =* 3.6 Hz, 1H), 6.00 (d, *J* = 2.8 Hz, 1H), 2.47 (s, 3H) ppm.

### Step 2: 3-(Difluoromethoxy)-1H-pyrazole

To a mixture of 1-(3-hydroxy-1*H*-pyrazol-1-yl)ethan-1-one (1000 mg, 7.93 mmol) and sodium 2-chloro-2,2-difluoroacetate (1.45 g, 9.52 mmol) in DMF (10 mL) was added K₂CO₃ (3.29 g, 23.79 mmol). The mixture was stirred at 80 °C for 10 hrs. The mixture was poured into sat. NH₄Cl (150 mL) and extracted with EA (100 mL * 3). The combined organic layers were washed with brine (150 mL * 2), dried over Na₂SO₄, filtered and concentrated under reduced pressure. The residue was purified by column chromatography (SiO₂, Petroleum ether/Ethyl acetate=10/1 to 1/1) to give the title compound (270 mg, 2.01 mmol, 25.39% yield) as a colorless oil.
LCMS (ESI) m/z: [M+H]⁺ = 135.1
¹H NMR (400 MHz, DMSO-d₆) δ = 12.49 (s, 1H), 7.69 (s, 1H), 7.41 - 7.04 (m, 1H), 5.97-5.96 (m, 1H) ppm.

### Step 3: 3-(Difluoromethoxy)-1-(oxetan-3-yl)-1H-pyrazole

To a mixture of 3-(difluoromethoxy)-1*H*-pyrazole (270 mg, 2.01 mmol), 3-iodooxetane (555.70 mg, 3.02 mmol) in DMF (5 mL) was added Cs₂CO₃ (1.31 g, 4.03 mmol). The mixture was stirred at 60 °C for 8 hrs. The mixture was poured into water (50 mL) and extracted with EA (50 mL * 2). The combined organic layers were washed with brine (100 mL), dried over Na₂SO₄, filtered and concentrated under reduced pressure. The residue was purified by column chromatography (SiO₂, Petroleum ether/Ethyl acetate=50/1 to 3/1) to give the title compound (270 mg, 1.18 mmol, 58.53% yield) as a colorless oil. LCMS (ESI) m/z: [M+H]⁺ = 427.1
¹H NMR (400 MHz, CDCl₃) δ= 7.41 (d, *J* = 2.4 Hz, 1H), 7.07 - 6.70 (m, 1H), 5.93 (d, *J* = 2.4 Hz, 1H), 5.34 - 5.27 (m, 1H), 5.08 - 4.97 (m, 4H) ppm.

### Step 4: 4-Bromo-3-(difluoromethoxy)-1-(oxetan-3-yl)-1H-pyrazole

To a mixture of 3-(difluoromethoxy)-1-(oxetan-3-yl)-1*H*-pyrazole (270 mg, 1.42 mmol) in MeCN (5 mL) was added NBS (278.00 mg, 1.56 mmol), then the mixture was stirred at 25°C for 2 hrs. The mixture was concentrated under vacuum and the residue was purified by column chromatography (SiO₂, Petroleum ether/Ethyl acetate=10/1 to 1/1) to give the title compound (300 mg, 1.05 mmol, 73.82% yield) as a white solid.
LCMS (ESI) m/z: [⁷⁹BrM+H]⁺ = 269.0
¹H NMR (400 MHz, CDCl₃) δ= 7.49 (s, 1H), 7.16 - 6.80 (m, 1H), 5.29 - 5.26 (m, 1H), 5.00 (d, *J* = 6.0 Hz, 4H) ppm.

### Intermediate 46: Pyrazolo[1,5-a]pyridin-2-yl trifluoromethanesulfonate

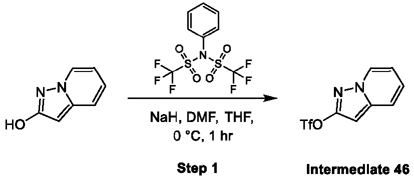

### Step 1: Pyrazolo[1,5-a]pyridin-2-yl trifluoromethanesulfonate

To a solution of pyrazolo[1,5-a]pyridin-2-ol (200 mg, 1.49 mmol) in DMF (1 mL) and THF (1 mL) was added NaH (65.60 mg, 1.64 mmol, 60% purity) at 0 °C under N₂, then 1,1,1-trifluoro-N-phenyl-N-(trifluoromethylsulfonyl)methanesulfonamide (639.21 mg, 1.79 mmol) was added to the reaction mixture at 0 °C. The resulting mixture was stirred at 0 °C for 1 hr under N₂. The reaction mixture was quenched by addition of water (20 mL) and then extracted with EA (20 mL * 3). The combined organic layers were washed with brine (50 mL), dried over Na₂SO₄, filtered and concentrated under reduced pressure. The residue was purified by column chromatography (SiO₂, PE/EA=5:1) to give the title compound (300 mg, 1.06 mmol, 71.05% yield) as a light yellow oil.
LCMS (ESI) m/z: [M+H]⁺ = 267.1

### Intermediate 47: 3-(4-Bromo-3-methoxyphenyl)oxetane

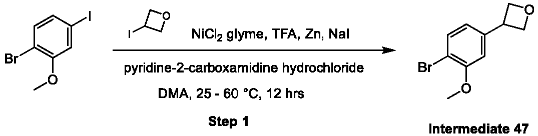

### Step 1: 3-(4-Bromo-3-methoxyphenyl)oxetane

To a mixture of 1-bromo-4-iodo-2-methoxy-benzene (500 mg, 1.60 mmol) and 3-iodooxetane (352.75 mg, 1.92 mmol) in DMA (4 mL) was added nickel(II) chloride ethylene glycol dimethyl ether complex (35.11 mg, 159.78 umol), pyridine-2-carboxamidine hydrochloride (25.18 mg, 159.78 umol), TFA (18.22 mg, 159.78 umol, 11.83 uL), Zn (208.96mg, 3.20 mmol) and Nal (119.75 mg, 798.90 umol) in one portion at 25 °C under N₂. The mixture was stirred at 60 °C for 12 hrs. The solids were removed by filtration and the filtrate concentrated under vacuum. The residue was purified by reversed phase column (0.1 % FA). The product fraction was concentrated to remove MeCN and the aqueous phase was extracted with ethyl acetate (20 mL*2). The combined organic phase was washed with brine (20 mL), dried with anhydrous Na₂SO₄, filtered and concentrated under vacuum to give the title compound (200 mg, 668.21 umol, 41.82% yield) as yellow oil.
LCMS (ESI) m/z: [M+H]⁺ =242.8, 244.9.

### Intermediate 48: 1-(8-Bromo-2,3-dihydro-4H-benzo[b][1,4]oxazin-4-yl)ethan-1-one

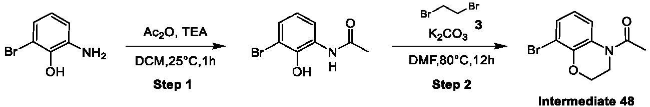

### Step 1: N-(3-Bromo-2-hydroxyphenyl)acetamide

To a solution of 2-amino-6-bromo-phenol (1 g, 5.32 mmol) in DCM (10 mL) was added Ac₂O (651.56 mg, 6.38 mmol, 597.76 uL) and TEA (1.61 g, 15.96 mmol, 2.22 mL) at 0°C under N₂. The mixture was heated to 25°C and stirred for 1 hr. Water was added (10 mL) and the mixture extracted by dichloromethane (50 mL * 3). The combined organic phase was washed with brine (50 mL * 2), dried over anhydrous sodium sulfate, filtered and concentrated under reduced pressure. The residue was purified by column chromatography (SiO₂, Petroleum ether/Ethyl acetate=100/1 to 3/1) to give the title compound (300 mg, 1.11 mmol, 20.84% yield) as a light brown solid.
LCMS (ESI) m/z: [⁷⁹BrM+H]⁺ = 229.9.
¹HNMR (400 MHz, CDCl₃) δ = 7.96 (br s, 1H), 7.90 (s, 1H), 7.59 - 7.53 (m, 1H), 7.32 - 7.28 (m, 1H), 6.80 - 6.72 (m, 1H), 2.24 (s, 3H).

### Step 2: 1-(8-Bromo-2,3-dihydro-4H-benzo[b][1,4]oxazin-4-yl)ethan-1-one

To a solution of N-(3-bromo-2-hydroxyphenyl)acetamide (100 mg, 434.67 umol) in DMF (2 mL) was added K₂CO₃ (180.22 mg, 1.30 mmol) and 1,2-dibromoethane (163.32 mg, 869.35 umol, 65.59 uL) under N₂. The mixture was heated to 80°C and stirred for 12 hrs. The mixture was poured into water (5 mL) and extracted with ethyl acetate (10 mL * 3). The combined organic phase was washed with brine (10 mL * 2), dried over anhydrous sodium sulfate, filtered and concentrated under reduced pressure. The residue was purified by prep-TLC (SiO₂, PE/EA=1:1) to give the title compound (100 mg, 351.43 umol, 26.95% yield) as a white solid.
LCMS (ESI) m/z: [⁷⁹BrM+H]⁺ = 255.9
¹HNMR (400 MHz, DMSO-d₆) δ = 7.77 - 7.67 (m, 1H), 7.33 (d, J = 7.9 Hz, 1H), 6.82 (t, J = 8.0 Hz, 1H), 4.40 - 4.33 (m, 2H), 3.89 (t, J = 4.5 Hz, 2H), 3.09 (s, 1H), 2.25 (s, 3H)

### Intermediate 49: 7-Bromo-5-fluoro-2-methylisoquinolin-1(2H)-one

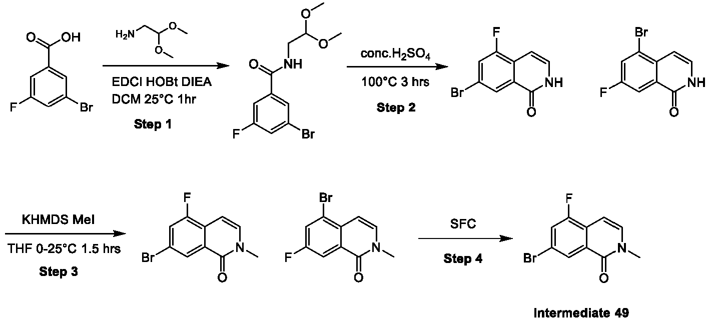

### Step 1: 3-Bromo-N-(2,2-dimethoxyethyl)-5-fluorobenzamide

To a solution of 3-bromo-5-fluoro-benzoic acid (5 g, 22.83 mmol), 2,2-dimethoxyethanamine (2.40 g, 22.83 mmol, 2.49 mL) in DCM (50 mL) was added EDCI (8.75 g, 45.66 mmol), HOBt (6.17 g, 45.66 mmol) and DIEA (7.38 g, 57.08 mmol). The mixture was stirred at 25 °C for 1 hr. The mixture was poured into water (100 mL) and extracted with DCM (30 mL*3). The combined organic layer was dried over Na₂SO₄, filtered and concentrated under vacuum. The residue was purified by column chormatograohy (SiO₂, Petroleum ether/ Ethyl acetate = 10:1) to give the title compound (6.1 g, 19.93 mmol, 87.28% yield) as a white solid.
LCMS (ESI) m/z: [M+H-OMe]⁺ = 275.9.
¹H NMR (400 MHz, MeOD-d4) δ = 7.84 (s, 1H), 7.58 - 7.53 (m, 2H), 4.57 - 4.53 (m, 1H), 3.48 (d, J = 5.6 Hz, 2H), 3.40 (s, 6H) ppm.

### Step 2: 7-Bromo-5-fluoroisoquinolin-1(2H)-one and 5-bromo-7-fluoroisoquinolin-1(2H)-one

A solution of 3-bromo-*N*-(2,2-dimethoxyethyl)-5-fluorobenzamide (5.6 g, 18.29 mmol) in H₂SO₄ (34 mL) was stirred at 100 °C for 3 hrs. The mixture poured into water and the resulting solid was collected by filtration and dried to give a mixture of the title compounds (3.5 g, 14.46 mmol, 79.05% yield) as a white solid.

### Step 3: 7-Bromo-5-fluoro-2-methylisoquinolin-1(2H)-one and 5-bromo-7-fluoro-2-methylisoquinolin-1(2H)-one

To a solution of 7-bromo-5-fluoroisoquinolin-1(2*H*)-one and 5-bromo-7-fluoroisoquinolin-1(2H)-one (1.5 g, 6.20 mmol) in THF (15 mL) was added KHMDS (1 M, 7.44 mL) at 0 °C. The mixture was stirred for 0.5 hr and then iodomethane (3.52 g, 24.79 mmol) was added. The mixture was stirred for 1 hr at 25 °C before pouring into water (5 mL) and extracting with EA (10 mL*3). The organic layer was concentrated, and the residue was purified by column chromatography (SiO₂, Petroleum ether/ Ethyl acetate = 1:1, Rf = 0.3) to give a mixture of the title compounds (750 mg, 2.93 mmol, 47.26% yield) as a yellow solid.
LCMS (ESI) m/z: [M+H]⁺ = 256.1.

### Step 4: 7-Bromo-5-fluoro-2-methylisoquinolin-1(2H)-one

The mixture of 7-bromo-5-fluoro-2-methylisoquinolin-1(2*H*)-one and 5-bromo-7-fluoro-2-methylisoquinolin-1(2*H*)-one (750 mg, 2.93 mmol) was purified by SFC separation: {method column: DAICEL CHIRALPAK AD (250mm*30mm,10um); mobile phase: [0.1% NH₃H₂O MEOH]; B%: 40%-40%,4.25;105min.} to give the title compound (270 mg, 1.05 mmol, 35.95% yield) as a white solid. LCMS (ESI) m/z: [M+H]⁺ = 256.2.
¹H NMR (400 MHz, MeOD-d4) δ = 8.22 (s, 1H), 7.67 - 7.63 (m, 1H), 7.47 (d, J = 7.6 Hz, 1H), 6.72 (d, J = 7.6 Hz, 1H), 3.61 (s, 3H) ppm.

### Intermediate 50: 6-Bromo-7-methoxy-1-methyl-1H-indazole

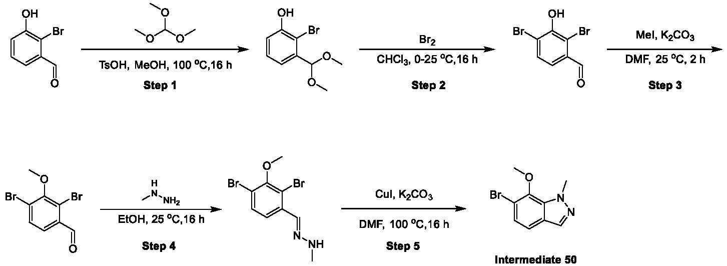

### Step 1: 2-Bromo-3-(dimethoxymethyl)phenol

To a solution of 2-bromo-3-hydroxybenzaldehyde (2 g, 9.95 mmol) and trimethoxymethane (5.28 g, 49.75 mmol, 5.45 mL) in MeOH (30 mL) was added TsOH (171.33 mg, 994.94 umol). The mixture was stirred at 100 °C for 16 hrs. The solvent was removed under vacuum and the residue was purified by flash silica gel chromatography (ISCO^{®}; 40 g SepaFlash^{®} Silica Flash Column, Eluent of 0~60% EA/Petroleum ether gradient @100 mL/min) to give the title compound (1.6 g, 6.48 mmol, 65.08% yield) as a yellow oil.
LCMS (ESI) m/z: [M+H]⁺ = 214.9
¹H NMR (400 MHz, CDCl₃-d) δ = 7.29 - 7.24 (m, 1H), 7.23 - 7.18 (m, 1H), 7.07 - 7.02 (m, 1H), 5.79 (s, 1H), 5.53 (s, 1H), 3.40 (s, 6H) ppm.

### Step 2: 2,4-Dibromo-3-hydroxybenzaldehyde

To a solution of 2-bromo-3-(dimethoxymethyl)phenol (1.4 g, 5.67 mmol) in CHCl₃ (7 mL) was added a solution of Br₂ (1.18 g, 7.37 mmol, 379.72 uL) in CHCl₃ (7 mL) at 0 °C. The reaction was stirred at 25 °C for 16 hrs. The reaction was quenched by aq. Na₂S₂O₃ (80 mL) and extracted with EA (50 mL*3). The combined organic layers were washed with brine (50 mL), dried over Na₂SO₄, filtered and concentrated. The residue was purified by flash silica gel chromatography (ISCO^{®}; 25 g SepaFlash^{®} Silica Flash Column, Eluent of 0~60% EA/Petroleum ether gradient @ 100 mL/min) to give the title compound (1.3 g, 4.64 mmol, 81.97 % yield) as a white solid.
LCMS (ESI) m/z: [Br M+H]⁺ = 280.8.
¹H NMR (400 MHz, DMSO-d₆) δ = 10.44 (s, 1H), 10.17 (d, *J* = 0.8 Hz, 1H), 7.83 - 7.65 (m, 1H), 7.27 (d, *J* = 8.4 Hz, 1H) ppm.

### Step 3: 2,4-Dibromo-3-methoxybenzaldehyde

To a solution of 2,4-dibromo-3-hydroxybenzaldehyde (1.3 g, 4.64 mmol) and K₂CO₃ (1.28 g, 9.29 mmol) in DMF (15 mL) was added Mel (988.80 mg, 6.97 mmol, 433.69 uL). The mixture was stirred at 25 °C for 2 hrs, then poured into water (60 mL) and extracted with EA (30 mL*3). The combined organic layers were washed with brine (30 mL), dried over Na₂SO₄, filtered and concentrated under vacuum. The residue was purified by flash silica gel chromatography (ISCO^{®}; 12 g SepaFlash^{®} Silica Flash Column, Eluent of 0~60% EA/Petroleum ether gradient @ 60 mL/min) to give the title compound (1.2 g, crude) as a white solid.
¹H NMR (400 MHz, DMSO-d₆) δ = 10.17 (s, 1H), 7.89 - 7.82 (m, 1H), 7.53 (d, *J* = 8.4 Hz, 1H), 3.85 (s, 3H) ppm.

### Step 4: (E)-1-(2,4-Dibromo-3-methoxybenzylidene)-2-methylhydrazine

To a solution of 2,4-dibromo-3-methoxybenzaldehyde (500 mg, 1.70 mmol) and methylhydrazine (391.84 mg, 3.40 mmol, 447.82 uL) in EtOH (5 mL) was stirred at 25 °C for 16 hrs. The mixture was poured into water (80 mL) and extracted with EA (30 mL*3). The combined organic layer were washed with brine (30 mL), dried over Na₂SO₄, filtered and concentrated under vacuum. The residue was purified by flash silica gel chromatography (ISCO^{®}; 12 g SepaFlash^{®} Silica Flash Column, Eluent of 0~50% EA/Petroleum ether gradient @ 60 mL/min) to give the title compound (250 mg, 776.41 umol, 45.64% yield) as a yellow oil.
LCMS (ESI) m/z: [Br M+H]⁺ = 322.8.
¹H NMR (400 MHz, CDCl₃-d) δ = 7.72 (s, 1H), 7.58 - 7.51 (m, 1H), 7.48 - 7.41 (m, 1H), 5.90 (br s, 1H), 3.90 (s, 3H), 3.02 (s, 3H) ppm.

### Step 5: 6-Bromo-7-methoxy-1-methyl-1H-indazole

To a solution of (*E*)-1-(2,4-dibromo-3-methoxybenzylidene)-2-methylhydrazine (230 mg, 714.29 umol) in DMF (4 mL) was added K₂CO₃ (296.16 mg, 2.14 mmol) and Cul (13.60 mg, 71.43 umol). The mixture was stirred at 100 °C for 16 hrs. Water (40 mL) was added and the products extracted into EA (20 mL*3). The combined organic layers were washed with brine (20 mL), dried over Na₂SO₄, filtered and concentrated under vacuum. The residue was purified by flash silica gel chromatography (ISCO^{®}; 12 g SepaFlash^{®} Silica Flash Column, Eluent of 0~60% Ethylacetate/Petroleum ethergradient @ 40 mL/min) to give the title compound (120 mg, 497.75 umol, 69.68% yield) as a yellow oil.
LCMS (ESI) m/z: [Br M+H]⁺ = 242.9.
¹H NMR (400 MHz, CDCl₃-d) δ = 7.98 (s, 1H), 7.41 - 7.35 (m, 1H), 7.30 (d, *J* = 10.0 Hz, 1H), 4.33 (s, 3H), 4.05 (s, 3H) ppm.

### Intermediate 51: 2-(3-Methyloxetan-3-yl)pyrimidin-4-yl trifluoromethanesulfonate

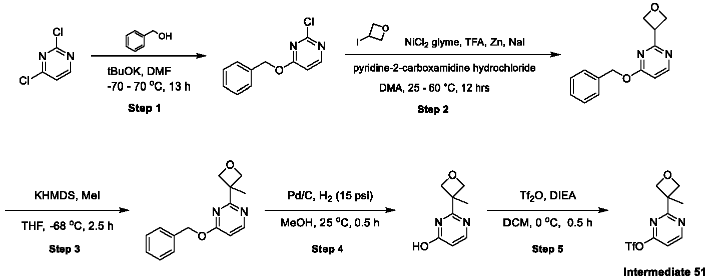

### Step 1: 4-(Benzyloxy)-2-chloropyrimidine

A mixture of phenylmethanol (29.03 g, 268.50 mmol, 27.92 mL) and t-BuOK (15.06 g, 134.25 mmol) in THF (80 mL) was heated to 70 °C and stirred for 0.5 hr. The reaction mixture was cooled to 0 °C and slowly added dropwise to the solution of 2,4-dichloropyrimidine (20 g, 134.25 mmol) in DMF (100 mL), maintaining the temperature below -70 °C. After stirring for 0.5 hr, the reaction was allowed to warm to 25 °C and stirred for 12 hrs. The reaction mixture was added dropwise to cold water (150 mL) and white solid was formed. The solid was collected by filtration and dried to give the title compound (22 g, 84.75 mmol, 63.13% yield) as a white solid.
LCMS (ESI) m/z: [M+H]⁺ = 221.1
¹H NMR (400 MHz, DMSO-d₆) δ = 8.49 (d, *J* = 5.6 Hz, 1H), 7.50 - 7.46 (m, 2H), 7.42 - 7.35 (m, 3H), 7.06 (d, *J* = 5.6 Hz, 1H), 5.41 (s, 2H) ppm.

### Step 2: 4-(Benzyloxy)-2-(oxetan-3-yl)pyrimidine

To a mixture of 4-(benzyloxy)-2-chloropyrimidine (20 g, 90.64 mmol) and 3-iodooxetane (20.01 g, 108.77 mmol) in DMA (400 mL) was added nickel(II) chloride ethylene glycol dimethyl ether complex (1.99 g, 9.06 mmol), pyridine-2-carboxamidine hydrochloride (1.43 g, 9.06 mmol), TFA (1.03 g, 9.06 mmol, 671.08 uL), Zn (11.85 g, 181.28 mmol) and Nal (6.79 g, 45.32 mmol) in one portion at 25 °C. The mixture was stirred at 60 °C for 12 hours under N₂. The reaction mixture was filtered, poured into H₂O (800 mL) and extracted with EA (800 mL*3). The combined organic layer was washed with water (800 mL) and brine (800 mL), dried over Na₂SO₄, filtered and concentrated. The residue was purified by flash silica gel chromatography (Eluent of 0~20% EA/Petroleum ether gradient) to give the title compound (1 g, 2.48 mmol, 2.73% yield) as a yellow oil.
LCMS (ESI) m/z: [M+H]+ = 243.1.
¹H NMR (400 MHz, DMSO-d₆) δ = 8.61 - 8.47 (m, 1H), 7.49 - 7.31 (m, 5H), 7.14 - 6.84 (m, 1H), 5.49 - 5.38 (m, 2H), 4.96 - 4.66 (m, 4H), 4.37 (d, *J* = 8.8 Hz, 1H) ppm.

### Step 3: 4-(Benzyloxy)-2-(3-methyloxetan-3-yl)pyrimidine

To a solution of 4-(benzyloxy)-2-(oxetan-3-yl)pyrimidine (1 g, 4.13 mmol) in THF (4 mL) was added KHMDS (1 M, 6.19 mL) at -68 °C. The mixture was stirred at -68 °C for 0.5 hr. Iodomethane (2.93 g, 20.64 mmol, 1.28 mL) was then added at -68 °C. The mixture was stirred at 25 °C for 2 hrs. The reaction mixture was quenched with aq. NH₄Cl (40 mL) at 0 °C and extracted with EA (40 mL*3). The combined layers were dried over anhydrous Na₂SO₄, filtered and concentrated under reduced pressure. The residue was purified by flash silica gel chromatography (SiO₂, Petroleum ether/Ethyl acetate=1/0 to 1/1) to give the title compound (220 mg, 858.37 umol, 20.80% yield) as a yellow oil.
LCMS (ESI) m/z: [M+H]⁺= 257.0
¹H NMR (400 MHz, DMSO-d₆) δ = 8.53 (d, *J* = 5.6 Hz, 1H), 7.50 - 7.44 (m, 2H), 7.43 - 7.32 (m, 3H), 6.86 (d, *J* = 6.0 Hz, 1H), 5.43 (s, 2H), 4.96 (d, *J* = 5.6 Hz, 2H), 4.48 (d, *J* = 5.6 Hz, 2H), 1.66 (s, 3H) ppm.

### Step 4: 2-(3-Methyloxetan-3-yl)pyrimidin-4-ol

To a solution of 4-(benzyloxy)-2-(3-methyloxetan-3-yl)pyrimidine (200 mg, 780.34 umol) in MeOH (15 mL) was added Pd/C (40 mg, 10% purity). The mixture was degassed and purged with H₂ balloon 3 times. The mixture was stirred at 25 °C for 0.5 hr under H₂ atmosphere (15 psi). The reaction was filtered to remove Pd/C and concentrated under reduced pressure to give the title compound (110 mg, 661.95 umol, 84.83% yield) as a white solid.
LCMS (ESI) m/z: [M+H]⁺= 167.0.
¹H NMR (400 MHz, DMSO-d₆) δ = 7.89 (d, *J* = 6.8 Hz, 1H), 6.19 (d, *J* = 6.8 Hz, 1H), 4.89 (d, *J* = 6.0 Hz, 2H), 4.38 (d, *J* = 6.0 Hz, 2H), 1.61 (s, 3H) ppm.

### Step 5: 2-(3-Methyloxetan-3-yl)pyrimidin-4-yl trifluoromethanesulfonate

To a solution of 2-(3-methyloxetan-3-yl)pyrimidin-4-ol (50 mg, 300.88 umol) and DIEA (116.66 mg, 902.65 umol, 157.22 uL) in DCM (1 mL) was added Tf₂O (101.87 mg, 361.06 umol, 59.57 uL) at 0 °C. The mixture was stirred at 0 °C for 0.5 hr. The reaction mixture was quenched with aq. NaHCO₃ (20 mL) and extracted with DCM (20 mL*3). The combined organic layer was dried over anhydrous Na₂SO₄, filtered and concentrated under reduced pressure to give the title compound (88 mg, 295.07 umol, 98.07% yield) as a yellow oil.
LCMS (ESI) m/z: [M+H]⁺= 299.0.

### Intermediate 52: 4-Chloro-2-(1-fluorocyclopropyl)pyrimidine

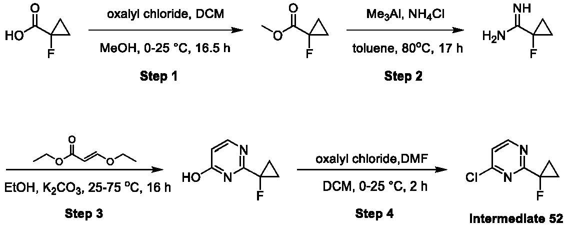

### Step 1: Methyl 1-fluorocyclopropane-1-carboxylate

To a mixture of 1-fluorocyclopropanecarboxylic acid (4 g, 38.43 mmol) in DCM (20 mL) was added oxalyl dichloride (7.32 g, 57.65 mmol, 5.05 mL) at 0 °C. The reaction was stirred at 25 °C for 16 hrs. The mixture was concentrated and then dissolved in DCM (20 mL). MeOH (3.69 g, 115.30 mmol, 4.67 mL) was then added at 0 °C and the mixture was stirred at 25 °C for 30 mins. The reaction was quenched with the addition of aq. NaHCO₃ (50 mL) and the products were extracted with DCM (30 mL*3). The combined organic layers were concentrated at 25 °C to give the title product (2 g, crude) as a yellow oil.
¹H NMR (400 MHz, CDCl₃-d) δ = 3.76 (s, 3H), 1.39 - 1.29 (m, 4H) ppm.

### Step 2: 1-Fluorocyclopropane-1-carboximidamide

To a mixture of NH₄Cl (4.53 g, 84.67 mmol) in toluene (30 mL) was added AlMe₃ (2 M, 42.33 mL) at 0 °C. The mixture was stirred at 25 °C for 1 hr. Methyl 1-fluorocyclopropane-1-carboxylate (2 g, 16.93 mmol) was added into the mixture at 0 °C. The reaction was stirred at 80 °C for 16 hrs. The reaction mixture was cooled to 0 °C, MeOH (50 mL) was added and the mixture stirred for 10 min. The mixture was filtered and the filtrate was concentrated under vacuum to give the title product (2.1 g, crude) as a white solid.

### Step 3: 2-(1-Fluorocyclopropyl)pyrimidin-4-ol

To a mixture of 1-fluorocyclopropane-1-carboximidamide (2.02 g, 19.77 mmol) and K₂CO₃ (5.46 g, 39.54 mmol) in EtOH (30 mL) was added ethyl (*E*)-3-ethoxyprop-2-enoate (0.95 g, 6.59 mmol, 951.90 uL) at 25 °C. The reaction was stirred at 75 °C for 16 hrs. The reaction mixture was filtered and the filtrate was concentrated under vacuum. The residue was purified by flash silica gel chromatography (ISCO^{®}; 40 g SepaFlash^{®} Silica Flash Column, Eluent of 0-20% DCM/MeOH gradient @ 100 mL/min) to give the title compound (140 mg, 908.26 umol, 13.78% yield) as a yellow solid.
LCMS (ESI) m/z: [M+H]⁺ = 155.1
¹H NMR (400 MHz, CDCl₃-d) δ = 11.80 - 10.90 (m, 1H), 7.85 (d, *J* = 6.8 Hz, 1H), 6.33 (d, *J* = 6.8 Hz, 1H), 1.68 - 1.52 (m, 4H) ppm.

### Step 4: 4-Chloro-2-(1-fluorocyclopropyl)pyrimidine

To a mixture of 2-(1-fluorocyclopropyl)pyrimidin-4-ol (70 mg, 454.13 umol) and DMF (3.32 mg, 45.41 umol, 3.49 uL) in DCM (1 mL) was added oxalyl chloride (63.41 mg, 499.54 umol, 43.73 uL) at 0 °C. The reaction was stirred at 25 °C for 2 hrs. The mixture was poured into aq. NaHCO₃ (30 mL) and extracted with EA (20 mL*3). The combined organic layers were washed with brine (20 mL), dried over Na₂SO₄, filtered and concentrated under vacuum. The residue was purified by flash silica gel chromatography (ISCO^{®}; 4 g SepaFlash^{®} Silica Flash Column, Eluent of 0~50%PE/EA gradient @ 36 mL/min) to give the title compound (35 mg, 202.80 umol, 44.66% yield) as a yellow oil.
¹H NMR (400 MHz, CDCl₃-d) δ = 8.58 (d, *J* = 5.6 Hz, 1H), 7.22 (d, *J* = 5.2 Hz, 1H), 1.68 - 1.61 (m, 2H), 1.58 - 1.52 (m, 2H) ppm.

### Intermediate 53: 3-(Dimethylamino)pyridin-2(1H)-one

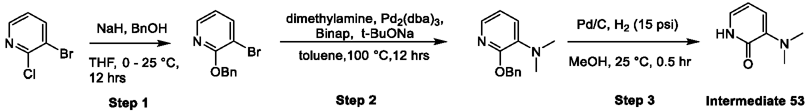

### Step 1: 2-(Benzyloxy)-3-bromopyridine

To a mixture of phenylmethanol (3.37 g, 31.18 mmol, 3.24 mL) in THF (50 mL) was added NaH (1.25 g, 31.18 mmol, 60% purity) in portions at 0 °C under N₂. The mixture was stirred at 0 °C for 30 min, then to the mixture was added 3-bromo-2-chloro-pyridine (5 g, 25.98 mmol) at 0 °C. The mixture was stirred at 25°C for 11.5 hrs. The mixture was poured into water (300 mL) and the products extracted with ethyl acetate (100 mL*2). The combined organic phase was washed with brine (100 mL*1), dried over anhydrous Na₂SO₄, filtered and concentrated under vacuum. The residue was purified by silica gel chromatography (Petroleum ether / Ethyl acetate = 20 / 1) to give the title compound (5.2 g, 19.69 mmol, 75.78% yield) as a light yellow oil.

### Step 2: 2-(Benzyloxy)-N,N-dimethylpyridin-3-amine

To a mixture of 2-(benzyloxy)-3-bromopyridine (500 mg, 1.89 mmol) and dimethylamine hydrochloride (255.62 mg, 5.67 mmol, 287.22 uL) in toluene (10 mL) was added Pd₂(dba)₃ (86.54 mg, 94.50 umol), BINAP (117.68 mg, 189.00 umol) and *t*-BuONa (908.15 mg, 9.45 mmol) in one portion at 25 °C under N₂. The mixture was stirred at 100 °C for12 hrs. The mixture was poured into water (20 mL) and the products extracted with ethyl acetate (10 mL*2). The combined organic phase was washed with brine (10 mL*1), dried with anhydrous Na₂SO₄, filtered and concentrated under vacuum. The residue was purified by silica gel chromatography (Petroleum ether / Ethyl acetate = 15 / 1) to give the title compound (200 mg, 876.08 umol, 46.35% yield) as a yellow oil.

### Step 3: 3-(Dimethylamino)pyridin-2(1H)-one

To a solution of 2-(benzyloxy)-*N,N*-dimethylpyridin-3-amine (100 mg, 438.04 umol) and MeOH (2 mL) was added Pd / C (10 mg, 438.04 umol, 10% purity). The mixture was degassed and purged with H₂ 3 times, and then the mixture was stirred at 25 °C for 0.5 hr under H₂(15 psi). The mixture was filtered and the filtrate was concentrated to give the title compound (50 mg, 361.88 umol, 82.61% yield) as a white solid.
LCMS (ESI) m/z: [M+H]⁺ = 139.0

### Intermediate 54: 1-Cyclopropylimidazolidin-2-one

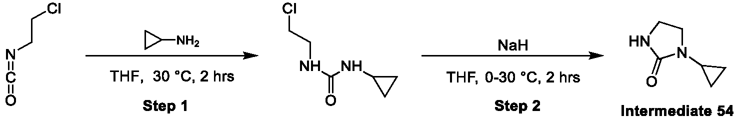

### Step 1: 1-(2-Chloroethyl)-3-cyclopropylurea

To a mixture of cyclopropanamine (541.06 mg, 9.48 mmol) in THF (10 mL) was added dropwise 1-chloro-2-isocyanato-ethane (1 g, 9.48 mmol), then the mixture was stirred at 30 °C for 2 hrs. The reaction was concentrated under vacuum to give the title compound (1.52 g, 9.35 mmol, 98.63% yield) as white solid, which was used directly in the next step.
¹HNMR (400 MHz, CDCl₃) δ = 6.30 (s, 1H), 6.13 (s, 1H), 3.61 - 3.53 (m, 2H), 3.33 - 3.28 (m, 2H), 2.43 - 2.35(m, 1H), 0.61 - 0.53 (m, 2H), 0.37 - 0.29 (m, 2H) ppm.

### Step 2: 1-Cyclopropylimidazolidin-2-one

To a mixture of 1-(2-chloroethyl)-3-cyclopropylurea (1.5 g, 9.22 mmol) in THF (50 mL) was added portionwise NaH (442.76 mg, 11.07 mmol, 60% purity) at 0 °C, and then the mixture was stirred at 30 °C for 2 hrs. The reaction was quenched by adding sat. NH₄Cl (30 mL) and the products were extracted with DCM (30 mL * 5). The combined organic layers were dried over Na₂SO₄, filtered and concentrated under vacuum. The residue was triturated with PE (10 mL) at 20 and the solid was collected by filtered and dried to give the title compound (0.8 g, 6.34 mmol, 68.75% yield) as a gray solid.
LCMS (ESI) m/z: [M+H]⁺ = 127.1.
¹H NMR (400 MHz, DMSO-d₆) δ = 6.28 (s, 1H), 3.30 - 3.23 (m, 2H), 3.20 - 3.12 (m, 2H), 2.36 - 2.27 (m, 1H), 0.58 - 0.51 (m, 4H) ppm.

### Intermediate 55: 2-Cyclopropyl-1,2,5-thiadiazolidine 1,1-dioxide

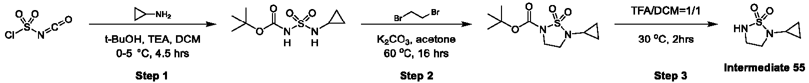

### Step 1: tert-Butyl (N-cyclopropylsulfamoyl)carbamate

To a solution of sulfurisocyanatidic chloride (5 g, 35.33 mmol, 3.07 mL) in DCM (60 mL) was added 2-methylpropan-2-ol (2.62 g, 35.33 mmol, 3.38 mL) at 0 °C. After stirring for 90 min, the resulting solution and TEA (10.91 g, 107.77 mmol) in DCM (60 mL) were added dropwise to a solution of cyclopropanamine (2.02 g, 35.33 mmol, 2.45 mL) in DCM (75 mL) and TEA (6.54 g, 64.66 mmol). The reaction mixture was stirred at 5 °C for 3 hrs then poured into water 200 mL and extracted with DCM (200 mL * 3). The combined organic layer was washed with brine (500 mL), dried over Na₂SO₄, filtered and concentrated. The residue was triturated with PE (100 mL), and the solid was filtered and dried under vacuum to give the title compound (2.5 g, 10.58 mmol, 29.95% yield) as a white solid.
¹H NMR (400 MHz, CDCl₃) δ = 7.17 (br s, 1H), 5.47 (s, 1H), 2.46 - 2.43 (m, 1H), 1.52 (s, 9H), 0.79 - 0.72 (m, 4H) ppm

### Step 2: tert-Butyl 5-cyclopropyl-1,2,5-thiadiazolidine-2-carboxylate 1,1-dioxide

To a solution of *tert*-butyl (*N*-cyclopropylsulfamoyl)carbamate (200 mg, 846.42 umol) and 1,2-dibromoethane (182.20 mg, 969.84 umol) in acetone (3 mL) was added K₂CO₃ (346.34 mg, 2.51 mmol). The resulting mixture was stirred at 60 °C for 16 hrs. The reaction mixture was poured into water (20 mL) and the products were extracted with EA (20 mL * 3). The combined organic layer was washed with brine (50 mL), dried over Na₂SO₄, filtered and concentrated to give the title compound (120 mg, 457.45 umol, 54.04% yield) as a white solid.
¹H NMR (400 MHz, CDCl₃) δ = 3.77 - 3.73 (m, 2H), 3.41 - 3.38 (m, 2H), 2.37 - 2.34 (m, 1H), 1.54 (s, 9H), 0.89 - 0.84 (m, 2H), 0.77 - 0.77 (m, 2H) ppm.

### Step 3: 2-Cyclopropyl-1,2,5-thiadiazolidine 1,1-dioxide

To a solution of *tert*-butyl 5-cyclopropyl-1,2,5-thiadiazolidine-2-carboxylate 1,1-dioxide (120 mg, 457.45 umol) in DCM (1.5 mL) was added TFA (1.5 mL). The mixture was stirred at 30 °C for 2 hrs then concentrated under vacuum to give the title compound (74 mg, 456.20 umol, 99.73% yield) as a yellow solid
¹H NMR (400 MHz, CDCl₃) δ = 3.52 - 3.45 (m, 4H), 2.35 - 2.28 (m, 1H), 0.82 - 0.79 (m, 2H), 0.74 - 0.72 (m, 2H) ppm.

### Intermediate 56: 3-(Difluoromethyl)-4,5,6,7-tetrahydropyrazolo[1,5-a]pyrazine hydrochloride

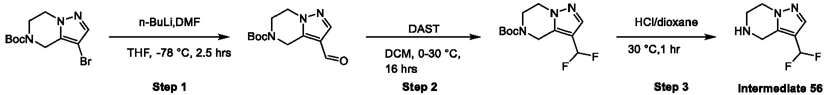

### Step 1: tert-Butyl 3-formyl-6,7-dihydropyrazolo[1,5-a]pyrazine-5(4H)-carboxylate

To a solution of *tert*-butyl 3-bromo-6,7-dihydro-4*H*-pyrazolo[1,5-*a*]pyrazine-5-carboxylate (3000 mg, 9.93 mmol) in THF (30 mL) was added n-BuLi (2.5 M, 8.34 mL) under N₂ at -78 °C and stirred at -78 °C for 0.5 hr. DMF (798.27 mg, 10.92 mmol, 840.28 uL) was then added and the resulting mixture was stirred at -78 °C for another 2 hrs. The reaction mixture was quenched with water (200 mL) and the products were extracted with EA (200 mL * 2). The combined organic layers were washed with brine (200 mL), dried over Na₂SO₄, filtered and concentrated under reduced pressure. The residue was purified by column chromatography (SiO₂, PE/EA=1:1) to give the title compound (400 mg, 1.45 mmol, 14.59% yield) as a light yellow oil.
LCMS (ESI) m/z: [M+H]⁺ = 252.2.
¹H NMR (400 MHz, CDCl₃) δ = 9.88 (s, 1H), 7.95 (s, 1H), 4.92 (s, 2H), 4.23 (t, J = 5.2 Hz, 2H), 3.93 (t, J = 5.2 Hz, 2H), 1.51 (s, 9H) ppm

### Step 2: tert-Butyl 3-(difluoromethyl)-6,7-dihydropyrazolo[1,5-a]pyrazine-5(4H)-carboxylate

To a solution of *tert*-butyl 3-formyl-6,7-dihydropyrazolo[1,5-*a*]pyrazine-5(4*H*)-carboxylate (400 mg, 1.59 mmol) in DCM (8 mL) was added DAST (1.28 g, 7.96 mmol, 1.05 mL) at 0 °C, and the mixture was stirred at 30 °C for 4 hrs. Additional DAST (2.57 g, 15.92 mmol, 2.10 mL) was added to the mixture and stirring continued at 30 °C for 12 hr. The mixture was quenched with sat. NaHCO₃ (200 mL) and extracted with EA (200 mL *2). The combined organic layers were washed with brine 200 (mL), dried over Na₂SO₄, filtered and concentrated under reduced pressure. The residue was purified by column chromatography (SiO₂, PE/EA=2:1) to give the title compound (250 mg, 850.78 umol, 53.45% yield) as a light yellow oil.
LCMS (ESI) m/z: [M+H]⁺ = 274.0.
¹H NMR (400 MHz, CHLOROFORM-d) δ = 7.60 (s, 1H), 6.88 - 6.51 (m, 1H), 4.76 (br s, 2H), 4.21 (br t, *J* = 5.2 Hz, 2H), 3.91 (br t, *J* = 5.2 Hz, 2H), 1.51 (s, 9H) ppm.

### Step 3: 3-(Difluoromethyl)-4,5,6,7-tetrahydropyrazolo[1,5-a]pyrazine hydrochloride

A solution of *tert*-butyl 3-(difluoromethyl)-6,7-dihydropyrazolo[1,5-*a*]pyrazine-5(4*H*)-carboxylate (100 mg, 365.93 umol) in 4M HCl/dioxane (2 mL) was stirred at 30 °C for 1 hr. The reaction mixture was concentrated under reduced pressure to give the title compound (120 mg, crude) as a white solid.

### Intermediates 57 and 58: (R)-5-Fluoro-5-methyl-5,8-dihydro-6H-pyrano[3,4-b]pyridine-3-carboxylic acid and (S)-5-fluoro-5-methyl-5,8-dihydro-6H-pyrano[3,4-b]pyridine-3-carboxylic acid

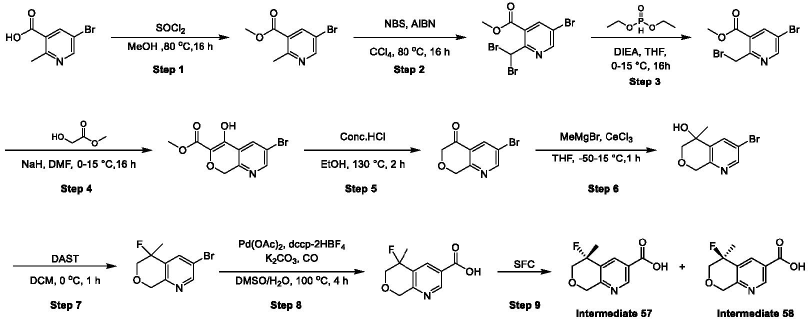

### Step 1: Methyl 5-bromo-2-methylnicotinate

To a solution of 5-bromo-2-methylnicotinic acid (10 g, 46.29 mmol, 1 *eq*) in MeOH (100 mL) was added SOCl₂ (8.26 g, 69.43 mmol, 5.04 mL). The mixture was stirred at 80 °C for 16 hrs. Room temperature was attained and the solvent removed under vacuum. The residue was taken up in aq .NaHCO₃ (200 mL) and extracted with EA (100 mL*3). The combined organic layers were washed with brine (100 mL), dried over Na₂SO₄, filtered and concentrated to give the title compound (8.8 g, 38.25 mmol, 82.63% yield) as a brown solid.
LCMS (ESI) m/z: [Br M+H]⁺ = 229.9
¹H NMR (400 MHz, CDCl₃-d) δ = 8.66 (d, *J* = 2.4 Hz, 1H), 8.32 (d, *J* = 2.4 Hz, 1H), 3.93 (s, 3H), 2.78 (s, 3H) ppm.

### Step 2: Methyl 5-bromo-2-(dibromomethyl)nicotinate

To a solution of methyl 5-bromo-2-methylnicotinate (5.6 g, 24.34 mmol) in CCl₄ (60 mL) was added NBS (4.77 g, 26.78 mmol) and AIBN (399.71 mg, 2.43 mmol). The mixture was stirred at 80 °C for 16 hrs. The reaction mixture was filtered and concentrated under reduced pressure to give the title compound (10 g, crude) as yellow oil.

### Step 3: Methyl 5-bromo-2-(bromomethyl)nicotinate

To a solution of methyl 5-bromo-2-(dibromomethyl)nicotinate (11 g, 28.36 mmol) and DIEA (1.47 g, 11.34 mmol, 1.98 mL) in THF (100 mL) was added diethyl phosphonate (1.57 g, 11.34 mmol, 1.46 mL) at 0°C. The mixture was stirred at 15 °C for 16 hrs. The mixture was filtered, poured into water (300 mL) and extracted with EA (150 mL*3). The combined organic layers were washed with brine (100 mL), dried over Na₂SO₄, filtered and concentrated. The residue was purified by flash silica gel chromatography (ISCO^{®}; 120 g SepaFlash^{®} Silica Flash Column, Eluent of 0~50% EA/Petroleum ether gradient @ 120 mL/min) to give the title compound (7.4 g, 23.95 mmol, 84.46% yield) as a yellow oil.
LCMS (ESI) m/z: [Br M+H]⁺ = 309.7

### Step 4: Methyl 3-bromo-5-hydroxy-8H-pyrano[3,4-b]pyridine-6-carboxylate

To a solution of methyl 5-bromo-2-(bromomethyl)nicotinate (9.9 g, 32.04 mmol) and methyl 2-hydroxyacetate (5.77 g, 64.09 mmol, 4.93 mL) in DMF (100 mL) was added NaH (2.56 g, 64.09 mmol, 60% purity) at 0 °C. The mixture was stirred at 15 °C for 16 hrs. The mixture was poured into aq. NaHCO₃ (600 mL) and extracted with EA (200 mL*3). The combined organic layers were washed with brine (200 mL), dried over Na₂SO₄, filtered and concentrated to give the title compound (7.6 g, crude) as a white solid.

### Step 5: 3-Bromo-6H-pyrano[3,4-b]pyridin-5(8H)-one

To a solution of methyl 3-bromo-5-hydroxy-8*H*-pyrano[3,4-*b*]pyridine-6-carboxylate (7.6 g, 26.57 mmol) in EtOH (80 mL) was added HCl (12 M, 152.00 mL). The mixture was stirred at 130 °C for 2 hrs. The mixture was adjusted to PH=8 with aq. NaHCO₃ and the products were extracted with EA (300 mL*3). The combined organic layers were washed with brine (300 mL), dried over Na₂SO₄, filtered and concentrated. The residue was purified by flash silica gel chromatography (ISCO^{®}; 80 g SepaFlash^{®} Silica Flash Column, Eluent of 0~60% EA/Petroleum ether gradient @60 mL/min) to give the title compound (3.3 g, 14.47 mmol, 54.47% yield) as a yellow solid.

### Step 6: 3-Bromo-5-methyl-5,8-dihydro-6H-pyrano[3,4-b]pyridin-5-ol

To a solution of 3-bromo-6*H*-pyrano[3,4-*b*]pyridin-5(8*H*)-one (3.3 g, 14.47 mmol) and CeCl₃ (1.78 g, 7.24 mmol) in THF (60 mL) was added MeMgBr (3 M, 14.47 mL) at -50 °C under N₂. The mixture was stirred at 15 °C for 1 hr. Water (150 mL) was added, the mixture was filtered and the products were extracted with EA (80 mL*3). The combined organic layers were washed with brine (80 mL), dried over Na₂SO₄, filtered and concentrated. The residue was purified by flash silica gel chromatography (ISCO^{®}; 40 g SepaFlash^{®} Silica Flash Column, Eluent of 0~60% EA/Petroleum ether gradient @80 mL/min) to give the title compound (2.9 g, 11.88 mmol, 82.10% yield) as a white solid.

### Step 7: 3-Bromo-5-fluoro-5-methyl-5,8-dihydro-6H-pyrano[3,4-b]pyridine

To a solution of 3-bromo-5-methyl-5,8-dihydro-6H-pyrano[3,4-b]pyridin-5-ol (2.9 g, 11.88 mmol in DCM (30 mL) was added DAST (2.11 g, 13.07 mmol, 1.73 mL) at 0 °C. The reaction was stirred at 0 °C for 1 hr. The mixture was poured into aq. NaHCO₃ (100 mL) and extracted with DCM (50 mL*3). The combined organic layers were washed with brine (50 mL), dried over Na₂SO₄, filtered and concentrated. The residue was purified by flash silica gel chromatography (ISCO^{®}; 40 g SepaFlash^{®} Silica Flash Column, Eluent of 0~60% EA/Petroleum ether gradient @100 mL/min) to give the title compound (2.4 g, 9.75 mmol, 82.09% yield) as a yellow oil.

### Step 8: 5-Fluoro-5-methyl-5,8-dihydro-6H-pyrano[3,4-b]pyridine-3-carboxylic acid

A mixture of 3-bromo-5-fluoro-5-methyl-5, 8-dihydro-6*H*-pyrano[3,4-*b*]pyridine (2.4 g, 9.75 mmol), 1,3-bis(dicyclohexylphosphino)propane bis(tetrafluoroborate) (597.14 mg, 975.31 umol), Pd(OAc)₂ (218.97 mg, 975.31 umol), H₂O (351.50 mg, 19.51 mmol, 351.50 uL) and K₂CO₃ (2.02 g, 14.63 mmol) in DMSO (40 mL) was degassed and purged with CO 3 times. The mixture was stirred at 100 °C for 4 hrs under CO atmosphere (15Psi). The mixture was filtered, poured into water (100 mL) and extracted with EA (60 mL*2). The EA layer was discarded. The aqueous phase was adjusted to PH=5 by aq. HCl (1 M) and extracted with EA (60 mL*5). The combined organic layers were washed with brined (60 mL*2), dried over Na₂SO₄, filtered and concentrated under vacuum to give the title compound (1.6 g, 7.27 mmol, 74.57% yield) as a yellow solid.
LCMS (ESI) m/z: [M+H]⁺ = 212.0
¹H NMR (400 MHz, DMSO-d₆) δ = 14.29 - 12.75 (m, 1H), 9.01 (s, 1H), 8.45 (s, 1H), 4.93 - 4.69 (m, 2H), 4.24 - 4.03 (m, 1H), 3.97 - 3.79 (m, 1H), 1.76 - 1.57 (m, 3H) ppm.

### Step 9: (R)-5-Fluoro-5-methyl-5,8-dihydro-6H-pyrano[3,4-b]pyridine-3-carboxylic acid and (S)-5-fluoro-5-methyl-5,8-dihydro-6H-pyrano[3,4-b]pyridine-3-carboxylic acid

5-Fluoro-5-methyl-5,8-dihydro-6*H*-pyrano[3,4-b]pyridine-3-carboxylic acid (1.6 g, 7.58 mmol) was separated by SFC (column: DAICEL CHIRALPAK IG (250mm*30mm,10um); mobile phase: [0.1%NH₃H₂O MeOH];B%: 30%-30%,4 min;150 minmin) and SFC (column: DAICEL CHIRALPAK IG (250mm*30mm,10um);mobile phase: [0.1%NH₃H₂O EtOH];B%: 20%-20%,3.8 min;80 minmin). The product fractions were concentrated under vacuum, adjusted to PH=5 by aq. HCl (1 M) and extracted with EA (50 mL*6). The combined organic layers were dried over Na₂SO₄, filtered and concentrated to give Intermediate 50 (610 mg, 2.88 mmol, 37.96% yield) as a white solid and Intermediate 51 (400 mg, 1.83 mmol, 62.21% yield) as a white solid. Stereochemistry was assigned arbitrarily.

### (R)-5-Fluoro-5-methyl-5,8-dihydro-6H-pyrano[3,4-b]pyridine-3-carboxylic acid

LCMS (ESI) m/z: [M+H]⁺ = 212.0
¹H NMR (400 MHz, DMSO-d₆) δ = 14.20 - 12.71 (m, 1H), 9.01 (s, 1H), 8.45 (s, 1H), 4.87 - 4.71 (m, 2H), 4.16 - 4.05 (m, 1H), 3.97 - 3.82 (m, 1H), 1.75 - 1.63 (m, 3H) ppm.
Chiral SFC: IG-3_5CM_MEOH(DEA)_5_40_3ML_T35.M; Rt = 1.251 mins, ee % = 100 %.

### (S)-5-Fluoro-5-methyl-5,8-dihydro-6H-pyrano[3,4-b]pyridine-3-carboxylic acid

LCMS (ESI) m/z: [M+H]⁺ = 212.0
¹H NMR (400 MHz, DMSO-d₆) δ = 13.97 - 13.03 (m, 1H), 9.04 - 8.98 (m, 1H), 8.51 - 8.38 (m, 1H), 4.87 - 4.71 (m, 2H), 4.16 - 4.04 (m, 1H), 3.98 - 3.81 (m, 1H), 1.74 - 1.61 (m, 3H) ppm.
Chiral SFC: IG-3_5CM_MEOH(DEA)_5_40_3ML_T35.M; Rt = 1.597 mins, ee % = 100 %.

### Intermediates 59 and 60: (R)-4-Cyano-8-fluoro-4-methylisochromane-6-carboxylic acid and (S)-4-cyano-8-fluoro-4-methylisochromane-6-carboxylic acid

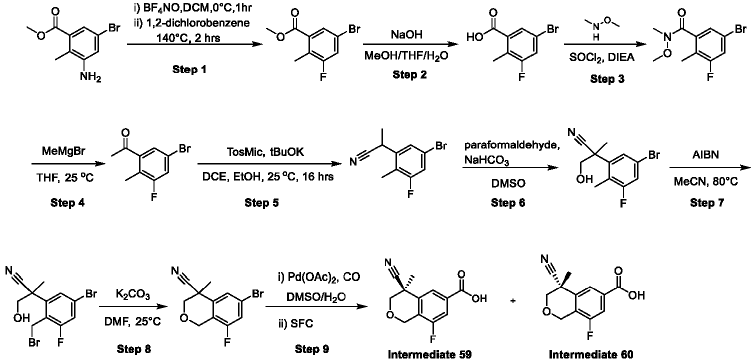

### Step 1: Methyl 5-bromo-3-fluoro-2-methylbenzoate

To a mixture of methyl 3-amino-5-bromo-2-methyl-benzoate (50 g, 204.85 mmol) in DCM (500 mL) was added nitrosonium tetrafluoroborate (31.11 g, 266.30 mmol) portionwise at 0°C under N₂. The mixture was stirred at 0 °C for 1 hr. o-Xylene (869.90 g, 8.19 mol, 990.78 mL) was then added and the mixture slowly heated to 130 °C before stirring at 130 °C for 2 hrs. The reaction mixture was poured into water (200 mL) and extracted with EA (100 mL*3). The combined organic layers were washed with brine (200 mL), dried over anhydrous Na₂SO₄, filtered and concentrated under reduced pressure. The residue was purified by column chromatography (SiO₂, PE/EA = 50/1 to 10/1) to give the title compound (49 g, 198.33 mmol, 96.82% yield) as a yellow oil.
¹H NMR (400 MHz, CHLOROFORM -d) δ = 7.75 (s, 1H), 7.29-7.26 (m, 1H), 3.83 (s, 3H), 2.37 (d, *J* = 2.4 Hz, 3H) ppm.

### Step 2: 5-Bromo-3-fluoro-2-methylbenzoic acid

To a mixture of methyl 5-bromo-3-fluoro-2-methylbenzoate (10 g, 40.48 mmol) in MeOH (10 mL), THF (10 mL) and H₂O (5 mL) was added LiOH·H₂O (5.10 g, 121.43 mmol). The mixture was stirred at 25 °C for 1 hr. The mixture was poured into water (80 mL) and adjusted to pH = 4 by 1N HCl. The mixture was then extracted with EA (30.0 mL*3). The combined organics were washed with brine (80 mL), dried over Na₂SO₄, filtered and concentrated to give the title compound (8.5 g, 36.48 mmol, 90.12% yield) as a white solid.
¹H NMR (400 MHz, DMSO-d₆) δ = 13.54 - 13.49 (m, 1H), 7.76 - 7.73 (m, 2H), 2.36 (d, *J* = 2.4 Hz, 3H) ppm.

### Step 3: 5-Bromo-3-fluoro-N-methoxy-N,2-dimethylbenzamide

To a mixture of 5-bromo-3-fluoro-2-methylbenzoic acid (14.7 g, 63.08 mmol) and DMF (46.11 mg, 630.81 umol) in DCM (150 mL) was added (COCl)₂ (24.02 g, 189.24 mmol). The mixture was stirred at 25 °C for 1 hr and then concentrated under vacuum. The residue was dissolved in DCM (150 mL) and the resulting soltion was added to a mixture of *N*-methoxymethanamine (9.23 g, 94.62 mmol, 1.5 eq) and DIEA (32.61 g, 252.32 mmol, 43.95 mL) in DCM (50 mL). The reaction was stirred at 25 °C for 16 hrs. The reaction mixture was poured into water (400 mL) and extracted with DCM (300 mL*2). The combined organic layer was washed with brine (400 mL), dried over anhydrous Na₂SO₄, filtered and concentrated under vacuum. The residue was purified by column chromatography (SiO₂, PE:EtOAc=100:1-8:1) to give the title compound (11.5 g, 41.65 mmol, 66.03% yield) as a white solid.
¹H NMR (400 MHz, DMSO-d₆) δ = 7.58-7.55 (m, 1H), 7.40 (s, 1H), 3.43 (br s, 3H), 3.29 (br d, *J* = 8.8 Hz, 3H), 2.09 (d, *J* = 2.0 Hz, 3H) ppm.

### Step 4: 1-(5-Bromo-3-fluoro-2-methylphenyl)ethan-1-one

To a solution of 5-bromo-3-fluoro-*N*-methoxy-N,2-dimethylbenzamide (11 g, 39.84 mmol) in THF (100 mL) was added MeMgBr (3 M, 19.92 mL) at 0 °C. The reaction mixture was stirred at 25 °C for 1 hr. The reaction mixture was poured into sat. NH₄Cl (300 mL) and extracted with EA (80 mL*3). The combined organic layer was washed with brine (200 mL), dried over anhydrous Na₂SO₄, filtered and concentrated under vacuum. The residue was purified by column chromatography (SiO₂, PE:EtOAc = 100:1-5:1) to give the title compound (9.1 g, 39.38 mmol, 98.85% yield) as a white solid.
¹H NMR (400 MHz, DMSO-d₆) δ = 7.81 (s, 1H), 7.71-7.68 (m, 1H), 2.57 (s, 3H), 2.23 (d, *J* = 2.0 Hz, 3H) ppm.

### Step 5: 2-(5-Bromo-3-fluoro-2-methylphenyl)propanenitrile

To a mixture of 1-(5-bromo-3-fluoro-2-methylphenyl)ethan-1-one (10 g, 43.28 mmol) and p-toluenesulfonylmethyl isocyanide (12.67 g, 64.92 mmol) in DME (500 mL) and EtOH (15.95 g, 346.23 mmol) was added t-BuOK (10.68 g, 95.21 mmol) at 0 °C. The resulting mixture was stirred at 25 °C for 12 hrs. The reaction mixture was poured into sat. NH₄Cl (400 mL) and extracted with EtOAc (200 mL*2). The combined organic phase was washed with brine (300 mL), dried over anhydrous Na₂SO₄, filtered and concentrated under vacuum. The residue was purified by column chromatography (SiO₂, PE:EtOAc=30:1-5:1) to give the title compound (7.7 g, 31.81 mmol, 73.49% yield) as a white solid.
¹H NMR (400 MHz, DMSO-d₆) δ = 7.54-7.51 (m, 1H), 7.45 (s, 1H), 4.53-4.47 (m, 1H), 2.20 (d, *J* = 2.4 Hz, 3H), 1.54 (d, *J* = 7.2 Hz, 3H) ppm.

### Step 6: 2-(5-Bromo-3-fluoro-2-methylphenyl)-3-hydroxy-2-methylpropanenitrile

To a mixture of 2-(5-bromo-3-fluoro-2-methylphenyl)propanenitrile (7.7 g, 31.81 mmol) and NaHCO₃ (267.20 mg, 3.18 mmol) in DMSO (60 mL) was added paraformaldehyde (1.15 g, 38.17 mmol). The mixture was stirred at 25 °C for 16 hrs. Water (400 mL) was added and the mixture was extracted with EtOAc (200 mL*2). The combined organic phase was washed with brine (400 mL*2), dried over anhydrous Na₂SO₄, filtered and concentrated under vacuum. The residue was purified by column chromatography (SiO₂, PE;EtOAc=10:1-3:1) to give the title compound (8.0 g, 29.40 mmol, 92.43% yield) as a white solid.
¹H NMR (400 MHz, DMSO-d₆) δ = 7.58-7.56 (m, 1H), 7.36 (s, 1H), 5.84-5.81 m, 1H), 3.95-3.91 (m, 1H), 3.69-3.66 (m, 1H), 2.40 (d, *J* = 40 Hz, 3H), 1.70 (s, 3H) ppm.

### Step 7: 2-(5-Bromo-2-(bromomethyl)-3-fluorophenyl)-3-hydroxy-2-methylpropanenitrile

To a mixture of 2-(5-bromo-3-fluoro-2-methylphenyl)-3-hydroxy-2-methylpropanenitrile (8 g, 29.40 mmol) in MeCN (80 mL) was added NBS (10.99 g, 61.74 mmol) and AIBN (1.45 g, 8.82 mmol). The mixture was stirred at 80 °C for 2 hrs. The solvent was removed under reduced pressure and the residue purified by column chromatography (SiO₂, PE:EtOAc=10:1-3:1) to give the title compound (8.5 g, 24.22 mmol, 82.37% yield) as a colorless oil.
¹H NMR (400 MHz, DMSO-d₆) δ = 7.74-7.71 (m, 1H), 7.44 (s, 1H), 5.93 (br s, 1H), 4.97 - 4.86 (m, 2H), 3.95-3.91 (m, 1H), 3.77-3.73 (m, 1H), 1.76 (s, 3H) ppm.

### Step 8: 6-Bromo-8-fluoro-4-methylisochromane-4-carbonitrile

To a mixture of 2-(5-bromo-2-(bromomethyl)-3-fluorophenyl)-3-hydroxy-2-methylpropanenitrile (6.3 g, 17.95 mmol) in DMF (320 mL) was added K₂CO₃ (7.44 g, 53.84 mmol). The mixture was stirred at 25 °C for 16 hrs. Water (3000 mL) was added and the mixture was extracted with EtOAc (500 mL*2). The combined organic phase was washed with brine (1000 mL*2), dried over anhydrous Na₂SO₄, filtered and concentrated under vacuum. The residue was purified by column chromatography (SiO₂, PE;EtOAc = 10:1-3:1) to give the title compound (2.5 g, 9.26 mmol, 51.57% yield) as a white solid.
¹H NMR (400 MHz, DMSO-d₆) δ = 7.75 (s, 1H), 7.61-7.59 (m, 1H), 4.87 - 4.71 (m, 2H), 4.20 (d, *J* = 11.2 Hz, 1H),3.81 (d, *J* = 11.6 Hz, 1H), 1.67 (s, 3H) ppm.

### Step 9: (R)-4-Cyano-8-fluoro-4-methylisochromane-6-carboxylic acid and (S)-4-cyano-8-fluoro-4-methylisochromane-6-carboxylic acid

A mixture of 6-bromo-8-fluoro-4-methylisochromane-4-carbonitrile (2.1 g, 7.77 mmol), K₂CO₃ (1.61 g, 11.66 mmol), Pd(OAc)₂ (174.55 mg, 777.50 umol) and 1,3-bis(dicyclohexylphosphino)propane bis(tetrafluoroborate) (952.06 mg, 1.55 mmol) in DMSO (40 mL) and H₂O (4 mL) was degassed and purged with CO 3 times. The mixture was stirred under CO (15 psi) atmosphere at 100 °C for 4 hrs. The mixture was poured into water (300 mL) and extracted with EA (50.0 mL*2). The combined organics were discarded, the aqueous was adjusted to pH = 5 by aq. HCl (1M), and the products were extracted with EA (50.0 mL*3). The combined organics were washed with brine (1000 mL*2), dried over Na₂SO₄, filtered and evaporated to dryness to give racemic 4-cyano-8-fluoro-4-methylisochromane-6-carboxylic acid. The racemate was separated by SFC (column: DAICEL CHIRALPAK IC (250mm*30mm,5um);mobile phase: [0.1% NH₃H₂O EtOH];B%: 20%-20%,3.1 min;600 minmin). The eluent of peak 1 (Rt=1.120 min) was concentrated under vacuum. The residue was diluted with H₂O (50 mL) and adjusted to pH = 5 with 1N HCl, then the mixture was extracted with EA (50 mL*2). The combined organic layers were dried over anhydrous Na₂SO₄, filtered and concentrated to give the title compound (570 mg, 2.30 mmol, 29.58% yield) as a yellow solid. The eluent of peak 2 (Rt=1.258min) was concentrated under vacuum. The residue was diluted with H₂O (50 mL) and adjusted to pH = 5 with 1N HCl, then the mixture was extracted with EA (50 mL*2). The combined organic layers were dried over anhydrous Na₂SO₄, filtered and concentrated to give a yellow solid. This product was further purified by SFC separation (column: DAICEL CHIRALPAK IC (250mm*30mm,5um);mobile phase: [0.1%NH₃H₂O EtOH];B%: 20%-20%,3.2min;73min) to give the title compound (590 mg, 2.48 mmol, 31.84% yield) as a yellow solid. Stereochemistry was assigned arbitrarily.

### (R)-4-Cyano-8-fluoro-4-methylisochromane-6-carboxylic acid

¹H NMR (400 MHz, DMSO-d₆) δ = 13.84 - 13.85 (m, 1H), 7.95 (s, 1H), 7.66-7.64 (m, 1H), 4.97 - 4.84 (m, 2H), 4.21 (d, *J* = 11.6 Hz, 1H), 3.90 (d, *J* = 11.6 Hz, 1H), 1.69 (s, 3H) ppm.
Chiral SFC: IC-3-EtOH(DEA)-5-40-3mL-35T.Icm, Rt= 1.120 min, ee% =95.99%.

### (S)-4-Cyano-8-fluoro-4-methylisochromane-6-carboxylic acid

¹H NMR (400 MHz, DMSO-d₆) δ = 13.58 - 13.50 (m, 1H), 7.96 (s, 1H), 7.67-7.64 (m, 1H), 4.97 - 4.84 (m, 2H), 4.21 (d, *J* = 11.6 Hz, 1H), 3.90 (d, *J* = 11.6 Hz, 1H), 1.69 (s, 3H) ppm.
Chiral SFC: IC-3-EtOH(DEA)-5-40-3mL-35T.Icm, Rt= 1.159 min, ee% =99.13%.

### Intermediate 61: 3-Bromo-6H-pyrano[3,4-b]pyridin-5(8H)-one-6,6-d₂

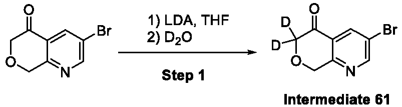

### Step 1: 3-Bromo-6H-pyrano[3,4-b]pyridin-5(8H)-one-6,6-d₂

To a solution of 3-bromo-6H-pyrano[3,4-b]pyridin-5(8H)-one (3.00 g, 13.155 mmol, 1.00 equiv) in THF (250.00 mL) was added NaHDMS (52 mL, 1M, 52.620 mmol, 4.00 equiv) under nitrogen at 0 °C. The resulting mixture was stirred at 0 °C for 2 hours. D₂O (100.00 mL) was then added to the reaction mixture and stirring at room temperature continued for 16 hours. The aqueous layer was extracted with EtOAc (50 mL x 3). The organic layer was dried over Na₂SO₄, filtered and evaporated to afford crude product. The crude product was purified by flash silica chromatography, elution gradient 0 to 50% EtOAc in petroleum ether. Pure fractions were evaporated to dryness to afford the title compound (1.1g, 36.35%) as a yellow solid.
LCMS (ESI) m/z: [M+H]⁺ = 230.

### Intermediate 62: 3-Chloro-6H-pyrano[3,4-b]pyridin-5(8H)-one-8,8-d₂

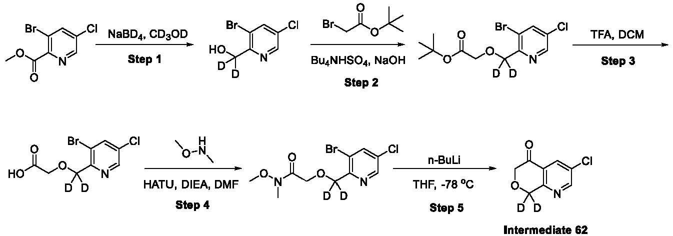

### Step 1: (3-Bromo-5-chloropyridin-2-yl)methan-d₂-ol

To a stirred solution of methyl 3-bromo-5-chloropyridine-2-carboxylate (1.00 g, 3.992 mmol, 1.00 equiv) in CD₃OD (1000 mL) was added NaBD₄ (670.71 mg, 15.969 mmol, 4.00 equiv) at 0 °C under N₂ atmosphere. The resulting mixture was stirred for 2 h at room temperature under N₂ atmosphere. The reaction was quenched with water at room temperature. The aqueous mixture was extracted with EtOAc (3x100 mL). The combined organic extracts were concentrated under vacuum. The residue was purified by silica gel column chromatography, eluting with PE/EtOAc (12:1) to give the title compound (644 mg, 71.86%) as a light yellow solid.
(LCMS (ESI) m/z [M+H]⁺ =224.

### Step 2: tert-Butyl 2-((3-bromo-5-chloropyridin-2-yl)methoxy-d₂)acetate

To a stirred solution of (3-bromo-5-chloropyridin-2-yl)methan-*d*₂-ol (5.10 g, 22.719 mmol, 1.00 equiv) and tert-butyl 2-bromoacetate(8.86 g, 45.438 mmol, 2.00 equiv) in toluene (30.00 mL) were added Bu₄NHSO₄ (0.77 g, 2.272 mmol, 0.10 equiv) and 10 M NaOH (30 mL) at 0 °C. The resulting mixture was stirred overnight at room temperature. The reaction was quenched by the addition of sat. NH₄Cl (10 mL) at room temperature. The aqueous layer was extracted with EtOAc (3x10 mL). The combined organic extracts were concentrated under vacuum. The residue was purified by silica gel column chromatography, eluting with PE/EtOAc (12:1) to give the title compound (6.5g, crude) as a light yellow oil.
(LCMS (ESI) m/z [M+H]⁺ =338.

### Step 3: 2-((3-Bromo-5-chloropyridin-2-yl)methoxy-d₂)acetic acid

To a stirred solution of *tert*-butyl 2-((3-bromo-5-chloropyridin-2-yl)methoxy-*d*₂)acetate (6.50 g, 19.196 mmol, 1.00 equiv) in DCM (40.00 mL) was added TFA (10.00 mL) at room temperature. The resulting mixture was stirred for 1 h at room temperature. The resulting mixture was concentrated under reduced pressure to give the title compound (6.4g, crude) as a white solid.
(LCMS (ESI) m/z [M+H]⁺ =282.

### Step 4: 2-((3-Bromo-5-chloropyridin-2-yl)methoxy-d₂)-N-methoxy-N-methylacetamide

To a stirred solution of 2-((3-bromo-5-chloropyridin-2-yl)methoxy-*d*₂)acetic acid (6.40 g, 22.654 mmol, 1.00 equiv) and *N,O*-dimethylhydroxylamine (1.66 g, 27.185 mmol, 1.20 equiv) in DMF (60.00 mL) were added HATU (10.34 g, 27.185 mmol, 1.20 equiv) and DIEA (8.78 g, 67.962 mmol, 3.00 equiv) at room temperature. The resulting mixture was stirred for 2 h at room temperature. Water was added and the aqueous mixture was extracted with EtOAc. The combined organic extracts were concentrated under reduced pressure. The residue was purified by prep-HPLC to give the title compound (4.45g, 60.33%) as a light yellow oil.
(LCMS (ESI) m/z [M+H]⁺ =325.

### Step 5: 3-Chloro-6H-pyrano[3,4-b]pyridin-5(8H)-one-8,8-d₂

To a stirred solution of 2-((3-bromo-5-chloropyridin-2-yl)methoxy-*d*₂)-*N*-methoxy-*N-*methylacetamide (3 g, 9.214 mmol, 1.00 equiv) in THF (40.00 mL) was added nBuLi (4.42 mL, 11.057 mmol, 1.20 equiv) at -78 °C under nitrogen atmosphere. The resulting mixture was stirred for 2 h at -78 °C under nitrogen atmosphere. The reaction was quenched by the addition of sat. NH₄Cl (10 mL) at room temperature. The aqueous mixture was extracted with EtOAc (3x10 mL). The combined organic extracts were concentrated under reduced pressure. The residue was purified by prep-HPLC to give the title compound (380 mg, 22.22%) as a light yellow oil.
(LCMS (ESI) m/z [M+H]⁺ =187.

### Intermediate 63: Ethyl 2-azabicyclo[2.2.2]octane-4-carboxylate

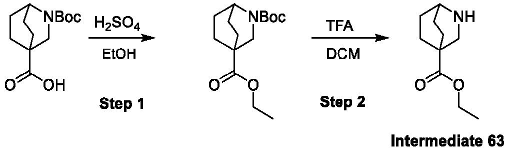

### Step 1. 2-tert-Butyl 4-ethyl 2-azabicyclo[2.2.2]octane-2,4-dicarboxylate

2-[(*tert*-Butoxy)carbonyl]-2-azabicyclo[2.2.2]octane-4-carboxylic acid (0.2 g, 0.783 mmol) was dissolved in EtOH (2.6 mL). Catalytic sulfuric acid (0.26 mL) was added and the reaction mixture stirred overnight. The excess solvent was then removed under vacuum. The crude residue was dissolved in EtOAc, the organic layer washed with saturated NaHCO₃, dried over Na₂SO₄, filtered, and concentrated under reduced pressure to give the title compound as a yellow oil (0.14 g).
LCMS (ESI) m/z: [M+H-Boc]+ = 184.2.

### Step 2. Ethyl 2-azabicyclo[2.2.2]octane-4-carboxylate

2-Azabicyclo[2.2.2]octane-2,4-dicarboxylate (0.065 g, 0.24 mmol) was dissolved in DCM in (1.2 mL) and cooled to 0°C. Trifluoroacetic acid (0.19 mL, 2.4 mmol) was added dropwise and the reaction mixture stirred for 1 h at 0°C. The solvent was then removed under vacuum. The crude residue was dissolved in DCM, the organic layer washed with saturated NaHCO₃, dried over Na₂SO₄, filtered, and concentrated under reduced pressure to give the title compound as a yellow oil (0.04 g).
LCMS (ESI) m/z: [M+H]+ = 184.2.

### Intermediate 64: 2-Chloro-8-fluoro-1,6-naphthyridine-7-carbonitrile

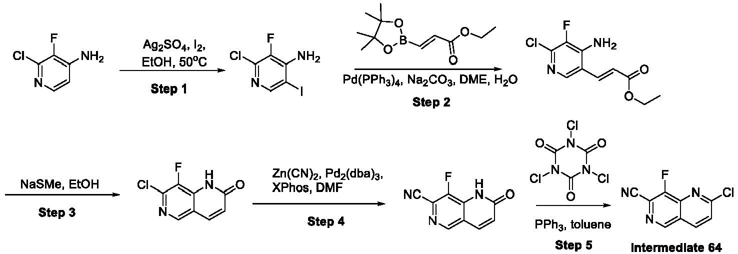

### Step 1: 2-Chloro-3-fluoro-5-iodopyridin-4-amine

To a stirred solution of 2-chloro-3-fluoropyridin-4-amine (6.00 g, 40.942 mmol, 1.00 equiv) and Ag₂SO₄ (12.77 g, 40.956 mmol, 1.00 equiv) in ethanol (150 mL) was added I₂ (10.39 g, 40.942 mmol, 1 equiv). The resulting mixture was stirred at 55 °C for 2 h. The solid was filtered out and the filtrate was concentrated under reduced pressure. The residue was dissolved in DCM (300 mL), washed with water (50 mL x 2) and saturated brine (30 mL x 1), dried over anhydrous Na₂SO₄. After filtration, the filtrate was concentrated under reduced pressure to give the title compound (8.8 g, 78.89%) as a light yellow solid. LCMS (ESI) m/z: [M+H]⁺ = 273.

### Step 2: Ethyl (E)-3-(4-amino-6-chloro-5-fluoropyridin-3-yl)acrylate

To a stirred solution of 2-chloro-3-fluoro-5-iodopyridin-4-amine (6.00 g, 22.022 mmol, 1.00 equiv) and ethyl (2*E*)-3-(4,4,5,5-tetramethyl-1,3,2-dioxaborolan-2-yl)prop-2-enoate (6.47 g, 28.629 mmol, 1.3 equiv) in DME (90 mL) and H₂O (30 mL) were added Pd(PPh₃)₄ (2.54 g, 2.202 mmol, 0.1 equiv) and Na₂CO₃ (4.67 g, 44.045 mmol, 2 equiv). The resulting mixture was stirred at 100 °C for 3 h under nitrogen atmosphere. The reaction mixture was concentrated under reduced pressure. The residue was purified by silica gel column chromatography, eluting with PE/EtOAc (5:1) to give the title compound (5.0 g,92.80%) as a light yellow oil.
LCMS (ESI) m/z: [M+H]⁺ = 245.

### Step 3: 7-Chloro-8-fluoro-1,6-naphthyridin-2(1H)-one

A solution of ethyl (E)-3-(4-amino-6-chloro-5-fluoropyridin-3-yl)acrylate (5.00 g, 20.437 mmol, 1.00 equiv) and NaSMe (17.88 g, 51.093 mmol, 2.5 equiv, 20% in H₂O) in ethanol (60 mL) was stirred for 4 h at 60 °C. The mixture was acidified to pH 7 with 1 N. HCl. The resulting mixture was extracted with EtOAc (200 mL x 2). The combined organic layers were washed with water (100 mL) and dried over anhydrous Na₂SO₄. After filtration, the filtrate was concentrated under reduced pressure. The residue was purified by silica gel column chromatography, eluting with PE/EtOAc (5:1) to give the title compound (2.23 g, 54.95%) as a light yellow solid.
LCMS (ESI) m/z: [M+H]⁺ = 199.

### Step 4: 8-Fluoro-2-oxo-1,2-dihydro-1,6-naphthyridine-7-carbonitrile

To a stirred solution of 7-chloro-8-fluoro-1,6-naphthyridin-2(1H)-one (1.00 g, 5.036 mmol, 1.00 equiv) and Zn(CN)₂ (1.18 g, 10.072 mmol, 2 equiv) in DMF (5 mL) were added Pd₂(dba)₃ (0.92 g, 1.007 mmol, 0.2 equiv) and XPhos (0.96 g, 2.014 mmol, 0.4 equiv). The resulting mixture was stirred at 100 °C for 4 h under nitrogen atmosphere. The reaction mixture was concentrated under reduced pressure. The residue was purified by prep-HPLC to give the title compound (561 mg,58.90%) as a light yellow solid. LCMS (ESI) m/z: [M+H]⁺ = 190.

### Step 5: 2-Chloro-8-fluoro-1,6-naphthyridine-7-carbonitrile

A solution of trichloroisocyanuric acid (568.01 mg, 2.444 mmol, 0.67 equiv) and PPh₃ (1913.59 mg, 7.296 mmol, 2 equiv) in toluene (1 mL) was stirred overnight at room temperature under nitrogen atmosphere. To the above mixture was added 8-fluoro-2-oxo-1,2-dihydro-1,6-naphthyridine-7-carbonitrile (690.00 mg, 3.648 mmol, 1.00 equiv) at room temperature. The resulting mixture was stirred for an additional 4 h at 110 °C under nitrogen atmosphere. The mixture was concentrated under reduced pressure. The residue was purified by silica gel column chromatography, eluting with PE/EtOAc (1:1) to give the title compound (346 mg, 45.69%) as a white solid.
LCMS (ESI) m/z: [M+H]⁺ = 208.

### Intermediate 65: tert-Butyl ((6-bromoisoquinolin-3-yl)methyl)carbamate

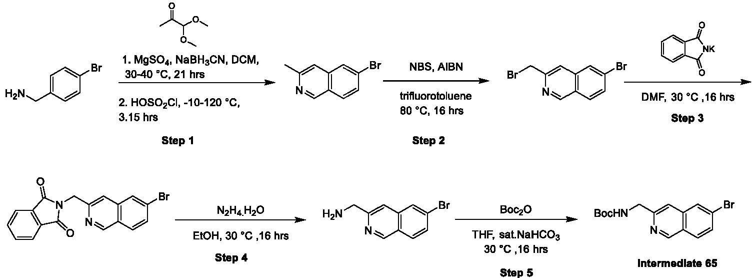

### Step 1: 6-Bromo-3-methylisoquinoline

To a solution of (4-bromophenyl)methanamine (30 g, 161.25 mmol) in DCM (500 mL) was added 1,1-dimethoxypropan-2-one (20.95 g, 177.37 mmol) and MgSO₄ (60.00 g, 498.47 mmol). The resulting mixture was stirred at 40 °C for 16 hrs. NaBH₃CN (12.16 g, 193.50 mmol) was then added and the mixture stirred at 30 °C for 5 hrs before filtering. The filtrate was concentrated to give a yellow oil. Chlorosulfonic acid (157.50 g, 1.35 mol) was cooled to -10 °C and the above crude product was added dropwise for 3 hrs under N₂. The reaction mixture was heated to 120 °C for 10 mins. The reaction mixture was cooled to 25 °C and poured into ice water (2 L). The solution was neutralized with 2 M NaOH and extracted with EA (1000 mL * 3). The combined organic layer was washed with brine (2000 mL), dried over Na₂SO₄, filtered and concentrated. The residue was purified by column chromatography (SiO₂, Petroleum ether/Ethyl acetate=10:1-5:1) to give the title compound (8.5 g, 38.27 mmol, 23.74% yield) as a yellow solid.
¹H NMR (400 MHz, DMSO-d₆) δ = 9.06 (s, 1H), 7.8 (d, *J* = 1.2 Hz, 1H), 7.71 (d, *J* = 8.4 Hz, 1H), 7.52 - 7.50 (m, 1H), 7.30 (s, 1H), 2.62 (s, 3H) ppm.

### Step 2: 6-Bromo-3-(bromomethyl)isoquinoline

To a solution of 6-bromo-3-methylisoquinoline (8.3 g, 37.37 mmol) in trifluoromethylbenzene (200 mL) was added NBS (7.97 g, 44.77 mmol) and AIBN (919.64 mg, 5.60 mmol). The mixture was stirred at 80 °C for 16 hrs. The reaction mixture was concentrated and the residue was purified by column chromatography (SiO₂, Petroleum ether/Ethyl acetate=40:1-20:1) to give the title compound (3 g, 9.22 mmol, 24.68% yield) as a yellow solid.
LCMS (ESI) m/z: [⁷⁹BrM+H]⁺= 301.9
¹H NMR (400 MHz, DMSO-d₆) δ = 9.22 (s, 1H), 8.02 - 8.00 (m, 2H), 7.86 (d, *J* = 8.8 Hz, 1H), 7.72 - 7.68 (m, 2H), 4.72 (s, 2H) ppm.

### Step 3: 2-((6-Bromoisoquinolin-3-yl)methyl)isoindoline-1,3-dione

A mixture of 6-bromo-3-(bromomethyl)isoquinoline (3 g, 9.97 mmol) and (1,3-dioxoisoindolin-2-yl)potassium (2.03 g, 10.96 mmol) in DMF (65 mL) was stirred at 30 °C for 16 hrs. The reaction mixture was poured into NaHCO₃ solution (200 mL) and the products were extracted with DCM (200 mL * 3). The combined organic layer was washed with brine (500 mL), dreid over Na₂SO₄,filtered and concentrated to give the title compound (3.6 g, 9.80 mmol, 98.36% yield) as a yellow solid.
¹H NMR (400 MHz, DMSO-d₆) δ = 9.15 (s, 1H), 7.95 - 7.89 (m, 3H), 7.76 - 7.74 (m, 3H), 7.65 (s, 1H), 7.54 (s, 1H), 5.30 (s, 2H) ppm.

### Step 4: (6-Bromoisoquinolin-3-yl)methanamine

To a solution of 2-((6-bromoisoquinolin-3-yl)methyl)isoindoline-1,3-dione (3.6 g, 9.80 mmol) in EtOH (60 mL) was added NH₂NH₂.H₂O (1.72 g, 34.31 mmol). The mixture was stirred at 30 °C for 16 hrs. The reaction mixture was concentrated under vacuum to give the title compound (2.32 g, 9.79 mmol, 100.00% yield) as a yellow solid.
¹H NMR (400 MHz, DMSO-d₆) δ = 9.25 - 9.23 (m, 1H), 8.22 - 8.20 (m, 1H), 8.05 (d, *J* = 8.8 Hz, 1H), 7.78 - 7.71 (m, 2H), 3.95 (s, 2H) ppm.

### Step 5: tert-Butyl ((6-bromoisoquinolin-3-yl)methyl)carbamate

To a solution of (6-bromoisoquinolin-3-yl)methanamine (2.32 g, 9.79 mmol) in THF (25 mL) was added NaHCO₃ (1.64 g, 19.57 mmol) and Boc₂O (2.56 g, 11.74 mmol). The mixture was stirred at 30 °C for 2 hrs. The reaction mixture was poured into water (100 mL) and the products were extracted with EA (50 mL * 3). The combined organic layer was washed with brine (100 mL), dried over Na₂SO₄, filtered and concentrated. The residue was purified by column chromatography (SiO₂, Petroleum ether/Ethyl acetate=20:1-2:1) to give the title compound (2.2 g, 6.25 mmol, 63.87% yield) as a yellow solid.
LCMS (ESI) m/z: [⁷⁹BrM+H]⁺= 339.1
¹H NMR (400 MHz, DMSO-d₆) δ = 9.17 (s, 1H), 7.97 - 7.95 (m, 1H), 7.83 (d, *J* = 8.8 Hz, 1H), 7.67 - 7.64 (m, 1H), 7.53 (s, 1H), 4.58 (br d, *J* = 6.0 Hz, 2H), 1.48 (s, 9H) ppm.

### Intermediate 66: 2-((6-Bromocinnolin-3-yl)methyl)isoindoline-1,3-dione

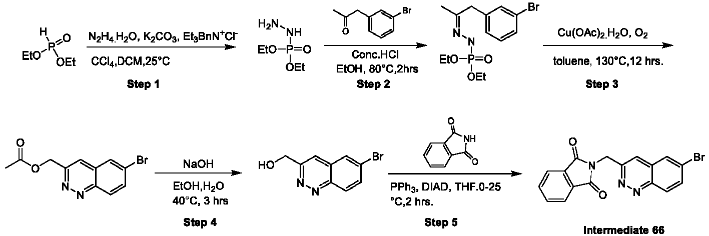

### Step 1: Diethyl hydrazineylphosphonate

Hydrazine hydrate (21.32 g, 362.05 mmol, 20.70 mL) was added dropwise with efficient stirring to a mixture of CCl₄ (180 mL), DCM (300 mL), anhydrous K₂CO₃ (75.06 g, 543.08 mmol) and benzyl(triethyl)ammonium chloride (824.66 mg, 3.62 mmol) at 25°C. Stirring was continued for 15 min at 25°C and a solution of diethyl phosphonate (50 g, 362.05 mmol, 46.73 mL) in DCM (60 mL) was then added at 25°C with occasional external cooling. After the addition had been completed, stirring was continued for 4 hrs at 25°C. Potassium carbonate was then filtered off and the filter cake washed with DCM. The filtrate was concentrated under reduced pressure to provide the title compound (56 g, 333.07 mmol, 92.00% yield) as a light yellow oil that was used directly in the next step.

### Step 2: Diethyl (Z)-(2-(1-(3-bromophenyl)propan-2-ylidene)hydrazineyl)phosphonate

A mixture of 1-(3-bromophenyl)propan-2-one (20.9 g, 98.09 mmol), diethyl hydrazineylphosphonate (17.32 g, 102.99 mmol) and HCl (0.6 mL) in EtOH (400 mL) was stirred at 80 °C for 2 hrs. The reaction mixture was concentrated to afford a yellow oil. The oil was dissolved in water (100 mL) and sat. NaHCO₃ was added to adjust pH to 9. The products were extracted with DCM (50 mL*3). The combined organic phase was dried over anhydrous Na₂SO₄ and concentrated to afford a brown residue. The residue was purified by column chromatography (SiO₂, Dichloromethane / Methanol = 1 / 0 to 10/1) to give the title compound (26.65 g, 66.04 mmol, 67.33% yield) as a light yellow solid.
LCMS (ESI) m/z: [M+H] ⁺ = 362.9/364.9

### Step 3: (6-Bromocinnolin-3-yl)methyl acetate

To a solution of diethyl (*Z*)-(2-(1-(3-bromophenyl)propan-2-ylidene)hydrazineyl)phosphonate (3.65 g, 10.05 mmol) in toluene (92 mL) was added Cu(OAc)₂.H₂O (2.01 g, 10.05 mmol, 2.01 mL) at 25 °C. The mixture was stirred at 130 °C for 12 hrs under O₂ (15psi). The mixture was diluted with H₂O (500 mL) and extracted with EA (500 mL*2). The combined organic layers were dried over anhydrous Na₂SO₄, filtered and concentrated. The residue was purified by column chromatography (SiO₂, DCM / EA = 1 / 0 to 0/1) to give the title compound (1 g, 3.56 mmol, 17.70% yield) as a brown solid.
¹H NMR (400 MHz, CDCl₃) δ = 8.45 (d, *J* = 9.2 Hz, 1H), 8.05 (d, *J* = 1.6 Hz, 1H), 7.94-7.91 (m, 1H), 7.83 (s, 1H), 5.70 (s, 2H), 2.20 (s, 3H) ppm.

### Step 4: (6-Bromocinnolin-3-yl)methanol

To a solution of (6-bromocinnolin-3-yl)methyl acetate (1 g, 3.56 mmol) in MeOH / THF / H₂O = 2 / 2 / 1 (10 mL) was added NaOH (213.44 mg, 5.34 mmol). The mixture was stirred at 40 °C for 3 hrs. The reaction mixture was diluted with H₂O (30 mL) and extracted with DCM (30 mL*2). The combined organic layers were dried over anhydrous Na₂SO₄, filtered and concentrated. The residue was triturated with MTBE (20 mL) and the yellow solid was collected by filtration to give the title compound (580 mg, 2.43 mmol, 68.20% yield) as a yellow solid.
LCMS (ESI) m/z: [M+H] ⁺ =238.8/240.8.
¹H NMR (400 MHz, DMSO-d6) δ = 8.47 (d, *J* = 1.6 Hz, 1H), 8.41 (d, *J* = 9.2 Hz, 1H), 8.16 (s, 1H), 8.03-8.01 (m, 1H), 5.82-5.79 (m, 1H), 5.07 (d, *J* = 5.6 Hz, 2H) ppm.

### Step 5: 2-((6-Bromocinnolin-3-yl)methyl)isoindoline-1,3-dione

To a solution of (6-bromocinnolin-3-yl)methanol (580 mg, 2.43 mmol), isoindoline-1,3-dione (392.65 mg, 2.67 mmol) and PPh₃ (954.51 mg, 3.64 mmol) in THF(20 mL) was added a solution of DEAD (633.79 mg, 3.64 mmol, 661.58 uL) in THF (10 mL) at 0 °C. The mixture was stirred at 25 °C for 2 hrs under N₂. The reaction mixture was diluted with H₂O (100 mL) and extracted with DCM (100 mL*2). The combined organic layers were dried over anhydrous Na₂SO₄, filtered and concentrated. The residue was triturated with MeOH (20 mL) and the off-white solid collected by filtration to give the title compound (550 mg, 1.49 mmol, 61.57% yield).
LCMS (ESI) m/z: [M+H] ⁺ =369.9.
¹H NMR (400 MHz, DMSO-d6) δ = 8.39-8.37 (m, 2H), 8.21 (s, 1H), 8.04-8.02 (m, 1H), 7.96-7.93 (m, 2H), 7.90-7.89 (m, 2H), 5.34 (s, 2H) ppm.

### Intermediate 67: tert-Butyl ((3-bromo-1,7-naphthyridin-6-yl)methyl)carbamate

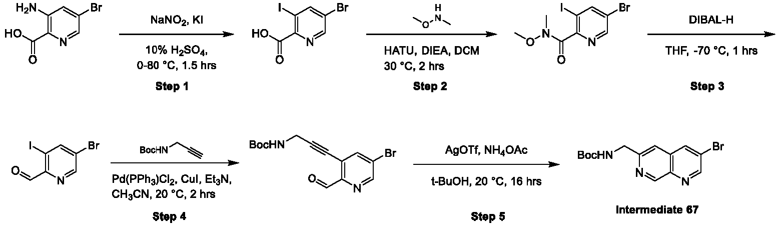

### Step 1: 5-Bromo-3-iodopicolinic acid

To a solution of 3-amino-5-bromo-pyridine-2-carboxylic acid (4 g, 18.43 mmol) in 10% H₂SO₄ (30 mL) was added NaNO₂ (1.91 g, 27.65 mmol) at 0 °C. The mixture was stirred at 0 °C for 30 min. KI (9.18 g, 55.29 mmol) was added and the reaction was stirred at 0 °C for 30 min and then heated to 80 °C for 1 hr. The reaction mixture was cooled to 20 °C and slowly poured into ice water (80 mL). The resulting yellow solid was collected by filtration and dried to give the title compound (4.5 g, 13.72 mmol, 74.46% yield).
LCMS (ESI) m/z: [⁷⁹BrM+H]⁺ = 327.8.
¹HNMR (400 MHz, DMSO-d₆) δ = 8.73 (d, J=2.0 Hz, 1H), 8.70 (d, J=2.0 Hz, 1H) ppm.

### Step 2: 5-Bromo-3-iodo-N-methoxy-N-methylpicolinamide

To a solution of 5-bromo-3-iodopicolinic acid (2 g, 6.10 mmol) in DCM (15 mL) was added HATU (3.48 g, 9.15 mmol), DIPEA (3.94 g, 30.50 mmol) and *N*,O-dimethylhydroxylamine (713.95 mg, 7.32 mmol). The mixture was stirred at 30 °C for 2 hrs. Water (30 mL) was added and the products extracted with ethyl acetate (30 mL *3). The combined organic phase was washed with brine (30 mL), dried over Na₂SO₄, filtered and concentrated under reduced pressure. The residue was purified by column chromatography (SiO₂, Petroleum ether/Ethyl acetate=1/1) to give the title compound (2.2 g, 5.93 mmol, 97.23% yield) as a yellow oil.
LCMS (ESI) m/z: [⁸¹BrM+H]⁺ = 372.8.

### Step 3: 5-Bromo-3-iodopicolinaldehyde

To a solution of 5-bromo-3-iodo-*N*-methoxy-*N*-methylpicolinamide (1 g, 2.70 mmol) in THF (10 mL) was added drop wise DIBAL-H (1 M, 5.39 mL, 5.39 mmol) at -70 °C under N₂. The mixture was stirred at -70 °C for 1 hr. The reaction was quenched by adding water (50 mL) and the products were extracted with ethyl acetate (50 mL *3). The combined organic layers were washed with brine (30 mL), dried over Na₂SO₄, concentrated under vacuum. The residue was purified by column chromatography (SiO₂, Petroleum ether/Ethyl acetate=3/1) to give the title compound (0.6 g, 1.92 mmol, 71.36% yield) as a yellow oil.
LCMS (ESI) m/z: [⁷⁹BrM+H]⁺ = 311.8.
¹HNMR (400 MHz, CDCl₃) δ = 9.98 (s, 1H), 8.83 (d, *J*=2.0 Hz, 1H), 8.53 (d, *J*=2.0 Hz, 1H) ppm.

### Step 4: tert-Butyl (3-(5-bromo-2-formylpyridin-3-yl)prop-2-yn-1-yl)carbamate

5-Bromo-3-iodopicolinaldehyde (150 mg, 480.92 umol), *tert*-butyl *N*-prop-2-ynylcarbamate (74.64 mg, 480.92 umol), Et₃N (486.64 mg, 4.81 mmol, 669.39 uL), Cul (9.16 mg, 48.09 umol) and Pd(PPh₃)₂Cl₂ (33.76 mg, 48.09 umol) in CH₃CN (6 mL) was de-gassed and purged with N₂ before stirring at 20 °C for 2 hrs under N₂. The reaction was quenched by adding water (30 mL) and the products were extracted with ethyl acetate (30 mL *3). The combined organic phase was washed with brine (30 mL), dried over Na₂SO₄, filtered and concentrated to give the title compound (0.2 g, crude) as a yellow oil that was used in the next step without further purification.
LCMS (ESI) m/z: [⁷⁹BrM+H]⁺ = 339.0.

### Step 5: tert-Butyl ((3-bromo-1,7-naphthyridin-6-yl)methyl)carbamate

To a solution of *tert*-butyl (3-(5-bromo-2-formylpyridin-3-yl)prop-2-yn-1-yl)carbamate (0.2 g, 589.65 umol) in tBuOH (5 mL) was added NH₄OAc (68.18 mg, 884.48 umol) and AgOTf (30.30 mg, 117.93 umol). The mixture was stirred at 20 °C for 16 hrs. The reaction mixture was quenched by adding water (30 mL) and the products were extracted with ethyl acetate (30 mL *3). The combined organic phase was washed with brine (30 mL), dried over Na₂SO₄, filtered and concentrated. The residue was purified by column chromatography (SiO₂, Petroleum ether/Ethyl acetate=1/1) to give the title compound (110 mg, 315.49 umol, 53.51% yield) as a yellow oil.
LCMS (ESI) m/z: [⁷⁹BrM+H]⁺ = 338.1.

### Intermediate 68: 2-((2-Chlorothiazolo[5,4-c]pyridin-6-yl)methyl)isoindoline-1,3-dione

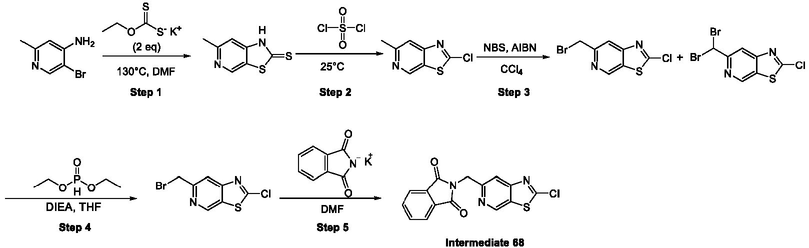

### Step 1: 6-Methylthiazolo[5,4-c]pyridine-2(1H)-thione

A solution of 5-bromo-2-methylpyridin-4-amine (7.50 g, 40.098 mmol, 1.00 equiv) and potassium ethyl xanthate (25.71 g, 160.393 mmol, 4 equiv) in DMF (75 mL) was stirred for 15 h at 130 °C. The resulting mixture was diluted with water (200 mL) and extracted with EtOAc (300 mL). The organic layer was discarded, and the aqueous layer was concentrated under reduced pressure. The residue was slurried with EtOAc (500 mL) and stirred at 25°C for 1 h. The solid was removed by filtration and the filtrate was concentrated under reduced pressure to give the title compound (3.5 g, 47.89%) as a light yellow solid.
LCMS (ESI) m/z: [M+H]⁺ = 183.

### Step 2: 2-Chloro-6-methylthiazolo[5,4-c]pyridine

A solution of 6-methylthiazolo[5,4-*c*]pyridine-2(1*H*)-thione (2.10 g, 11.522 mmol, 1.00 equiv) in sulfonyl chloride (20.00 mL) was stirred at room temperature for 2 hours. The mixture was poured into ice-water (200 mL). The aqueous mixture was basified to pH 9 with Na₂CO₃. And extracted with EtOAc (200 mL x 3). The combined organic layers were washed with water (50 mL x1), dried over anhydrous Na₂SO₄, filtered and concentrated under reduced pressure to give the title compound (0.726 g, 34.13%) as a light yellow solid.
LCMS (ESI) m/z: [M+H]⁺ = 185.

### Step 3: 6-(Bromomethyl)-2-chlorothiazolo[5,4-c]pyridine and 2-chloro-6-(dibromomethyl)thiazolo[5,4-c]pyridine

To a stirred solution of 2-chloro-6-methylthiazolo[5,4-c]pyridine (1.83 g, 9.911 mmol, 1.00 equiv) and NBS (1.76 g, 9.911 mmol, 1 equiv) in CCl₄ (30 mL) was added AIBN (0.16 g, 0.991 mmol, 0.1 equiv). The resulting mixture was stirred at 80 °C for 15 hours under nitrogen atmosphere. The reaction mixture was poured into water (30 mL) and extracted with DCM (60 mL). The combined organic layers were washed with brine (30 mL x 1), dried over anhydrous Na₂SO₄, filtered and concentrated under reduced pressure. The residue was purified by silica gel column chromatography, eluting with PE/EtOAc (10:1) to give a mixture of the title compounds (1.57g, 60.11%) as a light yellow solid.
LCMS (ESI) m/z: [M+H]⁺ = 265.

### Step 4: 6-(Bromomethyl)-2-chlorothiazolo[5,4-c]pyridine

To a stirred solution of a mixture of 6-(bromomethyl)-2-chlorothiazolo[5,4-c]pyridine and 2-chloro-6-(dibromomethyl)thiazolo[5,4-*c*]pyridine (1.87 g, 5.461 mmol, 1.00 equiv) and diethyl phosphonate (4.53 g, 32.766 mmol, 6 equiv) in THF (25 mL) was added DIEA (4.23 g, 32.766 mmol, 6 equiv). The mixture was stirred at room temperature for 15 hours. The mixture was concentrated under vacuum and the residue was purified by silica gel column chromatography, eluting with PE/EtOAc (12:1) to give the title compound (1.42g, 98.67%) as a light yellow solid.
LCMS (ESI) m/z: [M+H]⁺ = 265.

### Step 5: 2-((2-Chlorothiazolo[5,4-c]pyridin-6-yl)methyl)isoindoline-1,3-dione

A solution of 6-(bromomethyl)-2-chlorothiazolo[5,4-c]pyridine (100.00 mg, 0.379 mmol, 1.00 equiv) and potassium phthalimide (84.34 mg, 0.455 mmol, 1.2 equiv) in DMF (1 mL) was stirred for 1 hour at room temperature. The mixture was poured into water (2 mL) and stirred for 10 min. The resulting mixture was filtered, the filter cake was washed with water (5 mL x 2). The solid was dried under vacuum to give the title compound (114 mg, 91.11%) as a white solid.
LCMS (ESI) m/z: [M+H]⁺ = 330.

### Intermediate 69: tert-Butyl ((6-chloropyrido[3,2-c]pyridazin-3-yl)methyl)carbamate

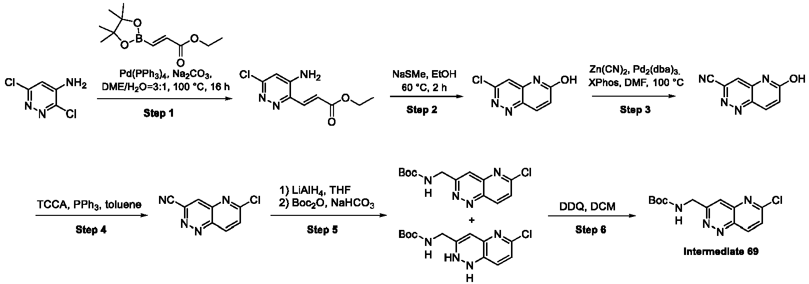

### Step 1: Ethyl (E)-3-(4-amino-6-chloropyridazin-3-yl)acrylate

To a stirred solution of 3,6-dichloropyridazin-4-amine (24.00 g, 146.350 mmol, 1.00 equiv), ethyl (2E)-3-(4,4,5,5-tetramethyl-1,3,2-dioxaborolan-2-yl)prop-2-enoate (39.70 g, 175.620 mmol, 1.20 equiv) in DME (270.00 mL) and H₂O (90.00 mL) was added Pd(PPh₃)₄ (16.91 g, 14.635 mmol, 0.10 equiv) and Na₂CO₃ (31.02 g, 292.701 mmol, 2.00 equiv). The mixture was stirred at 100 °C for overnight. The resulting mixture was concentrated under reduced pressure. The residue was diluted with water (300 mL) and extracted with EtOAc (300 mL x 5). The combined organic layers were washed with brine (80 mL), dried over anhydrous Na₂SO₄, filtered and evaporated. The crude product was purified by silica gel column chromatography, elution gradient 0 to 100% EtOAc in petroleum ether to give a mixture of 3,6-dichloropyridazin-4-amine and the title compound (15 g, crude) as a yellow solid. The mixture was recrystallized from EtOAc (260 mL) to give the title compound (10 g, 30.02%) as a yellow solid.
LCMS (ESI) m/z: [M+H]⁺ = 228.

### Step 2: 3-Chloropyrido[3,2-c]pyridazin-6-ol

To a stirred solution of ethyl (*E*)-3-(4-amino-6-chloropyridazin-3-yl)acrylate (11.80 g, 51.834 mmol, 1.00 equiv) in EtOH (150 mL) was added NaSMe (9.08 g, 129.548 mmol, 2.50 equiv) dropwise at room temperature. The resulting mixture was stirred for 2 h at 60 °C. The mixture was diluted with water (100 mL) and neutralized to pH 7 with 1M HCl (aq.). The resulting mixture was filtered and the filter cake was washed with tert-butyl methyl ether (10 mL x 3). The filter cake was dried under vacuum to give the title compound (6.3 g, 63.59%) as a grey solid.
LCMS (ESI) m/z: [M+H]⁺ =182.

### Step 3: 6-Hydroxypyrido[3,2-c]pyridazine-3-carbonitrile

To a stirred solution of 3-chloropyrido[3,2-c]pyridazin-6-ol (3500.00 mg, 19.275 mmol, 1.00 equiv) in DMF (40 mL) was added Zn(CN)₂ (4527.53 mg, 38.551 mmol, 2 equiv), Pd₂(dba)₃ (3530.15 mg, 3.855 mmol, 0.2 equiv) and XPhos (3675.53 mg, 7.710 mmol, 0.40 equiv). The mixture was stirred at 100 °C overnight under nitrogen atmosphere. The resulting mixture was concentrated under reduced pressure. The crude product was purified by prep-HPLC to give the title compound (900 mg, 31.64%) as a yellow solid.
LCMS (ESI) m/z: [M+H]⁺ = 173.

### Step 4: 6-Chloropyrido[3,2-c]pyridazine-3-carbonitrile

To a stirred solution of PPh₃ (3047.23 mg, 11.618 mmol, 2 equiv) in 1,4-dioxane (100 mL) was added trichloroisocyanuric acid (904.51 mg, 3.892 mmol, 0.67 equiv) in portions. The resulting mixture was stirred for 23 h at room temperature. To the above mixture was added 6-hydroxypyrido[3,2-c]pyridazine-3-carbonitrile (1000.00 mg, 5.809 mmol, 1.00 equiv) in portions. The resulting mixture was stirred for additional 4 h at 108 °C. The resulting mixture was concentrated under reduced pressure. The residue was purified by silica gel column chromatography, elution gradient 0 to 100% PE in EtOAc to give the title compound (581 mg, 52.48%) as a yellow solid.
LCMS (ESI) m/z: [M+H]⁺ = 191.

### Step 5: tert-Butyl ((6-chloropyrido[3,2-c]pyridazin-3-yl)methyl)carbamate and tert-butyl ((6-chloro-1,2-dihydropyrido[3,2-c]pyridazin-3-yl)methyl)carbamate

To a stirred solution of 6-chloropyrido[3,2-c]pyridazine-3-carbonitrile (300.00 mg, 1.574 mmol, 1.00 equiv) in THF (8 mL) was added a 2.5 M solution of LiAlH₄ (1.9 mL, 4.722 mmol, 3.00 equiv) in THF dropwise at 0 °C. The resulting mixture was stirred for 30 min at 0 °C. The reaction was quenched by the addition of sat. NaHCO₃ (aq.) (10 mL) at 0 °C. To the above mixture was added Boc₂O (1030.60 mg, 4.722 mmol, 3.00 equiv) dropwise at 0 °C. The resulting mixture was stirred for additional 1 h at room temperature. The mixture was concentrated under reduced pressure to give a mixture of the title compounds (231 mg, crude) that was used directly without further purification.
LCMS (ESI) m/z: [M+H]⁺ = 295.

### Step 6: tert-Butyl ((6-chloropyrido[3,2-c]pyridazin-3-yl)methyl)carbamate

To a stirred solution of the mixture of *tert*-butyl ((6-chloropyrido[3,2-c]pyridazin-3-yl)methyl)carbamate and *tert*-butyl ((6-chloro-1,2-dihydropyrido[3,2-*c*]pyridazin-3-yl)methyl)carbamate (231 mg, crude) in DCM (8 mL) was added DDQ (714.64 mg, 3.148 mmol, 2.00 equiv) in portions. The resulting mixture was stirred for 2 h at room temperature. The resulting mixture was filtered and the filter cake was washed with DCM (5 mL x 3). The filtrate was concentrated under reduced pressure. The residue was purified by prep-HPLC to give the title compound (112 mg, 24.14%) as a brown solid. LCMS (ESI) m/z: [M+H]⁺ = 295.

### Intermediate 70: tert-Butyl ((5-methyl-5,6,7,8-tetrahydro-2,6-naphthyridin-3-yl)methyl)carbamate

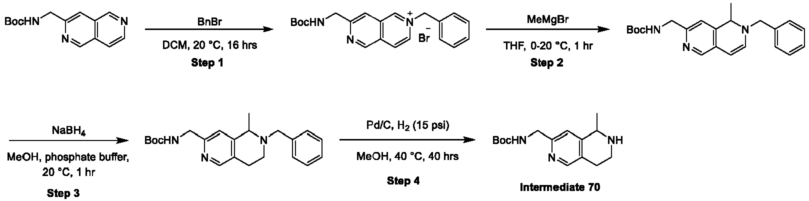

### Step 1: 2-Benzyl-7-(((tert-butoxycarbonyl)amino)methyl)-2,6-naphthyridin-2-ium bromide

To a solution of *tert*-butyl ((2,6-naphthyridin-3-yl)methyl)carbamate (3.6 g, 13.88 mmol) in DCM (10 mL) was added (bromomethyl)benzene (3.56 g, 20.83 mmol). The mixture was stirred at 30 °C for 16 hrs. The reaction mixture was concentrated and the residue was triturated in MTBE (50 mL) to give the title compound (4.5 g, crude) as a gray solid.
LCMS (ESI) m/z: [M]⁺ = 350.3.

### Step 2: tert-Butyl ((6-benzyl-5-methyl-5,6-dihydro-2,6-naphthyridin-3-yl)methyl)carbamate

2-Benzyl-7-(((*tert*-butoxycarbonyl)amino)methyl)-2,6-naphthyridin-2-ium bromide (2.0 g, 4.65 mmol) was added portionwise to a solution of MeMgBr (3 M, 9.30 mL) in THF (50 mL) at 0 °C under N₂. The mixture was stirred at 20 °C for 1 hr. The reaction was quenched by adding sat. NH₄Cl (50mL) and the products were extracted with ethyl acetate (50 mL * 3). The combined organic layers were washed with brine (30 mL), dried over Na₂SO₄, filtered and concentrated to give the title compound (2 g, crude) as a yellow oil.
LCMS (ESI) m/z: [M+H]⁺ = 366.3.

### Step 3: tert-Butyl ((6-benzyl-5-methyl-5,6,7,8-tetrahydro-2,6-naphthyridin-3-yl)methyl)carbamate

To a solution of *tert*-butyl ((6-benzyl-5-methyl-5,6-dihydro-2,6-naphthyridin-3-yl)methyl)carbamate (2 g, 5.47 mmol) in MeOH (20 mL) and phosphate buffer pH 7.0 (20 mL) was added NaBH₄ (828.09 mg, 21.89 mmol) at 0 °C. The mixture was stirred at 20 °C for 1 hr. The reaction was quenched by adding 0.5N HCl (50mL) and the products were extracted with ethyl acetate (50 mL*3). The combined organic layers were washed with brine (30 mL), dried over Na₂SO₄, filtered and concentrated. The residue was purified by column chromatography (SiO₂, Petroleum ether/Ethyl acetate=10/1 to 0/1) to give the title compound (1.3 g, crude) as a yellow oil.
LCMS (ESI) m/z: [M+H]⁺ = 368.2.

### Step 4: tert-Butyl ((5-methyl-5,6,7,8-tetrahydro-2,6-naphthyridin-3-yl)methyl)carbamate

A mixture of *tert*-butyl ((6-benzyl-5-methyl-5,6,7,8-tetrahydro-2,6-naphthyridin-3-yl)methyl)carbamate (0.5 g, 1.36 mmol) and Pd/C (666.67 mg, 634.92 umol, 10% purity) in MeOH (15 mL) was degassed and purged with H₂ 3 times. The mixture was stirred at 40 °C for 40 hrs under H₂ atmosphere (15 psi). The reaction mixture was filtered and the filtrate was concentrated under vacuum to give the title compound (0.35 g, crude) as a yellow oil.
LCMS (ESI) m/z: [M+H]⁺ = 278.3.
¹H NMR (400 MHz, DMSO-d₆) δ = 8.10 (s, 1H), 7.40 - 7.22 (m, 1H), 7.01 (s, 1H), 4.18 - 4.12 (m, 2H), 3.93 -3.82 (m, 1H), 3.20 - 3.15 (m, 1H), 3.13 - 3.04 (m, 1H), 2.82 - 2.74 (m, 1H), 2.70 - 2.65 (m, 1H), 2.59 (br s, 1H), 1.44 - 1.38 (m, 9H), 1.32 - 1.29 (m, 3H) ppm.

### Intermediate 71: 2-((5,6,7,8-tetrahydropyrido[4,3-c]pyridazin-3-yl)methyl)isoindoline-1,3-dione hydrochloride

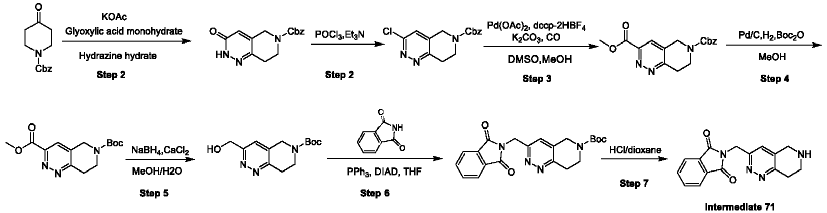

### Step 1: Benzyl 3-oxo-3,5,7,8-tetrahydropyrido[4,3-c]pyridazine-6(2H)-carboxylate

A mixture of benzyl 4-oxopiperidine-1-carboxylate (25 g, 107.18 mmol), KOAc (0.4 g, 4.08 mmol) and glyoxylic acid monohydrate (10 g, 135.07 mmol) was stirred at 90 °C for 12 hours. N₂H₄.H₂O (5.67 g, 96.19mmol, 5.5 mL) was then added dropwise to the mixture at 90°C before stirring at 90°C for an additional 0.5 hr. The mixture was diluted with MeOH (100mL), cooled to 25 °C and filtered. The solid was washed with MeOH and dried to give the title compound (10 g, 35.05 mmol, 32.70% yield) as a white solid.
LCMS (ESI) m/z: [M+H]⁺ = 286.0.
¹H NMR (400 MHz, DMSO-d6) δ= 7.48 - 7.25 (m, 5H), 6.80 (s, 1H), 5.13 (s, 2H), 4.54 (br s, 2H), 3.67 (br s, 2H), 2.75-2.72 (m,2H).

### Step 2: Benzyl 3-chloro-7,8-dihydropyrido[4,3-c]pyridazine-6(5H)-carboxylate

To a mixture of benzyl 3-oxo-3,5,7,8-tetrahydropyrido[4,3-*c*]pyridazine-6(2*H*)-carboxylate (9 g, 31.55 mmol) in POCl₃ (38.61g, 251.81 mmol, 23.40 mL) was added Et₃N (65.43 mg, 646.61 umol) in one portion at 25°C under N₂. The mixture was stirred at 80 °C 3 hours. The mixture was poured into water (2000 mL, 25°C) and stirred for 5 min. Sat. NaHCO₃ was added to the mixture to adjust pH=7. The aqueous phase was extracted with EA (1000 mL*2). The combined organic phase was washed with brine (1000mL), dried with anhydrous Na₂SO₄, filtered and concentrated under vacuum. The residue was purified by silica gel chromatography (Petroleum ether/Ethyl acetate=3/1) to give the title compound (7.7 g, 25.35 mmol, 80.36% yield) as a yellow oil.
LCMS (ESI) m/z: [M+H]⁺ = 304.0.
¹H NMR (400 MHz, MeOD) δ = 7.75 - 7.63 (m, 1H), 7.46 - 7.28 (m, 5H), 5.23 - 5.15 (m, 2H), 4.82 - 4.74 (m, 2H),4.63 - 4.57 (m, 1H), 3.96 - 3.84 (m, 2H), 3.24 - 3.12 (m, 2H).

### Step 3: 6-Benzyl 3-methyl 7,8-dihydropyrido[4,3-c]pyridazine-3,6(5H)-dicarboxylate

To a solution of benzyl 3-chloro-7,8-dihydropyrido[4,3-*c*]pyridazine-6(5*H*)-carboxylate (7.5 g, 24.69 mmol) in DMSO (225 mL) was added Pd(OAc)₂ (277.18 mg, 1.23 mmol), K₂CO₃ (5.12 g, 37.04 mmol), 1,3-bis(dicyclohexylphosphino)propane bis(tetrafluoroborate) (1.51 g, 2.47 mmol) and MeOH (11.88 g, 370.69mmol, 15.00 mL) under N₂ at 25°C. The suspension was degassed under vacuum and purged with CO several times. The mixture was stirred under CO (15 psi) at 100°C for 1.5 hours. The combined mixture was poured into water (1500 mL) and extracted with ethyl acetate (500 mL*2). The combined organic phase was washed with brine (500mL*2), dried with anhydrous Na₂SO₄, filtered and concentrated under vacuum. The residue was purified by silica gel chromatography (Petroleum ether/Ethyl acetate=1/2) to give the title compound (3.3 g, 9.92 mmol, 40.16% yield) as a yellow oil. LCMS (ESI) m/z: [M+H]⁺ = 327.9.
¹H NMR (400 MHz, DMSO-d6) δ = 8.21 - 8.12 (m, 1H), 7.43 - 7.28 (m, 5H), 5.17 - 5.10 (m, 2H), 4.89 - 4.68 (m, 2H), 3.99 -3.90 (m, 3H), 3.86 - 3.75 (m, 2H), 3.26 - 3.13 (m, 2H).

### Step 4: 6-(tert-Butyl) 3-methyl 7,8-dihydropyrido[4,3-c]pyridazine-3,6(5H)-dicarboxylate

To a solution of 6-benzyl 3-methyl 7,8-dihydropyrido[4,3-*c*]pyridazine-3,6(5*H*)-dicarboxylate (3.2 g, 9.78 mmol) in MeOH (65 mL) was added Boc₂O (3.20 g, 14.66 mmol, 3.37 mL) and Pd/C (300 mg, 10% purity) under N₂. The suspension was degassed and purged with H₂ several times. The mixture was stirred under H₂ (15 psi) at 25°C for 2 hours. The reaction mixture was filtered and the filtrate was concentrated to give the title compound (2.9 g, crude) as a light yellow oil, which was used directly in the next step.
LCMS (ESI) m/z: [M+H]⁺ = 294.0.
¹H NMR (400 MHz, CDCl₃) δ = 7.89 - 7.84 (m, 1H), 4.66 - 4.60 (m, 2H), 3.79 - 3.72 (m, 2H), 3.32 - 3.21 (m, 2H),1.44 - 1.42 (m, 9H).

### Step 5: tert-Butyl 3-(hydroxymethyl)-7,8-dihydropyrido[4,3-c]pyridazine-6(5H)-carboxylate

To a mixture of 6-(*tert*-butyl) 3-methyl 7,8-dihydropyrido[4,3-*c*]pyridazine-3,6(5H)-dicarboxylate (2.8 g, 9.55 mmol) and CaCl₂ (4.24 g, 38.18 mmol) in MeOH (28 mL)/THF (14 mL) was added NaBH₄ (722.29 mg, 19.09 mmol) in one portion at 0°C under N₂. The mixture was stirred at 25 °C for 1 hour. The mixture was poured into sat. NH₄Cl (100 mL) and extracted with ethyl acetate (100 mL*2). The combined organic phase was washed with brine (100 mL*1), dried with anhydrous Na₂SO₄, filtered and concentrated under vacuum. The residue was purified by reverse phase column (FA). The eluent was concentrated to remove MeCN and the aqueous phase was extracted with ethyl acetate (50 mL*2). The combined organic phase was washed with brine (50 mL*1), dried with anhydrousNa₂SO₄, filtered and concentrated to give the title compound (1.3 g, 4.90 mmol, 51.33% yield) as a light yellow oil.
LCMS (ESI) m/z: [M+H]⁺ = 266.0.
¹H NMR (400 MHz, MeOD) δ = 7.68 - 7.60 (m, 1H), 4.85 - 4.84 (m, 2H), 4.75 - 4.66 (m, 2H), 3.85 - 3.77 (m, 2H),3.21 - 3.08 (m, 2H), 2.03 - 1.99 (m, 2H), 1.51 - 1.49 (m, 9H).

### Step 6: tert-butyl 3-((1,3-dioxoisoindolin-2-yl)methyl)-7,8-dihydropyrido[4,3-c]pyridazine-6(5H)-carboxylate

To a mixture of tert-butyl 3-(hydroxymethyl)-7,8-dihydropyrido[4,3-c]pyridazine-6(5H)-carboxylate (1.15 g, 4.33 mmol), PPh₃ (1.71 g, 6.50 mmol) and isoindoline-1,3-dione (701.53 mg, 4.77 mmol) in THF (12 mL) was added DEAD (1.13 g, 6.50 mmol, 1.18 mL) dropwise at 0°C under N₂. The mixture was stirred at 25 °C for 2 hours. The combined mixture was poured into water (50 mL) and extracted with ethyl acetate (30 mL*2). The combined organic phase was washed with brine (30mL*1), dried with anhydrous Na₂SO₄, filtered and concentrated under vacuum. The residue was purified by silica gel chromatography (Petroleum ether/Ethyl acetate=1/1) to give the title compound (1.2 g, 3.04 mmol, 70.19% yield) as a yellow oil.
LCMS (ESI) m/z: [M+H]⁺ = 395.0.
¹H NMR (400 MHz, CDCl₃) δ = 7.85 - 7.75 (m, 2H), 7.71 - 7.65 (m, 2H), 7.13 - 7.08 (m, 1H), 5.14 - 5.08 (m, 2H),4.56 - 4.48 (m, 2H), 3.75 - 3.64 (m, 2H), 3.17 - 3.07 (m, 2H), 1.43 - 1.39 (m, 9H).

### Step 7: 2-((5,6,7,8-Tetrahydropyrido[4,3-c]pyridazin-3-yl)methyl)isoindoline-1,3-dione hydrochloride

To a mixture of *tert*-butyl 3-((1,3-dioxoisoindolin-2-yl)methyl)-7,8-dihydropyrido[4,3-*c*]pyridazine-6(5H)-carboxylate (950 mg, 2.41 mmol) in dioxane (5 mL) was added HCl/dioxane (4 M, 5 mL) in one portion at 25°C under N₂. The mixture was stirred at 25 °C for 30 min. The mixture was filtered and the solid was washed with MTBE (5mL) to give the title compound (360 mg, 1.09 mmol, 45.19% yield) as a yellow solid.
LCMS (ESI) m/z: [M+H]⁺ = 294.9.
¹H NMR (400 MHz, MeOD) δ = 8.11 - 8.04 (m, 1H), 7.96 - 7.81 (m, 4H), 5.25 (s, 2H), 4.62 (s, 2H), 3.77 - 3.68 (m, 2H), 3.57 - 3.49 (m, 2H).

### Intermediate 72: 2-Bromo-6-(1-((tert-butyldimethylsilyl)oxy)cyclopropyl)pyridine

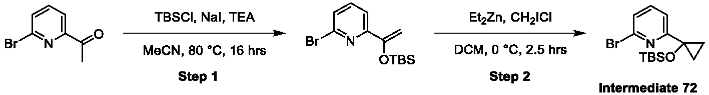

### Step 1: 2-Bromo-6-(1-((tert-butyldimethylsilyl)oxy)vinyl)pyridine

To a solution of 1-(6-bromo-2-pyridyl)ethanone (5 g, 25.00 mmol), TBSCI (15.07 g, 99.98 mmol, 12.25 mL) and Nal (14.99 g, 99.98 mmol) in MeCN (100 mL) was added TEA (11.38 g, 112.48 mmol, 15.66 mL). The mixture was stirred at 80 °C for 16 hrs. The reaction mixture was poured into water (500 mL) and extracted with EA (500 mL*3). The combined organic layer was washed with brine (800 mL), dried over Na₂SO₄, filtered and concentrated. The residue was purified by flash silica gel chromatography (ISCO^{®}; 40 g SepaFlash^{®} Silica Flash Column, Eluent of 0~10% Ethyl acetate/Petroleum ether gradient) to give the title compound (7.5 g, 23.79 mmol, 95.16% yield) as a yellow oil.
LCMS (ESI) m/z: [81BrM+H]+ = 316.1.
1H NMR (400 MHz, CDCl₃) δ = 7.53 (s, 2H), 7.37 - 7.35 (m, 1H), 5.69 (d, J = 1.2 Hz, 1H), 4.60 (d, J = 1.2 Hz, 1H), 1.01 (s, 9H), 0.25 (s, 6H) ppm.

### Step 2: 2-Bromo-6-(1-((tert-butyldimethylsilyl)oxy)cyclopropyl)pyridine

To a solution of diethylzinc (1 M, 38.18 mL) in DCM (180 mL) at 0 °C was added a solution of chloro(iodo)methane (13.47 g, 76.36 mmol, 5.54 mL) in DCM (60 mL) dropwise. The reaction mixture was stirred at 0 °C for 30 minutes. Then to the mixture was added a solution of 2-bromo-6-(1-((tertbutyldimethylsilyl)oxy)vinyl)pyridine (4 g, 12.73 mmol) in DCM (180 mL). The reaction mixture was stirred at 0 °C for 2 hrs. The reaction mixture was poured into aq. NH₄Cl (200 mL) and extracted with DCM (100 mL*3). The combined organic layer was washed with brine (400 mL), dried over Na₂SO₄, filtered and concentrated. The residue was purified by flash silica gel chromatography (ISCO^{®}; 120 g SepaFlash^{®} Silica Flash Column, Eluent of 0~10% Ethyl acetate/Petroleum ether gradient) to give the title compound (700 mg, 2.13 mmol, 16.75% yield) as a colorless solid.
LCMS (ESI) m/z: [79BrM+H]+ = 328.1.
1H NMR (400 MHz, CDCl₃) δ = 7.60 - 7.58 (m, 1H), 7.49 - 7.45 (m, 1H), 7.23 - 7.21 (m, 1H), 1.45 - 1.39 (m, 2H), 1.27 - 1.23 (m, 2H), 0.95 (s, 9H), 0.10 (s, 6H) ppm.

### Intermediate 73: 6-Phenyl-2,7-naphthyridine-3-carbonitrile

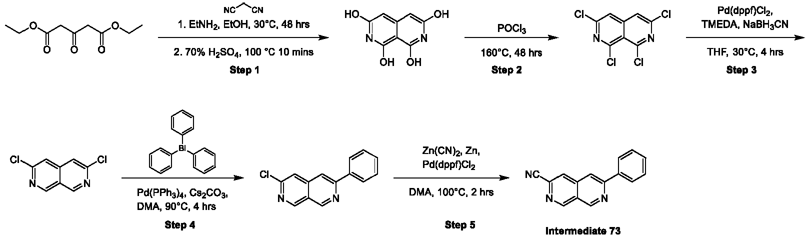

### Step 1: 2,7-Naphthyridine-1,3,6,8-tetraol

To a solution of propanedinitrile (5.39 g, 81.60 mmol, 5.13 mL) in EtOH (70 mL) was added diethyl 3-oxopentanedioate (15 g, 74.18 mmol, 13.51 mL) and ethylamine (167.21 mg, 3.71 mmol, 242.68 uL). The mixture was stirred at 30 °C for 48 hrs. The reaction mixture was concentrated under reduced pressure. The residue was added to H₂SO₄ (70%, 20 mL) and stirred at 100 °C for 10 mins. The mixture was cooled to 20 °C and quenched by pouring slowly into ice water (180 mL), generating a yellow solid. The solid was collected and dried to give the title compound (14 g, 72.11 mmol, 97.21% yield).
LCMS (ESI) m/z: [M+H]+ = 195.0.
1HNMR (400 MHz, DMSO-d6) δ = 11.38 (s, 2H), 5.79 (s, 2H) ppm.

### Step 2: 1,3,6,8-Tetrachloro-2,7-naphthyridine

A mixture of 2,7-naphthyridine-1,3,6,8-tetraol (5 g, 25.75 mmol) and POCl₃ (49.50 g, 322.83 mmol, 30.00 mL) was stirred at 160 °C for 48 hrs. The reaction mixture was quenched by addition of sat. NaHCO3 (1000 mL) and extracted with ethyl acetate (300 mL *3). The combined organic phase was washed with brine (100 mL *3), dried over Na₂SO₄, filtered and concentrated under reduced pressure. The residue was purified by column chromatography (SiO₂, Petroleum ether/Ethyl acetate=10/1) to give the title compound (1.6 g, 5.97 mmol, 23.19% yield) as a yellow solid.
LCMS (ESI) m/z: [M+H]+ = 268.9.
1HNMR (400 MHz, CDCl₃) δ = 7.58 (s, 2H) ppm.

### Step 3: 3,6-Dichloro-2,7-naphthyridine

A mixture of 1,3,6,8-tetrachloro-2,7-naphthyridine (1 g, 3.73 mmol), NaBH₃CN (1.17 g, 18.66 mmol), Pd(dppf)Cl₂ (273.10 mg, 373.24 umol) and TMEDA (1.08 g, 9.33 mmol, 1.41 mL) in THF (10 mL) was degassed and purged with N₂ 3 times. The mixture was stirred at 30 °C for 4 hrs under N₂ atmosphere. The reaction mixture was quenched by addition of water (30 mL), and extracted with ethyl acetate (30 mL *3). The combined organic phase was washed with brine (10 mL *3), dried over Na₂SO₄, filtered and concentrated under reduced pressure. The residue was purified by column chromatography (SiO₂, Petroleum ether/Ethyl acetate=5/1) to give the title compound (0.6 g, 2.83 mmol, 75.92% yield) as a yellow solid.
LCMS (ESI) m/z: [M+H]+ = 199.0.
1HNMR (400 MHz, CDCl₃) δ = 9.30 - 9.20 (m, 2H), 7.68 (m, 2H) ppm.

### Step 4: 3-Chloro-6-phenyl-2,7-naphthyridine

A mixture of 3,6-dichloro-2,7-naphthyridine (150 mg, 753.63 umol), triphenylbismuth (109.50 mg, 248.70 umol), Cs₂CO₃ (982.19 mg, 3.01 mmol) and Pd(PPh₃)₄ (87.09 mg, 75.36 umol) in DMA (5 mL) was degassed and purged with N₂ 3 times. The mixture was stirred at 90 °C for 4 hrs under N₂ atmosphere. The reaction was quenched by adding water (15 mL) and the mixture was extracted with ethyl acetate (20 mL*3). The combined organic layers were washed with brine (10 mL *3), dried over Na₂SO₄, filtered and concentrated under vacuum The residue was purified by column chromatography (SiO₂, Petroleum ether/Ethyl acetate=1/1) to give the title compound (160 mg, 664.76 umol, 88.21% yield) as a yellow solid.
LCMS (ESI) m/z: [M+H]+ = 241.1.

### Step 5: 6-Phenyl-2,7-naphthyridine-3-carbonitrile

A mixture of 3-chloro-6-phenyl-2,7-naphthyridine (50 mg, 207.74 umol), Pd(dppf)Cl₂ (15.20 mg, 20.77 umol), Zn (2.72 mg, 41.55 umol) and Zn(CN)₂ (48.79 mg, 415.48 umol, 26.37 uL) in DMA (3 mL) was degassed and purged with N₂ for 3 times. The mixture was stirred at 100 °C for 2 hrs under N₂ atmosphere. The reaction mixture was quenched by addition of water (10 mL), and extracted with ethyl acetate (10 mL *3). The combined organic phase was washed with brine (10 mL), dried over Na₂SO₄, filtered and concentrated under reduced pressure. The residue was purified by prep-TLC (SiO₂, Petroleum ether/Ethyl acetate=1/1) to give the title compound (35 mg, 148.32 umol, 71.40% yield) as a yellow oil.
LCMS (ESI) m/z: [M+H]+ = 232.1.
1HNMR (400 MHz, CDCl3) δ = 9.61 (s, 1H), 9.46 (s, 1H), 8.22 - 8.17 (m, 3H), 8.12 (s, 1H), 7.60 - 7.49 (m, 3H) ppm.

### Example 2. Methyl (R)-3-((2-((4-cyano-4-methylchromane-6-carboxamido)methyl)pyridin-4-yl)ethynyl)benzoate

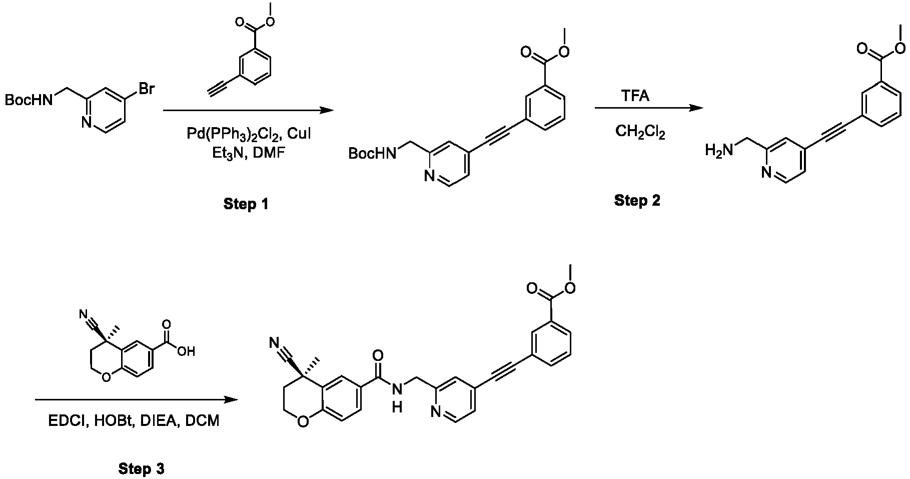

### Step 1. Methyl 3-[2-[2-[(tert-butoxycarbonylamino)methyl]-4-pyridyl]ethynyl]benzoate

Pd(PPh₃)₂Cl₂ (73.3 mg, 0.10 mmol) and methyl 3-ethynylbenzoate (669 mg, 4.18 mmol) were added to a solution of tert-butyl N-[(4-bromo-2-pyridyl)methyl]carbamate (300 mg, 1.04 mmol), Cul (19.9 mg, 0.10 mmol), and Et₃N (0.44 mL, 3.13 mmol) in DMF (3 mL). The mixture was stirred at 100°C for 1 hr. The mixture was filtered. The filtered liquid was diluted with water (20 mL) and extracted with EA (20 mL x 2). The combined organic layer was dried with anhydrous Na₂SO₄ and concentrated to afford aresidue. The residue was purified by column chromatography (SiO₂, Petroleum ether/Ethyl acetate=1/0 to 0/1). The eluent was concentrated to give the title compound (300 mg, 0.54 mmol) as colorless oil. LCMS (ESI) m/z: [M+H]+ = 367.1.

### Step 2. Methyl 3-[2-[2-(aminomethyl)-4-pyridyl]ethynyl]benzoate

TFA (0.24 mL, 3.28 mmol) was added to a solution of methyl 3-[2-[2-[(tert-butoxycarbonylamino)methyl]-4-pyridyl]ethynyl]benzoate (120 mg, 0.33 mmol) in DCM (1 mL). The mixture was stirred at 25°C for 1 hr. The mixture was diluted with water (3 mL) and extrated with DCM (3 mL x 2). The combined organic layer was discarded. The aqueous layer was adjusted to pH=10 with saturated NaHCO₃ solution and extracted with DCM (3 mL x 2). The combined organic layer was dried with anhydrous Na₂SO₄ and concentrated affording the title compound (40 mg, 0.14 mmol) as a white solid which was used into the next step without further purification.
LCMS (ESI) m/z: [M+H]+ = 267.1.

### Step 3. Methyl 3-[2-[2-[[[(4R)-4-cyano-4-methyl-chromane-6-carbonyl]amino]methyl]-4-pyridyl]ethynyl]benzoate

HOBt (30.4 mg, 0.23 mmol), EDCI (43.2 mg, 0.23 mmol), and DIPEA (0.078 mL, 0.45 mmol) were added to a solution of methyl 3-[2-[2-(aminomethyl)-4-pyridyl]ethynyl]benzoate (40 mg, 0.15 mmol) and (4R)-4-cyano-4-methyl-chromane-6-carboxylic acid (35.9 mg, 0.17 mmol) in DCM (0.5 mL). The mixture was stirred at 25°C for 1 hr. The reaction mixture was diluted with water (10 mL) and extracted with DCM (10 mL x 2). The combined organic layers were dried with anhydrous Na₂SO₄ and concentrated to afford a residue. The residue was purified by prep-HPLC (FA condition) (column: 3_Phenomenex Luna C18 75*30mm*3um; mobile phase: [water (0.225%FA)-ACN]; B%: 45%-75%, 7min). The eluent was lyophilized to afford the title compound (23.5 mg, 0.051 mmol) as a white solid. LCMS (ESI) m/z: [M+H]+ = 466.2.
¹H NMR (400 MHz, DMSO-d6) δ = 9.15 - 9.12 (m, 1H), 8.60 - 8.58 (m, 1H), 8.12 (d, J = 2.0 Hz, 2H), 8.03 - 8.01 (m, 1H), 7.89 - 7.86 (m, 2H), 7.63 - 7.59 (m, 1H), 7.47 - 7.44 (m, 2H), 6.95 (d, J = 8.4 Hz, 1H), 4.60 (d, J = 4.4 Hz, 2H), 4.35 - 4.33 (m, 2H), 3.87 (s, 3H), 2.47 - 2.41 (m, 1H), 2.23 - 2.21 (m, 1H), 1.79 (s, 3H) ppm.

The following examples in Table 4 were prepared using standard chemical manipulations and procedures similar to those used for the preparation of **Example 2.**

**Table 4. Compounds of the Invention**

| **#** | **LCMS (m/z)** | **1H NMR** |
|---|---|---|
| **1** | 408.1 | 1H NMR (400MHz, DMSO-d6) δ = 9.28 - 9.24 (m, 1H), 8.59 - 8.57 (m, 1H), 8.15 (d, J = 1.6 Hz, 1H), 7.91 - 7.88 (m, 1H), 7.65 - 7.56 (m, 2H), 7.52 - 7.40 (m, 5H), 7.28 (d, J = 8.0 Hz, 1H), 4.95 - 4.77 (m, 2H), 4.61 (d, J = 4.4 Hz, 2H), 4.21 (d, J = 11.6 Hz, 1H), 3.86 (d, J = 11.6 Hz, 1H), 1.69 (s, 3H) ppm. |
| **2** | 438.0 | 1H NMR (400 MHz, DMSO-d6) δ = 9.33 - 9.21 (m, 1H), 8.58 (d, J = 4.8 Hz, 1H), 8.15 (d, J = 1.6 Hz, 1H), 7.95 - 7.85 (m, 1H), 7.47 - 7.40 (m, 2H), 7.38 - 7.32 (m, 1H), 7.28 (d, J = 8.0 Hz, 1H), 7.20 - 7.13 (m, 2H), 7.07 - 7.01 (m, 1H), 4.93 - 4.79 (m, 2H), 4.66 - 4.57 (m, 2H), 4.21 (d, J = 11.2 Hz, 1H), 3.86 (d, J = 11.6 Hz, 1H), 3.78 (s, 3H), 1.69 (s, 3H) ppm. |
| **3** | 466.1 | 1H NMR (400 MHz, DMSO-d6) δ = 9.35 - 9.21 (m, 1H), 8.60 (d, J = 4.4 Hz, 1H), 8.21 - 8.10 (m, 2H), 8.06 - 7.98 (m, 1H), 7.95 - 7.81 (m, 2H), 7.66 - 7.58 (m, 1H), 7.52 - 7.45 (m, 2H), 7.29 (d, J = 8.0 Hz, 1H), 4.95 - 4.78 (m, 2H), 4.62 (d, J = 5.6 Hz, 2H), 4.21 (d, J = 11.6 Hz, 1H), 3.90 - 3.82 (m, 4H), 1.69 (s, 3H). ppm. |
| **5** | 473.2 | 1H NMR (400 MHz, DMSO-d6) δ = 9.25 - 9.23 (m, 1H), 8.51 - 8.49 (m, 1H), 8.14 - 8.13 (m, 1H), 7.90 - 7.88 (m, 1H), 7.32 - 7.20 (m, 3H), 4.94 - 4.77 (m, 2H), 4.58 - 4.56 (m, 2H), 4.22 - 4.20 (m, 1H), 3.88 - 3.84 (m, 1H), 3.75 - 3.62 (m, 1H), 3.54 (s, 3H), 3.48 - 3.41 (m, 1H), 3.29 (s, 2H), 2.84 - 2.74 (m, 1H), 1.98 - 1.85 (m, 1H), 1.68 (s, 3H), 1.67 - 1.56 (m, 2H), 1.48 - 1.34 (m, 1H) ppm |
| **13** | 412.9 | 1H NMR (400 MHz, DMSO-d6) δ = 9.22 - 9.19 (m, 1H), 8.58 (d, J = 4.8 Hz, 1H), 7.93 (m, 1H), 7.77 (s, 1H), 7.61 - 7.59 (m, 2H), 7.55 - 7.54 (m, 1H), 7.48 - 7.42 (m, 6H), 6.67 - 6.39 (m, 1H), 4.95 - 4.90 (m, 4H), 4.61 (m, 2H) ppm. |
| **14** | 401.2 | 1H NMR (400 MHz, DMSO-d6) δ = 9.26 - 9.24 (m, 1H), 8.58 (d, J = 4.8 Hz, 1H), 8.22 (s, 1H), 7.93 (m, 1H), 7.60 - 7.59 (m, 2H), 7.45 - 7.42 (m, 5H), 7.29 (m, 1H), 4.86 - 4.82 (m, 1H), 4.74 - 4.73 (m, 1H), 4.62 - 4.59 (m, 2H), 4.06 - 4.03 (m, 1H), 3.87 - 3.76 (m, 1H), 1.70 - 1.65 (m, 3H) ppm. |
| **15** | 422.3 | 1H NMR (400 MHz, MeOD-d4) δ = 9.24 (m, 1H), 8.50 (s, 1H), 8.14 (d, J = 1.2 Hz, 1H), 7.89 (m, 1H), 7.64 - 7.57 (m, 2H), 7.53 - 7.43 (m, 3H), 7.39 (s, 1H), 7.28 (d, J = 8.0 Hz, 1H), 4.92 - 4.79 (m, 2H), 4.57 (m, 2H), 4.21 (d, J = 11.6 Hz, 1H), 3.85 (d, J = 11.6 Hz, 1H), 2.43 (s, 3H), 1.77 - 1.61 (m, 3H) ppm. |
| **16** | 426.3 | 1H NMR (400 MHz, MeOD-d4) δ = 8.41 - 8.25 (m, 1H), 8.16 - 8.03 (m, 1H), 7.92 - 7.79 (m, 1H), 7.65 - 7.55 (m, 2H), 7.54 - 7.37 (m, 4H), 7.31 - 7.18 (m, 1H), 4.96 - 4.89 (m, 2H), 4.84 - 4.77 (m, 2H), 4.29 - 4.12 (m, 1H), 4.03 - 3.83 (m, 1H), 1.75 (s, 3H) ppm. |
| **17** | 458.1 | 1H NMR (400 MHz, DMSO-d6) δ = 9.29 - 9.27 (m, 1H), 8.60 (d, J = 4.8 Hz, 1H), 8.15 (d, J = 1.2 Hz, 1H), 7.91 - 7.89 (m, 1H), 7.80 - 7.78 (m, 2H), 7.65 - 7.64 (m, 1H), 7.63 - 7.56 (m, 1H), 7.47 - 7.45 (m, 2H), 7.29 (d, J = 8.0 Hz, 1H), 7.27 - 6.91 (m, 1H), 4.91 - 4.84 (m, 2H), 4.62 (m, 2H), 4.22 (d, J = 11.2 Hz, 1H), 3.87 (d, J = 11.2 Hz, 1H), 1.68 (s, 3H) ppm. |
| **20** | 426.3 | 1H NMR (400 MHz, MeOD-d4) δ = 8.48 (d, J = 1.2 Hz, 1H), 8.12 (d, J = 1.6 Hz, 1H), 7.87 (m, 1H), 7.61 - 7.53 (m, 3H), 7.49 - 7.38 (m, 3H), 7.26 (d, J = 8.0 Hz, 1H), 4.90 (d, J = 3.6 Hz, 2H), 4.69 (s, 2H), 4.19 (d, J = 11.6 Hz, 1H), 3.92 (d, J = 11.6 Hz, 1H), 1.75 (s, 3H) ppm. |
| **21** | 473.3 | 1H NMR (400 MHz, MeOD-d4) δ = 8.45 (d, J = 5.6 Hz, 1H), 8.11 (d, J = 1.6 Hz, 1H), 7.90 - 7.83 (m, 1H), 7.37 (s, 1H), 7.30 - 7.23 (m, 2H), 4.90 (d, J = 3.6 Hz, 2H), 4.67 (s, 2H), 4.20 (d, J = 11.2 Hz, 1H), 3.92 (d, J = 11.6 Hz, 1H), 3.83 - 3.76 (m, 2H), 3.68 (s, 3H), 3.30 - 3.19 (m, 2H), 2.96 - 2.84 (m, 1H), 1.96 - 1.83 (m, 2H), 1.75 (s, 3H), 1.69 - 1.58 (m, 2H) ppm. |
| **22** | 459.2 | 1H NMR (400 MHz, DMSO-d6) δ = 9.23 - 9.20 (m, 1H), 8.50 (d, J = 5.2 Hz, 1H), 8.13 (d, J = 1.6 Hz, 1H), 7.89 - 7.87 (m, 1H), .32 - 7.24 (m, 3H), 4.95 - 4.78 (m, 2H), 4.57 (d, J = 4.8 Hz, 2H), 4.21 (d, J = 11.2 Hz, 1H), 3.86 (d, J = 11.6 Hz, 1H), 3.66 - 3.60 (m, 1H), 3.58 (s, 3H), 3.45 - 3.40 (m, 1H), 3.35 (s, 1H), 3.30 - 3.25 (m, 2H), 2.22 - 2.17 (m, 1H), 2.06 - 1.86 (m, 1H), 1.68 s, 3H) ppm. |
| **23** | 457.2 | 1H NMR (400 MHz, MeOD-d4) δ = 8.57 (d, J = 5.2 Hz, 1H), 8.19 (d, J = 1.6 Hz, 2H), 8.08 - 8.05 (m, 1H), 7.83 - 7.74 (m, 2H), 7.62 - 7.59 (m, 1H), 7.57 - 7.50 (m, 2H), 7.17 (d, J = 8.0 Hz, 1H), 4.82 (d, J = 3.2 Hz, 2H), 4.74 (s, 2H), 3.93 (s, 3H), 3.81 - 3.72 (m, 2H), 1.55 (s, 3H) ppm. |
| **37** | 452.2 | 1H NMR (400 MHz, DMSO-d6) δ = 9.11 - 9.08 (m, 1H), 8.59 (d, J = 4.8 Hz, 1H), 8.12 (d, J = 2.0 Hz, 2H), 8.03 - 8.01 (m, 1H), 7.95 - 9.92 (m, 1H), 7.89 - 7.87 (m, 1H), 7.63 - 7.59 (m, 1H), 7.48 - 7.46 (m, 2H), 7.05 (d, J = 8.8 Hz, 1H), 5.01 (d, J = 9.6 Hz, 1H), 4.60 (d, J = 6.0 Hz, 2H), 4.55 (d, J = 9.6 Hz, 1H), 3.87 (s, 3H), 1.76 (s, 3H) ppm |
| **39** | 465.3 | 1H NMR (400 MHz, DMSO+D2O) δ = 8.89 - 8.88 (m, 1H), 8.52 (d, J = 5.2 Hz, 1H), 8.06 (s, 1H), 7.99 (d, J = 8.4 Hz, 1H), 7.82 - 7.80 (m, 2H), 7.58 - 7.55 (m, 2H), 7.423(d, J = 5.2 Hz, 1H), 7.36 (s, 1H), 6.57 (d, J = 8.8 Hz, 1H), 4.51 (br s, 2H), 3.82 (s, 3H), 3.31 - 3.28 (m, 2H), 2.18 - 2.12 (m, 1H), 1.95 - 1.90 (m, 1H), 1.66 (s, 3H) ppm |
| **43** | 452.2 | 1H NMR (400MHz, DMSO-d6) δ = 9.00 (d, J = 8.0 Hz, 1H), 8.62 (d, J = 5.2 Hz, 1H), 8.17 (d, J = 1.6 Hz, 1H), 7.93 - 7.91 (m, 1H), 7.63 - 7.60 (m, 3H), 7.49 - 7.44 (m, 4H), 7.28 (d, J = 8.0 Hz, 1H), 5.44 - 5.38 (m, 1H), 4.92 - 4.81 (m, 2H), 4.22 (d, J = 11.6 Hz, 1H), 3.88 - 3.81 (m, 3H), 3.31 (s, 3H), 1.70 (s, 3H) ppm |
| **47** | 437.2 | 1H NMR (400 MHz, MeOD-d4) δ = 8.52 (d, J = 5.2 Hz, 1H), 8.21 (s, 1H), 8.02 - 7.94 (m, 1H), 7.60 - 7.48 (m, 3H), 7.45 - 7.33 (m, 5H), 6.54 - 6.21 (m, 1H), 4.84 - 4.78 (m, 2H), 4.71 (s, 2H), 4.34 - 4.26 (m, 1H), 4.15 - 4.04 (m, 1H) ppm |
| **48** | 437.2 | 1H NMR (400 MHz, MeOD-d4) δ = 8.52 (d, J = 5.2 Hz, 1H), 8.21 (s, 1H), 7.98 (d, J = 8.4 Hz, 1H), 7.59 - 7.47 (m, 3H),7.45 - 7.33 (m, 5H), 6.54 - 6.21 (m, 1H), 4.80 (s, 2H), 4.71 (s, 2H), 4.34 - 4.27 (m, 1H), 4.14 - 4.04 (m, 1H) ppm |

### Example 3. (4R)-4-Cyano-4-methyl-N-[[4-(2-phenyl-2,7-diazaspiro[3.4]octan-7-yl)-2-pyridyl]methyl]isochromane-6-carboxamide

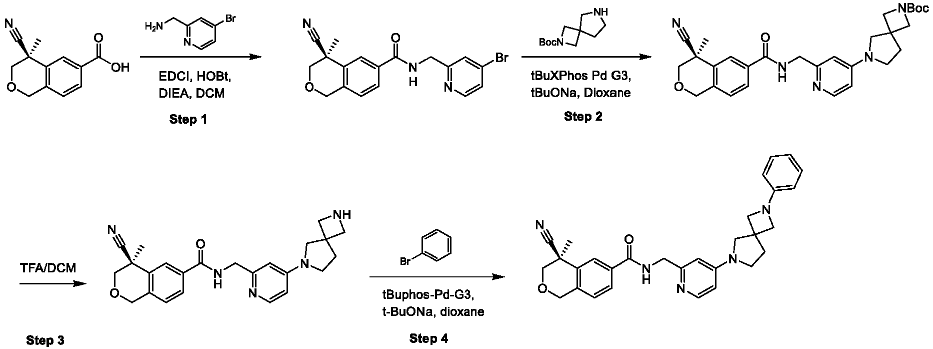

### Step 1. (4R)-N-[(4-Bromo-2-pyridyl)methyl]-4-cyano-4-methyl-isochromane-6-carboxamide

EDCI (1.54 g, 8.02 mmol), HOBt (1.08 g, 8.02 mmol), DIEA (4.66 mL, 26.7 mmol), and (4-bromo-2-pyridyl)methanamine (1 g, 5.35 mmol, HCl) were added to a solution of (4R)-4-cyano-4-methyl-isochromane-6-carboxylic acid (1.28 g, 5.88 mmol) in DCM (10 mL). The reaction mixture was stirred at 25°C for 2 hrs. The mixture was diluted with H₂O (40 mL), and then extracted with DCM (40 mL x 2). The combined organic layer was dried over anhydrous Na₂SO₄, filtered, and concentrated under reduced pressure to afford a residue. The residue was purified by flash silica gel chromatography SiO₂, Petroleum ether/Ethyl Acetate=1/0 to 1/10) affording the title compound (1.2 g, 3.10 mmol) as white a solid.
LCMS (ESI) m/z: [81BrM+H]+ = 388.0.
¹HNMR (400 MHz, CDCl₃) δ = 8.39 (d, J = 5.6 Hz, 1H), 8.01 (d, J = 1.6 Hz, 1H), 7.78 - 7.76 (m, 1H), 7.56 - 7.49 (m, 2H), 7.42 - 7.40 (m, 1H), 7.13 (d, J = 8.0 Hz, 1H), 4.87 (s, 2H), 4.74 (d, J = 5.2Hz, 2H), 4.14 - 4.11 (m, 1H), 3.94 (d, J = 11.2 Hz, 1H), 1.76 (s, 3H) ppm.

### Step 2. tert-Butyl 7-[2-[[[(4R)-4-cyano-4-methyl-isochromane-6-carbonyl]amino]methyl]-4-pyridyl]-2,7-diazaspiro[3.4]octane-2-carboxylate

[2-(2-aminophenyl)phenyl]-methylsulfonyloxy-palladium;ditert-butyl-[2-(2,4,6-triisopropylphenyl)phenyl]phosphane (41.1 mg, 51.8 µmol) and tBuONa (149.3 mg, 1.55 mmol) were added to a solution of (4R)-N-[(4-bromo-2-pyridyl)methyl]-4-cyano-4-methyl-isochromane-6-carboxamide (200 mg, 0.52 mmol) and tert-butyl 2,7-diazaspiro[3.4]octane-2-carboxylate (220 mg, 1.04 mmol) in dioxane (4 mL). The mixture was stirred at 70°C for 2 hrs. The reaction mixture was poured into water (80 mL) and extracted with EA (30 mL x 3). The combined organic layer was washed with brine (30 mL), dried over anhydrous Na₂SO₄, filtered, and concentrated. The crude product was purified by reversed-phase HPLC (0.1% FA condition). The fractions containing product were combined and extracted with EA (20 mL x 3). The combined organic layers were dried over anhydrous Na₂SO₄, filtered, and concentrated affording the title compound (80 mg, 0.11 mmol) as yellow oil.
LCMS (ESI) m/z: [M+H]+ = 518.2.

### Step 3. (4R)-4-Cyano-N-[[4-(2,6-diazaspiro[3.4]octan-6-yl)-2-pyridyl]methyl]-4-methyl-isochromane-6-carboxamide

TFA (0.5 mL) was added to a solution of tert-Butyl 7-[2-[[[(4R)-4-cyano-4-methyl-isochromane-6-carbonyl]amino]methyl]-4-pyridyl]-2,7-diazaspiro[3.4]octane-2-carboxylate (80 mg, 0.11 mmol) in DCM (1 mL). The mixture was stirred at 25°C for 1 hr. The reaction mixture was concentrated in vacuo. The crude residue was triturated with a mixture of PE (2 mL) and MTBE (1 mL). The residue was filtered and dried in vacuo affording the title compound (70 mg, TFA) as a yellow oil which was used directly for next step.
LCMS (ESI) m/z: [M+H]+ = 418.2.

### Step 4. (4R)-4-Cyano-4-methyl-N-[[4-(2-phenyl-2,7-diazaspiro[3.4]octan-7-yl)-2-pyridyl]methyl]isochromane-6-carboxamide

A mixture of (4R)-4-cyano-N-[[4-(2,6-diazaspiro[3.4]octan-6-yl)-2-pyridyl]methyl]-4-methyl-isochromane-6-carboxamide (65 mg, 0.12 mmol, TFA), bromobenzene (57.6 mg, 0.37 mmol, 39 µL), [2-(2-aminophenyl)phenyl]-methylsulfonyloxy-palladium;ditert-butyl-[2-(2,4,6-triisopropylphenyl)phenyl]phosphane (9.7 mg, 12 µmol), and t-BuONa (58.8 mg, 0.61 mmol) in dioxane (1.5 mL) was degassed and purged with N₂ three times. The reaction mixture was then stirred at 70°C for 3 hrs. The reaction mixture was then poured into water (80 mL) and extracted with EA (30 mL x 3). The combined organic layers were washed with brine (30 mL), dried over Na₂SO₄, filtered, and concentrated. The residue was purified by prep-HPLC (column: Waters Xbridge C18 150*50mm* 10um; mobile phase: [water (10mM NH4HCO3)-ACN]; B%: 46%-66%, 10min) and the fraction was lyophilized to give the title compound (3.62 mg, 7.13 µmol, 5.83% yield) as a white solid.
LCMS (ESI) m/z: [M+H]+ = 494.2.
¹HNMR (400 MHz, DMSO-d6) δ = 9.17 - 9.07 (m, 1H), 8.12 (d, J = 1.6 Hz, 1H), 8.07 (d, J = 6.0 Hz, 1H), 7.91 - 7.83 (m, 1H), 7.26 (d, J = 8.0 Hz, 1H), 7.20 - 7.12 (m, 2H), 6.72 - 6.64 (m, 1H), 6.52 - 6.39 (m, 4H), 4.92 - 4.78 (m, 2H), 4.52 - 4.42 (m, 2H), 4.20 (d, J = 11.6 Hz, 1H), 3.85 (d, J = 11.6 Hz, 1H), 3.77 (s, 4H), 3.54 (s, 2H), 3.40 - 3.35 (m, 2H), 2.27 - 2.18 (m, 2H), 1.67 (s, 3H) ppm.

The following examples in Table 5 were prepared using standard chemical manipulations and procedures similar to those used for the preparation of **Example 3.**

**Table 5. Compounds of the Invention**

| **#** | **LCMS (m/z)** | **1H NMR** |
|---|---|---|
| **10** | 480.2 | 1H NMR (400 MHz, DMSO-d6) δ = 9.16 - 9.06 (m, 1H), 8.13 - 8.05 (m, 2H), 7.89 - 7.83 (m, 1H), 7.26 (d, J = 8.0 Hz, 1H), 7.20 - 7.12 (m, 2H), 6.71 - 6.64 (m, 1H), 6.42 (d, J = 7.8 Hz, 2H), 6.36 - 6.27 (m, 2H), 4.92 - 4.77 (m, 2H), 4.51 - 4.37 (m, 2H), 4.20 (d, J = 11.6 Hz, 1H), 4.09 (s, 4H), 3.96 (s, 4H), 3.84 (d, J = 11.6 Hz, 1H), 1.67 (s, 3H) ppm. |
| **12** | 494.3 | 1H NMR (400 MHz, MeOD-d4) δ = 9.11 - 9.08 (m, 1H), 8.12 (d, J = 1.6 Hz, 1H), 8.08 (d, J = 5.6 Hz, 1H), 7.87 - 7.85 (m, 1H), 7.26 (d, J = 8.0 Hz, 1H), 7.16 - 7.12 (m, 2H), 6.60 - 6.57 (m, 1H), 6.51 (d, J = 8.0 Hz, 2H), 6.32 (d, J = 2.0 Hz, 1H), 6.29 - 6.27 (m, 1H), 4.93 - 4.77 (m, 2H), 4.52 - 4.35 (m, 2H), 4.20 (d, J = 11.2 Hz, 1H), 3.94 - 3.81 (m, 4H), 3.45 (s, 2H), 3.30 - 3.27 (m, 2H), 2.23 - 2.20 (m, 2H), 1.67 (s, 3H) ppm. |
| **28** | 439.3 | 1H NMR (400 MHz, MeOD-d4) δ = 8.14 - 8.09 (m, 2H), 7.86 - 7.84 (m, 1H), 7.26 (d, J = 8.0 Hz, 1H), 7.23 - 7.16 (m, 4H), 6.88 (d, J = 2.4 Hz, 1H), 6.80 - 6.77 (m, 1H), 4.89 (d, J = 3.2 Hz, 2H), 4.60 (d, J = 2.4 Hz, 2H), 4.53 (s, 2H), 4.19 (d, J = 11.2 Hz, 1H), 3.92 (d, J = 11.2 Hz, 1H), 3.65 - 3.62 (m, 2H), 2.97 - 2.94 (m, 2H), 1.75 (s, 3H) ppm. |
| **31** | 425.2 | 1H NMR (400 MHz, DMSO-d6) δ = 9.16 (t, J = 5.6 Hz, 1H), 8.18 - 8.14 (m, 3H), 7.89 - 7.88 (m, 1H), 7.40 - 7.38 (m, 2H), 7.32 -7.30 (m, 3H), 6.60 (d, J = 2.4 Hz, 1H), 6.54 - 6.53 (m, 1H), 4.91 - 4.84 (m, 2H), 4.67 - 4.64 (m, 4H), 4.53 - 4.48 (m, 2H), 4.22 (d, J = 11.2 Hz, 1H), 3.87 (d, J = 11.6 Hz, 1H), 1.69 (s, 3H) ppm |
| **33** | 439.4 | 1H NMR (400 MHz, MeOD) δ = 8.11 (d, J = 1.6 Hz, 1H), 8.05 (d, J = 6.0 Hz, 1H), 7.87-7.84 (m, 1H), 7.42 - 7.32 (m, 4H), 7.30 - 7.22 (m, 2H), 6.59 - 6.43 (m, 2H), 4.67 - 4.55 (m, 4H), 4.54-4.49 (m, 2H), 4.18 (d, J = 11.6 Hz, 1H), 4.15 - 4.04 (m, 3H), 3.92 (d, J = 11.2 Hz, 1H), 1.74 (s, 3H) ppm |
| **42** | 468.4 | 1H NMR (400 MHz, DMSO-d6) δ = 9.12-9.08 (m, 1H), 8.18 - 8.12 (m, 2H), 7.89-7.86 (m, 1H), 7.30 - 7.19 (m, 3H), 6.97 (d, J = 8.0 Hz, 2H), 6.86 (d, J = 2.4 Hz, 1H), 6.83 - 6.77 (m, 2H), 4.95 - 4.79 (m, 2H), 4.57 - 4.40 (m, 2H), 4.21 (d, J = 11.6 Hz, 1H), 3.86 (d, J = 11.6 Hz, 1H), 3.47 - 3.43 (m, 4H), 3.26 - 3.23 (m, 4H), 1.69 (s, 3H) ppm |
| **44** | 453.3 | 1H NMR (400 MHz, DMSO-d6) δ = 9.10 - 9.07 (m, 1H), 8.12 (d, J = 1.2 Hz, 1H), 8.06 (d, J = 6.0 Hz, 1H), 7.87 - 7.84 (m, 1H), 7.33 - 7.31 (m, 4H), 7.26 - 7.22 (m, 2H), 6.50 (s, 1H), 6.46 - 6.44 (m, 1H), 4.90 - 4.83 (m, 2H), 4.46 - 4.44 (m, 2H), 4.20 (d, J =11.6 Hz, 1H), 3.85 (d, J = 11.6 Hz, 1H), 3.74 - 3.72 (m, 1H), 3.53 - 3.46 (m, 4H), 2.38 - 2.34 (m, 1H), 2.10 - 2.08 (m, 1H), 1.66 (s, 3H) ppm |
| **46** | 440.2 | 1H NMR (400 MHz, MeOD) δ = 8.37 (d, J = 2.8 Hz, 1H), 8.10 (d, J = 1.6 Hz, 1H), 7.85-7.83 (m, 1H), 7.38-7.32 (m, 4H), 7.25-7.23 (m, 2H), 6.59 (d, J = 2.8 Hz, 1H), 4.87 (br s, 2H), 4.70 (s, 2H), 4.50-7.49 (m, 2H), 4.18 (d, J = 11.6 Hz, 1H), 4.15 - 4.06(m, 3H), 3.91 (d, J = 11.2 Hz, 1H), 1.72 (s, 3H) ppm |

### Example 4. (4R)-4-Cyano-4-methyl-N-[[4-(1-phenylazetidin-3-yl)-2-pyridyl]methyl]isochromane-6-carboxamide

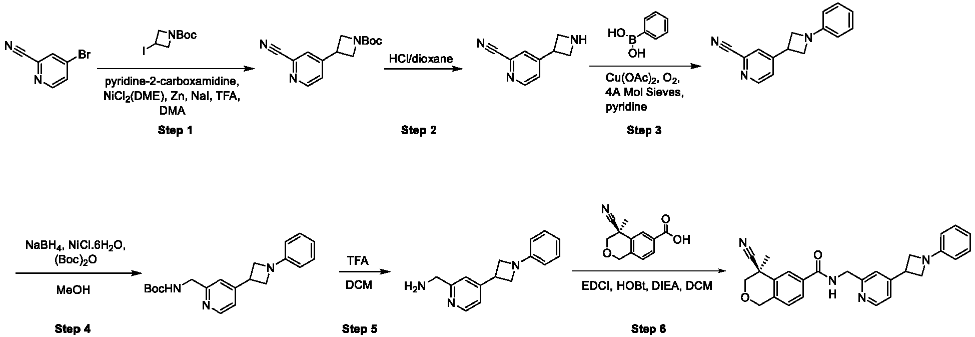

### Step 1. tert-Butyl 3-(2-cyano-4-pyridyl)azetidine-1-carboxylate

A mixture of 4-bromopyridine-2-carbonitrile (4 g, 21.9 mmol), tert-butyl 3-iodoazetidine-1-carboxylate (7.43 g, 26.2 mmol), dichloronickel;1,2-dimethoxyethane (480 mg, 2.19 mmol), pyridine-2-carboxamidine hydrochloride (344 mg, 2.19 mmol), Nal (1.64 g, 10.9 mmol), Zn (2.86 g, 43.7 mmol), and TFA (249 mg, 2.19 mmol, 162 µL) in DMA (80 mL) was degassed and purged with N₂ three times. The reaction mixture was stirred at 60°C for 4 hrs. The mixture was filtered. The filtrate was diluted with water (500 mL) and extracted with EA (500 mL x 2). The combined organic layers were dried with anhydrous Na₂SO₄ and concentrated to afford a crude residue. The residue was purified by column chromatography (SiO2, Petroleum ether/EA = 1/0 to 0/1). The eluent was concentrated affording the title compound (3.8 g, 14.6 mmol) as yellow oil.
LCMS (ESI) m/z: [M+H]+ =260.0.
¹H NMR (400 MHz, DMSO-d6) δ= 8.70 - 7.68 (m, 1H), 8.08 - 8.07 (m, 1H), 7.72 - 7.70 (m, 1H), 4.24 - 4.23 (m, 2H), 3.93 - 3.90 (m, 3H), 1.40 (s, 9H) ppm.

### Step 2. 4-(Azetidin-3-yl)pyridine-2-carbonitrile

A mixture of tert-butyl 3-(2-cyano-4-pyridyl)azetidine-1-carboxylate (2.0 g, 7.71 mmol) in HCl/dioxane (4N, 20 mL) was stirred at 25°C for 0.5 hrs. The mixture was concentrated affording the title compound (1.6 g, crude, HCl) as a white solid which was used into the next step without further purification.
LCMS (ESI) m/z: [M+H]+ = 160.0.

### Step 3. 4-(1-Phenylazetidin-3-yl)pyridine-2-carbonitrile

To a solution of phenylboronic acid (3.0 g, 24.5 mmol) and 4-(azetidin-3-yl)pyridine-2-carbonitrile (1.6 g, 8.18 mmol, HCl) in pyridine (16 mL) was added copper (II) acetate (1.71 g, 9.40 mmol) and 4A molecular sieves (1.6 g). The mixture was stirred at 80°C for 12 hrs under oxygen atmosphere. The reaction mixture was diluted with water (100 mL) and extracted with EA (100 mL x 2), the combined organic layer was dried with anhydrous Na₂SO₄ and concentrated to afford a residue. The residue was purified by column chromatography (SiO₂, Petroleum ether/Ethyl acetate=1/0 to 0/1). The eluent was concentrated affording the title compound (120 mg, 0.45 mmol) as a white solid.
LCMS (ESI) m/z: [M+H]+ = 236.0.
¹H NMR (400 MHz, DMSO-d6) δ = 8.71 (d, J = 4.8 Hz, 1H), 8.12 - 8.11 (m, 1H), 7.78 - 7.77 (m, 1H), 7.21 - 7.17 (m, 2H), 6.74 - 6.70 (m, 1H), 6.50 (d, J = 7.6 Hz, 2H), 4.22 - 4.19 (m, 2H), 4.04 - 4.01 (m, 1H), 3.87 - 3.84 (m, 2H) ppm.

### Step 4. tert-Butyl N-[[4-(1-phenylazetidin-3-yl)-2-pyridyl]methyl]carbamate

To a solution of 4-(1-phenylazetidin-3-yl)pyridine-2-carbonitrile (100 mg, 0.43 mmol) and NiCl₂•6H2O (253 mg, 1.06 mmol) in MeOH (2 mL) was added NaBH₄ (161 mg, 4.25 mmol) at 0°C. The mixture was stirred at 25°C for 1 hr. Boc₂O (1.39 g, 6.38 mmol) was added. Then the mixture was stirred at 25°C for 2 hrs. THF (2 mL) and NaHCO₃ (2 mL) was added to the mixture. Then the mixture was stirred at 25°C for 12 hrs. The mixture was diluted with saturated NH₄Cl solution (30 mL) and extracted with EA (30 mL x 2). The combined organic layers were dried with anhydrous Na₂SO₄ and concentrated to afford a residue. The residue was purified by flash silica gel chromatography (ISCO^{®};12 g SepaFlash^{®} Silica Flash Column, Eluent of 0-100% Ethylacetate/Petroleum ethergradient @ 40 mL/min). The eluent was concentrated affording the title compound (50 mg, 0.12 mmol) as a white solid.
LCMS (ESI) m/z: [M+H]+ = 340.3.

### Step 5. [4-(1-Phenylazetidin-3-yl)-2-pyridyl]methanamine

TFA (168 mg, 1.47 mmol, 109 µL) was added to a solution of tert-Butyl N-[[4-(1-phenylazetidin-3-yl)-2-pyridyl]methyl]carbamate (50 mg, 0.15 mmol) in DCM (1 mL). The mixture was stirred at 25 °C for 1 hr. The reaction mixture was dried by N₂ airflow. The crude product was triturated with PE/MTBE=1:1 (3 mL) affording the title compound (30 mg, 0.085 mmol, TFA) as a yellow solid which was used into the next step without further purification.
LCMS (ESI) m/z: [M+H]+ = 240.0.

### Step 6. (4R)-4-Cyano-4-methyl-N-[[4-(1-phenylazetidin-3-yl)-2-pyridyl]methyl]isochromane-6-carboxamide

HOBt (17.2 mg, 0.13 mmol), EDCI (24.4 mg, 0.13 mmol), and DIPEA (32.9 mg, 0.25 mmol, 44 µL) were added to a solution of [4-(1-phenylazetidin-3-yl)-2-pyridyl]methanamine (30 mg, 0.085 mmol, TFA salt) and (4R)-4-cyano-4-methylisochromane-6-carboxylic acid (20.3 mg, 0.093 mmol) in DCM (0.5 mL). The mixture was stirred at 25 °C for 1 hr. The reaction mixture was diluted with water (10 mL) and extracted with DCM (10 mL x 2). The combined organic layers were dried with anhydrous Na₂SO₄ and concentrated to afford a crude residue. The residue was purified by prep-HPLC (neutral condition) (column: Waters Xbridge C18 150*50mm* 10µm; mobile phase: [water (10mM NH₄HCO₃)-ACN]; B%: 36%-66%, 10min) affording the title compound (12.8 mg, 0.030 mmol) as a yellow gum.
LCMS (ESI) m/z: [M+H]+ = 439.3.
¹HNMR (400 MHz, CD₃OD) δ = 8.46 (d, J = 5.2 Hz, 1H), 8.08 (d, J = 1.6 Hz, 1H), 7.82 - 7.80 (m, 1H), 7.51 (s, 1H), 7.38 - 7.37 (m, 1H), 7.21 - 7.17 (m, 3H), 6.75 - 6.70 (m, 1H), 6.52 - 6.50 (m, 2H), 4.88 (s, 2H), 4.70 (s, 2H), 4.26 - 4.24 (m, 2H), 4.18 (d, J = 11.2 Hz, 1H), 3.91 - 3.83 (m, 4H), 1.71 (s, 3H) ppm.

The following examples in Table 6 were prepared using standard chemical manipulations and procedures similar to those used for the preparation of **Example 4.**

**Table 6. Compounds of the Invention**

| **#** | **LCMS (m/z)** | **1H NMR** |
|---|---|---|
| **18** | 438.3 | 1H NMR (400 MHz, DMSO-d6) δ = 9.23 (br s, 1H), 8.48 (d, J = 5.2 Hz, 1H), 8.16 (s, 1H), 7.90 (m, 1H), 7.39 - 7.24 (m, 7H), 7.23 - 7.21 (m, 1H), 4.86 (d, J = 12.0 Hz, 2H), 4.69 - 4.54 (m, 2H), 4.22 (d, J = 11.6 Hz, 1H), 3.86 (d, J = 11.6 Hz, 1H), 3.63 - 3.61 (m, 2H), 2.59 - 2.53 (m, 4H), 1.68 (s, 3H) ppm. |
| **19** | 438.3 | 1H NMR (400 MHz, DMSO-d6) δ = 9.23 - 9.20 (m, 1H), 8.44 (d, J = 5.2 Hz, 1H), 8.14 (d, J = 1.2 Hz, 1H), 7.91 - 7.89 (m, 1H), 7.31 - 7.24 (m, 6H), 7.23 - 7.17 (m, 2H), 4.93 - 4.79 (m, 2H), 4.59 (br d, J = 4.4 Hz, 2H), 4.22 (d, J = 11.6 Hz, 1H), 3.86 (d, J = 11.6 Hz, 1H), 3.57 - 3.45 (m, 2H), 2.77 - 2.67 (m, 2H), 2.20 - 2.08 (m, 2H), 1.67 (s, 3H) ppm. |

### Example 5. (R,E)-4-Cyano-N-((4-(1,2-difluoro-2-phenylvinyl)pyridin-2-yl)methyl)-4-methylisochromane-6-carboxamide

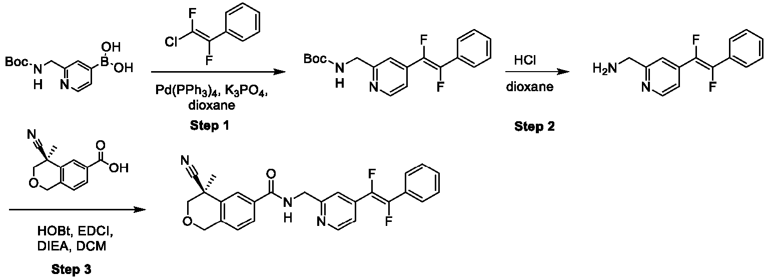

### Step 1. tert-Butyl N-[[4-[(E)-1,2-difluoro-2-phenyl-vinyl]-2-pyridyl]methyl]carbamate

Pd(PPh₃)₄ (53 mg, 0.046 mmol) and K₃PO₄ (292 mg, 1.37 mmol) were added to a mixture of (Z)-(2-chloro-1,2-difluorovinyl)benzene (80 mg, 0.46 mmol) and (2-(((tert-butoxycarbonyl)amino)methyl) pyridin-4-yl)boronic acid (173 mg, 0.69 mmol) in dioxane (3 mL) at 25 °C under N₂. The mixture was stirred at 100 °C for 2 hrs. The reaction mixture was poured into water (30 mL) and extracted with EA (30 mL x 2). The combined organic phase was washed with brine (20 mL), dried with anhydrous Na₂SO₄, filtered, and concentrated in vacuum. The residue was purified by prep-TLC (Petroleum ether/EA = 3/1) affording the title compound (60 mg, 0.14 mmol) as a light yellow solid.
LCMS (ESI) m/z: [M+H]+ = 347.2.

### Step 2. [4-[(E)-1,2-difluoro-2-phenyl-vinyl]-2-pyridyl]methanamine

4N HCl/dioxane (0.22 mL) was added to a mixture of tert-butyl N-[[4-[(E)-1,2-difluoro-2-phenyl-vinyl]-2-pyridyl]methyl]carbamate (60 mg, 0.17 mmol) in dioxane (1 mL) at 25°C. The mixture was stirred for 30 minutes. The mixture was concentrated under reduced pressure affording the title compound (40 mg, 0.14 mmol, HCl salt) as a light yellow solid.
LCMS (ESI) m/z: [M+H]+ = 247.2.

### Step 3. (4R)-4-Cyano-N-[[4-[(E)-1,2-difluoro-2-phenyl-vinyl]-2-pyridyl]methyl]-4-methyl-isochromane-carboxamide

HOBt (28.7 mg, 0.21 mmol), EDCI (40.7 mg, 0.21 mmol), and DIEA (0.12 mL, 0.71 mmol) were added to a mixture of [4-[(E)-1,2-difluoro-2-phenyl-vinyl]-2-pyridyl]methanamine (40 mg, 0.14 mmol, HCl salt) and (4R)-4-cyano-4-methyl-isochromane-6-carboxylic acid (36.9 mg, 0.17 mmol) in DCM (2 mL) at 25°C. The mixture was stirred for 2 hrs. The mixture was poured into water (20 mL) and extracted with DCM (10 mL x 2). The combined organic phase was washed with brine (10 mL), dried over anhydrous Na₂SO₄, filtered, and concentrated in vacuum. The residue was purified by reverse phase column chromatography. The eluent was lyophilized affording the title compound (22.3 mg, 0.050 mmol, FA salt) as a white solid.
LCMS (ESI) m/z: [M+H]+ = 446.3.
¹H NMR (400 MHz, CD₃OD) δ = 8.68 - 8.56 (m, 1H), 8.17 - 8.08 (m, 1H), 7.90 - 7.84 (m, 1H), 7.82 - 7.73 (m, 3H), 7.68 - 7.60 (m, 1H), 7.56 - 7.42 (m, 3H), 7.33 - 7.19 (m, 1H), 4.92 - 4.88 (m, 2H), 4.76 (s, 2H), 4.20 - 3.90 (m, 2H), 1.75 (s, 3H) ppm.

The following examples in Table 7 were prepared using standard chemical manipulations and procedures similar to those used for the preparation of **Example 5.**

**Table 7. Compounds of the Invention**

| **#** | **LCMS (m/z)** | **1H NMR** |
|---|---|---|
| **27** | 428.1 | 1H NMR (400 MHz, DMSO-d6) δ = 9.28 (m, 1H), 8.62 (d, J = 5.2 Hz, 1H), 8.16 (d, J = 1.2 Hz, 1H), 7.91 (m, 1H), 7.70 (d, J = 7.5 Hz, 2H), 7.66 - 7.60 (m, 2H), 7.48 - 7.41 (m, 2H), 7.40 - 7.33 (m, 1H), 7.29 (d, J = 8.0 Hz, 1H), 7.19 - 6.99 (m, 1H), 4.95 - 4.79 (m, 2H), 4.65 (m, 2H), 4.22 (d, J = 11.6 Hz, 1H), 3.86 (d, J = 11.6 Hz, 1H), 1.69 (s, 3H) ppm. |
| **29** | 428.3 | 1H NMR (400 MHz, MeOD-d4) δ = 8.48 (d, J = 5.2 Hz, 1H), 8.14 (d, J = 1.6 Hz, 1H), 7.89 - 7.87 (m, 1H), 7.75 - 7.73 (m, 2H), 7.67 (s, 1H), 7.56 - 7.55 (m, 1H), 7.45 - 7.44 (m, 3H), 7.27 (d, J = 8.0 Hz, 1H), 6.66 - 6.55 (m, 1H), 4.90 (d, J = 4.0 Hz, 2H), 4.73 (s, 2H), 4.20 (d, J = 11.6 Hz, 1H), 3.92 (d, J = 11.2 Hz, 1H), 1.76 (s, 3H) ppm. |

### Example 6. (4R)-N-[(4-Benzyloxy-2-pyridyl)methyl]-4-cyano-4-methyl-isochromane-6-carboxamide

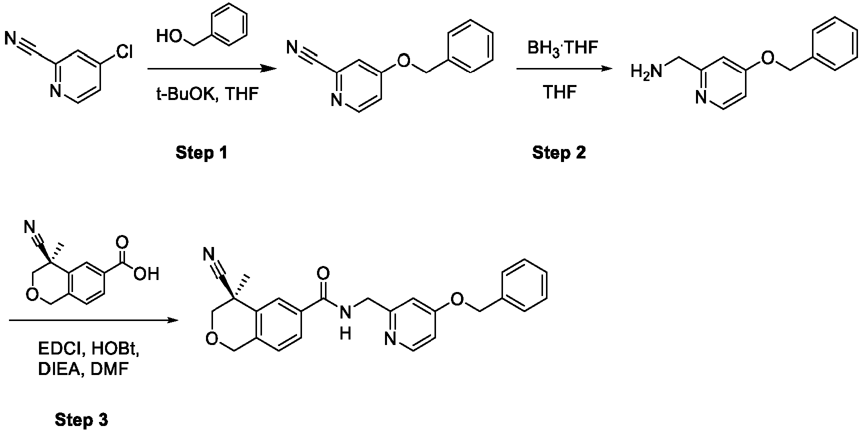

### Step 1. 4-Benzyloxypyridine-2-carbonitrile

t-BuOK (2.43 g, 21.65 mmol) was added to a solution of 4-chloropyridine-2-carbonitrile (1 g, 7.2 mmol) and phenylmethanol (0.90 mL, 8.7 mmol) in THF (10 mL) at 0 °C. The mixture was warmed to 25 °C and stirred for 1 hr. The reaction mixture was poured into saturated NH₄Cl (aq., 20 mL) and extracted with EA (7 mL x 2). The organic layer was washed with brine (10 mL), dried over anhydrous Na₂SO₄, filtered, and concentrated. The crude product was purified by column chromatography (SiO₂, Petroleum ether/EA = 10/1 to 1/1) affording the title compound (1.1 g, 4.24 mmol) as a yellow solid.
¹H NMR (400 MHz, DMSO-d6) δ = 8.63 (d, J = 5.2 Hz, 1H), 8.45 (d, J = 5.6 Hz, 1H), 8.04 (d, J = 2.0 Hz, 1H), 7.78 - 7.76 (m, 1H), 7.64 (d, J = 2.8 Hz, 1H), 7.45 - 7.39 (m, 2H), 7.23 - 7.21 (m, 1H), 5.29 (s, 2H) ppm.

### Step 2. (4-Benzyloxy-2-pyridyl)methanamine

BH₃·THF (1 M, 4.76 mL) was added dropwise to a solution of 4-benzyloxypyridine-2-carbonitrile (200 mg, 0.95 mmol) in THF (2 mL) at 0 °C. The reaction mixture was warmed 25 °C and stirred for 4 hrs. The reaction mixture was cooled to 0 °C and 2N HCl (5 mL) was added. The reaction mixture was heated to reflux for 30 minutes and then cooled to 25 °C. 2N NaOH was added until pH = 12 was achieved. The reaction mixture was then extracted with EA (5 mL x 2). The combined organic layers were dried over anhydrous Na₂SO₄, filtered, and concentrated affording the title compound (170 mg, 0.56 mmol) as yellow oil.
LCMS (ESI) m/z: [M+H]+ = 215.1.
¹H NMR (400 MHz, DMSO-d6) δ = 8.48 - 8.43 (m, 1H), 8.30 (d, J = 5.6 Hz, 2H), 7.64 (d, J = 2.8 Hz, 1H), 7.44 - 7.39 (m, 5H), 7.25 - 7.21 (m, 1H), 5.19 (s, 2H), 4.50 (d, J = 2.8 Hz, 2H) ppm.

### Step 3. (4R)-N-[(4-Benzyloxy-2-pyridyl)methyl]-4-cyano-4-methyl-isochromane-6-carboxamide

DIEA (0.41 mL, 2.3 mmol) was added to a solution of (4-benzyloxy-2-pyridyl)methanamine (100 mg, 0.47 mmol), (4R)-4-cyano-4-methyl-isochromane-6- carboxylic acid (101 mg, 0.47 mmol), EDCI (179 mg, 0.93 mmol), and HOBt (126 mg, 0.93 mmol) in DMF (2 mL). The mixture was stirred at 25°C for 1 hr. The reaction mixture was filtered and the filtrate was purified by prep-HPLC (column: Phenomenex Synergi C18 150*25*10µm; mobile phase: [water (0.225%FA)-ACN]; B%: 20%-50%, 9min) affording the title compound (51.8 mg, 0.11 mmol, FA salt) as a yellow gum.
LCMS (ESI) m/z: [M+H]+ = 414.2.
¹H NMR (400 MHz, DMSO-d6) δ = 9.21 - 9.18 (m, 1H), 8.40 - 8.30 (m, 1H), 8.14 (d, J = 1.6 Hz, 1H), 7.89 - 7.88 (m, 1H), 7.48 - 7.39 (m, 2H), 7.38 - 7.32 (m, 3H), 7.28 (d, J = 8.0 Hz, 1H), 6.99 - 6.91 (m, 2H), 5.17 (s, 2H), 4.95 - 4.80 (m, 2H), 4.58 - 4.49 (m, 2H), 4.22 (d, J = 11.6 Hz, 1H), 3.86 (d, J = 11.6 Hz, 1H), 1.69 (s, 3H) ppm.

The following examples in Table 8 were prepared using standard chemical manipulations and procedures similar to those used for the preparation of **Example 6.**

**Table 8. Compounds of the Invention**

| **#** | **LCMS (m/z)** | **1H NMR** |
|---|---|---|
| **34** | 470.3 | 1H NMR (400 MHz, MeOD) δ = 8.31 (d, J = 6.0 Hz, 1H), 8.10 (d, J = 1.6 Hz, 1H), 7.86 - 7.81 (m, 1H), 7.50 - 7.40 (m, 1H), 7.27 (d, J = 8.2 Hz, 1H), 7.01 (d, J = 2.4 Hz, 1H), 6.97 - 6.93 (m, 1H), 6.69 (d, J = 7.2 Hz, 1H), 6.26 (d, J = 8.4 Hz, 1H), 5.09 (s, 2H), 4.90 (d, J = 4.0 Hz, 2H), 4.64 (s, 2H), 4.20 (d, J = 11.6 Hz, 1H), 4.00 - 3.95 (m, 4H), 3.91 (d, J = 11.6 Hz, 1H), 2.40 - 2.33 (m, 2H), 1.75 (s, 3H) ppm |
| **36** | 415.2 | 1H NMR (400 MHz, DMSO-d6) δ= 9.19 - 9.16 (m, 1H), 8.51 - 8.50 (m, 1H), 8.38 - 8.37 (m, 1H), 8.35 - 8.33 (m, 1H), 8.12 (d, J= 1.2 Hz, 1H), 7.90 - 7.88 (m, 1H), 7.81 - 7.77 (m, 1H), 7.48 (d, J = 8.0 Hz, 1H), 7.33 - 7.30 (m, 1H), 7.28 (d, J = 8.0 Hz, 1H),6.96 (d, J = 2.8 Hz, 2H), 5.23 (s, 2H), 4.91 - 4.80 (m, 2H), 4.53 - 4.50 (m, 2H), 4.24 - 4.14 (m, 1H), 3.85 (d, J = 11.6 Hz, 1H),1.68 (s, 3H) ppm |
| **40** | 469.1 | 1H NMR (400 MHz, MeOD) δ = 8.30 (d, J = 5.6 Hz, 1H), 8.11 (d, J = 1.6 Hz, 1H), 7.89 - 7.79 (m, 1H), 7.27 (d, J = 8.0 Hz, 1H), 7.15 - 7.06 (m, 1H), 7.02 (d, J = 2.4 Hz, 1H), 6.98 - 6.93 (m, 1H), 6.72 (d, J = 7.6 Hz, 1H), 6.48 (s, 1H), 6.43 - 6.34 (m, 1H), 5.13 (s, 2H), 4.93 (br d, J = 9.6 Hz, 2H), 4.64 (s, 2H), 4.20 (d, J = 11.6 Hz, 1H), 3.92 (d, J = 11.2 Hz, 1H), 3.85 - 3.76 (m, 4H), 2.40 - 2.26 (m, 2H), 1.75 (s, 3H) ppm |
| **45** | 474.2 | 1H NMR (400 MHz, MeOD) δ = 8.30 (d, J = 6.0 Hz, 1H), 8.11 (d, J = 1.6 Hz, 1H), 7.88 - 7.80 (m, 1H), 7.26 (d, J = 8.0 Hz, 1H), 7.06 (s, 1H), 7.01 - 6.91 (m, 3H), 5.09 (s, 2H), 4.90 (br d, J = 3.6 Hz, 2H), 4.65 - 4.57 (m, 4H), 4.19 (d, J = 11.6 Hz, 1H), 3.92 (d, J = 11.2 Hz, 1H), 3.23 - 3.14 (m, 2H), 1.75 (s, 3H) ppm |
| **61** | 422.2 | 1H NMR (400 MHz, DMSO-d6) δ = 9.21 - 9.18 (m, 1H), 8.33 (d, J = 5.6 Hz, 1H), 8.14 (s, 1H), 7.90 - 7.88 (m, 1H), 7.28 (d, J = 8.0 Hz, 1H), 6.95 - 6.82 (m, 2H), 4.97 - 4.78 (m, 2H), 4.60 - 4.47 (m, 2H), 4.22 (d, J = 11.6 Hz, 1H), 3.97 - 3.78 (m, 5H), 3.31 - 3.25 (m, 2H), 2.06 - 1.92 (m, 1H), 1.74 - 1.57 (m, 5H), 1.34 - 1.28 (m, 2H) ppm |

### Example 7. (4R)-4-Cyano-4-methyl-N-[[4-[(1S,2S)-2-phenylcyclopropyl]-2-pyridyl]methyl]isochromane-6-carboxamide and (4R)-4-Cyano-4-methyl-N-[[4-[(1R,2R)-2-phenylcyclopropyl]-2-pyridyl]methyl]isochromane-6-carboxamide

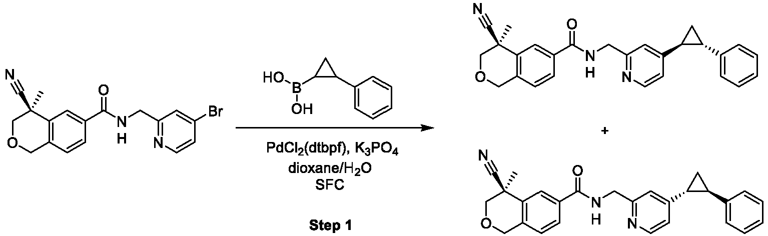

### Step 1. (4R)-4-Cyano-4-methyl-N-[[4-[(1S,2S)-2-phenylcyclopropyl]-2-pyridyl]methyl]isochromane-6-carboxamide and (4R)-4-cyano-4-methyl-N-[[4-[(1R,2R)-2-phenylcyclopropyl]-2-pyridyl]methyl]isochromane-6-carboxamide

PdCl₂(dtbpf) (33.7 mg, 0.052 mmol) and K₃PO₄ (165 mg, 0.78 mmol) were added to a solution of (4R)-N-[(4-bromo-2-pyridyl)methyl]-4-cyano-4-methyl-isochromane-6-carboxamide (100 mg, 0.26 mmol), and (2-phenylcyclopropyl)boronic acid (83.9 mg, 0.52 mmol) in dioxane (1 mL) and H₂O (0.1 mL). The mixture was stirred at 80 °C for 12 hrs. The reaction mixture was washed with water (50 mL) and extracted with EA (50 mL x 2). The combined organic phase was washed with brine (20 mL), dried with anhydrous Na₂SO₄, filtered, and concentrated in vacuo. The residue was purified by prep-HPLC (column: Phenomenex luna C18 150*25mm* 10µm; mobile phase: [water (0.1%TFA)-ACN]; B%: 32%-52%, 10min) affording a mixture of the title compounds (60 mg, 132.24 µmol, 51.08% yield) as a brown solid. The mixture was separated by chiral SFC (column: DAICEL CHIRALCEL OJ-H (250mm x 30mm, 5µm); mobile phase: [0.1%NH₃•H₂O MeOH]; B%: 40%-40%, 3.9 min; 70 min), the solution was concentrated under reduced pressure affording the title compounds.
1^{st} eluting compound (12.9 mg, 0.029 mmol, off-white solid):
   LCMS (ESI) m/z: [M+H]+ = 424.1.
   ¹H NMR (400 MHz, DMSO-d6) δ = 9.18 - 9.14 (m, 1H), 8.37 (d, J = 5.2 Hz, 1H), 8.13 (d, J = 1.2 Hz, 1H), 7.90 - 7.86 (m, 1H), 7.29 - 7.25 (m, 3H), 7.19 - 7.15 (m, 4H), 7.07 - 7.04 (m, 1H), 4.91 - 4.79 (m, 2H), 4.57 - 4.53 (m, 2H), 4.21 (d, J = 11.2 Hz, 1H), 3.85 (d, J = 11.6 Hz, 1H), 2.29 - 2.21 (m, 2H), 1.68 (s, 3H), 1.57 - 1.52 (m, 2H) ppm.
2^{nd} eluting compound (11.3 mg, 0.027 mmol, yellow solid):
   LCMS (ESI) m/z: [M+H]+ = 424.1.
   ¹H NMR (400 MHz, DMSO-d6) δ = 9.25 - 9.21 (m, 1H), 8.43 (d, J = 5.2 Hz, 1H), 8.13 (d, J = 1.6 Hz, 1H), 7.90 - 7.87 (m, 1H), 7.30 - 7.25 (m, 3H), 7.20 - 7.16 (m, 4H), 7.07 - 7.03 (m, 1H), 4.91 - 4.79 (m, 2H), 4.60 (d, J = 5.2 Hz, 2H), 4.21 (d, J = 11.6 Hz, 1H), 3.85 (d, J = 11.2 Hz, 1H), 2.36 - 2.30 (m, 2H), 1.67 (s, 3H), 1.64 - 1.59 (m, 2H) ppm.

The following examples in Table 9 were prepared using standard chemical manipulations and procedures similar to those used for the preparation of **Examples 7.**

**Table 9. Compounds of the Invention**

| **#** | **LCMS (m/z)** | **1H NMR** |
|---|---|---|
| **4** | 410.2 | 1H NMR (400 MHz, DMSO-d6) δ = 9.36 (m, 1H), 8.65 (d, J = 5.6 Hz, 1H), 8.15 (d, J = 1.6 Hz, 1H), 7.91 (m, 1H), 7.88 - 7.88 (m, 1H), 7.88 - 7.79 (m, 2H), 7.77 (s, 3H), 7.50 - 7.35 (m, 4H), 7.30 (d, J = 8.0 Hz, 1H), 4.94 - 4.80 (m, 2H), 4.72 (d, J = 5.6 Hz, 2H), 4.22 (d, J = 11.6 Hz, 1H), 3.88 - 3.85 (m, 1H), 1.69 (s, 3H) ppm. |
| **38** | 440.3 | 1H NMR (400 MHz, DMSO-d6) δ = 9.26-9.23 (m, 1H), 8.51 (d, J = 5.2 Hz, 1H), 8.17 (d, J = 1.6 Hz, 1H), 7.93-7.92 (m, 1H), 7.55 - 7.44 (m, 3H), 7.37 - 7.27 (m, 3H), 7.26 - 7.21 (m, 2H), 6.91-6.89 (m, 1H), 4.99 - 4.77 (m, 2H), 4.62- 4.60 (m, 2H), 4.23 (d, J = 11.2 Hz) ppm |

### Example 8. (4R)-4-Cyano-4-methyl-N-[[4-(2-phenylethyl)-2-pyridyl]methyl]isochromane-6-carboxamide

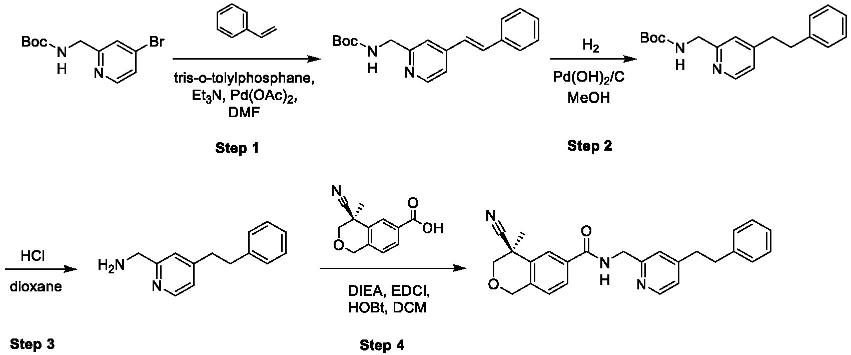

### Step 1. tert-butyl N-[[4-[(E)-styryl]-2-pyridyl]methyl]carbamate

Pd(OAc)₂ (7.8 mg, 0.0325 mmol) was added to a solution of tert-butyl ((4-bromopyridin-2-yl)methyl)carbamate (200 mg, 0.70 mmol), styrene (363 mg, 3.48 mmol), Et₃N (0.14 mL, 1.04 mmol), and tris-o-tolylphosphane (21.2 mg, 0.070 mmol) in DMF (2 mL). The mixture was stirred at 100 °C for 2 hrs. The reaction mixture was diluted with H₂O (10 mL) and extracted with DCM (10 mL x 2), the combined organic layers were dried over anhydrous Na₂SO₄, filtered, and concentrated in vacuo. The crude residue was purified by column chromatography (SiO₂, Petroleum ether/EA = 1/0 to 0/1) affording the title compound (200 mg, 0.64 mmol) as a yellow solid.
LCMS (ESI) m/z: [M+H]+ = 311.4
¹H NMR (400 MHz, CDCl3) δ = 8.51 (d, J = 5.2 Hz, 1H), 7.55 (d, J = 7.2 Hz, 2H), 7.42 - 7.40 (m, 2H), 7.38 - 7.28(m, 4H), 7.04 (d, J = 16.4 Hz, 1H), 4.47 (d, J = 5.6 Hz, 2H), 1.49 (s, 9H) ppm.

### Step 2. tert-Butyl N-[[4-(2-phenylethyl)-2-pyridyl]methyl]carbamate

Pd(OH)₂/C (30 mg) was added to a mixture of tert-butyl N-[[4-[(E)-styryl]-2-pyridyl]methyl]carbamate (100 mg, 0.32 mmol) in MeOH (2 mL). The mixture was degassed and purged with H₂ three times, and then the mixture was stirred at 25 °C for 12 hrs under a H₂ atmosphere (15 psi). The reaction mixture was filtered. The filtrate was concentrated affording the title compound (90 mg, 0.29 mmol) as a yellow oil.
LCMS (ESI) m/z: [M+H]+ = 313.0.
¹H NMR (400 MHz, CDCl₃) δ = 8.41 (d, J = 4.8 Hz, 1H), 7.31-7.27(m, 2H), 7.21 - 7.18 (m, 1H), 7.16- 7.14 (m, 2H), 7.06 (s, 1H), 6.98-6.97 (m, 1H), 4.41 (br d, J = 5.2 Hz, 2H), 2.92 (s, 4H), 1.47 (s, 9H) ppm.

### Step 3. [4-(2-Phenylethyl)-2-pyridyl]methanamine

A solution of tert-butyl N-[[4-(2-phenylethyl)-2-pyridyl]methyl]carbamate (90 mg, 0.29 mmol) in 4N HCl/dioxane (5.0 mL) was stirred at 25 °C for 1 hr. The reaction mixture was concentrated affording the title compound (70 mg, 0.28 mmol, HCl salt) as a white solid.
LCMS (ESI) m/z: [M+H]+ = 213.3.

### Step 4. (4R)-4-cyano-4-methyl-N-[[4-(2-phenylethyl)-2-pyridyl]methyl]isochromane-6-carboxamide

DIEA (0.14 mL, 0.80 mmol), EDCI (46.2 mg, 0.24 mmol), and HOBt (32.6 mg, 0.24 mmol) were added to a solution of [4-(2-Phenylethyl)-2-pyridyl]methanamine (40 mg, 0.16 mmol, HCl salt) and (4R)-4-cyano-4-methylisochromane-6-carboxylic acid (38.4 mg, 0.18 mmol) in DCM (0.5 mL). The mixture was stirred at 30 °C for 12 hrs. The reaction mixture was diluted with H₂O (3 mL) and extracted with DCM (3 mL x 2). The combined organic layers were dried over anhydrous Na₂SO₄, filtered, and concentrated under reduced pressure. The crude residue was purified by column chromatography (SiO₂, Petroleum ether/EA = 1/0 to 0/1) affording the title compound (23.5 mg, 0.054 mmol) as a yellow solid.
LCMS (ESI) m/z: [M+H]+ = 412.3.
¹H NMR (400 MHz, CD₃OD) δ = 8.34 (d, J = 4.8 Hz, 1H), 8.10 (d, J = 0.8 Hz, 1H), 7.85 - 7.83 (m, 1H), 7.27 (d, J = 8.0 Hz, 1H), 7.20 - 7.16 (m, 3H), 7.14 - 7.12 (m, 3H), 7.10 - 7.07 (m, 1H), 4.90 (d, J = 3.2 Hz, 2H), 4.64 (s, 2H), 4.20 (d, J = 11.6 Hz, 1H), 3.92 (d, J = 11.2 Hz, 1H), 2.95 - 2.92 (m, 4H), 1.74 (s, 3H) ppm.

### Example 9. Methyl 3-[(E)-2-[2-[[[(4R)-4-cyano-4-methyl-isochromane-6-carbonyl]amino]methyl]-4-pyridyl]vinyl]benzoate

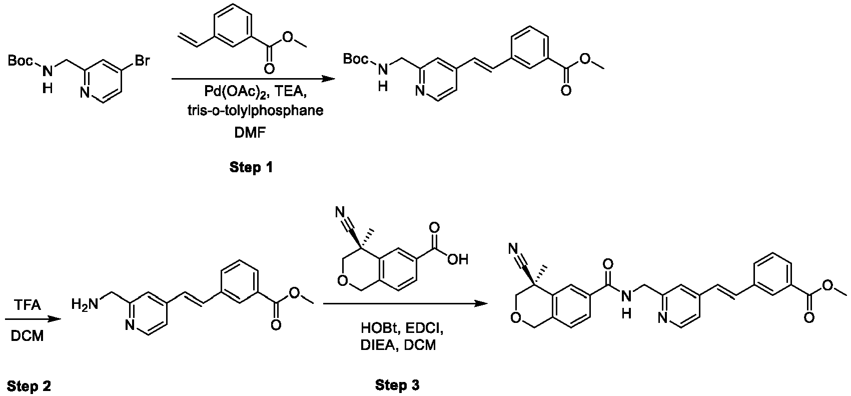

### Step 1. Methyl 3-[(E)-2-[2-[(tert-butoxycarbonylamino)methyl]-4-pyridyl]vinyl]benzoate

Pd(OAc)₂ (15.6 mg, 0.070 mmol), tris-o-tolylphosphane (42.4 mg, 0.14 mmol) and Et₃N (0.19 mL, 1.39 mmol) were added to a mixture of tert-butyl N-[(4-bromo-2-pyridyl)methyl]carbamate (200 mg, 0.70 mmol) and methyl 3-vinylbenzoate (226 mg, 1.39 mmol) in DMF (2 mL). The reaction mixture was stirred at 100 °C for 1 hr. The reaction mixture was poured into water (30 mL) and extracted with EA (20 mL x 2). The combined organic phase was washed with brine (20 mL), dried with anhydrous Na₂SO₄, filtered, and concentrated in vacuo. The crude residue was purified by silica gel chromatography (Petroleum ether/EA = 10/1 to 3/1) affording the title compound (200 mg, 0.54 mmol) as light yellow oil
LCMS (ESI) m/z: [M+H]+ = 369.2.
¹H NMR (400 MHz, CDCl₃) δ = 8.52 (d, J = 5.2 Hz, 1H), 8.24 (s, 1H), 8.00 (d, J = 7.6Hz, 1H), 7.71 (d, J = 7.6 Hz, 1H), 7.48 - 7.46 (m, 1H), 7.39 - 7.28 (m, 3H), 7.10 (d, J = 16.4 Hz, 1H), 4.48 (d, J = 5.6 Hz, 2H), 4.12 (s, 3H), 1.55 (s, 9H) ppm.

### Step 2. Methyl 3-[(E)-2-[2-(aminomethyl)-4-pyridyl]vinyl]benzoate

TFA (0.50 mL, 6.75 mmol) was added to a mixture of Methyl 3-[(E)-2-[2-[(tert-butoxycarbonylamino)methyl]-4-pyridyl]vinyl]benzoate (100 mg, 0.27 mmol) in DCM (1 mL). The reaction mixture was stirred at 25 °C for 1 hrs. The mixture was poured into saturated NaHCO₃ (30 mL) and extracted with DCM (20 mL x 2). The combined organic phase was washed with brine (20 mL x 2), dried over anhydrous Na₂SO₄, filtered, and concentrated in vacuo affording the title compound (60 mg, 0.22 mmol) as yellow oil.
LCMS (ESI) m/z: [M+H]+ = 269.1.

### Step 3. Methyl 3-[(E)-2-[2-[[[(4R)-4-cyano-4-methyl-isochromane-6-carbonyl]amino]methyl]-4-pyridyl]vinyl]benzoate

HOBt (22.7 mg, 0.17 mmol), EDCI (32.1 mg, 0.17 mmol, and DIEA (0.058 mL, 0.33 mmol) were added to a mixture of Methyl 3-[(E)-2-[2-(aminomethyl)-4-pyridyl]vinyl]benzoate (30 mg, 0.11 mmol) and (4R)-4-cyano-4-methyl-isochromane-6-carboxylic acid (29.1 mg, 0.13 mmol) in DCM (1 mL). The mixture was stirred at 25 °C for 12 hrs. The mixture was poured into water (20 mL) and extracted with DCM (20 mL x 2). The combined organic phase was washed with brine (20 mL), dried with anhydrous Na₂SO₄, filtered, and concentrated in vacuo. The crude residue was purified by prep-TLC (DCM/MeOH = 10/1) affording the title compound (25.2 mg, 0.052 mmol) as a blue solid.
LCMS (ESI) m/z: [M+H]+ = 468.3.
¹H NMR (400 MHz, CD₃OD) δ = 8.52 - 8.46 (m, 1H), 8.30 - 8.24 (m, 1H), 8.18 - 8.14 (m, 1H), 8.01 - 7.95 (m, 1H), 7.93 - 7.85 (m, 2H), 7.64 - 7.60 (m, 1H), 7.59 - 7.49 (m, 3H), 7.34 - 7.26 (m, 2H), 4.94 - 4.91 (m, 2H), 4.74 (s, 2H), 4.25 - 3.90 (m, 5H), 1.77 (s, 3H) ppm.

The following examples in Table 10 were prepared using standard chemical manipulations and procedures similar to those used for the preparation of **Example** 9.

**Table 10. Compounds of the Invention**

| **#** | **LCMS (m/z)** | **1H NMR** |
|---|---|---|
| **33** | 428.2 | 1H NMR (400 MHz, DMSO-d6) δ = 9.16-9.13 (m, 1H), 8.35 (d, J = 5.0 Hz, 1H), 8.14 (d, J = 1.4 Hz, 1H), 7.88-7.85 (dm, 1H),7.76 - 7.70 (m, 2H), 7.67 - 7.56 (m, 1H), 7.51 - 7.17 (m, 5H), 4.94 - 4.79 (m, 2H), 4.78 - 4.62 (m, 2H), 4.27 - 4.16 (m, 1H), 3.93- 3.80 (m, 1H), 1.68(s, 3H)ppm |
| **41** | 454.2 | 1H NMR (400 MHz, DMSO-d6) δ = 8.97 (d, J=8.0, 1H), 8.53 (d, J=5.2, 1H), 8.18 (d, J=1.2, 1H), 7.93-7.90 (m, 1H), 7.67 - 7.61 (m, 3H), 7.55 - 7.49 (m, 2H), 7.42-7.38 (m, 2H), 7.35-7.31 (m , 1H), 7.29-7.25 (m, 2H), 5.43-5.38 (m, 1H), 4.91 - 4.80 (m, 2H), 4.21 (d, J=11.6, 1H), 3.87 - 3.81 (m, 3H), 3.30 (s, 3H), 1.69 (s, 3H) ppm |

### Example 10. (4R)-4-Cyano-4-methyl-N-[[4-(phenoxymethyl)-2-pyridyl]methyl]isochromane-6-carboxamide.

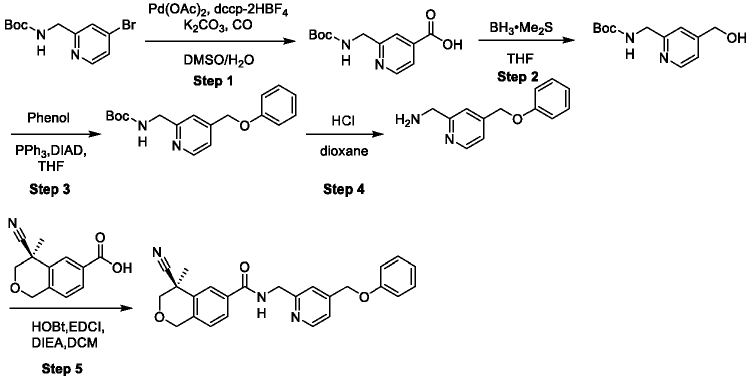

### Step1. 2-[(tert-Butoxycarbonylamino)methyl]pyridine-4-carboxylic acid

Pd(OAc)₂ (62.6 mg, 0.28 mmol), DCPP (171 mg, 0.28 mmol), H₂O (2 mL), and K₂CO₃ (578 mg, 4.18 mmol) were added to a solution of 2 tert-butyl N-[(4-bromo-2-pyridyl)methyl]carbamate (800 mg, 2.79 mmol) in DMSO (8 mL). The reaction mixture was purged with CO(g) three times. The resulting mixture was stirred at 100 °C for 16 hr under a CO atmosphere (15psi). The reaction mixture was diluted with H₂O (30mL) and extracted with EA (30 mL x 3). The combined organic layers were discarded, and the aqueous layers were combined and 1N HCl was added until a pH=4 was achieved. The reaction mixture was extracted with EA (30 mL x 3). The combined organic layers were washed with brine (60mL), dried over anhydrous Na₂SO₄, filtered, and concentrated under reduced pressure affording the title compound (436 mg, 1.68 mmol) as a yellow solid.
LCMS (ESI) m/z: [M+H]+ =253.2.
¹H NMR (400 MHz, DMSO-d6) δ = 13.63-13.56 (m, 1H), 8.67-8.66 (m, 1H), 7.71 (s, 1H), 7.68-7.66 (m, 1H), 7.55-7.53 (m, 1H), 4.29 (br d, J = 5.6 Hz, 2H), 1.40 (s, 9H) ppm.

### Step 2. tert-Butyl N-[[4-(hydroxymethyl)-2-pyridyl]methyl]carbamate

BH₃·Me₂S (10 M, 0.18 mL) was added dropwise to a solution of 2-[(tert-Butoxycarbonylamino)methyl]pyridine-4-carboxylic acid (150 mg, 0.59 mmol) in THF (2 mL) at 0°C. The mixture was warmed to 25°C and stirred for 12 hrs. MeOH (5 mL) was added to the reaction mixture at 0°C and then warmed and stirred at 70°C for 1 hr. The mixture was then diluted with H₂O(40 mL) and extracted with EA (40 mL x 2). The combined organic layers were dried over anhydrous Na₂SO₄, filtered, and concentrated in vacuo. The crude residue was purified by column chromatography (SiO₂, Petroleum ether/EA = 1/0 to 0/1) affording the title compound (100 mg, 0.42 mmol) as yellow oil.
LCMS (ESI) m/z: [M+H]+ =239.2.
¹H NMR (400 MHz, CDCl₃) δ = 8.50 (d, J = 5.2Hz, 1H), 7.29 - 7.27 (m, 1H), 7.19 (d, J = 4.8 Hz, 1H), 5.56 (br s, 1H), 4.74 (s, 2H), 4.44 (br d, J = 5.6 Hz, 2H), 1.46 (s, 9H) ppm.

### Step 3. tert-Butyl N-[[4-(phenoxymethyl)-2-pyridyl]methyl]carbamate

DIAD (0.098 mL, 0.50 mmol) was added to a solution of tert-Butyl N-[[4-(hydroxymethyl)-2-pyridyl]methyl]carbamate (80 mg, 0.34 mmol), PPh₃ (132 mg, 0.50 mmol), and phenol (0.060 mL, 0.67 mmol) in THF (1 mL) at 0 °C. The mixture was warmed and stirred at 25°C for 16 hr. The mixture was diluted with H₂O (10 mL) and extracted with EA (10 mL x 2). The combined organic layers were dried over anhydrous Na₂SO₄, filtered, and concentrated in vacuo. The crude residue was purified by column chromatography (SiO₂, Petroleum ether/EA = 1/0 to 1/1) affording the title compound (75 mg, 0.24 mmol) as a yellow solid.
LCMS (ESI) m/z: [M+H]+ =315.2.
¹H NMR (400 MHz, CDCl₃) δ = 8.54 (d, J = 5.2 Hz, 1H), 7.34-7.29 (m, 3H), 7.25 (br s, 1H), 7.00-6.94 (m, 3H), 5.54 (br s, 1H), 5.09 (s, 2H), 4.47 (br d, J =4.8 Hz, 2H), 1.47 (s, 9H) ppm.

### Step 4. [4-(Phenoxymethyl)-2-pyridyl]methanamine

A solution of tert-butyl N-[[4-(phenoxymethyl)-2-pyridyl]methyl]carbamate (40 mg, 0.13 mmol) in HCl/dioxane (4N, 1 mL) was stirred at 25 °C for 1 hr. The reaction mixture was concentrated affording the title compound (30 mg, 0.12 mmol, HCl salt) as a white solid.
LCMS (ESI) m/z: [M+H]+ =215.3.

### Step 5. (4R)-4-Cyano-4-methyl-N-[[4-(phenoxymethyl)-2-pyridyl]methyl]isochromane-6-carboxamide

DIEA (0.10 mL, 0.60 mmol), EDCI (34.4 mg, 0.18 mmol), and HOBt (24.2 mg, 0.18 mmol) were added to a solution of (4R)-4-cyano-4-methyl-isochromane-6-carboxylic acid (26 mg, 0.12 mmol) and [4-(phenoxymethyl)-2-pyridyl]methanamine (30 mg, 0.12 mmol, HCl salt) in DCM (0.5 mL). The mixture was stirred at 30°C for 12 hrs. The reaction mixture was diluted with H₂O (3 mL) and extracted with DCM (3 mL x 2). The combined organic layers were dried over anhydrous Na₂SO₄, filtered, and concentrated under reduced pressure. The crude residue was purified by column chromatography (SiO₂, Petroleum ether/EA = 1/0 to 0/1) affording the title compound (18.8 mg, 0.045 mmol) as a white solid.
LCMS (ESI) m/z: [M+H]+ = 414.4.
¹H NMR (400 MHz, CD3OD) δ = 8.49 (d, J = 5.12 Hz, 1H),8.10(d, J = 1.6 Hz, 1H), 7.84-7.82 (m, 1H),7.51-7.40(m, 1H),7.26-7.21(m,3H),6.95-6.91(m,3H) ,5.17 (s, 2H), 4.90 (d, J = 4.0 Hz, 2H), 4.71 (s, 2H), 4.20 (d, J = 11.6 Hz, 1H), 3.92 (d, J = 11.6 Hz, 1H), 1.74 (s, 3H) ppm.

### Examples 11 and 12: (R)-4-Cyano-4-methyl-N-((4-((1r,3R)-3-(6-morpholinopyridin-2-yl)cyclobutyl)pyridin-2-yl)methyl)isochromane-6-carboxamide and (R)-4-cyano-4-methyl-N-((4-((1s,3S)-3-(6-morpholinopyridin-2-yl)cyclobutyl)pyridin-2-yl)methyl)isochromane-6-carboxamide

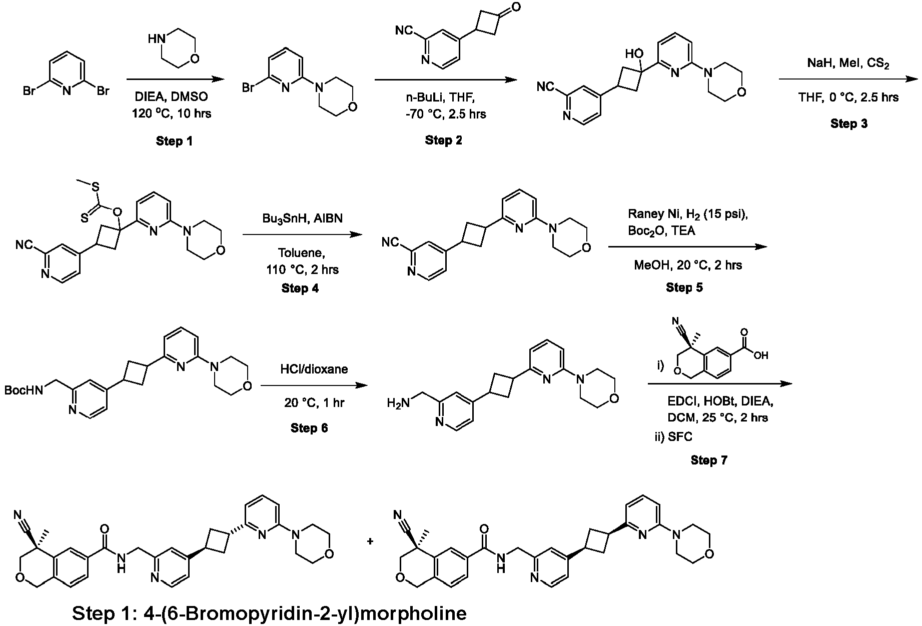

To a mixture of 2,6-dibromopyridine (2 g, 8.44 mmol) and morpholine (2.94 g, 33.77 mmol, 2.97 mL) in DMSO (20 mL) was added DIEA (3.27 g, 25.33 mmol). The mixture was stirred at 120 °C for 10 hrs. The mixture was poured into water (150 mL), then extracted with EA (100 mL * 3). The combined organic layers were washed with brine (250 mL), dried over Na₂SO₄, filtered and concentrated under reduced pressure. The residue was purified by column chromatography (SiO₂, DCM/Ethyl acetate=50/1 to 1/1) to give the title compound (1.6 g, 6.58 mmol, 77.96% yield) as a white solid.
¹H NMR (400 MHz, CDCl₃) δ = 7.34 - 7.30 (m, 1H), 6.80 (d, *J* = 7.6 Hz, 1H), 6.52 (d, *J* = 8.4 Hz, 1H), 3.87 - 3.75(m, 4H), 3.52 - 3.49 (m, 4H) ppm.

### Step 2: 4-(3-Hydroxy-3-(6-morpholinopyridin-2-yl)cyclobutyl)picolinonitrile

To a mixture of 4-(6-bromopyridin-2-yl)morpholine (1.41 g, 5.81 mmol) in THF (10 mL) was added n-BuLi (2.5 M, 2.32 mL) at -70 °C under N₂. The mixture was stirred at -70 °C for 0.5 hr, then the resulting solution was added to a solution of 4-(3-oxocyclobutyl)picolinonitrile (500 mg, 2.90 mmol) in THF (10 mL) at -70 °C under N₂. The mixture was stirred at -70°C for 2 hrs. The mixture was poured into sat. NH₄Cl (100 mL), and extracted with EA (100 mL * 2). The combined organic layers were washed with brine (150 mL), dried over Na₂SO₄, filtered and concentrated under reduced pressure. The residue was purified by column chromatography (SiO₂, Petroleum ether/Ethyl acetate=20/1 to 0/1) to give the title compound (430 mg, 1.25 mmol, 43.14% yield) as a white solid.
LCMS (ESI) m/z: [M+H]⁺ = 337.1
¹H NMR (400 MHz, DMSO-d₆) δ = 8.68 (d, *J* = 5.2Hz, 1H), 8.08 - 8.07 (m, 1H), 7.73-7.71( m, 1H), 7.58 - 7.54 (m,1H), 6.94 (d, *J* = 7.2 Hz, 1H), 6.72 (d, *J* = 8.4 Hz, 1H), 5.84 (s, 1H), 5.75 (s, 1H), 3.74 - 3.72 (m, 4H), 3.49 - 3.47 (m, 4H), 2.95-2.90 (m, 2H), 2.42 - 2.37 (m, 2H) ppm.

### Step 3: O-(3-(2-Cyanopyridin-4-yl)-1-(6-morpholinopyridin-2-yl)cyclobutyl) S-methyl carbonodithioate

To a mixture of NaH (59.45 mg, 1.49 mmol, 60% purity) in THF (2 mL) was added 4-(3-hydroxy-3-(6-morpholinopyridin-2-yl)cyclobutyl)picolinonitrile (200 mg, 594.55 umol) in THF (2 mL) at 0 °C under N₂. The mixture was stirred at 10 °C for 0.5 hr, then CS₂ (242.19 mg, 3.18 mmol, 192.22 uL) was added drop wise at 0 °C. The resulting mixture was stirred at 10 °C for 1hr, then Mel (219.41 mg, 1.55 mmol, 96.23 uL) was added to the mixture drop wise at 0 °C. The resulting mixture was stirred at 10 °C for 1 hr. The reaction mixture was diluted with H₂O (40 mL) and extracted with EA (30 mL*3). The combined organic layers were dried over anhydrous Na₂SO₄, filtered and concentrated. The residue was purified by column chromatography (SiO₂, Petroleum ether/Ethyl acetate=10:1-1:1) to give the title compound (250 mg, 533.34 umol, 89.70% yield) as a yellow oil.
LCMS (ESI) m/z: [M+H]⁺ = 427.1

### Step 4: 4-(3-(6-Morpholinopyridin-2-yl)cyclobutyl)picolinonitrile

To a mixture of O-(3-(2-cyanopyridin-4-yl)-1-(6-morpholinopyridin-2-yl)cyclobutyl) S-methyl carbonodithioate (125 mg, 293.05 umol) in toluene (3 mL) was added Bu₃SnH (255.88 mg, 879.14 umol, 232.62 uL) and AIBN (9.62 mg, 58.61 umol). The mixture was stirred at 110 °C for 2 hrs. The mixture was poured into sat. KF solution (50 mL) and stirred for 15 min. The mixture was extracted with EA (50 mL * 2). The combined organic layers was washed with brine (100 mL), dried over Na₂SO₄, filtered and concentrated. The residue was purified by column chromatography (SiO₂, Petroleum ether/Ethyl acetate=10/1 to 0/1) to give the title compound (80 mg, 187.27 umol, 63.91% yield) as a yellow oil. LCMS (ESI) m/z: [M+H]⁺ = 321.2

### Step 5: tert-Butyl ((4-(3-(6-morpholinopyridin-2-yl)cyclobutyl)pyridin-2-yl)methyl)carbamate

To a mixtrue of 4-(3-(6-morpholinopyridin-2-yl)cyclobutyl)picolinonitrile (80 mg, 249.70 umol) in MeOH (2 mL) and EA (2 mL) was added TEA (75.80 mg, 749.09 umol), (Boc)₂O (108.99 mg, 499.40 umol) and Raney-Ni (50 mg, 583.60 umol). The mixture was stirred at 25 °C for 2 hrs. The mixture was diluted with EA then filtered carefully with EA (15 mL * 3). The filtrate was washed with brine (50 mL), dried over Na₂SO₄, filtered and concentrated under vacuum. The residue was purified by column chromatography (SiO₂, Petroleum ether/Ethyl acetate=10/1 to 0/1) to give the title compound (90 mg, 188.68 umol, 75.56% yield) as a white solid.
LCMS (ESI) m/z: [M+H]⁺ = 425.3.

### Step 6: (4-(3-(6-Morpholinopyridin-2-yl)cyclobutyl)pyridin-2-yl)methanamine hydrochloride

A mixture of tert-butyl ((4-(3-(6-morpholinopyridin-2-yl)cyclobutyl)pyridin-2-yl)methyl)carbamate (90 mg, 212.00 umol) in HCl/dioxane (4 M, 4 mL) was stirred at 25 °C for 1 hr. The mixture was concentrated under vacuum to give the title compound (76 mg, crude, HCl salt) as a white solid, used directly in the next step.
LCMS (ESI) m/z: [M+H]⁺ = 325.2

### Step 7: (R)-4-Cyano-4-methyl-N-((4-((1r,3R)-3-(6-morpholinopyridin-2-yl)cyclobutyl)pyridin-2-yl)methyl)isochromane-6-carboxamide and (R)-4-cyano-4-methyl-N-((4-((1s,3S)-3-(6-morpholinopyridin-2-yl)cyclobutyl)pyridin-2-yl)methyl)isochromane-6-carboxamide

To a mixture of (4-(3-(6-morpholinopyridin-2-yl)cyclobutyl)pyridin-2-yl)methanamine hydrochloride (76 mg, 210.60 umol), (4*R*)-4-cyano-4-methyl-isochromane-6-carboxylic acid (54.89 mg, 252.72 umol) and DIEA (81.65 mg, 631.79 umol, 110.05 uL) in DCM (3 mL) was added EDCI (60.56 mg, 315.89 umol) and HOBt (42.68 mg, 315.89 umol). The mixture was stirred at 25 °C for 2 hrs. The mixture concentrated under vacuum to remove DCM. The residue was purified by prep-TLC (DCM:MeOH=10:1) to give crude racemic product (34 mg). The racemic product was further separated by SFC (column: DAICEL CHIRALPAK AD(250mm*30mm, 10um);mobile phase: [0.1%NH3H2O IPA];B%: 50%-50%,4.5 min;25minmin) to give peak 1 (Rf=0.743 min) and peak 2 (Rf=1.102min). The fraction of peak 1 (Rf=0.743 min) was concentrated under vacuum to give a residue, then purified by reversed phase HPLC (0.1% FA condition) and lyophilized to give (R)-4-cyano-4-methyl-N-((4-((1r,3R)-3-(6-morpholinopyridin-2-yl)cyclobutyl)pyridin-2-yl)methyl)isochromane-6-carboxamide (3.10 mg, 5.92 umol, 2.81% yield) as an off-white solid. The fraction of peak 2 (Rf=1.102 mins) was concentrated under vacuum to give a residue, then purified by reversed phase HPLC(0.1% FA condition) and lyophilized to give (*R*)-4-cyano-4-methyl-*N*-((4-((1*s*,3*S*)-3-(6-morpholinopyridin-2-yl)cyclobutyl)pyridin-2-yl)methyl)isochromane-6-carboxamide (7.55 mg, 14.42 umol, 6.85% yield) as an off-white solid.

### (R)-4-Cyano-4-methyl-N-((4-((1r,3R)-3-(6-morpholinopyridin-2-yl)cyclobutyl)pyridin-2-yl)methyl)isochromane-6-carboxamide

LCMS (ESI) m/z: [M+H]⁺ = 524.2
¹H NMR (400 MHz, MeOD) δ = 8.43 (d, *J* = 4.8 Hz, 1H), 8.12 (d, *J* = 1.6 Hz, 1H), 7.88-7.85 (m, 1H), 7.49-7.45(m, 1H), 7.39 (s, 1H), 7.32 - 7.30 (m, 1H), 7.26 (d, *J* = 8.0 Hz, 1H), 6.62 - 6.58 (m, 2H), 4.90 (s, 2H), 4.70 (s, 2H), 4.19 (d, *J* = 11.2 Hz, 1H), 3.91 (d, *J* = 11.2 Hz, 1H), 3.92 - 3.78 (m, 5H), 3.55 - 3.51 (m, 5H), 2.79-2.72 (m, 2H), 2.56 - 2.49 (m, 2H), 1.74 (s, 3H) ppm.
Chiral SFC: AD-3_5CM_IPA(DEA)_40_3ML_T35.M, Rt= 0.743 min, ee %= 100 %.

### (R)-4-Cyano-4-methyl-N-((4-((1s,3S)-3-(6-morpholinopyridin-2-yl)cyclobutyl)pyridin-2-yl)methyl)isochromane-6-carboxamide

LCMS (ESI) m/z: [M+H]⁺ = 524.2
¹H NMR (400 MHz, MeOD) δ = 8.40 (d, *J* = 4.8 Hz, 1H), 8.10 (d, *J* = 1.2 Hz, 1H), 7.86-7.83 (m, 1H), 7.44-7.41(m, 1H), 7.36 (s, 1H), 7.27 - 7.24 (m, 2H), 6.58 - 6.53 (m, 2H), 4.90 (s, 1H), 4.66 (m, 2H), 4.18 (d, *J* =11.2 Hz, 1H), 3.91 (d, *J* = 11.2 Hz, 1H), 3.76 - 3.73 (m, 4H), 3.56 - 3.42 (m, 6H), 2.72 - 2.64 (m, 2H), 2.50 - 2.42 (m, 2H), 1.72 (s, 3H) ppm.
Chiral SFC: AD-3_5CM_IPA(DEA)_40_3ML_T35.M, Rt= 1.100 mins, ee %= 100 %.

The following examples in Table 11 were prepared using standard chemical manipulations and procedures similar to those used for the preparation of **Examples 11 and 12.**

**Table 11. Compounds of the Invention**

| # | LCMS (m/z) | 1H NMR |
|---|---|---|
| 76 | 482.4 | 1H NMR (400 MHz, DMSO-d6) δ= 8.48 - 8.39 (m, 1H), 8.27 - 8.19 (m, 1H), 8.12 (d, J = 1.6 Hz, 1H), 7.87 - 7.85 (m, 1H), 7.52-7.49 (m, 1H), 7.42 (s, 1H), 7.33 -7.32 (m, 1H), 7.27 (d, J = 8.0 Hz, 1H), 6.60 - 6.56 (m, 2H),4.93 (br s, 2H), 4.71 (s, 2H), 4.20 - 4.17 (m, 1H), 3.91 (d, J = 11.6 Hz, 1H), 3.84 - 3.80 (m, 1H), 3.65 - 3.59 (m, 1H), 3.13 (s, 6H), 2.77 - 2.75 (m, 2H), 2.60 - 2.55 (m, 2H), 1.74 (s, 3H) ppm |
| 77 | 479.3 | 1H NMR (400 MHz, DMSO-d6) δ= 8.44 (d, J = 5.2 Hz, 1H), 8.12 (d, J = 1.6 Hz, 1H), 7.87 - 7.85 (m, 1H), 7.55 - 7.51 (m, 1H), 7.41 (s, 1H), 7.32 (d, J = 5.4 Hz, 1H), 7.26 (d, J = 8.0 Hz, 1H), 7.05 - 6.97 (m, 2H), 4.89 (s, 2H), 471 (s, 2H), 4.19 (d, J = 11.6 Hz, 1H), 3.91 (d, J = 11.6 Hz, 1H), 3.79 - 3.75 (m, 1H), 3.66 - 3.64 (m, 1H), 2.74 - 2.72 (m, 2H), 2.58 - 2.55 (m, 2H), 2.06 - 2.01 (m, 1H), 1.74 (s, 3H), 1.00 - 0.95 (m, 4H) ppm |
| 78 | 479.3 | 1H NMR (400 MHz, DMSO-d6) δ= 8.42 (d, J = 4.8 Hz, 1H), 8.12 (d, J = 2.0 Hz, 1H), 7.87 - 7.85 (m, 1H), 7.55 - 7.50 (m, 1H), 7.38 (s, 1H), 7.31 - 7.30 (m, 1H), 7.26 (d, J = 8.4 Hz, 1H), 6.99 - 6.95 (m, 2H), 4.90 (br s, 2H), 4.70 (s, 2H), 4.19 (d, J = 11.6 Hz, 1H), 3.91 (d, J = 11.6 Hz, 1H), 3.62 - 3.55 (m, 2H), 2.75 - 2.70 (m, 2H), 2.47 - 2.42 (m, 2H), 2.05 - 2.02 (m, 1H), 1.73 (s, 3H), 0.95 - 0.93 (m, 4H) ppm |
| 81 | 552.3 | 1H NMR (400 MHz, DMSO-d6) δ = 9.22 - 9.20 (m, 1H), 8.52 - 8.43 (m, 1H), 8.39 (s, 1H), 8.14 (d, J = 1.6 Hz, 1H), 7.89 - 7.87 (m, 1H), 7.48 - 7.44 (m, 1H), 7.28 - 7.26 (m, 3H), 6.66 - 6.57 (m, 2H), 4.91 - 4.79 (m, 2H), 4.64 - 4.55 (m, 2H), 4.22 - 4.16 (m, 3H), 3.85 (d, J = 11.2 Hz, 1H), 3.68 - 3.48 (m, 5H), 2.68 - 2.62 (m, 2H), 2.46 - 2.34 (m, 4H), 1.68 (s, 3H), 1.14 (d, J = 6.4 Hz, 6H) ppm |
| 82 | 552.3 | 1H NMR (400 MHz, DMSO-d6) δ = 9.20 - 9.17 (m, 1H), 8.44 (d, J = 4.8 Hz, 1H), 8.36 (s, 1H), 8.13 (d, J = 1.6 Hz, 1H), 7.89 - 7.87 (m, 1H), 7.45 - 7.41 (m, 1H), 7.28 - 7.24 (m, 3H), 6.65 (d, J = 8.4 Hz, 1H), 6.55 (d, J = 7.2 Hz, 1H), 4.91 - 4.80 (m, 2H), 4.61 - 4.56 (m, 2H), 4.23 - 4.14 (m, 3H), 3.86 (d, J = 11.6 Hz, 1H), 3.64 - 3.58 (m, 2H), 3.47 (br d, J = 7.6 Hz, 2H), 2.64 - 2.59 (m, 2H), 2.39 - 2.31 (m, 4H), 1.68 (s, 3H), 1.15 (d, J = 6.0 Hz, 6H) ppm |

### Examples 13 and 14: (R)-4-Cyano-N-((4-((1r,3R)-3-(3-methoxyphenyl)cyclobutyl)pyridin-2-yl)methyl)-4-methylisochromane-6-carboxamide and (R)-4-cyano-N-((4-((1s,3S)-3-(3-methoxyphenyl)cyclobutyl)pyridin-2-yl)methyl)-4-methylisochromane-6-carboxamide

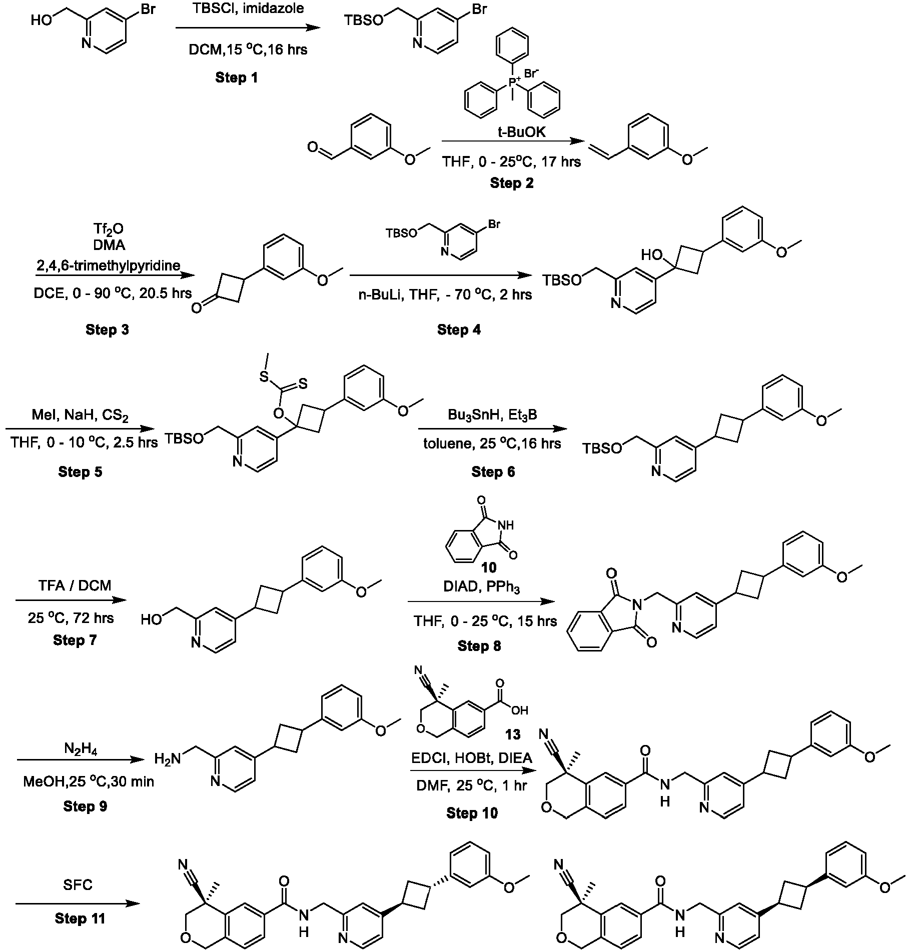

### Step 1: 4-Bromo-2-(((tert-butyldimethylsilyl)oxy)methyl)pyridine

To a mixture of (4-bromo-2-pyridyl)methanol (3 g, 15.96 mmol) and imidazole (1.63 g, 23.93 mmol) in DCM (75 mL) was added TBSCI (2.89 g, 19.15 mmol, 2.35 mL) at 15 °C. The mixture was stirred at 15 °C for 16 hrs. The mixture was filtered and the mother liquor was concentrated under reduced pressure. The residue was purified by silica gel chromatography (PE / EA = 20 / 1 - 5/1) and to afford the title compound (5.4 g, crude) as a yellow oil.
LCMS (ESI) m/z: [⁷⁹BrM+H]⁺ = 302.2.
¹H NMR (400 MHz, CDCl₃) δ = 8.32 (d, *J* = 5.2 Hz, 1H), 7.69 (d, *J* = 0.8 Hz, 1H), 7.35 - 7.27 (m, 1H), 4.82 (s, 2H), 0.98 (s, 9H), 0.14 (s, 6H) ppm.

### Step 2: 1-Methoxy-3-vinylbenzene

To a mixture of methyltriphenylphosphonium bromide (25.19 g, 70.51 mmol) in THF (100 mL) was added t-BuOK (8.57 g, 76.39 mmol) in several portions at 0 °C under N₂. The mixture was stirred at 0 °C for 1 hr under N₂. Then 3-methoxybenzaldehyde (8 g, 58.76 mmol, 7.14 mL) was added dropwise at 0 °C under N₂. The mixture was stirred at 25 °C for 16 hr. The mixture was diluted with PE (100 mL) and filtered. The mother liquor was concentrated under reduced pressure and the residue was purified by silica gel chromatography (PE - PE / EA = 100 / 1) to afford the title compound (4 g, 29.81 mmol, 50.7% yield) as a yellow oil.
¹H NMR (400 MHz, CDCl₃) δ = 7.27 - 7.22 (m, 1H), 7.02 (d, *J* = 7.6 Hz, 1H), 6.98 - 6.94 (m, 1H), 6.84 - 6.81 (m, 1H), 6.73 - 6.66 (m, 1H), 5.78-5.73 (m, 1H), 5.26 (d, *J* = 10.8 Hz, 1H), 3.83 (s, 3H) ppm.

### Step 3: 3-(3-Methoxyphenyl)cyclobutan-1-one

Trifluoromethanesulfonic anhydride (14.72 g, 52.17 mmol, 8.61 mL) was added dropwise at 0 °C under N₂ to a mixture of DMA (3.90 g, 44.72 mmol, 4.16 mL) and DCE (50 mL). The mixture was stirred at 0 °C for 30 min. A mixture of 1-methoxy-3-vinylbenzene (5 g, 37.26 mmol, 5.17 mL) and 2,4,6-trimethylpyridine (6.32 g, 52.17 mmol, 6.89 mL) in DCE (25 mL) was then added dropwise under N₂. The mixture was stirred at 90 °C for 16 hrs. Then the mixture was concentrated to afford a residue which was dissolved in CCl₄ (50 mL) and H₂O (50 mL). The mixture was stirred at 90°C for 4 hrs. The reaction mixture was diluted with H₂O (100 mL) and extracted with DCM (100 mL *2). The combined organic layers were dried over anhydrous Na₂SO₄, filtered and concentrated under reduced pressure. The residue was purified by silica gel chromatography (PE - PE/EA = 20 / 1) to afford the title compound (3.2 g, 18.16 mmol, 48.7% yield) as a yellow oil.
¹H NMR (400 MHz, CDCl₃) δ = 7.32 - 7.28 (m, 1H), 6.91 (d, *J* = 7.6 Hz, 1H), 6.88 - 6.78 (m, 2H), 3.83 (s, 3H), 3.71 - 3.65 (m, 1H), 3.56 - 3.44 (m, 2H), 3.33 - 3.22 (m, 2H) ppm.

### Step 4: 1-(2-(((tert-Butyldimethylsilyl)oxy)methyl)pyridin-4-yl)-3-(3-methoxyphenyl)cyclobutan-1-ol

To a mixture of 4-bromo-2-(((tert-butyldimethylsilyl)oxy)methyl)pyridine (3.29 g, 10.90 mmol) in THF (30 mL) was added n-BuLi (2.5 M, 5.45 mL) dropwise at -70 °C. The mixture was stirred at -70 °C for 1 hr. A solution of 3-(3-methoxyphenyl)cyclobutan-1-one (1.6 g, 9.08 mmol) in THF (10 mL) was then added dropwise at -70 °C. The mixture was stirred at -70 °C for 1 hr. The reaction mixture was added dropwise to H₂O (150 mL) and extracted with EA (100 mL *2). The combined organic layers were dried over anhydrous Na₂SO₄, filtered and concentrated under reduced pressure. The residue was purified by silica gel chromatography (PE / EA = 10 / 1 - 2 / 1) to afford the title compound (1.6 g, 4.00 mmol, 44.1% yield) as a yellow solid.
LCMS (ESI) m/z: [M+H]⁺ = 400.1.
¹H NMR (400 MHz, CDCl₃) δ = 8.56 (d, *J* = 5.2 Hz, 1H), 7.79 (d, *J* = 1.2 Hz, 1H), 7.43 - 7.40 (m, 1H), 7.30 - 7.27 (m, 1H), 6.90 (d, *J* = 7.6 Hz, 1H), 6.86 - 6.83 (m, 1H), 6.80 - 6.77 (m, 1H), 4.90 (s, 2H), 3.83 (s, 3H), 3.27 - 3.16 (m, 1H), 3.06 - 2.94 (m, 2H), 2.66 - 2.55 (m, 2H), 2.19 (s, 1H), 0.99 (s, 9H), 0.16 (s, 6H) ppm.

### Step 5: O-(1-(2-(((tert-Butyldimethylsilyl)oxy)methyl)pyridin-4-yl)-3-(3-methoxyphenyl)cyclobutyl) S-methyl carbonodithioate

To a mixture of NaH (250.23 mg, 6.26 mmol, 60% purity) in THF (8 mL) was added a solution of 1-(2-(((*tert*-Butyldimethylsilyl)oxy)methyl)pyridin-4-yl)-3-(3-methoxyphenyl)cyclobutan-1-ol (1 g, 2.50 mmol) in THF (12 mL) dropwise at 0 °C under N₂. The mixture was stirred at 10 °C for 30 min under N₂. Then CS₂ (1.02 g, 13.39 mmol, 809.05 uL) was added dropwise to the mixture at 0 °C. The mixture was stirred at 10 °C for 1 hr. Then Mel (923.53 mg, 6.51 mmol, 405.06 uL) was added to the mixture dropwise at 0 °C. The mixture was stirred at 10 °C for 1 hr. The reaction mixture was quenched with MeOH (2 mL), diluted with H₂O (40 mL) and extracted with EA (30 mL*3). The combined organic layers were dried over anhydrous Na₂SO₄, filtered and concentrated. The residue was purified by silica gel chromatography (PE / EA = 10 / 1- 3 / 1) to afford the title compound (1.08 g, 2.21 mmol, 88.1% yield) as a yellow solid. LCMS (ESI) m/z: [M+H]⁺ = 490.5.
¹H NMR (400 MHz, CDCl₃) δ = 8.52 (d, *J* = 5.2 Hz, 1H), 7.65 (d, *J* = 0.8 Hz, 1H), 7.26 - 7.23 (m, 2H), 6.87 (d, *J* = 7.6 Hz, 1H), 6.82 - 6.78 (m, 2H), 4.88 (s, 2H), 3.83 (s, 3H), 3.44 - 3.34 (m, 1H), 3.23 - 3.18 (m, 2H), 2.93 - 2.86 (m, 2H), 2.50 (s, 3H), 0.99 (s, 9H), 0.14 (s, 6H) ppm.

### Step 6: 2-(((tert-Butyldimethylsilyl)oxy)methyl)-4-(3-(3-methoxyphenyl)cyclobutyl)pyridine

To a mixture of O-(1-(2-(((*tert*-butyldimethylsilyl)oxy)methyl)pyridin-4-yl)-3-(3-methoxyphenyl)cyclobutyl) S-methyl carbonodithioate (1.08 g, 2.21 mmol) in toluene (11 mL) was added Bu₃SnH (1.93 g, 6.62 mmol, 1.75 mL) and triethylborane (1 M, 6.62 mL) at 25 °C. The mixture was stirred at 25 °C for 16 hrs. The mixture was poured into saturated KF aqueous solution (30 mL) and stirred at 15 °C for 30 min. The mixture was then extracted with EA (30 mL *3). The combined organic layers were washed with brine (30 mL), dried over anhydrous Na₂SO₄, filtered and concentrated under reduced pressure. The residue was purified by silica gel chromatography (PE / EA= 20 / 1 - 5 / 1) to afford the title compound (740 mg, 1.88 mmol, 85.0% yield) as a yellow oil.
LCMS (ESI) m/z: [M+H]⁺ = 384.2.

### Step 7: (4-(3-(3-Methoxyphenyl)cyclobutyl)pyridin-2-yl)methanol

A mixture of 2-(((*tert*-butyldimethylsilyl)oxy)methyl)-4-(3-(3-methoxyphenyl)cyclobutyl)pyridine (740 mg, 1.93 mmol) in DCM (4 mL) and TFA (12.32 g, 108.05 mmol, 8 mL) was stirred at 25 °C for 72 hrs. The mixture was poured into H₂O (50 mL) and the pH adjusted to 9 with NaHCO₃. The mixture was extracted with DCM (30 mL *2). The combined organic layers were dried over anhydrous Na₂SO₄, filtered and concentrated under reduced pressure. The residue was purified by silica gel chromatography (PE / EA = 5 / 1 - EA) to afford the title compound (470 mg, 1.75 mmol, 90.5% yield) as a yellow oil.
LCMS (ESI) m/z: [M+H]⁺ = 270.0.

### Step 8: 2-((4-(3-(3-Methoxyphenyl)cyclobutyl)pyridin-2-yl)methyl)isoindoline-1,3-dione

To a mixture of (4-(3-(3-methoxyphenyl)cyclobutyl)pyridin-2-yl)methanol (200 mg, 742.56 umol), isoindoline-1,3-dione (163.88 mg, 1.11 mmol) and PPh₃ (292.15 mg, 1.11 mmol) in THF (4 mL) was added DIAD (225.23 mg, 1.11 mmol, 216.57 uL) dropwise at 0 °C under N₂. The mixture was stirred at 25 °C for 15 hrs. The mixture was poured into water (30 mL) and extracted with ethyl acetate (20 mL*3). The combined organic phase was washed with brine (20 mL), dried with anhydrous Na₂SO₄, filtered and concentrated. The residue was purified by silica gel chromatography (PE / EA = 10 / 1 - 1 / 1) to afford the title compound (245 mg, 582.96 umol, 78.5% yield) as a yellow oil.
LCMS (ESI) m/z: [M+H]⁺ = 399.3.
¹H NMR (400 MHz, CDCl₃) δ = 8.50 - 8.39 (m, 1H), 7.94 - 7.86 (m, 2H), 7.80 - 7.69 (m, 2H), 7.26 - 6.70 (m, 6H), 5.04 - 4.98 (m, 2H), 3.87 - 3.77 (m, 3H), 3.69 - 3.41 (m, 2H), 2.86 - 2.15 (m, 4H) ppm.

### Step 9: (4-(3-(3-Methoxyphenyl)cyclobutyl)pyridin-2-yl)methanamine

To a mixture of 2-((4-(3-(3-methoxyphenyl)cyclobutyl)pyridin-2-yl)methyl)isoindoline-1,3-dione (245 mg, 614.88 umol) in MeOH (3 mL) was added NH₂NH₂.H₂O (307.81 mg, 6.15 mmol, 298.84 uL). The mixture was stirred at 25°C for 30 min. The mixture was poured into H₂O (20 mL) and extracted with EA (20 mL*3). The combined organic layers were dried over anhydrous Na₂SO₄, filtered and concentrated under reduced pressure to afford the title compound (170 mg, crude) as a yellow oil. LCMS (ESI) m/z: [M+H]⁺ = 269.2.

### Step 10: (R)-4-Cyano-N-((4-(3-(3-methoxyphenyl)cyclobutyl)pyridin-2-yl)methyl)-4-methylisochromane-6-carboxamide

To a mixture of (4-(3-(3-methoxyphenyl)cyclobutyl)pyridin-2-yl)methanamine (80 mg, 298.12 umol) (4*R*)-4-cyano-4-methyl-isochromane-6-carboxylic acid (64.76 mg, 298.12 umol), EDCI (85.72 mg, 447.17 umol) and HOBt (60.42 mg, 447.17 umol) in DMF (1.5 mL) was added DIEA (115.59 mg, 894.35 umol, 155.78 uL). The mixture was stirred at 25 °C for 1 hr. The mixture was poured into H₂O (15 mL) and extracted with EA (10 mL*2). The combined organic layer was washed with brine (10 mL), dried over anhydrous Na₂SO₄, filtered and concentrated under reduced pressure. The residue was purified by silica gel chromatography (PE / EA = 5 / 1 - EA) to afford the title compound (85 mg, 175.04 umol, 58.7% yield) as a yellow solid.
LCMS (ESI) m/z: [M+H]⁺ = 468.4.

### Step 11: (R)-4-Cyano-N-((4-((1r,3R)-3-(3-methoxyphenyl)cyclobutyl)pyridin-2-yl)methyl)-4-methylisochromane-6-carboxamide and (R)-4-cyano-N-((4-((1s,3S)-3-(3-methoxyphenyl)cyclobutyl)pyridin-2-yl)methyl)-4-methylisochromane-6-carboxamide

(*R*)-4-Cyano-*N*-((4-(3-(3-methoxyphenyl)cyclobutyl)pyridin-2-yl)methyl)-4-methylisochromane-6-carboxamide (85 mg, 181.80 umol) was separated by SFC (column: DAICEL CHIRALCEL OD (250mm*30mm, 10um); mobile phase: [0.1%NH₃H₂OIPA]; B%: 50%-50%, 5.0 min; 30 min). The mobile phase **(Peak 1,** Rt=0.487 min) was concentrated under reduced pressure to afford a yellow solid. The yellow solid was purified by reverse phase column (0.1 % FA condition). The eluent was concentrated under reduced pressure to remove MeCN and lyophilized to afford (*R*)-4-cyano-*N*-((4-((1s,3S)-3-(3-methoxyphenyl)cyclobutyl)pyridin-2-yl)methyl)-4-methylisochromane-6-carboxamide (34.42 mg, 73.62 umol, 40.5% yield, 100% purity) as a white solid. The mobile phase **(Peak 2,** Rt=0.679 min) was concentrated under reduced pressure to afford a yellow solid. The yellow solid was purified by reverse phase column (0.1 % FA condition). The eluent was concentrated under reduced pressure to remove MeCN and lyophilized to afford : (*R*)-4-cyano-*N*-((4-((1*r*,3*R*)-3-(3-methoxyphenyl)cyclobutyl)pyridin-2-yl)methyl)-4-methylisochromane-6-carboxamide (14.46 mg, 30.93 umol, 17.01% yield) as a white solid.

### (R)-4-Cyano-N-((4-((1s,3S)-3-(3-methoxyphenyl)cyclobutyl)pyridin-2-yl)methyl)-4-methylisochromane-6-carboxamide:

LCMS (ESI) m/z: [M+H]+ = 468.3.
¹H NMR (400 MHz, Me OD-d4) δ = 8.40 (d, *J* = 5.2 Hz, 1H), 8.11 (d, *J* = 1.6 Hz, 1H), 7.86 - 7.83 (m, 1H), 7.34 (s, 1H), 7.28 - 7.21 (m, 2H), 7.18 - 7.14 (m, 1H), 6.81 - 6.80 (m, 1H), 6.77 - 6.70 (m, 2H), 4.90 (d, *J =* 3.6 Hz, 2H), 4.69 (s, 2H), 4.19 (d, *J* = 11.2 Hz, 1H), 3.91 (d, *J* = 11.2 Hz, 1H), 3.75 (s, 3H), 3.61 - 3.48 (m, 2H), 2.86 - 2.74 (m, 2H), 2.26 - 2.15 (m, 2H), 1.73 (s, 3H) ppm.
Chiral SFC: AD-3_5CM_MEOH(DEA)_5_40_3ML_AT35.M, Rt = 0.493 min, ee% = 100%.

### (R)-4-Cyano-N-((4-((1r,3R)-3-(3-methoxyphenyl)cyclobutyl)pyridin-2-yl)methyl)-4-methylisochromane-6-carboxamide:

LCMS (ESI) m/z: [M+H]⁺ = 468.3.
¹H NMR (400 MHz, MeOD-d4) δ = 8.46 (d, *J* = 5.2 Hz, 1H), 8.14 (d, *J* = 1.2 Hz, 1H), 7.90 - 7.87 (m, 1H), 7.46 (s, 1H), 7.37 (d, *J* = 5.2 Hz, 1H), 7.32 - 7.21 (m, 2H), 6.92 (d, *J* = 7.6 Hz, 1H), 6.87 (s, 1H), 6.78 - 6.75 (m, 1H), 4.92 - 4.91 (m, 2H), 4.74 (s, 2H), 4.20 (d, *J* = 11.2 Hz, 1H), 3.93 (d, *J* = 11.6 Hz, 1H), 3.81 (s, 3H), 3.73 - 3.61 (m, 2H), 2.72 - 2.58 (m, 4H), 1.76 (s, 3H) ppm.
Chiral SFC: Cellucoat-IPA+ACN(DEA)-50-3mL-35T, Rt = 0.639 min, ee% = 100%.

The following examples in Table 12 were prepared using standard chemical manipulations and procedures similar to those used for the preparation of **Examples 13 and 14.**

**Table 12. Compounds of the Invention**

| # | LCMS (m/z) | 1H NMR |
|---|---|---|
| 70 | 468.4 | ¹H NMR (400 MHz, MeOD) δ = 8.30 (d, *J* = 5.2 Hz, 1H), 8.01 (d, *J* = 1.6 Hz, 1H), 7.77-7.74 (m, 1H), 7.24 (s, 1H), 7.16-7.12 (m, 2H), 7.03 (d, *J =* 8.0 Hz, 2H), 6.70 (d, *J* = 8.8 Hz, 2H), 4.81 - 4.79 (m, 2H), 4.58 (s, 2H), 4.09 (d, *J* = 11.6 Hz, 1H), 3.81 (d, *J =* 11.6 Hz, 1H), 3.64 (s, 3H), 3.41-3.35 (m, 2H), 2.68-2.62 (m, 2H), 2.10-2.05 (m, 2H), 1.61 (s, 3H) ppm |
| 72 | 468.4 | 1H NMR (400 MHz, MeOD) δ = 8.44 (d, J = 4.8 Hz, 1H), 8.12 (d, J = 1.6 Hz, 1H), 7.87-7.85 (m, 1H), 7.43 (s, 1H), 7.34 (d, J = 4.8 Hz, 1H), 7.26 (d, J = 8.0 Hz, 1H), 7.23 (d, J = 8.4 Hz, 2H), 6.87 (d, J = 8.4 Hz, 2H), 4.91 - 4.90 (m, 2H), 4.71 (s, 2H), 4.19 (d, J = 11.2 Hz, 1H), 3.92 (d, J = 11.2 Hz, 1H), 3.76 (s, 3H), 3.66-3.57 (m, 2H), 2.60-2.57 (m, 4H), 1.73 (s, 3H) ppm |

### Example 15: (R)-4-Cyano-4-methyl-N-((4-(6-methyl-5-oxo-3,4,5,6-tetrahydro-2,6-naphthyridin-2(1H)-yl)pyridin-2-yl)methyl)isochromane-6-carboxamide

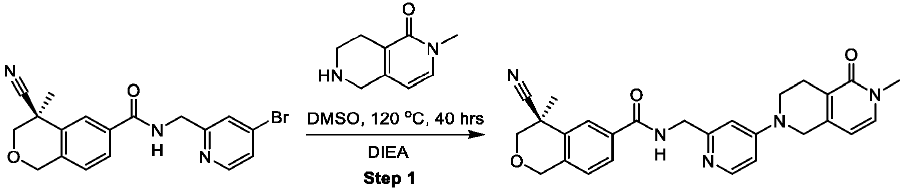

### Step 1: (R)-4-cyano-4-methyl-N-((4-(6-methyl-5-oxo-3,4,5,6-tetrahydro-2,6-naphthyridin-2(1H)-yl)pyridin-2-yl)methyl)isochromane-6-carboxamide

To a mixture of (4*R*)-*N*-[(4-bromo-2-pyridyl)methyl]-4-cyano-4-methyl-isochromane-6-carboxamide (30 mg, 77.67 umol) in DMSO (1 mL) was added 2-methyl-5,6,7,8-tetrahydro-2,6-naphthyridin-1(2H)-one (43.22 mg, 155.34 umol, TFA salt) and DIEA (50.19 mg, 388.36 umol, 67.64 uL) at 30 °C. The mixture was stirred at 120 °C for 40 hrs. The mixture was poured into H₂O (10 mL) and extracted with EA (10 mL *2). The aqueous phase was purified by reverse phase column (0.1 % FA condition). The eluent was concentrated under reduced pressure to remove MeCN and lyophilized to afford the title compound (10.33 mg, 20.04 umol, 25.8% yield, FA salt) as a yellow solid.
LCMS (ESI) m/z: [M+H]⁺ = 470.2.
¹H NMR (400 MHz, MeOD) δ = 8.65 - 8.41 (m, 1H), 8.18 - 8.09 (m, 2H), 7.87 - 7.84 (m, 1H), 7.50 (d, *J* = 6.8 Hz, 1H), 7.27 (d, *J* = 8.0 Hz, 1H), 7.04 (d, *J* = 2.4 Hz, 1H), 6.98 (br d, *J* = 6.4 Hz, 1H), 6.28 (d, *J* = 7.2 Hz, 1H), 4.89 (d, *J* = 2.6 Hz, 2H), 4.64 (s, 2H), 4.48 (s, 2H), 4.19 (d, *J* = 11.6 Hz, 1H), 3.92 (d, *J* = 11.6 Hz, 1H), 3.78 - 3.75 (m, 2H), 3.55 (s, 3H), 2.74 - 2.71 (m, 2H), 1.75 (s, 3H) ppm.
Chiral SFC: AD-3-MeOH+ACN(DEA)-50-3mL-35T, Rt = 0.893 min, ee% = 100%.

The following examples in Table 13 were prepared using standard chemical manipulations and procedures similar to those used for the preparation of **Example 15.**

**Table 13. Compounds of the Invention**

| # | LCMS (m/z) | 1H NMR |
|---|---|---|
| 49 | 487.3 | 1H NMR (400 MHz, DMSO-d6) δ= 9.09 - 9.06 (m, 1H), 8.27 (s, 1H), 8.13 - 8.17 (m, 2H), 7.87 - 7.85 (m, 1H), 7.27 (d, J = 8.0 Hz, 1H), 6.86 (d, J = 2.4 Hz, 1H), 6.80 - 6.78 (m, 1H), 6.75 - 6.71 (m, 1H), 6.67 - 6.64 (m, 1H), 4.91 - 4.80 (m, 2H), 4.50 - 4.41 (m, 4H), 4.21 (d, J = 11.6 Hz, 1H), 3.86 (d, J = 11.6 Hz, 1H), 3.77 (s, 3H), 3.63 - 3.60 (m, 2H), 2.68 - 2.65 (m, 2H), 1.68 (s, 3H) ppm |
| 50 | 453.2 | 1HNMR (400 MHz, MeOD-d4) δ = 9.18 - 9.05 (m, 1H), 8.15 (s, 0.5H), 8.14 (d, J = 1.6 Hz, 1H), 8.10 (d, J = 6.0 Hz, 1H), 7.89 - 7.85 (m, 1H), 7.27 (d, J = 8.0 Hz, 1H), 7.15 - 7.07 (m, 4H), 6.80 - 6.72 (m, 2H), 4.93 - 4.78 (m, 2H), 4.54 - 4.41 (m, 2H), 4.21 (d, J = 11.6 Hz, 1H), 3.86 (d, J = 11.6 Hz, 1H), 3.68 - 3.61 (m, 4H), 2.93 - 2.85 (m, 4H), 1.69 (s, 3H) ppm |
| 51 | 487.3 | 1H NMR (400 MHz, DMSO-d6) δ= 9.09 - 9.06 (m, 1H), 8.27 (s, 1H), 8.13 - 8.17 (m, 2H), 7.87 - 7.85 (m, 1H), 7.27 (d, J = 8.0 Hz, 1H), 6.86 (d, J = 2.4 Hz, 1H), 6.80 - 6.78 (m, 1H), 6.75 - 6.71 (m, 1H), 6.67 - 6.64 (m, 1H), 4.91 - 4.80 (m, 2H), 4.50 - 4.41 (m, 4H), 4.21 (d, J = 11.6 Hz, 1H), 3.86 (d, J = 11.6 Hz, 1H), 3.77 (s, 3H), 3.63 - 3.60 (m, 2H), 2.68 - 2.65 (m, 2H), 1.68 (s, 3H) ppm |
| 53 | 426.4 | 1H NMR (400 MHz, DMSO-d6) δ = 9.17-9.14 (m, 1H), 8.64 (s, 1H), 8.52 (d, J = 4.8 Hz, 1H), 8.18 - 8.16 (m, 2H), 7.91-7.88 (m, 1H), 7.47 (d, J = 5.2 Hz, 1H), 7.29 (d, J = 8.0 Hz, 1H), 6.61 (d, J = 2.0 Hz, 1H), 6.56-6.54 (m, 1H), 4.92 - 4.81 (m, 2H), 4.70 (d, J = 4.8 Hz, 4H), 4.58 - 4.45 (m, 2H), 4.22 (d, J = 11.2 Hz, 1H), 3.88 (d, J = 11.6 Hz, 1H), 1.70 (s, 3H) ppm |
| 54 | 469.1 | 1HNMR (400 MHz, DMSO-d6) δ = 9.13 - 9.04 (m, 1H), 8.12 (s, 1H), 8.05 (d, J = 6.0 Hz, 1H), 7.91 - 7.83 (m, 1H), 7.54 (d, J = 7.2 Hz, 2H), 7.40 - 7.32 (m, 2H), 7.31 - 7.21 (m, 2H), 6.47 - 6.36 (m, 2H), 5.64 - 5.35 (m, 1H), 4.93 - 4.76 (m, 2H), 4.49 - 4.40 (m, 2H), 4.20 (d, J = 11.6 Hz, 1H), 3.84 (d, J = 11.6 Hz, 1H), 3.56 - 3.45 (m, 4H), 2.43 - 2.34 (m, 1H), 2.24 - 2.11 (m, 1H), 1.66 (s, 3H) ppm |
| 55 | 467.3 | 1H NMR (400 MHz, MeOD) δ = 8.10 (s, 1H), 8.02 (d, J = 7.2 Hz, 1H), 7.87 - 7.85 (m, 1H), 7.32- 7.22 (m, 6H), 7.11 - 7.04 (m, 2H), 4.89 (d, J = 2.0 Hz, 2H), 4.65 (s, 2H), 4.33 - 4.25 (m, 1H), 4.24 - 4.13 (m, 2H), 3.93 - 3.91 (m, 1H), 3.30 - 3.23 (m, 2H), 2.89 - 2.72 (m, 1H), 2.09 - 2.06 (m, 1H), 2.02 - 1.86 (m, 2H), 1.71 (d, J = 5.2 Hz, 4H) ppm |
| 56 | 469.1 | 1HNMR (400 MHz, DMSO-d6) δ = 13.34 - 13.30 (m, 1H), 9.27 - 9.25 (m, 1H), 8.23-8.18 (m, 1H), 8.12 (d, J = 1.2 Hz, 1H), 7.88-7.86 (m, 1H), 7.31 - 7.20 (m, 4H), 6.91 - 6.85 (m, 2H), 4.92 - 4.78 (m, 4H), 4.64 - 4.62 (m, 2H), 4.21 (d, J= 11.6 Hz, 1H), 3.87 -3.85(m, 3H), 3.80 (s, 3H), 2.87-2.84 (m, 2H), 1.68 (s, 3H) ppm |
| 57 | 445.3 | 1H NMR (400 MHz, DMSO-d6) δ = 9.27 - 9.24 (m, 1H), 8.24 - 8.06 (m, 2H), 7.90 - 7.88 (m, 1H), 7.30 (d, J = 8.0 Hz, 1H), 7.13 (br s, 1H), 7.08 - 6.99 (m, 1H), 5.00 - 4.76 (m, 2H), 4.57 (br d, J = 5.6 Hz, 2H), 4.30 - 4.15 (m, 2H), 4.02 (br d, J = 13.2 Hz, 1H), 3.86 (d, J = 11.6 Hz, 1H), 3.07 - 3.05 (m, 1H), 2.74 - 2.70 (m, 1H), 1.78 - 1.54 (m, 7H), 1.31 - 1.19 (m, 3H), 1.18 - 1.05 (m, 2H), 1.04 - 0.88 (m, 2H) ppm |
| 58 | 483.1 | 1HNMR (400 MHz, DMSO-d6) δ = 9.29 (s, 1H), 8.21 - 8.06 (m, 2H), 7.89 (d, J = 8.0 Hz, 1H), 7.50 - 7.36 (m, 5H), 7.29 (d, J = 8.0 Hz, 1H), 6.92 - 6.70 (m, 2H), 4.96 - 4.78 (m, 2H), 4.59 (d, J = 5.6 Hz, 2H), 4.22 (d, J = 11.2 Hz, 1H), 4.07 (d, J = 12.8 Hz, 1H), 3.86 (d, J = 11.6 Hz, 1H), 3.65 (d, J = 12.0 Hz, 2H), 3.59 - 3.51 (m, 1H), 2.92 (s, 3H), 2.72 - 2.65 (m, 1H), 2.42 - 2.31 (m, 1H), 1.68 (s, 3H) ppm |
| 59 | 478.1 | 1H NMR (400 MHz, DMSO-d6) δ = 9.16 - 9.12 (m, 1H), 8.18 - 8.10 (m, 2H), 7.89 - 7.86 (m, 1H), 7.67 (d, J = 3.2 Hz, 1H), 7.59 - 7.53 (m, 2H), 7.26 (d, J = 8.0 Hz, 1H), 7.19 - 7.12 (m, 1H), 7.10 - 7.01 (m, 1H), 6.53 (d, J = 3.2 Hz, 1H), 6.46 (d, J = 2.0 Hz, 1H), 6.42 - 6.40 (m, 1H), 5.68 - 5.54 (m, 1H), 4.93 - 4.75 (m, 2H), 4.56 - 4.40 (m, 4H), 4.28 - 4.18 (m, 3H), 3.85 (d, J = 11.6 Hz, 1H), 1.68 (s, 3H) ppm |
| 63 | 489.2 | 1H NMR (400 MHz, DMSO-d6) δ = 9.09 - 9.06 (m, 1H), 8.21 - 8.08 (m, 2H), 7.87 (d, J = 8.0 Hz, 1H), 7.45 (s, 1H), 7.41 - 7.32 (m, 2H), 7.27 (d, J = 8.0 Hz, 1H), 7.14 - 6.84 (m, 2H), 6.83 - 6.77 (m, 1H), 4.98 - 4.76 (m, 2H), 4.57 (s, 2H), 4.54 - 4.42 (m, 2H), 4.21 (d, J = 11.2 Hz, 1H), 3.86 (d, J = 11.2 Hz, 1H), 3.63 - 3.60 (m, 2H), 2.95 (s, 2H), 1.68 (s, 3H) ppm. |
| 66 | 457.1 | 1H NMR (400 MHz, MeOD) δ = 8.10 (d, J = 2.0 Hz, 1H), 8.04 (d, J = 5.6 Hz, 1H), 7.84-7.82 (m, 1H), 7.24 (d, J = 8.0 Hz, 1H), 7.18-7.17(m, 4H), 6.98-6.95 (m, 1H), 4.87 (s, 2H), 4.72 (d, J = 2.4 Hz, 2H), 4.54 (s, 2H), 4.19 (d, J = 11.6 Hz, 1H), 3.91 (d, J =11.2 Hz, 1H), 3.73-3.70 (m, 2H), 3.00-2.97 (m, 2H), 1.73 (s, 3H) ppm. |
| 67 | 489.2 | 1H NMR (400 MHz, DMSO-d6) δ = 9.11 - 9.08 (m, 1H), 8.25 (s, 1H), 8.18 - 8.10 (m, 2H), 7.88 - 7.86 (m, 1H), 7.39 (d, J = 9.6 Hz, 3H), 7.27 (d, J = 8.0 Hz, 1H), 7.14 - 6.84 (m, 2H), 6.81 - 6.78 (m, 1H), 4.94 - 4.78 (m, 2H), 4.58 - 4.41 (m, 4H), 4.21 (d, J = 11.2 Hz, 1H), 3.86 (d, J = 11.6 Hz, 1H), 3.63 -3.60 (m, 2H), 2.96 - 2.94 (m, 2H), 1.68 (s, 3H) ppm. |
| 68 | 443.3 | 1H NMR (400 MHz, MeOD) δ = 8.29 (br s, 1H), 8.11 (d, J = 1.6 Hz, 1H), 7.98 (d, J = 6.0 Hz, 1H), 7.86-7.84 (m, 1H), 7.38-7.36 (m, 2H), 7.33-7.31 (m, 2H), 7.25 (d, J = 8.0 Hz, 1H), 6.79-6.75 (m, 1H), 4.98 (d, J = 3.2 Hz, 4H), 4.88 (s, 2H), 4.73 (d, J = 2.0 Hz, 2H), 4.19 (d, J = 11.6 Hz, 1H), 3.91 (d, J = 11.6 Hz, 1H), 1.73 (s, 3H) ppm. |
| 75 | 440.3 | 1H NMR (400 MHz, MeOD) δ = 8.85 (d, J = 3.2 Hz, 1H), 8.11 (d, J = 1.6 Hz, 1H), 7.87 - 7.84 (m, 1H), 7.29 - 7.18 (m, 5H), 6.99 (d, J = 2.8 Hz, 1H), 4.89 (d, J = 2.8 Hz, 2H), 4.73 (s, 2H), 4.61 (s, 2H), 4.18 (d, J = 11.2 Hz, 1H), 3.91 (d, J = 11.6 Hz, 1H), 3.73 - 3.69 (m, 2H), 3.02 - 2.98 (m, 2H), 1.74 (s, 3H) ppm |

### Example 16: (4R)-4-Cyano-N-((4-(3-fluoro-3-phenylpyrrolidin-1-yl)pyridin-2-yl)methyl)-4-methylisochromane-6-carboxamide

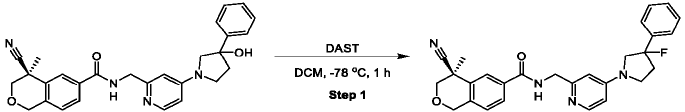

### Step 1: (4R)-4-Cyano-N-((4-(3-fluoro-3-phenylpyrrolidin-1-yl)pyridin-2-yl)methyl)-4-methylisochromane-6-carboxamide

To a solution of (4*R*)-4-cyano-*N*-((4-(3-hydroxy-3-phenylpyrrolidin-1-yl)pyridin-2-yl)methyl)-4-methylisochromane-6-carboxamide (20 mg, 42.69 umol) in DCM (0.5 mL) was added DAST (34.40 mg, 213.43 umol, 28.20 uL) at -78 °C. The mixture was stirred at -78 °C for 1 hr then poured into water (30 mL) and extracted with EA (10 mL *3). The combined organic layers were washed with brine (10 mL), dried over Na₂SO₄, filtered and concentrated. The residue was purified by prep-HPLC (column: Phenomenex Gemini NX-C18 (75*30mm*3um); mobile phase: [water (10mM NH₄HCO₃)-ACN]; B%: 38%-58%, 8min). The product fraction was concentrated under vacuum to remove MeCN and lyophilized to give the title compound (3.55 mg, 7.27 umol, 17.04% yield) as a white solid.
LCMS (ESI) m/z: [M+H]⁺ = 471.1.
¹HNMR (400 MHz, MeOD-d₄) δ = 8.15 - 803 (m, 2H), 7.89 - 7.77 (m, 1H), 7.54 (d, *J* = 7.6 Hz, 2H), 7.48 - 7.34 (m, 3H), 7.25 (d, *J* = 8.4 Hz, 1H), 6.59 (s, 1H), 6.56 - 6.44 (m, 1H), 4.88 (br d, *J* = 3.2 Hz, 3H), 4.17 (d, *J* = 11.2 Hz, 1H), 3.96 - 3.79 (m, 3H), 3.78 - 3.58 (m, 3H), 2.74 - 2.47 (m, 2H), 1.72 (s, 3H) ppm. Chiral SFC: IC-3-EtOH (DEA)-60-3mL-35T.lcm; Rt= 1.386 mins, Rt= 1.826 mins.

### Example 17: (R)-4-Cyano-N-((4-((3-cyanobenzyl)oxy)pyridin-2-yl)methyl)-4-methylisochromane-6-carboxamide

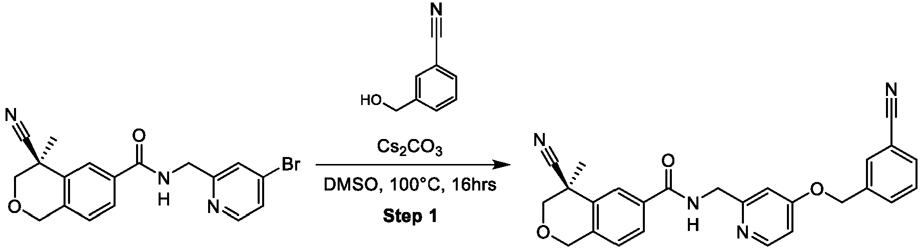

### Step 1: (R)-4-Cyano-N-((4-((3-cyanobenzyl)oxy)pyridin-2-yl)methyl)-4-methylisochromane-6-carboxamide

To a mixture of (*R*)-*N*-((4-bromopyridin-2-yl)methyl)-4-cyano-4-methylisochromane-6-carboxamide (100 mg, 258.91 umol) in DMSO (1 mL) was added Cs₂CO₃ (168.71 mg, 517.81 umol) and 3-(hydroxymethyl) benzonitrile (68.94 mg, 517.81 umol). The mixture was stirred at 100 °C for 16 hrs. Water (20 mL) was added and the mixture was extracted with EtOAc (20 mL*2). The combined organic phase was washed with brine (20 mL), dried over anhydrous Na₂SO₄, filtered and concentrated under vacuum. The residue was purified by prep. HPLC (FA condition) and the product fraction was concentrated under vacuum to remove MeCN followed by lyophilization to give the title compound (15.18 mg, 31.74 umol, 12.26% yield) as a white solid.
LCMS (ESI) m/z: [M+H]⁺=439.1
¹H NMR (400 MHz, DMSO-d6) δ = 9.18-9.15 (m, 1H), 8.36 (d, *J* = 5.6 Hz, 1H), 8.12 (d, *J* = 1.2 Hz, 1H), 7.90 - 7.86 (m, 2H), 7.81 - 7.76 (m, 2H), 7.59 - 7.55 (m, 1H), 7.27 (d, *J* = 8.4 Hz, 1H), 6.97-6.95 (m, 2H), 5.23 (s, 2H), 4.91 - 4.80 (m, 2H), 4.54 - 4.52 (m, 2H), 4.21 (d, *J* = 11.2 Hz, 1H), 3.85 (d, *J* = 11.6 Hz, 1H), 1.68 (s, 3H) ppm.
Chiral SFC: Cellucoat-MeOH(DEA)-40-3mL-35T.lcm, Rt= 0.680 min, ee value= 100 %.

The following examples in Table 14 were prepared using standard chemical manipulations and procedures similar to those used for the preparation of **Example 17.**

**Table 14. Compounds of the Invention**

| # | LCMS (m/z) | 1H NMR |
|---|---|---|
| 64 | 439.1 | 1H NMR (400 MHz, MeOD) δ = 8.38 - 8.32 (m, 1 H), 8.12 - 8.08 (m, 1 H), 7.84 - 7.80 (m, 1 H), 7.69 - 7.63 (m, 2 H),7.60 - 7.55 (m, 2 H), 7.30 - 7.25 (m, 1 H), 7.04 - 7.00 (m, 2 H), 5.35 - 5.29 (m, 2 H), 4.93 - 4.90 (m, 2 H), 4.66 - 4.63 (m, 2 H), 4.26-3.90 (m, 2 H), 1.75 (s, 3 H) ppm |

### Example 18: (R)-N-((4-(Benzylthio)pyridin-2-yl)methyl)-4-cyano-4-methylisochromane-6-carboxamide

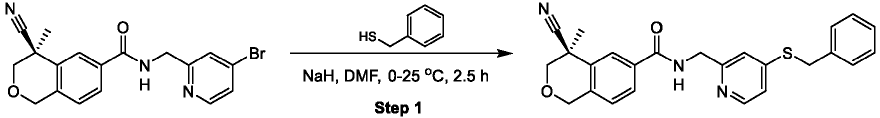

### Step 1: (R)-N-((4-(Benzylthio)pyridin-2-yl)methyl)-4-cyano-4-methylisochromane-6-carboxamide

To a solution of phenylmethanethiol (28.94 mg, 233.01 umol, 27.30 uL) in DMF (0.5 mL) was added NaH (8.70 mg, 217.48 umol, 60% purity) at 0 °C. The reaction mixture stirred at 0 °C for 0.5 hr. (*R*)-*N*-((4-bromopyridin-2-yl)methyl)-4-cyano-4-methylisochromane-6-carboxamide (30 mg, 77.67 umol) was then added into the mixture. The reaction was stirred at 25 °C for 2 hrs. The mixture was poured into water (60 mL) and extracted with EA (30 mL *3). The combined organic layers were washed with brine (30 mL), dried over Na₂SO₄, filtered and concentrated. The crude product was purified by prep-TLC (PE/EA=0/1) and lyophilized to give the title compound (23.19 mg, 53.16 umol, 68.45% yield) as a white solid.
LCMS (ESI) m/z: [M+H]⁺ = 430.0
¹HNMR (400 MHz, MeOD-d₄) δ = 8.23 (d, *J* = 5.6 Hz, 1H), 8.11 (d, *J* = 1.6 Hz, 1H), 7.90 - 7.79 (m, 1H), 7.39 - 7.31 (m, 2H), 7.29 - 7.24 (m, 2H), 7.23 - 7.12 (m, 4H), 4.90 (d, *J* = 4.0 Hz, 2H), 4.61 (s, 2H), 4.28 (s, 2H), 4.19 (d, *J* = 11.6 Hz, 1H), 3.91 (d, *J* = 11.2 Hz, 1H), 1.74 (s, 3H) ppm.
Chiral SFC: AD-3-MeOH+ACN (DEA)-60-3mL-35T.Icm; Rt= 0.585 mins, ee% =100%.

### Example 19: (4R)-4-Cyano-N-((4-(1-(6-(difluoromethyl)pyridin-2-yl)pyrrolidin-3-yl)pyridin-2-yl)methyl)-4-methylisochromane-6-carboxamide

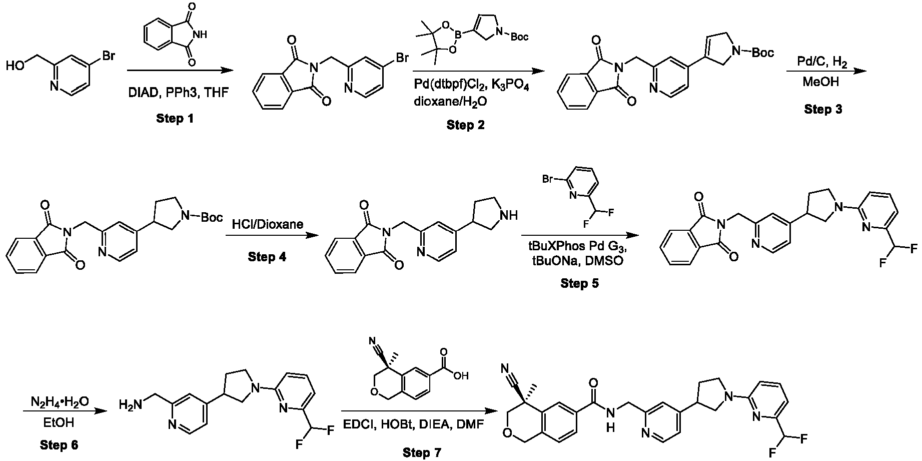

### Step 1: 2-((4-Bromopyridin-2-yl)methyl)isoindoline-1,3-dione

To a solution of (4-bromopyridin-2-yl)methanol (20 g, 106.37 mmol), isoindoline-1,3-dione (23.48 g, 159.56 mmol) and PPh₃ (41.85 g, 159.56 mmol) in THF (400 mL) was added DIAD (32.26 g, 159.56 mmol) at 0 °C. The resulting mixture was stirred at 30 °C for 2 hrs. The reaction mixture was poured into water (1000 mL) and EA (1000 mL), the solution was filtered and the filter cake was dried under vacuum to give the title compound (32 g, 100.90 mmol, 94.86% yield) as a white solid.

### Step 2: tert-Butyl 3-(2-((1,3-dioxoisoindolin-2-yl)methyl)pyridin-4-yl)-2,5-dihydro-1H-pyrrole-1-carboxylate

To a solution of 2-((4-bromopyridin-2-yl)methyl)isoindoline-1,3-dione (700 mg, 2.21 mmol) and *tert*-butyl 3-(4,4,5,5-tetramethyl-1,3,2-dioxaborolan-2-yl)-2,5-dihydro-1*H*-pyrrole-1-carboxylate (977.31 mg, 3.31 mmol) in dioxane (8 mL) and H₂O (2 mL) was added [1, 1'-bis(di-tert-butylphosphino)ferrocene]dichloropalladium(ll) (143.86 mg, 220.72 umol) and K₃PO₄ (937.06 mg, 4.41 mmol) at 25 °C under N₂ atmosphere. The mixture was stirred for 2 hr at 60 °C. Water (10 mL) was added and the mixture extracted with EA (20 mL *3). The combined organic layer was dried over Na₂SO₄, filtered and concentrated under vacuum. The residue was purified by column chromatography (SiO₂, Petroleum ether/Ethyl acetate=100/1 to 1/100) to give the title compound (890 mg, 2.20 mmol, 99.45% yield) as a yellow oil.
LCMS (ESI) m/z: [M+H]⁺ = 406.0.
¹HNMR (400 MHz, MeOD-d₄) δ = 8.43 - 8.37 (m, 1H), 7.93 - 7.88 (m, 2H), 7.86 - 7.81 (m, 2H),
7.46 (d, J = 4.4 Hz, 1H), 7.36 - 7.33 (m, 1H), 6.66 - 6.58 (m, 1H), 4.99 (s, 2H), 4.55 - 4.43 (m, 2H), 4.36 - 4.27 (m, 2H), 1.51 (d, J = 5.6 Hz, 9H) ppm.

### Step 3: tert-Butyl 3-(2-((1,3-dioxoisoindolin-2-yl)methyl)pyridin-4-yl)pyrrolidine-1-carboxylate

To a solution of *tert*-butyl 3-(2-((1,3-dioxoisoindolin-2-yl)methyl)pyridin-4-yl)-2,5-dihydro-1*H-*pyrrole-1-carboxylate (890 mg, 2.20 mmol) in MeOH (150 mL) was added Pd/C (100 mg, 220.35 umol, 10% purity) under H₂ atmosphere, then the mixture was stirred under hydrogen atmosphere (20 Psi) for 2 hrs at 25 °C. The mixture was filtered and the filtrate was concentrated to give the title compound (890 mg, 2.18 mmol, 99.51% yield) as a yellow oil which was used directly in the next step.
LCMS (ESI) m/z: [M+H]⁺ = 408.0.

### Step 4: 2-((4-(Pyrrolidin-3-yl)pyridin-2-yl)methyl)isoindoline-1,3-dione hydrochloride

To a solution of *tert*-butyl 3-(2-((1,3-dioxoisoindolin-2-yl)methyl)pyridin-4-yl)pyrrolidine-1-carboxylate (300 mg, 515.39 umol) in dioxane (3 mL) was added HCl/dioxane (4 M, 3.00 mL). The mixture was stirred at 25 °C for 0.5 hr and then concentrated to give the title compound (160 mg, 465.38 umol, 90.30% yield) as yellow oil which was used directly in the next step.
LCMS (ESI) m/z: [M+H]⁺ = 308.0.

### Step 5: 2-((4-(1-(6-(Difluoromethyl)pyridin-2-yl)pyrrolidin-3-yl)pyridin-2-yl)methyl)isoindoline-1,3-dione

To a solution of 2-((4-(pyrrolidin-3-yl)pyridin-2-yl)methyl)isoindoline-1,3-dione hydrochloride (160 mg, 465.38 umol) and 2-bromo-6-(difluoromethyl) pyridine (387.20 mg, 1.86 mmol) in DMSO (2 mL) was added DIEA (601.45 mg, 4.65 mmol, 810.59 uL) at 25 °C, then the mixture was stirred for 12 hrs at 140 °C. Water (3 mL) was added and the mixture extracted with EA (5 mL *3). The combined organic layer was dried over Na₂SO₄, filtered and concentrated. The residue was purified by column chromatography (SiO₂, PE: EA=1:1, Rf =0.3) to give the title compound (200 mg, 460.37 umol, 98.92% yield) as a yellow solid.
LCMS (ESI) m/z: [M+H]⁺ = 435.0.

### Step 6: (4-(1-(6-(Difluoromethyl)pyridin-2-yl)pyrrolidin-3-yl)pyridin-2-yl)methanamine

To a solution of 2-((4-(1-(6-(difluoromethyl)pyridin-2-yl)pyrrolidin-3-yl)pyridin-2-yl)methyl)isoindoline-1,3-dione (50 mg, 115.09 umol) in EtOH (0.5 mL) was added N₂H₄•H₂O (8.82 mg, 172.64 umol, 8.56 uL, 98% purity) at 25 °C, then the mixture was stirred at 75 °C for 2 hrs. The mixture was filtered and the filtrate was concentrated to give the title compound (30 mg, 98.57 umol, 85.65% yield) as a yellow solid which was used directly in the next step.
LCMS (ESI) m/z: [M+H]⁺ = 305.0.

### Step 7: (4R)-4-Cyano-N-((4-(1-(6-(difluoromethyl)pyridin-2-yl)pyrrolidin-3-yl)pyridin-2-yl)methyl)-4-methylisochromane-6-carboxamide

To a solution of (4-(1-(6-(difluoromethyl)pyridin-2-yl)pyrrolidin-3-yl)pyridin-2-yl)methanamine (20 mg, 65.72 umol), (*R*)-4-cyano-4-methylisochromane-6-carboxylic acid (14.27 mg, 65.72 umol) in DMF (0.2 mL) was added EDCI (25.20 mg, 131.43 umo), HOBt (17.76 mg, 131.43 umol) and DIEA (21.23 mg, 164.29 umol, 28.62 uL). The mixture was stirred at 25 °C for 1 hr. Water (3 mL) was added and the mixture extracted with EA (5 mL *3). The combined organic layer was dried over Na₂SO₄, filtered and concentrated. The crude product was purified by prep-HPLC (column: Phenomenex Synergi C18 150*25*10um; mobile phase: [water (0.225%FA)-ACN]; B%: 33%-63%, 8.5min) and lyophilized to give the title compound (8 mg, 15.77 umol, 24.00% yield) as a yellow solid.
LCMS (ESI) m/z: [M+H]⁺ = 504.3.
¹HNMR (400 MHz, MeOD-d₄) δ = 8.46 (d, J = 5.2 Hz, 1H), 8.11 (d, J = 1.6 Hz, 1H), 7.84 - 7.80 (m, 1H), 7.61 - 7.55 (m, 1H), 7.39 (d, J = 5.6 Hz, 1H), 7.32 (d, J = 5.2 Hz, 1H), 7.24 (d, J = 8.0 Hz, 1H), 6.84 - 6.81 (m, 1H), 6.60 - 6.30 (m, 2H), 4.91 (d, J = 4.0 Hz, 2H), 4.71 (s, 2H), 4.23 - 4.19 (m, 1H), 3.98 - 3.90 (m, 2H), 3.70 - 3.54 (m, 4H), 2.55 - 2.44 (m, 1H), 2.21 - 2.08 (m, 1H), 1.75 (d, J = 1.6 Hz, 3H) ppm.
Chiral SFC: AD-3-MeOH+ACN (DEA)-40-3ML-35T.Icm, Rt = 0.974 min/Rt= 1.137 min.

The following examples in Table 15 were prepared using standard chemical manipulations and procedures similar to those used for the preparation of **Example 19.**

**Table 15. Compounds of the Invention**

| # | LCMS (m/z) | 1H NMR |
|---|---|---|
| 62 | 453.2 | 1HNMR (400 MHz, CDCl3) δ = 8.51 (d, J = 5.2 Hz, 1H), 8.02 (d, J = 1.6 Hz, 1H), 7.80 - 7.76 (m, 1H), 7.61 - 7.53 (m, 1H), 7.26 (d, J = 4.0 Hz, 2H), 7.17 - 7.12 (m, 2H), 6.76 - 6.71 (m, 1H), 6.62 (d, J = 8.0 Hz, 2H), 4.88 (s, 2H), 4.76 (d, J = 4.8 Hz, 2H), 4.14 (d, J = 11.2 Hz, 1H), 3.94 (d, J = 11.6 Hz, 1H), 3.77 - 3.72 (m, 1H), 3.58 - 3.51 (m, 2H), 3.50 - 3.39 (m, 2H), 2.53 - 2.42 (m, 1H), 2.18 - 2.11 (m, 1H), 1.77 (s, 3H) ppm |

### Example 20: (R)-4-Cyano-N-((4-((3-((2-methoxyethyl)carbamoyl)phenyl)ethynyl)pyridin-2-yl)methyl)-4-methylisochromane-6-carboxamide

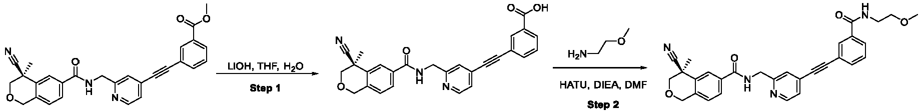

### Step 1: (R)-3-((2-((4-Cyano-4-methylisochromane-6-carboxamido)methyl)pyridin-4-yl)ethynyl)benzoic acid

A solution of methyl (*R*)-3-((2-((4-cyano-4-methylisochromane-6-carboxamido)methyl)pyridin-4-yl)ethynyl)benzoate (226.00 mg, 0.485 mmol, 1.00 equiv), LiOH.H₂O (61.12 mg, 1.456 mmol, 3.00 equiv) and H₂O (1.00 mL) in THF (5.00 mL) was stirred overnight at room temperature. The mixture was acidified to pH 5 with 2M HCl (aq.). The resulting mixture was diluted with water (30 mL) and extracted with EtOAc (3 x 40 mL). The combined organic layers were washed with brine (60 mL), dried over anhydrous Na₂SO₄. After filtration, the filtrate was concentrated under reduced pressure to afford the title compound (208 mg, 94.89%) as a light yellow solid.
LCMS (ESI) m/z: [M+H]⁺ = 452.
¹H NMR (300 MHz, DMSO-*d₆*) δ 13.23 (s, 1H), 9.28 (t, *J =* 5.9 Hz, 1H), 8.60 (dd, *J* = 5.0, 0.9 Hz, 1H), 8.19 - 8.07 (m, 2H), 8.01 (dt, *J* = 7.8, 1.5 Hz, 1H), 7.92 (dd, *J =* 80, 1.7 Hz, 1H), 7.85 (dt, *J* = 7.7, 1.5 Hz, 1H), 7.59 (t, *J* = 7.8 Hz, 1H), 7.54 - 7.44 (m, 2H), 7.29 (d, *J* = 8.1 Hz, 1H), 4.98 - 4.75 (m, 2H), 4.63 (d, *J* = 5.8 Hz, 2H), 4.22 (d, *J* = 11.5 Hz, 1H), 3.86 (d, *J* = 11.5 Hz, 1H), 1.70 (s, 3H).

### Step 2: (R)-4-Cyano-N-((4-((3-((2-methoxyethyl)carbamoyl)phenyl)ethynyl)pyridin-2-yl)methyl)-4-methylisochromane-6-carboxamide

To a solution of (*R*)-3-((2-((4-cyano-4-methylisochromane-6-carboxamido)methyl)pyridin-4-yl)ethynyl)benzoic acid (20.00 mg, 0.044 mmol, 1.00 equiv) and 2-methoxyethan-1-amine (3.99 mg, 0.053 mmol, 1.2 equiv) in DMF (1.00 mL) was added HATU (25.27 mg, 0.066 mmol, 1.5 equiv) and DIEA (17.18 mg, 0.133 mmol, 3 equiv) at room temperature. The resulting mixture was stirred for 1 h at room temperature. The reaction solution was purified by reverse flash chromatography to afford the title compound (10 mg, 44.39%) as a white solid.
LCMS (ESI) m/z [M+H]⁺ =509.10.
¹H NMR (400 MHz, DMSO-d6) δ 9.30 (t, *J* = 5.9 Hz, 1H), 8.63 (dd, *J* = 22.5, 5.2 Hz, 2H), 8.16 (d, *J* = 1.7 Hz, 1H), 8.07 (d, *J* = 1.8 Hz, 1H), 7.92 (dq, *J* = 8.0, 1.7 Hz, 2H), 7.75 (dt, *J* = 7.7, 1.4 Hz, 1H), 7.55 (t, *J =* 7.7 Hz, 1H), 7.46 (dd, *J* = 6.9, 1.7 Hz, 2H), 7.29 (d, *J* = 8.1 Hz, 1H), 4.96 - 4.78 (m, 2H), 4.63 (d, *J* = 5.9 Hz, 2H), 4.22 (d, *J* = 11.5 Hz, 1H), 3.86 (d, *J* = 11.5 Hz, 1H), 3.45 (tt, *J* = 9.6, 4.2 Hz, 4H), 3.26 (s, 3H), 1.69 (s, 3H).

The following examples in Table 16 were prepared using standard chemical manipulations and procedures similar to those used for the preparation of **Example 20.**

**Table 16. Compounds of the Invention**

| # | LCMS (m/z) | 1H NMR |
|---|---|---|
| 84 | 479.25 | 1H NMR (300 MHz, DMSO-d6) δ 9.29 (t, J = 5.9 Hz, 1H), 8.60 (dd, J = 5.0, 0.9 Hz, 1H), 8.15 (d, J = 1.8 Hz, 1H), 7.91 (dd, J = 8.1, 1.8 Hz, 1H), 7.72 - 7.58 (m, 2H), 7.57 - 7.42 (m, 4H), 7.29 (d, J = 8.0 Hz, 1H), 4.96 - 4.77 (m, 2H), 4.62 (d, J = 5.7 Hz, 2H), 4.22 (d, J = 11.5 Hz, 1H), 3.86 (d, J = 11.5 Hz, 1H), 2.94 (d, J = 26.3 Hz, 6H), 1.69 (s, 3H). |

### Example 21. (R)-4-Cyano-4-methyl-N-((2-phenyl-1,6-naphthyridin-7-yl)methyl)chromane-6-carboxamide

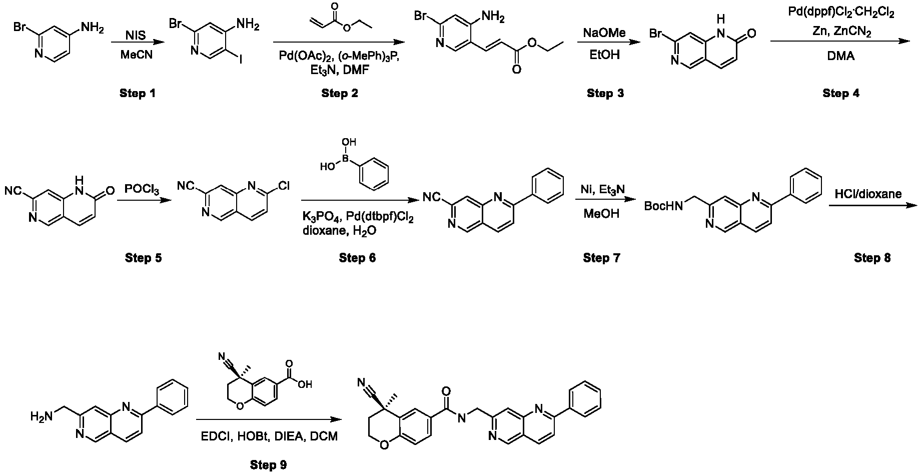

### Step 1. 2-Bromo-5-iodo-pyridin-4-amine

NIS (93.6 g, 416 mmol) was added to a solution of 2-bromopyridin-4-amine (60 g, 347 mmol) in MeCN (1.5 L) at 80°C. The reaction mixture was stirred at 80 °C for 36 hrs. The reaction mixture was concentrated under reduced pressure to give a residue. The residue was diluted with saturated Na₂SO₃ (1.5 L) and extracted with EA (1.5 L x 2). The combined organic layers were washed with brine (1 L), dried over Na₂SO₄, filtered, and concentrated under reduced pressure to give a residue. The residue was purified by column chromatography (SiO₂, PE/EA = 20:3) and concentrated under reduced pressure affording the title compound (65 g, 217 mmol) as a light yellow solid.
¹H NMR (400 MHz, CDCl3) δ = 8.31 (s, 1H), 6.79 (s, 1H), 4.75 (br s, 2H) ppm.

### Step 2. Ethyl-3-(4-amino-6-bromo-3-pyridyl)prop-2-enoate

Ethyl prop-2-enoate (45.1 mL, 415 mmol), Et₃N (43.3 mL, 311 mmol), Pd(OAc)₂ (2.3 g, 10.4 mmol), and tris-o-tolylphosphane (6.3 g, 20.7 mmol) was added to a solution of 2-bromo-5-iodo-pyridin-4-amine (62 g, 207 mmol) in DMF (620 mL). The mixture was stirred at 100°C for 3 hrs. The reaction mixture was diluted with water (4 L) and extracted with EA (2 L x 2). The combined organic layers were washed with brine (2 L), dried over Na₂SO₄, filtered, and concentrated under reduced pressure to give a residue. The residue was purified by column chromatography (SiO₂, PE/EA = 20:3) and concentrated under reduced pressure affording the title compound (50 g, 170 mmol) as a light yellow solid.
LCMS (ESI) m/z: [79BrM+H]+ = 271.1.
¹H NMR (400 MHz, DMSO-d6) δ = 8.23 (s, 1H), 7.73 (d, J = 16.0 Hz, 1H), 6.90 - 6.67 (m, 3H), 6.52 (d, J = 16.0 Hz, 1H), 4.18 (d, J = 7.2 Hz, 2H), 1.25 (d, J = 7.2 Hz, 3H) ppm.

### Step 3. 7-Bromo-1H-1,6-naphthyridin-2-one

NaSMe (24.2 mL, 380 mmol) was added to a solution of ethyl-3-(4-amino-6-bromo-3-pyridyl)prop-2-enoate (40 g, 148 mmol) in EtOH (200 mL). The reaction mixture was stirred at 60 °C for 2 hrs. The reaction mixture was diluted with water (400 mL) and then neutralized with 1N HCl to pH = 7.0. The solid was filtered and the filter cake was washed with water (50 mL). The filter cake was concentrated under reduced pressure affording the title compound (22 g, 96.8 mmol) as an off-white solid.
LCMS (ESI) m/z: [79BrM+H]+ = 224.9.
¹H NMR (400 MHz, DMSO-d6) δ = 12.08 (br s, 1H), 8.65 (s, 1H), 7.99 (d, *J =* 9.6 Hz, 1H), 7.36 (s, 1H), 6.62 (d, *J* = 9.6 Hz, 1H) ppm.

### Step 4. 2-Oxo-1H-1,6-naphthyridine-7-carbonitrile

Zn (406.80 mg, 6.22 mmol) was added to a solution of 7-bromo-1H-1,6-naphthyridin-2-one (7 g, 31.1 mmol), Zn(CN)₂ (3.95 mL, 62.2 mmol), and Pd(dppf)Cl₂ CH₂Cl₂ (5.08 g, 6.22 mmol) in DMA (140 mL). The reaction mixture was degassed and purged with N₂ three times then the mixture was stirred at 120 °C for 2 hrs. The reaction mixture was diluted with water (200 mL) and extracted with DCM/isopropanol (v/v=3:1) (150 mL x 2). The combined organic layers were filtered, washed with brine (200 mL), dried over Na₂SO₄, filtered, and concentrated under reduced pressure to give a residue. The residue was purified by column chromatography (SiO₂, PE/EA=1:1) and concentrated under reduced pressure affording the title compound (3 g, 17.5 mmol) as an off-white solid.
¹H NMR (400 MHz, DMSO-d6) δ = 12.37 (br s, 1H), 8.97 (s, 1H), 8.09 (d, J = 9.6 Hz, 1H), 7.67 (s, 1H), 6.77 (d, J = 9.6 Hz, 1H) ppm.

### Step 5. 2-Chloro-1,6-naphthyridine-7-carbonitrile

A mixture of 2-oxo-1H-1,6-naphthyridine-7-carbonitrile (3.0 g, 17.5 mmol) and POCl₃ (30 mL, 323 mmol) was stirred at 80°C for 2 hrs. The reaction mixture was poured into H₂O (2 L) and adjusted to pH = 7 with NaHCO₃. The solution was extracted with EA (1.5 L x 2), the combined organic layers were washed with brine (2 L), dried over Na₂SO₄, filtered, and concentrated under reduced pressure to afford the title compound (1.1 g, 5.76 mmol) as a brown solid.
LCMS (ESI) m/z: [M+H]⁺ = 190.1.
¹H NMR (400 MHz, DMSO-d6) δ = 9.58 (d, J = 0.8 Hz, 1H), 8.79 (d, J = 0.8 Hz, 1H), 8.69 (s, 1H), 8.00 (d, J = 8.8 Hz, 1H) ppm.

### Step 6. 2-Phenyl-1,6-naphthyridine-7-carbonitrile

A mixture of 2-chloro-1,6-naphthyridine-7-carbonitrile (270 mg, 1.42 mmol), phenylboronic acid (208.4 mg, 1.71 mmol), K₃PO₄ (907 mg, 4.27 mmol), ditert-butyl(cyclopentyl)phosphane; dichloropalladium;iron (92.8 mg, 142 µmol), and H₂O (0.3 mL) in dioxane (3 mL) was degassed and purged with N₂ three times. The mixture was stirred at 80 °C for 2 hrs. The reaction mixture was diluted with H₂O (30 mL) and extracted with EA (30 mL x 3). The combined organic layers were washed with brine (60 mL), dried over Na₂SO₄, filtered, and concentrated under reduced pressure to give a residue. The residue was purified by column chromatography (SiO₂, Petroleum ether/Ethyl acetate=30/1 to 3/1) to afford the title compound (248 mg, 1.07 mmol) as a white solid.
LCMS (ESI) m/z: [M+H]⁺ = 232.1
¹H NMR (400 MHz, DMSO-d6) δ = 9.52 (s, 1H), 8.80 (d, *J* = 8.8 Hz, 1H), 8.69 (s, 1H), 8.53 (d, *J* = 8.8 Hz, 1H), 8.37 - 8.30 (m, 2H), 7.65 - 7.60 (m, 3H) ppm.

### Step 7. tert-Butyl N-[(2-phenyl-1,6-naphthyridin-7-yl)methyl]carbamate

Boc₂O (340 mg, 1.56 mmol) and Et₃N (0.29 mL, 2.08 mmol) were added to a solution of 2-phenyl-1,6-naphthyridine-7-carbonitrile (240 mg, 1.04 mmol) in MeOH (10 mL). Ni (305 mg, 5.19 mmol) was added and the reaction mixture was degassed and purged with H₂ three times. The reaction mixture was stirred at 25 °C for 16 hrs under H₂ atmosphere (15 psi). The suspension was filtered and the filter cake was washed with MeOH (2 mL x 3), filtered, and concentrated under reduced pressure to give a residue. The residue was purified by column chromatography (SiO₂, Petroleum ether/Ethyl acetate=10/1 to 1/1) affording the title compound (110 mg, 0.33 mmol) as a colorless oil.
LCMS (ESI) m/z: [M+H]⁺ = 336.2.

### Step 8. (2-Phenyl-1,6-naphthyridin-7-yl)methanamine

A mixture of tert-butyl N-[(2-phenyl-1,6-naphthyridin-7-yl)methyl]carbamate (110 mg, 328 µmol) in HCl/dioxane (4N, 5 mL) was stirred at 25°C for 1 hr. The reaction mixture was concentrated under vacuum affording the title compound (118 mg, crude, HCl) as an off-white solid.
LCMS (ESI) m/z: [M+H]⁺ = 236.2.

### Step 9. (4R)-4-Cyano-4-methyl-N-[(2-phenyl-1,6-naphthyridin-7-yl)methyl]chromane-6-carboxamide

EDCI (27.5 mg, 0.14 mmol), HOBt (19.4 mg, 0.14 mmol), and DIEA (0.058 mL, 0.33 mmol) were added to a solution of (4R)-4-cyano-4-methyl-chromane-6-carboxylic acid (28.8 mg, 0.13 mmol) in DCM (1 mL). (2-phenyl-1,6-naphthyridin-7-yl)methanamine (30 mg, 0.11 mmol, HCl salt) was added to the reaction mixture and stirred at 25°C for 2 hrs. The reaction mixture was diluted with H₂O (20 mL) and extracted with EA (15 mL x 3). The combined organic layers were washed with brine (30 mL), dried over Na₂SO₄, filtered, and concentrated under reduced pressure to give a residue. The residue was purified by reversed phase flash chromatography. The eluent was concentrated under reduced pressure to remove MeCN and the residue was lyophilized affording the title compound (13.6 mg, 0.030 mmol) as an off-white solid.
LCMS (ESI) m/z: [M+H]⁺ = 435.1.
¹H NMR (400 MHz, CD₃OD) δ = 9.34 - 9.25 (m, 1H), 8.60 (d, *J* = 9.6 Hz, 1H), 8.22 - 8.14 (m, 4H), 7.95 (s, 1H), 7.88 (d, *J* = 2.4, 1H), 7.59 - 7.52 (m, 3H), 6.97 (d, *J* = 8.4 Hz, 1H), 4.91 (s, 3H), 4.41 - 4.36 (m, 2H), 2.52 - 2.44 (m, 1H), 2.27 - 2.18 (m, 1H), 1.85 (s, 3H) ppm.

The following examples in Table 17 were prepared using standard chemical manipulations and procedures similar to those used for the preparation of **Example 21.**

**Table 17. Compounds of the Invention**

| **#** | **LCMS (m/z)** | **1H NMR** |
|---|---|---|
| **1** | 435.1 | 1H NMR (400 MHz, MeOD-d4) δ = 9.31 (s, 1H), 8.59 (d, J = 8.8 Hz, 1H), 8.22 - 8.14 (m, 4H), 7.99 - 7.90 (m, 2H), 7.59 - 7.52 (m, 3H), 7.29 (d, J = 8.0 Hz, 1H), 4.94 - 4.90 (m, 4H), 4.21 (d, J = 11.6 Hz, 1H), 3.92 (d, J = 11.6 Hz, 1H), 1.77 (s, 3H) ppm. |
| **2** | 428.2 | 1H NMR (400 MHz, DMSO-d6) δ = 9.47 - 9.34 (m, 2H), 8.66 (d, J = 8.8 Hz, 1H), 8.34 - 8.25 (m, 4H), 7.98 (d, J = 8.0 Hz, 1H), 7.79 (s, 1H), 7.64 - 7.51 (m, 3H), 7.35 - 7.25 (m, 1H), 4.96 - 4.69 (m, 4H), 4.08 (m, 1H), 3.92 - 3.78 (m, 1H), 1.80 - 1.64 (m, 3H) ppm. |
| **41** | 490.2 | 1H NMR (400 MHz, DMSO-d6) δ = 9.41 - 9.37 (m, 1H), 9.28 (s, 1H), 8.50 (br d, J = 8.4 Hz, 1H), 8.20 - 8.16 (m, 4H), 7.95 (d, J = 8.0 Hz, 1H), 7.73 (s, 1H), 7.31 (d, J = 8.0 Hz, 1H), 6.51 (d, J = 8.8 Hz, 2H), 4.94 - 4.77 (m, 6H), 4.23 (br d, J = 11.2 Hz, 2H), 3.96 - 3.91 (m, 4H), 1.70 (s, 3H) ppm. |
| **42** | 490.3 | 1H NMR (400 MHz, DMSO-d6) δ = 9.39 - 9.38 (m, 2H), 8.62 (d, J = 8.4 Hz, 1H), 8.23 - 8.19 (m, 2H), 7.93 - 7.92 (m, 1H), 7.80 (s, 1H), 7.56 (d, J = 8.0 Hz, 1H), 7.35 - 7.27 (m, 3H), 6.59 - 6.57 (m, 1H), 4.93 - 4.80 (m, 4H), 4.23 (d, J = 11.6 Hz, 1H), 3.90 - 3.85 (m, 6H), 2.33 (br s, 1H), 1.70 (s, 3H) ppm. |
| **5** | 426.1 | 1H NMR (400 MHz, MeOD-d4) δ = 9.31 (s, 1H), 8.59 (d, J = 8.8 Hz, 1H), 8.26 - 8.13 (m, 4H), 7.95 (s, 1H), 7.83 (d, J = 1.6 Hz, 1H), 7.59 - 7.52 (m, 3H), 7.19 (d, J = 8.0 Hz, 1H), 4.92 (s, 2H), 4.84 (d, J = 3.6 Hz, 2H), 3.82 - 3.73 (m, 2H), 1.56 (s, 3H) ppm. |
| **6** | 424.3 | 1H NMR (400 MHz, MeOD-d4) δ = 9.30 (s, 1H), 8.59 (d, J = 0.8 Hz, 1H), 8.21 - 8.14 (m, 3H), 7.95 (s, 1H), 7.82 - 7.77 (m, 2H), 7.58 - 7.51 (m, 3H), 7.33 (d J = 7.6 Hz, 1H), 4.90 (s, 2H), 3.60 - 3.53 (m, 2H), 3.02 - 2.91 (m, 2H), 2.29 - 2.27 (m, 1H), 1.90 - 1.86 (m, 1H), 1.33 (s, 3H) ppm. |
| **7** | 465.1 | 1H NMR (400 MHz, MeOD-d4) δ = 9.30 (s, 1H), 8.59 - 8.57 (m, 1H), 8.17 - 8.14 (m, 2H), 7.96 (s, 1H), 7.94 - 7.91 (m, 1H), 7.77 - 7.72 (m, 2H), 7.48 - 7.44 (m, 1H), 7.28 (d, J = 8.4Hz, 1H), 7.11 - 7.09 (m, 1H), 4.92 - 4.90 (m, 4H), 4.20 (d, J = 11.6 Hz, 1H), 3.93 - 3.89 (m, 4H), 1.76 (s, 3H) ppm. |
| **3** | 449.2 | 1H NMR (400 MHz, DMSO-d6) δ = 9.39 (s, 1H), 9.10 (d, J = 8.0 Hz, 1H), 8.64 (d, J = 8.4 Hz, 1H), 8.30 - 8.27 (m, 3H), 8.18 (d, J = 1.6 Hz, 1H), 7.96 - 7.88 (m, 2H), 7.58 - 7.54 (m, 3H), 7.29 (d, J = 8.0 Hz, 1H), 5.44 - 5.39 (m, 1H), 4.92 - 4.81 (m, 2H), 4.22 (d, J = 11.6 Hz, 1H), 3.86 (d, J = 11.2 Hz, 1H), 1.72 - 1.63 (m, 6H) ppm. |
| **4** | 449.2 | 1H NMR (400 MHz, DMSO-d6) δ = 9.39 (s, 1H), 9.09 (d, J = 7.6 Hz, 1H), 8.64 (d, J = 8.4 Hz, 1H), 8.31 - 8.21 (m, 4H), 7.97 - 7.90 (m, 2H), 7.58 - 7.55 (m, 3H), 7.29 (d, J = 8.4 Hz, 1H), 5.45 - 5.41 (m, 1H), 4.92 - 4.81 (m, 2H), 4.22 (d, J = 11.6 Hz, 1H), 3.85 (d, J = 11.6 Hz, 1H), 1.69 - 1.65 (m, 6H) ppm. |
| **9** | 493.2 | 1H NMR (400 MHz, DMSO-d6) δ = 9.41 - 9.38 (m, 2H), 8.86 - 8.84 (m, 1H), 8.70 (d, J = 8.8 Hz, 1H), 8.55 (d, J = 8.0 Hz, 1H), 8.35 (d, J = 8.8 Hz, 1H), 8.20 (d, J = 1.2 Hz, 1H), 8.13 - 8.11 (m, 1H), 7.96 - 7.94 (m, 1H), 7.86 (s, 1H), 7.74 - 7.71 (m, 1H), 7.31 (d, J = 8.0 Hz, 1H), 4.93 - 4.81 (m, 4H), 4.22 (d, J = 11.6 Hz, 1H), 3.91 - 3.85 (m, 4H), 1.70 (s, 3H) ppm. |
| **10** | 399.2 | 1H NMR (400 MHz, MeOD-d4) δ = 9.17 (s, 1H), 8.34 (d, J = 8.4 Hz, 1H), 8.15 (s, 1H), 7.96 - 7.88 (m, 1H), 7.76 (s, 1H), 7.45 (d, J = 8.8 Hz, 1H), 7.28 (d, J = 8.0 Hz, 1H), 4.91 (d, J = 3.6 Hz, 2H), 4.87 - 4.86 (m, 2H), 4.20 (d, J = 11.6 Hz, 1H), 3.92 (d, J = 11.6 Hz, 1H), 2.35 - 2.26 (m, 1H), 1.76 (s, 3H), 1.21 - 1.17 (m, 4H) ppm. |
| **11** | 508.2 | 1H NMR (400 MHz, MeOD-d4) δ = 9.31 (s, 1H), 8.58 (d, J = 8.8 Hz, 1H), 8.18 (d, J = 1.6 Hz, 1H), 8.10 (d, J = 8.8 Hz, 1H), 7.97 - 7.90 (m, 2H), 7.29 (d, J = 8.4 Hz, 1H), 7.23 - 7.17 (m, 1H), 7.06 - 7.02 (m, 1H), 6.35 - 6.28 (m, 1H), 4.93 - 4.90 (m, 4H), 4.21 (d, J = 11.4 Hz, 1H), 4.01 - 3.89 (m, 5H), 2.46 - 2.36 (m, 2H), 1.77 (s, 3H) ppm. |
| **12** | 434.2 | 1H NMR (400MHz, DMSO-d6) δ = 9.43 (s, 1H), 9.12-9.10 (m, 1H), 8.71 (d, J = 8.8 Hz, 1H), 8.41 - 8.29 (m, 3H), 8.06 - 7.93 (m, 2H), 7.82 (s, 1H), 7.62-7.60 (m, 3H), 6.80 (d, J = 8.4 Hz, 1H), 4.83 (d, J = 5.6 Hz, 2H), 3.92 (d, J = 9.6 Hz, 1H), 3.48 (d, J = 9.6 Hz, 1H), 2.90 (s, 3H), 1.77 (s, 3H) ppm. |
| **14** | 485.1 | 1H NMR (400 MHz, DMSO-d6) δ = 9.42 (s, 2H), 8.71 (d, J = 8.4 Hz, 1H), 8.52 (s, 1H), 8.47 (d, J = 7.6 Hz, 1H), 8.21 (d, J = 1.2 Hz, 1H), 7.98 - 7.95 (m, 1H), 7.85 (s, 1H), 7.80 - 7.67 (m, 2H), 7.35 - 7.01 (m, 2H), 5.03 - 4.73 (m, 4H), 4.24 (d, J = 11.6 Hz, 1H), 3.88 (d, J = 11.6 Hz, 1H), 1.71 (s, 3H) ppm. |
| **15** | 421 | 1H NMR (400 MHz, DMSO-d6) δ = 9.37 (s, 1H), 9.24 (t, J = 6.0 Hz, 1H), 8.66 (d, J = 8.8 Hz, 1H), 8.30 - 8.27 (m, 3H), 8.17 (d, J = 1.6 Hz, 1H), 8.00 - 7.97 (m, 1H), 7.79 (s, 1H), 7.59 - 7.54 (m, 3H), 7.08 (d, J = 8.4 Hz, 1H), 5.02 (d, J = 9.6 Hz, 1H), 4.79 (d, J = 6.0 Hz, 2H), 4.56 (d, J = 9.6 Hz, 1H), 1.78 (s, 3H) ppm. |
| **16** | 446.2 | 1H NMR (400 MHz, DMSO-d6) δ = 9.40 - 9.33 (m, 2H), 8.66 (d, J = 8.8 Hz, 1H), 8.33 - 8.27 (m, 3H), 7.99 (d, J = 8.4 Hz, 1H), 7.82 (d, J = 7.6 Hz, 2H), 7.60 - 7.54 (m, 4H), 7.44 (d, J = 6.8 Hz, 1H), 6.76 - 6.37 (m, 1H), 4.96 (s, 4H), 4.81 (d, J = 6.0 Hz, 2H) ppm |
| **17** | 483.2 | 1H NMR (400 MHz, DMSO-d6) δ = 9.31 (s, 1H), 8.59 (d, J = 8.8 Hz, 1H), 8.20 - 8.07 (m, 2H), 7.99 - 7.85 (m, 2H), 7.61 (d, J = 1.6 Hz, 1H), 7.55 - 7.51 (m, 1H), 7.28 (d, J = 8.0 Hz, 1H), 6.89 - 6.85 (m, 1H), 4.96 - 4.89 (m, 4H), 4.20 (d, J = 11.6 Hz, 1H), 3.94 (s, 1H), 3.91 - 3.90 (m, 3H), 1.76 (s, 3H) ppm |
| **18** | 436.2 | 1H NMR (400 MHz, MeOD-d4) δ = 9.34 (s, 1H), 8.76 - 8.71 (m, 1H), 8.68 - 8.60 (m, 3H), 8.18 (d, J = 1.6 Hz, 1H), 8.03 - 7.96 (m, 2H), 7.95 - 7.92 (m, 1H), 7.55 - 7.50 (m, 1H), 7.29 (d, J = 8.0 Hz, 1H), 4.95 - 4.90 (m, 4H), 4.21 (d, J = 11.6 Hz, 1H), 3.93 (d, J = 11.6 Hz, 1H), 1.77 (s, 3H) ppm |
| **20** | 439.1 | 1H NMR (400 MHz, DMSO-d6) δ = 9.40-9.37 (m, 1H), 9.25 (s, 1H), 8.55 (s, 1H), 8.50 (d, J=8.4 Hz, 1H), 8.21 (s, 2H), 7.98 -7.93 (m, 2H), 7.65 (s, 1H), 7.32 (d, J=8.4 Hz, 1H), 4.94 - 4.84 (m, 2H), 4.78-4.77 (m, 2H), 4.25-4.22 (m, 1H), 3.92(s, 3H), 3.89-3.86 (m, 1H), 1.71 (s, 3H) ppm |
| **21** | 489.2 | 1H NMR (400 MHz, MeOD) δ = 9.29 (s, 1H), 8.64 (s, 1H), 8.57 (d, J = 8.8 Hz, 1H), 8.39 - 8.33 (m, 1H), 8.25 (d, J = 8.8 Hz, 1H), 8.20 - 8.14 (m, 2H), 7.98 - 7.92 (m, 2H), 7.71 (d, J = 8.8 Hz, 1H), 7.30 (d, J = 8.2 Hz, 1H), 4.93 - 4.91 (m, 4H), 4.21 (d, J = 11.4 Hz, 1H), 4.12 (s, 3H), 3.93 (d, J = 11.6 Hz, 1H), 1.77 (s, 3H) ppm |
| **22** | 435.2 | 1H NMR (400 MHz, DMSO-d6) δ= 9.38 - 9.35 (m, 1H), 9.01 (d, J = 2.0 Hz, 1H), 8.50 (d, J = 8.8 Hz, 1H), 8.38 (d, J = 8.8 Hz, 1H), 8.33 - 8.27 (m, 3H), 8.14 (d, J = 1.6 Hz, 1H), 7.92 - 7.90 (m, 1H), 7.61 - 7.50 (m, 3H), 7.29 (d, J = 8.0 Hz, 1H), 4.92 - 4.78(m, 4H), 4.21 (d, J = 11.6 Hz, 1H), 3.87 - 3.84 (m, 1H), 1.68 (s, 3H) ppm |
| **23** | 443.3 | 1H NMR (400 MHz, MeOD) δ= 9.37 (s, 1H), 8.57 (d, J = 8.6 Hz, 1H), 8.18 (d, J = 1.6 Hz, 1H), 7.98 (s, 1H),7.94-7.92 (m, 1H), 7.73 (d, J = 8.6 Hz, 1H), 7.31 (d, J = 8.2 Hz, 1H), 4.96 - 4.91 (m, 4H), 4.22 (d, J = 11.4 Hz, 1H), 4.11-4.09(m, 2H), 3.95 (d, J = 11.4 Hz, 1H), 3.64-3.63 (m, 2H), 3.31 - 3.23 (m, 1H), 2.11 - 1.87 (m, 4H), 1.78 (s, 3H)ppm |
| **24** | 465.1 | 1H NMR (400 MHz, DMSO-d6) δ = 9.40-9.35 (m, 2H), 8.59 (d, J = 8.4 Hz, 1H), 8.30-8.19 (m, 4H), 7.96-7.93 (m, 1H),7.7 (s, 1H), 7.31 (d, J=8.4, 1H), 7.11-7.09 (m, 2H), 4.93-4.79 (m, 4H), 4.23 (m, J=11.6, 1H), 3.86(d, J=11.2, 4H), 1.70 (s, 3H) ppm |
| **25** | 440.1 | 1H NMR (400 MHz, DMSO-d6) δ = 9.38 (s, 1H), 9.27 -9.26(m, 1H), 8.65 (d, J = 8.4 Hz, 1H), 8.34 - 8.25 (m, 3H), 7.98(s, 1H), 7.81 - 7.76 (m, 2H), 7.56 - 7.54(m, 3H), 7.16 (d, J = 8.0 Hz, 1H), 4.88 - 4.86 (m, 1H), 4.80 - 4.74(m, 4H), 3.96 - 3.93 (m, 1H), 3.59 - 3.57 (m, 1H), 3.42 (s, 2H), 1.19 (s, 3H) ppm |
| **26** | 489.3 | 1H NMR (400 MHz, DMSO-d6) δ= 9.43 - 9.40 (m, 2H), 8.70 (d, J = 8.4 Hz, 1H), 8.58 (s, 1H), 8.45 (d, J = 8.8 Hz, 1H), 8.21 (d,J = 1.2 Hz, 1H), 8.13 - 8.11 (m, 2H), 7.97 - 7.95 (m, 1H), 7.90 (d, J = 8.4 Hz, 1H), 7.84 (s, 1H), 7.32 (d, J = 8.0 Hz, 1H), 4.93 -4.81 (m, 4H), 4.23 (d, J = 11.6 Hz, 1H), 4.16 (s, 3H), 3.87 (d, J = 11.6 Hz, 1H), 1.70 (s, 3H) ppm |
| **27** | 465.4 | 1H NMR (400 MHz, MeOD) δ = 9.30 (s, 1H), 8.50 (br d, J = 8.8 Hz, 1H), 8.16 (d, J = 1.6 Hz, 1H), 8.05 (d, J = 8.4 Hz, 1H), 7.93-7.90 (m, 2H), 7.71-7.69 (m, 1H), 7.50-7.48 (m, 1H), 7.28(br d, J = 8.0 Hz, 1H), 7.19 (d, J = 8.4 Hz, 1H), 7.11-7.09 (m, 1H), 4.91 (s, 2H), 4.90 (br d, J = 3.6 Hz, 2H), 4.20 (d, J = 11.6 Hz, 1H), 3.92 (d, J = 11.6 Hz, 1H), 3.88 (s, 3H), 1.75 (s, 3H)ppm |
| **29** | 439.2 | 1H NMR (400 MHz, DMSO-d6) δ = 9.39 - 9.36 (m, 1H), 9.31 (s, 1H), 8.54 (d, J = 8.8 Hz, 1H), 8.23 - 8.17 (m, 2H), 7.96 - 7.93(m, 1H), 7.85 (d, J = 2.4 Hz, 1H), 7.75 - 7.71 (m, 1H), 7.31 (d, J = 8.4 Hz, 1H), 7.01 (d, J = 2.4 Hz, 1H), 4.94 - 4.83 (m, 2H), 4.82 - 4.77 (m, 2H), 4.23 (d, J = 11.6 Hz, 1H), 3.97 (s, 3H), 3.87 (d, J = 11.6 Hz, 1H), 1.70 (s, 3H) ppm |
| **30** | 436.2 | 1H NMR (400 MHz, MeOD-d4) δ = 9.41 - 9.34 (m, 2H), 8.71 - 8.63 (m, 3H), 8.24 (d, J = 8.8 Hz, 1H), 8.17 (d, J = 1.6 Hz, 1H), 7.99 (s, 1H), 7.95 - 7.91 (m, 1H), 7.67 - 7.59 (m, 1H), 7.29 (d, J = 8.0 Hz, 1H), 4.93 (s, 2H), 4.91 (d, J = 3.6 Hz, 2H), 4.20 (d, J = 11.6 Hz, 1H), 3.93 (d, J = 11.6 Hz, 1H), 1.77 (s, 3H) ppm |
| **32** | 434.2 | 1H NMR (400 MHz, DMSO-d6) δ = 9.31 (s, 1H), 9.00 - 8.98 (m, 1H), 8.62 (d, J = 8.8 Hz, 1H), 8.21 - 8.19 (m, 3H), 7.88 (br s, 1H), 7.71 (s, 1H), 7.65 (br d, J = 8.4 Hz, 1H), 7.54 - 7.53 (m, 3H), 6.60 (d, J = 8.4 Hz, 1H), 4.72 (br d, J = 5.2 Hz, 2H), 3.33 - 3.30 (m, 2H), 2.19 - 2.14 (m, 1H), 1.97 - 1.93 (m, 1H), 1.68 (s, 3H) ppm |
| **37** | 500.3 | 1H NMR (400 MHz, DMSO-d6) δ = 9.38-9.35 (m, 1H), 9.32 (s, 1H), 8.51 (d, J = 8.4 Hz, 1H), 8.19 (d, J = 1.6 Hz, 1H), 7.95-7.92 (m, 1H), 7.69 (s, 1H), 7.63 (d, J = 8.4 Hz, 1H), 7.30 (d, J = 8.0 Hz, 1H), 4.92 - 4.81 (m, 2H), 4.78 (d, J = 5.6 Hz, 2H), 4.23 - 4.14 (m, 2H), 4.03 - 3.96 (m, 1H), 3.86 (d, J = 11.6 Hz, 1H), 3.58 (s, 3H), 3.21 - 2.99 (m, 2H), 2.91 - 2.81 (m, 1H), 2.07 - 2.02 (m, 1H), 1.84 - 1.72 (m, 2H), 1.69 (s, 3H), 1.57 - 1.46 (m, 1H) ppm |
| **38** | 436.1 | 1H NMR (400 MHz, DMSO-d6) δ= 9.65 - 9.49 (m, 1H), 9.39 (s, 1H), 9.08 (d, J = 2.0 Hz, 1H), 8.69 - 8.62 (m, 1H), 8.57 (d, J = 2.0 Hz, 1H), 8.36 - 8.24 (m, 3H), 7.87 (s, 1H), 7.61 - 7.52 (m, 3H), 4.94 - 4.80 (m, 4H), 4.29 (d, J = 11.6 Hz, 1H), 3.93 (d, J = 11.6 Hz, 1H), 1.72 (s, 3H) ppm |
| **28** | 460.3 | 1H NMR (400 MHz, CDCl₃): δ = 9.33 (s, 1H), 8.53 (s, 1H), 8.47 (d, J = 8.1 Hz, 2H), 8.04 (d, J = 6.8 Hz, 2H), 7.84 (t, J = 7.8 Hz, 2H), 7.69 (t, J = 7.9 Hz, 1H), 7.15 (d, J = 7.8 Hz, 1H), 5.04 (s, 1H), 4.88 (s, 2H), 4.14 (d, J = 11.3 Hz, 1H), 3.94 (d, J = 11.4 Hz, 1H), 1.26 (s, 3H) ppm |

### Example 22. (4R)-4-cyano-4-methyl-N-[[2-(1-piperidyl)-1,6-naphthyridin-7-yl]methyl]isochromane-6-carboxamide

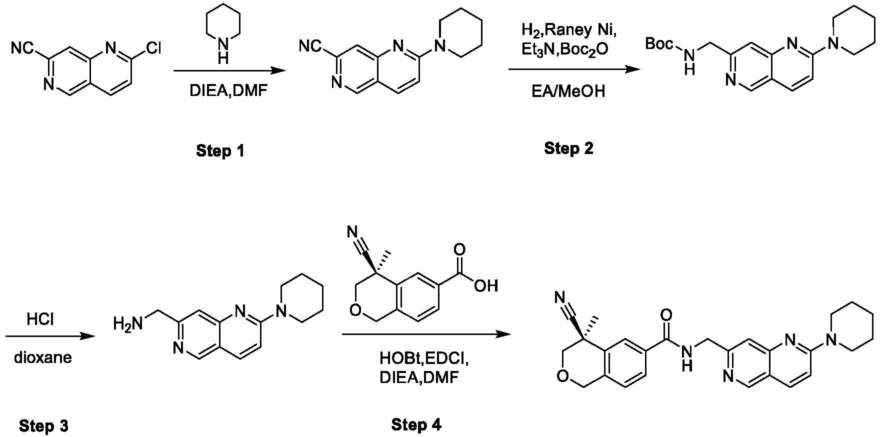

### Step 1. 2-(1-Piperidyl)-1,6-naphthyridine-7-carbonitrile

DIEA (0.37 mL, 2.11 mmol) and piperidine (0.31 mL, 3.16 mmol) were added to a mixture of 2-chloro-1,6-naphthyridine-7-carbonitrile (200 mg, 1.05 mmol) in DMSO (2 mL) at 20 °C. The mixture was heated and stirred at 100°C for 2 hr. The mixture was poured into H₂O (20 mL) and filtered. The filter cake was washed with MTBE (20 mL). The filter cake was dried under reduced pressure affording the title compound (170 mg, 0.62 mmol) as a yellow solid.
LCMS (ESI) m/z: [M+H]⁺ = 239.7.

### Step 2. tert-Butyl N-[[2-(1-piperidyl)-1,6-naphthyridin-7-yl]methyl]carbamate

A mixture of 2-(1-piperidyl)-1,6-naphthyridine-7-carbonitrile (170 mg, 0.71 mmol), Et₃N (0.20 mL, 1.43 mmol), Boc₂O (234 mg, 1.07 mmol), and Raney-Ni (61.1 mg) in MeOH (20 mL) and EA (20 mL) was stirred at 20 °C for 16 hr under a H₂ atmosphere (15 psi). The reaction mixture was vented to nitrogen and then filtered. The filtrate was concentrated under reduced pressure. The crude residue was purified by silica gel chromatography (Petroleum Ether/EA = 5/1) affording the title compound (165 mg, 0.48 mmol) as a white solid.
LCMS (ESI) m/z: [M+H]⁺ = 343.4.
¹H NMR (400 MHz, CD₃OD) δ = 8.72 (s, 1H), 8.02 (d, J = 9.6 Hz, 1H), 7.36 (s, 1H), 7.21 (d, J = 9.6 Hz, 1H), 4.40 (s, 2H), 3.87 - 3.79 (m, 4H), 1.81 - 1.73 (m, 2H), 1.72 - 1.63 (m, 4H), 1.49 (s, 9H) ppm.

### Step 3. [2-(1-Piperidyl)-1,6-naphthyridin-7-yl]methanamine

A mixture of tert-butyl N-[[2-(1-piperidyl)-1,6-naphthyridin-7-yl]methyl]carbamate (165 mg, 0.48 mmol) in HCl/dioxane (4N, 2 mL) was stirred at 20°C for 2 hrs. The mixture was concentrated under reduced pressure affording the title compound (135 mg, 0.56 mmol, HCl salt) as a white solid. LCMS (ESI) m/z: [M+H]⁺ = 243.0.

### Step 4. (4R)-4-Cyano-4-methyl-N-[[2-(1-piperidyl)-1,6-naphthyridin-7-yl]methyl]isochromane-6-carboxamide

DIEA (0.047 mL, 0.27 mmol) was added to a mixture of [2-(1-Piperidyl)-1,6-naphthyridin-7-yl]methanamine (25 mg, 0.090 mmol, HCl salt), (4R)-4-cyano-4-methylisochromane-6-carboxylic acid (21.4 mg, 0.099 mmol), EDCI (25.8 mg, 0.3 mmol), and HOBt (18.2 mg, 0.13 mmol) in DMF (0.5 mL). The mixture was stirred at 20°C for 16 hrs. The mixture was poured into H₂O (10 mL) and filtered. The filter cake was purified by prep-HPLC (column: Waters Xbridge 150*25 mm* 5 µm; mobile phase: [water(10 mM NH₄-HCO₃)-ACN];B%: 28%-58%,9 min) affording the title compound (20.2 mg, 0.046 mmol) as a white solid.
LCMS (ESI) m/z: [M+H]⁺ = 442.4.
¹H NMR (400 MHz, CD₃OD) δ = 8.77 (s, 1H), 8.15 (d, J = 1.6 Hz, 1H), 8.04 (d, J = 9.2 Hz, 1H), 7.92-7.88 (m, 1H), 7.41 (s, 1H), 7.27 (d, J = 8.0 Hz, 1H), 7.22 (d, J = 9.6 Hz, 1H), 4.90 (d, J = 4.0 Hz, 2H), 4.76 (s, 2H), 4.20 (d, J = 11.2 Hz, 1H), 3.91 (d, J = 11.2 Hz, 1H), 3.86 - 3.77 (m, 4H), 1.79 - 1.70 (m, 5H), 1.69 - 1.60 (m, 4H) ppm.

### Example 23. (R)-4-cyano-4-methyl-N-((2-phenyl-1,6-naphthyridin-7-yl)methyl)chromane-6-carboxamide

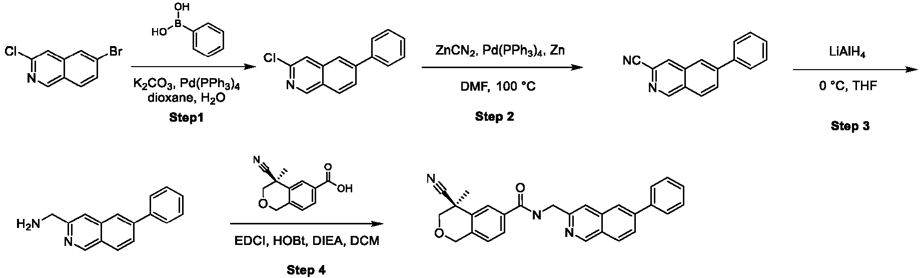

### Step 1. 3-chloro-6-phenylisoquinoline

6-bromo-3-chloroisoquinoline (0.500 g, 2.06 mmol), Pd(PPh₃)₄ (0.476 g, 0.412 mmol), phenylboronic acid (0.500 g, 2.06 mmol), and zinc (6.3 mg, 0.096 mmol) were added to a 40 mL vial and it was purged with nitrogen. 10 mL of a 1:1 mixture of DME/water was added to the vial and the reaction mixture was heated to 80°C and stirred under nitrogen atmosphere for 3h at 80 °C. After cooling down to rt, the reaction mixture was extracted with DCM (3 x 20 mL). The combined organic layers were dried over sodium sulfate, filtered, and concentrated under reduced pressure to give a residue. The residue was purified by column chromatography (SiO₂, 0-60% EtOAc in heptane) and concentrated under reduced pressure affording the title compound (310 mg, 1.29 mmol) as a light yellow solid.
LCMS (ESI) m/z: [M+H]⁺ = 240.1.
¹H NMR (400 MHz, CDCl3) δ = 9.09 (d, J = 1.1 Hz, 1H), 8.05 (d, J = 8.5 Hz, 1H), 7.93 (s, 1H), 7.86 (dd, J = 8.5, 1.7 Hz, 1H), 7.77 (s, 1H), 7.74 - 7.67 (m, 2H), 7.57 - 7.49 (m, 2H), 7.49 - 7.41 (m, 1H) ppm.

### Step 2. 6-phenylisoquinoline-3-carbonitrile

3-chloro-6-phenylisoquinoline (0.23 g, 0.9595 mmol), potassium carbonate (0.263 g, 1.91 mmol), Pd(PPh₃)₄ (0.221 g, 0.1919 mmol), and Zn(CN)₂ (0.167 g, 1.43 mmol) were added to a 20 mL vial and it was purged with nitrogen. DMF (4.8 mL) was added and the mixture was heated to 100°C and stirred at for 3 hrs. After cooling down to rt, the reaction was quenched by the addition of water and the crude mixture was extracted with DCM (3 x 20 mL). The combined organic layers were dried over sodium sulfate, filtered, and concentrated under reduced pressure to give a residue. The residue was purified by column chromatography (SiO₂, 0-70% EtOAc in heptane) and concentrated under reduced pressure affording the title compound (140 mg, 0.608 mmol, 64% yield) as a light yellow solid.
LCMS (ESI) m/z: [M+H]⁺ = 231.2.
¹H NMR (500 MHz, CDCl3) δ = 9.30 (t, J = 0.9 Hz, 1H), 8.20 (d, J = 1.0 Hz, 1H), 8.17 - 8.11 (m, 1H), 8.10 - 803 (m, 2H), 7.75 - 7.69 (m, 2H), 7.58 - 7.51 (m, 2H), 7.51 - 7.45 (m, 1H) ppm.

### Step 3. (6-phenylisoquinolin-3-yl)methanamine

A solution LiAlH₄ in THF (1.0 M, 0.22 mL, 0.22 mmol) was added dropwise to a solution of 6-phenylisoquinoline-3-carbonitrile (0.050 g, 0.217 mmol) in THF (1 mL) at 0 °C. The reaction mixture was allowed to stir for 2 h at which point it had warmed to rt. The reaction was quenched by addition of 1 mL of water followed by 0.5 mL of a 6N NaOH aqueous solution. The reaction mixture was extracted with 10% MeOH in DCM solution (3 x 5 mL). The combined organic layers were dried over sodium sulfate, filtered, and concentrated under reduced pressure to give the crude amine (51.2 mg) as a brown solid. LCMS (ESI) m/z: [79BrM+H]+ = 224.9.
¹H NMR (500 MHz, CDCl3) δ = 9.25 (s, 1H), 8.09 - 7.91 (m, 1H), 7.88 - 7.79 (m, 1H), 7.72 (d, J = 7.8 Hz, 2H), 7.56 - 7.39 (m, 4H), 3.75 (s, 2H) ppm.

### Step 4. (R)-4-cyano-4-methyl-N-((6-phenylisoquinolin-3-yl)methyl)isochromane-6-carboxamide

EDCI (49.0 mg, 0.256 mmol), HOBt (34.5 mg, 0.256 mmol), and DIEA (66.1 mg, 0.512 mmol, 89 µL) were added to a solution of (4R)-4-cyano-4-methyl-chromane-6-carboxylic acid (46.3 mg, 0.2133 mmol) in DCM (1 mL). 1-(6-phenylisoquinolin-3-yl)methanamine (50.0 mg, 0.2133 mmol) was added to the reaction mixture and stirred at 25 °C for 2 hrs The reaction mixture was diluted with H₂O (5 mL) and extracted with EA (5 mL x 3). The combined organic layers were dried over Na₂SO₄, filtered, and concentrated under reduced pressure to give a residue. The residue was purified by reversed phase flash (0.1% FA condition). The eluent was concentrated under reduced pressure to remove MeCN and the residue was lyophilized affording the title compound (39.4 mg, 0.0909 mmol) as an off-white solid. LCMS (ESI) m/z: [M+H]⁺ = 434.3.
¹H NMR (400 MHz, DMSO-d6) δ = 8.56 (d, J = 4.0 Hz, 2H), 7.51 (d, J = 1.6 Hz, 1H), 7.47 - 7.41 (m, 2H), 7.21 (ddd, J = 13.1, 8.3, 1.8 Hz, 2H), 7.10 (dd, J = 8.3, 1.3 Hz, 2H), 7.04 (s, 1H), 6.77 (dd, J = 8.3, 6.8 Hz, 2H), 6.71 - 6.65 (m, 1H), 6.54 (d, J = 8.1 Hz, 1H), 4.01 (d, J = 5.8 Hz, 2H), 3.46 (d, J = 11.4 Hz, 1H), 3.11 (d, J = 11.5 Hz, 1H), 2.75 (s, 1H), 0.94 (s, 3H) ppm.

### Example 24. (R)-4-cyano-4-methyl-N-((6-phenyl-5,6,7,8-tetrahydro-2,6-naphthyridin-3-yl)methyl)isochromane-6-carboxamide

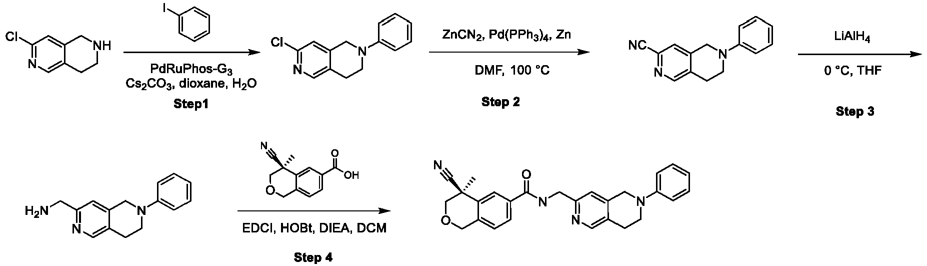

### Step 1. 7-chloro-2-phenyl-1,2,3,4-tetrahydro-2,6-naphthyridine

7-chloro-1,2,3,4-tetrahydro-2,6-naphthyridine hydrochloride (0.300 g, 1.46 mmol), iodobenzene (0.455 g, 2.19 mmol), Pd-RuPhos G3 (0.183 g, 0.219 mmol), and cesium carbonate (0.951 g, 2.92 mmol) were added to a 20 mL vial. The vial was purged with nitrogen before a 5:1 mixture of dioxane/water (7.3 mL) was added and the reaction mixture heated to 80 °C and stirred at this temperature for 3 h. After cooling to rt, 5 mL of water was added. The reaction mixture was extracted with 10% MeOH in DCM solution (3 x 5 mL). The combined organic layers were dried over sodium sulfate, filtered, and concentrated under reduced pressure to give a residue. The residue was purified by column chromatography (SiO₂, 0-100% EtOAc in heptane) and concentrated under reduced pressure affording the title compound (80.0 mg, 0.327 mmol) as a white solid.
LCMS (ESI) m/z: [M+H]⁺ = 245.1.
¹H NMR (400 MHz, CDCl3) δ = 8.20 (s, 1H), 7.35 - 7.28 (m, 1H), 7.28 (d, J = 0.8 Hz, 1H), 7.26 (s, 2H), 7.14 (s, 1H), 7.00 - 6.93 (m, 2H), 6.89 (td, J = 7.3, 1.1 Hz, 1H), 4.36 (s, 2H), 3.58 (t, J = 5.8 Hz, 2H), 2.95 (t, J = 5.8 Hz, 2H) ppm.

### Step 2. 6-phenyl-5,6,7,8-tetrahydro-2,6-naphthyridine-3-carbonitrile

7-chloro-2-phenyl-1,2,3,4-tetrahydro-2,6-naphthyridine (0.026 g, 0.106 mmol), Pd(PPh₃)₄ (0.0245 g, 0.0212 mmol), zinc (1.0 mg, 0.0159 mmol), and Zn(CN)₂ (18.7 mg, 0.159 mmol) were added to a 1 mL vial and it was purged with nitrogen. DMF (4.8 mL) was added and the mixture was heated to 100 °C and stirred at for 3 hrs. After cooling down to rt, the reaction was quenched by the addition of water and the crude mixture was extracted with DCM (3 x 5 mL). The combined organic layers were dried over sodium sulfate, filtered, and concentrated under reduced pressure to give a residue. The residue was purified by column chromatography (SiO₂, 0-70% EtOAc in heptane) and concentrated under reduced pressure affording the title compound (15.1 mg, 0.0642 mmol) as a light yellow solid.
LCMS (ESI) m/z: [M+H]⁺ = 236.1.
¹H NMR (400 MHz, CDCl₃) δ = 8.54 (s, 1H), 7.50 (s, 1H), 7.37 (dd, J = 8.7, 7.3 Hz, 2H), 7.13 (d, J = 8.1 Hz, 2H), 7.03 (t, J = 7.4 Hz, 1H), 4.45 (s, 2H), 3.65 (t, J = 5.8 Hz, 2H), 3.15 (t, J = 5.8 Hz, 2H) ppm.

### Step 3. (6-phenyl-5,6,7,8-tetrahydro-2,6-naphthyridin-3-yl)methanamine

To 6-phenyl-5,6,7,8-tetrahydro-2,6-naphthyridine-3-carbonitrile (0.015 g, 0.0642 mmol) in THF (0.5 mL) at 0 °C was added a solution LiAlH₄ in THF (1.0 M, 0.22 mL, 0.22 mmol) dropwise. The reaction mixture was allowed to stir for 2 h at which point it had warmed to rt. The reaction was quenched by addition of 1 mL of water followed by 0.5 mL of a 6N NaOH aqueous solution. The reaction mixture was extracted with 10% MeOH in DCM solution (3 x 5 mL). The combined organic layers were dried over sodium sulfate, filtered, and concentrated under reduced pressure to give the crude amine (15.0 mg) as a brown solid.
LCMS (ESI) m/z: [M+H]⁺ = 240.1.
¹H NMR (400 MHz, CDCl₃) δ = 8.55 - 8.28 (m, 1H), 7.26 (s, 2H), 7.07 - 6.76 (m, 4H) ppm, 4.40 (dd, J = 23.8, 6.7 Hz, 2H), 4.03 - 3.34 (m, 4H), 3.08 - 2.88 (m, 2H).

### Step 4. (R)-4-cyano-4-methyl-N-((6-phenyl-5,6,7,8-tetrahydro-2,6-naphthyridin-3-yl)methyl)isochromane-6-carboxamide

DIEA (19.4 mg, 0.150 mmol, 26 µL) was added to a vial containing 1-(6-phenyl-5,6,7,8-tetrahydro-2,6-naphthyridin-3-yl)methanamine (15.0 mg, 0.0627 mmol), EDCI (14.5 mg, 0.0752 mmol), HOBt (10.4 mg, 0.0752 mmol), and (4R)-4-cyano-4-methyl-chromane-6-carboxylic acid (13.6 mg, 0.0627 mmol) in DCM (0.5 mL). The reaction mixture stirred at 25 °C for 2 hrs. The reaction mixture was concentrated under reduced pressure to give a residue. The residue was purified by reversed phase flash chromatography affording the title compound (6.2 mg, 0.0141 mmol) as an off-white solid.
LCMS (ESI) m/z: [M+H]⁺ = 439.2.
¹H NMR (400 MHz, CDCl₃) δ = 8.38 (s, 1H), 8.28 (s, 1H), 8.09 - 8.00 (m, 2H), 7.81 (dd, J = 8.0, 1.7 Hz, 1H), 7.35 - 7.27 (m, 3H), 7.13 (dd, J = 15.5, 8.1 Hz, 2H), 7.02 - 6.95 (m, 2H), 6.89 (t, J = 7.3 Hz, 1H), 4.87 (d, J = 15.6 Hz, 4H), 4.75 (dd, J = 5.5, 3.1 Hz, 2H), 4.42 (s, 2H), 4.14 (dd, J = 11.3, 6.4 Hz, 2H), 3.93 (dd, J = 14.3, 11.3 Hz, 2H), 3.59 (t, J = 5.7 Hz, 2H), 3.50 (s, 2H), 3.00 (t, J = 5.7 Hz, 2H), 1.78 (s, 3H) ppm.

### Example 25. (4R)-4-cyano-4-methyl-N-[(2-phenylthiazolo[5,4-c]pyridin-6-yl)methyl]isochromane-6-carboxamide

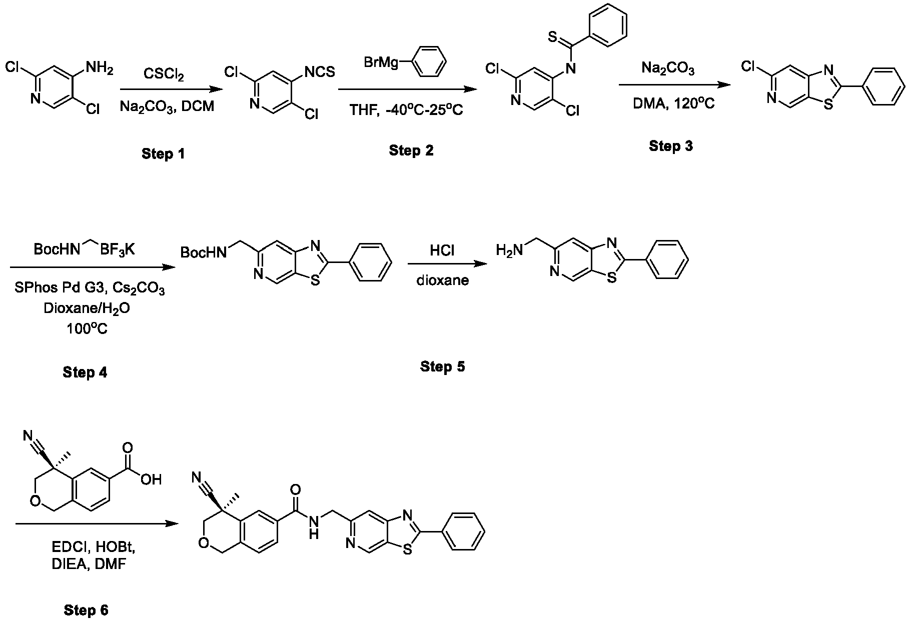

### Step 1. 2,5-Dichloro-4-isothiocyanato-pyridine

Thiocarbonyl dichloride (0.94 mL, 12.3 mmol) was added to a solution of 2,5-dichloropyridin-4-amine (1 g, 6.13 mmol) and Na₂CO₃ (2.60 g, 24.5 mmol) in DCM (15 mL) at 25 °C. The mixture was stirred for 15 hrs. The mixture was filtered and concentrated in vacuo. The crude residue was purified by column chromatography (SiO₂, Petroleum Ether/EA = 10/1 to 5/1) affording the title compound (750 mg, 3.66 mmol) as a brown solid.
¹H NMR (400 MHz, DMSO-d6) δ = 8.63 (s, 1H), 7.85 - 7.76 (m, 1H) ppm.

### Step 2. N-(2,5-Dichloro-4-pyridyl)benzenecarbothioamide

Phenylmagnesium bromide (3 M, 1.6 mL) was added slowly to a mixture of 2,5-Dichloro-4-isothiocyanato-pyridine (650 mg, 3.17 mmol) in THF (8 mL) at -40 °C. After the addition, the mixture was stirred at -40°C for 30 min. The reaction mixture was then warmed to 25°C and stirred for 2 hrs. The mixture was quenched via the addition of water (5 mL) and extracted with EA (5 mL). The organic layer was washed with brine (10 mL), dried over Na₂SO₄, filtered, and concentrated in vacuo. The crude residue was purified by column chromatography (SiO₂, Petroleum Ether/EA = 10/1 to 5/1) affording the title compound (650 mg, 2.30 mmol) as a white solid.
¹H NMR (400 MHz, CD₃OD) δ = 8.55 - 8.51 (m, 1H), 8.08 - 8.03 (m, 1H), 7.97 - 7.90 (m, 2H), 7.60 - 7.53 (m, 1H), 7.50 - 7.43 (m, 2H) ppm.

### Step 3. 6-Chloro-2-phenyl-thiazolo[5,4-c]pyridine

Na₂CO₃ (412 mg, 3.88 mmol) was added to a mixture of N-(2,5-Dichloro-4-pyridyl)benzenecarbothioamide (550 mg, 1.94 mmol) in DMA (8 mL) at 25 °C. The mixture was warmed and stirred at 120 °C for 3 hrs. The mixture was quenched with water (20 mL) and extracted with EA (15 mL x 3). The combined organic layer was washed with brine (20 mL), dried over Na₂SO₄, filtered, and concentrated in vacuo. The crude residue was purified by column chromatography (SiO₂, Petroleum Ether/EA = 10/1 to 5/1) affording the title compound (250 mg, 1.01 mmol) as a yellow solid.
LCMS (ESI) m/z: [M+H]⁺ = 247.0.
¹H NMR (400 MHz, DMSO-d6) δ = 9.25 (d, J = 0.4 Hz, 1H), 8.21 - 8.16 (m, 3H), 7.70 - 7.59 (m, 3H) ppm.

### Step 4. tert-Butyl N-[(2-phenylthiazolo[5,4-c]pyridin-6-yl)methyl]carbamate

SPhos Pd G3 (25.3 mg, 0.032 mmol) and Cs₂CO₃ (211 mg, 0.65 mmol) were added to a mixture of 6-chloro-2-phenyl-thiazolo[5,4-c]pyridine (80 mg, 0.32 mmol) and potassium;2-(tertbutoxycarbonylamino) ethyl-trifluoro-boranuide (122 mg, 0.49 mmol) in dioxane (3 mL) and H₂O (0.5 mL) at 20 °C. The mixture was warmed and stirred at 100 °C for 15 hrs. The mixture was filtered and the filtrate was concentrated in vacuo. The crude residue was purified by column chromatography (SiO₂, Petroleum Ether/EA = 5/1 to 3/1) affording the title compound (60 mg, 0.18 mmol) as a white solid. LCMS (ESI) m/z: [M+H]⁺ = 342.1.
¹H NMR (400 MHz, CDCl₃) δ = 9.13 (s, 1H), 8.15 - 8.13 (m, 2H), 7.94 (s, 1H), 7.62 - 7.49 (m, 4H), 5.50 (s, 1H), 4.62 (d, J = 5.2 Hz, 2H), 1.49 (s, 9H) ppm.

### Step 5. (2-Phenylthiazolo[5,4-c]pyridin-6-yl)methanamine

A mixture of tert-Butyl N-[(2-phenylthiazolo[5,4-c]pyridin-6-yl)methyl]carbamate (60 mg, 0.18 mmol) in HCl/dioxane (4N, 2 mL) was stirred at 25 °C for 2 hrs. The mixture was concentrated in vacuo affording the title compound (30 mg, 0.11 mmol, HCl salt) as a white solid.
LCMS (ESI) m/z: [M+H]⁺ = 242.1

### Step 6. (4R)-4-Cyano-4-methyl-N-[(2-phenylthiazolo[5,4-c]pyridin-6-yl)methyl]isochromane-6-carboxamide

EDCI (20.7 mg, 0.11 mmol), HOBt (14.6 mg, 0.11 mmol), and DIPEA (0.047 mL, 0.27 mmol) were added to a mixture of (2-phenylthiazolo[5,4-c]pyridin-6-yl)methanamine (15 mg, 0.054 mmol, HCl salt), and (4R)-4-cyano-4-methylisochromane-6-carboxylic acid (11.7 mg, 0.054 mmol) in DMF (1 mL) at 20 °C. The mixture was stirred for 15 hrs. The mixture was quenched via the addition of water (5 mL) and then extracted with EA (3 mL x 3). The combined organic layer was washed with brine (10 mL), dried over Na₂SO₄, filtered, and concentrated in vacuo. The crude product was purified by pre-HPLC (column: Phenomenex Synergi C18 150*25*10µm; mobile phase: [water (0.225%FA)-ACN]; B%: 39%-69%, 10min) affording the title compound (20.8 mg, 0.047 mmol) as gray oil.
LCMS (ESI) m/z: [M+H]+=441.1.
¹H NMR (400 MHz, DMSO-d6) δ = 9.41 - 9.30 (m, 2H), 8.21 - 8.13 (m, 3H), 7.96 - 7.90 (m, 2H), 7.68 - 7.57 (m, 3H), 7.29 (d, J = 8.0 Hz, 1H), 4.94 - 4.80 (m, 2H), 4.75 (d, J = 5.6 Hz, 2H), 4.22 (d, J = 11.6 Hz, 1H), 3.86 (d, J = 11.6 Hz, 1H), 1.70 (s, 3H) ppm.

The following examples in Table 18 were prepared using standard chemical manipulations and procedures similar to those used for the preparation of **Example 25.**

**Table 18. Compounds of the Invention**

| **#** | **LCMS (m/z)** | **1H NMR** |
|---|---|---|
| **31** | 446.1 | 1H NMR (400 MHz, DMSO-d6) δ = 9.35 (s, 1H), 9.20 - 9.18(m, 1H), 8.20 - 8.13 (m, 2H), 7.93 (d, J = 200 Hz, 2H), 7.76(d, J = 8.0 Hz, 1H), 7.68 - 7.57 (m, 3H), 7.14 (d, J = 8.0 Hz, 1H), 4.89 - 4.87 (m, 1H), 4.78 - 4.70 (m, 4H), 3.94 (d, J = 11.2 Hz, 1H), 3.59 - 3.54 (m, 1H), 3.41 (s, 2H), 1.18 (s, 3H) ppm |
| **33** | 427.1 | 1H NMR (400 MHz, DMSO-d6) δ = 9.35 (d, J = 0.8 Hz, 1H), 9.18-9.15 (m, 1H), 8.22 - 8.12 (m, 3H), 8.01 - 7.89 (m, 2H), 7.69 -7.57 (m, 3H), 7.06 (d, J = 8.4 Hz, 1H), 5.01 (d, J = 9.6 Hz, 1H), 4.73 (d, J = 6.0 Hz, 2H), 4.55 (d, J = 9.6 Hz, 1H), 1.78 (s, 3H) ppm |
| **34** | 441.1 | 1H NMR (400 MHz, DMSO-d6) δ = 9.35 (s, 1H), 9.21-9.19 (m, 1H), 8.20 - 8.12 (m, 3H), 7.91 (s, 1H), 7.88-7.86 (m, 1H), 7.67 -7.58 (m, 3H), 6.96 (d, J = 8.4 Hz, 1H), 4.73 (br d, J = 6.0 Hz, 2H), 4.40 - 4.24 (m, 2H), 2.44 - 2.41 (m, 1H), 2.23-2.18 (m, 1H), 1.80 (s, 3H) ppm |
| **35** | 432.2 | 1H NMR (400 MHz, DMSO-d6) δ = 9.35 (d, J = 0.4 Hz, 1H), 9.24-9.21 (m, 1H), 8.18 - 8.15 (m, 3H), 7.88 (s, 1H), 7.80-7.77 (m, 1H), 7.67 - 7.58 (m, 3H), 7.14 (d, J = 8.0 Hz, 1H), 5.29 (s, 1H), 4.79 - 4.67 (m, 4H), 3.70 - 3.67 (m, 1H), 3.61 - 3.58 (m, 1H), 1.43 (s, 3H) ppm |
| **36** | 440.2 | 1H NMR (400 MHz, DMSO+D2O) δ = 9.30 (d, J = 3.2 Hz, 1H), 8.92- 8.991 (m, 1H), 8.15 - 8.13 (m, 2H), 7.89 - 7.83 (m, 1H), 7.84 (s, 1H), 7.64 - 7.59 (m, 4H), 6.59 (d, J = 8.8 Hz, 1H), 4.74 - 4.62 (m, 2H), 3.34 - 3.31 (m, 2H), 2.21 - 2.15 (m, 1H), 1.99 - 1.94 (m, 1H), 1.70 (s, 3H) ppm |

### Example 26: (R)-4-Cyano-N-((2-(4-cyclobutyl-2-methoxyphenyl)-1,6-naphthyridin-7-yl)methyl)-4-methylisochromane-6-carboxamide

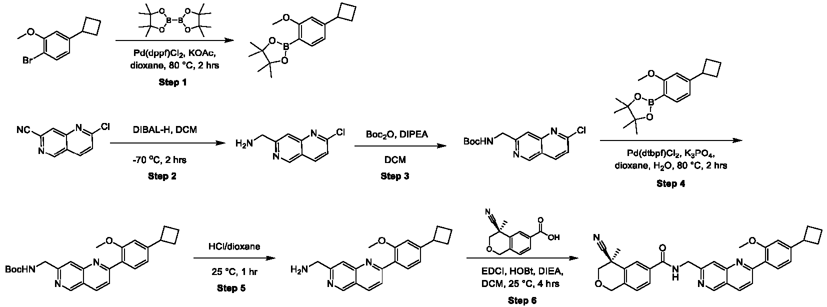

### Step 1: 2-(4-Cyclobutyl-2-methoxyphenyl)-4,4,5,5-tetramethyl-1,3,2-dioxaborolane

To a mixture 1-bromo-4-cyclobutyl-2-methoxybenzene (88.46 mg, 348.37 umol), 4,4,4',4',5,5,5',5'-octamethyl-2,2'-bi(1,3,2-dioxaborolane) (88.46 mg, 348.37 umol) and KOAc (85.47 mg, 870.92 umol) in dioxane (2 mL) was added [1,1-bis(di-tert-butylphosphino)ferrocene]dichloropalladium(II) (23.71 mg, 29.03 umol) under N₂. The mixture was stirred at 80 °C for 2 hrs. The mixture was diluted with EA (20 mL) and filtered. The filtrate was concentrated under vacuum to give the title compound (83 mg, crude) as black oil.
LCMS (ESI) m/z= [M+H]⁺ = 289.1.

### Step 2: (2-Chloro-1,6-naphthyridin-7-yl)methanamine

To a solution of 2-chloro-1,6-naphthyridine-7-carbonitrile (25 g, 131.86 mmol) in DCM (1000 mL) was added DIBAL-H (1 M, 329.64 mL, 2.5 eq) dropwise at -70 °C under N₂. The reaction mixture was stirred at -70°C for 2 h. The reaction mixture was quenched with water (500 mL) and sat. potassium sodium tartrate (1500 mL) and stirred for an additional 30 min. The mixture was extracted with DCM:MeOH=10:1 (6000 mL * 3). The combined organic layers were dried over Na₂SO₄, filtered and concentrated under reduced pressure to give the title compound (51 g, crude) as a brown solid, which was used directly in the next step.
LCMS (ESI) m/z: [³⁵CIM+H]⁺ =194.2

### Step 3: tert-Butyl ((2-chloro-1,6-naphthyridin-7-yl)methyl)carbamate

To a solution of (2-chloro-1,6-naphthyridin-7-yl)methanamine (51 g, 263.39 mmol) in DCM (1500 mL) was added (Boc)₂O (172.45 g, 790.16 mmol) and DIEA (102.12 g, 790.16 mmol). The mixture was stirred at 25 °C for 16 hr. The reaction mixture was diluted with water (1500 mL) and then filtered. The filtrate was extracted with DCM (1000 mL * 3). The combined organic layers were washed with brine (1500 mL), dried over Na₂SO₄, filtered and concentrated under reduced pressure. The residue was purified by column chromatography (SiO₂, Petroleum ether/Ethyl acetate=10/1 to 2/1 to 1/3) to give the title compound (21 g, 64.34 mmol, 24.43% yield) as a light yellow solid.
LCMS (ESI) m/z: [M+H]⁺ =293.9.
¹H NMR (400 MHz, DMSO-d₆) δ=9.37 (s, 1H), 8.62 (d, *J* = 8.4 Hz, 1H), 7.71 (d, *J* = 8.4 Hz, 1H), 7.58 - 7.53 (m, 2H), 4.39 (d, *J* = 6.4 Hz, 2H), 4.20 - 4.25 (m, 2H), 1.41 (s, 9H) ppm

### Step 4: tert-Butyl ((2-(4-cyclobutyl-2-methoxyphenyl)-1,6-naphthyridin-7-yl)methyl)carbamate

A mixture of tert-butyl ((2-chloro-1,6-naphthyridin-7-yl)methyl)carbamate (45 mg, 153.19 umol), 2-(4-cyclobutyl-2-methoxyphenyl)-4,4,5,5-tetramethyl-1,3,2-dioxaborolane (79.47 mg, 275.75 umol), [1,1-bis(di-tert-butylphosphino)ferrocene]dichloropalladium(ll) (9.98 mg, 15.32 umol) and K₃PO₄ (97.55 mg, 459.58 umol) in H₂O (0.4 mL) and dioxane (2 mL) was degassed and purged with N₂ for 3 times. The mixture was then stirred at 80 °C for 2 hrs under N₂ atmosphere. The mixture was concentrated under vacuum and the residue purified by column chromatography (SiO₂, Petroleum ether/Ethyl acetate=20/1 to 0/1), to give the title compound (55 mg, 120.62 umol, 78.73% yield) as a yellow solid.
LCMS (ESI) m/z= [M+H]⁺ = 420.3

### Step 5: (2-(4-Cyclobutyl-2-methoxyphenyl)-1,6-naphthyridin-7-yl)methanamine hydrochloride

A mixture of *tert*-butyl ((2-(4-cyclobutyl-2-methoxyphenyl)-1,6-naphthyridin-7-yl)methyl)carbamate (55 mg, 131.10 umol) in HCl/dioxane (4 M, 4 mL) was stirred at 25 °C for 1 hr under N₂ atmosphere. The mixture was concentrated under vacuum to give the title compound (46 mg, crude) as a yellow solid.
LCMS (ESI) m/z= [M+H]⁺ = 320.3

### Step 6: (R)-4-Cyano-N-((2-(4-cyclobutyl-2-methoxyphenyl)-1,6-naphthyridin-7-yl)methyl)-4-methylisochromane-6-carboxamide

To a mixture of (2-(4-cyclobutyl-2-methoxyphenyl)-1,6-naphthyridin-7-yl)methanamine hydrochloride (46 mg, 129.26 umol), EDCl (37.17 mg, 193.90 umol), (*R*)-4-cyano-4-methylisochromane-6-carboxylic acid (33.69 mg, 155.12 umol) and DIEA (83.53 mg, 646.32 umol) in DCM (1 mL) was added EDCl (37.17 mg, 193.90 umol) and HOBt (26.20 mg, 193.90 umol). The mixture was stirred at 25 °C for 4 hrs under N₂ atmosphere. The mixture was concentrated under vacuum and the residue was purified by reversed phase HPLC (0.1% FA condition). The product fraction was concentrated under vacuum to remove MeCN, then lyophilized to give the title compound (27.13 mg, 52.31 umol, 40.47% yield) as a yellow solid.
LCMS (ESI) m/z= [M+H]⁺ = 519.3.
¹H NMR (400 MHz, DMSO-d₆) δ = 9.39-9.36 (m, 1H), 9.34 (s, 1H), 8.51 (d, *J* = 8.4 Hz, 1H), 8.19 (d, *J* = 1.6 Hz, 1H), 8.06 (d, *J* = 8.4 Hz, 1H), 7.95-7.93 (m, 1H), 7.77 - 7.75 (m, 2H), 7.30 (d, *J* = 8.0 Hz, 1H), 7.02 (s, 1H), 6.97 (d, *J* = 8.0 Hz, 1H), 4.92 - 4.79 (m, 4H), 4.22 (d, *J* = 11.2Hz, 1H), 3.84 - 3.84 (m, 4H), 3.62-3.57 (m, 1H), 2.34-2.31 (m, 2H), 2.21-2.12 (m, 2H), 2.03 - 1.964 (m, 1H), 1.87 - 1.83 (m, 1H), 1.69 (s, 3H) ppm

### Example 27: (R)-4-Cyano-N-((2-(7-fluoro-1,2-dimethyl-1H-indol-4-yl)-1,6-naphthyridin-7-yl)methyl)-4-methylisochromane-6-carboxamide

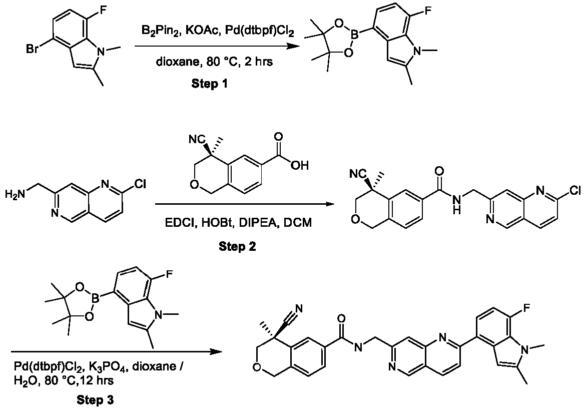

### Step 1: 7-Fluoro-1,2-dimethyl-4-(4,4,5,5-tetramethyl-1,3,2-dioxaborolan-2-yl)-1H-indole

To a solution of 4-bromo-7-fluoro-1,2-dimethyl-1H-indole (60 mg, 247.84 umol) and 4,4,4',4',5,5,5',5'-octamethyl-2,2'-bi(1,3,2-dioxaborolane) (69.23 mg, 272.63 umol) in dioxane (1 mL) was added KOAc (72.97 mg, 743.53 umol) and [1,1-bis(di-tert-butylphosphino)ferrocene]dichloropalladium(II) (16.15 mg, 24.78 umol). The mixture was stirred at 80°C for 2 hrs under N₂. The mixture was diluted with EA (10 mL) and filtered. The filtrate was concentrated to afford the title compound (70 mg, crude) as brown oil which was used directly in the next step.

### Step 2: (R)-N-((2-Chloro-1,6-naphthyridin-7-yl)methyl)-4-cyano-4-methylisochromane-6-carboxamide

To a dry 4 mL vial containing 1-(2-chloro-1,6-naphthyridin-7-yl)methanamine (0.040 g, 0.207 mmol, 1 equiv.) was added (*R*)-4-cyano-4-methylisochromane-6-carboxylic acid (0.045 g, 0.207 mmol, 1 equiv.), EDCl (0.048 g, 0.248 mmol, 1.2 equiv.), DIEA (0.087 mL, 0.496 mmol, 2.4 equiv.), HOBt (0.034 g, 0.248 mmol, 1.2 equiv.) and DCM (1.4 mL, 0.15 M). The reaction mixture was stirred at rt overnight and directly loaded onto silica gel for purification (5% MeOH in DCM as the eluent) to give (4R)-N-[(2-chloro-1,6-naphthyridin-7-yl)methyl]-4-cyano-4-methyl-3,4-dihydro-1H-2-benzopyran-6-carboxamide (68.4 mg, 0.207 mmol, 84% yield) as an off-white solid.
LCMS (m/z) 393.17 [M+H]

### Step 3: (R)-4-Cyano-N-((2-(7-fluoro-1,2-dimethyl-1H-indol-4-yl)-1,6-naphthyridin-7-yl)methyl)-4-methylisochromane-6-carboxamide

To a solution of (*R*)-*N*-((2-chloro-1,6-naphthyridin-7-yl)methyl)-4-cyano-4-methylisochromane-6-carboxamide (40 mg, 101.82 umol) and 7-fluoro-1,2-dimethyl-4-(4,4,5,5-tetramethyl-1,3,2-dioxaborolan-2-yl)-1H-indole (58.88 mg, 203.65 umol) in dioxane / H₂O = 4 / 1 (1 mL) was added K₃PO₄ (64.84 mg, 305.47 umol) and [1,1-bis(di-tert-butylphosphino)ferrocene]dichloropalladium(II) (6.64 mg, 10.18 umol). The mixture was stirred at 80°C for 12 hrs under N₂. The mixture was diluted with sat. NaHCO₃ (10 mL) and extracted with DCM (10mL*2). The combined organic layers were dried over anhydrous Na₂SO₄, filtered and concentrated. The residue was purified by reversed phase prep-HPLC (0.1% FA condition). The product fraction was concentrated to remove ACN and lyophilized to afford the title compound (12.07 mg, 22.87 umol, 22.46% yield) as a yellow solid.
LCMS (ESI) m/z: [M+H]⁺ = 520.2
¹H NMR (400 MHz, DMSO-d6) δ = 9.40 - 9.35 (m, 2H), 8.60 (d, *J* = 8.8 Hz, 1H), 8.20 - 8.16 (m, 2H), 7.95 - 7.93 (m, 1H), 7.84 (s,1H), 7.69 - 7.68 (m, 1H), 7.30 (d, *J* = 8.0 Hz, 1H), 7.08 - 7.01 (m, 2H), 4.92 - 4.81 (m, 4H), 4.23 (d, *J* = 11.2 Hz, 1H), 3.89 - 3.84 (m,4H), 2.44 (s, 3H), 1.70 - 1.68 (m, 3H) ppm.
Chiral SFC: Cellucoat-MeOH(DEA)-40-3mL-35T.|cm, Rt = 1.380; ee% = 100%.

### Example 28: (R)-4-Cyano-N-((2-(imidazo[1,2-b]pyridazin-3-yl)-1,6-naphthyridin-7-yl)methyl)-4-methylisochromane-6-carboxamide

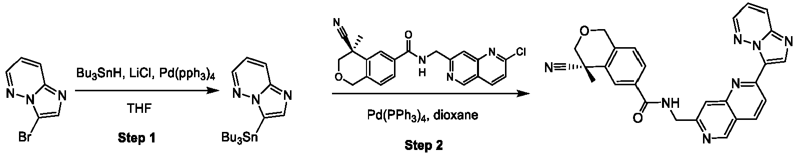

### Step 1: 3-(Tributylstannyl)imidazo[1,2-b]pyridazine

To a solution of 3-bromoimidazo[1,2-*b*]pyridazine (200 mg, 1.01 mmol) and Bu₃SnH (411.56 mg, 1.41 mmol, 374.14 uL) in THF (2 mL) was added LiCl (64.22 mg, 1.51 mmol, 31.02 uL) and Pd(PPh₃)₄ (116.71 mg, 101.00 umol) at 25°C. The mixture was stirred at 80°C for 2 hr under N₂. Water (5 mL) was added and the products extracted into EA (10 mL*3). The organic layer was concentrated and the residue was purified by column chromatography (SiO₂, PE:EA=5:1, Rf = 0.5) and concentrated to give the title compound (100 mg, 245.00 umol, 24.26% yield) as a colorless oil.
LCMS (ESI) m/z: [M+H]+ = 410.0.

### Step 2: (R)-4-Cyano-N-((2-(imidazo[1,2-b]pyridazin-3-yl)-1,6-naphthyridin-7-yl)methyl)-4-methylisochromane-6-carboxamide

To a solution of 3-(tributylstannyl)imidazo[1,2-b]pyridazine (37.41 mg, 91.64 umol) in dioxane (0.5 mL) was added (*R*)-*N*-((2-chloro-1,6-naphthyridin-7-yl)methyl)-4-cyano-4-methylisochromane-6-carboxamide (30 mg, 76.37 umol) and Pd(PPh₃)₄ (4.41 mg, 3.82 umol) at 25°C under N₂. The mixture was heated to 100°C and stirred for 2 hrs. The mixture was quenched with saturated aqueous KF (5 mL), then extracted with EA (10 mL*3). The organic layer was concentrated and the residue purified by prep-HPLC (FA method) to give the title compound (12 mg, 20.89 umol, 27.35% yield) as a light yellow solid. LCMS (ESI) m/z: [M+H]+ = 476.3.
¹HNMR (400 MHz, MeOD-d₄) δ = 9.34 - 9.28 (m, 1H), 8.96 (d, J = 8.8 Hz, 1H), 8.75 - 8.62 (m, 3H), 8.54 - 8.44 (m, H), 8.23 - 8.17 (m, 2H), 7.97 - 7.91 (m, 2H), 7.47 - 7.43 (m, 1H), 7.30 (d, J = 8.4 Hz, 1H), 4.93 - 4.91 (m, 4H), 4.21 (d, J = 1.6 Hz, 1H), 3.93 (d, J = 11.6 Hz, 1H), 1.77 (s, 3H) ppm.
Chiral SFC: Cellucoat-MeOH+ACN(DEA)-40-3mL-35T.|cm; Rt= 1.034min , ee% =100%.

The following examples in Table 19 were prepared using standard chemical manipulations and procedures similar to those used for the preparation of **Examples 21, 26, 27 and 28.**

**Table 19. Compounds of the Invention**

| # | LCMS (m/z) | 1H NMR |
|---|---|---|
| 128 | 577.3 | 1H NMR (400 MHz, DMSO-d6) δ = 9.46 - 9.37 (m, 2H), 8.72 - 8.70 (m, 2H), 8.43 (d, J = 8.0 Hz, 1H), 8.21 (s, 2H), 7.99 - 7.88 (m, 2H), 7.84 (s, 1H), 7.46 (d, J = 7.6 Hz, 1H), 7.31 (d, J = 8.4 Hz, 1H), 4.96 - 4.80 (m, 4H), 4.23 (d, J = 11.6 Hz, 1H), 3.87 (d, J = 11.6 Hz, 1H), 3.49 - 3.46 (m, 2H), 3.26 (s, 3H), 3.05 - 3.02 (m, 2H), 2.84 - 2.74 (m, 1H), 2.56 - 2.53 (m, 2H), 2.16 - 2.15 (m, 2H), 1.94 - 1.84 (m, 4H), 1.71 (s, 3H) ppm |
| 130 | 495.05 | 1H NMR (300 MHz, DMSO-d6) δ 9.49 (s, 1H), 9.38 - 9.22 (m, 2H), 8.82 (d, J = 9.0 Hz, 2H), 8.65 (d, J = 8.7 Hz, 1H), 8.14 (d, J = 1.8 Hz, 1H), 7.88 (dd, J = 8.1, 1.7 Hz, 1H), 7.27 (d, J = 8.1 Hz, 1H), 5.05 - 4.73 (m, 4H), 4.21 (d, J = 11.5 Hz, 1H), 3.85 (d, J = 11.5 Hz, 1H), 2.45 - 2.32 (m, 1H), 1.68 (s, 3H), 1.28 - 1.08 (m, 4H). |
| 131 | 533.3 | 1H NMR (400 MHz, MeOD) δ = 9.34 (s, 1H), 8.76 (d, J = 8.4 Hz, 1H), 8.62 (d, J = 8.0 Hz, 1H), 8.55 - 8.47 (m, 3H), 8.18 (d, J = 1.6 Hz, 1H), 7.98 (s, 1H), 7.96 - 7.90 (m, 2H), 7.46 (d, J = 7.6 Hz, 1H), 7.30 (d, J = 8.0 Hz, 1H), 4.94 - 4.91 (m, 4H), 4.21 (d, J = 11.6 Hz, 1H), 3.93 (d, J = 11.6 Hz, 1H), 3.49 (d, J = 12.0 Hz, 2H), 3.16 - 3.06 (m, 1H), 3.02 - 2.90 (m, 2H), 2.79 (s, 3H), 2.27 - 2.19 (m, 4H), 1.77 (s, 3H) ppm |
| 132 | 578.3 | 1H NMR (400 MHz, DMSO+D2O) δ = 9.44 - 9.42 (m, 1H), 9.35 (s, 1H), 8.69 - 8.53 (m, 2H), 8.26 (s, 1H), 8.15 (d, J = 1.6 Hz, 1H), 7.92 - 7.90 (m, 1H), 7.85 (d, J = 7.2 Hz, 1H), 7.78 (s, 1H), 7.73 - 7.69 (m, 1H), 7.30 (d, J = 8.0 Hz, 1H), 6.97 (d, J = 8.4 Hz, 1H), 4.95 - 4.70 (m, 4H), 4.20 (d, J = 11.6 Hz, 1H), 3.84 - 3.81 (m, 1H), 3.64 - 3.57 (m, 4H), 3.49 - 3.46 (m, 2H), 3.23 (s, 3H), 2.59 - 2.53 (m, 6H), 1.67 (s, 3H) ppm |
| 133 | 546.2 | 1H NMR (400 MHz, DMSO-d6) δ = 9.43 - 9.33 (m, 2H), 8.60 (d, J = 8.4 Hz, 1H), 8.41 (s, 1H), 8.24 - 8.18 (m, 2H), 7.95 - 7.93 (m, 1H), 7.85 (s, 1H), 7.72 (d, J = 7.2 Hz, 1H), 7.60 (d, J = 8.0 Hz, 1H), 7.30 (d, J = 8.0 Hz, 1H), 7.23 - 7.21 (m, 1H), 6.95 (s, 1H), 4.94 - 4.78 (m, 4H), 4.38 - 4.35 (m, 2H), 4.22 (d, J = 11.2 Hz, 1H), 3.86 (d, J = 11.6 Hz, 1H), 3.63 - 3.61 (m, 2H), 3.20 (s, 3H), 2.47 (s, 3H), 1.70 (s, 3H) ppm |
| 134 | 516.3 | 1H NMR (400 MHz, DMSO-d6) δ= 9.43 - 9.34 (m, 2H), 8.60 (d, J = 8.8 Hz, 1H), 8.44 - 8.39 (m, 1H), 8.26 - 8.17 (m, 2H), 7.94 (d, J = 1.6 Hz, 1H), 7.83 (s, 1H), 7.74 (d, J = 7.2 Hz, 1H), 7.59 (d, J = 8.4 Hz, 1H), 7.36 - 7.18 (m, 2H), 6.99 (s, 1H), 4.94 - 4.80 (m, 4H), 4.29 - 4.17 (m, 1H), 3.86 (d, J = 11.6 Hz, 1H), 3.74 (s, 3H), 2.85 - 2.79 (m, 2H), 1.69 (s, 3H), 1.30 - 1.27 (m, 3H) ppm |
| 135 | 528.3 | 1H NMR (400 MHz, DMSO-d6) δ= 9.47 - 9.29 (m, 2H), 8.59 (d, J = 8.8 Hz, 1H), 8.26 - 8.16 (m, 2H), 7.96 (d, J = 1.6 Hz, 1H), 7.84 (s, 1H), 7.74 (d, J = 7.2 Hz, 1H), 7.58 (d, J = 8.0 Hz, 1H), 7.35 - 7.20 (m, 2H), 6.83 (s, 1H), 4.94 - 4.80 (m, 4H), 4.22 (d, J = 11.2 Hz, 1H), 3.90 - 3.84 (m, 4H), 2.13 - 2.01 (m, 1H), 1.70 (s, 3H), 1.06 - 0.96 (m, 2H), 0.76 - 0.66 (m, 2H) ppm |
| 136 | 545.2 | 1H NMR (400 MHz, DMSO-d6) δ= 9.37-9.31 (m, 1H), 9.31-9.25 (m, 1H), 8.89 (s, 1H), 8.53 (d, J = 8.4 Hz, 1H), 8.16 (s, 1H), 7.99 - 7.90 (m, 2H), 7.69 (s, 1H), 7.35-7.25 (m, 1H), 6.69 - 6.35 (m, 1H), 5.62 - 5.59 (m, 1H), 4.98 - 4.93 (m, 4H), 4.92 - 4.81 (m, 2H), 4.87 (d, J = 6.0 Hz, 2H), 4.22 ( d, J = 11.6 Hz, 1H), 3.87 - 3.85 (m, 1H), 3.80- 3.66 (m, 2H), 1.69 (s, 3H) ppm |
| 137 | 507.3 | 1H NMR (400 MHz, DMSO-d6) δ = 9.51 (s, 1H), 9.44 - 9.41 (m, 1H), 9.05 (d, J = 5.2 Hz, 1H), 8.82 (d, J = 8.8 Hz, 1H), 8.71 (d, J = 8.4 Hz, 1H), 8.43 (d, J = 5.2 Hz, 1H), 8.21 (d, J = 1.6 Hz, 1H), 7.97 - 7.95 (m, 1H), 7.90 (s, 1H), 7.32 (d, J = 8.0 Hz, 1H), 5.13 (d, J = 5.6 Hz, 2H), 4.95 - 4.81 (m, 4H), 4.61 (d, J = 5.6 Hz, 2H), 4.23 (d, J = 11.6 Hz, 1H), 3.87 (d, J = 11.6 Hz, 1H), 1.80 (s, 3H), 1.71 (s, 3H) ppm |
| 138 | 506.2 | 1H NMR (400 MHz, DMSO-d6) δ = 9.46 - 9.32 (m, 2H), 8.62 (d, J = 8.8 Hz, 1H), 8.19 (d, J = 1.6 Hz, 1H), 7.96 - 7.94 (m, 2H), 7.80 (s, 1H), 7.65 (d, J = 2.0 Hz, 1H), 7.56 - 7.45 (m, 2H), 7.41 - 7.24 (m, 2H), 4.96 - 4.77 (m, 4H), 4.22 (d, J = 11.6 Hz, 1H), 3.86 (d, J = 11.6 Hz, 1H), 3.81 (s, 3H), 1.69 (s, 3H) ppm |
| 139 | 538.2 | 1H NMR (400 MHz, DMSO-d6) δ= 9.45 - 9.35 (m, 2H), 8.66 (d, J = 8.8 Hz, 1H), 8.47 - 8.39 (m, 1H), 8.26 (d, J = 8.8 Hz, 1H), 8.19 (d, J = 1.2 Hz, 1H), 7.98 - 7.85 (m, 3H), 7.79 (d, J = 8.4 Hz, 1H), 7.63 (s, 1H), 7.57 - 7.40 (m, 2H), 7.29 (d, J = 7.6 Hz, 1H), 4.95 - 4.79 (m, 4H), 4.22 (d, J = 11.2 Hz, 1H), 3.92 (s, 3H), 3.86 (d, J = 11.6 Hz, 1H), 1.70 (s, 3H) ppm |
| 140 | 531.2 | 1H NMR (400 MHz, DMSO-d6) δ = 9.49 - 9.34 (m, 1H), 9.25 (s, 1H), 8.56 - 8.46 (m, 2H), 8.42 (br s, 1H), 8.20 (s, 1H), 8.02 (d, J = 8.8 Hz, 1H), 7.95 (d, J = 8.4 Hz, 1H), 7.61 (s, 1H), 7.32 (d, J = 8.4 Hz, 1H), 4.98 - 4.82 (m, 2H), 4.77 (br d, J = 6.0 Hz, 2H), 4.53 - 4.39 (m, 1H), 4.23 (d, J = 11.6 Hz, 1H), 400 (s, 3H), 3.87 (d, J = 11.2 Hz, 1H), 2.62 - 2.56 (m, 1H), 2.34 - 2.22 (m, 1H), 1.70 (s, 3H) ppm |
| 141 | 532.2 | 1H NMR (400 MHz, DMSO-d6) δ = 9.45 - 9.30 (m, 2H), 8.63 (d, J = 8.8 Hz, 1H), 8.29 - 8.15 (m, 2H), 7.94 (d, J = 7.2 Hz, 1H), 7.78 (d, J = 7.6 Hz, 1H), 7.66 (d, J = 8.0 Hz, 1H), 7.39 - 7.25 (m, 2H), 7.23 (s, 1H), 4.97 - 4.71 (m, 4H), 4.65 (s, 2H), 4.29 - 4.16 (m, 1H), 3.86 (d, J = 11.2 Hz, 1H), 3.83 - 3.76 (m, 3H), 3.29 (s, 3H), 1.70 (s, 3H) ppm |
| 144 | 479.20 | ¹H NMR (400 MHz, DMSO-*d*₆) δ 9.44 - 9.38 (m, 2H), 8.56 (d, *J* = 8.7 Hz, 1H), 8.19 (d, *J* = 1.8 Hz, 1H), 8.12 (d, *J* = 8.2 Hz, 1H), 7.99 - 7.90 (m, 1H), 7.82 (s, 1H), 7.75 (d, *J* = 7.8 Hz, 1H), 7.30 (d, *J* = 8.1 Hz, 1H), 7.06 (d*, J* = 1.5 Hz, 1H), 6.96 - 6.90 (m, 1H), 4.95 - 4.79 (m, 4H), 4.22 (d*, J =* 11.4 Hz, 1H), 3.91 - 3.83 (m, 4H), 2.39 (s, 3H), 1.70 (s, 3H). |
| 145 | 531.2 | 1H NMR (400 MHz, DMSO-d6) δ= 9.38 - 9.30 (m, 2H), 8.98 (s, 1H), 8.59 (d, J = 8.8 Hz, 1H), 8.18 (d, J = 1.6 Hz, 1H), 8.00 - 8.00 (m, 1H), 7.96 - 7.69 (m, 3H), 7.30 (d, J = 8.0 Hz, 1H), 5.73 - 5.66 (m, 1H), 5.01 - 4.94 (m, 4H), 4.92 - 4.85 (m, 2H), 4.81 - 4.78 (m, 2H), 4.22 (d, J = 11.2 Hz, 1H), 3.87 (d, J = 11.6 Hz, 1H), 1.69 (s, 3H)ppm |
| 146 | 520.2 | 1H NMR (400 MHz, DMSO-d6) δ = 9.37-9.34 (m, 1H), 9.24 (s, 1H), 8.39 (d, J = 8.8 Hz, 1H), 8.19 (d, J = 1.6 Hz, 1H), 8.10 (d, J = 8.8 Hz, 1H), 7.95-7.93 (m, 1H), 7.85 (d, J = 8.4 Hz, 1H), 7.68 (s, 1H), 7.30 (d, J = 8.0 Hz, 1H), 6.12 - 6.07 (m, 2H), 4.92 - 4.81 (m, 2H), 4.77 (br d, J = 6.0 Hz, 2H), 4.22 (d, J = 11.6 Hz, 1H), 3.94-3.90 (m, 4H), 3.87 - 3.84 (m, 4H), 2.38 - 2.33 (m, 2H), 1.69 (s, 3H) ppm |
| 147 | 538.2 | 1H NMR (400 MHz, DMSO-d6) δ = 9.46 - 9.39 (m, 2H), 8.64 - 8.62 (m, 1H), 8.25 - 8.20 (m, 2H), 7.95 (d, J = 8.0 Hz, 1H), 7.85 (s, 1H), 7.81 - 7.79 (m, 2H), 7.53 - 7.52 (m, 1H), 7.35 - 7.27 (m, 3H), 6.53 - 6.26 (m, 1H), 4.92 - 4.74 (m, 6H), 4.23 (d, J = 11.2 Hz, 1H), 3.87 (d, J = 11.2 Hz, 1H), 1.70 (s, 3H) ppm |
| 148 | 521.2 | 1H NMR (400 MHz, DMSO-d6) δ = 9.39 - 9.35 (m, 2H), 8.51 (d, J = 8.8 Hz, 1H), 8.19 (d, J = 1.6 Hz, 1H), 8.07 (d, J = 8.4 Hz, 1H), 7.99-7.93 (m, 1H), 7.76 - 7.74 (m, 2H), 7.30 (d, J = 8.0 Hz, 1H), 7.15 - 7.11 (m, 2H), 4.92 - 4.79(m, 4H), 4.22 (d, J = 11.6 Hz, 1H), 3.89 - 3.84 (m, 4H), 1.69 (s, 3H), 1.34 (s, 9H) ppm |
| 149 | 504.2 | 1H NMR (400 MHz, DMSO-d6) δ = 9.45 - 9.31 (m, 2H), 8.59 - 8.57 (m, 1H), 8.17 (d, J = 1.6 Hz, 1H), 7.93 - 7.91 (m, 1H), 7.78 - 7.69 (m, 2H), 7.29 (d, J = 8.0 Hz, 1H), 7.14 - 7.12 (m, 1H), 6.75 - 6.62 (m, 2H), 4.92 - 4.84 (m, 2H), 4.80 - 4.79 (m, 2H), 4.21 (d, J = 11.6 Hz, 1H), 3.86 (d, J = 11.6 Hz, 1H), 3.24 - 3.18 (m, 2H), 2.89 (s, 3H), 2.65 - 2.62 (m, 2H), 1.81 - 1.75 (m, 2H), 1.69 (s, 3H) ppm |
| 150 | 503.2 | 1H NMR (400 MHz, DMSO-d6) δ = 9.44 (s, 1H), 9.40 (s, 1H), 8.71 (d, J = 8.4 Hz, 1H), 8.36 - 8.31 (m, 2H), 8.20 (d, J = 1.6 Hz, 1H), 7.96 - 7.91 (m, 2H), 7.76 (d, J = 4.8 Hz, 1H), 7.30 (d, J = 8.0 Hz, 1H), 6.99 (d, J = 0.8 Hz, 1H), 4.94 - 4.81 (m, 4H), 4.22 (d, J = 11.6 Hz, 1H), 3.87 (d, J = 11.2 Hz, 1H), 3.80 (s, 3H), 2.52 (s, 3H), 1.70 (s, 3H) ppm |
| 151 | 489.2 | 1H NMR (400 MHz, DMSO-d6) δ = 9.50 - 9.32 (m, 2H), 9.19 (s, 1H), 8.64 (d, J = 8.8 Hz, 1H), 8.41 (d, J = 8.8 Hz, 1H), 8.21 (d, J = 1.6 Hz, 1H), 8.07 - 7.98 (m, 2H), 7.95 - 7.93 (m, 1H), 7.82 (d, J = 8.4 Hz, 1H), 7.45 - 7.41 (m, 1H), 7.30 (d, J = 8.4 Hz, 1H), 4.92 - 4.78 (m, 4H), 4.25 - 4.17 (m, 4H), 3.87 (d, J = 11.6 Hz, 1H), 1.71 (s, 3H) ppm |
| 152 | 519.2 | 1H NMR (400 MHz, DMSO-d6) δ = 9.45 - 9.36 (m, 2H), 8.85 (s, 1H), 8.68 (d, J = 8.4 Hz, 1H), 8.41 - 8.36 (m, 1H), 8.20 (d, J = 1.2 Hz, 1H), 8.00 - 7.93 (m, 3H), 7.88 (d, J = 8.4 Hz, 1H), 7.58 - 7.53 (m, 1H), 7.31 (d, J = 8.0 Hz, 1H), 4.94 - 4.81 (m, 5H), 4.53 - 4.50 (m, 2H), 4.23 (d, J = 11.2 Hz, 1H), 3.89 - 3.81 (m, 3H), 1.70 (s, 3H) ppm |
| 153 | 479.25 | ¹H NMR (400 MHz, Methanol-*d*₄) δ 9.30 (d, *J =* 0.8 Hz, 1H), 8.48 (dd, J = 8.7, 0.9 Hz, 1H), 8.16 (d, *J* = 1.8 Hz, 1H), 8.04 (d, *J* = 8.6 Hz, 1H), 7.95 - 7.89 (m, 2H), 7.57 - 7.50 (m, 1H), 7.33 - 7.23 (m, 2H), 7.06 (d, *J* = 8.4 Hz, 1H), 4.93 - 4.88 (m, 4H), 4.19 (d, *J* = 11.4 Hz, 1H), 3.91 (d, *J* = 11.4 Hz, 1H), 3.85 (s, 3H), 2.34 (s, 3H), 1.75 (s, 3H). |
| 154 | 547.1 | 1H NMR (400 MHz, DMSO-d6) δ= 9.41 - 9.38 (m, 1H), 9.29 (s, 1H), 8.70 (s, 1H), 8.59 (d, J = 8.8 Hz, 1H), 8.19 (d, J = 1.6 Hz, 1H), 7.95 - 7.91 (m, 2H), 7.75 - 7.38 (m, 2H), 7.31 (d, J = 8.0 Hz, 1H), 5.60 - 5.53 (m, 1H), 4.93 - 4.86 (m, 6H), 4.82-4.77 (m, 2H), 4.23 (d, J = 11.2 Hz, 1H), 3.86 (d, J = 11.2 Hz, 1H), 1.70 (s, 3H) ppm |
| 155 | 550.2 | 1H NMR (400 MHz, DMSO-d6) δ = 9.37 - 9.34 (m, 1H), 9.27 (s, 1H), 8.43 (d, J = 8.4 Hz, 1H), 8.19 (d, J = 1.2 Hz, 1H), 8.12 (d, J = 8.4 Hz, 1H), 7.95-7.93 (m, 1H), 7.87 (d, J = 8.4 Hz, 1H), 7.71 (s, 1H), 7.30 (d, J = 8.0 Hz, 1H), 6.67 - 6.65 (m, 2H), 4.92 - 4.77 (m, 4H), 4.22 (d, J = 11.6 Hz, 1H), 3.89 - 3.84 (m, 4H), 3.77 - 3.74 (m, 4H), 3.28 - 3.25 (m, 4H), 1.70 (s, 3H) ppm |
| 156 | 499.1 | 1H NMR (400 MHz, METHANOL-d4) δ = 9.44 - 9.35 (m, 2H), 8.56 (d, J = 8.4 Hz, 1H), 8.19 (s, 1H), 8.05 (d, J = 8.8 Hz, 1H), 7.95 - 7.93 (m, 1H), 7.86 - 7.76 (m, 2H), 7.36 - 7.27 (m, 2H), 7.17 - 7.15 (m, 1H), 4.96 - 4.76 (m, 4H), 4.22 (d, J = 11.6 Hz, 1H), 3.90 (s, 3H), 3.86 (d, J = 11.6 Hz, 1 H), 1.69 (s, 3H) ppm |
| 157 | 515.2 | 1H NMR (400 MHz, DMSO-d6) δ = 9.40 (s, 2H), 8.58 (d, J = 8.4 Hz, 1H), 8.19 (d, J = 1.2 Hz, 1H), 8.07 (d, J = 8.8 Hz, 1H), 7.99 - 7.86 (m, 2H), 7.80 (s, 1H), 7.39 (s, 1H), 7.30 (d, J = 8.0 Hz, 2H), 7.25 - 6.93 (m, 1H), 4.96 - 4.76 (m, 4H), 4.22 (d, J = 11.6 Hz, 1H), 3.91 (s, 3H), 3.86 (d, J = 11.2 Hz, 1H), 1.69 (s, 3H) ppm |
| 158 | 493.2 | 1H NMR (400 MHz, DMSO-d6) δ = 9.51 (s, 1H), 9.48 - 9.39 (m, 1H), 9.04 (d, J = 5.2 Hz, 1H), 8.87 - 8.78 (m, 1H), 8.73 (d, J = 8.4 Hz, 1H), 8.45 (d, J = 5.2 Hz, 1H), 8.20 (d, J = 1.6 Hz, 1H), 7.97 - 7.94 (m, 1H), 7.90 (s, 1H), 7.32 (d, J = 8.0 Hz, 1H), 5.03 - 4.82 (m, 8H), 4.73 - 4.52 (m, 1H), 4.23 (d, J = 11.2 Hz, 1H), 3.87 (d, J = 11.2 Hz, 1H), 1.71 (s, 3H) ppm |
| 159 | 518.1 | 1H NMR (400 MHz, DMSO-d6) δ = 9.42 - 9.35 (m, 2H), 8.62 (d, J = 8.4 Hz, 1H), 8.28 - 8.17 (m, 2H), 7.95 - 7.93 (m, 1H), 7.84 (s, 1H), 7.77 (d, J = 7.2 Hz, 1H), 7.69 (d, J = 8.0 Hz, 1H), 7.52 (d, J = 3.2 Hz, 1H), 7.35 - 7.26 (m, 2H), 7.18 (d, J = 2.8 Hz, 1H), 4.94 - 4.81 (m, 5H), 4.31 - 4.28 (m, 2H), 4.22 (d, J = 11.6 Hz, 1H), 3.87 (d, J = 11.2 Hz, 1H), 3.77 - 3.71 (m, 2H), 1.70 (s, 3H) ppm |
| 160 | 501.15 | ¹H NMR (300 MHz, DMSO-*d*₆) δ 9.49 - 9.38 (m, 2H), 8.88 - 8.72 (m, 2H), 8.55 (dd, *J* = 7.9, 1.0 Hz, 1H), 8.30 (s, 1H), 8.10 - 7.95 (m, 2H), 7.85 (s, 1H), 7.81 - 7.73 (m, 1H), 7.32 (d, *J* = 8.1 Hz, 1H), 6.69 (s, 1H), 5.16 - 5.04 (m, 2H), 4.94 - 4.68 (m, 6H), 4.09 (t, *J* = 13.0 Hz, 1H), 3.86 (dd, *J =* 25.2, 12.5 Hz, 1H), 1.71 (d, *J* = 20.7 Hz, 3H). |
| 162 | 489.2 | 1H NMR (400 MHz, DMSO-d6) δ= 9.40 - 9.35 (m, 2H), 8.61 (d, J = 8.8 Hz, 1H), 8.51 - 8.47 (m, 1H), 8.22 - 8.19 (m, 2H), 7.96-7.93(m, 1H), 7.75 (s, 1H), 7.50 - 7.23 (m, 3H), 4.93 - 4.76 (m, 4H), 4.26-4.16 (m, 1H), 4.04 (s, 3H), 3.87 (d, J = 11.2 Hz, 1H), 1.70 (s, 3H) ppm |
| 163 | 532.3 | 1H NMR (400 MHz, DMSO-d6) δ = 9.45 - 9.41 (m, 2H), 8.73 (d, J = 8.6 Hz, 1H), 8.71 - 8.57(m, 2H), 8.20 (d, J = 1.2 Hz, 1H), 8.01 - 7.99 (m, 1H), 7.96 - 7.94 (m, 1H), 7.84 (s, 1H), 7.70 - 7.68 (m, 1H), 7.32 (d, J = 8.4 Hz, 1H), 4.93 - 4.82 (m, 4H), 4.24 (d, J = 11.2 Hz, 1H), 3.88 (d, J = 11.6 Hz, 1H), 3.37 (s, 3H), 1.70 (s, 3H), 1.53 (s, 6H) ppm |
| 164 | 475.3 | 1HNMR (400 MHz, DMSO-d6) δ = 9.48 - 9.33 (m, 2H), 8.79 - 8.78 (m, 1H), 8.66 (d, J = 8.8 Hz, 1H), 8.39 (d, J = 8.8 Hz, 1H), 8.21 (d, J = 1.6 Hz, 1H), 7.97 - 7.95 (m, 1H), 7.86 - 7.71 (m, 2H), 7.40 (s, 1H), 7.35 - 7.24 (m, 2H), 7.02 - 6.98 (m, 1H), 4.96 - 4.76 (m, 4H), 4.23 (d, J = 11.6 Hz, 1H), 3.87 (d, J = 11.6 Hz, 1H), 1.71 (s, 3H), ppm |
| 165 | 543.2 | 1H NMR (400 MHz, DMSO-d6) δ = 9.46 - 9.42 (m, 2H), 8.85 - 8.83 (m, 1H), 8.78 - 8.76 (m, 1H), 8.51 (d, J = 8.0 Hz, 1H), 8.46 (s, 1H), 8.30 (d, J = 8.0 Hz, 1H), 8.22 (d, J = 1.6 Hz, 1H), 7.99 - 7.95 (m, 2H), 7.87 (s, 1H), 7.32 (d, J = 8.0 Hz, 1H), 6.69 (d, J = 9.6 Hz, 1H), 4.94 - 4.83 (m, 4H), 4.23 (d, J = 11.6 Hz, 1H), 3.88 (d, J = 11.6 Hz, 1H), 3.15 - 3.08 (m, 1H), 1.71 (s, 3H), 1.40 - 1.35 (m, 2H), 0.92 - 0.88 (m, 2H) ppm |
| 167 | 453.20 | ¹H NMR (300 MHz, DMSO-*d*₆) δ 9.35 - 9.15 (m, 2H), 8.82 - 8.64 (m, 1H), 8.48 - 8.30 (m, 3H), 8.20 - 8.09 (m, 1H), 7.94 - 7.81 (m, 1H), 7.72 - 7.57 (m, 3H), 7.27 (d, *J* = 8.1 Hz, 1H), 5.02 - 4.69 (m, 4H), 4.22 (d, *J* = 11.5 Hz, 1H), 3.85 (d, *J* = 11.5 Hz, 1H), 1.68 (s, 3H). |
| 168 | 499.20 | ¹H NMR (400 MHz, DMSO-d6) δ 9.45 - 9.38 (m, 2H), 8.70 (d, J = 8.6 Hz, 1H), 8.62 (d, J = 8.6 Hz, 1H), 8.36 (d, J = 7.7 Hz, 1H), 8.28 (d, J = 1.8 Hz, 1H), 7.98 (dt, J = 8.0, 1.7 Hz, 1H), 7.89 (t, J = 7.8 Hz, 1H), 7.82 (s, 1H), 7.67 (d, J = 7.9 Hz, 1H), 7.31 (d, J = 8.1 Hz, 1H), 4.94 - 4.69 (m, 5H), 4.08 (t, J = 13.0 Hz, 1H), 3.95 - 3.78 (m, 3H), 1.70 (d, J = 20.7 Hz, 3H), 1.29 (q, J = 3.7 Hz, 2H), 1.00 (q, J = 3.7 Hz, 2H). |
| 169 | 535.20 | ¹H NMR (400 MHz, DMSO-d6) δ 9.43 (t, J = 1.4 Hz, 2H), 8.83 - 8.65 (m, 2H), 8.52 (dd, J = 7.9, 1.0 Hz, 1H), 8.29 (d, J = 1.9 Hz, 1H), 8.09 - 7.97 (m, 2H), 7.88 - 7.78 (m, 2H), 7.32 (d, J = 8.1 Hz, 1H), 6.47 (s, 1H), 4.92 - 4.69 (m, 4H), 4.09 (t, J = 13.0 Hz, 1H), 3.85 (dd, J = 25.2, 12.5 Hz, 1H), 3.42 (d, J = 13.5 Hz, 2H), 2.98 - 2.80 (m, 2H), 1.70 (d, J = 20.7 Hz, 3H). |
| 170 | 518.2 | 1H NMR (400 MHz, DMSO-d6) δ = 9.45 - 9.41 (m, 2H), 8.73 (d, J = 8.8 Hz, 1H), 8.61 (d, J = 8.8 Hz, 1H), 8.57 (d, J = 8.0 Hz, 1H), 8.40 (br s, 1H), 8.20 (d, J = 1.6 Hz, 1H), 8.00 - 7.94 (m, 2H), 7.84 (s, 1H), 7.63 - 7.62 (m, 1H), 7.32 (d, J = 8.0 Hz, 1H), 5.65 (br s, 1H), 4.93 - 4.82 (m, 4H), 4.24 (d, J = 11.6 Hz, 1H), 3.88 (d, J = 11.6 Hz, 1H), 1.70 (s, 3H), 1.52 (s, 6H) ppm |
| 171 | 506.2 | 1H NMR (400 MHz, DMSO-d6) δ = 9.40 - 9.38 (m, 2H), 8.66 (d, J = 8.4 Hz, 1H), 8.28 (d, J = 8.4 Hz, 1H), 8.19 (d, J = 1.6 Hz, 1H), 7.94 - 7.92 (m, 1H), 7.86 (s, 1H), 7.67 - 7.64 (m, 1H), 7.55 - 7.53 (m, 1H), 7.49 (d, J = 3.2 Hz, 1H), 7.30 (d, J = 8.0 Hz, 1H), 7.18 (d, J = 2.4 Hz, 1H), 4.92-4.81 (m, 4H), 4.23 - 4.20 (d, J = 11.6 Hz, 1H), 3.87 (d, J = 11.6 Hz, 1H), 3.83 (s, 3H), 1.69(s, 3H) ppm |
| 172 | 502.4 | 1H NMR (400 MHz, DMSO) δ = 9.44 - 9.34 (m, 2H), 8.65 - 8.57 (m, 1H), 8.31 - 8.27 (m, 1H), 8.26 - 8.16 (m, 2H), 7.98 -7.91 (m, 1H), 7.85 (s, 1H), 7.73 (d, J = 6.8 Hz, 1H), 7.62 - 7.54 (m, 1H), 7.34 - 7.20 (m, 2H), 7.01 - 6.95 (m, 1H), 4.93 - 4.85 (m, 2H), 4.82 (br d, J = 5.4 Hz, 2H), 4.22 (d, J = 11.2 Hz, 1H), 3.86 (d, J = 11.2 Hz, 1H), 3.76 - 3.73 (m, 3H), 2.47 - 2.45 (m, 3H), 1.72 - 1.69 (m, 3H) ppm |
| 173 | 514.2 | 1H NMR (400 MHz, DMSO-d6) δ = 9.41 - 9.38 (m, 2H), 8.62 (d, J = 8.8 Hz, 1H), 8.24 (d, J = 8.4 Hz, 1H), 8.20 (d, J = 1.2 Hz, 1H), 7.95-7.93 (m, 1H), 7.83 - 7.80 (m, 2H), 7.76 (d, J = 8.0 Hz, 1H), 7.48 (d, J = 3.6 Hz, 1H), 7.37-7.33 (m, 1H), 7.30 (d, J = 8.0 Hz, 1H), 7.16 (d, J = 3.2 Hz, 1H), 4.92 - 4.81 (m, 4H), 4.23 (d, J = 11.6 Hz, 1H), 3.87 (d, J = 11.2 Hz, 1H), 3.54-3.49 (m, 1H), 1.70 (s, 3H), 1.12 - 1.08 (m, 2H), 0.99 - 0.96 (m, 2H) ppm |
| 174 | 516.3 | 1H NMR (400 MHz, DMSO-d6) δ = 9.42 - 9.38 (m, 2H), 8.62 (d, J = 8.4 Hz, 1H), 8.41 (d, J = 2.0 Hz, 1H), 8.24 - 8.20 (m, 2H), 7.95 (d, J = 6.4 Hz, 1H), 7.83 (s, 1H), 7.77-7.71 (m, 2H), 7.65 (d, J = 3.2 Hz, 1H), 7.33 - 7.29 (m, 2H), 7.21 (d, J = 2.8 Hz, 1H), 4.93 - 4.81 (m, 5H), 4.23 (d, J = 11.2 Hz, 1H), 3.86 (d, J = 11.2 Hz, 1H), 1.70 (s, 3H), 1.49 (d, J = 6.8 Hz, 6H) ppm |
| 175 | 521.3 | 1H NMR (400 MHz, MeOD) δ = 9.35 - 9.31 (m, 1H), 8.56 - 8.48 (m, 1H), 8.22 - 8.16 (m, 1H), 8.14 - 8.06 (m, 1H), 8.00 - 7.91 (m, 2H), 7.81 - 7.76 (m, 1H), 7.34 - 7.28 (m, 1H), 7.27 - 7.19 (m, 2H), 5.19 - 5.13 (m, 2H), 4.98 - 4.95 (m, 2H), 4.94 - 4.93 (m, 2H), 4.86 - 4.83 (m, 3H), 4.44 - 4.33 (m, 1H), 4.26 - 4.18 (m, 1H), 3.95 (s, 3H), 3.94 - 3.89 (m, 1H), 1.77 (s, 3H) ppm |
| 176 | 475.20 | ¹H NMR (400 MHz, DMSO-d6) δ 9.59 (t, J = 5.9 Hz, 1H), 9.46 (s, 2H), 9.10 (t, J = 1.7 Hz, 1H), 8.79 (s, 1H), 8.75 (d, J = 8.7, 0.8 Hz, 1H), 8.62 (t, J = 1.8 Hz, 1H), 8.56 (d, J = 8.6 Hz, 1H), 7.93 (s, 1H), 4.94 - 4.78 (m, 2H), 2.41 - 2.30 (m, 1H), 1.71 (d, J = 20.9 Hz, 3H), 1.23 - 1.10 (m, 4H). |
| 177 | 502.2 | 1H NMR (400 MHz, DMSO-d6) δ = 9.47 - 9.35 (m, 2H), 8.61 (d, J = 8.4 Hz, 1H), 8.42 (br s, 1H), 8.17 (s, 1H), 7.92 (br d, J = 7.4 Hz, 1H), 7.86 - 7.76 (m, 2H), 7.54 (d, J = 8.0 Hz, 1H), 7.32 - 7.22 (m, 2H), 7.19 - 7.11 (m, 2H), 4.93 - 4.76 (m, 4H), 4.21 (d, J = 11.2 Hz, 1H), 3.85 (br d, J = 11.6 Hz, 1H), 3.78 (s, 3H), 1.82 (s, 3H), 1.68 (s, 3H) ppm |
| 178 | 502.3 | 1H NMR (400 MHz, DMSO-d6) δ = 9.47 - 9.32 (m, 2H), 8.62 (d, J = 8.8 Hz, 1H), 8.24 (d, J = 8.7 Hz, 1H), 8.20 (d, J = 1.2 Hz, 1H), 7.95 (d, J = 8.0 Hz, 1H), 7.84 (s, 1H), 7.78 (d, J = 7.6 Hz, 1H), 7.69 (d, J = 8.4 Hz, 1H), 7.55 (d, J = 3.2 Hz, 1H), 7.36 - 7.26 (m, 2H), 7.19 (d, J = 3.2 Hz, 1H), 4.98 - 4.76 (m, 4H), 4.36 - 4.16 (m, 3H), 3.87 (d, J = 11.2 Hz, 1H), 1.70 (s, 3H), 1.41 - 1.37 (m, 3H) ppm |
| 179 | 488.3 | 1H NMR (400 MHz, DMSO-d6) δ = 9.46 - 9.32 (m, 2H), 8.62 (d, J = 8.8 Hz, 1H), 8.25 (d, J = 8.8 Hz, 1H), 8.20 (d, J = 1.2 Hz, 1H), 7.95 (d, J = 1.6 Hz, 1H), 7.85 (s, 1H), 7.80 (d, J = 7.2 Hz, 1H), 7.65 (d, J = 8.4 Hz, 1H), 7.48 (d, J = 3.2 Hz, 1H), 7.38 - 7.27 (m, 2H), 7.20 (d, J = 2.8 Hz, 1H), 4.96 - 4.76 (m, 4H), 4.23 (d, J = 11.6 Hz, 1H), 3.90 - 3.83 (m, 4H), 1.70 (s, 3H) ppm |
| 180 | 517.3 | 1H NMR (400 MHz, DMSO-d6) δ= 9.57 - 9.36 (m, 2H), 8.89 - 8.73 (m, 2H), 8.53 (d, J = 8.0 Hz, 1H), 8.48 - 8.43 (m, 1H), 8.41 - 8.33 (m, 1H), 8.22 (s, 1H), 8.10 - 8.01 (m, 1H), 8.00 - 7.94 (m, 1H), 7.88 (s, 1H), 7.32 (d, J = 8.4 Hz, 1H), 6.79 (d, J = 9.6 Hz, 1H), 4.98 - 4.77 (m, 4H), 4.23 (d, J = 11.6 Hz, 1H), 3.89 - 3.83 (m, 4H), 1.71 (s, 3H) ppm |
| 181 | 477.2 | 1H NMR (400 MHz, DMSO-d6) δ= 9.55-9.52 (m, 1H), 9.36 (s, 1H), 9.16 (s, 1H), 9.08 (d, J = 2.0 Hz, 1H), 8.72 (s, 1H),8.65 (s, 1H), 8.55 (d, J = 2.0 Hz, 1H), 8.36-8.33 (m, 1H), 8.25 (d, J = 8.8 Hz, 1H), 7.89 (s, 1H), 4.91 - 4.85 (m, 2H), 4.80(d, J = 6 Hz, 2H), 4.29 (d, J = 11.6 Hz, 1H), 3.93 (d, J = 11.6 Hz, 1H), 2.31 - 2.27 (m, 1H), 1.71 (s, 3H), 1.16 - 1.10 (m,4H) ppm. |
| 182 | 489.2 | 1H NMR (400 MHz, DMSO) δ = 9.45 - 9.34 (m, 2H), 8.87 - 8.82 (m, 1H), 8.72 - 8.64 (m, 1H), 8.39 (d, J = 8.8 Hz, 1H), 8.20 (d, J = 1.6 Hz, 1H), 8.02 (d, J = 7.6 Hz, 1H), 7.97 - 7.93 (m, 2H), 7.91 - 7.81 (m, 1H), 7.63 - 7.53 (m, 1H), 7.34 - 7.26 (m, 1H), 4.95 - 4.80 (m, 4H), 4.27 (s, 1H), 4.15 - 4.11 (m, 3H), 3.87 (d, J = 11.6 Hz, 1H), 1.73 - 1.67 (m, 3H) ppm |
| 184 | 505.3 | 1H NMR (400 MHz, DMSO-d6) δ = 9.38-9.35 (m, 1H), 9.33 (s, 1H), 8.60 (d, J = 8.8 Hz, 1H), 8.42 (s, 1H), 8.18 (d, J = 1.2Hz, 1H), 8.00 - 7.68 (m, 4H), 7.30 (d, J = 8.0 Hz, 1H), 4.92 - 4.78 (m, 4H), 4.22 (d, J = 11.6 Hz, 1H), 4.15 (s, 3H), 3.87 (d, J = 11.6 Hz, 1H), 1.69 (s, 3H) ppm |
| 185 | 527.3 | 1H NMR (400 MHz, DMSO-d6) δ = 9.39 - 9.36 (m, 1H), 9.21 (s, 1H), 8.46 - 8.45 (m, 2H), 8.43 (s, 1H), 8.19 (d, J = 1.6 Hz, 1H), 8.00 (d, J = 8.8 Hz, 1H), 7.95-7.92 (m, 1H), 7.57 (s, 1H), 7.31 (d, J = 8.0 Hz, 1H), 5.49 - 5.29 (m, 1H), 5.05 - 4.98 (m, 1H), 4.92 - 4.81 (m, 2H), 4.75 (br d, J = 4.8 Hz, 2H), 4.22 (d, J = 11.2 Hz, 1H), 4.00 (s, 3H), 3.86 (d, J = 11.2 Hz, 1H), 2.86 - 2.75 (m, 2H), 2.73 - 2.68 (m, 2H), 1.69 (s, 3H) ppm |
| 186 | 521.2 | 1H NMR (400 MHz, DMSO-d6) δ = 9.36-9.33 (m, 1H), 9.25 (s, 1H), 8.66 (s, 1H), 8.50 (d, J = 8.4 Hz, 1H), 8.17 (d, J = 1.6 Hz, 1H), 8.00 (d, J = 8.8 Hz, 1H), 7.92-7.90 (m, 1H), 7.68 (s, 1H), 7.29 (d, J = 8.0 Hz, 1H), 5.55-5.48 (m, 1H), 4.95 - 4.85 (m, 6H), 4.81 - 4.75 (m, 2H), 4.22 (d, J = 11.6 Hz, 1H), 3.86 (d, J = 11.2 Hz, 1H), 3.00 - 2.92 (m, 1H), 1.69 (s, 3H), 0.97 - 0.89 (m, 4H) ppm |
| 187 | 495.4 | 1H NMR (400 MHz, DMSO-d6) δ= 9.40 - 9.31 (m, 1H), 9.26 (s, 1H), 8.49 (d, J = 8.8 Hz, 1H), 8.35 (s, 1H), 8.17 (d, J = 1.6 Hz, 1H), 7.99 (d, J = 8.8 Hz, 1H), 7.92 (d, J = 8.0 Hz, 1H), 7.69 (s, 1H), 7.30 (d, J = 8.4 Hz, 1H), 5.81 - 5.63 (m, 1H), 5.08 - 4.69 (m, 8H), 4.31 - 4.12 (m, 1H), 3.86 (d, J = 11.6 Hz, 1H), 2.74 - 2.67 (m, 3H), 1.69 (s, 3H) ppm |
| 188 | 495.4 | 1H NMR (400 MHz, DMSO-d6) δ= 9.39 - 9.36 (m, 1H), 9.26 (s, 1H), 8.71 (s, 1H), 8.50 (d, J = 8.8 Hz, 1H), 8.18 (d, J = 1.6 Hz, 1H), 7.93 (d, J = 9.2 Hz, 2H), 7.70 (s, 1H), 7.31 (d, J = 8.4 Hz, 1H), 5.65 - 5.47 (m, 1H), 4.93 (d, J = 7.2 Hz, 4H), 4.90 - 4.72 (m, 4H), 4.23 (d, J = 11.6 Hz, 1H), 3.87 (d, J = 11.6 Hz, 1H), 2.64 (s, 3H), 1.70 (s, 3H) ppm |
| 189 | 478.20 | ¹H NMR (300 MHz, DMSO-d6) δ 9.79 (t, J = 5.7 Hz, 1H), 9.14 (d, J = 2.0 Hz, 1H), 9.08 (dd, J = 8.9, 1.0 Hz, 1H), 8.96 (d, J = 9.0 Hz, 1H), 8.63 (d, J = 2.0 Hz, 1H), 8.45 (d, J = 7.4 Hz, 1H), 8.34 (s, 1H), 7.98 (t, J = 7.8 Hz, 1H), 7.61 (d, J = 7.6 Hz, 1H), 5.23 (d, J = 5.7 Hz, 2H), 5.01 - 4.84 (m, 2H), 4.36 (d, J = 11.5 Hz, 1H), 4.00 (d, J = 11.6 Hz, 1H), 2.36 - 2.28 (m, 1H), 1.79 (s, 3H), 1.24 - 1.05 (m, 4H). |
| 192 | 499.2 | 1H NMR (400 MHz, DMSO-d6) δ = 9.57-9.54 (m, 1H), 9.31 (s, 1H), 9.09 (s, 1H), 8.84 (s, 1H), 8.62 - 8.57 (m, 2H), 8.07 (d, J = 8.8 Hz, 1H), 7.83 - 7.53 (m, 2H), 4.86 - 4.73 (m, 4H), 4.15 - 4.06 (m, 4H), 3.96 - 3.86 (m, 1H), 1.71 (d, J = 20.8 Hz ,3H) ppm |
| 193 | 505.2 | 1H NMR (400 MHz, DMSO-d6) δ= 9.42-9.38 (m, 1H), 9.31 (s, 1H), 8.85 (s, 1H), 8.58 (d, J = 8.8 Hz, 1H), 8.20 (d, J =1.6 Hz, 1H), 8.06 (d, J = 8.8 Hz, 1H), 7.96-7.94 (m, 1H), 7.81 - 7.52 (m, 2H), 7.31 (d, J = 8.4 Hz, 1H), 4.93 - 4.77(m, 4H), 4.23 (d, J = 11.2 Hz, 1H), 4.08 - 4.05 (m, 3H), 3.88-3.85 (m, 1H), 1.70 (s, 3H) ppm |
| 194 | 541.2 | 1H NMR (400 MHz, DMSO-d6) δ = 9.40 - 9.38 (m, 2H), 8.68 (d, J = 8.8 Hz, 1H), 8.33 (d, J = 8.8 Hz, 1H), 8.28 (d, J = 8.0 Hz, 1H), 8.20 (d, J = 1.2 Hz, 1H), 8.10 (s, 1H), 7.96-7.93 (m, 1H), 7.83 (s, 1H), 7.63-7.59 (m, 1H), 7.40 (d, J = 7.6 Hz, 1H), 7.31 (d, J = 8.0 Hz, 1H), 6.70 - 6.42 (m, 1H), 4.99 - 4.86 (m, 6H), 4.82 (br d, J = 4.4 Hz, 2H), 4.23 (d, J = 11.6 Hz, 1H), 3.87 (d, J = 11.2 Hz, 1H), 1.70 (s, 3H) ppm |
| 195 | 469.3 | 1H NMR (400 MHz, MeOD-d4) δ = 9.18 (s, 1H), 8.49 (d, J = 9.2 Hz, 1H), 8.21 - 8.13 (m, 2H), 7.91 - 7.89 (m, 1H), 7.76 (s, 1H), 7.28 (d, J = 8.0 Hz, 1H), 5.84 (s, 1H), 4.91 - 4.90 (m, 4H), 4.20 (d, J = 11.6 Hz, 1H), 3.98 - 3.89 (m, 4H), 2.79 (s, 3H), 1.76 (s, 3H) ppm |
| 196 | 517 | 1H NMR (400 MHz, DMSO-d6) δ = 9.55 - 9.35 (m, 2H), 8.94 - 8.70 (m, 3H), 8.30 (d, J = 8.4 Hz, 1H), 8.22 (s, 1H), 7.98 - 7.95 (m, 1H), 7.87 (s, 1H), 7.69 (d, J = 7.2 Hz, 1H), 7.32 (d, J = 8.0 Hz, 1H), 6.71 (d, J = 7.2 Hz, 1H), 4.94 - 4.84 (m, 4H), 4.23 (d, J = 11.2 Hz, 1H), 3.87 (d, J = 11.2 Hz, 1H), 3.59 (s, 3H), 1.71 (s, 3H) ppm |
| 197 | 525.3 | 1H NMR (400 MHz, DMSO-d6) δ = 9.40-9.37 (m, 1H), 9.22 (s, 1H), 8.47 (d, J = 8.0 Hz, 1H), 8.41 (s, 1H), 8.21 (d, J = 1.6 Hz, 1H), 8.02 (d, J = 8.8 Hz, 1H), 7.97-7.94 (m, 1H), 7.60 (s, 1H), 7.32 (d, J = 8.0 Hz, 1H), 4.96 - 4.82 (m, 3H), 4.76 (br d, J = 5.6 Hz, 2H), 4.24 (d, J = 11.6 Hz, 1H), 4.02 - 3.96 (m, 4H), 3.94 (d, J = 5.2 Hz, 2H), 3.88 (d, J = 11.6 Hz, 1H), 3.82 - 3.77 (m, 1H), 2.39 - 2.33 (m, 2H), 1.70 (s, 3H) ppm |
| 198 | 477.15 | ¹H NMR (300 MHz, Chloroform-*d*) δ 9.12 (s, 1H), 8.92 (d, J = 8.3 Hz, 1H), 8.58 - 8.27 (m, 3H), 8.22 - 7.93 (m, 3H), 7.56 (t, *J* = 7.7 Hz, 1H), 7.45 - 7.38 (m, 1H), 5.37 (s, 2H), 4.92 (s, 2H), 4.25 (d, *J* = 11.3 Hz, 1H), 4.05 (d, *J* = 11.0 Hz, 1H), 2.14 (s, 1H), 1.35 (s, 3H), 1.16 (d, *J* = 7.9 Hz, 2H), 1.01 - 0.85 (m, 2H). |
| 199 | 476.15 | ¹H NMR (300 MHz, DMSO-*d*₆) δ 9.59 - 9.21 (m, 2H), 8.93 (d, *J* = 5.1 Hz, 1H), 8.25 (s, 1H), 8.14 - 7.85 (m, 3H), 7.30 (d, *J* = 8.1 Hz, 1H), 5.02 - 4.57 (m, 4H), 4.08 (t, *J* = 13.0 Hz, 1H), 3.84 (dd, J = 25.2, 12.5 Hz, 1H), 2.42 - 2.20 (m, 1H), 1.69 (d, *J* = 20.7 Hz, 3H), 1.35 - 1.01 (m, 4H). |
| 200 | 509.2 | 1H NMR (400 MHz, DMSO-d6) δ = 9.42 - 9.38 (m, 1H), 9.21 (s, 1H), 8.47 - 8.42 (m, 2H), 8.40 (br s, 1H), 8.20 (d, J = 1.2 Hz, 1H), 8.01 (d, J = 8.8 Hz, 1H), 7.96 - 7.94 (m, 1H), 7.58 (s, 1H), 7.33 - 7.30 (m, 1H), 4.93 - 4.82 (m, 2H), 4.76 - 4.71 (m, 3H), 4.23 (d, J = 11.2 Hz, 1H), 4.02 - 3.98 (m, 3H), 3.87 (d, J = 11.6 Hz, 1H), 2.46 - 2.42 (m, 2H), 2.36 - 2.33 (m, 2H), 1.79 - 1.67 (m, 5H) ppm |
| 201 | 504.2 | 1H NMR (400 MHz, DMSO) δ = 9.43 - 9.34 (m, 1H), 9.23 (s, 1H), 8.55 - 8.43 (m, 2H), 8.27 (s, 1H), 8.06 - 7.93 (m, 2H), 7.57 (s, 1H), 7.31 (d, J = 8.0 Hz, 1H), 5.56 - 5.44 (m, 1H), 4.95 - 4.83 (m, 5H), 4.80 - 4.70 (m, 3H), 4.13 - 4.04 (m, 4H), 3.90 - 3.82 (m, 1H), 1.76 - 1.61 (m, 3H) ppm |
| 202 | 519.3 | 1H NMR (400 MHz, DMSO-d6) δ= 9.40 - 9.37 (m, 1H), 9.24 (s, 1H), 8.55 - 8.42 (m, 2H), 8.20 (d, J = 1.2 Hz, 1H), 8.05 - 7.92 (m, 2H), 7.60 (s, 1H), 7.31 (d, J = 8.0 Hz, 1H), 6.61 - 6.17 (m, 1H), 4.95 - 4.81 (m, 2H), 4.76 (d, J = 5.6 Hz, 2H), 4.56 - 4.54 (m, 2H), 4.23 (d, J = 11.6 Hz, 1H), 4.00 (s, 3H), 3.86 (d, J = 11.6 Hz, 1H), 1.70 (s, 3H) ppm |
| 203 | 513.3 | 1H NMR (400 MHz, DMSO-d6) δ = 9.37 (s, 1H), 9.21 (s, 1H), 8.46 (d, J = 8.8 Hz, 1H), 8.35 (s, 1H), 8.20 (s, 1H), 8.00 (d, J = 8.8 Hz, 1H), 7.95 (br d, J = 7.2 Hz, 1H), 7.59 (s, 1H), 7.31 (d, J = 8.0 Hz, 1H), 4.93 - 4.82 (m, 2H), 4.75 (d, J = 5.6 Hz, 2H), 4.24 - 4.16 (m, 3H), 3.98 (s, 3H), 3.86 (d, J = 11.6 Hz, 1H), 3.69 - 3.67 (m, 2H), 3.24 (s, 3H), 1.70 (s, 3H) ppm |
| 204 | 495.3 | 1H NMR (400 MHz, MeOD-d4) δ = 9.40 (s, 1H), 8.94 (d, J = 5.2 Hz, 1H), 8.70 (s, 2H), 8.50 (d, J = 5.2 Hz, 1H), 8.18 (d, J = 1.6 Hz, 1H), 8.02 (s, 1H), 7.97 - 7.90 (m, 1H), 7.30 (d, J = 8.0 Hz, 1H), 4.94 (s, 2H), 4.92 (d, J = 3.6 Hz, 2H), 4.21 (d, J = 11.2 Hz, 1H), 3.93 (d, J = 11.6 Hz, 1H), 1.77 (s, 3H), 1.68 (s, 2H), 1.66 - 1.61 (m, 2H) ppm |
| 205 | 501.29 | ¹H NMR (300 MHz, DMSO-*d*₆) δ 9.49 (d, *J* = 4.0 Hz, 2H), 8.79 (s, 2H), 8.51 (d, *J* = 7.7 Hz, 1H), 8.36 (s, 1H), 8.04 (t, *J* = 8.0 Hz, 2H), 7.95 - 7.80 (m, 2H), 7.39 (d, *J* = 8.0 Hz, 1H), 5.25 (s, 1H), 5.05 - 4.67 (m, 4H), 4.16 (t, *J* = 13.0 Hz, 1H), 3.92 (dd, J = 25.1, 12.5 Hz, 1H), 1.97 (dt, J = 33.8, 6.6 Hz, 2H), 1.78 (d, *J* = 20.7 Hz, 3H), 1.62 (s, 3H), 0.81 (t, *J* = 7.3 Hz, 3H). |
| 206 | 477.25 | ¹H NMR (400 MHz, DMSO-*d*₆) δ 9.66 (s, 1H), 9.35 (t, 1H), 9.25 (s, 1H), 8.70 (s, 1H), 8.65 (s, 1H), 8.45 (d, *J* = 8.5 Hz, 1H), 8.29 (d, *J* = 8.6 Hz, 1H), 8.19 (s, 1H), 7.92 (d, *J* = 8.0 Hz, 1H), 7.30 (d, *J* = 8.1 Hz, 1H), 4.99 - 4.79 (m, 4H), 4.23 (d, *J* = 11.5 Hz, 1H), 3.87 (d, *J* = 11.5 Hz, 1H), 2.38 - 2.27 (m, 1H), 1.71 (s, 3H), 1.18 - 1.08 (m, 4H). |
| 208 | 477.3 | 1H NMR (400 MHz, DMSO-d6) δ = 9.57 (s, 1H), 9.42 (s, 1H), 9.08 (d, J = 2.0 Hz, 1H), 8.71 - 8.66 (m, 1H), 8.64 - 8.60 (m, 1H), 8.57 (d, J = 2.0 Hz, 1H), 8.34 (d, J = 8.0 Hz, 1H), 7.91 - 7.82 (m, 2H), 7.47 (d, J = 8.0 Hz, 1H), 4.93 - 4.80 (m, 4H), 4.29 (d, J = 11.6 Hz, 1H), 3.93 (d, J = 11.6 Hz, 1H), 2.24 (s, 1H), 1.72 (s, 3H), 1.10 - 1.08 (m, 2H), 1.06 - 1.03 (m, 2H) ppm |
| 210 | 492.2 | 1H NMR (400 MHz, DMSO-d6) δ= 9.58 - 9.55 (m, 1H), 9.40 (s, 1H), 9.08 (d, J = 2.0 Hz, 1H), 8.71 - 8.54 (m, 3H), 7.93 - 7.84 (m, 2H), 7.73 - 7.69 (m, 1H), 6.53 (d, J = 8.0 Hz, 1H), 4.94 - 4.81 (m, 4H), 4.30 (d, J = 11.6 Hz, 1H), 4.09 - 4.05 (m, 4H), 3.93 (d, J = 11.6 Hz, 1H), 2.43 - 2.35 (m, 2H), 1.73 (s, 3H) ppm |
| 211 | 473.15 | 1H NMR (300 MHz, Chloroform-d) δ 9.70 (s, 1H), 9.39 (s, 1H), 9.12 (dd, J = 2.2, 1.2 Hz, 1H), 8.73 - 8.67 (m, 2H), 8.60 - 8.46 (m, 2H), 8.13 (s, 1H), 7.63 (t, J = 4.8 Hz, 1H), 5.11 (d, J = 4.9 Hz, 2H), 4.18 (t, J = 11.4 Hz, 1H), 4.00 (dd, J = 15.8, 12.1 Hz, 1H), 2.35 - 2.21 (m, 1H), 1.85 (d, J = 21.0 Hz, 3H), 1.40 - 1.30 (m, 2H), 1.30 - 1.20 (m, 2H). |
| 212 | 473.15 | ¹H NMR (400 MHz, Chloroform-d) δ 9.43 (s, 1H), 8.96 - 8.64 (m, 3H), 8.54 (d, J = 8.8 Hz, 1H), 8.43 (s, 1H), 8.17 (d, J = 1.8 Hz, 1H), 8.04 - 7.81 (m, 2H), 7.51 (d, J = 7.8 Hz, 1H), 7.13 (d, J = 8.1 Hz, 1H), 5.09 - 4.73 (m, 5H), 4.08 (t, J = 11.9 Hz, 1H), 3.82 (dd, J = 19.0, 12.1 Hz, 1H), 1.73 (d, J = 20.8 Hz, 3H), 1.62 (d, J = 6.5 Hz, 3H). |
| 213 | 473.15 | ¹H NMR (400 MHz, Chloroform-d) δ 9.44 (s, 1H), 9.05 - 8.90 (m, 2H), 8.68 (d, J = 7.8 Hz, 1H), 8.60 - 8.42 (m, 2H), 8.18 (s, 1H), 8.02 - 7.82 (m, 2H), 7.52 (d, J = 7.7 Hz, 1H), 7.13 (d, J = 8.1 Hz, 1H), 5.13 - 4.98 (m, 3H), 4.91 - 4.71 (m, 2H), 4.08 (t, J = 12.0 Hz, 1H), 3.82 (dd, J = 19.2, 12.1 Hz, 1H), 1.78 - 1.58 (m, 6H). |
| 217 | 513 | 1H NMR (400 MHz, MeOD) δ = 9.33 - 9.25 (m, 1H), 8.85 - 8.78 (m, 1H), 8.70 - 8.53 (m, 2H), 8.44 - 8.33 (m, 1H), 8.10 - 8.06 (m, 1H), 7.93 - 7.87 (m, 1H), 7.85 - 7.80 (m, 1H), 7.27 - 7.13 (m, 1H), 4.84 (s, 2H), 4.83 - 4.80 (m, 2H), 4.15 - 4.06 (m, 1H), 3.87 - 3.75 (m, 1H), 3.19 - 3.12 (m, 1H), 2.54 - 2.35 (m, 1H), 2.07 - 1.84 (m, 1H), 1.66 (s, 3H) ppm |
| 218 | 513 | 1H NMR (400 MHz, MeOD) δ = 9.35 - 9.25 (m, 1H), 8.87 - 8.78 (m, 1H), 8.69 - 8.56 (m, 2H), 8.46 - 8.32 (m, 1H), 8.14 - 8.04 (m, 1H), 7.94 - 7.89 (m, 1H), 7.86 - 7.81 (m, 1H), 7.23 - 7.17 (m, 1H), 4.84 (s, 2H), 4.81 (d, J = 3.2 Hz, 2H), 4.15 - 4.07 (m, 1H), 3.87 - 3.81 (m, 1H), 3.20 - 3.12 (m, 1H), 2.53 - 2.37 (m, 1H), 2.08 - 1.90 (m, 1H), 1.66 (s, 3H) ppm |
| 221 | 478.3 | 1H NMR (400 MHz, DMSO-d6) δ = 9.67 - 9.54 (m, 1H), 9.46 (s, 2H), 9.08 (d, J = 2.0 Hz, 1H), 8.84 - 8.70 (m, 2H), 8.60 - 8.50 (m, 2H), 7.93 (s, 1H), 4.96 - 4.76 (m, 4H), 4.29 (d, J = 11.6 Hz, 1H), 3.93 (d, J = 11.6 Hz, 1H), 2.38 - 2.28 (m, 1H), 1.72 (s, 3H), 1.20 - 1.02 (m, 4H) ppm |
| 222 | 473.15 | ¹H NMR (300 MHz, Chloroform-*d*) δ 9.63 (s, 1H), 9.37 - 9.29 (m, 1H), 9.07 - 9.03 (m, 1H), 8.65 (t, *J* = 4.3 Hz, 2H), 8.47 - 8.42 (m, 2H), 8.10 (s, 1H), 7.72 - 7.63 (m, 1H), 5.01 (dd, J = 24.4, 4.0 Hz, 2H), 4.88 (m, 2H), 2.27 - 2.17 (m, 1H), 1.84 - 1.75 (m, 3H), 1.28 - 1.23 (m, 2H), 1.22 - 1.15 (m, 2H). |
| 223 | 480.20 | ¹H NMR (400 MHz, DMSO-*d*₆) δ 9.54 (t, *J* = 5.7 Hz, 1H), 9.00 - 8.89 (m, 2H), 8.21 - 8.13 (m, 2H), 7.99 - 7.92 (m, 1H), 7.89 - 7.82 (m, 1H), 7.78 - 7.70 (m, 1H), 7.30 (d, *J* = 8.1 Hz, 1H), 6.88 (d, *J* = 8.4 Hz, 1H), 5.12 (d, *J* = 5.7 Hz, 2H), 4.95 - 4.78 (m, 2H), 4.22 (d, *J* = 11.5 Hz, 1H), 3.86 (d, *J* = 11.5 Hz, 1H), 3.17 (s, 6H), 1.70 (s, 3H). |
| 224 | 509.3 | 1H NMR (400 MHz, MeOD) δ = 9.29 (s, 1H), 9.16 - 9.15 (m, 1H), 8.71 (d, J = 8.8 Hz, 1H), 8.62 (s, 1H), 8.57 - 8.55 (m, 1H), 7.97 (s, 1H), 7.82 (d, J = 7.2 Hz, 1H), 7.67 - 7.65 (m, 1H), 6.79 (d, J = 8.4 Hz, 1H), 6.56 - 6.27 (m, 1H), 4.93 (s, 2H), 4.89 - 4.88 (m, 2H), 4.40 - 4.33(m, 1H), 4.16 - 4.11 (m, 1H), 3.19 (s, 6H) ppm |
| 225 | 495.5 | 1H NMR (400 MHz, MeOD) δ = 9.15 (s, 1H), 8.44 - 8.36 (m, 1H), 8.29 - 8.25 (m, 1H), 8.21 - 8.11 (m, 2H), 7.97 - 7.90 (m, 1H), 7.81 - 7.75 (m, 1H), 7.34 - 7.25 (m, 1H), 4.92 (br d, J = 3.6 Hz, 4H), 4.24 - 4.18 (m, 1H), 4.05 (s, 3H), 3.96 - 3.92 (m, 1H), 3.64 - 3.57 (m, 1H), 1.81 - 1.73 (m, 3H), 1.15 - 1.07 (m, 2H), 1.05 - 1.00 (m, 2H) ppm |
| 226 | 526.20 | ¹H NMR (300 MHz, Chloroform-d) δ 9.28 (d, J = 2.5 Hz, 1H), 8.79 - 8.72 (m, 1H), 8.38 (d, *J* = 8.7 Hz, 1H), 8.08 - 7.90 (m, 3H), 7.76 - 7.65 (m, 2H), 7.63 - 7.55 (m, 1H), 6.73 - 6.68 (m, 1H), 6.38 - 5.95 (m, 1H), 5.04 - 4.82 (m, 4H), 4.37 (t, *J* = 11.7 Hz, 1H), 4.02 (t, *J* = 11.4 Hz, 1H), 3.32 - 3.11 (m, 6H). |
| 227 | 509.3 | 1H NMR (400 MHz, MeOH) δ = 9.28 (s, 1H), 9.15 - 9.14 (m, 1H), 8.70 - 8.67 (m, 1H), 8.62 (s, 1H), 8.56 - 8.54 (m, 1H), 7.96 (s,1H), 7.81 (d, J = 7.6 Hz, 1H), 7.69 - 7.65 (m, 1H), 6.78 (d, J = 8.4 Hz, 1H), 6.56 - 6.27 (m, 1H), 4.92 (s, 2H), 4.91 - 4.89 (m, 2H), 4.40 - 4.33 (m, 1H), 4.16 - 4.10 (m, 1H), 3.18 (s, 6H) ppm |
| 228 | 507.3 | 1H NMR (400 MHz, MeOH) δ = 9.46 (s, 1H), 9.34 (s, 1H), 9.15 - 9.14 (m, 1H), 8.64 - 8.58 (m, 4H), 8.01 (s, 1H), 6.55 - 6.26 (m, 1H), 4.94 (s, 2H), 4.88 (br s, 2H), 4.40 - 4.33 (m, 1H), 4.16 - 4.10 (m, 1H), 2.33 - 2.28 (m, 1H), 1.23 - 1.21 (m, 2H), 1.19 - 1.17 (m, 2H) ppm |
| 229 | 507.3 | 1H NMR (400 MHz, MeOD) δ = 9.47 (s, 1H), 9.34 (s, 1H), 9.15 - 9.14 (m, 1H), 8.65 - 8.58 (m, 4H), 8.02 (s, 1H), 6.55 - 6.26 (m, 1H), 4.94 (s, 2H), 4.89 (br s, 2H), 4.40 - 4.33 (m, 1H), 4.16 - 4.13 (m, 1H), 2.32 - 2.27 (m, 1H), 1.23 - 1.17 (m, 4H) ppm |
| 231 | 440.3 | 1H NMR (400 MHz, DMSO-d6) δ = 9.45 (s, 1H), 9.42 - 9.39 (m, 1H), 8.77 - 8.75 (m, 1H), 8.19 - 8.17 (m, 2H), 7.96 - 7.93 (m, 1H), 7.81 (s, 1H), 7.31 - 7.29 (m, 2H), 4.93 - 4.81 (m, 4H), 4.23 (d, J = 11.6 Hz, 1H), 3.87 (d, J = 11.6 Hz, 1H), 2.35 (s, 3H), 1.70 (s, 3H) ppm |
| 232 | 501.3 | 1H NMR (400 MHz, DMSO-d6) δ = 9.53 (s, 1H), 9.46 - 9.43 (m, 1H), 9.18 (d, J = 5.2 Hz, 1H), 8.85 (d, J = 8.4 Hz, 1H), 8.74 - 8.66 (m, 2H), 8.21 (d, J = 1.2 Hz, 1H), 8.00 - 7.87 (m, 2H), 7.32 (d, J = 8.0 Hz, 1H), 4.97 - 4.80 (m, 4H), 4.23 (d, J = 11.2 Hz, 1H), 3.87 (d, J = 11.6 Hz, 1H), 2.21 - 2.11 (m, 3H), 1.71 (s, 3H) ppm |
| 233 | 486.4 | 1H NMR (400 MHz, MeOD) δ = 9.32 (s, 1H), 8.64 - 8.53 (m, 2H), 8.41 - 8.35 (m, 1H), 8.24 (s, 1H), 7.98 - 7.88 (m, 2H), 7.28 (d, J = 7.6 Hz, 1H), 7.16 - 7.06 (m, 1H), 4.95 - 4.90 (m, 3H), 4.83 - 4.76 (m, 1H), 4.28 - 4.20 (m, 1H), 4.12 - 4.03 (m, 1H), 3.94 - 3.83 (m, 1H), 1.80 - 1.66 (m, 3H), 1.34 - 1.29 (m, 2H), 1.19 - 1.11 (m, 2H) ppm |
| 235 | 515.3 | 1H NMR (400 MHz, DMSO-d6) δ = 9.45 (d, J = 0.8 Hz, 1H), 9.40 - 9.32 (m, 1H), 8.17 (d, J = 1.6 Hz, 1H), 8.07 (d, J = 5.2 Hz, 1H), 8.02 (s, 1H), 7.93 - 7.91 (m, 1H), 7.48 - 7.40 (m, 1H), 7.29 (d, J = 8.0 Hz, 1H), 4.95 - 4.74 (m, 4H), 4.26 - 4.14 (m, 5H), 3.86 (d, J = 11.6 Hz, 1H), 2.44 - 2.35 (m, 2H), 1.69 (s, 3H) ppm |
| 236 | 511.1 | 1H NMR (400 MHz, MeOD) δ = 9.16 (s, 1H), 8.45 - 8.39 (m, 1H), 8.34 (s, 1H), 8.20 - 8.13 (m, 2H), 7.95 - 7.90 (m, 1H), 7.77 (s, 1H), 7.29 (d, J = 8.0 Hz, 1H), 5.52 - 5.43 (m, 1H), 5.14 - 5.08 (m, 2H), 5.05 - 4.98 (m, 2H), 4.92 (d, J = 3.6 Hz, 2H), 4.60 (s, 2H), 4.23 - 4.19 (m, 1H), 4.13 (s, 3H), 3.95 - 3.90 (m, 1H), 1.77 (s, 3H) ppm |
| 237 | 524.30 | ¹H NMR (300 MHz, Chloroform-d) δ 9.64 (s, 1H), 9.34 (s, 1H), 8.69 - 8.63 (m, 2H), 8.45 (d, *J* = 8.4 Hz, 1H), 8.11 (s, 1H), 7.95 (s, 1H), 7.77 - 7.66 (m, 2H), 6.19 (td, J = 54.0, 8.9 Hz, 1H), 5.09 - 5.03 (d, *J* = 5.2 Hz, 2H), 4.93 - 4.88 (m, 2H), 4.45 - 4.31 (t, *J* = 11.5 Hz, 1H), 4.11 - 3.96 (t, *J =* 13.0 Hz, 1H), 2.29 - 2.18 (m, 1H), 1.28 - 1.18 (m, 4H). |
| 238 | 473.30 | ¹H NMR (400 MHz, DMSO-*d*₆) δ 9.53 (t, J = 5.8 Hz, 1H), 9.00 - 8.95 (m, 1H), 8.91 (d, *J* = 9.0 Hz, 1H), 8.30 - 8.24 (m, 1H), 8.15 (d, *J* = 1.0 Hz, 1H), 8.00 - 7.93 (m, 1H), 7.86 (d, *J* = 7.3 Hz, 1H), 7.77 - 7.68 (m, 1H), 7.30 (d, *J* = 8.1 Hz, 1H), 6.90 - 6.85 (m, 1H), 5.20 - 5.05 (m, 2H), 4.90 - 4.80 (m, 1H), 4.78 - 4.67 (m, 1H), 4.07 (t, *J* = 13.0 Hz, 1H), 3.91 - 3.76 (m, 1H), 3.17 (s, 6H), 1.69 (d, *J* = 20.7 Hz, 3H). |
| 239 | 513.20 | ¹H NMR (300 MHz, DMSO-d6) δ 9.62 (t, J = 5.8 Hz, 1H), 9.11 (dd, J = 9.0, 1.0 Hz, 1H), 8.98 (d, J = 9.0 Hz, 1H), 8.58 (d, J = 8.3 Hz, 1H), 8.45 (d, J = 7.5 Hz, 1H), 8.34 (d, J = 2.0 Hz, 1H), 8.27 (s, 1H), 8.13 (t, J = 8.0 Hz, 1H), 8.04 (dd, J = 7.9, 1.8 Hz, 1H), 7.38 (d, J = 8.2 Hz, 1H), 5.28 - 5.14 (m, 2H), 5.00 - 4.74 (m, 2H), 4.32 (t, J = 7.1 Hz, 2H), 4.15 (t, J = 13.0 Hz, 1H), 3.91 (dd, J = 25.1, 12.5 Hz, 1H), 2.73 (t, J = 8.0 Hz, 2H), 2.21 (p, J = 7.7 Hz, 2H), 1.76 (d, J = 20.7 Hz, 3H). |
| 240 | 470.15 | ¹H NMR (400 MHz, Chloroform-d) δ 8.96 (d, J = 9.0 Hz, 1H), 8.84 (dd, J = 9.1, 1.0 Hz, 1H), 8.42 (dd, J = 7.8, 1.0 Hz, 1H), 8.21 (s, 1H), 8.12 (s, 1H), 7.83 (dt, J = 8.1, 1.6 Hz, 1H), 7.75 (t, J = 7.8 Hz, 1H), 7.57 (d, J = 5.8 Hz, 1H), 7.32 (dd, J = 7.8, 1.0 Hz, 1H), 7.14 (d, J = 8.1 Hz, 1H), 5.26 (d, J = 5.5 Hz, 2H), 4.93 - 4.75 (m, 2H), 4.07 (t, J = 11.7 Hz, 1H), 3.86 (dd, J = 17.1, 12.0 Hz, 1H), 2.15 (tt, J = 8.1, 4.8 Hz, 1H), 1.74 (d, J = 20.8 Hz, 3H), 1.26 - 1.16 (m, 2H), 1.09 (dt, J = 8.2, 3.2 Hz, 2H). |
| 241 | 506.80 | ¹H NMR (300 MHz, DMSO-*d*₆) δ 9.46 - 9.42 (m, 3H), 8.92 (t, *J =* 1.9 Hz, 1H), 8.79 - 8.74 (m, 2H), 8.59 - 8.54 (m, 2H), 7.91 (s, 1H), 7.25 - 6.65 (m, 1H), 4.83 (d, *J* = 5.3 Hz, 2H), 4.58 - 4.54 (m, 1H), 4.41 - 4.35 (m, 1H), 2.37 (dd, J = 12.3, 6.0 Hz, 1H), 1.17 (d, *J* = 5.4 Hz, 4H). |
| 243 | 506.80 | ¹H NMR (300 MHz, DMSO-*d*₆) δ 9.47 - 9.42 (m, 3H), 8.92 (t, *J =* 1.9 Hz, 1H), 8.83 - 8.74 (m, 2H), 8.59 - 8.55 (m, 2H), 7.93 (s, 1H), 7.24 - 6.65 (m, 1H), 4.84 (d, *J* = 5.3 Hz, 2H), 4.58 - 4.52 (m, 1H), 4.42 - 4.38 (m, 1H), 2.37 (dd, J = 12.3, 6.0 Hz, 1H), 1.17 (d, *J* = 5.4 Hz, 4H). |
| 244 | 492.10 | ¹H NMR (300 MHz, DMSO-*d*₆) δ 9.59 (s, 1H), 9.49 (m, 1H), 9.14 (s, 1H), 8.88 - 8.48 (m, 2H), 8.12 (m, 1H), 7.54 (d, *J* = 4.9 Hz, 1H), 5.13 - 4.71 (m, 4H), 4.36 - 4.08 (m, 5H), 3.98 (dd, J = 25.3, 12.7 Hz, 1H), 2.45 (t, *J =* 7.4 Hz, 2H), 1.78 (d, *J* = 20.9 Hz, 3H). |
| 245 | 491.10 | ¹H NMR (300 MHz, DMSO-*d*₆) δ 9.50 - 9.25 (m, 2H), 8.61 (d, *J* = 4.9 Hz, 1H), 8.24 (m, 1H), 7.95 (d, *J* = 12.5 Hz, 2H), 7.46 (d, J = 4.9 Hz, 1H), 7.29 (d, *J* = 8.1 Hz, 1H), 4.99 - 4.58 (m, 4H), 4.26 - 4.04 (m, 5H), 3.84 (dd, J *=* 25.2, 12.5 Hz, 1H), 2.39 (q, *J* = 7.4 Hz, 2H), 1.69 (d, *J* = 20.7 Hz, 3H). |
| 246 | 505.3 | 1H NMR (400 MHz, MeOD) δ = 9.47-9.46 (m, 1H), 9.34 (s, 1H), 9.05 (d, J = 2.0 Hz, 1H), 8.64 - 8.58 (m, 4H), 8.00 (s, 1H), 6.25 - 5.97 (m, 1H), 4.94 (s, 2H), 4.86 (br s, 2H), 4.28 (d, J = 12.0 Hz, 1H), 3.77 (br d, J = 1.2 Hz, 1H), 2.33 - 2.28 (m, 1H), 1.23 - 1.20(m, 2H), 1.18 - 1.17 (m, 2H) ppm |
| 248 | 472.3 | 1H NMR (400 MHz, MeOD) δ = 9.40 - 9.37 (m, 2H), 8.65 (s, 2H), 8.27 (s, 1H), 7.98 (d, J = 8.0 Hz, 1H), 7.83 - 7.79 (m, 2H), 7.68 - 7.66 (m, 1H), 7.30 (d, J = 8.0 Hz, 1H), 6.82 (d, J = 8.4 Hz, 1H), 4.88 - 4.75 (m, 4H), 4.11 - 4.04 (m, 1H), 3.89 - 3.79 (m, 1H), 3.15 (s, 6H), 1.72 - 1.67 (m, 3H) ppm |
| 249 | 515.4 | 1H NMR (400 MHz, DMSO-d6) δ = 9.42 - 9.37 (m, 2H), 8.70 - 8.69 (m, 2H), 8.49 - 8.47 (m, 1H), 8.28 (s, 1H), 8.03 - 8.01 (m, 2H), 7.88 - 7.83 (m, 2H), 7.31 (d, J = 8.4 Hz, 1H), 5.77 (s, 1H), 4.85 - 4.76 (m, 4H), 4.12 - 4.07 (m, 4H), 3.89 - 3.86 (m, 2H), 2.67 - 2.66 (m, 1H), 2.16 - 2.11 (m, 1H), 1.72 -1.67 (m, 3H) ppm |
| 251 | 461.2 | 1H NMR (400 MHz, DMSO-d6) δ= 9.30 - 9.27 (m, 1H), 9.16 (s, 1H), 8.25 (s, 1H), 8.21 (s, 1H), 8.07 - 8.03 (m, 2H), 7.96 - 7.94 (m, 1H), 7.89 - 7.86 (m, 1H), 7.58 (s, 1H), 7.29 (d, J = 8.0 Hz, 1H), 4.84 - 4.83 (m, 1H), 4.75 - 4.70 (m, 3H), 4.12 - 4.01 (m, 1H), 3.94 (s, 3H), 3.88 - 3.79 (m, 1H), 3.74 (s, 3H), 1.68 (d, J = 20.4 Hz, 1H) ppm |
| 252 | 469.20 | ¹H NMR (300 MHz, Methanol-*d*₄) δ 9.49 (s, 1H), 9.37 (s, 1H), 9.11 (s, 1H), 8.74 (dd, J = 7.8, 2.5 Hz, 4H), 8.13 (s, 1H), 5.04 (s, 2H), 3.13 - 3.00 (m, 2H), 2.53 - 2.03 (m, 5H), 1.88 (d, *J* = 21.1 Hz, 3H), 1.35 - 1.24 (m, 4H). |
| 253 | 478.10 | ¹H NMR (300 MHz, Methanol-*d*₄) δ 9.48 (s, 1H), 9.37 (s, 1H), 8.77 (d, *J =* 2.3 Hz, 1H), 8.71 - 8.58 (m, 3H), 8.56 (d, *J* = 2.3 Hz, 1H), 8.04 (s, 1H), 4.93 (s, 2H), 4.58 (dd, J = 6.5, 4.1 Hz, 2H), 2.60 - 2.46 (m, 1H), 2.38 - 2.21 (m, 2H), 1.87 (s, 3H), 1.29 - 1.12 (m, 4H). |
| 254 | 480.10 | ¹H NMR (300 MHz, Methanol-*d*₄) δ 9.12 (d, *J =* 9.1 Hz, 1H), 9.03 (dd, J = 9.1, 1.0 Hz, 1H), 8.95 - 8.88 (m, 1H), 8.41 - 8.36 (m, 1H), 8.35 - 8.23 (m, 2H), 8.06 - 7.92 (m, 2H), 7.36 (d, *J* = 8.0 Hz, 1H), 6.97 (t, *J* = 55.2 Hz, 1H), 5.31 (s, 2H), 4.93 - 4.82 (m, 2H), 4.16 (t, *J =* 12.3 Hz, 1H), 3.97 (dd, *J* = 20.0, 12.2 Hz, 1H), 1.81 (d, J = 20.6 Hz, 3H). |
| 255 | 470.85 | ¹H NMR (300 MHz, DMSO-d6) δ 9.56 (t, *J =* 5.8 Hz, 1H), 9.49 (s, 1H), 9.06 (dd, J = 8.9, 1.0 Hz, 1H), 8.84 - 8.79 (m, 2H), 8.27 - 8.24 (m, 2H), 7.96 (dt, J = 8.0, 1.7 Hz, 1H), 7.31 (d, *J* = 8.1 Hz, 1H), 5.16 - 5.14 (m, 2H), 4.90 - 4.70 (m, 2H), 4.08 (t, *J* = 13.0 Hz, 1H), 3.84 (dd, J = 25.1, 12.5 Hz, 1H), 2.42 - 2.34 (m, 1H), 1.69 (d, *J* = 20.7 Hz, 3H), 1.22 - 1.13 (m, 4H). |
| 257 | 485.2 | 1HNMR (400 MHz, DMSO-d6) δ = 9.35 - 9.32 (m, 2H), 8.46 (s, 1H), 8.25 (s, 1H), 8.17 (d, J = 8.8 Hz, 1H), 8.04 - 8.01 (m, 2H), 7.97-7.94 (m, 1H), 7.76 - 7.73 (m, 2H), 7.29 (d, J = 8.0 Hz, 1H), 4.87 - 4.70 (m, 4H), 4.19 - 4.13 (m, 1H), 410-4.03 (m, 1H), 3.87 - 3.78 (m, 1H), 1.75 (d, J = 20.8 Hz, 1H) ppm |
| 259 | 515.2 | 1H NMR (400 MHz, DMSO-d6) δ = 9.48 (s, 1H), 9.43 -9.41 (m, 1H), 8.77 - 8.75 (m, 1H), 8.66 (d, J = 8.4 Hz, 1H), 8.61 (d, J = 5.2 Hz, 1H), 8.37 - 8.32 (m, 1H), 8.28 (s, 1H), 7.98 - 7.97 (m, 1H), 7.85 (s, 1H), 7.74 (d, J = 4.8 Hz, 1H), 7.32 (d, J = 8.4 Hz, 1H), 4.85 - 4.82 (m, 4H), 4.08 (s, 1H), 3.89 - 3.83 (m, 5H), 3.74 - 3.71 (m, 4H), 1.72 - 1.67 (m, 3H) ppm |
| 260 | 486.2 | 1H NMR (400 MHz, DMSO-d6) δ = 9.47 - 9.39 (m, 3H), 8.89 - 8.82 (m, 2H), 8.76 (d, J = 8.8 Hz, 1H), 8.44 (d, J = 8.8 Hz, 1H), 8.28 (s, 1H), 8.20 - 8.18 (m, 1H), 7.99 - 7.96 (m, 1H), 7.86 (s, 1H), 7.31 (d, J = 8.0 Hz, 1H), 4.89 - 4.72 (m, 4H), 4.11 - 4.05 (m, 1H), 3.89 - 3.80 (m, 1H), 2.87 (d, J = 4.8 Hz, 3H), 1.72 - 1.67 (m, 3H) ppm |
| 261 | 469.3 | 1H NMR (400 MHz, MeOD-d4) δ = 9.29 - 9.23 (m, 2H), 8.49 (d, J = 1.6 Hz, 1H), 8.16 (d, J = 1.6 Hz, 1H), 8.13 (s, 1H), 7.92 - 7.89 (m, 1H), 7.77 (s, 1H), 7.28 (d, J = 8.0 Hz, 1H), 4.91- 4.86 (m, 4H), 4.20 (d, J = 11.2 Hz, 1H), 4.05 (s, 3H), 3.93 (d, J= 11.6 Hz, 1H), 3.83 (s, 3H), 1.76 (s, 3H) ppm |
| 262 | 491.2 | 1H NMR (400 MHz, MeOD) δ = 9.23 (s, 1H), 8.50 (s, 1H), 8.38 - 8.30 (m, 1H), 8.17 - 8.11 (m, 2H), 7.92 - 7.90 (m, 1H), 7.88 - 7.82 (m, 3H), 7.74 - 7.71 (m, 1H), 7.27 (d, J = 8.0 Hz, 1H), 4.93 - 4.87 (m, 4H), 4.19 (d, J = 11.6 Hz, 1H), 3.92 (d, J = 11.6 Hz, 1H), 1.75 (s, 3H), 1.53 - 1.48 (m, 2H), 1.25 - 1.21 (m, 2H) ppm |
| 263 | 537 | 1H NMR (400 MHz, DMSO-d6) δ = 9.44-9.41 (m, 1H), 9.31 (s, 1H), 8.58 (s, 1H), 8.34 (d, J = 10 Hz, 1H), 8.17 (d, J = 10 Hz, 1H), 8.14 (s, 1H), 7.96 - 7.93 (m, 1H), 7.83 (s, 1H), 7.66-7.64 (m, 1H), 7.42 (d, J = 7.6 Hz, 1H), 6.75-6.60 (m, 1H), 6.42 (d, J = 8.4 Hz, 1H), 5.0 (d, J = 16 Hz, 1H), 4.84 (d, J = 16 Hz, 1H), 4.77-4.76 (m, 2H), 4.27-4.15 (m, 2H), 4.06-4.03 (m, 4H), 2.40-2.33 (m, 2H) ppm |
| 264 | 439.3 | 1H NMR (400 MHz, DMSO-d6) δ= 13.19 - 12.78 (m, 1H), 9.44 - 9.26 (m, 2H), 8.58 - 8.45 (m, 1H), 8.28 - 8.13 (m, 2H), 7.96 - 7.94 (m, 1H), 7.72 (s, 1H), 7.31 (d, J = 8.0 Hz, 1H), 6.84 - 6.72 (m, 1H), 4.94 - 4.82 (m, 2H), 4.79 (d, J = 6.0 Hz, 2H), 4.23 (d, J = 11.2 Hz, 1H), 3.87 (d, J = 11.2 Hz, 1H), 2.30 (s, 3H), 1.70 (s, 3H) ppm |
| 265 | 528.4 | 1H NMR (400 MHz, DMSO-d6) δ = 9.35 - 9.34 (m, 2H), 8.66 - 8.58 (m, 2H), 8.37 - 8.35 (m, 1H), 8.27 - 8.21 (m, 2H), 7.94 (d, J = 4.0 Hz, 2H), 7.82 - 7.79 (m, 1H), 7.30 (d, J = 8.0 Hz, 1H), 4.85 - 4.75 (m, 4H), 4.15 - 4.04 (m, 2H), 3.89 - 3.83 (m, 1H), 3.68 - 3.65 (m, 3H), 3.52 - 3.50 (m, 1H), 3.30 (s, 3H), 2.13 - 2.10 (m, 2H), 1.72 - 1.67 (m, 3H) ppm |
| 266 | 528.4 | 1H NMR (400 MHz, DMSO-d6) δ = 9.36 - 9.34 (m, 2H), 8.68 (s, 1H), 8.58 (s, 1H), 8.37 - 8.34 (m, 1H), 8.27 - 8.21 (m, 2H), 8.02 - 7.96 (m, 2H), 7.82 (s, 1H), 7.30 (d, J = 8.0 Hz, 1H), 4.85 - 4.75 (m, 4H), 4.15 - 4.08 (m, 2H), 3.89 - 3.83 (m, 1H), 3.68 - 3.65 (m, 3H), 3.54 - 3.50 (m, 1H), 3.30 (s, 3H), 2.14 - 2.08 (m, 2H), 1.72 - 1.67 (m, 3H) ppm |
| 267 | 542.2 | 1H NMR (400 MHz, DMSO-d6) δ= 9.48 (s, 1H), 9.42-9.39 (m, 1H), 8.77 (d, J = 8.4 Hz, 1H), 8.68 (d, J = 8.8 Hz, 1H), 8.64 (d, J = 4.8 Hz, 1H), 8.27 (s, 1H), 7.98 - 7.96 (m, 1H), 7.85 (s, 1H), 7.78 (d, J = 5.2 Hz, 1H), 7.31 (d, J = 8.0 Hz, 1H), 4.88 - 4.81(m, 3H), 4.76 - 4.71 (m, 1H), 4.38 - 4.28 (m, 2H), 4.14 - 4.04 (m, 3H), 3.89 - 3.79 (m, 1H), 3.49-3.46 (m, 2H), 2.91 (s, 3H),1.72 - 1.63 (m, 3H) ppm |
| 268 | 486.2 | 1H NMR (400 MHz, DMSO-d6) δ = 9.43 - 9.39 (m, 2H), 8.69 - 8.63 (m, 2H), 8.53 - 8.42 (m, 1H), 8.27 (s, 1H), 8.19 (d, J = 4.4Hz, 1H), 8.07 (d, J = 4 Hz, 1H), 7.97 - 7.95 (m, 1H), 7.78 (s, 1H), 7.30 (d, J = 8.0 Hz, 1H), 4.88 - 4.70 (m, 4H), 4.23 - 4.19(m, 1H), 4.18-4.04 (m, 1H), 3.88 - 3.79 (m, 1H), 1.68 (d, J = 20.4 Hz, 1H), 1.10 - 1.00 (m, 4H) ppm |
| 269 | 502.2 | 1H NMR (400 MHz, DMSO-d6) δ = 9.44 - 9.41 (m, 1H), 9.33 (s, 1H), 8.63 - 8.57 (m, 2H), 8.32 - 8.29 (m, 1H), 8.22 - 8.20 (m, 1H), 8.15 (s, 1H), 7.88 (s, 1H), 7.82 - 7.77 (m, 2H), 4.94 (d, J = 16.8 Hz, 1H), 4.78 - 4.71 (m, 3H), 4.16 - 4.04 (m, 5H), 3.89 - 3.80 (m, 1H), 2.45 - 2.38 (m, 2H), 1.72 - 1.66 (m, 3H) ppm |
| 270 | 509.2 | 1H NMR (400 MHz, DMSO-d6) δ = 9.44 - 9.41 (m, 1H), 9.33 (s, 1H), 8.63 - 8.60 (m, 2H), 8.32 - 8.29 (m, 1H), 8.22 - 8.20 (m, 1H), 8.03 (d, J = 1.2 Hz, 1H), 7.88 - 7.83 (m, 2H), 7.78 - 7.76 (m, 1H), 4.97 - 4.83 (m, 2H), 4.77 (d, J = 5.6 Hz, 2H), 4.23 (d, J = 11.6 Hz, 1H), 4.16 - 4.12 (m, 4H), 3.87 (d, J = 11.2 Hz, 1H), 2.45 - 2.40 (m, 2H), 1.70 (s, 3H) ppm |
| 271 | 485.3 | 1H NMR (400 MHz, DMSO-d6) δ = 9.40 (s, 2H), 8.66 (d, J = 0.8 Hz, 1H), 8.55 (d, J = 8.4 Hz, 1H), 8.35 (d, J = 1.2 Hz, 1H), 8.28 (s, 1H), 7.95 (s, 2H), 7.89 - 7.79 (m, 2H), 7.35 - 7.28 (m, 1H), 6.29 (s, 1H), 4.92 - 4.69 (m, 4H), 4.14 - 4.03 (m, 1H), 3.91 - 3.77 (m, 1H), 1.70 (d, J = 20.8 Hz, 3H), 1.42 - 1.41 (m, 2H), 1.19 - 1.18 (m, 2H) ppm |
| 272 | 508.2 | 1H NMR (400 MHz, DMSO-d6) δ = 9.36-9.32 (m, 2H), 8.24-8.17 (m, 2H), 7.99-7.92 (m, 2H), 7.82-7.80 (m, 1H), 7.29 (d, J = 8.0 Hz, 1H), 6.90 (d, J = 8.0 Hz, 1H), 4.88 - 4.76 (m, 4H), 4.22 (d, J = 11.6 Hz, 1H), 4.14-4.10 (m, 4H), 3.87-3.84 (d, J = 11.6 Hz, 1H), 2.37-2.32 (m, 1H), 1.68 (s, 3H) ppm |
| 273 | 520.1 | 1H NMR (400 MHz, MeOD) δ = 9.30 (s, 1H), 8.67 - 8.54 (m, 2H), 8.27 - 8.23 (m, 1H), 8.09 - 8.08 (m, 1H), 7.96 - 7.91 (m, 2H), 7.76 - 7.73 (m, 1H), 7.27 (d, J = 8.4 Hz, 1H), 6.89 - 6.87 (m, 1H), 4.92 (s, 2H), 4.91 - 4.89 (m, 1H), 4.81 - 4.80 (m, 1H), 4.15 - 4.02 (m, 1H), 3.96 - 3.81 (m, 1H), 3.50 (s, 6H), 1.73 (d, J = 20.4 Hz, 3H) ppm |
| 274 | 475.20 | ¹H NMR (300 MHz, DMSO-d₆) δ 9.31 (t, *J* = 5.9 Hz, 1H), 9.20 (d, *J* = 0.9 Hz, 1H), 8.44 (s, 1H), 8.17 (d, *J* = 1.7 Hz, 1H), 8.01 - 7.86 (m, 1H), 7.75 (d, *J* = 0.9 Hz, 1H), 7.29 (d, *J* = 8.1 Hz, 1H), 5.00 - 4.78 (m, 2H), 4.71 (d, *J* = 5.8 Hz, 2H), 4.23 (d, *J* = 11.5 Hz, 1H), 4.03 (s, 3H), 3.85 (d, *J* = 21.7 Hz, 4H), 1.70 (s, 3H). |
| 275 | 463.20 | ¹H-NMR (300 MHz, DMSO-d₆) δ 9.50 (t, J = 5.8 Hz, 1H), 8.75 (dd, J = 9.1, 1.0 Hz, 1H), 8.44 (s, 1H), 8.29 - 8.20 (m, 2H), 7.95 (dt, J = 8.1, 1.7 Hz, 1H), 7.84 (s, 1H), 7.30 (d, J = 8.2 Hz, 1H), 5.05 (dd, J = 5.9, 2.8 Hz, 2H), 4.86 (dd, J = 15.6, 2.5 Hz, 1H), 4.73 (dd, J = 15.7, 4.4 Hz, 1H), 4.08 (t, J = 13.0 Hz, 1H), 4.00 (s, 3H), 3.93 - 3.80 (m, 1H), 3.79 (s, 3H), 1.69 (d, J = 20.7 Hz, 3H). LCMS (ESI) m/z [M+H]⁺ =271. |
| 276 | 485.90 | ¹H NMR (400 MHz, Methanol-d₄) δ 8.97 (s, 1H), 8.94 - 8.83 (m, 2H), 8.28 (s, 2H), 7.91 (d, *J* = 5.2 Hz, 2H), 7.27 (d, *J* = 8.1 Hz, 1H), 5.21 (s, 2H), 4.84 (m, 2H), 4.26 (t, *J* = 7.5 Hz, 4H), 4.07 (t, *J* = 12.2 Hz, 1H), 3.94 - 3.81 (m, 1H), 2.54 (p, *J* = 7.5 Hz, 2H), 1.75 (s, 3H). |
| 277 | 429.20 | ¹H NMR (400 MHz, DMSO-d₆) δ 9.53 (t, J = 5.8 Hz, 1H), 8.95 (dd, J = 9.0, 1.0 Hz, 1H), 8.58 (d, *J* = 9.1 Hz, 1H), 8.41 - 8.33 (m, 2H), 8.30 - 8.22 (m, 1H), 8.14 (s, 1H), 8.00 - 7.92 (m, 1H), 7.64 - 7.56 (m, 3H), 7.30 (d, *J =* 8.1 Hz, 1H), 5.17 - 5.09 (m, 2H), 4.90 - 4.82 (m, 1H), 4.78 - 4.68 (m, 1H), 4.07 (t, *J* = 13.0 Hz, 1H), 3.92 - 3.75 (m, 1H), 1.69 (d, *J* = 20.7 Hz, 3H). |
| 278 | 650.30 | ¹H NMR (300 MHz, DMSO-d₆) δ9.53 - 9.43 (m, 2H), 8.80 - 8.66 (m, 2H), 8.31 - 8.25 (m, 1H), 8.08 - 7.95 (m, 2H), 7.92 - 7.78 (m, 2H), 7.39 (d, *J =* 8.1 Hz, 1H), 7.12 (d, *J* = 8.4 Hz, 1H), 5.05 - 4.85 (m, 4H), 4.31 (d, *J =* 11.5 Hz, 1H), 3.95 (d, *J* = 11.5 Hz, 1H), 3.79 - 3.68 (m, 10H), 3.63 - 3.45 (m, 4H), 3.30 (s, 3H), 2.72 (t, *J* = 6.6 Hz, 2H), 1.78 (s, 3H). |
| 280 | 494.1 | 1H NMR (400 MHz, METHANOL-d4) δ = 9.45 (s, 1H), 9.31 - 9.20 (m, 1H), 8.68 (d, J = 0.8 Hz, 1H), 8.36 (d, J = 4.8 Hz, 1H), 8.16 (d, J = 1.6 Hz, 1H), 7.96 (s, 1H), 7.93 - 7.87 (m, 1H), 7.51 - 7.40 (m, 1H), 7.28 (d, J = 8.0 Hz, 1H), 4.93 - 4.90 (m, 4H), 4.20 (d, J = 11.6 Hz, 1H), 3.92 (d, J = 11.2 Hz, 1H), 2.58 - 2.37 (m, 1H), 1.76 (s, 3H), 1.16 - 1.08 (m, 4H) ppm |
| 284 | 534.3 | 1H NMR (400 MHz, DMSO-d6) δ = 9.48 (s, 1H), 9.44 - 9.41 (m, 1H), 8.80 - 8.75 (m, 1H), 8.73 - 8.68 (m, 1H), 8.65 (d, J = 5.2 Hz, 1H), 8.21 (d, J = 1.6 Hz, 1H), 7.98 - 7.95 (m, 1H), 7.88 (s, 1H), 7.79 (d, J = 4.8 Hz, 1H), 7.32 (d, J = 8.0 Hz, 1H), 4.97 - 4.82 (m, 4H), 4.76 (br s, 2H), 4.24 (d, J = 11.6 Hz, 1H), 4.16 - 4.04 (m, 1H), 4.00 - 3.67 (m, 4H), 3.22 - 3.13 (m, 1H), 1.93 (d, J = 8.8 Hz, 1H), 1.71 (s, 3H) ppm |
| 285 | 518.3 | 1H NMR (400 MHz, DMSO-d6) δ = 9.47 (s, 1H), 9.43 - 9.40 (m, 1H), 8.81 - 8.72 (m, 1H), 8.65 (d, J = 8.8 Hz, 1H), 8.54 (d, J = 4.8 Hz, 1H), 8.20 (d, J = 1.6 Hz, 1H), 7.96 - 7.93 (m 1H), 7.86 (s, 1H), 7.67 (d, J = 4.8 Hz, 1H), 7.31 (d, J = 8.0 Hz, 1H), 5.12 - 4.96 (m, 1H), 4.95 - 4.79 (m, 4H), 4.23 (d, J = 11.6 Hz, 1H), 3.87 (d, J = 11.6 Hz, 1H), 3.58 (br s, 2H), 3.03 - 2.95 (m, 1H), 2.05 (br s, 2H), 1.70 (s, 3H), 1.47 - 1.38 (m, 2H) ppm |
| 287 | 645.00 | ¹H NMR (300 MHz, DMSO-d₆) δ 9.42 (d, *J* = 5.1 Hz, 2H), 8.73 - 8.58 (m, 2H), 8.20 (d, *J* = 1.7 Hz, 1H), 8.01 - 7.88 (m, 2H), 7.85 - 7.70 (m, 2H), 7.31 (d, *J* = 8.1 Hz, 1H), 7.04 (d, *J* = 8.5 Hz, 1H), 4.96 - 4.73 (m, 4H), 4.23 (d, *J* = 11.5 Hz, 1H), 3.87 (d, *J* = 11.5 Hz, 1H), 3.66 (d, *J* = 17.0 Hz, 8H), 2.84 (d, *J* = 11.0 Hz, 2H), 2.62 (t, *J* = 7.3 Hz, 1H), 2.21 (s, 3H), 2.01 (s, 2H), 1.71 (s, 3H), 1.65 (dd, J = 8.1, 3.3 Hz, 4H). |
| 288 | 606.40 | ¹H NMR (300 MHz, DMSO-d₆) δ 9.41 (s, 2H), 8.77 - 8.54 (m, 2H), 8.20 (s, 1H), 8.01 - 7.88 (m, 2H), 7.84 - 7.71 (m, 2H), 7.31 (d, *J* = 8.1 Hz, 1H), 7.04 (d, *J* = 8.5 Hz, 1H), 4.85 (dd, J = 19.0, 6.6 Hz, 4H), 4.23 (d, *J* = 11.5 Hz, 1H), 3.87 (d, *J =* 11.6 Hz, 1H), 3.78 - 3.51 (m, 10H), 3.24 (s, 3H), 2.64 (t, *J* = 6.5 Hz, 2H), 1.71 (s, 3H). |
| 289 | 445.15 | ¹H NMR (300 MHz, DMSO-d₆) δ 9.32 (t, *J* = 5.9 Hz, 1H), 9.24 (d, *J* = 0.9 Hz, 1H), 8.63 (s, 1H), 8.24 - 8.11 (m, 2H), 7.92 (d, 1H), 7.84 - 7.78 (m, 1H), 7.30 (d, *J* = 8.1 Hz, 1H), 5.00 - 4.62 (m, 4H), 4.23 (d, *J* = 11.5 Hz, 1H), 3.94 (s, 3H), 3.91 (d, 1H), 1.70 (s, 3H). |
| 290 | 435.20 | ¹H NMR (300 MHz, Methanol-d₄) δ 9.27 - 9.20 (m, 1H), 9.11 - 9.02 (m, 1H), 8.64 - 8.57 (m, 1H), 8.26 - 8.13 (m, 2H), 8.06 - 8.00 (m, 1H), 7.69 - 7.53 (m, 3H), 4.93 - 4.90 (m, 2H), 4.88 - 4.83 (m, 2H), 4.20 - 4.07 (m, 1H), 4.02 - 3.89 (m, 1H), 1.77 (d, *J* = 20.7 Hz, 3H). |
| 291 | 524.3 | 1H NMR (400 MHz, DMSO-d6) δ = 9.47 - 9.37 (m, 2H), 8.93 (s, 1H), 8.75 - 870 (m, 1H), 8.59 (d, J = 8.4 Hz, 1H), 8.22 - 8.17 (m, 2H), 7.97 - 7.94 (m, 1H), 7.86 (s, 1H), 7.31 (d, J = 8.0 Hz, 1H), 5.63 - 5.43 (m, 1H), 4.96 - 4.77 (m, 4H), 4.23 (d, J = 11.6 Hz, 1H), 4.00 - 3.57 (m, 5H), 2.36 - 2.15 (m, 2H), 1.71 (s, 3H) ppm |
| 292 | 570.4 | 1H NMR (400 MHz, DMSO-d6) δ = 9.46 (s, 1H), 9.41-9.38 (m, 1H), 8.75 (d, J = 8.0 Hz, 1H), 8.63 (d, J = 8.4 Hz, 1H), 8.58 (d, J = 4.8 Hz, 1H), 8.26 (s, 1H), 7.97 - 7.95 (m, 1H), 7.84 (s, 1H), 7.70 (d, J = 4.8 Hz, 1H), 7.30 (d, J = 8.4 Hz, 1H), 4.88 - 4.70 (m, 4H), 4.58 - 4.55 (m, 2H), 4.51 - 4.48 (m, 2H), 4.10-4.04 (m, 1H), 3.89 (br d, J = 5.6 Hz, 4H), 3.85 - 3.79 (m, 1H), 3.48-3.42 (m, 1H), 2.38-2.36 (m, 4H), 1.71 - 1.66 (m, 3H) ppm |
| 293 | 460.3 | 1H NMR (400 MHz, METHANOL-d4) δ = 9.36 (s, 1H), 8.60 (s, 2H), 8.23 - 8.21 (m, 2H), 8.08 (d, J = 4.0 Hz, 1H), 7.93 - 7.91 (m, 2H), 7.27 (d, J = 7.6 Hz, 1H), 4.92 (s, 2H), 4.90 (br d, J = 2.8 Hz, 1H), 4.81 (br d, J = 3.6 Hz, 1H), 4.10 - 4.04 (m, 1H), 3.92 - 3.84 (m, 4H), 1.75 - 1.70 (m, 3H) ppm |
| 294 | 509.3 | 1H NMR (400 MHz, METHANOL-d4) δ = 9.43 (s, 1H), 9.18 (s, 1H), 8.61 (s, 1H), 8.16 (d, J = 0.8 Hz, 1H), 8.00 - 7.88 (m, 3H), 7.27 (d, J = 8.0 Hz, 1H), 6.91 - 6.88 (m, 1H), 4.93 - 4.89 (m, 4H), 4.29 - 4.17 (m, 5H), 3.92 (d, J = 11.6 Hz, 1H), 2.50 - 2.39 (m, 2H), 1.75 (s, 3H) ppm |
| 295 | 484.30 | 1H NMR (400 MHz, MeOD) δ = 9.30 (s, 1H), 8.64 - 8.62 (m, 1H), 8.57 - 8.55 (m, 1H), 8.25 (s, 1H), 7.95 - 7.93 (m, 2H), 7.85 (d, J= 7.2 Hz, 1H), 7.70 - 7.66 (m, 1H), 7.29 (d, J = 8.0 Hz, 1H), 6.52 - 6.50 (m, 1H), 4.92 (s, 2H), 4.82 - 4.77 (m, 2H), 4.15-4.05 (m,5H), 3.93 - 3.88 (m, 1H), 2.48 - 2.41 (m, 2H), 1.76 - 1.71 (m, 3H) ppm |
| 296 | 503.1 | 1H NMR (400 MHz, DMSO-d6) δ = 9.57 - 9.54 (m, 1H), 9.47 (s, 1H), 9.08 - 9.07 (m, 1H), 8.72 (d, J = 8.0 Hz, 1H), 8.61 (s, 1H), 8.07 - 7.99 (m, 2H), 7.88 (s, 1H), 7.16 - 7.14 (m, 1H), 4.91 - 4.71 (m, 4H), 4.21 - 4.06 (m, 5H), 3.97 - 3.84 (m, 1H), 2.39 - 2.33 (m, 2H), 1.79 - 1.64 (m, 3H) ppm |
| 297 | 469.2 | 1H NMR (400 MHz, DMSO-d6) δ = 9.37 - 9.33 (m, 2H), 8.61 - 8.59 (m, 1H), 8.17 (d, J = 1.6 Hz, 1H), 8.09 (d, J = 8.8 Hz, 1H), 7.93 - 7.91 (m, 1H), 7.78 (s, 1H), 7.55 (s, 1H), 7.30 (d, J = 8.0 Hz, 1H), 4.92 - 4.78 (m, 4H), 4.23 - 4.20 (m, 4H), 3.89- 3.87 (m, 4H), 1.69 (s, 3H) ppm |
| 299 | 501.4 | 1H NMR (400 MHz, MeOD) δ = 9.91 (s, 1H), 9.39 (s, 1H), 9.05 (s, 1H), 8.70 (d, J = 0.8 Hz, 2H), 8.18 (d, J = 1.6 Hz, 1H), 8.05 (s, 1H), 7.92 (d, J = 1.6 Hz, 1H), 7.30 (d, J = 8.0 Hz, 1H), 4.97 - 4.94 (m, 2H), 4.92 (d, J = 3.6 Hz, 2H), 4.21 (d, J = 11.2 Hz, 1H), 3.93 (d, J = 11.2 Hz, 1H), 2.22 - 2.12 (m, 3H), 1.77 (s, 3H) ppm |
| 300 | 433.90 | ¹H NMR (300 MHz, Methanol-d₄) δ 9.29 (s, 1H), 8.35 - 8.22 (m, 3H), 8.10 - 7.97 (m, 2H), 7.74 - 7.61 (m, 3H), 7.41 - 7.27 (m, 1H), 5.01 - 4.97 (m, 1H), 4.94 - 4.90 (m, 2H), 4.90 - 4.82 (m, 1H), 4.24 - 4.08 (m, 1H), 4.06 - 3.88 (m, 1H), 1.82 (d, *J* = 20.5 Hz, 3H). |
| 301 | 471.00 | ¹H NMR (400 MHz, DMSO-d₆) δ 9.59 (t, *J* = 5.9 Hz, 1H), 9.47 (d, *J* = 4.1 Hz, 2H), 9.10 (t, *J* = 1.8 Hz, 1H), 8.82 - 8.73 (m, 2H), 8.63 (t, *J* = 1.8 Hz, 1H), 8.56 (d, *J* = 8.6 Hz, 1H), 7.94 (s, 1H), 4.93 - 4.72 (m, 4H), 4.13 (t, *J* = 12.9 Hz, 1H), 3.91 (dd, J = 25.3, 12.6 Hz, 1H), 2.36 (dt, J = 8.5, 5.6 Hz, 1H), 1.72 (d, *J* = 20.9 Hz, 3H), 1.19 - 1.12 (m, 4H). |
| 302 | 529.3 | 1H NMR (400 MHz, CDCl3) δ = 9.41 (s, 1H), 8.83 (d, J = 8.6 Hz, 1H), 8.68 (d, J = 7.5 Hz, 1H), 8.56 (d, J = 8.6 Hz, 1H), 8.30 (s, 2H), 8.21 (s, 1H), 8.03 (t, J = 7.8 Hz, 1H), 7.95 - 7.85 (m, 1H), 7.63 (d, J = 7.7 Hz, 1H), 7.14 (d, J = 8.2 Hz, 1H), 5.07 (br d, J = 5.5 Hz, 2H), 4.94 - 4.70 (m, 2H), 4.20 - 3.93 (m, 6H), 3.84 (br dd, J = 12.1, 19.0 Hz, 1H), 2.37 - 2.22 (m, 2H), 1.80 - 1.71 (m, 3H), 1.67 (br d, J = 12.8 Hz, 2H) ppm |
| 305 | 457.4 | 1H NMR (400 MHz, CHLOROFORM-d) δ = 9.25 (s, 1H), 8.38 - 8.36 (m, 1H), 8.28 (s, 1H), 8.12 - 8.10 (m, 2H), 7.91 (s, 1H), 7.84 (d, J = 8.0 Hz, 1H), 7.16 (d, J = 8.4 Hz, 1H), 4.99 (d, J = 5.2 Hz, 2H), 4.91 - 4.76 (m, 2H), 4.12 - 4.03 (m, 4H), 3.90 - 3.80 (m, 1H), 1.78 - 1.73 (m, 3H) ppm |
| 306 | 524.5 | 1H NMR (400 MHz, DMSO-d6) δ = 9.47 (s, 1H), 9.43 - 9.39 (m, 1H), 8.79 - 8.74 (m, 1H), 8.67 (d, J = 8.4 Hz, 1H), 8.59 (d, J = 5.2 Hz, 1H), 8.20 (d, J = 1.6 Hz, 1H), 7.96 - 7.94 (m, 1H), 7.87 (s, 1H), 7.72 (d, J = 5.2 Hz, 1H), 7.31 (d, J = 8.4 Hz, 1H), 5.60 - 5.39 (m, 1H), 4.94 - 4.81 (m, 4H), 4.23 (d, J = 11.6 Hz, 1H), 4.01 - 3.59 (m, 5H), 2.33 - 2.15 (m, 2H), 1.70 (s, 3H) ppm |
| 307 | 555.2 | 1H NMR (400 MHz, DMSO-d6) δ = 9.47 (s, 1H), 9.42 - 9.39 (m, 1H), 8.75 (d, J = 8.8 Hz, 1H), 8.64 (d, J = 8.8 Hz, 1H), 8.58 (d, J = 5.2 Hz, 1H), 8.28 (s, 1H), 7.97 (d, J = 8.0 Hz, 1H), 7.85 (s, 1H), 7.69 (d, J = 5.2 Hz, 1H), 7.31 (d, J = 8.0 Hz, 1H), 5.22 - 5.03 (m, 2H), 4.92 - 4.70 (m, 4H), 4.11 - 4.04 (m, 1H), 3.97 - 3.78 (m, 3H), 3.35 (s, 1H), 3.25 - 3.20 (m, 1H), 2.98 - 2.88 (m, 1H), 2.82 - 2.76 (m, 1H), 2.02 - 1.93 (m, 1H), 1.90 - 1.80 (m, 1H), 1.77 - 1.63 (m, 4H), 1.40 - 1.31 (m, 1H) ppm |
| 308 | 555.2 | 1H NMR (400 MHz, DMSO-d6) δ = 9.47 (s, 1H), 9.41 - 7.38 (m, 1H), 8.75 (d, J = 8.8 Hz, 1H), 8.64 (d, J = 8.8 Hz, 1H), 8.58 (d, J = 4.8 Hz, 1H), 8.27 (s, 1H), 7.97 (d, J = 8.0 Hz, 1H), 7.85 (s, 1H), 7.69 (d, J = 4.8 Hz, 1H), 7.31 (d, J = 8.0 Hz, 1H), 5.24 - 5.01 (m, 2H), 4.93 - 4.69 (m, 4H), 4.11 - 4.04 (m, 1H), 4.00 - 3.76 (m, 3H), 3.37 - 3.34 (m, 1H), 3.25 - 3.20 (m, 1H), 2.97 - 2.88 (m, 1H), 2.82 - 2.76 (m, 1H), 1.98 - 1.96 (m, 1H), 1.92 - 1.80 (m, 1H), 1.75 - 1.65 (m, 4H), 1.36 - 1.32 (m, 1H) ppm |
| 309 | 526.4 | 1H NMR (400 MHz, MeOD) δ = 9.40 (s, 1H), 9.12 (d, J = 7.6 Hz, 1H), 8.86 (d, J = 8.8 Hz, 1H), 8.71 (d, J = 8.8 Hz, 1H), 8.48 - 8.38 (m, 2H), 8.19 (d, J = 1.6 Hz, 1H), 8.04 (s, 1H), 7.95 - 7.92 (m, 1H), 7.49 - 7.14 (m, 2H), 4.92 (d, J = 3.6 Hz, 2H), 4.58 (s, 2H), 4.21 (d, J = 11.2 Hz, 1H), 3.94 (d, J = 11.2 Hz, 1H), 1.75 (s, 3H) ppm |
| 310 | 534.5 | 1H NMR (400 MHz, DMSO-d6) δ = 9.47 (s, 1H), 9.43 - 9.39 (m, 1H), 8.79 - 8.72 (m, 1H), 8.65 (br s, 1H), 8.57 (d, J = 5.2 Hz, 1H), 8.20 (d, J = 1.6 Hz, 1H), 7.96 - 7.94 (m, 1H), 7.87 (s, 1H), 7.71 (d, J = 4.8 Hz, 1H), 7.31 (d, J = 8.0 Hz, 1H), 5.32 - 4.96 (m, 1H), 4.95 - 4.69 (m, 5H), 4.23 (d, J = 11.2 Hz, 1H), 3.87 (br d, J = 11.2 Hz, 2H), 3.78 - 3.44 (m, 3H), 2.02 - 1.96 (m, 1H), 1.95 - 1.87 (m, 1H), 1.70 (s, 3H) ppm |
| 311 | 524.5 | 1H NMR (400 MHz, DMSO-d6) δ = 9.46 - 9.36 (m, 2H), 8.93 (s, 1H), 8.72 (d, J = 8.8 Hz, 1H), 8.59 (d, J = 8.4 Hz, 1H), 8.24 - 8.13 (m, 2H), 7.97 - 7.94 (m, 1H), 7.86 (s, 1H), 7.31 (d, J = 8.4 Hz, 1H), 5.64 - 5.44 (m, 1H), 4.96 - 4.76 (m, 4H), 4.23 (d, J = 11.2 Hz, 1H), 4.03 - 3.69 (m, 4H), 3.64 - 3.59 (m, 1H), 2.34 - 2.12 (m, 2H), 1.71 (s, 3H) ppm |
| 316 | 580.25 | ¹H NMR (400 MHz, DMSO-d₆) δ 9.51 - 9.34 (m, 2H), 8.83 - 8.65 (m, 3H), 8.44 (dt, J = 8.0, 1.4 Hz, 1H), 8.35 (d, *J* = 8.7 Hz, 1H), 8.21 (d, *J* = 1.8 Hz, 1H), 8.07 - 7.91 (m, 2H), 7.85 (s, 1H), 7.66 (t, *J* = 7.8 Hz, 1H), 7.32 (d, *J =* 8.1 Hz, 1H), 4.99 - 4.75 (m, 4H), 4.23 (d, *J* = 11.4 Hz, 1H), 3.87 (d, *J =* 11.5 Hz, 1H), 3.65 - 3.50 (m, 4H), 3.51 - 3.41 (m, 4H), 3.23 (s, 3H), 1.71 (s, 3H). |
| 317 | 575.35 | ¹H NMR (400 MHz, DMSO-d₆) δ 9.42 (d, *J* = 3.2 Hz, 2H), 8.77 - 8.64 (m, 2H), 8.55 - 8.40 (m, 2H), 8.35 (d, *J* = 8.7 Hz, 1H), 8.21 (d, *J* = 1.7 Hz, 1H), 8.07 - 7.92 (m, 2H), 7.85 (s, 1H), 7.65 (t, *J* = 7.8 Hz, 1H), 7.32 (d, *J =* 8.1 Hz, 1H), 5.02 - 4.72 (m, 4H), 4.23 (d, *J* = 11.4 Hz, 1H), 3.95 - 3.72 (m, 2H), 2.83 (d, *J* = 11.1 Hz, 2H), 2.21 (s, 3H), 2.04 (q, *J* = 11.9, 8.9 Hz, 2H), 1.87 - 1.76 (m, 2H), 1.71 (s, 3H), 1.63 (qd, J = 12.1, 3.9 Hz, 2H). |
| 318 | 510 | 1H NMR (400 MHz, CHLOROFORM-d) δ ppm 1.71 - 1.81 (m, 3 H) 3.88 (dd, J=17.31, 12.02 Hz, 1 H) 4.09 (t, J=11.69 Hz, 1 H) 4.67 (td, J=13.06, 4.52 Hz, 2 H) 4.75 - 4.94 (m, 2 H) 5.01 (d, J=5.29 Hz, 2 H) 6.11 - 6.52 (m, 1 H) 7.11 - 7.20 (m, 2 H) 7.27 (s, 8 H) 7.36 (br s, 1 H) 7.84 (d, J=8.16 Hz, 1 H) 7.98 (s, 1 H) 8.11 (s, 1 H) 8.39 (s, 2 H) 8.65 (d, J=9.92 Hz, 1 H) 9.30 (s, 1 H) ppm. |
| 319 | 515.2 | 1H NMR (400 MHz, CDCl3) δ = 9.27-9.33 (m, 1H), 9.18-9.23 (m, 1H), 8.55 (d, J=8.4 Hz, 1H), 8.40 (d, J=8.4 Hz, 1H), 8.26-8.31 (m, 1H), 8.12 (s, 1H), 8.02-8.07 (m, 1H), 7.83-7.89 (m, 1H), 7.34-7.38 (m, 1H), 7.19 (s, 1H), 4.99-5.05 (m, 2H), 4.76-4.93 (m, 2H), 4.06-4.14 (m, 1H), 3.89-3.95 (m, 4H), 3.82-3.89 (m, 1H), 3.69-3.75 (m, 4H), 1.83 (s, 3H) ppm |
| 320 | 534.1 | 1HNMR (400 MHz, DMSO-d6) δ = 9.70 - 9.63 (m, 1H), 9.61 - 9.54 (m, 1H), 8.79 - 8.71 (m, 1H), 8.27 - 8.22 (m, 1H), 8.22 - 8.19 (m, 1H), 8.11 - 8.02 (m, 1H), 7.99 - 7.90 (m, 1H), 7.65 - 7.50 (m, 1H), 7.36 - 7.25 (m, 1H), 7.11 - 7.00 (m, 1H), 4.95 - 4.79 (m, 5H), 4.42 - 4.34 (m, 2H), 4.26 - 4.18 (m, 1H), 3.97 - 3.90 (m, 2H), 3.86 (d, J = 11.6 Hz, 1H), 2.28 (s, 3H), 1.70 (s, 3H) ppm. |
| 321 | 518.4 | 1H NMR (400 MHz, DMSO-d6) δ = 9.46 (s, 1H), 9.42 - 9.39 (m, 1H), 8.75 (d, J = 8.4 Hz, 1H), 8.63 (d, J = 8.4 Hz, 1H), 8.53 (d, J = 4.8 Hz, 1H), 8.20 (d, J = 1.6 Hz, 1H), 7.96 - 7.93 (m, 1H), 7.86 (s, 1H), 7.68 (d, J = 5.2 Hz, 1H), 7.31 (d, J = 8.4 Hz, 1H), 4.95 - 4.80 (m, 4H), 4.23 (d, J = 11.6 Hz, 1H), 3.87 (d, J = 11.2 Hz, 3H), 3.57 (br d, J = 3.2 Hz, 2H), 1.70 (s, 5H), 0.8 - 0.75 (m, 1H), 0.22 - 0.19 (m, 1H) ppm. |
| 322 | 536.4 | 1H NMR (400 MHz, DMSO-d6) δ = 9.53 - 9.36 (m, 2H), 8.76 (d, J = 8.4 Hz, 1H), 8.66 (d, J = 8.8 Hz, 1H), 8.60 (d, J = 4.8 Hz, 1H), 8.20 (s, 1H), 7.95 (d, J = 7.6 Hz, 1H), 7.87 (s, 1H), 7.73 (d, J = 4.8 Hz, 1H), 7.31 (d, J = 8.0 Hz, 1H), 4.96 - 4.78 (m, 4H), 4.70 - 4.52 (m, 2H), 4.23 (d, J = 11.6 Hz, 1H), 3.95 (d, J = 11.2 Hz, 1H), 3.87 (d, J = 11.2 Hz, 1H), 3.62 - 3.51 (m, 2H), 3.09 - 3.01 (m, 1H), 2.76 - 2.67 (m, 1H), 1.70 (s, 3H), 1.20 (d, J = 6.4 Hz, 3H) ppm |
| 323 | 536.2 | 1H NMR (400 MHz, METHANOL-d4) δ = 9.32 (s, 1H), 8.62 (d, J = 8.8 Hz, 1H), 8.31 (d, J = 8.4 Hz, 2H), 8.22 (d, J = 8.4 Hz, 1H), 8.18 (d, J = 1.6 Hz, 1H), 8.06 - 7.97 (m, 3H), 7.94-7.92 (m, 1H), 7.29 (d, J = 8.0 Hz, 1H), 4.90 - 4.90 (m, 4H), 4.21 (d, J = 11.6 Hz, 1H), 3.93 (d, J = 11.2 Hz, 1H), 3.59 (s, 4H), 3.39 (s, 3H), 1.76 (s, 3H) ppm |
| 324 | 492.2 | 1H NMR (400 MHz, METHANOL-d4) δ = 9.33 (s, 1H), 8.62 (d, J = 8.8 Hz, 1H), 8.32 - 8.29 (m, 2H), 8.22 (d, J = 8.4 Hz, 1H), 8.18 (d, J = 1.6 Hz, 1H), 7.99 - 7.97 (m, 3H), 7.94-7.92 (m, 1H), 7.29 (d, J = 8.0 Hz, 1H), 4.92 - 4.91 (m, 4H), 4.21 (d, J = 11.2 Hz, 1H), 3.93 (d, J = 11.6 Hz, 1H), 2.95 (s, 3H), 1.76 (s, 3H) ppm |
| 325 | 470.2 | 1H NMR (400 MHz, DMSO-d6) δ = 9.43 (s, 2H), 9.42 (br s, 1H), 8.77 (s, 1H), 8.74(d, J = 8.4 Hz, 1H), 8.54 (d, J = 8.4 Hz, 1H), 8.27 (s, 1H), 7.98-7.96 (m, 1H), 7.85 (s, 1H), 7.31 (d, J = 8.0 Hz, 1H), 4.88-4.81 (m, 3H), 4.75-4.74(m, 1H), 4.11-4.04 (m, 1H), 3.88-3.79 (m, 1H), 2.35-2.32 (m, 1H), 1.71-1.66 (m, 3H), 1.15-1.13 (m, 4H) ppm. |
| 329 | 469.2 | 1H NMR (400 MHz, MeOD) δ = 8.31 - 8.30 (m, 1H), 8.21-8.19 (m, 2H), 8.15 (d, J = 1.6 Hz, 1H), 8.06 (d, J = 5.2 Hz, 2H), 7.90-7.89 (m, 1H), 7.28 (d, J = 8.0 Hz, 1H), 5.09 (s, 2H), 4.90 (s, 2H), 4.20 (d, J = 11.2 Hz, 1H), 4.03 (s, 3H), 3.93 (d, J = 11.6 Hz, 1H), 3.82 - 3.81 (m, 3H), 1.75 (s, 3H) ppm |
| 333 | 480.2 | 1H NMR (400 MHz, DMSO-d6) δ = 9.46-9.43 (m, 1H), 9.20 (s, 1H), 8.46 (d, J=0.4, 1H), 8.35 (s, 1H), 8.16 (s, 1H), 8.00-7.96(m, 1H), 7.79 (d, J = 10.4 Hz, 1H), 7.57 (s, 1H), 4.95 (d, J = 16.8 Hz, 1H), 4.77 - 4.71 (m, 3H), 4.16 - 4.04 (m,1H), 3.97 (s, 3H), 3.90 - 3.78 (m, 1H), 3.76 (s, 3H), 1.75 - 1.61 (m, 3H) ppm. |
| 334 | 463.2 | 1H NMR (400 MHz, DMSO-d₆) δ = 9.54-9.52 (m, 1H), 9.21 (s, 1H), 9.09-9.08 (m, 1H), 8.62 (s, 1H), 8.46-8.42 (m, 1H), 8.35 (s, 1H), 8.00 (d, J = 8.8 Hz, 1H), 7.60 (s, 1H), 4.86 - 4.74 (m, 4H), 4.16 - 4.09 (m, 1H), 3.98 (s, 3H), 3.93 - 3.83(m, 1H), 3.78 (s, 3H), 1.71 (d, J=21.2, 3H) ppm |
| 335 | 492.2 | 1H NMR (400 MHz, DMSO-d6) δ = 9.49-9.46 (m, 1H), 8.99 (d, J = 1.6 Hz, 1H), 8.81-8.78 (m, 1H), 8.55 (d, J = 9.2 Hz, 1H), 8.36 (d, J = 5.6 Hz, 1H), 8.31 (s, 1H), 8.17 (d, J = 1.6 Hz, 1H), 7.94-7.92 (m, 1H), 7.30 (d, J = 8.0 Hz, 1H), 6.41 (d, J =6.0 Hz, 1H), 5.06 (d, J = 5.6 Hz, 2H), 4.92-4.81 (m, 2H), 4.23-4.14 (m, 5H), 3.87 (d, J = 11.6 Hz, 1H), 2.45-2.40 (m, 2H), 1.69 (s, 3H) ppm. |
| 336 | 492.2 | 1H NMR (400 MHz, DMSO-d6) δ = 9.52-9.49 (m, 1H), 8.84 (s, 1H), 8.59-8.58 (m, 2H), 8.51 (d, J = 5.2 Hz, 1H), 8.23 (s, 1H), 8.18 (d, J = 1.6 Hz, 1H), 7.95-7.92 (m, 1H), 7.42 (d, J = 5.2 Hz, 1H), 7.30 (d, J = 8.0 Hz, 1H), 5.09 (d, J = 6.0 Hz, 2H), 4.92-4.81 (m, 2H), 4.23 (d, J = 11.6 Hz, 1H), 4.16-4.12 (m, 4H), 3.87 (d, J = 11.2 Hz, 1H), 2.37-2.33(m, 2H), 1.68(s, 3H) ppm. |
| 337 | 477.2 | 1H NMR (400 MHz, DMSO-d6) δ = 9.72 (d, J = 2.4 Hz, 1H), 9.46 (s, 1H), 9.42-9.40 (m, 1H), 9.21 (d, J = 1.2 Hz, 1H), 8.82 (d, J = 5.2 Hz, 1H), 8.19 (d, J = 1.2 Hz, 1H), 8.07 (d, J = 5.2 Hz, 1H), 7.97-7.94 (m, 2H), 7.31 (d, J = 8.0 Hz, 1H), 4.97 93-4.81(m, 4H), 4.24 (d, J = 11.6 Hz, 1H), 3.88 (d, J = 11.6 Hz, 1H), 2.34-2.30 (m, 1H), 1.69 (s, 3H), 1.15-1.12 (m, 4H) ppm. |
| 338 | 462.2 | 1HNMR (400 MHz, DMSO-d6) δ = 9.33-9.30 (m, 1H), 9.26 - 9.20 (m, 2H), 8.43 - 8.35 (m, 2H), 8.25 (s, 1H), 7.96(d, J = 8.0 Hz, 1H), 7.71 (s, 1H), 7.29 (d, J = 8.0 Hz, 1H), 4.88 - 4.82 (m, 1H), 4.76 - 4.70 (m, 3H), 4.10 - 4.04 (m, 1H), 3.96 (s,3H), 3.88 - 3.80 (m, 1H), 3.77 (s, 3H), 1.71 - 1.61 (m, 3H) ppm. |
| 339 | 536.3 | 1H NMR (400 MHz, DMSO-d6) δ = 9.47 (s, 1H), 9.44 - 9.40 (m, 1H), 8.79 - 8.74 (m, 1H), 8.67 (d, J = 8.8 Hz, 1H), 8.57 (d, J = 4.8 Hz, 1H), 8.49 (s, 1H), 8.21 (d, J = 1.2 Hz, 1H), 7.97 - 7.95 (m, 1H), 7.87 (s, 1H), 7.68 (d, J = 5.2 Hz, 1H), 7.32 (d, J = 8.4 Hz, 1H), 4.96 - 4.81 (m, 4H), 4.24 (d, J = 11.6 Hz, 1H), 4.12 (br s, 1H), 3.90 - 3.51 (m, 5H), 3.30 (s, 3H), 2.16 - 2.07 (m, 2H), 1.71 (s, 3H) ppm |
| 340 | 534.3 | 1H NMR (400 MHz, DMSO-d6) δ = 9.47 (s, 1H), 9.43 - 9.39 (m, 1H), 8.78 - 8.73 (m, 1H), 8.70 - 8.60 (m, 1H), 8.57 (d, J = 5.2 Hz, 1H), 8.20 (d, J = 1.6 Hz, 1H), 7.97 - 7.94 (m, 1H), 7.87 (s, 1H), 7.71 (d, J = 5.2 Hz, 1H), 7.31 (d, J = 8.0 Hz, 1H), 4.95 - 4.70 (m, 6H), 4.23 (d, J = 11.2 Hz, 1H), 3.90 - 3.84 (m, 2H), 3.77 - 3.49 (m, 3H), 2.00 - 1.89 (m, 2H), 1.70 (s, 3H) ppm |
| 341 | 524.2 | 1H NMR (400 MHz, DMSO-d6) δ = 9.47 (s, 1H), 9.43 - 9.39 (m, 1H), 8.80 - 8.74 (m, 1H), 8.67 (d, J = 8.4 Hz, 1H), 8.59 (d, J = 4.8 Hz, 1H), 8.20 (d, J = 1.6 Hz, 1H), 7.97 - 7.87 (m, 1H), 7.87 (s, 1H), 7.72 (d, J = 4.8 Hz, 1H), 7.31 (d, J = 8.4 Hz, 1H), 5.60 - 5.39 (m, 1H), 4.95 - 4.80 (m, 4H), 4.23 (d, J = 11.2 Hz, 1H), 4.09 - 3.54 (m, 5H), 2.32 - 2.12 (m, 2H), 1.71 (s, 3H) ppm |
| 342 | 477.2 | 1H NMR (400 MHz, DMSO-d6) δ = 9.67 (d, J = 2.0 Hz, 1H), 9.44 (s, 1H), 9.42-9.41 (m, 1H), 9.22 (s, 1H), 9.13 (d, J = 1.6 Hz, 1H), 8.71 (s, 1H), 8.20 (d, J = 1.6 Hz, 1H), 7.97-7.95 (m, 1H), 7.92 (s, 1H), 7.32 (d, J = 8.0 Hz, 1H), 4.93-4.81 (m, 4H), 4.24 (d, J = 11.6 Hz, 1H), 3.88 (d, J = 11.6 Hz, 1H), 2.34 - 2.31 (m, 1H), 1.70 (s, 3H), 1.19-1.12 (m, 4H) ppm. |
| 343 | 462.2 | 1H NMR (400 MHz, DMSO-d6) δ = 9.39-9.36 (m, 1H), 9.20 (s, 1H), 8.46 - 8.44 (m, 2H), 8.36 (s, 1H), 8.28 (s, 1H), 8.00 -7.96 (m, 2H), 7.55 (s, 1H), 7.31 (d, J = 8.4 Hz, 1H), 4.88 - 4.84 (m, 1H), 4.76 - 4.71 (m, 3H), 4.11-4.04 (m, 1H), 3.97 (s,3H), 3.69 - 3.79 (m, 1H), 3.75 (s, 3H), 168 (d, J=12.4, 3H) ppm. |
| 344 | 473.1 | 1H NMR (400 MHz, DMSO-d6) δ = 9.42 (s, 1H), 9.37-9.34 (m, 1H), 9.09 (s, 1H), 8.58 (s, 1H), 8.18 (d, J = 1.2 Hz, 1H), 8.00 (s, 1H), 7.95-7.92 (m, 1H), 7.30 (d, J = 8.4 Hz, 1H), 4.94 - 4.80 (m, 2H), 4.77 (d, J = 6.0 Hz, 2H), 4.22 (d, J = 11.6 Hz, 1H), 4.07 (s, 3H), 3.86 (d, J = 11.6 Hz, 1H), 1.70 (s, 3H) ppm |
| 345 | 491.2 | 1H NMR (400 MHz, MeOD) δ = 9.21 (s, 1H), 8.42 (d, J = 8.8 Hz, 1H), 8.24 (s, 1H), 7.96 -7.92(m, 2H), 7.86-7.85 (m, 1H), 7.28 (d, J = 8.0 Hz, 1H), 7.10 (d, J = 1.6 Hz, 1H), 4.88 (s, 3H), 4.81-4.76(m, 1H), 4.61- 4.50 (m, 2H), 4.10-4.04 (m, 1H), 3.92 - 3.84 (m, 1H), 3.73 (s, 3H), 3.66-3.63(m, 2H), 2.47-2.46 (m, 2H), 1.76 - 1.70 (m, 3H) ppm. |
| 346 | 490 | 1H NMR (400 MHz, DMSO-d6) δ = 9.63 (s, 1H), 9.42 (s, 2H), 8.73 (s, 2H), 8.21 (d, J = 1.6 Hz, 1H), 8.09 (d, J = 3.2 Hz, 1H), 8.01 - 7.93 (m, 1H), 7.88 (s, 1H), 7.31 (d, J = 8.0 Hz, 1H), 6.76 (d, J = 3.2 Hz, 1H), 4.97 - 4.81 (m, 4H), 4.23 (d, J = 11.6 Hz, 1H), 3.97 (s, 3H), 3.87 (d, J = 11.6 Hz, 1H), 1.71 (s, 3H) ppm. |
| 347 | 484.2 | 1H NMR (400 MHz, MeOH) δ = 9.63 (s, 1H), 9.36 (s, 1H), 8.85 (s, 1H), 8.23 (s, 1H), 7.93-7.91 (m, 2H), 7.66-7.63(m, 1H), 7.36 (d, J = 7.6 Hz, 1H), 7.27 (d, J = 8.0 Hz, 1H), 6.45(d, J = 8.4 Hz, 1H), 4.90 (s, 3H), 4.80 (d, J = 3.2 Hz, 1H), 4.14-4.03 (m, 5H), 3.91-3.86 (m, 1H), 2.46-2.42 (m, 2H), 1.75-1.69 (m, 3H) ppm. |
| 348 | 498.1 | 1H NMR (400 MHz, DMSO-d6) δ = 9.41 (s, 1H), 9.36 - 9.33 (m, 1H), 8.61 (d, J = 4.8 Hz, 1H), 8.17 (d, J = 1.6 Hz, 1H), 7.98 (s, 1H), 7.93 - 7.90 (m, 1H), 7.46 (d, J = 4.8 Hz, 1H), 7.29 (d, J = 8.0 Hz, 1H), 4.93 - 4.80 (m, 2H), 4.76 (d, J = 6.0 Hz, 2H), 4.22 (d, J = 11.2 Hz, 1H), 4.17 - 4.13 (m, 4H), 3.86 (d, J = 11.6 Hz, 1H), 2.41 - 2.34 (m, 2H), 1.69 (s, 3H) ppm |
| 349 | 467.1 | 1H NMR (400 MHz, MeOD-d4) δ = 8.90 (s, 1H), 8.77 (s, 1H), 8.66 (d, J = 8.8 Hz, 1H), 8.56 (d, J = 8.8 Hz, 1H), 8.28-8.26 (m, 2H), 8.15 (s, 1H), 7.90 (d, J = 8.0 Hz, 1H), 7.27 (d, J = 8.0 Hz, 1H), 5.17 (s, 2H), 4.92-4.81 (m, 2H), 4.20 (d, J = 11.2 Hz, 1H), 4.13 (s, 3H), 3.92 (d, J = 11.2 Hz, 1H), 1.74 (s, 3H) ppm |
| 351 | 491 | 1H NMR (400 MHz, MeOD-d4) δ = 8.61-8.60 (m, 2H), 8.50-8.49 (m, 1H), 8.25 (s, 1H), 8.17 (s, 1H), 7.91 (d, J = 8.0 Hz, 1H), 7.68-7.64 (m, 1H), 7.38 (d, J = 7.6 Hz, 1H), 7.30-7.28 (m, 1H), 7.65 (d, J = 8.4 Hz, 1H), 5.17 (s, 2H), 4.92-4.81 (m, 2H), 4.20 (d, J = 11.2 Hz, 1H), 4.16-4.12 (m, 4H), 3.92 (d, J = 11.2 Hz, 1H), 2.49-2.42 (m, 2H), 1.76 (s, 3H) ppm |
| 352 | 539.1 | 1H NMR (400 MHz, DMSO-d6) δ = 9.49-9.46(m, 1H), 9.42 (s, 1H), 8.93 (s, 1H), 8.73 (d, J = 8.4 Hz, 1H),8.52 (d, J = 8.8Hz, 1H), 8.14(s, 1H), 8.01 (s, 1H), 7.96 (d, J = 10.4 Hz, 1H), 7.87 (s, 1H), 6.88-6.59 (m, 1H), 5.00-4.96 (m, 1H), 4.86-4.82(m, 3H), 4.30-4.20 (m, 1H), 4.18-4.16 (m, 4H), 4.12 (s, 1H), 2.46-2.43 (m, 2H) ppm. |
| 353 | 498.2 | 1H NMR (400 MHz, DMSO-d6) δ = 9.44 - 9.29 (m, 2H), 8.68 (s, 1H), 8.17 (d, J = 1.2 Hz, 1H), 8.08 (s, 1H), 7.98 - 7.90 (m, 2H), 7.29 (d, J = 8.4 Hz, 1H), 4.95 - 4.80 (m, 2H), 4.75 (d, J = 6.0 Hz, 2H), 4.26 - 4.12 (m, 5H), 3.86 (d, J = 11.6 Hz, 1H), 2.46 - 2.40 (m, 2H), 1.70 (s, 3H) ppm |
| 354 | 472 | 1H NMR (400 MHz, DMSO-d6) δ = 9.38 (s, 1H), 9.36 - 9.33 (m, 1H), 8.82 - 8.79 (m, 1H), 8.47 - 8.45 (m, 1H), 8.40 (s, 1H), 8.17 (d, J = 1.2 Hz, 1H), 7.96 - 7.90 (m, 2H), 7.33 - 7.25 (m, 2H), 4.93 - 4.81 (m, 2H), 4.76 (br d, J = 6.0 Hz, 2H), 4.22 (d, J = 11.6 Hz, 1H), 4.18 (s, 3H), 3.86 (d, J = 11.6 Hz, 1H), 1.70 (s, 3H) ppm |
| 355 | 487 | 1H NMR (400 MHz, DMSO-d6) δ = 9.90 (s, 1H), 9.50 (s, 1H), 9.45 - 9.42 (m, 1H), 9.14 (s, 1H), 8.82 (d, J = 8.4 Hz, 1H), 8.59 (d, J = 8.4 Hz, 1H), 8.20 (d, J = 1.2 Hz, 1H), 8.02 - 7.89 (m, 2H), 7.44 - 7.11 (m, 2H), 4.96 - 4.78 (m, 4H), 4.23 (d, J = 11.2 Hz, 1H), 3.87 (d, J = 11.6 Hz, 1H), 1.71 (s, 3H) ppm |
| 356 | 492 | 1H NMR (400 MHz, MeOD-d4) δ = 8.68 (s, 1H), 8.62-8.61 (m, 1H), 8.55-8.52 (m, 2H), 8.27 (s, 1H), 8.17 (s, 1H), 7.93-7.92 (m, 1H), 7.83 (s, 1H), 7.29 (d, J = 8.0 Hz, 1H), 5.18 (s, 2H), 4.92-4.81 (m, 2H), 4.27-4.19 (m, 5H), 3.92 (d, J = 11.2 Hz, 1H), 2.58-2.50 (m, 2H), 1.77 (s, 3H) ppm |
| 357 | 466 | 1H NMR (400 MHz, MeOD-d4) δ = 8.48-8.46 (m, 2H), 8.25-8.16 (m, 5H), 7.92-7.90 (m, 2H), 7.29-7.27 (m, 1H), 7.15-7.13 (m, 1H), 5.17 (s, 2H), 4.92-4.81 (m, 2H), 4.20 (d, J = 11.2 Hz, 1H), 4.00 (s, 3H), 3.92 (d, J = 11.2 Hz, 1H), 1.76 (s, 3H) ppm |
| 358 | 487 | 1H NMR (400 MHz, MeOD) δ = 9.32 (s, 1H), 9.02 (d, J = 5.2 Hz, 1H), 8.71-8.63 (m, 3H), 8.07 (d, J = 1.6 Hz, 1H), 7.93 (s, 1H), 7.84-7.82 (m, 1H), 7.21 (d, J = 8.4 Hz, 1H), 6.91-6.64 (m, 1H), 4.85 (s, 2H), 4.82 (d, J = 3.2 Hz, 2H), 4.12 (d, J = 11.6 Hz, 1H), 3.84 (d, J = 11.6 Hz, 1H), 1.67 (s, 3H) ppm |
| 359 | 505.2 | 1H NMR (400 MHz, MeOD) δ = 9.36 (s, 1H), 8.88 (d, J = 8.8 Hz, 1H), 8.69 (d, J = 8.4 Hz, 1H), 8.58 (d, J = 8.0 Hz, 1H), 8.52 (br s, 1H), 8.18 (d, J = 1.6 Hz, 1H), 8.00 - 7.91 (m, 3H), 7.45 (d, J = 6.8 Hz, 1H), 7.30 (d, J = 8.0 Hz, 1H), 4.93 (s, 2H), 4.91 (d, J = 3.2 Hz, 2H), 4.42 -4.37(m, 4H), 4.29-4.26 (m, 1H), 4.22 (d, J = 11.2 Hz, 1H), 3.94 (d, J = 11.6 Hz, 1H), 2.98 (s, 3H), 1.76 (s, 3H) ppm. |
| 360 | 479.2 | 1H NMR (400 MHz, MeOD) δ = 9.27 (s, 1H), 8.68 (d, J = 8.4 Hz, 1H), 8.55 (d, J = 8.8 Hz, 1H), 8.17(d, J = 1.6 Hz, 1H), 7.94-7.91 (m, 2H), 7.81 (d, J = 7.2 Hz, 1H), 7.67-7.65 (m, 1H), 7.29 (d, J = 8.0 Hz, 1H), 6.77 (d, J = 8.4 Hz, 1H), 4.91-4.90 (m, 4H), 4.21 (d, J = 11.2 Hz, 1H), 3.93 (d, J = 11.6 Hz, 1H), 3.18 (s, 6H), 1.76 (s, 3H) ppm. |
| 361 | 505.1 | 1H NMR (400 MHz, MeOD) δ = 9.31 (s, 1H), 8.72 (d, J = 8.8 Hz, 1H), 8.58 (d, J = 8.4 Hz, 1H), 8.20 (d, J = 1.6 Hz, 1H), 8.01 - 7.93 (m, 2H), 7.80 (d, J = 7.2 Hz, 1H), 7.67 - 7.64 (m, 1H), 7.31 (d, J = 8.0 Hz, 1H), 6.62 (d, J = 8.4 Hz, 1H), 4.95 - 4.92 (m, 4H), 4.23 (d, J = 11.2 Hz, 1H), 3.95 (d, J = 11.2 Hz, 1H), 3.61 - 3.57 (m, 4H), 2.17 - 2.05 (m, 4H), 1.79 (s, 3H) ppm |
| 362 | 536.3 | 1H NMR (400 MHz, METHANOL-d4) δ = 9.48 (d, J = 0.8 Hz, 1H), 9.43-9.40 (m, 1H), 8.77-8.75 (m, 1H), 8.66 (d, J = 8.8 Hz, 1H), 8.61 (d, J = 4.8 Hz, 1H), 8.20 (d, J = 1.6 Hz, 1H), 7.96-7.94 (m, 1H), 7.87 (s, 1H), 7.73 (d, J = 5.2 Hz, 1H), 7.32 (d, J = 8.0 Hz, 1H), 4.93 - 4.82 (m, 4H), 4.67 - 4.58 (m, 2H), 4.23 (d, J = 11.6 Hz, 1H), 3.97-3.94 (m, 1H), 3.88 (d, J = 11.2 Hz, 1H), 3.61 - 3.52 (m, 2H), 3.09 - 3.02 (m, 1H), 2.76-2.70 (m, 1H), 1.70 (s, 3H), 1.21 (d, J = 6.4 Hz, 3H) ppm |
| 363 | 536.2 | 1H NMR (400 MHz, DMSO-d6) δ= 9.47 (s, 1H), 9.45 - 9.35 (m, 1H), 8.76 (d, J = 8.8 Hz, 1H), 8.68 - 8.56 (m, 2H), 8.20 (d, J = 1.2 Hz, 1H), 8.02 - 7.92 (m, 1H), 7.87 (s, 1H), 7.73 (d, J = 4.8 Hz, 1H), 7.31 (d, J = 8.0 Hz, 1H), 4.96 - 4.74 (m, 5H), 4.44 (br d, J = 12.0 Hz, 1H), 4.23 (d, J = 11.6 Hz, 1H), 4.05 - 3.92 (m, 1H), 3.87 (d, J = 11.6 Hz, 1H), 3.81 - 3.73 (m, 1H), 3.69 - 3.61 (m, 1H), 3.56 - 3.43 (m, 1H), 3.28 - 3.20 (m, 1H), 1.70 (s, 3H), 1.25 (d, J = 6.4 Hz, 3H) ppm. |
| 364 | 457.2 | 1H NMR (400 MHz, DMSO-d6) δ 9.41 (t, J = 6.0 Hz, 1H), 9.33 (s, 1H), 8.62 - 8.51 (m, 2H), 8.24 (d, J = 1.8 Hz, 1H), 7.99 (dd, J = 8.1, 1.8 Hz, 1H), 7.85 (d, J = 8.6 Hz, 1H), 7.69 (s, 1H), 7.35 (d, J = 8.1 Hz, 1H), 4.95 (d, J = 16.0 Hz, 1H), 4.88 (d, J = 16.1 Hz, 1H), 4.82 (d, J = 5.8 Hz, 2H), 4.27 (d, J = 11.4 Hz, 1H), 3.91 (d, J = 11.5 Hz, 1H), 3.85 (s, 3H), 1.74 (s, 3H). |
| 365 | 491.3 | 1H NMR (400 MHz, DMSO-d6) δ 9.39 (d, J = 11.6 Hz, 2H), 8.74 (s, 1H), 8.51 (d, J = 5.2 Hz, 1H), 8.39 (dd, J = 8.5, 1.7 Hz, 1H), 8.27 (d, J = 8.7 Hz, 1H), 8.23 (d, J = 1.7 Hz, 1H), 7.99 (dd, J = 8.1, 1.7 Hz, 1H), 7.89 (s, 1H), 7.45 (d, J = 5.2 Hz, 1H), 7.34 (d, J = 8.1 Hz, 1H), 4.99 - 4.79 (m, 4H), 4.26 (d, J = 11.4 Hz, 1H), 4.18 (t, J = 7.5 Hz, 4H), 3.91 (d, J = 11.6 Hz, 1H), 2.40 (d, J = 7.6 Hz, 1H), 1.74 (s, 3H). |
| 366 | 535.4 | 1H NMR (400 MHz, DMSO-d6) δ 9.37 (d, J = 5.0 Hz, 2H), 8.71 (s, 1H), 8.61 (s, 1H), 8.40 (dd, J = 8.7, 1.6 Hz, 1H), 8.29 - 8.20 (m, 2H), 8.06 (s, 1H), 7.99 (dd, J = 8.0, 1.8 Hz, 1H), 7.87 (s, 1H), 7.34 (d, J = 8.1 Hz, 1H), 4.95 (d, J = 16.0 Hz, 1H), 4.88 (d, J = 15.9 Hz, 1H), 4.82 (d, J = 5.8 Hz, 2H), 4.35 (d, J = 4.3 Hz, 0H), 4.26 (d, J = 11.4 Hz, 1H), 4.18 (s, 1H), 3.91 (d, J = 11.5 Hz, 1H), 3.73 (d, J = 11.6 Hz, 2H), 3.68 (dd, J = 11.7, 4.3 Hz, 1H), 3.57 (q, J = 8.8 Hz, 1H), 2.15 (td, J = 8.7, 8.3, 4.3 Hz, 2H), 1.74 (s, 3H). |
| 367 | 468.2 | 1H NMR (400 MHz, DMSO-d₆) δ 9.31 (t, J = 5.9 Hz, 1H), 9.21 (s, 1H), 8.27 - 8.19 (m, 2H), 8.14 - 8.05 (m, 2H), 7.97 (dd, J = 8.0, 1.7 Hz, 1H), 7.92 (dd, J = 8.6, 1.7 Hz, 1H), 7.64 (s, 1H), 7.33 (d, J = 8.1 Hz, 1H), 4.95 (d, J = 16.0 Hz, 1H), 4.88 (d, J = 16.0 Hz, 1H), 4.84 - 4.73 (m, 2H), 4.26 (d, J = 11.5 Hz, 1H), 3.99 (s, 3H), 3.91 (d, J = 11.6 Hz, 1H), 3.79 (s, 3H), 1.74 (s, 3H). |
| 368 | 483.3 | 1H NMR (500 MHz, DMSO-d6) δ 9.35 (s, 1H), 8.61 (s, 1H), 8.44 (s, 1H), 8.37 (dd, J = 8.7, 1.7 Hz, 1H), 8.30 (s, 1H), 8.21 (d, J = 8.6 Hz, 1H), 8.00 (d, J = 8.2 Hz, 1H), 7.83 (s, 1H), 7.68 (t, J = 7.8 Hz, 1H), 7.45 (d, J = 7.4 Hz, 1H), 7.33 (d, J = 8.0 Hz, 1H), 6.44 (d, J = 8.2 Hz, 1H), 4.90 (d, J = 15.9 Hz, 1H), 4.85 - 4.74 (m, 3H), 4.16 - 4.05 (m, 5H), 3.88 (dd, J = 25.2, 12.5 Hz, 1H), 2.44 - 2.36 (m, 2H), 1.73 (d, J = 20.7 Hz, 3H). |
| 369 | 490.3 | 1H NMR (500 MHz, DMSO-d6) δ 9.42 - 9.33 (m, 2H), 8.61 (s, 1H), 8.42 - 8.34 (m, 3H), 8.26 - 8.18 (m, 3H), 7.98 (dd, J = 8.1, 1.7 Hz, 1H), 7.84 (s, 1H), 7.72 - 7.65 (m, 1H), 7.45 (d, J = 7.5 Hz, 1H), 7.34 (d, J = 8.1 Hz, 1H), 6.64 (s, 1H), 6.44 (d, J = 8.3 Hz, 1H), 4.91 (q, J = 15.9 Hz, 3H), 4.80 (d, J = 6.0 Hz, 2H), 4.26 (d, J = 11.5 Hz, 1H), 4.08 (td, J = 7.4, 4.3 Hz, 5H), 3.91 (d, J = 11.5 Hz, 1H), 1.74 (s, 3H). |
| 370 | 484.3 | 1H NMR (400 MHz, DMSO-d6) δ 9.36 (d, J = 6.3 Hz, 2H), 8.69 - 8.62 (m, 2H), 8.38 - 8.22 (m, 3H), 8.01 (dd, J = 8.0, 1.9 Hz, 1H), 7.94 (d, J = 7.7 Hz, 1H), 7.84 (d, J = 6.4 Hz, 1H), 7.33 (d, J = 8.1 Hz, 1H), 4.94 - 4.72 (m, 4H), 4.18 (dt, J = 12.2, 7.4 Hz, 4H), 4.10 (d, J = 13.0 Hz, 1H), 3.88 (dd, J = 24.9, 12.5 Hz, 1H), 2.45 (ddq, J = 11.3, 7.4, 3.7 Hz, 2H), 1.73 (d, J = 20.7 Hz, 3H) |
| 371 | 491.3 | 1H NMR (400 MHz, DMSO-d6) δ 9.37 (s, 2H), 8.70 - 8.62 (m, 2H), 8.35 (dd, J = 8.6, 1.6 Hz, 1H), 8.29 - 8.20 (m, 2H), 8.02 - 7.90 (m, 2H), 7.86 (s, 1H), 7.34 (d, J = 8.1 Hz, 1H), 4.95 (d, J = 16.0 Hz, 1H), 4.88 (d, J = 15.9 Hz, 1H), 4.81 (d, J = 5.8 Hz, 2H), 4.26 (d, J = 11.5 Hz, 1H), 4.18 (dt, J = 12.5, 7.5 Hz, 5H), 3.91 (d, J = 11.5 Hz, 1H), 2.52 - 2.39 (m, 3H), 1.74 (s, 3H), 1.08 (d, J = 6.1 Hz, 2H). |
| 372 | 492.2 | 1H NMR (400 MHz, DMSO-d6) δ = 9.83 (d, J = 1.6 Hz, 1H), 9.43 (s, 1H), 9.39 - 9.33 (m, 1H), 9.19 (s, 1H), 8.35 (d, J = 6.0 Hz, 1H), 8.18 (s, 1H), 8.01 (s, 1H), 7.94 (d, J = 8.0 Hz, 1H), 7.29 (d, J = 8.0 Hz, 1H), 6.42 (d, J = 6.0 Hz, 1H), 4.95 - 4.84 (m, 2H), 4.80 (d, J = 5.6 Hz, 2H), 4.25 - 4.12 (m, 5H), 3.86 (d, J = 11.6 Hz, 1H), 2.45 - 2.39 (m, 2H), 1.69 (s, 3H) ppm. |
| 373 | 521.1 | 1H NMR (400 MHz, DMSO-d6) δ = 9.48 - 9.36 (m, 2H), 8.72 - 8.58 (m, 2H), 8.21 (s, 1H), 8.04 - 7.90 (m, 2H), 7.85- 7.73 (m, 2H), 7.32 (d, J = 8.0 Hz, 1H), 7.03 (d, J = 8.4 Hz, 1H), 5.00 - 4.78 (m, 4H), 4.24 (d, J = 11.6 Hz, 1H), 3.88 (d, J =11.6 Hz, 1H), 3.82 - 3.71 (m, 4H), 3.65 - 3.55 (m, 4H), 1.71 (s, 3H) ppm |
| 374 | 510 | 1H NMR (400 MHz, CDCl3) δ = 9.28 (s, 1H), 9.20 (s, 1H), 8.57 (d, J = 8.4 Hz, 1H), 8.40 (d, J = 8.4 Hz, 1H), 8.04 -8.03 (m, 2H), 7.96 (s, 1H), 7.83 - 7.81 (m, 1H), 7.48 - 7.44 (m, 1H), 7.16 (d, J = 8.0 Hz, 1H), 5.62 - 5.47 (m, 1H), 5.00 (d, J = 5.2 Hz, 2H), 4.99 - 4.88 (m, 2H), 4.53 - 4.48 (m, 2H), 4.37 - 4.31 (m, 2H), 4.15 (d, J = 11.2 Hz, 1H), 3.96 (d, J = 11.2 Hz, 1H), 1.77 (s, 3H) ppm |
| 375 | 539.5 | 1H NMR (400 MHz, DMSO-d6) δ = 9.50-9.47 (m, 1H), 9.42 (s, 1H), 8.93 (s, 1H), 8.73 (d, J = 8.8 Hz, 1H), 8.52 (d, J = 8.8 Hz, 1H), 8.14 (s, 1H), 8.01 (s, 1H), 7.96 (d, J = 10.4 Hz, 1H), 7.87 (s, 1H), 6.88-6.59 (m, 1H), 5.00-4.96 (m, 1H), 4.86-4.82 (m, 3H), 4.30 - 4.22 (m, 1H), 4.19-4.16 (m, 4H), 4.16 - 4.10 (m, 1H), 2.46-2.42 (m, 2H) ppm. |
| 376 | 536.5 | 1H NMR (400 MHz, DMSO-d6) δ = 9.49 (s, 1H), 9.44 - 9.41 (m, 1H), 8.77 (d, J = 8.8 Hz, 1H), 8.68 - 8.60 (m, 2H), 8.20 (d, J = 1.6 Hz, 1H), 7.96 - 7.93 (m, 1H), 7.89 (s, 1H), 7.72 (d, J = 5.2 Hz, 1H), 7.31 (d, J = 8.0 Hz, 1H), 4.94 - 4.85 (m, 2H), 4.83 (d, J = 6.0 Hz, 2H), 4.78 - 4.78 (m, 1H), 4.44 (d, J = 12.4 Hz, 1H), 4.22 (d, J = 11.2 Hz, 1H), 3.98 - 3.95 (m, 1H), 3.88 (s, 1H), 3.75 (s, 1H), 3.66 - 3.62 (m, 1H), 3.55 - 3.43 (m, 1H), 3.29 - 3.21 (m, 1H), 1.70 (s, 3H), 1.25 (d, J = 6.8 Hz, 3H) ppm. |
| 377 | 492.1 | 1H NMR (400 MHz, DMSO-d6) δ = 9.67 (d, J = 2.4 Hz, 1H), 9.45-9.39 (m, 2H), 9.12 (s, 1H), 8.53 -8.51 (m, 1H), 8.19 (d, J = 1.6 Hz, 1H), 7.96 - 7.93 (m, 2H), 7.46 (d, J = 4.8 Hz, 1H), 7.31 - 7.29 (m, 1H), 4.89-4.80 (m, 4H), 4.24 -4.20 (m, 1H), 4.17 - 4.13 (m, 4H), 3.87 (d, J = 11.6 Hz, 1H), 2.37 - 2.35 (m, 2H), 1.69 (s, 3H) ppm |
| 378 | 519.1 | 1H NMR (400 MHz, MeOD-d4) δ = 9.68 (s, 1H), 8.96 - 8.81 (m, 2H), 8.61 - 8.46 (m, 2H), 8.35 (s, 1H), 8.17 (d, J = 1.6 Hz, 1H), 8.09 - 7.98 (m, 1H), 7.97 - 7.88 (m, 1H), 7.31 (d, J = 8.0 Hz, 1H), 5.02 (s, 2H), 4.91 (d, J = 2.4 Hz, 2H), 4.37 - 4.29 (m, 2H), 4.20 (d, J = 11.2 Hz, 1H), 3.94 (d, J = 11.6 Hz, 1H), 2.78 - 2.69 (m, 2H), 2.30 - 2.19 (m, 2H), 1.76 (s, 3H) ppm. |
| 379 | 536.3 | 1H NMR (400 MHz, METHANOL-d4) δ = 9.39 (s, 1H), 8.75 - 8.73 (m, 1H), 8.68 - 8.66 (m, 1H), 8.52 (d, J = 5.2 Hz, 1H), 8.20 (d, J = 1.6 Hz, 1H), 8.01 (s, 1H), 7.96-7.93 (m, 1H), 7.77 (d, J = 4.8 Hz, 1H), 7.32 (d, J = 8.0 Hz, 1H), 4.95 (s, 2H), 4.94 (d, J = 3.6 Hz, 2H), 4.24 - 4.18 (m, 2H), 3.95 (d, J = 11.6 Hz, 1H), 3.91 - 3.65 (m, 4H), 3.42 (s, 3H), 2.30 - 2.11 (m, 2H), 1.78 (s, 3H) ppm |
| 380 | 506.2 | 1H NMR (400 MHz, MeOD) δ = 9.36 (s, 1H), 8.75 - 8.67 (m, 1H), 8.67 - 8.61 (m, 1H), 8.48 (d, J = 5.2 Hz, 1H), 8.18 (d, J = 1.6 Hz, 1H), 7.99 (s, 1H), 7.96 - 7.90 (m, 1H), 7.71 (d, J = 5.2 Hz, 1H), 7.30 (d, J = 8.2 Hz, 1H), 4.94 (s, 2H), 4.92 (d, J = 3.6 Hz, 2H), 4.21 (d, J = 11.6 Hz, 1H), 3.93 (d, J = 11.6 Hz, 1H), 3.68 (br s, 4H), 2.13 - 2.01 (m, 4H), 1.77 (s, 3H) ppm |
| 381 | 521.3 | 1H NMR (400 MHz, DMSO-d6) δ = 9.31 - 9.28 (m, 1H), 8.91 (s, 1H), 8.20 - 8.17 (m, 2H), 7.95 - 7.92 (m, 1H), 7.54 (d, J = 7.2 Hz, 1H), 7.38 (d, J = 9.2 Hz, 1H), 7.33 - 7.27 (m, 2H), 6.17 (d, J = 6.8 Hz, 1H), 4.94 - 4.80 (m, 2H), 4.71 (s, 2H), 4.69 - 4.61 (m, 2H), 4.23 (d, J = 11.6 Hz, 1H), 3.98 - 3.94 (m, 2H), 3.87 (d, J = 11.6 Hz, 1H), 3.40 (s, 3H), 2.54 (br s, 2H), 1.70 (s, 3H) ppm |
| 384 | 491.2 | 1H NMR (400 MHz, DMSO-d6) δ = 9.67 (d, J = 2.0 Hz, 1H), 9.39 - 9.37 (m, 2H), 8.96 (d, J = 1.6 Hz, 1H), 8.19 (d, J = 1.6 Hz, 1H), 7.96-7.93 (m, 1H), 7.89 (s, 1H), 7.70- 7.66 (m, 1H), 7.47 (d, J = 7.6 Hz, 1H), 7.30 (d, J = 8.0 Hz, 1H), 6.46 (d,J = 8.0 Hz, 1H), 4.93 - 4.79 (m, 4H), 4.22 (d, J = 11.2 Hz, 1H), 4.07-4.03 (m, 4H), 3.86 (d, J = 11.6 Hz, 1H), 2.38 - 2.34(m, 2H), 1.69 (s, 3H) ppm. |
| 385 | 492.2 | 1H NMR (400 MHz, DMSO-d6) δ = 9.65 (d, J = 2 Hz, 1H), 9.42 - 9.38 (m, 2H), 9.04 (d, J = 1.6 Hz, 1H), 8.66 (s, 1H), 8.19 (d, J = 1.6 Hz, 1H), 7.98 - 7.94 (m, 2H), 7.90 (s, 1H), 7.30 (d, J = 8.0 Hz, 1H), 4.93 - 4.80 (m, 4H), 4.24 - 4.14 (m, 5H), 3.86 (d, J =11.6 Hz, 1H), 2.44 - 2.41 (m, 2H), 1.69 (s, 3H) ppm |
| 386 | 467.1 | 1H NMR (400 MHz, DMSO-d6) δ = 9.74 (d, J=3, 1H), 9.46-9.41 (m, 2H), 9.18 (d, J=2, 1H), 9.08 (s, 1H),8.41(s, 1H), 8.19 (d, J=1.6, 1H), 7.97-7.91 (m, 2H), 7.30 (d, J=8, 1H), 4.93-4.81 (m, 4H), 4.23(d, J=11.6, 1H), 4.08 (s, 3H) , 3.86(d, J=11.6, 1H), 1.69(s, 3H) ppm. |
| 387 | 507.2 | 1H NMR (400 MHz, DMSO-d6) δ = 9.48 - 9.38 (m, 2H), 8.73 (d, J = 8.8 Hz, 1H), 8.61 (d, J = 8.8 Hz, 1H), 8.47 (d, J = 7.2 Hz, 1H), 8.21 (d, J = 1.6 Hz, 1H), 8.08 - 8.04 (m, 1H), 7.97 - 7.94 (m, 1H), 7.86 (s, 1H), 7.71 (d, J = 8.0 Hz, 1H), 7.32 (d, J = 8.0 Hz, 1H), 4.95 - 4.86 (m, 2H), 4.83 (d, J = 4.8 Hz, 2H), 4.23 (d, J = 11.2 Hz, 1H), 3.87 (d, J = 11.2 Hz, 1H), 3.43 (s, 3H), 2.17 (s, 3H), 1.71 (s, 3H) ppm. |
| 388 | 466.4 | 1H NMR (400 MHz, MeOD) δ = 9.38 (s, 1H), 9.20 (d, J = 2.0 Hz, 1H), 8.52 (d, J = 1.6 Hz, 1H), 8.29 - 8.24 (m, 1H), 8.16 (d, J = 1.6 Hz, 1H), 7.95 - 7.88 (m, 3H), 7.27 (d, J = 8.0 Hz, 1H), 7.17 - 7.11 (m, 1H), 4.92 - 4.89 (m, 4H), 4.19 (d, J = 11.6 Hz, 1H), 4.00 (s, 3H), 3.92 (d, J = 11.6 Hz, 1H), 1.78 - 1.71 (m, 3H) ppm |
| 389 | 508.2 | 1H NMR (400 MHz, DMSO-d6) δ = 9.45 - 9.31 (m, 2H), 8.65 - 8.63(m, 1H), 8.51 - 8.45 (m, 2H), 8.20 (d, J = 1.6 Hz,1H), 7.96 - 7.94 (m, 1H), 7.78 (s, 1H), 7.31 (d, J = 8.4 Hz, 1H), 4.99 - 4.73 (m, 4H), 4.52 - 4.43 (m, 2H), 4.23 (d, J =11.6 Hz, 1H), 3.87 (d, J = 11.6 Hz, 1H), 3.63 - 3.54 (m, 2H), 3.21 (s, 3H), 1.70 (s, 3H) ppm |
| 390 | 503.2 | 1H NMR (400 MHz, METHANOL-d4) δ = 9.37 (s, 1H), 8.72 - 8.62 (m, 2H), 8.46 (d, J = 8.8 Hz, 1H), 8.17 (d, J = 1.6 Hz, 1H), 8.04 - 7.72 (m, 3H), 7.30 (d, J = 8.4 Hz, 1H), 7.17 (d, J = 9.6 Hz, 1H), 4.94 - 4.91 (m, 4H), 4.21 (d, J = 11.6 Hz, 1H), 3.93 (d, J = 11.6 Hz, 1H), 1.77 (s, 3H) ppm. |
| 391 | 505.2 | 1H NMR (400 MHz, DMSO-d6) δ =9.43 - 9.40 (m, 2H), 8.74 (d, J = 8.8 Hz, 1H), 8.63 (d, J = 8.8 Hz, 1H), 8.33 (d, J = 7.6 Hz, 1H), 8.20 (d, J = 1.2 Hz, 1H), 803 - 7.99 (m, 1H), 7.97 - 7.94 (m, 1H), 7.84 (s, 1H), 7.75 - 7.73 (m, 1H), 7.31 (d, J = 8.0 Hz, 1H), 4.93 - 4.81 (m, 4H), 4.23 (d, J = 11.6 Hz, 1H), 3.89 - 3.84 (m, 3H), 3.17 - 3.15 (m, 2H), 1.71 (s, 3H) ppm |
| 392 | 504.1 | 1H NMR (400 MHz, DMSO-d6) δ = 9.45 - 9.37 (m, 2H), 8.68 (d, J = 8.8 Hz, 1H), 8.32 (s, 1H), 8.27 (d, J = 8.8 Hz, 1H), 8.20 (d, J = 1.2 Hz, 1H), 8.18 (s, 1H), 8.01 - 7.94 (m, 2H), 7.83 (s, 1H), 7.58 - 7.54 (m, 2H), 7.32 - 7.29 (m, 1H), 4.93 - 4.81 (m, 4H), 4.23 (d, J = 11.6 Hz, 1H), 3.88 - 3.85 (m, 1H), 3.75 - 3.73 (m, 2H), 3.12 - 3.10 (m, 2H), 1.70 (s, 3H) ppm. |
| 393 | 504.1 | 1H NMR (400 MHz, DMSO-d6) δ = 9.45 - 9.37 (m, 2H), 8.68 (d, J = 8.8 Hz, 1H), 8.32 (s, 1H), 8.27 (d, J = 8.8 Hz, 1H), 8.20 (d, J = 1.2 Hz, 1H), 8.18 (s, 1H), 8.01 - 7.94 (m, 2H), 7.83 (s, 1H), 7.58 - 7.54 (m, 2H), 7.32 - 7.29 (m, 1H), 4.93 - 4.81 (m, 4H), 4.23 (d, J = 11.6 Hz, 1H), 3.88 - 3.85 (m, 1H), 3.75 - 3.73 (m, 2H), 3.12 - 3.10 (m, 2H), 1.70 (s, 3H) ppm. |
| 394 | 536.3 | 1H NMR (EW18644-416-P1A, 400 MHz, METHANOL-d4) δ = 9.34 (s, 1H), 8.93 (s, 1H), 8.64 - 8.57 (m, 2H), 8.18 (d, J = 1.6 Hz, 1H), 7.98 (s, 1H), 7.96 - 7.86 (m, 2H), 7.30 (d, J = 8.4 Hz, 1H), 4.93 (s, 2H), 4.92 (d, J = 3.6 Hz, 2H), 4.32 - 4.25 (m, 2H), 4.21 (d, J = 11.6 Hz, 1H), 4.01 - 3.97 (m, 2H), 3.93 (d, J = 11.6 Hz, 1H), 3.65 (d, J = 6.4 Hz, 2H), 3.41 (s, 3H), 3.15 - 308 (m, 1H), 1.77 (s, 3H) ppm |
| 395 | 486.1 | 1H NMR (400 MHz, DMSO-d6)δ= 9.59 - 9.56 (m, 1H), 9.43 (s, 1H), 9.10 (s, 1H), 8.94 (s, 1H), 8.72 (d, J = 8.4 Hz, 1H), 8.62 (s, 1H), 8.52 (d, J = 8.8 Hz, 1H), 8.01 (s, 1H), 7.90 (s, 1H), 4.94 - 4.69 (m, 4H), 4.22 - 4.08 (m, 5H), 4.01 - 3.81 (m, 1H), 2.49 -2.40 (m, 2H), 1.72 (d, J = 20.4 Hz, 3H) ppm |
| 396 | 506.3 | 1H NMR (400 MHz, DMSO-d6) δ = 9.45 - 9.37 (m, 2H), 8.68 (d, J = 8.4 Hz, 1H), 8.39 - 8.17 (m, 4H), 7.96 - 7.94 (m, 1H), 7.82 (s, 1H), 7.64 - 7.49 (m, 2H), 7.31 (d, J = 8.0 Hz, 1H), 4.95 - 4.77 (m, 4H), 4.23 (d, J = 11.6 Hz, 1H), 3.87 (d, J = 11.6 Hz, 1H), 3.22 (s, 3H), 2.00 - 1.73 (m, 3H), 1.70 (s, 3H) ppm. |
| 397 | 521.3 | 1H NMR (400 MHz, METHANOL-d4) δ = 9.41 (s, 1H), 8.93 (d, J = 5.2 Hz, 1H), 8.82 - 8.80 (m, 1H), 8.73 - 8.71 (m, 1H), 8.47 (d, J = 5.2 Hz, 1H), 8.20 (d, J = 2.0 Hz, 1H), 8.02 (s, 1H), 7.96-7.93 (m, 1H), 7.32 (d, J = 8.4 Hz, 1H), 4.96 (s, 2H), 4.93 (d, J = 3.6 Hz, 2H), 4.23 (d, J = 11.6 Hz, 1H), 4.14 - 4.10 (m, 2H), 3.95 (d, J = 11.2 Hz, 1H), 3.70-3.63 (m, 2H), 3.30 - 3.26 (m, 1H), 2.16 - 2.05 (m, 4H), 1.78 (s, 3H) ppm |
| 398 | 503.2 | 1H NMR (400 MHz, DMSO-d6) δ = 9.50-9.47 (m, 1H), 9.42 (s, 1H), 8.92 (s, 1H), 8.72 (d, J = 8.4 Hz, 1H), 8.51 (d, J = 8.4 Hz, 1H), 8.16 (s, 1H), 8.00 (s, 1H), 7.85 (s, 1H), 7.80 (d, J = 10.4 Hz, 1H), 4.96 (d, J = 16.0 Hz, 1H), 4.83-4.80 (m, 2H), 4.76-4.75 (m, 1H), 4.18-4.15 (m, 4H), 4.08-4.05 (m, 1H), 3.89-3.83 (m, 1H), 2.47 - 2.39 (m, 2H), 1.72-1.67 (m, 3H) ppm. |
| 399 | 510.3 | 1H NMR (400 MHz, DMSO-d₆) δ = 9.50-9.47 (m, 1H), 9.41 (s, 1H), 8.92 (s, 1H), 8.71 (d, J = 8.4 Hz, 1H), 8.51 (d, J = 8.4 Hz, 1H), 8.09 (s, 1H), 7.99 (s, 1H), 7.86 (s, 1H), 7.79 (d, J = 10.4 Hz, 1H), 4.98-4.85 (m, 2H), 4.83 (d, J = 5.6 Hz, 2H), 4.25(d, J = 11.6Hz, 1H), 4.18-4.16 (m, 4H), 3.89 (d, J = 11.6 Hz, 1H), 2.45-2.41 (m, 2H), 1.71 (s, 3H) ppm |
| 400 | 453.2 | 1H NMR (400 MHz, DMSO-d6) δ = 9.50-9.48 (m, 1H), 9.39 (s, 1H), 8.67 (d, J = 8.0 Hz, 1H), 8.33 - 8.29 (m, 3H), 8.11 (s, 1H), 7.83 (s, 1H), 7.80 - 7.78 (m, 1H), 7.60 - 7.56 (m, 3H), 4.99 - 4.84 (m, 2H), 4.82 (br d, J = 5.6 Hz, 2H), 4.25 (d, J = 11.6 Hz, 1H), 3.89 (d, J = 11.6 Hz, 1H), 1.72 (s, 3H) ppm |
| 401 | 485.3 | 1H NMR (400 MHz, DMSO-d6) δ = 9.48 - 9.34 (m, 2H), 8.92 (s, 1H), 8.71 (d, J = 8.8 Hz, 1H), 8.50 (d, J = 8.8 Hz, 1H), 8.27 (s, 1H), 8.07 - 7.92 (m, 2H), 7.84 (s, 1H), 7.30 (d, J = 8.0 Hz, 1H), 4.93 - 4.69 (m, 4H), 4.19-4.15 (m, 4H), 4.11-4.04 (m, 1H), 3.92 - 3.78 (m, 1H), 2.46 - 2.40 (m, 2H), 1.78 - 1.59 (m, 3H) ppm |
| 403 | 534.4 | 1H NMR (400 MHz, CHLOROFORM-d) δ = 9.32 (s, 1H), 8.67 (d, J = 8.4 Hz, 1H), 8.56 (d, J = 5.2 Hz, 1H), 8.44 (d, J = 8.8 Hz, 1H), 8.03 - 8.02 (m, 2H), 7.86 (d, J = 5.2 Hz, 1H), 7.81 - 7.80 (m, 1H), 7.39 - 7.38 (m, 1H), 7.16 (d, J = 8.0 Hz, 1H), 5.01 (d, J = 5.2 Hz, 2H), 4.91 (s, 4H), 4.88 (s, 2H), 4.42 (s, 4H), 4.16 (d, J = 11.2 Hz, 1H), 3.97 (d, J = 11.2 Hz, 1H), 1.77 (s, 3H) ppm |
| 404 | 528.5 | 1H NMR (400 MHz, CDCl3-d) δ = 9.31 (s, 2H), 8.57 (d, J = 8.4 Hz, 1H), 8.42 (d, J = 8.4 Hz, 1H), 8.04 - 8.03 (m, 3H), 7.84 - 7.81 (m, 1H), 7.41 - 7.40 (m, 1H), 7.17 (d, J = 8.0 Hz, 1H), 5.02 (d, J = 5.2 Hz, 2H), 4.89 (s, 2H), 4.60 - 4.54 (m, 4H), 4.16 (d, J = 11.2 Hz, 1H), 3.97 (d, J = 11.2 Hz, 1H), 1.78 (s, 3H) ppm |
| 405 | 481.3 | 1H NMR (400 MHz, MeOD-d4) δ = 9.14 (s, 1H), 8.39 (d, J = 8.4 Hz, 1H), 8.17 (d, J = 1.6 Hz, 1H), 8.13 (s, 1H), 8.00 (d, J = 8.8 Hz, 1H), 7.94-7.92 (m, 1H), 7.81 (s, 1H), 7.29 (d, J = 8.4 Hz, 1H), 4.92 (d, J = 3.6 Hz, 2H), 4.59 - 4.55 (m, 4H), 4.247 - 4.19 (m, 3H), 3.93 (d, J = 11.2 Hz, 1H), 2.38 - 2.35 (m, 2H), 1.77 (s, 3H) ppm |
| 406 | 477.3 | 1H NMR (400 MHz, CDCl3) δ = 9.61 (s, 1H), 9.33 - 9.30 (m, 1H), 8.62 - 8.59 (m, 2H), 8.42 - 8.40 (m, 1H), 8.04 - 8.03 (m, 2H), 7.84 - 7.81 (m, 1H), 7.42 - 7.41 (m, 1H), 7.16 (d, J = 8.0 Hz, 1H), 5.02 (d, J = 5.2 Hz, 2H), 4.89 (s, 2H), 4.16 (d, J = 11.6 Hz, 1H), 3.97 (d, J = 11.6 Hz, 1H), 2.22 - 2.19 (m, 1H), 1.78 (s, 3H), 1.25 - 1.23 (m, 2H), 1.18 - 1.16 (m, 2H) ppm |
| 407 | 480.2 | 1H NMR (400 MHz, MeOD-d4) δ = 9.33 (s, 1H), 8.90 (s, 1H), 8.64 (d, J = 1.6 Hz, 2H), 8.18 - 8.17 (m, 2H), 7.98 (s, 1H), 7.94 - 7.92 (m, 1H), 7.30 (d, J = 8.0 Hz, 1H), 4.93 (s, 2H), 4.92 (d, J = 3.6 Hz, 2H), 4.22 (d, J = 11.6 Hz, 1H), 3.94 (d, J = 11.6 Hz, 1H), 3.26 (s, 6H), 1.76 (s, 3H) ppm |
| 408 | 451.3 | 1H NMR (400 MHz, CDCl3) δ = 9.67 (s, 1H), 9.32 (s, 1H), 8.69 (d, J = 8.8 Hz, 1H), 8.59 (s, 1H), 8.45 - 8.43 (m, 1H), 8.07 (s, 1H), 8.04 (s, 1H), 7.84 - 7.81 (m, 1H), 7.44 - 7.43 (m, 1H), 7.16 (d, J = 8.0 Hz, 1H), 5.02 (d, J = 5.2 Hz, 2H), 4.89 (s, 2H), 4.16 (d, J = 11.2 Hz, 1H), 3.96 (d, J = 11.6 Hz, 1H), 2.70 (s, 3H), 1.78 (s, 3H) ppm |
| 409 | 522.4 | 1H NMR (400 MHz, MeOD-d4) δ = 9.33 (s, 1H), 8.95 (s, 1H), 8.63 - 8.58 (m, 2H), 8.17 (s, 1H), 7.98 (s, 1H), 7.94 - 7.91 (m, 2H), 7.30 (d, J = 8.4 Hz, 1H), 4.93 (s, 2H), 4.92 (d, J = 3.6 Hz, 2H), 4.49 - 4.45 (m, 1H), 4.43 - 4.40 (m, 2H), 4.22 (d, J = 11.2 Hz, 1H), 4.07 - 4.03 (m, 2H), 3.94 (d, J = 11.6 Hz, 1H), 3.38 (s, 3H), 1.76 (s, 3H) ppm |
| 410 | 494.2 | 1H NMR (400 MHz, METHANOL-d4)δ= 9.24 (s, 1H), 8.70 (d, J = 8.4 Hz, 1H), 8.53 (d, J = 8.4 Hz, 1H), 8.39 (d, J = 7.6 Hz, 1H), 8.08 (s, 1H), 7.93 - 7.80 (m, 3H), 7.69 (d, J = 7.6 Hz, 1H), 7.20 (d, J = 7.6 Hz, 1H), 5.05 (s, 1H), 4.84 - 4.81(m, 4H), 4.12 (d, J = 11.6 Hz, 1H), 3.83 (d, J = 11.2 Hz, 1H), 1.67 (s, 3H), 1.56 (s, 6H) ppm |
| 412 | 509.3 | 1H NMR (400 MHz, DMSO-d6) δ= 9.46 (s, 1H), 9.42 - 9.39 (m, 1H), 8.72 (d, J = 8.4 Hz, 1H), 8.47 (br s, 1H), 8.18 (s, 1H), 8.04 - 8.00 (m, 2H), 7.95 - 7.93 (m, 1H), 7.83 (s, 1H), 7.30 (d, J = 8.0 Hz, 1H), 7.16 - 7.14 (m, 1H), 4.92 - 4.81 (m, 4H), 4.22 (d, J = 11.6 Hz, 1H), 4.16 - 4.13 (m, 4H), 3.86 (d, J = 11.6 Hz, 1H), 2.37 - 2.33 (m, 2H), 1.69 (s, 3H) ppm |
| 413 | 429.1 | 1H NMR (400 MHz, MeOD-d4) δ = 9.31 (s, 1H), 9.09 - 9.04 (m, 1H), 8.63 - 8.56 (m, 2H), 8.23 - 8.14 (m, 3H), 7.98 (s, 1H), 7.59 - 7.52 (m, 3H), 4.94 (s, 2H), 4.85 (br s, 1H), 4.83 (d, J = 3.6 Hz, 1H), 4.17 - 4.06 (m, 1H), 4.00 - 3.87 (m, 1H), 1.87 - 1.63 (m, 3H) ppm. |
| 414 | 492.3 | 1H NMR (400 MHz, DMSO-d6) δ = 9.40 - 9.36 (m, 1H), 9.33 (s, 1H), 9.18 (d, J = 1.6 Hz, 1H), 8.59 (d, J =8.8 Hz, 1H), 8.42 (d, J = 8.8 Hz, 1H), 8.19 (s, 1H), 7.97 - 7.93 (m, 2H), 7.78 (s, 1H), 7.31 (d, J = 8.4 Hz, 1H), 4.92 - 4.78 (m, 4H), 4.23 - 4.16 (m, 5H), 3.87 (d, J = 11.6 Hz, 1H), 2.46 - 2.41 (m, 2H), 1.70 (s, 3H) ppm |
| 415 | 506.6 | 1H NMR (400 MHz, MeOD-d4) δ = 9.33 (s, 1H), 8.87 (s, 1H), 8.66 - 8.63 (m, 2H), 8.18 - 8.17 (m, 1H), 8.00 - 7.98 (m, 2H), 7.94 - 7.92 (m, 1H), 7.30 (d, J = 8.0 Hz, 1H), 4.93 (s, 2H), 4.92 (d, J = 3.6 Hz, 2H), 4.22 (d, J = 11.6 Hz, 1H), 3.94 (d, J = 11.6 Hz, 1H), 3.66 - 3.61 (m, 4H), 2.12 - 2.09 (m, 4H), 1.76 (s, 3H) ppm |
| 416 | 522.2 | 1HNMR (400 MHz, METHANOL-d4) δ = 9.37 (s, 1H), 8.70 - 8.63 (m, 2H), 8.55 (d, J = 5.2 Hz, 1H), 8.18 (d, J = 1.6 Hz, 1H), 7.99 (s, 1H), 7.94 - 7.91 (m, 1H), 7.78 (d, J = 5.2 Hz, 1H), 7.29 (d, J = 8.4 Hz, 1H), 4.96 - 4.90 (m, 4H), 4.21 (d, J = 11.2 Hz, 1H), 3.94 (d, J = 2.8 Hz, 1H), 3.92 (d, J = 4.4 Hz, 4H), 3.83 - 3.78 (m, 4H), 1.77 (s, 3H) ppm |
| 417 | 477.4 | 1H NMR (400 MHz, METHANOL-d4) δ = 9.39 (s, 1H), 8.78 (d, J = 7.2 Hz, 1H), 8.74 - 8.66 (m, 2H), 8.35 (d, J = 7.2 Hz, 1H), 8.18 (d, J = 1.6 Hz, 1H), 8.00 (s, 1H), 7.94 - 7.92 (m, 1H), 7.30 (d, J = 8.0 Hz, 1H), 4.94 (s, 2H), 4.92 (d, J = 3.6 Hz, 2H), 4.21 (d, J = 11.6 Hz, 1H), 3.93 (s, 1H), 2.43 - 2.32 (m, 1H), 1.77 (s, 3H), 1.29 - 1.23 (m, 2H), 1.21 - 1.13 (m, 2H) ppm. |
| 418 | 521.2 | 1H NMR (400 MHz, DMSO-d6) δ = 9.42 - 9.36 (m, 2H), 8.68 (d, J = 8.8 Hz, 1H), 8.37 - 8.31 (m, 2H), 8.31 - 8.27 (m, 1H), 8.20 (d, J = 1.2 Hz, 1H), 7.96 - 7.94 (m, 1H), 7.85 (s, 1H), 7.68 - 7.60 (m, 2H), 7.31 (d, J = 8.0 Hz, 1H), 4.94 - 4.79 (m, 8H), 4.23 (d, J = 11.6 Hz, 1H), 3.87 (d, J = 11.6 Hz, 1H), 3.07 (s, 3H), 1.70 (s, 3H) ppm. |
| 419 | 522.2 | 1H NMR (400 MHz, METHANOL-d4) δ = 9.35 (s, 1H), 8.86 (d, J = 8.4 Hz, 1H), 8.68 - 8.61 (m, 2H), 8.18 (d, J = 1.6 Hz, 1H), 8.06 - 8.05 (m, 1H), 8.00 (s, 1H), 7.94 - 7.93 (m, 1H), 7.65 -7.63 (m, 1H), 7.30 (d, J = 8.0 Hz, 1H), 5.22 (d, J = 7.2 Hz, 2H), 4.97 - 4.94 (m, 4H), 4.92 (d, J = 4.0 Hz, 2H), 4.21 (d, J = 11.2 Hz, 1H), 3.93 (d, J = 11.6 Hz, 1H), 3.29 (s, 3H), 1.77 (s, 3H) ppm |
| 420 | 491.3 | 1H NMR (400 MHz, METHANOL-d4) δ = 9.40 (s, 1H), 8.89 (d, J = 5.2 Hz, 1H), 8.82 (d, J = 8.8 Hz, 1H), 8.71 (d, J = 8.4 Hz, 1H), 8.43 (d, J = 5.2 Hz, 1H), 8.18 (d, J = 1.2 Hz, 1H), 8.02 (s, 1H), 7.93 - 7.92 (m, 1H), 7.30 (d, J = 8.4 Hz, 1H), 4.94 (s, 2H), 4.92 (d, J = 3.2 Hz, 2H), 4.21 (d, J = 11.2 Hz, 1H), 3.96 - 3.90 (m, 2H), 2.65 - 2.53 (m, 2H), 2.52 - 2.41 (m, 2H), 2.25 - 2.12 (m, 1H), 2.09 - 1.98 (m, 1H), 1.77 (s, 3H) ppm |
| 421 | 504.3 | 1H NMR (400 MHz, DMSO-d6) δ = 9.45 - 9.37 (m, 2H), 8.66 (d, J = 8.4 Hz, 1H), 8.30 (d, J = 8.4 Hz, 1H), 8.21 (s, 2H), 8.14 (d, J = 7.2 Hz, 1H), 7.96 (d, J = 8.0 Hz, 1H), 7.83 (s, 1H), 7.60 - 7.50 (m, 2H), 7.32 (d, J = 8.0 Hz, 1H), 4.98 - 4.76 (m, 4H), 4.23 (d, J = 11.6 Hz, 1H), 3.88 (d, J = 11.2 Hz, 1H), 3.78 - 3.65 (m, 3H), 3.21 - 3.18 (m, 2H), 2.31 (s, 3H), 1.71 (s, 3H) ppm |
| 422 | 536.3 | ¹H NMR (400 MHz, METHANOL-d4) δ = 9.37 (s, 1H), 8.76 - 8.62 (m, 2H), 8.50 (d, J = 5.0 Hz, 1H), 8.18 (s, 1H), 7.99 (s, 1H), 7.93 (dd, J = 1.7, 8.1 Hz, 1H), 7.74 (d, J = 5.1 Hz, 1H), 7.30 (d, J = 8.0 Hz, 1H), 4.95 - 4.91 (m, 4H), 4.27 - 4.12 (m, 2H), 3.90 - 3.62 (m, 4H), 3.93 (d, J = 11.3 Hz, 1H), 3.40 (s, 3H), 2.27 - 2.11 (m, 2H), 1.77 (s, 3H) ppm |
| 423 | 467.1 | ¹H NMR (400 MHz, DMSO-d6) δ = 9.46 (s, 1H), 9.42 (br t, J = 5.7 Hz, 1H), 9.30 (s, 1H), 8.78 (d, J = 8.7 Hz, 1H), 8.61 (d, J = 8.8 Hz, 1H), 8.49 (s, 1H), 8.20 (s, 1H), 7.96 (d, J = 8.4 Hz, 1H), 7.88 (s, 1H), 7.31 (d, J = 8.3 Hz, 1H), 4.94 - 4.81 (m, 4H), 4.23 (d, J = 11.4 Hz, 1H), 4.10 (s, 3H), 3.87 (d, J = 11.6 Hz, 1H), 1.71 (s, 3H) ppm |
| 424 | 480.2 | ¹H NMR (400 MHz, DMSO-d6) δ = 9.47 (s, 1H), 9.41 (br t, J = 5.7 Hz, 1H), 8.76 (d, J = 8.6 Hz, 1H), 8.69 - 8.63 (m, 1H), 8.56 (d, J = 4.9 Hz, 1H), 8.20 (s, 1H), 7.98 - 7.93 (m, 1H), 7.86 (s, 1H), 7.66 (d, J = 4.9 Hz, 1H), 7.31 (d, J = 8.2 Hz, 1H), 4.94 - 4.81 (m, 4H), 4.23 (d, J = 11.6 Hz, 1H), 3.87 (d, J = 11.6 Hz, 1H), 3.25 (s, 6H), 1.70 (s, 3H) ppm |
| 427 | 505.2 | ¹H NMR (400 MHz, DMSO-d6) δ = 9.50 - 9.40 (m, 2H), 8.80 - 8.68 (m, 3H), 8.28 - 8.19 (m, 2H), 7.97 (d, J = 8.0 Hz, 1H), 7.87 (s, 1H), 7.32 (d, J = 8.0 Hz, 1H), 4.97 - 4.77 (m, 4H), 4.71 - 4.61 (m, 2H), 4.23 (d, J = 11.2 Hz, 1H), 3.87 (d, J = 11.6 Hz, 1H), 3.15 (s, 3H), 1.71 (s, 3H) ppm |
| 428 | 446.3 | ¹H NMR (400 MHz, MeOD) δ = 9.30 (s, 1H), 8.59 (d, J = 8.8 Hz, 1H), 8.23 - 8.14 (m, 3H), 8.09 (s, 1H), 7.95 (s, 1H), 7.69 (brd, J = 10.6 Hz, 1H), 7.60 - 7.51 (m, 3H), 4.97 - 4.88 (m, 4H), 4.10 - 3.99 (m, 1H), 3.94 - 3.81 (m, 1H), 1.82 - 1.64 (m, 3H) ppm |
| 429 | 485.2 | ¹H NMR (400 MHz, DMSO-d6) δ = 9.46 (s, 1H), 9.41 - 7.38 (m, 1H), 8.75 (d, J = 8.8 Hz, 1H), 8.61 - 8.53 (m, 2H), 8.27 (s, 1H), 7.97 (d, J = 8.0 Hz, 1H), 7.85 (s, 1H), 7.71 - 7.70 (m, 1H), 7.31 (d, J = 8.0 Hz, 1H), 4.90 - 4.70 (m, 4H), 4.18 - 4.14 (m, 4H), 4.11 - 4.04 (m, 1H), 3.91 - 3.79 (m, 1H), 2.42 - 2.35 (m, 2H), 1.74 - 1.65 (m, 3H) ppm |
| 430 | 522.2 | ¹H NMR (400 MHz, METHANOL-d4) δ = 9.37 (s, 1H), 8.65 (s, 2H), 8.50 (d, J = 5.2 Hz, 1H), 8.17 (d, J = 1.6 Hz, 1H), 7.98 (s,1H), 7.96 - 7.90 (m, 1H), 7.80 (d, J = 5.2 Hz, 1H), 7.29 (d, J = 8.0 Hz, 1H), 4.96 - 4.89 (m, 4H), 4.48 - 4.36 (m, 3H), 4.21 (d, J = 11.6 Hz, 1H), 4.06 (br d, J = 6.8 Hz, 2H), 3.93 (d, J = 11.2 Hz, 1H), 3.38 (s, 3H), 1.77 (s, 3H) ppm |
| 431 | 495.2 | 1H NMR (400 MHz, DMSO-d6) δ= 9.40 - 9.37 (m, 2H), 8.52 (d, J = 8.8 Hz, 1H), 8.25 (d, J = 8.8 Hz, 1H), 8.19 (d, J = 1.6 Hz,1H), 7.95 - 7.93 (m, 1H), 7.85 - 7.83 (m, 1H), 7.78 - 7.76 (m, 1H), 7.49 - 7.44 (m, 1H), 7.30 (d, J = 8.0 Hz, 1H), 7.22 (d, J = 8.4 Hz, 1H), 7.11 - 7.07 (m, 1H), 4.92 - 4.79 (m, 5H), 4.23 - 4.13 (m, 3H), 3.86 (d, J = 11.6 Hz, 1H), 3.74 - 3.71 (m, 2H), 1.69 (s, 3H) ppm |
| 432 | 484.2 | 1H NMR (400 MHz, MeOD) δ = 9.40 (s, 1H), 8.68 (d, J = 8.4 Hz, 1H), 8.17 (d, J = 1.6 Hz, 1H), 8.12 - 804 (m, 2H), 7.99 (s, 1H), 7.95 - 7.88 (m, 1H), 7.55 - 7.51 (m, 1H), 7.29 (d, J = 8.4 Hz, 1H), 4.94 (s, 2H), 4.91 (d, J = 4.0 Hz, 2H), 4.20 (d, J = 11.6 Hz, 1H), 4.07 (s, 3H), 3.92 (d, J = 11.6 Hz, 1H), 1.76 (s, 3H) ppm |
| 434 | 534.3 | 1H NMR (400 MHz, DMSO-d6) δ = 9.45 - 9.37 (m, 2H), 8.97 (s, 1H), 8.71 (d, J = 8.8 Hz, 1H), 8.52 - 8.41 (m, 2H), 8.20 (s, 1H), 7.97 (d, J = 8.4 Hz, 1H), 7.87 (s, 1H), 7.74 - 7.65 (m, 1H), 7.31 (d, J = 8.4 Hz, 1H), 6.73 (d, J = 7.6 Hz, 1H), 4.93 - 4.80 (m, 4H), 4.23 (d, J = 11.6 Hz, 1H), 3.87 (d, J = 11.6 Hz, 1H), 3.57 (s, 3H), 1.71 (s, 3H) ppm |
| 435 | 522.5 | 1H NMR (400 MHz, MeOD-d4) δ = 9.33 (s, 1H), 8.99 (s, 1H), 8.61 - 8.56 (m, 2H), 8.32 (s, 1H), 8.18 (d, J = 1.6 Hz, 1H), 7.98 (s, 1H), 7.96 - 7.91 (m, 1H), 7.30 (d, J = 8.0 Hz, 1H), 4.93 - 4.90 (m, 4H), 4.22 (d, J = 11.6 Hz, 1H), 3.94 (d, J = 11.6 Hz, 1H), 3.87 - 3.85 (m, 4H), 3.75 - 3.72 (m, 4H), 1.76 (s, 3H) ppm |
| 437 | 491.3 | 1H NMR (400 MHz, DMSO-d6) δ = 9.49 - 9.38 (m, 2H), 8.72 (d, J = 8.4 Hz, 1H), 8.31 (d, J = 8.4 Hz, 1H), 8.26 - 8.18 (m, 2H), 7.96 (d, J = 8.0 Hz, 1H), 7.86 (s, 1H), 7.44 (d, J = 5.2 Hz, 1H), 7.31 (d, J = 8.4 Hz, 1H), 7.14 (s, 1H), 4.97 - 4.80 (m, 4H), 4.23 (d, J = 11.6 Hz, 1H), 4.06 - 4.02 (m, 4H), 3.88 (d, J = 11.6 Hz, 1H), 2.38 - 2.32 (m, 2H), 1.71 (s, 3H) ppm |
| 438 | 442.3 | 1H NMR (400 MHz, DMSO-d6) δ = 9.46 (s, 1H), 9.38 - 9.36 (m, 1H), 8.91 - 8.80 (m, 2H), 8.18 (s, 1H), 8.14 - 8.07 (m, 2H), 8.02 (s, 1H), 7.94 (d, J = 8.0 Hz, 1H), 7.30 (d, J = 8.0 Hz, 1H), 4.94 - 4.82 (m, 2H), 4.78 (d, J = 6.0 Hz, 2H), 4.23 (d, J = 11.2 Hz, 1H), 3.87 (d, J = 11.6 Hz, 1H), 1.70 (s, 3H) ppm |
| 439 | 442.1 | 1H NMR (400 MHz, DMSO-d6) δ = 9.53 - 9.24 (m, 3H), 8.83 - 8.81 (m, 1H), 8.54 - 8.52 (m, 1H), 8.18 (s, 1H), 7.99 (s, 1H), 7.95 - 7.93 (m, 1H), 7.67 - 7.64 (m, 1H), 7.30 (d, J = 8.0 Hz, 1H), 4.95 - 4.81 (m, 2H), 4.77 (d, J = 6.0 Hz, 2H), 4.23 (d, J = 11.6 Hz, 1H), 3.87 (d, J = 11.6 Hz, 1H), 1.71 (s, 3H) ppm |
| 440 | 442.1 | 1H NMR (400 MHz, DMSO-d6) δ = 9.39 (s, 1H), 9.38 - 9.35 (m, 1H), 8.79 - 8.78 (m, 1H), 8.38 (d, J = 8.0 Hz, 1H), 8.18 (s, 1H), 8.11 - 8.07 (m, 1H), 7.98 (s, 1H), 7.95 - 7.93 (m, 1H), 7.70 - 7.66 (m, 1H), 7.30 (d, J = 8.0 Hz, 1H), 4.95 - 4.81 (m, 2H), 4.77 (d, J = 6.0 Hz, 2H), 4.23 (d, J = 11.6 Hz, 1H), 3.87 (d, J = 11.6 Hz, 1H), 1.71 (s, 3H) ppm |
| 441 | 491.3 | 1H NMR (400 MHz, DMSO-d6) δ = 9.46 - 9.35 (m, 2H), 8.76 (d, J = 2.0 Hz, 1H), 8.68 (d, J = 8.8 Hz, 1H), 8.32 (d, J = 8.4 Hz, 1H), 8.20 (d, J = 1.2 Hz, 1H), 7.99 - 7.92 (m, 2H), 7.84 (s, 1H), 7.58 - 7.57 (m, 1H), 7.31 (d, J = 8.4 Hz, 1H), 4.98 - 4.77(m, 4H), 4.23 (d, J = 11.6 Hz, 1H), 3.99 - 3.98 (m, 4H), 3.88 (d, J = 11.6 Hz, 1H), 2.41 - 2.39 (m, 2H), 1.71 (s, 3H) ppm. |
| 442 | 522.4 | 1HNMR (400 MHz, METHANOL-d4) δ = 9.34 (s, 1H), 8.62 - 8.55 (m, 2H), 8.17 (d, J = 1.6 Hz, 1H), 7.98 (s, 1H), 7.93 - 7.91 (m, 1H), 7.29 (d, J = 8.0 Hz, 1H), 7.13 (s, 1H), 4.92 (d, J = 3.6 Hz, 4H), 4.24 - 4.19 (m, 5H), 3.96 - 3.91 (m, 4H), 2.45 - 2.37 (m, 2H), 1.76 (s, 3H) ppm. |
| 443 | 523.3 | 1H NMR (400 MHz, DMSO-d6) δ = 9.51 (s, 1H), 9.43 (m, 1H), 8.83 (d, J = 8.6 Hz, 1H), 8.50 (d, J = 8.6 Hz, 1H), 8.20 (d, J = 1.6 Hz, 1H), 7.96 (m, 1H), 7.91 - 7.82 (m, 2H), 7.32 (d, J = 8.4 Hz, 1H), 7.06 (d, J = 7.6 Hz, 1H), 4.98 - 4.78 (m, 4H), 4.23 (d, J = 11.4 Hz, 1H), 3.87 (d, J = 11.4 Hz, 1H), 3.59 (s, 3H), 1.71 (s, 3H) ppm |
| 444 | 451.3 | 1HNMR (400 MHz, METHANOL-d4) δ = 9.40 (s, 1H), 8.86 (d, J = 5.6 Hz, 1H), 8.79 - 8.67 (m, 2H), 8.44 (d, J = 5.2 Hz, 1H), 8.18 (s, 1H), 8.01 (s, 1H), 7.95 - 7.92 (m, 1H), 7.30 (d, J = 8.0 Hz, 1H), 4.94 (s, 2H), 4.92 (d, J = 3.6 Hz, 2H), 4.21 (d, J = 11.6 Hz, 1H), 3.93 (d, J = 11.6 Hz, 1H), 2.83 (s, 3H), 1.77 (s, 3H) ppm |
| 445 | 548.5 | 1H NMR (400 MHz, DMSO-d6) δ = 9.45 - 9.35 (m, 2H), 8.69 (d, J = 8.6 Hz, 1H), 8.51 - 8.48 (m, 1H), 8.43 (d, J = 8.0 Hz, 1H), 8.35 (d, J = 8.8 Hz, 1H), 8.20 (d, J = 1.4 Hz, 1H), 7.96 (m, 1H), 7.84 (s, 1H), 7.78 (d, J = 7.6 Hz, 1H), 7.68 - 7.60 (m, 1H), 7.31 (d, J = 8.4 Hz, 1H), 4.98 - 4.76 (m, 4H), 4.48 (m, 1H), 4.33 - 4.12 (m, 4H), 3.87 (m, 2H), 3.22 (s, 3H), 1.70 (s, 3H) ppm |
| 446 | 484.3 | 1H NMR (400 MHz, DMSO-d6) δ = 9.50 (s, 1H), 9.42 (t, J = 6.0 Hz, 1H), 8.77 (d, J = 8.7 Hz, 1H), 8.39 (d, J = 2.5 Hz, 1H), 8.22 - 8.18 (m, 1H), 8.09 (dd, J = 1.8, 8.5 Hz, 1H), 7.95 (dd, J = 1.6, 8.1 Hz, 1H), 7.87 (s, 1H), 7.38 (d, J = 5.1 Hz, 1H), 7.31 (d, J = 8.2 Hz, 1H), 4.95 - 4.79 (m, 4H), 4.23 (d, J = 11.5 Hz, 1H), 3.91 (s, 3H), 3.87 (d, J = 11.5 Hz, 1H), 1.71 (s, 3H) ppm |
| 447 | 476.4 | 1H NMR (400 MHz, METHANOL-d4) δ = 9.38 (s, 1H), 9.04 (d, J = 7.6 Hz, 1H), 8.81 (d, J = 8.8 Hz, 1H), 8.69 (d, J = 8.8 Hz, 1H), 8.28 (d, J = 7.2 Hz, 1H), 8.24 (d, J = 2.4 Hz, 1H), 8.19 (s, 1H), 8.02 (s, 1H), 7.93 - 7.92 (m, 1H), 7.31 (d, J = 8.0 Hz, 1H), 6.89 - 6.88 (m, 1H), 4.94 (s, 2H), 4.92 (d, J = 3.6 Hz, 2H), 4.22 (d, J = 11.6 Hz, 1H), 3.95 (d, J = 11.2 Hz, 1H), 1.77 (s, 3H) ppm |
| 448 | 517 | 1H NMR (400 MHz, MeOD-d4) δ = 9.36 (s, 1H), 9.16 (s, 1H), 8.80 - 8.78 (m, 1H), 8.69 - 8.67 (m, 1H), 8.42 (s, 1H), 8.35 (d, J = 8.4 Hz, 1H), 8.18 (d, J = 1.6 Hz, 1H), 8.07 (d, J = 8.4 Hz, 1H), 8.00 (s, 1H), 7.95 - 7.93 (m, 1H), 7.31 (d, J = 8.4 Hz, 1H), 4.94 (s, 2H), 4.92 (d, J = 3.6 Hz, 2H), 4.22 (d, J = 11.6 Hz, 1H), 3.94 (d, J = 11.2 Hz, 1H), 3.87 (s, 3H), 1.77 (s, 3H) ppm |
| 449 | 520.2 | 1H NMR (400 MHz, METHANOL-d4) δ = 9.32 (br s, 1H), 8.63 - 8.55 (m, 2H), 8.42 (dd, J = 2.2, 11.0 Hz, 1H), 8.22 - 8.13 (m, 2H), 8.10 - 7.76 (m, 3H), 7.31 (d, J = 8.2 Hz, 1H), 4.93 (s, 4H), 4.22 (d, J = 11.4 Hz, 1H), 3.95 (d, J = 11.4 Hz, 1H), 1.78 (s, 3H) ppm |
| 450 | 493 | 1H NMR (400 MHz, DMSO-d6) δ= 9.53 - 9.47 (m, 1 H), 9.46 - 9.38 (m, 1 H), 8.97 - 8.73 (m, 4 H), 8.53 - 8.36 (m, 2 H), 8.25 -8.15 (m, 1 H), 8.01 - 7.95 (m, 1 H), 7.94 - 7.88 (m, 1 H), 7.40 - 7.23 (m, 1 H), 5.00 - 4.72 (m, 4 H), 4.30 - 4.16 (m, 1 H), 3.94 - 3.83(m, 1 H), 2.95 - 2.80 (m, 3 H), 1.80 - 1.59 (m, 3 H) ppm |
| 451 | 436.2 | 1H NMR (400 MHz, METHANOL-d4) δ = 9.40 (s, 1H), 8.78 - 8.74 (m, 2H), 8.71 (d, J = 8.4 Hz, 1H), 8.33 - 8.26 (m, 3H), 8.20 (d, J = 1.6 Hz, 1H), 8.02 (s, 1H), 7.96 - 7.94 (m, 1H), 7.31 (d, J = 8.0 Hz, 1H), 4.96 (s, 2H), 4.93 (d, J = 3.6 Hz, 2H), 4.23 (d, J = 11.6 Hz, 1H), 3.95 (d, J = 11.6 Hz, 1H), 1.79 (s, 3H) ppm |
| 452 | 516.2 | 1H NMR (400 MHz, DMSO-d6) δ = 9.41 (s, 2H), 9.12 (s, 1H), 8.69 (d, J = 8.8 Hz, 1H), 8.64 - 8.62 (m, 1H), 8.40 (d, J = 8.8 Hz, 1H), 8.21 (s, 1H), 7.98 (d, J = 8.0 Hz, 1H), 7.89 - 7.81 (m, 2H), 7.59 (d, J = 7.2 Hz, 1H), 7.32 (d, J = 8.0 Hz, 1H), 6.72 (d, J = 7.2 Hz, 1H), 4.96 - 4.78 (m, 4H), 4.24 (d, J = 11.6 Hz, 1H), 3.88 (d, J = 11.6 Hz, 1H), 3.56 (s, 3H), 1.72 (s, 3H) ppm |
| 454 | 469.1 | 1H NMR (400 MHz, DMSO-d6) δ = 9.39 (t, J = 5.2 Hz, 1H), 9.21 (s, 1H), 8.46 (d, J = 8.8 Hz, 1H), 8.37 (s, 1H), 8.20 (d, J = 1.6 Hz, 1H), 8.02 - 7.93 (m, 2H), 7.57 (s, 1H), 7.31 (d, J = 8.2 Hz, 1H), 4.95 - 4.81 (m, 2H), 4.76 (br d, J = 5.9 Hz, 2H), 4.23 (d, J = 11.4 Hz, 1H), 3.98 (s, 3H), 3.87 (d, J = 11.4 Hz, 1H), 3.76 (s, 3H), 1.70 (s, 3H) ppm |
| 456 | 491.3 | 1HNMR (400 MHz, DMSO-d6) δ = 9.40 - 9.34 (m, 1H), 9.28 (s, 1H), 9.00 (d, J = 2.0 Hz, 1H), 8.52 (d, J = 8.4 Hz, 1H), 8.42 - 8.40(m, 1H), 8.21 - 8.15 (m, 2H), 7.95 - 7.93(m, 1H), 7.71 (s, 1H), 7.31 (d, J = 8.0 Hz, 1H), 6.46 (d, J = 9.2 Hz, 1H), 4.94 - 4.81 (m, 2H), 4.80 - 4.75 (m, 2H), 4.23 (d, J = 11.2 Hz, 1H), 4.07 - 4.03 (m, 4H), 3.87 (d, J = 11.6 Hz, 1H), 2.39 - 2.35 (m, 2H), 1.70 (s, 3H) ppm |
| 459 | 475.2 | 1HNMR (400 MHz, DMSO-d6) δ = 9.45 - 9.39 (m, 2H), 8.80 (d, J = 7.2 Hz, 1H), 8.73 - 8.68 (m, 2H), 8.43 (d, J = 8.4 Hz, 1H), 8.21 (s, 1H), 8.09 (d, J = 2.0 Hz, 1H), 7.97 - 7.95(m, 1H), 7.87 - 7.82 (m, 2H), 7.32 (d, J = 7.6 Hz, 1H), 6.84 (s, 1H), 4.95 - 4.85 (m, 2H), 4.82 (d, J = 4.4 Hz, 2H), 4.23 (d, J = 11.2 Hz, 1H), 3.87 (d, J = 11.2 Hz, 1H), 1.71 (s, 3H) ppm |
| 460 | 509.9 | 1H NMR (400 MHz, DMSO-d6) δ = 9.46 (s, 1H), 9.45 - 9.42 (m, 1H), 8.71 (d, J = 8.4 Hz, 1H), 8.43 (d, J = 4.4 Hz, 1H), 8.20 (d, J = 1.6 Hz, 1H), 8.11 - 8.01 (m, 2H), 7.96 - 7.94 (m, 1H), 7.83 (s, 1H), 7.78 - 7.71 (m, 1H), 7.45 - 7.41 (m, 1H), 7.31 (d, J = 8.0 Hz, 1H), 4.97 - 4.79 (m, 4H), 4.23 (d, J = 11.6 Hz, 1H), 3.87 (d, J = 11.6 Hz, 1H), 2.81 (d, J = 4.8 Hz, 3H), 1.70 (s, 3H) ppm. |
| 462 | 469.3 | 1H NMR (400 MHz, DMSO-d6) δ= 9.37 - 9.36 (m, 1H), 9.25 (s, 1H), 8.50 (d, J = 8.8 Hz, 1H), 8.19 (d, J = 1.2 Hz, 1H), 8.09 (s,1H), 7.94 - 7.91 (m, 2H), 7.69 (s, 1H), 7.30 (d, J = 8.0 Hz, 1H), 4.88 - 4.76 (m, 4H), 4.22 (d, J = 11.6 Hz, 1H), 4.06 (s, 3H), 3.87 (d, J = 11.2 Hz, 1H), 3.72 (s, 3H), 1.69 (s, 3H) ppm |
| 464 | 507.5 | 1H NMR (400 MHz, MeOD) δ = 9.35 (s, 1H), 8.76 - 8.72 (m, 1H), 8.72 - 8.68 (m, 1H), 8.67 - 8.62 (m, 1H), 8.18 (d, J = 2.0 Hz, 1H), 8.14 - 8.09 (m, 1H), 8.00 (s, 1H), 7.95 - 7.92 (m, 1H), 7.74 (d, J = 7.2 Hz, 1H), 7.30 (d, J = 8.4 Hz, 1H), 4.94 (s, 2H), 4.92 (d, J = 3.6 Hz, 2H), 4.21 (d, J = 11.6 Hz, 1H), 3.93 (d, J = 11.6 Hz, 1H), 3.19 (d, J = 10.4 Hz, 6H), 1.77 (s, 3H) ppm |
| 465 | 534.6 | 1H NMR (400 MHz, MeOD) δ = 9.30 (s, 1H), 8.69 - 8.63 (m, 1H), 8.60 - 8.55 (m, 1H), 8.18 (d, J = 1.6 Hz, 1H), 8.00 - 7.90 (m, 3H), 7.76 - 7.71 (m, 1H), 7.29 (d, J = 8.0 Hz, 1H), 6.98 (d, J = 8.4 Hz, 1H), 4.93 - 4.89 (m, 4H), 4.21 (d, J = 11.2 Hz, 1H), 3.93 (d, J = 11.6 Hz, 1H), 3.75 (br s, 4H), 2.72 - 2.68 (m, 4H), 2.44 (s, 3H), 1.77 (s, 3H) ppm |
| 466 | 493.2 | 1H NMR (400 MHz, MeOD) δ = 9.37 (s, 1H), 8.98 (d, J = 8.8 Hz, 1H), 8.82 (d, J = 7.6 Hz, 1H), 8.68 (d, J = 8.0 Hz, 1H), 8.26 - 8.21 (m, 1H), 8.20 - 8.13 (m, 2H), 8.01 (s, 1H), 7.95 - 7.92 (m, 1H), 7.30 (d, J = 8.0 Hz, 1H), 4.94 (s, 2H), 4.92 (br d, J = 4.0 Hz, 2H), 4.21 (d, J = 11.2 Hz, 1H), 3.93 (d, J = 11.2 Hz, 1H), 3.06 (s, 3H), 1.77 (s, 3H) ppm |
| 467 | 510.2 | 1H NMR (400 MHz, DMSO-d6) δ = 9.44 - 9.39 (m, 2H), 8.68 (d, J = 8.4 Hz, 1H), 8.53 - 8.51 (m, 1H), 8.45 - 8.43 (m, 2H), 8.33 (d, J = 8.4 Hz, 1H), 8.20 (d, J = 1.6 Hz, 1H), 7.96 - 7.94 (m, 1H), 7.82 (s, 1H), 7.48 - 7.47 (m, 1H), 7.31 (d, J = 8.0 Hz, 1H), 4.93 - 4.80 (m, 4H), 4.23 (d, J = 11.2 Hz, 1H), 3.87 (d, J = 11.6 Hz, 1H), 2.81 (d, J = 4.8 Hz, 3H), 1.70 (s, 3H) ppm. |
| 468 | 536.3 | 1H NMR (400 MHz, MeOD-d4) δ = 9.32 (s, 1H), 8.67 - 8.66 (m, 1H), 8.62 (d, J = 8.4 Hz, 1H), 8.41 - 8.34 (m, 1H), 8.23 (d, J = 8.74 Hz, 1H), 8.18 (d, J = 1.6 Hz, 1H), 8.02 - 7.91 (m, 3H), 7.65 (t, J = 8.0 Hz, 1H), 7.29 (d, J = 8.0 Hz, 1H), 4.92 - 4.87 (m, 4H), 4.21 (d, J = 11.6 Hz, 1H), 3.93 (d, J = 11.2 Hz, 1H), 3.60 (s, 4H), 3.38 (s, 3H), 1.76 (s, 3H) ppm. |
| 469 | 486.2 | 1H NMR (400 MHz, DMSO-d6) δ = 9.46-9.42 (m, 2H), 8.77-8.74 (m, 2H), 8.65 (d, J=8.8 Hz, 1H), 8.50-8.44 (m, 1H), 8.24-8.20 (m, 2H), 7.97-7.50 (m, 1H), 7.88 (d, J=5.6 Hz, 2H), 7.31 (d, J=8.8 Hz, 1H), 7.26-6.98 (m, 1H), 4.93-4.82(m, 4H), 4.23(d, J=11.6 Hz, 1H), 3.93-3.80(m, 1H), 1.70 (s, 3H) ppm. |
| 470 | 429.2 | 1H NMR (400 MHz, DMSO-d6) δ = 9.43 - 9.38 (m, 2H), 9.28 (d, J = 1.6 Hz, 1H), 8.67 (d, J = 8.8 Hz, 1H), 8.64 - 8.61 (m, 1H), 8.32 (d, J = 8.4 Hz, 1H), 8.20 (d, J = 1.2 Hz, 1H), 7.96 - 7.94 (m, 1H), 7.87 (d, J = 8.4 Hz, 1H), 7.81 (s, 1H), 7.31 (d, J = 8.0 Hz, 1H), 6.30 (s, 1H), 4.94 - 4.85 (m, 2H), 4.81 (d, J = 6.0 Hz, 2H), 4.23 (d, J = 11.2 Hz, 1H), 3.87 (d, J = 11.2 Hz, 1H), 1.70 (s, 3H), 1.34 - 1.29 (m, 2H), 1.20 - 1.14 (m, 2H) ppm |
| 472 | 480.3 | 1HNMR (400 MHz, MeOD-d4) δ = 9.22 - 9.14 (m, 1H), 9.13 - 9.07 (m, 2H), 8.49 - 8.41 (m, 1H), 8.17 (s, 1H), 8.05 - 7.97 (m, 1H), 7.92 (d, J = 8.0 Hz, 1H), 7.86 - 7.81 (m, 1H), 7.27 (d, J = 8.0 Hz, 1H), 4.90 (d, J = 3.2 Hz, 2H), 4.89 - 4.88 (m, 2H), 4.20 (d, J = 11.2 Hz, 1H), 3.92 (d, J = 11.6 Hz, 1H), 3.27 - 3.24 (m, 6H), 1.75 (s, 3H) ppm |
| 474 | 533 | 1HNMR (400 MHz, METHANOL-d4) δ = 9.40 (s, 1H), 8.64 (d, J = 8.8 Hz, 1H), 8.23 (d, J = 8.8 Hz, 1H), 8.18 (s, 1H), 8.05 - 8.01 (m, 1H), 7.95 - 7.89 (m, 2H), 7.74 (d, J = 7.6 Hz, 1H), 7.53 - 7.45 (m, 1H), 7.33 - 7.21 (m, 2H), 4.95 (s, 2H), 4.91 (d, J = 2.8 Hz, 2H), 4.21 (d, J = 11.6 Hz, 1H), 3.99 (d, J = 12.8 Hz, 2H), 3.95 - 3.89 (m, 1H), 3.64 (d, J = 11.2 Hz, 2H), 3.30 (s, 2H), 3.22 - 3.07 (m, 2H), 2.98 (s, 3H), 1.80 - 1.73 (m, 3H) ppm |
| 475 | 534.2 | 1H NMR (400 MHz, MeOD-d4) δ = 9.25 (s, 1H), 8.53 (d, J = 8.4 Hz, 1H), 8.44 - 8.20 (m, 1H), 8.17 (s, 1H), 8.11 - 8.08 (m, 1H), 8.01 - 7.95 (m, 1H), 7.93 - 7.90 (m, 2H), 7.29 (d, J = 8.0 Hz, 1H), 7.06 (d, J = 8.4 Hz, 1H), 4.91 - 4.90 (m, 4H), 4.39 - 4.37 (m, 2H), 4.21 (d, J = 11.2 Hz, 1H), 4.00 - 3.99 (m, 2H), 3.93 (d, J = 11.6 Hz, 1H), 2.38 (s, 3H), 1.76 (s, 3H) ppm |
| 476 | 504.1 | 1H NMR (400 MHz, MeOD-d4) δ = 9.31 (s, 1H), 8.59 (d, J = 8.4 Hz, 1H), 8.20 - 8.18 (m, 2H), 7.96 - 7.84 (m, 3H), 7.84 (s, 1H), 7.47 (d, J = 8.0 Hz, 1H), 7.30 (d, J = 8.0 Hz, 1H), 4.92 - 4.90 (m, 4H), 4.21 (d, J = 11.6 Hz, 1H), 3.93 (d, J = 11.6 Hz, 1H), 3.65 (s, 2H), 3.32 (s, 3H), 1.77 (s, 3H) ppm. |
| 477 | 502.3 | 1H NMR (400 MHz, DMSO-d6) δ = 9.58 (s, 1H), 9.44 - 9.40 (m, 1H), 9.32 (s, 1H), 8.73 - 8.69 (m, 2H), 8.65 - 8.61 (m, 2H), 8.21 (d, J = 1.6 Hz, 1H), 8.13 (d, J = 8.4 Hz, 1H), 8.01 - 7.94 (m, 3H), 7.73 (s, 1H), 7.33 (d, J = 8.0 Hz, 1H), 4.96 - 4.78 (m, 4H), 4.24 (d, J = 11.6 Hz, 1H), 3.88 (d, J = 11.6 Hz, 1H), 1.71 (s, 3H) ppm |
| 478 | 486.2 | 1H NMR (400 MHz, DMSO-d6) δ = 9.55 (d, J = 1.2 Hz, 1H), 9.45 (s, 1H), 9.43 - 9.38 (m, 1H), 8.86 - 8.83 (m, 1H), 8.76 (d, J = 8.4 Hz, 1H), 8.42 (d, J = 8.4 Hz, 1H), 8.20 (d, J = 1.2 Hz, 1H), 7.97 - 7.94 (m, 1H), 7.91 - 7.85 (m, 2H), 7.31 (d, J = 8.0 Hz, 1H), 7.24 - 6.91 (m, 1H), 4.95 - 4.80 (m, 4H), 4.23 (d, J = 11.6 Hz, 1H), 3.87 (d, J = 11.6 Hz, 1H), 1.70 (s, 3H) ppm |
| 479 | 507.2 | 1H NMR (400 MHz, DMSO-d6) δ = 9.46 - 9.35 (m, 2H), 8.67 (d, J = 8.8 Hz, 1H), 8.50 (s, 1H), 8.31 (d, J = 8.8 Hz, 1H), 8.25 - 8.17 (m, 2H), 7.96 - 7.94 (m, 1H), 7.87 - 7.76 (m, 2H), 7.62 - 7.58 (m, 1H), 7.31 (d, J = 8.0 Hz, 1H), 6.74 - 6.18 (m, 1H), 4.94 - 4.76 (m, 8H), 4.23 (d, J = 11.6 Hz, 1H), 3.87 (d, J = 11.6 Hz, 1H), 1.70 (s, 3H) ppm. |
| 480 | 519.2 | 1H NMR (400 MHz, DMSO-d6) δ = 9.42 - 9.39 (m, 1H), 9.36 (s, 1H), 8.52 (d, J = 8.8 Hz, 1H), 8.20 (d, J = 1.6 Hz, 1H), 8.13 (s, 1H), 8.05 - 8.01 (m, 2H), 7.96 - 7.94 (m, 1H), 7.78 (s, 1H), 7.33 - 7.27 (m, 2H), 4.93 - 4.85 (m, 2H), 4.81 (d, J = 5.6 Hz, 2H), 4.22 (d, J = 11.6 Hz, 1H), 4.05 (s, 3H), 3.95 (s, 3H), 3.86 (d, J = 11.6 Hz, 1H), 1.70 (s, 3H) ppm |
| 481 | 483.1 | 1H NMR (400 MHz, DMSO-d6) δ = 9.43 - 9.34 (m, 2H), 8.54 (d, J = 8.4 Hz, 1H), 8.19 (d, J = 1.2 Hz, 1H), 8.05 (d, J = 8.4 Hz, 1H), 7.95 - 7.93 (m, 1H), 7.86 - 7.77 (m, 1H), 7.77 (s, 1H), 7.30 (d, J = 8.0 Hz, 1H), 7.14 - 7.11 (m, 1H), 6.93 - 6.92 (m, 1H), 4.94 - 4.76 (m, 4H), 4.22 (d, J = 11.6 Hz, 1H), 3.88 - 3.84 (m, 4H), 1.70 (s, 3H) ppm. |
| 482 | 466.2 | 1H NMR (400 MHz, DMSO-d6) δ = 9.46 - 9.36 (m, 2H), 9.05 (d, J = 1.6 Hz, 1H), 8.71 (d, J = 8.8 Hz, 1H), 8.45 (d, J = 2.8 Hz, 1H), 8.43 (br s, 1H), 8.38 (d, J = 8.4 Hz, 1H), 8.20 - 8.15 (m, 2H), 7.97 - 7.92 (m, 1H), 7.85 (s, 1H), 7.30 (d, J = 8.4 Hz, 1H), 4.95 - 4.77 (m, 4H), 4.26 - 4.19 (m, 1H), 3.94 (s, 3H), 3.86 (d, J = 11.6 Hz, 1H), 1.70 (s, 3H) ppm |
| 483 | 492.2 | 1H NMR (400 MHz, DMSO-d6) δ = 9.46 - 9.35 (m, 2H), 8.73 - 8.68 (m, 2H), 8.66 - 8.60 (m, 1H), 8.43 (d, J = 8.0 Hz, 1H), 8.37 (br s, 1H), 8.34 (d, J = 8.8 Hz, 1H), 8.20 (d, J = 1.2 Hz, 1H), 8.02 - 7.93 (m, 2H), 7.84 (s, 1H), 7.69 - 7.61 (m, 1H), 7.31 (d, J =8.0 Hz, 1H), 4.96 - 4.75 (m, 4H), 4.23 (d, J = 11.2 Hz, 1H), 3.87 (d, J = 11.2 Hz, 1H), 2.83 (d, J = 4.4 Hz, 3H), 1.71 (s, 3H) ppm |
| 484 | 518.2 | 1HNMR (400 MHz, DMSO-d6) δ = 9.41 - 9.36 (m, 2H), 8.69 (d, J = 8.0 Hz, 1H), 8.50 (s, 1H), 8.43 - 8.39 (m, 1H), 8.34 (d, J = 8.4 Hz, 1H), 8.21 (d, J = 1.2 Hz, 1H), 7.96 - 7.94 (m, 1H), 7.84 (s, 1H), 7.76 (d, J = 7.6 Hz, 1H), 7.65 - 7.62 (m, 1H), 7.31 (d, J = 7.6 Hz, 1H), 4.93 - 4.81 (m, 4H), 4.36 - 4.32 (m, 2H), 4.24 - 4.16 (m, 1H), 4.10 - 4.06 (m, 2H), 3.86 (d, J = 11.6 Hz, 1H), 2.29 - 2.26 (m, 2H), 1.70 (s, 3H) ppm. |
| 485 | 504.2 | 1H NMR (400 MHz, DMSO-d6) δ = 9.51 - 9.29 (m, 2H), 8.68 (d, J = 8.4 Hz, 1H), 8.59 - 8.50 (m, 2H), 8.41 (d, J = 8.8 Hz, 1H), 8.20 (s, 1H), 7.97 - 7.95 (m, 1H), 7.85 (s, 1H), 7.77 (d, J = 8.4 Hz, 1H), 7.31 (d, J = 8.0 Hz, 1H), 4.98 - 4.77 (m, 4H),4.56 (s, 2H), 4.24 (s, 1H), 3.87 (d, J = 7.2 Hz, 1H), 3.12 (s, 3H), 1.71 (s, 3H) ppm. |
| 487 | 509.2 | 1H NMR (400 MHz, DMSO-d6) δ = 9.57 - 9.54 (m, 1H), 9.42 - 9.37 (m, 1H), 9.07 (d, J = 2.0 Hz, 1H), 8.65 (d, J = 8.8 Hz, 1H), 8.56 (d, J = 2.0 Hz, 1H), 8.26 (d, J = 8.8 Hz, 1H), 7.87 (s, 1H), 7.37 - 7.30 (m, 1H), 7.12 - 7.11 (m, 1H), 6.39 - 6.35 (m, 1H), 4.93 - 4.80 (m, 4H), 4.29 (d, J = 11.6 Hz, 1H), 3.95 - 3.89 (m, 5H), 2.52 (s, 1H), 2.38 - 2.34 (m, 1H), 1.72 (s, 3H) ppm. |
| 488 | 466.1 | 1H NMR (400 MHz, DMSO-d6) δ = 9.46 - 9.32 (m, 2H), 8.61 (d, J = 8.8 Hz, 1H), 8.39 - 8.25 (m, 2H), 8.23 - 8.12 (m, 2H), 7.94(d, J = 8.0 Hz, 1H), 7.79 (s, 1H), 7.30 (d, J = 8.0 Hz, 1H), 7.19 (d, J = 2.0 Hz, 1H), 4.98 - 4.73 (m, 4H), 4.22 (d, J =11.6 Hz, 1H), 3.98 (s, 3H), 3.86 (d, J = 11.6 Hz, 1H), 1.69 (s, 3H) ppm. |
| 489 | 466.3 | 1H NMR (400 MHz, DMSO-d6) δ = 9.40 - 9.38 (m, 2H), 8.79 (s, 1H), 8.61 - 8.56 (m, 2H), 8.18 (d, J = 1.2 Hz, 1H), 8.06 (d, J =8.4 Hz, 1H), 7.95-7.93 (m, 1H), 7.82 (s, 1H), 7.30 - 7.27 (m, 2H), 4.92 - 4.80 (m, 4H), 4.22 (d, J = 11.6 Hz, 1H),3.94 (s, 3H), 3.86 (d, J = 11.6 Hz, 1H), 1.69 (s, 3H) ppm |
| 490 | 506.2 | 1H NMR (400 MHz, DMSO-d6) δ=9.41 - 9.38 (m, 2H), 8.68 (d, J = 8.8 Hz, 1H), 8.41 (br s, 1H), 8.37 - 8.30 (m, 3H), 8.20 (d, J= 1.2 Hz, 1H), 7.96 - 7.94 (m, 1H), 7.82 (s, 1H), 7.64 - 7.60 (m, 1H), 7.57 - 7.55 (m, 1H), 7.31 (d, J = 8.0 Hz, 1H), 4.93 - 4.80 (m, 4H), 4.23 (d, J = 11.2 Hz, 1H), 3.87 (d, J = 11.2 Hz, 1H), 3.02 - 2.94 (m, 6H), 1.70 (s, 3H) ppm |
| 491 | 519.2 | 1H NMR (400 MHz, DMSO-d6) δ = 9.46 - 9.35 (m, 2H), 8.65 (d, J = 8.0 Hz, 1H), 8.22 - 8.14 (m, 2H), 8.12 (s, 1H), 7.98 - 7.91 (m, 1H), 7.83 (s, 1H), 7.65 - 7.59 (m, 1H), 7.56 - 7.50 (m, 1H), 7.30 (d, J = 8.4 Hz, 1H), 4.95 - 4.77 (m, 4H), 4.27 (s, 3H), 4.22 (d, J = 11.2 Hz, 1H), 3.86 (d, J = 11.6 Hz, 1H), 3.59 (s, 3H), 1.70 (s, 3H) ppm |
| 492 | 466.2 | 1HNMR (400 MHz, DMSO-d6) δ = 9.45 - 9.37 (m, 2H), 8.76 - 8.64 (m, 2H), 8.25 - 8.18 (m, 2H), 7.97 - 7.94 (m, 1H), 7.93 - 7.88 (m, 1H), 7.83 (s, 1H), 7.31 (d, J = 8.0 Hz, 1H), 7.01 (d, J = 8.0 Hz, 1H), 4.95 - 4.84 (m, 2H), 4.82 (d, J = 5.6 Hz, 2H), 4.23 (d, J = 11.6 Hz, 1H), 4.04 (s, 3H), 3.87 (d, J = 11.6 Hz, 1H), 1.71 (s, 3H) ppm |
| 493 | 475.2 | 1HNMR (400 MHz, DMSO-d6) δ = 9.38 - 9.35 (m, 1H), 9.24 (s, 1H), 8.97 (s, 1H), 8.88 - 8.83 (m, 2H), 8.50 (d, J = 8.4 Hz, 1H), 8.23 - 8.18 (m, 2H), 7.96 - 7.93 (m, 1H), 7.80 (s, 1H), 7.60 - 7.53 (m, 1H), 7.31 (d, J = 8.0 Hz, 1H), 7.17 - 7.11 (m, 1H), 4.95 - 4.83 (m, 2H), 4.83 - 4.74 (m, 2H), 4.23 (d, J = 11.5 Hz, 1H), 3.88 (d, J = 11.2 Hz, 1H), 1.71 (s, 3H) ppm |
| 494 | 435.2 | 1H NMR (400 MHz, METHANOL-d4) δ = 8.51 (d, J = 8.8 Hz, 1H), 8.25 - 8.21 (m, 3H), 8.15 (d, J = 2.0 Hz, 1H), 7.91 - 7.88 (m, 1H), 7.84 - 7.81 (m, 2H), 7.53 - 7.51 (m, 2H), 7.49 - 7.45 (m, 1H), 7.27 (d, J = 8.0 Hz, 1H), 5.15 (s, 2H), 4.89 (d, J = 3.6 Hz, 2H), 4.20 (d, J = 11.2 Hz, 1H), 3.92 (d, J = 11.6 Hz, 1H), 1.74 (s, 3H) ppm |
| 495 | 504.2 | 1H NMR (400 MHz, DMSO-d6) δ = 9.44 - 9.41 (m, 2H), 8.72 (d, J = 8.4 Hz, 1H), 8.52 (s, 1H), 8.44 (s, 1H), 8.41 - 8.39 (m, 1H), 8.37 (d, J = 8.8 Hz, 1H), 8.22 (d, J = 1.2 Hz, 1H), 7.98-7.96 (m, 1H), 7.83 - 7.81 (m, 2H), 7.33 (d, J = 8.0 Hz, 1H), 4.94 - 4.82 (m, 4H), 4.57 (s, 2H), 4.24 (d, J = 11.6 Hz, 1H), 3.88 (d, J = 11.2 Hz, 1H), 3.12 (s, 3H), 1.71 (s, 3H) ppm |
| 496 | 491.2 | 1H NMR (400 MHz, DMSO-d6) δ = 9.50 - 9.31 (m, 2H), 8.74 - 8.54 (m, 2H), 8.21 (s, 1H), 7.98 - 7.95 (m, 1H), 7.88 (d, J = 7.2 Hz, 1H), 7.82 (s, 1H), 7.72 - 7.70 (m, 1H), 7.32 (d, J = 8.0 Hz, 1H), 6.54 (d, J = 8.0 Hz, 1H), 4.95 - 4.80 (m, 4H), 4.24 (d, J = 11.6 Hz, 1H), 4.09 - 4.05 (m, 4H), 3.88 (d, J = 11.6 Hz, 1H), 2.43 - 2.33 (m, 2H), 1.71 (s, 3H) ppm |
| 497 | 492.2 | 1HNMR (400 MHz, DMSO-d6) δ = 9.40 - 9.37 (m, 1H), 9.25 (s, 1H), 8.63 (s, 1H), 8.51 (d, J = 8.4 Hz, 1H), 8.26 - 8.20 (m, 2H), 8.00 - 7.94 (m, 2H), 7.66 (s, 1H), 7.32 (d, J = 8.0 Hz, 1H), 5.11 - 5.07 (m, 1H), 4.94 - 4.77 (m, 2H), 4.78 (br d, J = 5.2 Hz, 2H), 4.24 (d, J = 11.6 Hz, 1H), 4.02 - 3.96 (m, 3H), 3.89 - 3.83 (m, 2H), 2.44 - 2.35 (m, 2H), 1.71 (s, 3H) ppm. |
| 498 | 504.1 | 1H NMR (400 MHz, DMSO-d6) δ = 9.40-9.37 (m, 1H), 9.32 (s, 1H), 8.59 (d, J = 8.4 Hz, 1H), 8.28-8.24 (m, 3H), 8.20 (d, J =1.2 Hz, 1H), 7.97-7.94 (m, 1H), 7.74 (s, 1H), 7.32 (d, J = 8.4 Hz, 1H), 7.15 (d, J = 8.4 Hz, 1H), 4.93-4.89 (m, 2H), 4.80 (d, J= 6.0 Hz, 2H), 4.24 (d, J = 11.6 Hz, 1H), 3.87 (d, J = 11.6 Hz, 1H), 3.66 (s, 2H), 3.18 (s, 3H), 1.70 (s, 3H) ppm. |
| 499 | 502.4 | 1H NMR (400 MHz, MeOD) δ = 9.23 (s, 1H), 9.18 (s, 1H), 9.14 (d, J = 1.6 Hz, 1H), 8.56 (d, J = 4.4 Hz, 1H), 8.53 - 8.51 (m, 2H), 8.33 (d, J = 8.0 Hz, 1H), 8.18 (s, 1H), 8.06 (d, J = 8.4 Hz, 1H), 7.95-7.93 (m, 1H), 7.86 (s, 1H), 7.61-7.60 (m, 1H), 7.31(d, J = 8.0 Hz, 1H), 4.92-4.90 (m, 4H), 4.22 (d, J = 11.2 Hz, 1H), 3.95 (d, J = 11.6 Hz, 1H), 1.77 (s, 3H).ppm |
| 500 | 486.1 | 1HNMR (400 MHz, MeOD-d4) δ = 9.31 (s, 1H), 9.05 (d, J = 2.0 Hz, 1H), 8.65 - 8.57 (m, 1H), 8.55 (d, J = 2.0 Hz, 1H), 8.41 (s, 1H), 8.34 (d, J = 7.2 Hz, 1H), 8.20 (d, J = 8.4 Hz, 1H), 8.00 (s, 1H), 7.75 - 7.63 (m, 2H), 6.89 (s, 1H), 4.95 - 4.89 (m, 4H), 4.26 (d, J = 11.6 Hz, 1H), 3.97 (d, J = 11.6 Hz, 1H), 1.78 (s, 3H) ppm. |
| 501 | 453.1 | 1HNMR (400 MHz, MeOD-d4) δ = 9.30 (s, 1H), 8.55 (s, 1H), 8.18 (s, 1H), 8.04 - 7.88 (m, 3H), 7.30 (d, J = 8.4 Hz, 1H), 6.82 (s, 1H), 4.93 (s, 4H), 4.30 (s, 3H), 4.22 (d, J = 11.6 Hz, 1H), 3.94 (d, J = 10.8 Hz, 1H), 2.32 (s, 3H), 1.78 (s, 3H) ppm |
| 504 | 475.1 | 1H NMR (400 MHz, DMSO-d6) δ = 9.42-9.39 (m, 1H), 9.32 (s, 1H), 9.13 (s, 1H), 8.61 (d, J = 8.4 Hz, 1H), 8.56 (s, 1H), 8.21 (d, J = 1.2 Hz, 1H), 8.14 - 7.90 (m, 3H), 7.75 - 7.72 (m, 1H), 7.32 (d, J = 8.0 Hz, 1H), 4.94 - 4.83 (m, 2H), 4.80 (br d, J = 5.6 Hz, 2H), 4.24 (d, J = 11.6 Hz, 1H), 3.88 (d, J = 11.2 Hz, 1H), 1.71 (s, 3H) ppm |
| 506 | 492.3 | 1H NMR (400 MHz, MeOD-d4) δ = 9.36 (s, 1H), 8.64 (s, 2H), 8.47 (d, J = 5.2 Hz, 1H), 8.17 (d, J = 1.2 Hz, 1H), 7.98 (s,1H), 7.93 - 7.91 (m, 1H), 7.76 (d, J = 4.8 Hz, 1H), 7.29 (d, J = 8.4 Hz, 1H), 4.93 - 4.90 (m, 4H), 4.27 - 4.19 (m, 5H), 3.93 (d, J = 11.6 Hz, 1H), 2.47 - 2.43 (m, 2H), 1.77 (s, 3H) ppm |
| 507 | 466.3 | 1H NMR (400 MHz, MeOD) δ = 9.37 (s, 1 H), 8.60 (d, J = 8.8 Hz, 1 H), 8.52 (s, 1 H), 8.36 (d, J = 4.8 Hz, 1 H), 8.16-8.13 (m, 2 H), 7.96 (s, 1H), 7.91-7.86 (m, 1 H), 7.83 (d, J = 4.8 Hz, 1 H), 7.29 (d, J = 8.0 Hz, 1 H), 4.95 - 4.93 (m, 2 H), 4.91 (d, J = 3.6 Hz, 2 H), 4.21 (d, J = 11.6 Hz, 1 H), 4.03 (s, 3H), 3.93 (d, J = 11.2 Hz, 1 H), 1.75 (s, 3H) ppm. |
| 508 | 435.1 | 1HNMR (400 MHz, DMSO-d6) δ = 9.60 (s, 1H), 9.52 (s, 1H), 9.45 - 9.38 (m, 1H), 8.50 (s, 1H), 8.26 (d, J=7.2 Hz, 2H), 8.21 (s, 1H), 8.00 - 7.93 (m, 1H), 7.80 (s, 1H), 7.58 - 7.44 (m, 3H), 7.32 (d, J=8.0 Hz, 1H), 4.96 - 4.83 (m, 2H), 4.80 (d, J=5.6 Hz, 2H), 4.24 (d, J=11.6 Hz, 1H), 3.88 (d, J=11.6 Hz, 1H), 1.71 (s, 3H) ppm |
| 509 | 453.1 | 1HNMR (400 MHz, MeOD-d4) δ = 9.18 (s, 1H), 8.42 (d, J = 8.8 Hz, 1H), 8.27 (s, 1H), 8.16 (d, J = 1.6 Hz, 1H), 7.92 - 7.90 (m, 1H), 7.86 - 7.80 (m, 2H), 7.29 (d, J = 8.4 Hz, 1H), 4.91 (d, J = 3.6 Hz, 2H), 4.89 (s, 2H), 4.20 (d, J = 11.6 Hz, 1H), 3.93 (d, J = 11.2 Hz, 1H), 3.90 (s, 3H), 2.62 (s, 3H), 1.76 (s, 3H) ppm |
| 510 | 466.2 | 1H NMR (400 MHz, MeOD) δ = 9.68 - 9.61 (m, 1H), 9.10 - 8.99 (m, 1H), 8.96 - 8.81 (m, 2H), 8.35 - 8.21 (m, 2H), 8.18 (d, J = 2.0 Hz, 1H), 7.96 - 7.93 (m, 1H), 7.32 (d, J = 8.0 Hz, 1H), 6.85 - 6.77 (m, 1H), 5.02 (s, 2H), 4.93 (d, J = 2.8 Hz, 2H), 4.22 (d, J = 11.6 Hz, 1H), 3.96 (d, J = 11.6 Hz, 1H), 3.81 (s, 3H), 1.81 - 1.75 (m, 3H) ppm |
| 511 | 435.1 | 1HNMR (400 MHz, DMSO-d6) δ = 9.45 - 9.39 (m, 2H), 9.39 (d, J=2.4 Hz, 1H), 8.76 (d, J=2.4 Hz, 1H), 8.20 (d, J=1.6 Hz, 1H), 7.98 - 7.92 (m, 3H), 7.87 (s, 1H), 7.61 - 7.55 (m, 2H), 7.53 - 7.48 (m, 1H), 7.31 (d, J=8.0 Hz, 1H), 4.95 - 4.85 (m, 2H), 4.81 (d, J=6.0 Hz, 2H), 4.23 (d, J=11.6 Hz, 1H), 3.89 - 3.86 (m, 1H), 1.70 (s, 3H) ppm |
| 512 | 466.2 | 1HNMR (400 MHz, MeOD-d4) δ = 9.36 (s, 1H), 8.65 (d, J = 8.8 Hz, 1H), 8.30 (d, J = 5.6 Hz, 1H), 8.23 - 8.13 (m, 2H), 7.99 (s, 1H), 7.94 - 7.91 (m, 1H), 7.77 - 7.71 (m, 1H), 7.58 (s, 1H), 7.29 (d, J = 8.0 Hz, 1H), 4.96 - 4.90 (m, 4H), 4.20 (d, J = 11.6 Hz, 1H), 3.99 (s, 3H), 3.93 (d, J = 11.6 Hz, 1H), 1.76 (s, 3H) ppm |
| 513 | 481.1 | 1H NMR (400 MHz, MeOD-d4) δ = 9.20 (s, 1H), 8.58 (s, 1H), 8.47 - 8.45 (m, 1H), 8.36 (s, 1H), 8.18 (d, J = 1.6 Hz, 1H), 7.95 - 7.91 (m, 2H), 7.83 (s, 1H), 7.29 (d, J = 8.4 Hz, 1H), 5.68- 5.61 (m, 1H), 5.09 (s, 2H), 5.08 (s, 2H), 4.92 (d, J = 3.6 Hz, 2H), 4.89 (s, 2H), 4.21 (d, J = 11.6 Hz, 1H), 3.93 (d, J = 11.6 Hz, 1H), 1.77 (s, 3H) ppm |
| 515 | 501.2 | 1H NMR (400 MHz, DMSO-d6) δ = 9.47 (s, 1H), 9.39 - 9.38 (m, 1H), 8.71 (d, J = 5.6 Hz, 1H), 8.17 (d, J = 1.6 Hz, 1H),7.93 - 7.92 (m, 1H), 7.83 (d, J = 8.8 Hz, 1H), 7.80 (s, 1H), 7.36 - 7.35 (m, 1H), 7.30 - 7.17 (m, 2H), 4.96 - 4.73 (m, 4H), 4.21 (d, J = 3.6 Hz, 1H), 3.91 - 3.81 (m, 4H), 1.68 (s, 3H) ppm |
| 516 | 483.2 | 1H NMR (400 MHz, DMSO-d6) δ = 9.46 - 9.38 (m, 2H), 8.65 (s, 1H), 8.19 (d, J = 1.6 Hz, 1H), 8.01 (d, J = 8.4 Hz, 1H), 7.95 (d, J = 1.6, 1H), 7.82 (s, 1H), 7.61 (d, J = 7.6 Hz, 1H), 7.32 - 7.29 (m, 1H), 7.34 - 7.22 (m, 2H), 4.94 - 4.80 (m, 4H), 4.23 (d, J = 11.6 Hz, 1H), 3.87 (d, J = 11.6 Hz, 1H), 3.82 (d, J = 1.6 Hz, 3H), 1.70 (s, 3H) ppm. |
| 517 | 483.2 | 1H NMR (400 MHz, DMSO-d6) δ = 9.43 - 9.33 (m, 2H), 8.57 (d, J = 8.2 Hz, 1H), 8.18 (d, J = 1.6 Hz, 1H), 8.09 (d, J = 8.4 Hz,1H), 7.93 (d, J = 1.6, 1H), 7.79 (s, 1H), 7.60 (d, J = 2.0 Hz, 1H), 7.38 - 7.27 (m, 2H), 7.26 - 7.21 (m, 1H), 4.93 -4.76 (m, 4H), 4.21 (d, J = 11.6 Hz, 1H), 3.88 - 3.83 (m, 4H), 1.69 (s, 3H) ppm |
| 518 | 471.2 | 1HNMR (400 MHz, DMSO-d6) δ = 9.42 - 9.39 (m, 2H), 8.72 (d, J = 8.8 Hz, 1H), 8.44 (s, 1H), 8.36 (d, J = 8.8 Hz, 1H), 8.19 (d, J = 1.3 Hz, 1H), 8.06 - 8.02 (m, 2H), 7.96 - 7.94 (m, 1H), 7.84 (s, 1H), 7.48 - 7.42 (m, 1H), 7.31 (d, J = 8.0 Hz, 1H), 4.93 - 4.81 (m, 4H), 4.23 (d, J = 11.2 Hz, 1H), 3.87 (d, J = 11.6 Hz, 1H), 1.70 (s, 3H) ppm |
| 519 | 485.2 | 1HNMR (400 MHz, DMSO-d6) δ = 9.43 - 9.31 (m, 2H), 8.69 - 8.61 (m, 2H), 8.21- 8.10(m, 2H), 7.96- 7.94 (m, 1H), 7.78 - 7.76 (m, 1H), 7.66 (s, 1H), 7.31 (d, J = 8.0 Hz, 1H), 4.93 - 4.78 (m, 4H), 4.24 - 4.19 (m, 1H), 3.87 (d, J = 11.6 Hz, 1H), 2.11 (s, 3H), 1.70 (s, 3H) ppm |
| 520 | 437.1 | 1H NMR (400 MHz, DMSO-d6) δ=9.74 (d, J = 1.2 Hz, 1H), 9.47 (s, 1H), 9.44 - 9.41 (m, 1H), 8.85 - 8.76 (m, 3H), 8.60 (d, J =8.8 Hz, 1H), 8.20 (d, J = 1.2 Hz, 1H), 7.96 - 7.94 (m, 1H), 7.90 (s, 1H), 7.31 (d, J = 8.0 Hz, 1H), 4.93 - 4.83 (m, 4H), 4.23 (d, J= 11.6 Hz, 1H), 3.87 (br d, J = 11.6 Hz, 1H), 1.70 (s, 3H) ppm |
| 521 | 485.2 | 1HNMR (400 MHz, DMSO-d6) δ = 9.46 (s, 1H), 9.42 - 9.36 (m, 1H), 8.72 (d, J = 8.8 Hz, 1H), 8.17 (d, J = 1.6 Hz, 1H), 7.98 - 7.91 (m, 2H), 7.86 - 7.77 (m, 3H), 7.75 - 7.66 (m, 2H), 7.61 (s, 0.3H), 7.47 (s, 0.5H), 7.33 (s, 0.2H), 7.29 (d, J = 8.0 Hz, 1H), 4.94 - 4.75 (m, 4H), 4.21 (d, J = 11.6 Hz, 1H), 3.86 (d, J = 11.6 Hz, 1H), 1.69 (s, 3H) ppm |
| 522 | 435.2 | 1H NMR (400 MHz, DMSO-d₆) δ = 9.61 (s, 1H), 9.34 - 9.31 (m, 1H), 8.27 - 8.16 (m, 3H), 8.08 (d, J = 1.6 Hz, 1H),7.95 - 7.86 (m, 3H), 7.58 - 7.51 (m, 2H), 7.50 - 7.45 (m, 1H), 7.29 (d, J = 8.4 Hz, 1H), 4.97 - 4.77 (m, 4H), 4.23 (d, J = 11.2 Hz,1H), 3.87 (d, J = 11.2 Hz, 1H), 1.70 (s, 3H) ppm |
| 525 | 464.2 | 1HNMR (400MHz, DMSO-d6) δ = 9.53 - 9.30 (m, 2H), 8.66 (d, J = 8.4 Hz, 1H), 8.39 - 8.21 (m, 4H), 8.09 (d, J = 8.0 Hz, 1H), 7.81 (s, 1H), 7.66 - 7.51 (m, 3H), 7.43 (d, J = 8.0 Hz, 1H), 6.98 - 6.55 (m, 1H), 5.01 - 4.74 (m, 4H), 4.36 - 4.09 (m, 2H) ppm |

### Example 29: (R)-4-Cyano-N-((2-(3-(dimethylamino)-2-oxopyridin-1(2H)-yl)-1,6-naphthyridin-7-yl)methyl)-4-methylisochromane-6-carboxamide

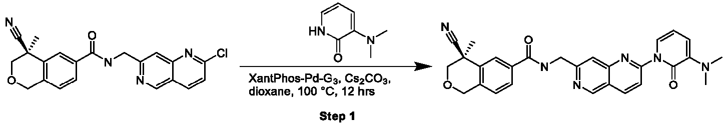

### Step 1: (R)-4-Cyano-N-((2-(3-(dimethylamino)-2-oxopyridin-1(2H)-yl)-1,6-naphthyridin-7-yl)methyl)-4-methylisochromane-6-carboxamide

To a solution of (R)-N-((2-chloro-1,6-naphthyridin-7-yl)methyl)-4-cyano-4-methylisochromane-6-carboxamide (30 mg, 76.37 umol) and 3-(dimethylamino)pyridin-2(1H)-one (21.10 mg, 152.73 umol) in dioxane (1 mL) was added Cs₂CO₃ (74.65 mg, 229.10 umol) and XantPhos Pd G₃ (7.24 mg, 7.64 umol). The mixture was stirred at 100 °C for 12 hrs under N₂. The mixture was diluted with H₂O (10 mL) and extracted with EA (10 mL*2). The combined organic layers were dried over anhydrous Na₂SO₄, filtered and concentrated. The residue was purified by reverse phase prep-HPLC (0.1% FA condition) to give the title compound (12.66 mg, 22.61 umol, 29.61% yield, FA salt) as a yellow solid.
LCMS (ESI) m/z: [M+H]⁺ = 495.4.
¹H NMR (400 MHz, MeOD) δ = 9.39 (s, 1H), 8.65 (d, *J* = 8.8 Hz, 1H), 8.15 (d, *J* = 1.6 Hz, 1H), 7.99 (d, *J* = 8.8 Hz, 1H), 7.91 - 7.89 (m, 2H), 7.60 - 7.58 (m, 1H), 7.28 (d, *J* = 8.0 Hz, 1H), 6.93 - 6.91 (m, 1H), 6.46 - 6.43 (m, 1H), 4.92 (s, 2H), 4.90 (d, *J* = 4.0 Hz, 2H), 4.21 (d, *J* = 11.6 Hz, 1H), 3.93 (d, *J* = 11.6 Hz, 1H), 2.85 (s, 6H), 1.75 (s, 3H) ppm.
Chiral SFC: Cellucoat-MeOH+ACN(DEA)-40-3mL-35T.Icm, Rt = 0.908 min, ee value = 100%.

The following examples in Table 20 were prepared using standard chemical manipulations and procedures similar to those used for the preparation of **Examples 29.**

**Table 20. Compounds of the Invention**

| # | LCMS (m/z) | 1H NMR |
|---|---|---|
| 142 | 498.4 | 1H NMR (400 MHz, DMSO-d6) δ = 9.48 - 9.45 (m, 1H), 8.75 (d, J = 4.4 Hz, 1H), 8.50 (d, J = 9.6 Hz, 1H), 8.30 - 8.27 (m, 1H), 8.18 - 8.13 (m, 2H), 8.05 (s, 1H), 7.95 - 7.92 (m, 1H), 7.30 (d, J = 8.0 Hz, 1H), 5.01 (d, J = 5.6 Hz, 2H), 4.94 - 4.80 (m, 2H), 4.22 (d, J = 11.6 Hz, 1H), 4.02 - 3.98 (m, 4H), 3.86 (d, J = 11.6 Hz, 1H), 2.42 - 2.34 (m, 2H), 1.69 (s, 3H) ppm |
| 219 | 530.5 | 1H NMR (400 MHz, METHANOL-d4) δ = 8.29 (d, J = 9.6 Hz, 1H), 8.14 (d, J = 1.6 Hz, 1H), 7.94 - 7.87 (m, 3H), 7.68 (s, 1H), 7.27 (d, J = 8.0 Hz, 1H), 7.21 (d, J = 2.4 Hz, 1H), 7.06 - 6.76 (m, 1H), 5.04 (s, 2H), 4.90 (s, 2H), 4.84 (s, 2H), 4.37 - 4.35 (m, 2H), 4.20 (d, J = 11.6 Hz, 1H), 4.11 (t, J = 5.6 Hz, 2H), 3.92 (d, J = 11.6 Hz, 1H), 1.75 (s, 3H) ppm |
| 220 | 530.4 | 1H NMR (400 MHz, DMSO-d6) δ = 9.36 - 9.33 (m, 1H), 9.14 (s, 1H), 9.08 (d, J = 2.8 Hz, 1H), 8.19 (d, J = 1.6 Hz, 1H), 7.96 -7.94 (m, 1H), 7.71 (s, 1H), 7.66 (d, J = 2.4 Hz, 1H), 7.57 (s, 1H), 7.30 (d, J = 8.0 Hz, 1H), 7.25 - 6.92 (m, 1H), 4.94 - 4.81 (m, 2H), 4.79 (s, 2H), 4.72 (d, J = 6.0 Hz, 2H), 4.32 - 4.30 (m, 2H), 4.23 (d, J = 11.2 Hz, 1H), 4.12 - 4.05 (m, 2H), 3.86 (d, J = 11.6 Hz, 1H), 1.69 (s, 3H)ppm |
| 279 | 512 | 1H NMR (400 MHz, DMSO-d6) δ= 9.36 - 9.33 (m, 1H), 9.22 (s, 1H), 8.55 (d, J = 9.2 Hz, 1H), 8.25 (s, 1H), 7.96 - 7.94 (m, 1H), 7.52 - 7.47 (m, 2H), 7.30 (d, J = 8.0 Hz, 1H), 4.87 - 4.73 (m, 4H), 4.12 - 4.07 (m, 3H), 3.88 - 3.82 (m, 1H), 3.61 - 3.57 (m, 2H), 2.45 - 2.43 (m, 1H), 1.71 - 1.66 (m, 3H), 0.77 - 0.75 (m, 4H) ppm |
| 282 | 492.2 | 1H NMR (400 MHz, MeOH) δ = 9.43 - 9.36 (m, 1H), 8.69 - 8.61 (m, 1H), 8.17 - 8.13 (m, 1H), 8.05 - 7.99 (m, 1H),7.94 - 7.87 (m, 2H), 7.87 - 7.81 (m, 1H), 7.31 - 7.24 (m, 1H), 7.24 - 7.19 (m, 1H), 6.49 - 6.38 (m, 1H), 4.94 - 4.92 (m, 2H), 4.91- 4.89 (m, 2H), 4.23 - 3.87 (m, 2H), 2.13 - 2.02 (m, 1H), 1.79 - 1.73 (m, 3H), 1.02 - 0.89 (m, 2H), 0.74 - 0.66 (m, 2H) ppm |
| 283 | 507 | 1H NMR (400 MHz, MeOD) δ = 9.40 (s, 1H), 8.65 (d, J = 8.4 Hz, 1H), 8.17 (d, J = 1.2 Hz, 1H), 7.98 (d, J = 8.4 Hz,1H), 7.95 - 7.89 (m, 2H), 7.43-7.41 (m, 1H), 7.29 (d, J = 8.0 Hz, 1H), 6.46 - 6.38 (m, 2H), 4.95 - 4.93 (m, 2H), 4.93- 4.91 (m, 2H), 4.22 (d, J = 11.2 Hz, 1H), 4.03-4.01 (m, 4H), 3.94 (d, J = 11.2 Hz, 1H), 2.36-2.32 (m, 2H), 1.77 (s, 3H) ppm |
| 303 | 490.1 | 1HNMR (400 MHz, MeOD-d4) δ = 9.20 - 9.13 (m, 1H), 8.98 (s, 1H), 8.32 - 8.27 (m, 1H), 8.07 - 8.02 (m, 1H), 8.01 - 7.95 (m, 1H), 7.85 - 7.78 (m, 2H), 7.73 - 7.65 (m, 1H), 7.64 - 7.59 (m, 1H), 7.58 - 7.43 (m, 2H), 7.18 - 7.12 (m, 1H), 5.30 - 5.23 (m, 2H), 4.95 (d, J = 5.1 Hz, 2H), 4.91 - 4.85 (m, 2H), 4.20 - 4.10 (m, 1H), 4.01 - 3.91 (m, 1H), 1.81 - 1.75 (m, 3H) ppm |
| 312 | 483.2 | 1H NMR (400 MHz, DMSO-d6) δ = 9.41 - 9.29 (m, 1H), 9.14 (s, 1H), 8.61 - 8.55 (m, 1H), 8.43 - 8.36 (m, 1H), 8.22 - 8.16 (m, 1H), 7.98 - 7.89 (m, 1H), 7.50 (s, 1H), 7.34 - 7.27 (m, 1H), 4.94 - 4.80 (m, 2H), 4.77 - 4.66 (m, 2H), 4.27 - 4.18 (m, 1H), 4.04 - 3.98 (m, 2H), 3.90 - 3.83 (m, 1H), 3.47 - 3.43 (m, 2H), 2.66 - 2.60 (m, 1H), 1.70(s, 3H), 0.72 - 0.69 (m, 4H) ppm |
| 313 | 497.3 | 1H NMR (400 MHz, DMSO-d6) δ = 9.36 - 9.33 (m, 1H), 9.15 - 9.13 (m, 1H), 8.56 (d, J = 9.2 Hz, 1H), 8.38 (d, J = 9.2 Hz, 1H), 8.19 (d, J = 1.2 Hz, 1H), 7.95 - 7.92 (m, 1H), 7.49 (s, 1H), 7.31 (d, J = 8.0 Hz, 1H), 4.93 - 4.86 (m, 2H), 4.73 (br d, J = 4.4 Hz, 2H), 4.47 - 4.42 (m, 1H), 4.23 (d, J = 11.6 Hz, 1H), 4.09 - 4.05 (m, 2H), 3.87 (d, J = 11.6 Hz, 1H), 3.61 - 3.57 (m, 2H), 2.28 - 2.23 (m, 2H), 2.04 - 1.99 (m, 2H), 1.70 - 1.64 (m, 5H) ppm |
| 315 | 497.2 | 1H NMR (400 MHz, CD3OD) δ = 9.13 (s, 1H), 8.37 - 8.26 (m, 1H), 8.18 (s, 1H), 8.14 - 8.07 (m, 1H), 7.96 - 7.87 (m, 1H), 7.72 (s, 1H), 7.36 - 7.18 (m, 1H), 4.95 - 4.92 (m, 2H), 4.86 - 4.85 (m, 2H), 4.25 - 4.18 (m, 1H), 4.13- 4.05 (m, 2H), 3.97 - 3.90 (m, 1H), 3.49 - 3.44 (m, 2H), 2.81 - 2.68 (m, 1H), 2.14 - 2.05 (m, 2H), 1.79 - 1.74 (m, 3H), 0.88 - 0.81 (m, 2H), 0.79 - 0.70 (m, 2H) ppm |
| 350 | 511.1 | 1H NMR (400 MHz, MeOD-d4) δ = 9.13 (s, 1H), 8.28 (d, J = 10.2 Hz, 1H), 8.17 (s, 1H), 8.03 (d, J = 10.2 Hz, 1H), 7.92 (d, J = 8.0 Hz, 1H), 7.71 (s, 1H), 7.30 (d, J = 7.6 Hz, 1H), 4.97-4.81 (m, 4H), 4.81-4.80 (m, 1H), 4.22 (d, J = 11.2 Hz, 1H), 4.11-4.08 (m, 2H), 3.94 (d, J = 11.2 Hz, 1H), 3.52-3.49 (m, 2H), 2.29-2.17 (m, 2H), 2.14-2.11 (m, 4H), 1.77-1.73 (m, 5H) ppm |
| 471 | 442.3 | 1H NMR (400 MHz, DMSO-d6) δ=9.39 - 9.33 (m, 1H), 9.23 (s, 1H), 8.62 (d, J = 8.8 Hz, 1H), 8.51 (d, J = 9.2 Hz, 1H), 8.18 (s, 1H), 7.94 - 7.91 (m, 1H), 7.58 (s, 1H), 7.30 (d, J = 8.0 Hz, 1H), 4.92 - 4.75 (m, 4H), 4.22 (d, J = 11.6 Hz, 1H), 4.12 - 4.08 (m, 2H), 3.86 (d, J = 11.2 Hz, 1H), 2.67 - 2.63 (m, 2H), 2.08 - 2.04 (m, 2H), 1.69 (s, 3H) ppm |

### Example 30: (R)-4-Cyano-4-methyl-N-((2-(6-methyl-5-oxo-3,4,5,6-tetrahydro-2,6-naphthyridin-2(1H)-yl)-1,6-naphthyridin-7-yl)methyl)isochromane-6-carboxamide

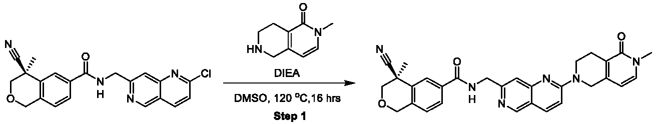

### Step 1: (R)-4-Cyano-4-methyl-N-((2-(6-methyl-5-oxo-3,4,5,6-tetrahydro-2,6-naphthyridin-2(1H)-yl)-1,6-naphthyridin-7-yl)methyl)isochromane-6-carboxamide

To a mixture of 2-methyl-5,6,7,8-tetrahydro-2,6-naphthyridin-1(2H)-one (30 mg, 107.83 umol, TFA salt) in DMSO (1 mL) was added (R)-N-((2-chloro-1,6-naphthyridin-7-yl)methyl)-4-cyano-4-methylisochromane-6-carboxamide (28.24 mg, 71.88 umol) and DIEA (46.45 mg, 359.42 umol, 62.60 uL). The mixture was stirred at 120 °C for 16 hrs. The mixture was poured into H₂O (15 mL) and extracted with EA / MeOH = 10 / 1 (15 mL *3). The combined organic layers were washed with brine (10 mL), dried over anhydrous Na₂SO₄, filtered and concentrated under reduced pressure. The residue was purified by prep-TLC (DCM / MeOH = 10 / 1, Rf=0.3) to afford a yellow solid. The yellow solid was further purified by reverse phase column (0.1 % FA condition). The product fraction was concentrated under reduced pressure to remove MeCN and lyophilized to give the title compound (8.23 mg, 15.81 umol, 22.0% yield) as a white solid.
LCMS (ESI) m/z: [M+H]⁺ = 521.3.
¹H NMR (400 MHz, DMSO-d6) δ = 9.31 - 9.28 (m, 1H), 8.91 (s, 1H), 8.20 - 8.17 (m, 2H), 7.95 - 7.92 (m, 1H), 7.54 (d, *J* = 7.2 Hz, 1H), 7.38 (d, *J* = 9.2 Hz, 1H), 7.33 - 7.27 (m, 2H), 6.17 (d, *J* = 6.8 Hz, 1H), 4.94 - 4.80 (m, 2H), 4.71 (s, 2H), 4.69 - 4.61 (m, 2H), 4.23 (d, *J* = 11.6 Hz, 1H), 3.98 - 3.94 (m, 2H), 3.87 (d, *J* = 11.6 Hz, 1H), 3.40 (s, 3H), 2.54 (br s, 2H), 1.70 (s, 3H) ppm.
Chiral SFC: Cellucoat-MeOH+ACN(DEA)-40-3mL-35T, Rt = 1.153 min, ee% = 100%.

The following examples in Table 21 were prepared using standard chemical manipulations and procedures similar to those used for the preparation of Examples 30.

**Table 21. Compounds of the Invention**

| # | LCMS (m/z) | 1H NMR |
|---|---|---|
| 382 | 530.3 | 1H NMR (400 MHz, DMSO-d6) δ = 9.32 - 9.29 (m, 1H), 8.98 (s, 1H), 8.36 - 8.35 (m, 1H), 8.28 (d, J = 9.6 Hz, 1H), 8.18 (d, J =1.2 Hz, 1H), 7.94 - 7.92 (m, 1H), 7.69 (s, 1H), 7.47 (d, J = 9.2 Hz, 1H), 7.36 (s, 1H), 7.30 (d, J = 8.0 Hz, 1H), 7.25 - 6.97 (m, 1H), 5.10 (s, 2H), 4.92 - 4.81 (m, 2H), 4.69 (br d, J = 5.2 Hz, 2H), 4.29 - 4.21 (m, 5H), 3.87 (d, J = 11.6 Hz, 1H), 1.69 (s, 3H) ppm |
| 383 | 492.2 | 1H NMR (400 MHz, DMSO-d6) δ= 9.31 (s, 1H), 8.64 - 8.57 (m, 2H), 8.49 (br s, 1H), 8.40 (d, J = 7.6 Hz, 1H), 8.18 (d, J = 1.6 Hz, 1H), 7.97 - 7.86 (m, 4H), 7.29 (d, J = 8.0 Hz, 1H), 4.92 (br d, J = 5.6 Hz, 5H), 4.21 (d, J = 11.2 Hz, 1H), 3.93 (d, J = 11.6 Hz, 1H), 1.76 (s, 3H), 1.54 - 1.51 (m, 2H), 1.27 - 1.24 (m, 2H) ppm |
| 425 | 498.5 | 1H NMR (400 MHz, MeOD) δ = 9.15 (s, 1H), 8.52 (d, J = 4.4 Hz, 1H), 8.48 (d, J = 8.8 Hz, 1H), 8.16 (d, J = 1.6 Hz, 1H), 8.08 (d, J = 8.8 Hz, 1H), 7.93-7.90 (m, 1H), 7.72 (s, 1H), 7.29 (d, J = 8.0 Hz, 1H), 4.91 (d, J = 3.2 Hz, 2H), 4.86 (br s, 2H), 4.20 (d, J = 11.2 Hz, 1H), 4.11 - 4.07 (m, 4H), 3.93 (d, J = 11.6 Hz, 1H), 2.50 - 2.43 (m, 2H), 1.76 (s, 3H) ppm |
| 426 | 480.5 | 1H NMR (400 MHz, MeOD) δ = 9.16 (s, 1H), 8.57 (d, J = 2.8 Hz, 1H), 8.50 (d, J = 9.2 Hz, 1H), 8.16 (d, J = 1.6 Hz, 1H), 8.09 (d, J = 8.8 Hz, 1H), 7.93-7.90 (m, 1H), 7.73 (s, 1H), 7.29 (d, J = 8.0 Hz, 1H), 5.99 (d, J = 3.2 Hz, 1H), 4.91 (d, J = 3.6 Hz, 2H), 4.87 (br s, 2H), 4.20 (d, J = 11.6 Hz, 1H), 4.03 - 3.99 (m, 4H), 3.93 (d, J = 11.2 Hz, 1H), 2.47 - 2.39 (m, 2H), 1.76 (s, 3H) ppm |
| 453 | 494.3 | 1HNMR (400 MHz, METHANOL-d4) δ = 8.83 (d, J = 2.8 Hz, 1H), 8.19 - 8.08 (m, 2H), 7.93 - 7.90 (m, 1H), 7.46 (s, 1H), 7.36 - 7.25 (m, 3H), 4.91 (d, J = 3.6 Hz, 2H), 4.82 - 4.74 (m, 4H), 4.23 - 4.13 (m, 3H), 3.93 (d, J = 11.6 Hz, 1H), 3.73 (d, J = 1.6 Hz, 3H), 2.86 (s, 2H), 1.76 (s, 3H) ppm |
| 455 | 494.3 | 1HNMR (400 MHz, METHANOL-d4) δ = 8.83 (s, 1H), 8.18 - 8.07 (m, 2H), 7.91 (d, J = 8.0 Hz, 1H), 7.46 (s, 1H), 7.34 - 7.28 (m, 2H), 4.91 (d, J = 3.6 Hz, 2H), 4.81 - 4.77 (m, 4H), 4.24 - 4.12 (m, 3H), 3.93 (d, J = 11.6 Hz, 1H), 3.83 - 3.72 (m, 3H), 2.86 - 2.81 (m, 2H), 1.76 (s, 3H) ppm |
| 457 | 494.5 | 1H NMR (400 MHz, MeOD) δ = 8.85 (s, 1H), 8.35-8.33 (m, 1H), 8.16-8.13 (m, 2H), 7.91-7.89 (m, 1H), 7.49 (s, 1H), 7.38-7.35(m, 1H), 7.28-7.26 (m, 2H), 4.96 (s, 2H), 4.90 (d, J = 3.2 Hz, 2H), 4.78 (s, 2H), 4.21 (d, J = 11.2 Hz, 1H), 4.03-4.01(m, 2H), 3.93 (d, J = 11.2 Hz, 1H), 3.79-3.78 (m, 3H), 2.71 (br s, 2H), 1.76 (s, 3H) ppm. |
| 458 | 494.5 | 1H NMR (400 MHz, MeOD) δ = 8.82-8.81 (m, 1H), 8.27 (s, 1H), 8.15 (s, 1H), 8.13-8.08 (m, 1H), 7.91-7.89 (m, 1H), 7.45 (s, 1H), 7.33 (s, 1H), 7.30-7.26 (m, 2H), 4.89 (s, 2H), 4.77 (s, 2H), 4.21-4.17 (m, 1H), 4.05 (s, 2H), 3.93-3.89 (m, 1H), 3.82 (s, 3H), 2.72 (s, 2H), 1.75 (d, J = 1.2 Hz, 3H) ppm. |
| 461 | 480.2 | 1H NMR (400 MHz, MeOD-d4) δ =8.85 (s, 1H), 8.29 (s, 1H), 8.19 - 8.12 (m, 2H), 7.93 - 7.91 (m, 1H), 7.51 (s, 1H), 7.34 - 7.26 (m, 2H), 7.01 (d, J = 9.2 Hz, 1H), 4.91 (d, J = 3.2 Hz, 2H), 4.83 - 4.75 (m, 4H), 4.74 - 4.64 (m, 2H), 4.21 (d, J = 11.6 Hz, 1H), 3.93 (d, J = 11.6 Hz, 1H), 3.85 (s, 3H), 1.77 (s, 3H) ppm. |
| 473 | 425.3 | 1HNMR (400 MHz, DMSO-d6) δ = 9.44 - 9.41 (m, 1H), 9.38 (s, 1H), 8.86 (d, J = 2.4 Hz, 1H), 8.75 (d, J = 8.8 Hz, 1H), 8.28 (d, J = 8.8 Hz, 1H), 8.21 (s, 1H), 8.15 - 8.13 (m, 1H), 7.98 - 7.93 (m, 2H), 7.73 (s, 1H), 7.32 (d, J = 8.0 Hz, 1H), 6.68 - 6.67(m, 1H), 4.95 - 4.84 (m, 2H), 4.83 - 4.79 (m, 2H), 4.24 (d, J = 11.6 Hz, 1H), 3.88 (d, J = 11.6 Hz, 1H), 1.71 (s, 3H) ppm |
| 486 | 480.2 | 1H NMR (400 MHz, DMSO-d6) δ = 9.32 - 9.30 (m, 1H), 8.95 (s, 1H), 8.28 - 8.15 (m, 2H), 7.94 - 7.91 (m, 1H), 7.48 - 7.39 (m, 2H), 7.36 - 7.25 (m, 2H), 6.17 (d, J = 1.6 Hz, 1H), 5.01 (s, 2H), 4.94 - 4.80 (m, 2H), 4.67 (d, J = 3.2, 2H), 4.30 - 4.18 (m, 5H), 3.86 (d, J = 11.6 Hz, 1H), 1.69 (s, 3H) ppm. |
| 502 | 540.3 | 1H NMR (400 MHz, Chloroform-d) δ 8.71 (s, 1H), 8.13 (s, 1H), 7.92 (d, J = 8.6 Hz, 1H), 7.62 (s, 1H), 7.11 (d, J = 8.1 Hz, 1H), 6.93 (s, 1H), 5.35 (s, 1H), 4.90 (d, J = 5.7 Hz, 2H), 4.85 (d, J = 4.6 Hz, 2H), 4.21 (q, J = 7.2 Hz, 2H), 4.13 (d, J = 11.3 Hz, 1H), 3.87 (d, J = 11.3 Hz, 1H), 3.68 (s, 2H), 2.15 - 1.15 (m, 14H) |
| 503 | 439 | 1H NMR (400 MHz, MeOD) δ = 9.23 (s, 1H), 8.69 (d, J = 2.4 Hz, 1H), 8.59 (d, J = 8.8 Hz, 1H), 8.24 (d, J = 8.8 Hz, 1H), 8.17 (d, J = 1.6 Hz, 1H), 7.95 - 7.90 (m, 1H), 7.80 (s, 1H), 7.29 (d, J = 8.2 Hz, 1H), 6.42 (d, J = 2.8 Hz, 1H), 4.91 (br d, J = 3.8 Hz, 4H), 4.20 (d, J = 11.6 Hz, 1H), 3.93 (d, J = 11.2 Hz, 1H), 2.37 (s, 3H), 1.76 (s, 3H) ppm |
| 505 | 439.2 | 1HNMR (400 MHz, DMSO-d6) δ= 9.43 - 9.31 (m, 2H), 8.69 - 8.61 (m, 2H), 8.21- 8.10(m, 2H), 7.96- 7.94 (m, 1H), 7.78 - 7.76 (m, 1H), 7.66 (s, 1H), 7.31 (d, J = 8.0 Hz, 1H), 4.93 - 4.78 (m, 4H), 4.24 - 4.19 (m, 1H), 3.87 ( d, J = 11.6 Hz, 1H), 2.11 (s, 3H), 1.70 (s, 3H) ppm. |
| 514 | 428.3 | 1H NMR (400 MHz, DMSO-d6) δ = 9.30 - 9.27 (m, 1H), 8.85 (s, 1H), 8.19 (s, 1H), 8.09 (d, J = 9.2 Hz, 1H), 7.95 - 7.93 (m, 1H), 7.30 (d, J = 8.0 Hz, 1H), 7.24 (s, 1H), 6.92 (d, J = 9.2 Hz, 1H), 4.95 - 4.79 (m, 2H), 4.66 - 4.64 (m, 2H), 4.23 (d, J = 11.6 Hz, 1H), 3.86 (d, J = 11.6 Hz, 1H), 3.11 - 3.09 (m, 1H), 2.56 - 2.52 (m, 2H), 1.96 (br s, 4H), 1.90 - 1.76 (m, 1H), 1.70 (s, 3H) ppm |

### Example 31: (R)-N-((2-(6-(Azetidin-1-yl)pyridin-2-yl)-2H-pyrazolo[4,3-c]pyridin-6-yl)methyl)-4-cyano-4-methylisochromane-6-carboxamide

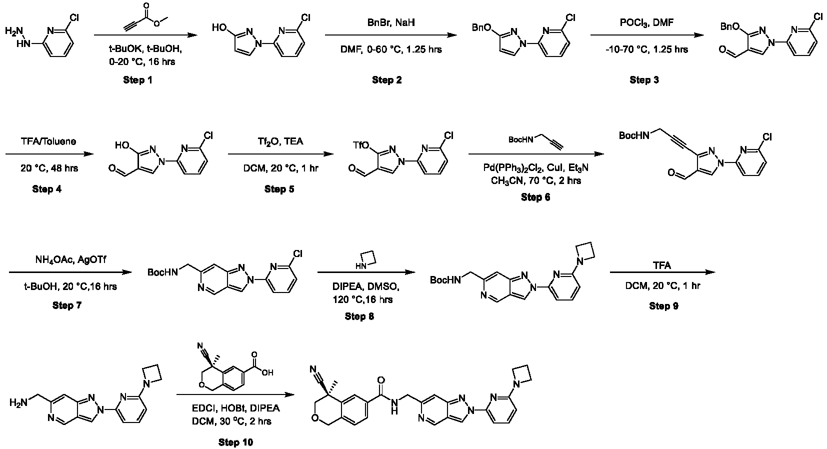

### Step 1: 1-(6-Chloropyridin-2-yl)-1H-pyrazol-3-ol

Methyl prop-2-ynoate (1.29 g, 15.32 mmol, 1.28 mL) was added dropwise to a solution of (6-chloro-2-pyridyl)hydrazine (2 g, 13.93 mmol) in t-BuOH (10 mL) at 20 °C. t-BuOK (3.13 g, 27.86 mmol) was then added at 0 °C before stirring at 20 °C for 16 hrs. The reaction was quenched by adding water (100 mL) and the mixture was adjusted to pH 9 with 1N HCl solution, forming a yellow precipitate. The solid was collected by filtration, washed with water (20 mL) and dried to give the title compound (1.6 g, 8.18 mmol, 58.72% yield).
LCMS (ESI) m/z: [M+H]⁺ = 196.1.
¹H NMR (400 MHz, CDCl₃) δ = 8.38 - 8.36 (m, 1H), 7.83 - 7.71 (m, 1H), 7.57 - 7.48 (m, 1H), 7.21 - 7.11 (m, 1H), 6.06 - 5.89 (m, 1H), 4.71 (br s, 1H) ppm.

### Step 2: 2-(3-(Benzyloxy)-1H-pyrazol-1-yl)-6-chloropyridine

NaH (173.21 mg, 4.33 mmol, 60% purity) was added portion wise to a solution of 1-(6-chloropyridin-2-yl)-1H-pyrazol-3-ol (0.77 g, 3.94 mmol) in DMF (10 mL) at 0 °C. The mixture was stirred at 0 °C for 15 mins. (Bromomethyl)benzene (673.27 mg, 3.94 mmol, 467.55 uL) was added dropwise at 0 °C and the mixture was stirred at 60 °C for 1 hr. The reaction was quenched by pouring into water (50 mL), forming a yellow precipitate was formed. The solid was collected by filtration, washed with water (20 mL) and dried. The residue was purified by column chromatography (SiO₂, Petroleum ether/Ethyl acetate=3/1) to give the title compound (0.5 g, 1.75 mmol, 44.45% yield).
LCMS (ESI) m/z: [M+H]⁺ = 286.1.
¹H NMR (400 MHz, CDCl₃) δ = 8.38 - 8.36 (m, 1H), 7.76 - 7.67 (m, 2H), 7.55 - 7.46 (m, 2H), 7.44 - 7.34 (m, 4H), 7.14 - 7.05 (m, 1H), 6.00 - 5.97 (m, 1H), 5.32 (s, 2H) ppm.

### Step 3: 3-(Benzyloxy)-1-(6-chloropyridin-2-yl)-1H-pyrazole-4-carbaldehyde

POCl₃ (429.30 mg, 2.80 mmol, 260.18 uL) was added dropwise to a solution of DMF (204.64 mg, 2.80 mmol, 215.41 uL) at -10 °C and the mixture was stirred at 0 °C for 15 mins. 2-(3-(Benzyloxy)-1H-pyrazol-1-yl)-6-chloropyridine (0.2 g, 699.97 umol) was added portion wise at 0 °C. The mixture was then stirred at 70 °C for 1 hr. The reaction was quenched by adding sat. NaHCO₃ (20 mL) and a yellow precipitate was formed. The solid was collected by filtration, washed with water (10 mL) and dried to give the title compound (0.21 g, 669.35 umol, 95.63% yield).
LCMS (ESI) m/z: [M+H]⁺ = 314.1.
¹H NMR (400 MHz, CDCl₃) δ = 9.92 (s, 1H), 8.91 (s, 1H), 7.84 - 7.74 (m, 2H), 7.57 - 7.49 (m, 2H), 7.45 - 7.36 (m, 3H), 7.26 - 7.24 (m, 1H), 5.46 (s, 2H) ppm.

### Step 4: 1-(6-Chloropyridin-2-yl)-3-hydroxy-1H-pyrazole-4-carbaldehyde

TFA (3.39 g, 29.71 mmol, 2.20 mL) was added dropwise to a solution of 3-(benzyloxy)-1-(6-chloropyridin-2-yl)-1H-pyrazole-4-carbaldehyde (110 mg, 350.61 umol) in toluene (2.2 mL). The mixture was stirred at 20 °C for 48 hrs. The reaction mixture was concentrated under vacuum. The residue was triturated with MTBE (5 mL) to give the title compound (50 mg, crude) as yellow solid.
LCMS (ESI) m/z: [M+H]⁺ = 224.0.

### Step 5: 1-(6-Chloropyridin-2-yl)-4-formyl-1H-pyrazol-3-yl trifluoromethanesulfonate

To a solution of 1-(6-chloropyridin-2-yl)-3-hydroxy-1*H*-pyrazole-4-carbaldehyde (40 mg, 178.88 umol) in DCM (3 mL) was added TEA (54.30 mg, 536.64 umol) and dropwise Tf₂O (75.70 mg, 268.32 umol). The mixture was stirred at 20 °C for 1 hr. The reaction was quenched by adding water (50 mL) and the products were extracted with ethyl acetate (30 mL * 3). The combined organic layers were washed with brine (30 mL), dried over Na₂SO₄, filtered and concentrated. The residue was purified by column chromatography (SiO₂, Petroleum ether/Ethyl acetate=1/1) to give the title compound (30 mg, crude) as a yellow oil.

### Step 6: tert-Butyl (3-(1-(6-chloropyridin-2-yl)-4-formyl-1H-pyrazol-3-yl)prop-2-yn-1-yl)carbamate

A mixture of *tert*-butyl prop-2-yn-1-ylcarbamate (13.74 mg, 88.56 umol), 1-(6-chloropyridin-2-yl)-4-formyl-1*H*-pyrazol-3-yl trifluoromethanesulfonate (30 mg, 84.35 umol), Et₃N (85.35 mg, 843.46 umol), Cul (1.61 mg, 8.43 umol) and Pd(PPh₃)₂Cl₂ (5.92 mg, 8.43 umol) in CH₃CN (2 mL) was de-gassed and purged with N₂. The mixture was stirred at 70 °C for 2 hours under N₂. The reaction mixture was quenched by adding (water 30 mL) and the products were extracted with ethyl acetate (30 mL * 3). The combined organic phase was washed with brine (30mL), dried over Na₂SO₄, filtered and concentrated under reduced pressure. The residue was purified by prep-TLC (SiO2, Petroleum ether/Ethyl acetate=1/1) to give the title compound (40 mg, crude) as a yellow oil.
LCMS (ESI) m/z: [M+Na]⁺ = 383.1.

### Step 7: tert-Butyl ((2-(6-chloropyridin-2-yl)-2H-pyrazolo[4,3-c]pyridin-6-yl)methyl)carbamate

To a solution of *tert*-butyl (3-(1-(6-chloropyridin-2-yl)-4-formyl-1*H*-pyrazol-3-yl)prop-2-yn-1-yl)carbamate (40 mg, 110.87 umol) in t-BuOH (2 mL) was added NH₄OAc (12.82 mg, 166.30 umol) and AgOTf (5.70 mg, 22.17 umol) under N₂. The mixture was stirred at 20 °C for 16 hrs. The reaction was quenched by addition of NaHCO₃ (0.2 g), and the mixture was stirred at 20 °C for 30 mins. The resulting mixture was filtered and the solid was washed with ethyl acetate (30 mL * 3). The combined organic phase was dried over Na₂SO₄, filtered and concentrated under reduced pressure. The residue was purified by column chromatography (SiO₂, Petroleum ether/Ethyl acetate=0/1) to give the title compound (35 mg, crude) as a yellow solid.
LCMS (ESI) m/z: [M+H]⁺ = 360.1.
¹H NMR (400 MHz, CDCl₃) δ = 9.27 (s, 1H), 9.24 - 9.14 (m, 1H), 8.28 - 8.18 (m, 1H), 7.97 - 7.84 (m, 1H), 7.53 (s, 1H), 7.45 - 7.36 (m, 1H), 5.48 - 5.29 (m, 1H), 4.58 - 4.48 (m, 2H), 1.48 (s, 9H) ppm.

### Step 8: tert-Butyl ((2-(6-(azetidin-1-yl)pyridin-2-yl)-2H-pyrazolo[4,3-c]pyridin-6-yl)methyl)carbamate

Azetidine (15.23 mg, 266.81 umol) and DIEA (114.94 mg, 889.36umol) were added dropwise to a solution of tert-butyl ((2-(6-chloropyridin-2-yl)-2H-pyrazolo[4,3-c]pyridin-6-yl)methyl)carbamate (32 mg, 88.94 umol) in DMSO (2 mL). The mixture was stirred at 120 °C for 16 hrs. The reaction mixture was cooled to 20 °C and poured into water (20 mL). The products were extracted with ethyl acetate (20 mL * 3). The combined organic phases were washed with water (20 mL), dried over Na₂SO₄, filtered and concentrated to give the title compound (22 mg, crude) as a yellow oil.
LCMS (ESI) m/z: [M+H]⁺ = 381.2.

### Step 9: (2-(6-(Azetidin-1-yl)pyridin-2-yl)-2H-pyrazolo[4,3-c]pyridin-6-yl)methanamine

TFA (410.67 mg, 3.60 mmol, 266.67 uL) was added to a solution of *tert*-butyl ((2-(6-(azetidin-1-yl)pyridin-2-yl)-2*H*-pyrazolo[4,3-c]pyridin-6-yl)methyl)carbamate (20 mg, 52.57 umol) in DCM (2 mL). The mixture was stirred at 20 °C for 1 hr. The solvent was evaporated by a flow of N₂ gas to give the title compound (12 mg, crude) as a yellow oil, which was used in the next step without further purification. LCMS (ESI) m/z: [M+H]⁺ = 281.2.

### Step 10: (R)-N-((2-(6-(Azetidin-1-yl)pyridin-2-yl)-2H-pyrazolo[4,3-c]pyridin-6-yl)methyl)-4-cyano-4-methylisochromane-6-carboxamide

To a solution of (2-(6-(azetidin-1-yl)pyridin-2-yl)-2*H*-pyrazolo[4,3-c]pyridin-6-yl)methanamine (12 mg, 42.81 umol) in DCM (2 mL) was added (*R*)-4-cyano-4-methylisochromane-6-carboxylic acid (11.16 mg, 51.37 umol), DIEA (27.66 mg, 214.04 umol), HOBt (6.94 mg, 51.37 umol) and EDCI (9.85 mg, 51.37 umol). Water (30 mL) was added and the products were extracted with ethyl acetate (30 mL * 3). The combined organic phase was washed with brine (10 ml * 3), dried over Na₂SO₄, filtered and concentrated under reduced pressure. The residue was purified by prep-HPLC (0.1% NH₃.H₂O condition) to give the title compound (4.44 mg, 9.26 umol, 21.63% yield) as a yellow solid.
LCMS (ESI) m/z: [M+H]⁺ = 480.2.
¹H NMR (400 MHz, DMSO-d₆) δ = 9.38 (s, 1H), 9.30 - 9.22 (m, 2H), 8.21 - 8.15 (m, 1H), 7.95 - 7.90 (m, 1H), 7.80 - 7.73 (m, 1H), 7.46 (s, 1H), 7.43 - 7.37 (m, 1H), 7.32 - 7.26 (m, 1H), 6.51 - 6.43 (m, 1H), 4.93 - 4.81 (m, 2H), 4.72 - 4.65 (m, 2H), 4.27 - 4.21 (m, 1H), 4.11 - 4.07 (m, 4H), 3.90 - 3.84 (m, 1H), 2.42 - 2.38 (m, 2H), 1.70 (s, 3H) ppm.

The following examples in Table 22 were prepared using standard chemical manipulations and procedures similar to those used for the preparation of **Example 31.**

**Table 22. Compounds of the Invention**

| # | LCMS (m/z) | 1H NMR |
|---|---|---|
| 524 | 424.1 | 1HNMR (400 MHz, DMSO-d6) δ = 9.39 (s, 1H), 9.29 - 9.22 (m, 2H), 8.18 (d, J = 1.6 Hz, 1H), 8.10 (d, J = 7.6 Hz, 2H), 7.95 - 7.91 (m, 1H), 7.65 - 7.59 (m, 2H), 7.53 - 7.47 (m, 2H), 7.29 (d, J = 8.0 Hz, 1H), 4.95 - 4.80 (m, 2H), 4.69 (d, J = 5.6 Hz, 2H), 4.22 (d, J = 11.4 Hz, 1H), 3.86 (d, J = 11.6 Hz, 1H), 1.70 (s, 3H) ppm |

### Example 32: (R)-4-Cyano-N-((6-(2-methoxyphenyl)-5,6,7,8-tetrahydro-2,6-naphthyridin-3-yl)methyl)-4-methylisochromane-6-carboxamide

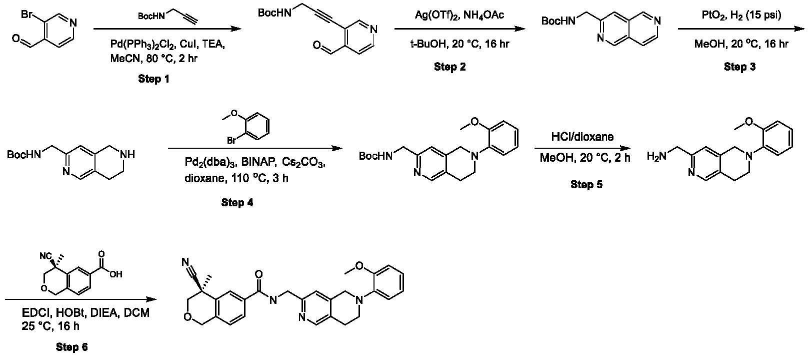

### Step 1: tert-Butyl (3-(4-formylpyridin-3-yl)prop-2-yn-1-yl)carbamate

A mixture of 3-bromopyridine-4-carbaldehyde (15 g, 80.64 mmol), tert-butyl prop-2-yn-1-ylcarbamate (13.14 g, 84.67 mmol), Et₃N (81.60 g, 806.43 mmol, 112.25 mL), Cul (1.54 g, 8.06 mmol) and Pd(PPh₃)₂Cl₂ (5.66 g, 8.06 mmol) in CH₃CN (120 mL) was stirred at 80 °C for 2 hrs under N₂. The reaction mixture was quenched by addition of water (300 mL) and extracted with ethyl acetate (300 mL * 3). The combined organic phase was washed with brine (300 mL), dried over Na₂SO₄, filtered and concentrated under reduced pressure. The residue was purified by column chromatography (SiO₂, Petroleum ether/Ethyl acetate=1/1) to give the title compound (18 g, 69.15 mmol, 85.75% yield) as a brown oil.
LCMS (ESI) m/z: [M+H]⁺ = 261.1.
¹HNMR (400 MHz, CDCl₃) δ = 10.48 (s, 1H), 8.66 (s, 1H), 8.78 - 8.68 (m, 1H), 7.78 - 7.62 (m, 1H), 4.88 (br s, 1H), 4.32 - 4.18 (m, 2H), 1.46 (s, 9H) ppm.

### Step 2: tert-Butyl ((2,6-naphthyridin-3-yl)methyl)carbamate

To a solution of tert-butyl (3-(4-formylpyridin-3-yl)prop-2-yn-1-yl)carbamate (17 g, 65.31 mmol) in t-BuOH (60 mL) was added NH₄OAc (7.55 g, 97.97 mmol) and AgOTf (3.36 g, 13.06 mmol). The mixture was stirred at 20 °C for 16 hrs. The reaction mixture was quenched by addition of NaHCO₃ (22g), and the reaction mixture was stirred at 20 °C for 30 mins. The mixture was filtered and the filter cake was washed with ethyl acetate (30 mL * 3). The combined organic phases were concentrated under reduced pressure and the residue was purified by column chromatography (SiO₂, Petroleum ether/Ethyl acetate=0/1) to give the title compound (8.5 g, 32.39 mmol, 49.59% yield) as a yellow oil.
LCMS (ESI) m/z: [M+H]⁺ = 260.1.
¹HNMR (400 MHz, CDCl₃) δ = 9.36 - 9.25 (m, 2H), 8.75 - 8.61 (m, 1H), 7.82 - 7.71 (m, 1H), 5.46 (br s, 1H), 4.64 - 4.62 (m, 2H), 1.46 (s, 9H) ppm.

### Step 3: tert-Butyl ((5,6,7,8-tetrahydro-2,6-naphthyridin-3-yl)methyl)carbamate

A mixture of tert-butyl ((2,6-naphthyridin-3-yl)methyl)carbamate (8 g, 30.85 mmol) and PtO₂ (700.59 mg, 3.09 mmol) in MeOH (80 mL) was degassed and purged with H₂ 3 times. The mixture was stirred at 20 °C for 16 hrs under H₂ atmosphere (15 psi). The reaction was filtered and the filter cake was washed with MeOH (50 mL * 2). The filtrate was concentrated under vacuum and the residue was purified by column chromatography (SiO₂, DCM:MeOH=10/1) to give crude product (5 g) as a black oil. The crude product was purified by reversed-phase HPLC (0.1% FA condition). The desired fraction was adjusted to pH 9 with sat. NaHCO₃ and extracted with DCM (200 mL * 3). The combined organic phases were dried over Na₂SO₄, filtered and concentrated to give the title compound (3.8 g, 14.43 mmol, 46.77% yield) as a yellow oil.
LCMS (ESI) m/z: [M+H]⁺ = 264.2.
¹HNMR (400 MHz, CDCl₃) δ = 8.26 (s, 1H), 6.92 (s, 1H), 5.46 (br s, 1H), 4.37 - 4.35 (m, 2H), 3.98 (s, 2H), 3.17 - 3.13 (m, 2H), 2.79 - 2.75 (m, 2H), 1.46 (s, 9H) ppm.

### Step 4: tert-Butyl ((6-(2-methoxyphenyl)-5,6,7,8-tetrahydro-2,6-naphthyridin-3-yl)methyl)carbamate

A mixture of *tert*-butyl ((5,6,7,8-tetrahydro-2,6-naphthyridin-3-yl)methyl)carbamate (100 mg, 379.75 umol), 1-bromo-2-methoxy-benzene (213.08 mg, 1.14 mmol, 142.05 uL), BINAP (47.29 mg, 75.95 umol), Pd₂(dba)₃ (34.77 mg, 37.97 umol) and Cs₂CO₃ (371.18 mg, 1.14 mmol) in dioxane (3 mL) was degassed and purged with N₂ 3 times. The mixture was stirred at 110 °C for 3 hrs under N₂ atmosphere. The mixture was poured into water (40 mL) and extracted with EA (20 mL*3). The combined organic layers were washed with brine (20 mL), dried over Na₂SO₄, filtered and concentrated under vacuum. The residue was purified by reversed-phase HPLC (0.1% FA condition) to give the title compound (80 mg, 155.90 umol, 41.06% yield) as a yellow solid.
LCMS (ESI) m/z: [M+H]⁺ = 370.1

### Step 5: (6-(2-methoxyphenyl)-5,6,7,8-tetrahydro-2,6-naphthyridin-3-yl)methanamine hydrochloride

To a solution of *tert*-butyl ((6-(2-methoxyphenyl)-5,6,7,8-tetrahydro-2,6-naphthyridin-3-yl)methyl)carbamate (35 mg, 94.73umol) in MeOH (0.5 mL) was added HCl/dioxane (4 M, 0.5 mL). The mixture was stirred at 20 °C for 2 hrs. The solvent was removed under vacuum to give the title compound (30 mg, crude) as a yellow oil.
LCMS (ESI) m/z: [M+H]⁺ = 270.2.

### Step 6: (R)-4-Cyano-N-((6-(2-methoxyphenyl)-5,6,7,8-tetrahydro-2,6-naphthyridin-3-yl)methyl)-4-methylisochromane-6-carboxamide

To a solution of (6-(2-methoxyphenyl)-5,6,7,8-tetrahydro-2,6-naphthyridin-3-yl)methanamine hydrochloride (30 mg, 98.10 umol) and ((*R*)-4-cyano-4-methylisochromane-6-carboxylic acid (27.70 mg, 127.53 umol) in DCM (1 mL) was added EDCI (28.21 mg, 147.15 umol), DIEA (63.40 mg, 490.51 umol, 85.44 uL) and HOBt (19.88 mg, 147.15 umol). The mixture was stirred at 25 °C for 16 hrs. The mixture was poured into water (30 mL) and extracted with EA (20 mL*3). The combined organic layers were washed with brine (20 mL), dried over Na₂SO₄, filtered and concentrated. The residue was purified by prep-HPLC (column: Phenomenex Gemini-NX C18 75*30mm*3um; mobile phase: [water (10mM NH₄HCO₃)-ACN];B%: 26%-56%,8min) to give the title compound (15.64 mg, 31.70 umol, 32.31% yield) as an off-white solid.
LCMS (ESI) m/z: [M+H]⁺ = 469.3
¹H NMR (400 MHz, METHANOL-d₄) δ = 8.31 (s, 1H), 8.11 (d, *J* = 1.6 Hz, 1H), 7.90 - 7.81 (m, 1H), 7.29 - 7.20 (m, 2H), 7.08 - 6.96 (m, 3H), 6.94 - 6.86 (m, 1H), 4.89 (d, *J* = 3.2 Hz, 2H), 4.67 (s, 2H), 4.23 (s, 2H), 4.19 (d, *J* = 11.6 Hz, 1H), 3.91 (d, *J* = 11.6 Hz, 1H), 3.87 (s, 3H), 3.42 - 3.35 (m, 2H), 3.03 - 2.94 (m, 2H), 1.74 (s, 3H) ppm.

The following examples in Table 23 were prepared using standard chemical manipulations and procedures similar to those used for the preparation of **Example 32.**

**Table 23. Compounds of the Invention**

| # | LCMS (m/z) | 1H NMR |
|---|---|---|
| 209 | 495.4 | 1H NMR (400 MHz, DMSO-d6) δ = 9.25 - 9.13 (m, 1H), 8.39 - 8.31 (m, 1H), 8.17 - 8.08 (m, 2H), 7.79 (s, 2H), 7.36 - 7.21 (m, 2H), 5.55 - 5.42 (m, 1H), 4.94 - 4.81 (m, 2H), 4.61 - 4.47 (m, 2H), 4.38 - 4.27 (m, 1H), 4.26 - 4.17 (m, 1H), 3.95 - 3.77 (m, 2H), 2.95 - 2.82 (m, 2H), 2.08 - 1.93 (m, 1H), 1.69 (s, 3H), 1.47 - 1.35 (m, 3H), 1.01 - 0.82 (m, 3H) ppm |
| 230 | 497.5 | 1H NMR (400 MHz, MeOD) δ = 8.31 (s, 1H), 8.16 - 8.09 (m, 1H), 7.88 - 7.84 (m, 1H), 7.54 (d, J = 9.6 Hz, 1H), 7.30 -7.23 (m, 2H), 6.91 (d, J = 9.6 Hz, 1H), 4.90 (br s, 2H), 4.67 (s, 2H), 4.55 - 4.50 (m, 2H), 4.19 (d, J = 11.6 Hz, 1H), 4.08 - 4.00 (m, 1H), 3.96 - 3.88 (m, 1H), 3.73 - 3.65 (m, 2H), 2.99 - 2.91 (m, 2H), 1.76 - 1.74 (m, 3H), 1.14 - 1.10 (m, 2H), 0.96 - 0.88 (m, 2H) ppm |
| 234 | 496.5 | 1H NMR (400 MHz, MeOD) δ = 8.11 (d, J = 2.0 Hz, 1H), 7.87 - 7.85 (m, 1H), 7.60 (s, 1H), 7.36 - 7.32 (m, 1H), 7.26 (d, J = 8.0 Hz, 1H), 6.18 (d, J = 8.0 Hz, 1H), 5.72 (d, J = 7.6 Hz, 1H), 4.90 (br s, 2H), 4.85 - 4.85 (m, 2H), 4.76 (s, 2H), 4.19 (d, J = 11.6 Hz, 1H), 3.99 - 3.96 (m, 2H), 3.95 - 3.89 (m, 5H), 3.22 - 3.19 (m, 2H), 2.34 - 2.26 (m, 2H), 1.74 (s, 3H) ppm |
| 247 | 497.3 | 1H NMR (400 MHz, MeOD) δ = 8.11 (d, J = 1.6 Hz, 1H), 7.8 - 7.85 (m, 1H), 7.62 (s, 1H), 7.49 (s, 1H), 7.26 (d, J = 8.4 Hz, 1H), 7.03 (s, 1H), 4.89 (d, J = 3.6 Hz, 2H), 4.86 - 4.86 (m, 2H), 4.81 (s, 2H), 4.19 (d, J = 11.6 Hz, 1H), 4.07 - 3.99 (m, 6H), 3.91 (d, J = 11.6 Hz, 1H), 3.26 - 3.23 (m, 2H), 2.46 - 2.36 (m, 2H), 1.74 (s, 3H) ppm |
| 258 | 513.4 | 1H NMR (400 MHz, CHLOROFORM-d) δ = 8.38 (s, 1H), 8.01 (d, J = 1.6 Hz, 1H), 7.80 - 7.72 (m, 2H), 7.59 - 7.52 (m, 1H), 7.17 - 7.12 (m, 2H), 6.26 - 6.23 (m, 1H), 4.87 (s, 2H), 4.73 (d, J = 4.8 Hz, 2H), 4.43 (s, 2H), 4.22 - 4.11 (m, 5H), 3.94 (d, J = 11.2 Hz, 1H), 3.61 - 3.58 (m, 2H), 2.99 - 2.97 (m, 2H), 2.45 - 2.33 (m, 2H), 1.77 (s, 3H) ppm |
| 286 | 440.1 | 1HNMR (400 MHz, DMSO-d6) δ = 9.27 - 9.14 (m, 1H), 8.37 - 8.31 (m, 1H), 8.16 - 8.11 (m, 2H), 7.93 - 7.88 (m, 1H), 7.59 -7.51 (m, 1H), 7.31 - 7.26 (m, 1H), 7.22 (s, 1H), 6.96 - 6.87 (m, 1H), 6.67 - 6.60 (m, 1H), 4.93 - 4.81 (m, 2H), 4.68 (s, 2H), 4.60 - 4.51 (m, 2H), 4.27 - 4.19 (m, 1H), 3.89 - 3.83 (m, 3H), 2.89 - 2.84 (m, 2H), 1.69 (s, 3H)ppm |
| 298 | 496.3 | 1HNMR (400 MHz, DMSO-d6) δ = 8.54 - 8.46 (m, 1H), 8.34 (s, 1H), 8.14 - 8.13 (m, 1H), 7.91 - 7.86 (m, 1H),7.49 - 7.44 (m, 1H), 7.32 (s, 1H), 7.29 - 7.27 (m, 1H), 7.02 (s, 1H), 4.91 (br s, 2H), 4.75 - 4.72 (m, 2H), 4.60 (s, 2H), 4.26 - 4.17 (m, 2H), 4.08 (s, 4H), 3.96 - 3.90 (m, 3H), 2.98 - 2.94 (m, 2H), 2.47 - 2.39 (m, 2H), 1.77 (s, 3H) ppm |
| 304 | 490.5 | 1H NMR (400 MHz, CHLOROFORM-d) δ = 8.40 (s, 1H), 8.03 (d, J = 1.6 Hz, 1H), 7.80-7.78 (m, 1H), 7.66 - 7.62 (m, 2H), 7.14 (d, J = 8.4 Hz, 1H), 6.96 (d, J = 7.6 Hz, 1H), 6.78 (d, J = 8.4 Hz, 1H), 6.62 - 6.34 (m, 1H), 4.87 (s, 2H), 4.76 - 4.74 (m, 4H), 4.14 (d, J = 11.2 Hz, 1H), 3.95 - 3.91 (m, 3H), 2.99-2.96 (m, 2H), 1.76 (s, 3H) ppm |
| 314 | 526.3 | 1H NMR (400 MHz, DMSO-d6) δ = 9.28 - 9.17 (m, 1H), 8.34 (s, 1H), 8.14 (m, 1H), 7.95 - 7.86 (m, 1H), 7.66 (s, 1H), 7.53 (s, 1H), 7.31 - 7.26 (m, 1H), 7.22 (s, 1H), 4.92 - 4.81 (m, 2H), 4.66(s, 2H), 4.61 - 4.50(m, 2H), 4.25 - 4.19 (m, 1H), 3.89 - 3.80 (m, 3H), 3.71 - 3.65 (m, 4H), 3.46 - 3.42 (m, 4H), 2.91 - 2.82 (m, 2H), 1.66 (s,3H) ppm |
| 326 | 484.2 | 1H NMR (400 MHz, DMSO-d6) δ = 9.22 - 9.19 (m, 1H), 8.34 (s, 1H), 8.17 - 8.12 (m, 2H), 7.92 - 7.85 (m, 2H), 7.27 (d, J = 8.4 Hz, 1H), 7.21 (s, 1H), 6.12 (d, J = 6.0 Hz, 1H), 4.94 - 4.78 (m, 2H), 4.71 (s, 2H), 4.61 - 4.48 (m, 2H), 4.21 (d, J = 11.6 Hz, 1H), 3.90 - 3.79 (m, 3H), 3.05 (s, 6H), 2.85 - 2.82 (m, 2H), 1.68 (s, 3H) ppm. |
| 327 | 481.2 | 1H NMR (400 MHz, MeOD) δ = 8.35 (s, 1H), 8.32 (br s, 1H), 8.12 (d, J = 1.6 Hz, 1H), 8.04 (d, J = 6.8 Hz, 1H), 7.87-7.85 (m, 1H), 7.32 (s, 1H), 7.27 (d, J = 8.0 Hz, 1H), 6.72 (d, J = 6.4 Hz, 1H), 4.89 (d, J = 3.2 Hz, 2H), 4.83 (s, 2H), 4.67(s, 2H), 4.20 (d, J = 11.6 Hz, 1H), 3.98 -3.96 (m, 2H), 3.92 (d, J = 11.6 Hz, 1H), 2.97-2.94 (m, 2H), 204-201(m, 1H), 1.74 (s, 3H), 1.14-1.12 (m, 2H), 1.06-1.04(m, 2H) ppm. |
| 328 | 481.2 | 1H NMR (400 MHz, MeOD) δ = 8.32 (s, 1H), 8.12 (d, J = 1.6 Hz, 1H), 7.96 (s, 1H), 7.88-7.85 (m, 1H), 7.74 (s, 1H), 7.31(s, 1H), 7.27 (d, J = 8.0 Hz, 1H), 4.89 (d, J = 3.6 Hz, 2H), 4.73 (s, 2H), 4.67 (s, 2H), 4.20 (d, J = 11.6 Hz, 1H), 3.92-3.89 (m, 3H), 2.96-2.93 (m, 2H), 2.02-1.97 (m, 1H), 1.74 (s, 3H), 1.01-0.95 (m, 4H) ppm. |
| 330 | 526.2 | 1H NMR (400 MHz, DMSO-d6) δ = 9.22 - 9.19 (m, 1H), 8.34 (s, 1H), 8.14 (d, J = 1.2 Hz, 1H), 7.94 - 7.87 (m, 2H), 7.28 (d, J =8.4 Hz, 1H), 7.21 (s, 1H), 6.21 (d, J = 6.0 Hz, 1H), 4.91 - 4.80 (m, 2H), 4.70 (s, 2H), 4.60 - 4.52 (m, 2H), 4.21 (d, J = 11.2 Hz, 1H), 3.93 - 3.75 (m, 3H), 3.62 (s, 8H), 2.85 - 2.82 (m, 2H), 1.68 (s, 3H) ppm. |
| 331 | 510.2 | 1H NMR (400 MHz, DMSO-d6) δ = 9.34 - 9.12 (m, 1H), 8.40 - 8.27 (m, 1H), 8.14 (d, J = 1.2 Hz, 1H), 7.95 - 7.82 (m, 2H), 7.36 - 7.14 (m, 2H), 6.20 - 6.07 (m, 1H), 4.93 - 4.78 (m, 2H), 4.71 (s, 2H), 4.62 - 4.47 (m, 2H), 4.27 - 4.15 (m, 1H), 3.84 - 3.83 (m, 3H), 3.50 - 3.43 (m, 4H), 2.83 - 2.82 (m, 2H), 1.92 - 1.82 (m, 4H), 1.68 (s, 3H) ppm. |
| 332 | 496.2 | 1HNMR (400 MHz, DMSO-d6) δ = 9.32 - 9.11 (m, 1H), 8.41 - 8.30 (m, 2H), 8.14 (s, 1H), 7.94 - 7.85 (m, 2H), 7.32 - 7.25 (m, 1H), 7.19 (s, 1H), 6.22 - 6.15 (m, 1H), 4.96 - 4.87 (m, 2H), 4.69 (s, 2H), 4.59 - 4.50 (m, 2H), 4.27 - 4.17 (m, 1H), 3.97 - 3.90 (m, 4H), 3.88 (s, 1H), 2.87 - 2.80 (m, 2H), 2.24 - 2.19 (m, 2H), 1.69 (s, 3H) ppm |
| 402 | 495.5 | 1H NMR (400 MHz, CHLOROFORM-d) δ = 8.35 (s, 1H), 8.01 (d, J = 1.6 Hz, 1H), 7.79 - 7.76 (m, 1H), 7.57 - 7.36(m, 1H), 7.35 - 7.31 (m, 1H), 7.18 (s, 1H), 7.13 (d, J = 8.0 Hz, 1H), 6.01 (d, J = 8.0 Hz, 1H), 5.68 (d, J = 7.6 Hz, 1H), 4.87 (s, 2H), 4.72 (d, J = 4.8 Hz, 2H), 4.69 (s, 2H), 4.14 (d, J = 11.6 Hz, 1H),3.99 - 3.95 (m, 4H), 3.94 (d, J = 11.2 Hz, 1H), 3.87 - 3.84 (m, 2H), 2.96 - 2.89 (m, 2H), 2.38 - 2.31 (m, 2H), 1.76 (s, 3H) ppm. |

### Example 33: (R)-4-cyano-4-methyl-N-((6-phenylthiazolo[5,4-c]pyridazin-3-yl)methyl)isochromane-6-carboxamide

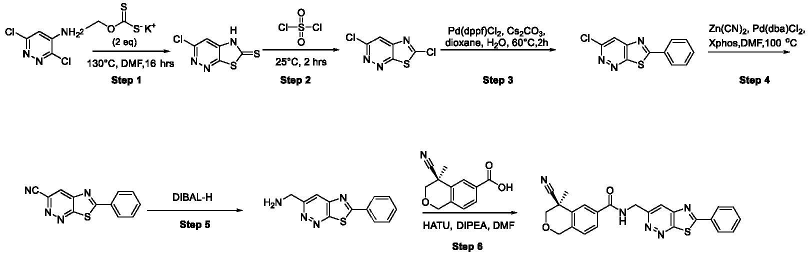

### Step 1: 3-Chlorothiazolo[5,4-c]pyridazine-6(5H)-thione

A mixture of 3,6-dichloropyridazin-4-amine (5.00 g, 30.490 mmol, 1.00 equiv) and potassium ethyl xanthogenate (14.78 g, 121.957 mmol, 4.00 equiv) in DMF (10 mL) was stirred at 130 °C for 12 h. The resulting mixture was diluted with water (500 mL) and extracted with EtOAc (500 mL x 3). The combined organic layers were washed with brine (100 mL x 1), dried over anhydrous Na₂SO₄, filtered and concentrated under reduced pressure to give the title compound (4.8 g, 79.67%) as a white solid that was used directly without further purification.
LCMS (ESI) m/z [M+H]⁺ =204.

### Step 2: 3,6-Dichlorothiazolo[5,4-c]pyridazine

A mixture of 3-chlorothiazolo[5,4-c]pyridazine-6(5H)-thione (2.00 g, 9.820 mmol, 1.00 equiv) in sulfonyl chloride (3.00 mL) was stirred for 2 h at 0 degrees C under nitrogen atmosphere. The residue was neutralized to pH 9 with saturated aq. Na₂CO₃. The resulting mixture was extracted with CH₂Cl₂ (1 L x 3) and concentrated under reduced pressure. The residue was purified by silica gel column chromatography, elution gradient 0 to 100% EtOAc in petroleum ether. Pure fractions were evaporated to dryness to afford intermediate 3 (1.3 g, 61.68%) as a white solid which was used directly without further purification.
LCMS (ESI) m/z [M+H] ⁺ =206.

### Step 3: 3-Chloro-6-phenylthiazolo[5,4-c]pyridazine

To a stirred mixture of 3,6-dichlorothiazolo[5,4-c]pyridazine (800.00 mg, 3.883 mmol, 1.00 equiv) and Cs₂CO₃ (2530.15 mg, 7.765 mmol, 2.00 equiv) in dioxane (4.00 mL) and H₂O (1.00 mL) was added Pd(dppf)Cl₂ (284.10 mg, 0.388 mmol, 0.10 equiv). The resulting mixture was stirred for 2.5 h at room temperature. The resulting mixture was diluted with water (30.0 mL) and extracted with EtOAc (30.0 mL x 3). The combined organic layers were washed with brine (25.0 mL x 1), dried over anhydrous Na₂SO₄, filtered and concentrated under reduced pressure. The residue was purified by prep-HPLC to give the title compound (320 mg, 29.95%) as a white solid.
LCMS (ESI) m/z [M+H]⁺ =248.

### Step 4: 6-Phenylthiazolo[5,4-c]pyridazine-3-carbonitrile

To a stirred mixture of 3-chloro-6-phenylthiazolo[5,4-c]pyridazine (310.00 mg, 1.252 mmol, 1.00 equiv) and Zn(CN)₂ (293.97 mg, 2.503 mmol, 2.00 equiv) in DMF (4.00 mL) were added Pd₂(dba)₃ (229.21 mg, 0.250 mmol, 0.20 equiv) and XPhos (238.65 mg, 0.501 mmol, 0.40 equiv). The reaction mixture was stirred at 40 °C under nitrogen atmosphere for 2 h. The resulting mixture was diluted with water (30.0 mL) and extracted with EtOAc (30.0 mL x 3). The combined organic layers were washed with brine (25.0 mL x 1), dried over anhydrous Na₂SO₄, filtered and concentrated under reduced pressure. The residue was purified by silica gel column chromatography, elution gradient 0 to 100% EtOAc in petroleum ether to give the title compound (150 mg, 48.29%) as a yellow solid.
LCMS (ESI) m/z [M+H]⁺ = 239.

### Step 5: (6-Phenylthiazolo[5,4-c]pyridazin-3-yl)methanamine

To a stirred mixture of 6-phenylthiazolo[5,4-c]pyridazine-3-carbonitrile (130.00 mg, 0.546 mmol, 1.00 equiv) in DCM (4.00 mL) was added DIBAL-H (77.59 mg, 0.546 mmol, 1.00 equiv) dropwise at 0 °C. The mixture was stirred for 30 min. The resulting mixture was diluted with water (30.0 mL) and extracted with EtOAc (30.0 mL x 3). The combined organic layers were washed with brine (25.0 mL x 1), dried over anhydrous Na₂SO₄, filtered and concentrated under reduced pressure. The residue was purified by silica gel column chromatography, elution gradient 0 to 100% EtOAc in petroleum ether, to give the title compound (40 mg, 29.05%) as an off-white solid.
LCMS (ESI) m/z [M+H]⁺ = 243.

### Step 6: (R)-4-Cyano-4-methyl-N-((6-phenylthiazolo[5,4-c]pyridazin-3-yl)methyl)isochromane-6-carboxamide

To a stirred mixture of (*R*)-4-cyano-4-methylisochromane-6-carboxylic acid (16.14 mg, 0.025 mmol, 1.00 equiv) and (6-phenylthiazolo[5,4-c]pyridazin-3-yl)methanamine (18 mg, 0.025 mmol, 1.00 equiv) in DMF (1.50 mL) were added HATU (22.55 mg, 0.021 mmol, 1.00 equiv) and DIEA (24.00 mg, 0.062 mmol, 3.00 equiv). The resulting mixture was stirred for 1 h at room temperature. Without any additional work-up, the mixture was purified by prep-HPLC (Column: Xselect CSH F-Phenyl OBD column, 19*250, 5um; Mobile Phase A: Water (0.05% FA), Mobile Phase B: ACN; Flow rate:25 mL/min; Gradient:27 B to 55 B in 10 min, 55 B to B in min, B to B in min, B to B in min, B to B in min; 254/220 nm; RT₁:8.4; RT₂:; Injection Volume: mL; Number of Runs:;) to give the title compound (6.0 mg, 31.71%) as a white solid.
LCMS (ESI) m/z [M+H]⁺ = 443.05.
¹H NMR (300 MHz, Methanol-*d*₄) δ 8.32 - 8.24 (m, 2H), 8.22 (s, 1H), 8.16 (d, *J =* 1.8 Hz, 1H), 7.91 (dd, J = 8.1, 1.8 Hz, 1H), 7.66 (dt, J = 14.7, 7.1 Hz, 3H), 7.30 (d, *J* = 8.2 Hz, 1H), 5.09 (s, 2H), 4.92 (s, 2H), 4.22 (d, *J* = 11.4 Hz, 1H), 3.94 (d, *J* = 11.4 Hz, 1H), 1.77 (s, 3H).

### Example 34: (S)-N-((2-(6-Cyclopropylpyrazin-2-yl)pyrido[3,4-b]pyrazin-7-yl)methyl)-4-fluoro-4-methylisochromane-6-carboxamide

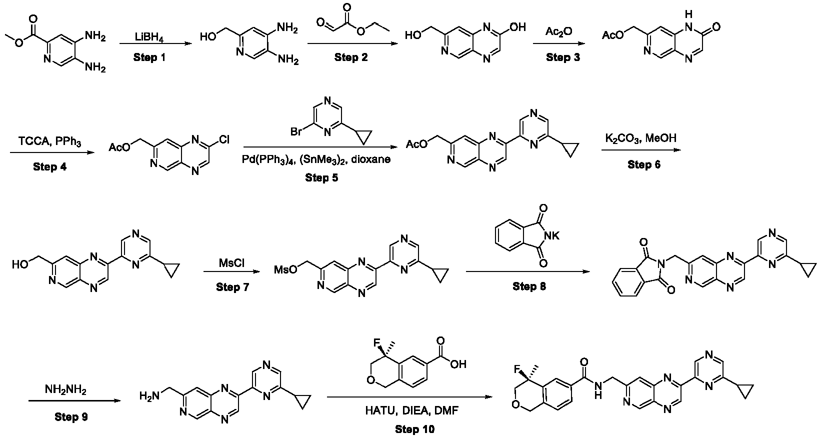

### Step 1: (4,5-Diaminopyridin-2-yl)methanol

To a stirred mixture of methyl 4,5-diaminopyridine-2-carboxylate (8.00 g, 47.856 mmol, 1.00 equiv) in THF (80 mL) was added LiBH4 (2M in THF, 119.64 mL, 239.280 mmol, 5.00 equiv) dropwise at 0 °C. The resulting mixture was stirred for 13 h at room temperature. The mixture was quenched with sat. aq. NaHCO3 (500 mL) and concentrated under vacuum to give a crude product. The crude product was purified by aluminum oxide column chromatography, eluted with DCM/MeOH from 20/1 to 2/1 to give the title compound (6 g, 90.10%) as an off-white solid.
LCMS (ESI) m/z: [M+H]+ = 140.

### Step 2: 7-(Hydroxymethyl)pyrido[3,4-b]pyrazin-2-ol

To a solution of (4,5-diaminopyridin-2-yl)methanol (5.00 g, 35.930 mmol, 1.00 equiv) in n-BuOH (500.00 mL) was added ethyl glyoxylate (7.70 g, 37.727 mmol, 1.05 equiv, 50%). The resulting mixture was stirred for 8 h at 100 °C. The reaction was cooled and filtered, and the filtrate was concentrated to give the title compound (3.1 g, 48.70%) as a yellow solid that was used directly without further purification.
LCMS (ESI) m/z: [M+H]+ = 178.

### Step 3: (2-Oxo-1,2-dihydropyrido[3,4-b]pyrazin-7-yl)methyl acetate

To a solution of 7-(hydroxymethyl)pyrido[3,4-b]pyrazin-2-ol (3.10 g, 17.498 mmol, 1.00 equiv) and DMAP (1068.85 mg, 8.749 mmol, 0.50 equiv) in DCM (50 mL) were added TEA (2.66 g, 26.287 mmol, 1.50 equiv) and Ac2O (5.36 g, 52.503 mmol, 3.00 equiv). The resulting mixture was stirred for 2 h at room temperature. The resulting mixture was concentrated and the residue was purified by silica gel column chromatography eluted with DCM/MeOH=50/1 to give the title compound (3.1 g, 80.82%) as a yellow solid.
LCMS (ESI) m/z: [M+H]+ = 220.

### Step 4: (2-Chloropyrido[3,4-b]pyrazin-7-yl)methyl acetate

To a solution of PPh₃ (5.80 g, 22.112 mmol, 4.8 equiv) in toluene (100.00 mL) was added trichloroisocyanuric acid (1.71 g, 7.371 mmol, 1.60 equiv). The resulting mixture was stirred for 24 h at room temperature. To the above mixture, (2-oxo-1,2-dihydropyrido[3,4-b]pyrazin-7-yl)methyl acetate (1.53 g, 6.97 mmol, 1.00 equiv) was added. The resulting mixture was stirred for an additional 5 h at 110 °C. The mixture was concentrated and the residue was purified by silica gel column chromatograph eluting with PE/EA from 50/1 to 8/1 to give the title compound (610 mg, 55.72%) as an off-white solid.
LCMS (ESI) m/z: [M+H]+ = 238.

### Step 5: (2-(6-Cyclopropylpyrazin-2-yl)pyrido[3,4-b]pyrazin-7-yl)methyl acetate

To a stirred solution of (2-chloropyrido[3,4-b]pyrazin-7-yl)methyl acetate (250.00 mg, 1.052 mmol, 1.00 equiv) and Sn₂Me₆ (413.60 mg, 1.262 mmol, 1.20 equiv) in dioxane (2.00 mL) was added Pd(PPh₃)₄ (121.57 mg, 0.105 mmol, 0.10 equiv) at room temperature under nitrogen atmosphere. The resulting mixture was stirred at 100 °C for 2 h under nitrogen atmosphere. To the above mixture was added additional (2-chloropyrido[3,4-b]pyrazin-7-yl)methyl acetate (250.00 mg, 1.052 mmol, 1.00 equiv) at room temperature and the resulting mixture was stirred overnight at 100 °C. The solvent was removed under reduced pressure and the residue was purified by silica gel column chromatography, eluting with PE/EtOAc (12:1) to give the title compound (298 mg, crude) as a light yellow solid.
(LCMS (ESI) m/z [M+H]+ =321.

### Step 6: (2-(6-Cyclopropylpyrazin-2-yl)pyrido[3,4-b]pyrazin-7-yl)methanol

To a stirred solution of (2-(6-cyclopropylpyrazin-2-yl)pyrido[3,4-*b*]pyrazin-7-yl)methyl acetate (285.00 mg, 0.887 mmol, 1.00 equiv) in MeOH (5.00 mL) was added K2CO3 (245.15 mg, 1.774 mmol, 2.00 equiv). The resulting mixture was stirred at 60 °C for 2 h. The solid was filtered off and the filtrate was concentrated under vacuum to give the title compound (48 mg, 30.32%) as a light yellow solid that was used directly without further purification.
(LCMS (ESI) m/z [M+H]+ =280.

### Step 7: (2-(6-Cyclopropylpyrazin-2-yl)pyrido[3,4-b]pyrazin-7-yl)methyl methanesulfonate

To a stirred solution (2-(6-cyclopropylpyrazin-2-yl)pyrido[3,4-*b*]pyrazin-7-yl)methanol (270.00 mg, 0.967 mmol, 1.00 equiv) and TEA (195.64 mg, 1.934 mmol, 2.00 equiv) in DCM (5.00 mL) was added MsCl (166.10 mg, 1.451 mmol, 1.50 equiv) at room temperature. The resulting mixture was stirred for 2 h at room temperature. The resulting mixture was concentrated under vacuum. The residue was purified by silica gel column chromatography, eluting with PE/EtOAc (1:1) to give the title compound (115 mg, 33.29%) as a light yellow solid.
(LCMS (ESI) m/z [M+H]⁺ =358.

### Step 8: 2-((2-(6-Cyclopropylpyrazin-2-yl)pyrido[3,4-b]pyrazin-7-yl)methyl)isoindoline-1,3-dione

To a stirred solution of (2-(6-cyclopropylpyrazin-2-yl)pyrido[3,4-*b*]pyrazin-7-yl)methyl methanesulfonate (105.00 mg, 0.294 mmol, 1.00 equiv) in DMF (2.00 mL) was added potassium 1,3-dioxoisoindolin-2-ide (108.84 mg, 0.588 mmol, 2.00 equiv) at room temperature. The resulting mixture was stirred for 2 h at room temperature. The reaction was quenched with water (10 mL) and the precipitated solids were collected by filtration and washed with H₂O (3x10 mL). The resulting solid was dried under vacuum to give the title compound (69 mg, 57.50%) as a light yellow solid.
(LCMS (ESI) m/z [M+H]⁺ =409.

### Step 9: (2-(6-Cyclopropylpyrazin-2-yl)pyrido[3,4-b]pyrazin-7-yl)methanamine

To a stirred solution of 2-((2-(6-cyclopropylpyrazin-2-yl)pyrido[3,4-*b*]pyrazin-7-yl)methyl)isoindoline-1,3-dione (60.00 mg, 0.147 mmol, 1.00 equiv) in EtOH (5.00 mL) was added hydrazine hydrate (0.50 mL) at room temperature. The resulting mixture was stirred for 2 h at 80 °C under nitrogen atmosphere. The resulting mixture was concentrated under vacuum to the title compound (72 mg, crude) as a light yellow solid that was used directly without further purification.
(LCMS (ESI) m/z [M+H]⁺ =279.

### Step 10: (S)-N-((2-(6-Cyclopropylpyrazin-2-yl)pyrido[3,4-b]pyrazin-7-yl)methyl)-4-fluoro-4-methylisochromane-6-carboxamide

To a stirred solution of (*S*)-4-fluoro-4-methylisochromane-6-carboxylic acid (20.00 mg, 0.095 mmol, 1.00 equiv) and HATU (43.41 mg, 0.114 mmol, 1.20 equiv) in DMF (2.00 mL) were added DIEA (36.89 mg, 0.285 mmol, 3.00 equiv) and (2-(6-cyclopropylpyrazin-2-yl)pyrido[3,4-*b*]pyrazin-7-yl)methanamine (29.13 mg, 0.105 mmol, 1.10 equiv) at room temperature. The resulting mixture was stirred for 2 h at room temperature. Without any additional work-up, the mixture was purified by prep-HPLC to give the title compound (20 mg, 44.68%) as a light yellow solid.
LCMS (ESI) m/z: [M+H]+ =471.20.
1H NMR (400 MHz, DMSO-d6) δ 9.84 (s, 1H), 9.55 (s, 1H), 9.46 (t, J = 5.8 Hz, 1H), 9.40 (s, 1H), 8.85 (s, 1H), 8.28 (d, J = 1.8 Hz, 1H), 8.02 - 7.90 (m, 2H), 7.31 (d, J = 8.0 Hz, 1H), 4.95 - 4.81 (m, 3H), 4.79 - 4.69 (m, 1H), 4.08 (t, J = 13.0 Hz, 1H), 3.85 (dd, J = 25.1, 12.5 Hz, 1H), 2.43 - 2.31 (m, 1H), 1.70 (d, J = 20.7 Hz, 3H), 1.29 - 1.09 (m, 4H).

The following examples in Table 24 were prepared using standard chemical manipulations and procedures similar to those used for the preparation of **Example 34.**

**Table 24. Compounds of the Invention**

| # | LCMS (m/z) | 1H NMR |
|---|---|---|
| 143 | 513.20 | ¹H NMR (300 MHz, Methanol-*d*₄) δ 9.97 (s, 1H), 9.47 (d, *J* = 0.8 Hz, 1H), 8.55 (dd, *J* = 8.4, 0.8 Hz, 1H), 8.39 (dd, *J* = 7.6, 0.9 Hz, 1H), 8.29 - 8.24 (m, 1H), 8.06 - 7.90 (m, 3H), 7.30 (d, *J* = 8.1 Hz, 1H), 4.97 (s, 2H), 4.86 - 4.76 (m, 2H), 4.37 - 4.29 (m, 2H), 4.10 (t, *J* = 12.3 Hz, 1H), 3.98 - 3.83 (m, 1H), 2.79 - 2.69 (m, 2H), 2.32 - 2.18 (m, 2H), 1.75 (d, 3H). |
| 161 | 463.10 | ¹H NMR (400 MHz, DMSO-*d*₆) δ 9.42 (t, *J =* 5.9 Hz, 1H), 9.31 (d, *J* = 1.2 Hz, 2H), 8.49 (s, 1H), 8.28 (d, *J* = 1.7 Hz, 1H), 7.97 (dt, J = 8.0, 1.7 Hz, 1H), 7.63 (d, *J* = 0.9 Hz, 1H), 7.31 (d, *J* = 8.0 Hz, 1H), 4.87 (dd, J = 15.9, 2.5 Hz, 1H), 4.85 - 4.69 (m, 3H), 4.08 (t, *J* = 13.0 Hz, 1H), 4.00 (s, 3H), 3.85 (dd, J = 25.3, 12.5 Hz, 1H), 3.79 (s, 3H), 1.70 (d, *J* = 20.7 Hz, 3H). |
| 183 | 487.05 | ¹H NMR (300 MHz, DMSO-*d*₆) δ 9.88 (s, 1H), 9.66 - 9.56 (m, 2H), 9.13 - 9.06 (m, 1H), 8.62 (d, *J* = 4.9 Hz, 2H), 8.04 - 7.98 (m, 1H), 7.66 (d, *J* = 5.0 Hz, 1H), 4.95 - 4.71 (m, 4H), 4.26 - 4.08 (m, 5H), 3.92 (dd, *J* = 25.3, 12.7 Hz, 1H), 2.46 - 2.31 (m, 2H), 1.72 (d, *J* = 20.9 Hz, 3H). |
| 190 | 486.20 | ¹H NMR (400 MHz, DMSO-*d*₆) δ 9.86 (s, 1H), 9.56 (s, 1H), 9.45 (t, *J* = 5.9 Hz, 1H), 8.60 (d, *J* = 4.9 Hz, 1H), 8.31 - 8.24 (m, 1H), 8.01 - 7.91 (m, 2H), 7.66 (d, *J* = 5.0 Hz, 1H), 7.31 (d, *J* = 8.1 Hz, 1H), 4.92 - 4.82 (m, 3H), 4.77 - 4.71 (m, 1H), 4.19 (t, *J* = 7.5 Hz, 4H), 4.08 (t, *J* = 13.0 Hz, 1H), 3.84 (dd, J = 25.2, 12.5 Hz, 1H), 2.44 - 2.32 (m, 2H), 1.69 (d, *J* = 20.7 Hz, 3H). |
| 207 | 503.25 | ¹H NMR (300 MHz, DMSO-*d*₆) δ 9.57 (d, *J* = 0.8 Hz, 1H), 9.49 - 9.38 (m, 2H), 8.27 (s, 1H), 8.11 - 8.06 (m, 1H), 8.01 - 7.91 (m, 2H), 7.31 (d, *J* = 8.1 Hz, 1H), 7.25 - 7.17 (m, 1H), 4.92 - 4.81 (m, 3H), 4.78 - 4.68 (m, 1H), 4.23 - 4.17 (m, 3H), 4.10 - 4.02 (m, 1H), 3.85 (dd, J = 25.1, 12.6 Hz, 1H), 2.43 - 2.32 (m, 2H), 1.70 (d, *J* = 20.7 Hz, 3H). |
| 214 | 485.30 | ¹H NMR (300 MHz, DMSO-*d*₆) δ 9.90 (s, 1H), 9.50 (s, 1H), 9.48 - 9.39 (m, 1H), 8.28 (s, 1H), 7.98 (d, *J* = 7.9 Hz, 1H), 7.93 - 7.64 (m, 3H), 7.31 (d, *J* = 8.2 Hz, 1H), 6.60 (d, *J* = 8.0 Hz, 1H), 4.97 - 4.62 (m, 4H), 4.21 - 3.96 (m, 5H), 3.85 (dd, *J* = 24.9, 12.6 Hz, 1H), 2.45 - 2.22 (m, 2H), 1.70 (d, *J* = 20.7 Hz, 3H). |
| 215 | 504.20 | ¹H NMR (400 MHz, DMSO-*d*₆) δ 9.59 (d, *J* = 12.5 Hz, 2H), 9.40 (s, 1H), 9.08 (s, 1H), 8.61 (s, 1H), 8.12 - 7.97 (m, 2H), 7.20 (s, 1H), 4.97 - 4.71 (m, 4H), 4.27 - 4.12 (m, 5H), 3.91 (dd, J = 25.2, 12.6 Hz, 1H), 2.45 - 2.31 (m, 2H), 1.71 (d, *J* = 20.9 Hz, 3H). |
| 216 | 486.30 | ¹H NMR (300 MHz, DMSO-*d*₆) δ 9.90 (s, 1H), 9.67 - 9.57 (m, 1H), 9.51 (s, 1H), 9.10 (d, *J* = 2.1 Hz, 1H), 8.63 (s, 1H), 7.96 (s, 1H), 7.90 - 7.67 (m, 2H), 6.61 (d, *J* = 8.0 Hz, 1H), 4.97 - 4.71 (m, 4H), 4.26 - 4.02 (m, 5H), 3.92 (dd, J = 25.3, 12.6 Hz, 1H), 2.45 - 2.26 (m, 2H), 1.72 (d, *J* = 20.9 Hz, 3H). |
| 256 | 459.05 | ¹H NMR (300 MHz, DMSO-*d*₆) δ 9.54 - 9.39 (m, 3H), 8.28 (d, *J =* 1.8 Hz, 1H), 8.02 - 7.93 (m, 1H), 7.92 - 7.81 (m, 2H), 7.66 - 7.53 (m, 1H), 7.37 = 7.24 (m, 2H), 7.22 = 7.11 (m, 1H), 4.94 - 4.80 (m, 3H), 4.73 (dd, *J* = 15.8, 4.5 Hz, 1H), 4.08 (t, *J* = 13.0 Hz, 1H), 3.98 - 3.76 (m, 4H), 1.70 (d, *J* = 20.7 Hz, 3H). |
| 463 | 435.9 | 1H NMR (400 MHz, DMSO-d6) δ = 9.71 (s, 1H), 9.49 (s, 1H), 9.47 - 9.41 (m, 1H), 8.43 - 8.34 (m, 2H), 8.20 (d, J = 1.6 Hz, 1H), 7.99 - 7.93 (m, 1H), 7.90 (s, 1H), 7.67 - 7.59 (m, 3H), 7.36 - 7.27 (m, 1H), 5.01 - 4.79 (m, 4H), 4.29 - 4.16 (m, 1H), 3.89 - 3.85 (m, 1H), 1.70 (s, 3H) ppm. |

### Examples 35 and 36: (R)-4-Cyano-N-((2-(4-fluoro-3-methyl-1H-pyrazol-1-yl)-1,6-naphthyridin-7-yl)methyl)-4-methylisochromane-6-carboxamide and (R)-4-cyano-N-((2-(4-fluoro-1H-pyrazol-1-yl)-1,6-naphthyridin-7-yl)methyl)-4-methylisochromane-6-carboxamide

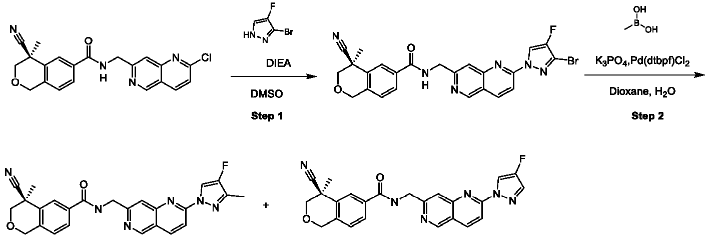

### Step 1: (R)-N-((2-(3-Bromo-4-fluoro-1H-pyrazol-1-yl)-1,6-naphthyridin-7-yl)methyl)-4-cyano-4-methylisochromane-6-carboxamide

A mixture of (*R*)-N-((2-chloro-1,6-naphthyridin-7-yl)methyl)-4-cyano-4-methylisochromane-6-carboxamide (30 mg, 76.37 umol), 3-bromo-4-fluoro-1H-pyrazole (62.99 mg, 381.85 umol) and DIEA (98.70 mg, 763.70 umol, 133.02 uL) in DMSO (0.5 mL) was stirred at 120 °C for 2 hrs. The reaction was poured into water (4 mL) and extracted with EA (4 mL*3). The combined organic layers were dried by Na₂SO₄, filtered and concentrated under reduced pressure. The residue was purified by prep-HPLC (FA condition) to give the title compound (30 mg, 56.38 umol, 73.83% yield,) as a white solid.
LCMS (ESI) m/z: [81BrM+H]+ = 523.1.
1HNMR (400 MHz, DMSO-d6) δ = 9.45 - 9.42 (m, 1H), 9.41 (s, 1H), 9.08 (d, J = 4.8 Hz, 1H), 8.78 (d, J = 8.8 Hz, 1H), 8.23 - 8.16 (m, 2H), 7.97 - 7.94 (m, 1H), 7.71 (s, 1H), 7.32 (d, J = 8.4 Hz, 1H), 4.95 - 4.83 (m, 2H), 4.81 (d, J = 6.0 Hz, 2H), 4.24 (d, J = 11.2 Hz, 1H), 3.88 (d, J = 11.6 Hz, 1H), 1.71 (s, 3H) ppm.

### Step 2: (R)-4-Cyano-N-((2-(4-fluoro-3-methyl-1H-pyrazol-1-yl)-1,6-naphthyridin-7-yl)methyl)-4-methylisochromane-6-carboxamide and (R)-4-cyano-N-((2-(4-fluoro-1H-pyrazol-1-yl)-1,6-naphthyridin-7-yl)methyl)-4-methylisochromane-6-carboxamide

A mixture of (R)-N-((2-(3-bromo-4-fluoro-1*H*-pyrazol-1-yl)-1,6-naphthyridin-7-yl)methyl)-4-cyano-4-methylisochromane-6-carboxamide (30 mg, 57.54 umol), methylboronic acid (4.13 mg, 69.05 umol), K₃PO₄ (36.64 mg, 172.62 umol) and [1,1' -bis(di-tert-butylphosphino)ferrocene]dichloropalladium(II) (37.50 mg, 57.54 umol) in dioxane (1 mL) was degassed and purged with N₂ 3 times. The mixture was stirred at 60 °C for 2 hrs under N₂ atmosphere. The reaction was poured into 4 mL of H₂O and extracted with EA (4 mL*3). The combined organic layers were dried over Na₂SO₄, filtered and concentrated under reduced pressure. The residue was purified by prep-HPLC (column: Phenomenex Luna C18 150*25mm*10um;mobile phase: [water(0.225%FA)- ACN];B%: 38%-68%,10min) to give (R)-4-cyano-N-((2-(4-fluoro-3-methyl-1*H*-pyrazol-1-yl)-1,6-naphthyridin-7-yl)methyl)-4-methylisochromane-6-carboxamide (2.83 mg, 5.35 umol, 9.30% yield, FA salt) as a yellow solid and (R)-4-cyano-N-((2-(4-fluoro-1*H*-pyrazol-1-yl)-1,6-naphthyridin-7-yl)methyl)-4-methylisochromane-6-carboxamide (4.77 mg, 9.44 umol, 16.41% yield, FA salt) as a yellow solid.

### (R)-4-cyano-N-((2-(4-fluoro-3-methyl-1H-pyrazol-1-yl)-1,6-naphthyridin-7-yl)methyl)-4-methylisochromane-6-carboxamide

LCMS (ESI) m/z: [M+H]+= 457.3.
1HNMR (400 MHz, METHANOL-d4) δ = 9.23 (s, 1H), 8.65 (d, J = 4.8 Hz, 1H), 8.58 (d, J = 9.2 Hz, 1H), 8.24 (d, J = 9.2 Hz, 1H), 8.17 (s, 1H), 7.93 - 7.90 (m, 1H), 7.80 (s, 1H), 7.29 (d, J = 8.0 Hz, 1H), 4.91 (d, J = 3.2 Hz, 2H), 4.89 (s, 2H), 4.21 (d, J = 11.6 Hz, 1H), 3.93 (d, J = 11.2 Hz, 1H), 2.34 (s, 3H), 1.77 (s, 3H) ppm.

### (R)-4-cyano-N-((2-(4-fluoro-1H-pyrazol-1-yl)-1,6-naphthyridin-7-yl)methyl)-4-methylisochromane-6-carboxamide

LCMS (ESI) m/z: [M+H]+= 443.2.
1HNMR (400 MHz, METHANOL-d4) δ = 9.26 (s, 1H), 8.75 (d, J = 4.8 Hz, 1H), 8.62 (d, J = 8.8 Hz, 1H), 8.30 (d, J = 8.8 Hz, 1H), 8.17 (s, 1H), 7.93 - 7.90 (m, 1H), 7.86 - 7.80 (m, 2H), 7.29 (d, J = 8.0 Hz, 1H), 4.91 (d, J = 3.6 Hz, 2H), 4.90 (s, 2H), 4.21 (d, J = 11.2 Hz, 1H), 3.93 (d, J = 11.2 Hz, 1H), 1.77 (s, 3H) ppm.

### Example 37: Methyl 1-(7-(((R)-4-cyano-4-methylisochromane-6-carboxamido)methyl)-1,6-naphthyridin-2-yl)-3-azabicyclo[4.1.0]heptane-3-carboxylate

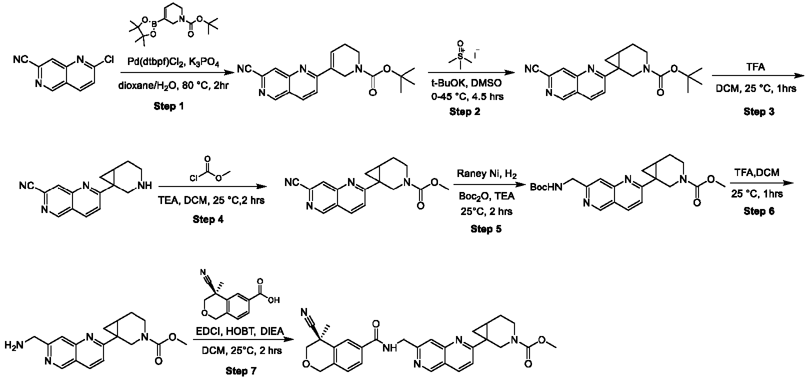

### Step 1: tert-Butyl 5-(7-cyano-1,6-naphthyridin-2-yl)-3,6-dihydropyridine-1(2H)-carboxylate

To a mixture of 2-chloro-1,6-naphthyridine-7-carbonitrile (400 mg, 2.11 mmol) and tert-butyl 5-(4,4,5,5-tetramethyl-1,3,2-dioxaborolan-2-yl)-3,6-dihydro-2H-pyridine-1-carboxylate (848.03 mg, 2.74 mmol) in dioxane (4 mL) and H₂O (0.4 mL) was added [1,1' -bis(di-tert-butylphosphino)ferrocene]dichloropalladium(ll) (137.50 mg, 210.97 umol) and K₃PO₄ (1.34 g, 6.33 mmol). The mixture was purged with N₂ for 1 min and the resulting mixture was stirred at 80 °C for 2 hrs. The reaction mixture was diluted with H₂O (30 mL) and extracted with EA (40 mL*3). The combined organic layers were washed with brine (30 mL*2), dried over Na₂SO₄, filtered and concentrated under reduced pressure. The residue was slurried in MeOH (8 mL), and the precipitate was collected by filtration and dried. Further purification by column chromatography (SiO₂, PE:EtOAc=20:1-1:1) gave the title compound (460 mg, 1.37 mmol, 64.82% yield) as a yellow solid.
LCMS (ESI) m/z: [M+H]+ = 337.2.

### Step 2: tert-Butyl 1-(7-cyano-1,6-naphthyridin-2-yl)-3-azabicyclo[4.1.0]heptane-3-carboxylate

To a mixture of t-BuOK (70.05 mg, 624.28 umol) in DMSO (2 mL) was added trimethylsulfoxonium iodide (137.39 mg, 624.28 umol) at 0 °C and the mixture was stirred at 25 °C for 30 min. The mixture was then added to the solution of *tert*-butyl 5-(7-cyano-1,6-naphthyridin-2-yl)-3,6-dihydropyridine-1(2*H*)-carboxylate (210 mg, 624.28 umol) dissolved in DMSO (1 mL) and the resulting mixture was stirred at 45 °C for 4 hrs. The reaction mixture was diluted with H₂O (30 mL) and extracted with EA (40 mL*3). The combined organic layers were washed with brine (30 mL * 2), dried over Na₂SO₄, filtered and concentrated under reduced pressure. The residue was purified by column chromatography (SiO₂, PE:EtOAc = 20:1-1:1) to give the title compound (80 mg, 215.17 umol, 34.47 % yield) as a colorless oil.
LCMS (ESI) m/z: [M+H]+ = 351.2.
1H NMR (400 MHz, CDCl₃) δ = 9.21 (s, 1H), 8.30 - 8.19 (m, 2H), 7.71 - 7.40 (m, 1H), 4.32 - 4.25 (m, 1H), 4.24 - 4.14 (m, 1H), 3.76 - 3.52 (m, 1H), 3.20 - 2.96 (m, 1H), 2.18 - (m, 1H), 1.97 - 1.82 (m, 2H), 1.78 - 1.60 (m, 1H), 1.54 - 1.46 (m, 10H) ppm.

### Step 3: 2-(3-Azabicyclo[4.1.0]heptan-1-yl)-1,6-naphthyridine-7-carbonitrile

To a mixture of tert-butyl 1-(7-cyano-1,6-naphthyridin-2-yl)-3-azabicyclo[4.1.0]heptane-3-carboxylate (120 mg, 342.45 umol) in DCM (2 mL) was added TFA (770.00 mg, 6.75 mmol, 0.5 mL) at 0 °C and the mixture was stirred at 25 °C for 1 hr. The reaction mixture was poured into sat. NaHCO₃ (20 mL) and extracted with EA (20 mL * 3). The combined organic layers were washed with brine (30 mL), dried over Na₂SO₄, filtered and concentrated under reduced pressure to give the title compound (85 mg, crude) as a yellow solid.

### Step 4: Methyl 1-(7-cyano-1,6-naphthyridin-2-yl)-3-azabicyclo[4.1.0]heptane-3-carboxylate

To a mixture of 2-(3-azabicyclo[4.1.0]heptan-1-yl)-1,6-naphthyridine-7-carbonitrile (85 mg, 339.60 umol) and TEA (171.82 mg, 1.70 mmol, 236.34 uL) in DCM (1 mL) was added methyl chloroformate (96.27 mg, 1.02 mmol, 78.91 uL) dropwise at 0 °C, and the mixture was stirred at 25 °C for 2 hrs. The reaction mixture was diluted with H₂O (30 mL) and extracted with EA (40 mL * 3). The combined organic layers were washed with brine (30 mL * 2), dried over Na₂SO₄, filtered and concentrated under reduced pressure. The residue was purified by column chromatography (SiO₂, PE : EtOAc = 20:1-1:1) to give the title compound (55 mg, 173.78 umol, 51.17 % yield) as a colorless oil.
LCMS (ESI) m/z: [M+H]+ = 309.1.
1H NMR (400 MHz, DMSO-d6) δ = 9.43 (d, J = 0.8 Hz, 1H), 8.60 (d, J = 8.8 Hz, 1H), 8.52 (s, 1H), 7.76 - 7.60 (m, 1H), 4.35 - 4.15 (m, 2H), 3.64 (s, 3H), 3.63 - 3.50 (m, 1H), 3.16 - 2.97 (m, 1H), 2.18 - 2.06 (m, 1H), 1.96 - 1.78 (m, 2H), 1.54 - 1.53 (m, 1H), 1.10 - 1.08(m, 1H) ppm.

### Step 5: Methyl 1-(7-(((tert-butoxycarbonyl)amino)methyl)-1,6-naphthyridin-2-yl)-3-azabicyclo[4.1.0]heptane-3-carboxylate

To a mixture of methyl 1-(7-cyano-1,6-naphthyridin-2-yl)-3-azabicyclo[4.1.0]heptane-3-carboxylate (55 mg, 178.38 umol) in MeOH (10 mL) was added TEA (36.10 mg, 356.76 umol, 49.66 uL) and Boc₂O (58.40 mg, 267.57 umol, 61.47 uL). The mixture was purged with N₂ and Raney-Ni (76.41 mg, 891.89 umol) was added. The mixture was then purged with H₂ 3 times and the resulting mixture was stirred at 25 °C under H₂ (15psi) for 2 hrs. The reaction mixture was filtered and the filtrate was concentrated under vacuum. The residue was purified by column chromatography (SiO₂, PE : EtOAc =20:1-1:1) to give the title compound (35 mg, 84.27 umol, 47.24% yield) as a colorless oil.
LCMS (ESI) m/z: [M+H]+ = 413.2.

### Step 6: Methyl 1-(7-(aminomethyl)-1,6-naphthyridin-2-yl)-3-azabicyclo[4.1.0]heptane-3-carboxylate

To a solution of methyl 1-(7-(((tert-butoxycarbonyl)amino)methyl)-1,6-naphthyridin-2-yl)-3-azabicyclo[4.1.0]heptane-3-carboxylate (35 mg, 84.85 umol) in DCM (3 mL) was added TFA (1 mL) at 0°C. The mixture was stirred at 25 °C for 1 hr. The reaction mixture was poured into sat. NaHCO₃ (20 mL ) and extracted with EA (20 mL * 3). The combined organic layers were washed with brine (30 mL), dried over Na₂SO₄, filtered and concentrated under reduced pressure to give the title compound (28 mg, crude) as a yellow solid.
LCMS (ESI) m/z: [M+H]+ = 313.2.

### Step 7: Methyl 1-(7-(((R)-4-cyano-4-methylisochromane-6-carboxamido)methyl)-1,6-naphthyridin-2-yl)-3-azabicyclo[4.1.0]heptane-3-carboxylate

To a solution of (*R*)-4-cyano-4-methylisochromane-6-carboxylic acid (23.37 mg, 107.57 umol) in DCM (1 mL) was added EDCI (22.34 mg, 116.53 umol), HOBt (15.75 mg, 116.53 umol) and DIEA (34.76 mg, 268.92 umol,46.84 uL). Methyl 1-(7-(aminomethyl)-1,6-naphthyridin-2-yl)-3-azabicyclo[4.1.0]heptane-3-carboxylate (28 mg, 89.64 umol) was then added and the mixture was stirred at 25 °C for 2 hrs. The reaction mixture was poured into NaHCO₃ (30 mL) and extracted with EA (20 mL*3). The combined organic layers were washed with brine (30 mL), dried over Na₂SO₄, filtered and concentrated. The residue was purified by prep-HPLC (0.1% FA condition) to give the title compound (5.30 mg, 10.36 umol, 11.56% yield) as a white solid.
LCMS (ESI) m/z: [M+H]+ = 512.2.
1H NMR (400 MHz, DMSO-d6) δ = 9.41 - 9.32 (m, 1H), 9.28 (s, 1H), 8.45 (d, J = 8.8 Hz, 1H), 8.19 (d, J = 1.6 Hz, 1H), 7.94(d, J = 1.6, 1H), 7.64 (s, 1H), 7.51 - 7.39 (m, 1H), 7.31 (d, J = 8.4 Hz, 1H), 4.94 - 4.81 (m, 2H), 4.77 (d, J = 5.6 Hz, 2H), 4.33 - 4.19 (m, 2H), 4.18 - 4.07 (m, 1H), 3.87 (d, J = 11.2 Hz, 1H), 3.61 (s, 3H), 3.52 - 3.43 (m, 1H), 3.13 - 2.97 (m, 1H), 2.14 - 2.04 (m, 1H), 1.88 - 1.76 (m, 2H), 1.70 (s, 3H), 1.53 - 1.43 (m, 1H), 1.04 - 0.96 (m, 1H) ppm.

### Example 38. Assay for ATPase catalytic activity of BRM and BRG-1

The ATPase catalytic activity of BRM or BRG-1 was measured by an in vitro biochemical assay using ADP-Glo^{™} (Promega, V9102). The ADP-Glo^{™} kinase assay is performed in two steps once the reaction is complete. The first step is to deplete any unconsumed ATP in the reaction. The second step is to convert the reaction product ADP to ATP, which will be utilized by the luciferase to generate luminesce and be detected by a luminescence reader, such as Envision.

The assay reaction mixture (10 µL) contains 30 nM of BRM or BRG-1, 20 nM salmon sperm DNA (from Invitrogen, UltraPure^{™} Salmon Sperm DNA Solution, cat# 15632011), and 400 µM of ATP in the ATPase assay buffer, which comprises of 20 mM Tris, pH 8, 20 mM MgCl₂, 50 mM NaCl, 0.1% Tween-20, and 1 mM fresh DTT (Pierce^{™} DTT (Dithiothreitol), cat# 20290). The reaction is initiated by the addition of the 2.5 µL ATPase solution to 2.5 µL ATP/DNA solution on low volume white Proxiplate-384 plus plate (PerkinElmer,cat # 6008280) and incubates at room temperature for 1 hour. Then following addition of 5 µL of ADP-Glo^{™} Reagent provided in the kit, the reaction incubates at room temperature for 40 minutes. Then 10 µL of Kinase Detection Reagent provided in the kit is added to convert ADP to ATP, and the reaction incubates at room temperature for 60 minutes. Finally, luminescence measurement is collected with a plate-reading luminometer, such as Envision.

BRM and BRG-1 were synthesized from high five insect cell lines with a purity of greater than 90%. IC₅₀ data for compounds 1-126 from the ATPase catalytic activity assay described herein are shown in Table 25 below.

**Table 25. BRM and BRG-1 Inhibition Data for Compounds of the Invention**

| **Compound #** | **BRM ATPase: IC50 (uM)** | **BRM Max % Inhibition** | **BRG1 ATPase: IC50 (uM)** | **BRG1 Max % Inhibition** |
|---|---|---|---|---|
| **1** | +++ | **90** | **+** | **56** |
| 2 | +++ | 96 | + | 65 |
| 3 | ++++ | 99 | + | 78 |
| 4 | +++ | 97 | + | 74 |
| 5 | + | 64 | + | 12 |
| 6 | +++ | 95 | + | 44 |
| 7 | + | 81 | + | 12 |
| 8 | + | 73 | + | 9 |
| 9 | +++ | 95 | + | 56 |
| 10 | + | 71 | + | 30 |
| 11 | + | 82 | + | 26 |
| 12 | + | 79 | + | 22 |
| 13 | + | 84 | + | 44 |
| 14 | ++ | 94 | + | 64 |
| 15 | ++ | 94 | + | 64 |
| 16 | +++ | 94 | + | 51 |
| 17 | +++ | 94 | + | 58 |
| 18 | ++++ | 93 | + | 87 |
| 19 | +++ | 97 | ++ | 89 |
| 20 | ++ | 83 | + | 41 |
| 21 | + | 78 | + | 31 |
| 22 | + | 70 | + | 31 |
| 23 | ++ | 85 | + | 35 |
| 24 | +++ | 94 | + | 65 |
| 25 | +++ | 94 | + | 68 |
| 26 | ++++ | 100 | +++ | 90 |
| 27 | +++ | 97 | + | 62 |
| 28 | ++++ | 98 | ++ | 86 |
| 29 | +++ | 98 | + | 69 |
| 30 | +++ | 92 | + | 35 |
| 31 | +++ | 96 | + | 73 |
| 32 | + | 89 | + | 55 |
| 33 | +++ | 98 | + | 78 |
| 34 | +++ | 94 | + | 53 |
| 35 | ++ | 91 | + | 47 |
| 36 | + | 64 | + | 44 |
| 37 | + | 76 | + | 21 |
| 38 | ++++ | 97 | + | 86 |
| 39 | + | 75 | + | 28 |
| 40 | +++ | 96 | + | 67 |
| 41 | ++ | 85 | + | 55 |
| 42 | +++ | 97 | ++ | 84 |
| 43 | + | 68 | + | 34 |
| 44 | +++ | 97 | + | 69 |
| 45 | ++++ | 96 | ++ | 79 |
| 46 | + | 78 | + | 29 |
| 47 | +++ | 90 | + | 47 |
| 48 | + | 67 | + | 11 |
| 49 | +++ | 84 | + | 26 |
| 50 | +++ | 90 | + | 53 |
| 51 | ++++ | 99 | +++ | 94 |
| 52 | +++ | 86 | + | 34 |
| 53 | +++ | 88 | + | 48 |
| 54 | +++ | 81 | + | 42 |
| 55 | +++ | 82 | + | 33 |
| 56 | ++++ | 98 | +++ | 92 |
| 57 | +++ | 85 | + | 37 |
| 58 | +++ | 92 | ++ | 50 |
| 59 | +++ | 93 | ++ | 56 |
| 60 | +++ | 93 | ++ | 75 |
| 61 | +++ | 85 | + | 25 |
| 62 | +++ | 90 | + | 42 |
| 63 | ++++ | 99 | ++ | 84 |
| 64 | +++ | 87 | + | 36 |
| 65 | +++ | 90 | + | 39 |
| 66 | +++ | 99 | ++ | 76 |
| 67 | +++ | 97 | ++ | 84 |
| 68 | ++++ | 98 | +++ | 91 |
| 69 | +++ | 90 | ++ | 50 |
| 70 | +++ | 94 | ++ | 55 |
| 71 | +++ | 89 | + | 42 |
| 72 | ++++ | 94 | +++ | 91 |
| 73 | +++ | 84 | + | 44 |
| 74 | ++++ | 98 | +++ | 87 |
| 75 | +++ | 97 | ++ | 54 |
| 76 | +++ | 98 | + | 91 |
| 77 | +++ | 99 | ++ | 102 |
| 78 | ++++ | 96 | ++ | 79 |
| 79 | +++ | 96 | ++ | 74 |
| 80 | +++ | 83 | + | 29 |
| 81 | ++++ | 98 | +++ | 90 |
| 82 | +++ | 88 | + | 38 |
| 83 | ++++ | 101 | ++++ | 99 |
| 84 | +++ | 89 | ++ | 53 |
| 85 | ++++ | 98 | + | 77 |
| 86 | +++ | 96 | + | 62 |
| 87 | ++ | 88 | + | 87 |
| 88 | +++ | 96 | + | 73 |
| 89 | + | 76 | + | 15 |
| 90 | + | 82 | + | 24 |
| 91 | ++++ | 97 | ++ | 85 |
| 92 | +++ | 95 | + | 64 |
| 93 | ++++ | 99 | +++ | 93 |
| 94 | + | 83 | + | 45 |
| 95 | ++++ | 99 | +++ | 93 |
| 96 | + | 84 | + | 20 |
| 97 | +++ | 93 | + | 47 |
| 98 | ++++ | 97 | + | 81 |
| 99 | + | 63 | + | 22 |
| 100 | ++ | 83 | + | 25 |
| 101 | ++++ | 98 | ++ | 78 |
| 102 | +++ | 93 | + | 56 |
| 103 | +++ | 94 | + | 71 |
| 104 | ++ | 84 | + | 33 |
| 105 | ++++ | 98 | ++ | 87 |
| 106 | + | 80 | + | 32 |
| 107 | + | 71 | + | 16 |
| 108 | ++++ | 99 | ++ | 91 |
| 109 | + | 75 | + | 11 |
| 110 | ++++ | 99 | ++ | 86 |
| 111 | ++++ | 101 | +++ | 93 |
| 112 | +++ | 94 | + | 55 |
| 113 | + | 79 | + | 32 |
| 114 | ++ | 86 | + | 42 |
| 115 | + | 84 | + | 19 |
| 116 | + | 76 | + | 22 |
| 117 | **+** | 74 | + | 27 |
| 118 | **+** | 89 | + | 40 |
| 119 | + | 81 | + | 18 |
| 120 | + | 8 | + | 0 |
| 121 | + | 81 | + | 44 |
| 122 | +++ | 94 | + | 62 |
| 123 | +++ | 96 | + | 74 |
| 124 | ++++ | 97 | + | 98 |
| 125 | +++ | 98 | ++ | 86 |
| 126 | +++ | 95 | + | 51 |
| 127 | ++++ | 96 | ++ | 74 |
| 128 | +++ | 82 | + | 39 |
| 129 | ++++ | 100 | ++++ | 98 |
| 130 | ++++ | 97 | +++ | 90 |
| 131 | +++ | 83 | + | 25 |
| 132 | +++ | 97 | ++ | 71 |
| 133 | ++++ | 102 | ++++ | 98 |
| 134 | ++++ | 101 | ++++ | 97 |
| 135 | ++++ | 103 | ++++ | 98 |
| 136 | +++ | 83 | + | 25 |
| 137 | +++ | 88 | + | 39 |
| 138 | ++++ | 101 | +++ | 94 |
| 139 | ++++ | 100 | +++ | 94 |
| 140 | +++ | 94 | ++ | 48 |
| 141 | ++++ | 100 | +++ | 96 |
| 142 | ++++ | 98 | +++ | 88 |
| 143 | ++++ | 103 | +++ | 89 |
| 144 | ++++ | 102 | +++ | 95 |
| 145 | +++ | 94 | ++ | 65 |
| 146 | +++ | 98 | +++ | 80 |
| 147 | ++++ | 101 | +++ | 90 |
| 148 | +++ | 90 | + | 32 |
| 149 | +++ | 95 | ++ | 67 |
| 150 | ++++ | 99 | ++++ | 97 |
| 151 | +++ | 93 | ++ | 56 |
| 152 | +++ | 96 | ++ | 85 |
| 153 | +++ | 87 | + | 37 |
| 154 | ++++ | 101 | +++ | 95 |
| 155 | +++ | 96 | ++ | 67 |
| 156 | ++++ | 99 | +++ | 97 |
| 157 | ++++ | 99 | +++ | 96 |
| 158 | +++ | 96 | ++ | 75 |
| 159 | ++++ | 99 | +++ | 95 |
| 160 | +++ | 93 | ++ | 62 |
| 161 | +++ | 94 | ++ | 53 |
| 162 | +++ | 91 | ++ | 59 |
| 163 | ++++ | 98 | +++ | 87 |
| 164 | +++ | 92 | ++ | 68 |
| 165 | ++++ | 97 | +++ | 80 |
| 166 | +++ | 91 | + | 40 |
| 167 | +++ | 93 | ++ | 69 |
| 168 | ++++ | 99 | +++ | 93 |
| 169 | ++++ | 98 | +++ | 91 |
| 170 | +++ | 96 | ++ | 80 |
| 171 | ++++ | 98 | +++ | 92 |
| 172 | ++++ | 99 | ++++ | 97 |
| 173 | ++++ | 98 | +++ | 94 |
| 174 | ++++ | 98 | +++ | 95 |
| 175 | ++++ | 97 | +++ | 86 |
| 176 | ++++ | 98 | ++ | 83 |
| 177 | +++ | 94 | ++ | 66 |
| 178 | ++++ | 99 | +++ | 96 |
| 179 | ++++ | 99 | +++ | 96 |
| 180 | ++++ | 100 | +++ | 99 |
| 181 | ++++ | 100 | +++ | 97 |
| 182 | ++++ | 99 | +++ | 95 |
| 183 | ++++ | 98 | +++ | 82 |
| 184 | +++ | 94 | ++ | 67 |
| 185 | ++++ | 99 | +++ | 87 |
| 186 | ++++ | 97 | ++ | 66 |
| 187 | +++ | 88 | + | 42 |
| 188 | +++ | 93 | + | 41 |
| 189 | ++++ | 99 | +++ | 93 |
| 190 | ++++ | 98 | +++ | 89 |
| 191 | ++++ | 97 | ++ | 76 |
| 192 | +++ | 94 | ++ | 60 |
| 193 | ++++ | 99 | ++ | 86 |
| 194 | +++ | 93 | ++ | 60 |
| 195 | +++ | 84 | + | 47 |
| 196 | +++ | 86 | + | 59 |
| 197 | +++ | 89 | + | 59 |
| 198 | +++ | 94 | ++ | 76 |
| 199 | +++ | 85 | + | 14 |
| 200 | +++ | 97 | ++ | 71 |
| 201 | ++++ | 99 | ++ | 76 |
| 202 | ++++ | 94 | ++ | 67 |
| 203 | +++ | 84 | + | 26 |
| 204 | ++++ | 97 | ++ | 82 |
| 205 | +++ | 91 | ++ | 52 |
| 206 | ++++ | 99 | +++ | 85 |
| 207 | ++++ | 98 | +++ | 89 |
| 208 | ++++ | 98 | +++ | 92 |
| 209 | +++ | 89 | ++ | 51 |
| 210 | ++++ | 99 | +++ | 96 |
| 211 | +++ | 97 | ++ | 82 |
| 212 | +++ | 89 | + | 39 |
| 213 | +++ | 97 | ++ | 63 |
| 214 | ++++ | 99 | +++ | 93 |
| 215 | ++++ | 97 | +++ | 90 |
| 216 | ++++ | 98 | +++ | 90 |
| 217 | ++++ | 98 | ++ | 79 |
| 218 | ++++ | 99 | ++ | 79 |
| 219 | +++ | 94 | ++ | 57 |
| 220 | +++ | 92 | + | 47 |
| 221 | ++++ | 99 | +++ | 88 |
| 222 | ++++ | 98 | ++ | 83 |
| 223 | ++++ | 101 | ++++ | 101 |
| 224 | ++++ | 101 | ++++ | 100 |
| 225 | +++ | 92 | ++ | 72 |
| 226 | +++ | 98 | +++ | 85 |
| 227 | ++++ | 99 | +++ | 94 |
| 228 | ++++ | 100 | +++ | 98 |
| 229 | ++++ | 100 | +++ | 90 |
| 230 | +++ | 97 | ++ | 62 |
| 231 | +++ | 91 | + | 46 |
| 232 | ++++ | 98 | +++ | 85 |
| 233 | +++ | 98 | ++ | 74 |
| 234 | **+++** | 96 | ++ | 49 |
| 235 | ++++ | 99 | +++ | 72 |
| 236 | ++++ | 100 | +++ | 90 |
| 237 | ++++ | 100 | +++ | 92 |
| 238 | ++++ | 100 | ++++ | 96 |
| 239 | ++++ | 101 | +++ | 93 |
| 240 | ++++ | 101 | +++ | 89 |
| 241 | +++ | 97 | ++ | 63 |
| 242 | +++ | 94 | ++ | 51 |
| 243 | +++ | 81 | + | 9 |
| 244 | +++ | 88 | + | 25 |
| 245 | +++ | 96 | ++ | 75 |
| 246 | +++ | 88 | + | 23 |
| 247 | +++ | 93 | + | 65 |
| 248 | ++++ | 98 | +++ | 100 |
| 249 | +++ | 97 | ++ | 71 |
| 250 | +++ | 87 | + | 25 |
| 251 | ++++ | 100 | +++ | 91 |
| 252 | +++ | 87 | + | 33 |
| 253 | +++ | 92 | ++ | 57 |
| 254 | ++++ | 99 | ++ | 77 |
| 255 | ++++ | 100 | +++ | 90 |
| 256 | ++++ | 98 | ++ | 76 |
| 257 | +++ | 92 | ++ | 65 |
| 258 | +++ | 96 | +++ | 75 |
| 259 | ++++ | 99 | +++ | 88 |
| 260 | +++ | 83 | + | 42 |
| 261 | +++ | 94 | ++ | 61 |
| 262 | ++++ | 102 | ++++ | 99 |
| 263 | ++++ | 101 | +++ | 98 |
| 264 | +++ | 98 | ++ | 72 |
| 265 | ++++ | 104 | ++++ | 99 |
| 266 | ++++ | 103 | +++ | 96 |
| 267 | ++++ | 103 | +++ | 88 |
| 268 | +++ | 97 | ++ | 67 |
| 269 | ++++ | 104 | +++ | 97 |
| 270 | ++++ | 104 | ++++ | 98 |
| 271 | ++++ | 104 | +++ | 95 |
| 272 | ++++ | 104 | +++ | 95 |
| 273 | +++ | **98** | ++ | 64 |
| 274 | **+++** | 93 | ++ | 45 |
| 275 | ++++ | 96 | ++ | 77 |
| 276 | ++++ | 98 | +++ | 95 |
| 277 | +++ | 96 | ++ | 69 |
| 278 | ++++ | 101 | +++ | 96 |
| 279 | +++ | 83 | ++ | 67 |
| 280 | +++ | 96 | +++ | 82 |
| 281 | +++ | 92 | + | 48 |
| 282 | +++ | 87 | + | 45 |
| 283 | +++ | 96 | ++ | 63 |
| 284 | ++++ | 99 | +++ | 93 |
| 285 | ++++ | 98 | +++ | 92 |
| 286 | +++ | 85 | + | 43 |
| 287 | ++++ | 99 | ++ | 99 |
| 288 | ++++ | 101 | +++ | 98 |
| 289 | +++ | 90 | + | 41 |
| 290 | +++ | 94 | ++ | 53 |
| 291 | ++++ | 100 | ++++ | 97 |
| 292 | ++++ | 99 | +++ | 94 |
| 293 | +++ | 88 | + | 33 |
| 294 | ++++ | 100 | +++ | 86 |
| 295 | ++++ | 100 | +++ | 93 |
| 296 | ++++ | 100 | +++ | 88 |
| 297 | +++ | 96 | ++ | 66 |
| 298 | ++++ | 99 | +++ | 82 |
| 299 | ++++ | 99 | ++ | 78 |
| 300 | +++ | 99 | ++ | 63 |
| 301 | ++++ | 96 | +++ | 86 |
| 302 | +++ | 93 | ++ | 58 |
| 303 | +++ | 96 | +++ | 87 |
| 304 | +++ | 86 | + | 38 |
| 305 | +++ | 87 | + | 40 |
| 306 | ++++ | 99 | +++ | 94 |
| 307 | ++++ | 98 | +++ | 97 |
| 308 | ++++ | 99 | ++++ | 96 |
| 309 | ++++ | 97 | +++ | 82 |
| 310 | ++++ | 97 | +++ | 84 |
| 311 | ++++ | 99 | +++ | 95 |
| 312 | +++ | 83 | + | 46 |
| 313 | +++ | 92 | ++ | 72 |
| 314 | ++++ | 96 | +++ | 83 |
| 315 | +++ | 81 | + | 33 |
| 316 | +++ | 98 | + | 92 |
| 317 | +++ | 100 | + | 96 |
| 318 | +++ | 107 | ++ | 55 |
| 319 | ++++ | 104 | +++ | 92 |
| 320 | +++ | 107 | ++ | 80 |
| 321 | ++++ | 105 | +++ | 95 |
| 322 | ++++ | 106 | ++++ | 97 |
| 323 | +++ | 108 | ++ | 76 |
| 324 | +++ | 105 | ++ | 81 |
| 325 | ++++ | 101 | +++ | 90 |
| 326 | +++ | 97 | ++ | 70 |
| 327 | +++ | 89 | + | 48 |
| 328 | ++++ | 99 | ++ | 81 |
| 329 | ++++ | 100 | +++ | 86 |
| 330 | +++ | 93 | ++ | 64 |
| 331 | +++ | 97 | ++ | 73 |
| 332 | +++ | 96 | ++ | 67 |
| 333 | ++++ | 100 | ++ | 79 |
| 334 | +++ | 96 | ++ | 61 |
| 335 | ++++ | 101 | +++ | 84 |
| 336 | ++++ | 99 | +++ | 92 |
| 337 | +++ | 99 | ++ | 63 |
| 338 | +++ | 97 | ++ | 56 |
| 339 | ++++ | 100 | +++ | 95 |
| 340 | ++++ | 99 | +++ | 91 |
| 341 | ++++ | 100 | +++ | 93 |
| 342 | ++++ | 97 | ++ | 80 |
| 343 | ++++ | 101 | ++ | 78 |
| 344 | +++ | 94 | ++ | 57 |
| 345 | +++ | 81 | + | 25 |
| 346 | ++++ | 102 | ++++ | 94 |
| 347 | ++++ | 100 | +++ | 79 |
| 348 | ++++ | 99 | ++ | 72 |
| 349 | ++++ | 100 | +++ | 87 |
| 350 | +++ | 96 | + | 54 |
| 351 | ++++ | 101 | +++ | 95 |
| 352 | ++++ | 99 | +++ | 89 |
| 353 | ++++ | 93 | +++ | 82 |
| 354 | +++ | 79 | + | 39 |
| 355 | +++ | 93 | ++ | 74 |
| 356 | ++++ | 97 | +++ | 95 |
| 357 | ++++ | 97 | +++ | 87 |
| 358 | +++ | 92 | ++ | 67 |
| 359 | +++ | 89 | + | 39 |
| 360 | ++++ | 99 | + | 99 |
| 361 | ++++ | 98 | + | 98 |
| 362 | ++++ | 99 | + | 98 |
| 363 | ++++ | 98 | +++ | 94 |
| 364 | +++ | 90 | ++ | 62 |
| 365 | ++++ | 99 | +++ | 98 |
| 366 | ++++ | 99 | ++++ | 99 |
| 367 | ++++ | 102 | +++ | 93 |
| 368 | ++++ | 103 | +++ | 96 |
| 369 | ++++ | 104 | ++++ | 95 |
| 370 | ++++ | 103 | +++ | 96 |
| 371 | ++++ | 104 | ++++ | 97 |
| 372 | +++ | 93 | + | 47 |
| 373 | ++++ | 103 | ++++ | 100 |
| 374 | ++++ | 101 | +++ | 94 |
| 375 | ++++ | 100 | ++++ | 101 |
| 376 | +++ | 98 | ++ | 79 |
| 377 | ++++ | 98 | ++ | 89 |
| 378 | ++++ | 100 | +++ | 99 |
| 379 | ++++ | 100 | +++ | 94 |
| 380 | ++++ | 101 | ++++ | 98 |
| 381 | ++++ | 101 | ++++ | 98 |
| 382 | +++ | 98 | ++ | 77 |
| 383 | ++++ | 98 | ++ | 79 |
| 384 | ++++ | 101 | ++++ | 100 |
| 385 | ++++ | 98 | +++ | 91 |
| 386 | ++++ | 98 | +++ | 94 |
| 387 | +++ | 97 | ++ | 73 |
| 388 | +++ | 97 | ++ | 71 |
| 389 | +++ | 93 | ++ | 56 |
| 390 | ++++ | 100 | ++++ | 97 |
| 391 | ++++ | 96 | ++ | 81 |
| 392 | ++++ | 100 | +++ | 95 |
| 393 | ++++ | 99 | ++++ | 96 |
| 394 | ++++ | 101 | +++ | 93 |
| 395 | +++ | 92 | ++ | 62 |
| 396 | +++ | 74 | + | 32 |
| 397 | +++ | 93 | ++ | 66 |
| 398 | ++++ | 98 | +++ | 91 |
| 399 | ++++ | 100 | +++ | 98 |
| 400 | +++ | 96 | ++ | 68 |
| 401 | ++++ | 97 | +++ | 91 |
| 402 | ++++ | 96 | ++ | 74 |
| 403 | ++++ | 99 | +++ | 91 |
| 404 | +++ | 93 | +++ | 78 |
| 405 | +++ | 85 | ++ | 55 |
| 406 | ++++ | 100 | +++ | 93 |
| 407 | ++++ | 99 | +++ | 97 |
| 408 | +++ | 91 | ++ | 65 |
| 409 | ++++ | 96 | +++ | 95 |
| 410 | +++ | 93 | ++ | 75 |
| 411 | +++ | 91 | ++ | 50 |
| 412 | ++++ | 99 | +++ | 98 |
| 413 | +++ | 92 | ++ | 51 |
| 414 | +++ | 91 | ++ | 67 |
| 415 | ++++ | 99 | ++++ | 95 |
| 416 | ++++ | 99 | +++ | 95 |
| 417 | ++++ | 97 | +++ | 91 |
| 418 | +++ | 90 | + | 51 |
| 419 | +++ | 93 | ++ | 64 |
| 420 | ++++ | 98 | +++ | 89 |
| 421 | +++ | 84 | + | 37 |
| 422 | ++++ | 99 | ++++ | 97 |
| 423 | ++++ | 98 | +++ | 89 |
| 424 | ++++ | 99 | ++++ | 96 |
| 425 | ++++ | 98 | +++ | 93 |
| 426 | +++ | 97 | ++ | 78 |
| 427 | ++++ | 99 | +++ | 87 |
| 428 | +++ | 97 | ++ | 65 |
| 429 | ++++ | 99 | +++ | 93 |
| 430 | ++++ | 100 | +++ | 92 |
| 431 | +++ | 86 | ++ | 53 |
| 432 | ++++ | 98 | +++ | 88 |
| 433 | +++ | 96 | ++ | 59 |
| 434 | ++++ | 100 | ++++ | 92 |
| 435 | ++++ | 100 | +++ | 93 |
| 436 | ++++ | 100 | +++ | 85 |
| 437 | ++++ | 99 | +++ | 87 |
| 438 | +++ | 84 | + | 28 |
| 439 | +++ | 88 | + | 36 |
| 440 | +++ | 88 | + | 40 |
| 441 | +++ | 97 | ++ | 80 |
| 442 | ++++ | 96 | ++ | 75 |
| 443 | +++ | 96 | +++ | 82 |
| 444 | +++ | 93 | ++ | 67 |
| 445 | +++ | 96 | ++ | 72 |
| 446 | +++ | 87 | + | 44 |
| 447 | +++ | 95 | ++ | 84 |
| 448 | ++++ | 97 | +++ | 93 |
| 449 | +++ | 92 | + | 50 |
| 450 | +++ | 96 | ++ | 73 |
| 451 | +++ | 93 | ++ | 58 |
| 452 | ++++ | 97 | +++ | 98 |
| 453 | +++ | 91 | ++ | 61 |
| 454 | ++++ | 97 | +++ | 88 |
| 455 | +++ | 94 | ++ | 75 |
| 456 | +++ | 95 | ++ | 80 |
| 457 | +++ | 95 | ++ | 61 |
| 458 | +++ | 95 | ++ | 74 |
| 459 | ++++ | 97 | +++ | 86 |
| 460 | +++ | 98 | ++ | 75 |
| 461 | +++ | 93 | ++ | 65 |
| 462 | +++ | 93 | ++ | 71 |
| 463 | ++++ | 98 | ++ | 82 |
| 464 | +++ | 88 | + | 48 |
| 465 | ++++ | 99 | +++ | 89 |
| 466 | ++++ | 99 | ++ | 79 |
| 467 | ++++ | 99 | +++ | 86 |
| 468 | ++++ | 100 | ++ | 80 |
| 469 | ++++ | 98 | +++ | 85 |
| 470 | +++ | 97 | ++ | 82 |
| 471 | +++ | 96 | + | 63 |
| 472 | +++ | 86 | + | 51 |
| 473 | +++ | 96 | + | 72 |
| 474 | +++ | 93 | + | 53 |
| 475 | ++++ | 99 | +++ | 98 |
| 476 | +++ | 94 | ++ | 63 |
| 477 | +++ | 88 | + | 49 |
| 478 | +++ | 95 | ++ | 63 |
| 479 | +++ | 96 | ++ | 72 |
| 480 | +++ | 93 | ++ | 73 |
| 481 | ++++ | 98 | +++ | 95 |
| 482 | +++ | 90 | ++ | 55 |
| 483 | +++ | 93 | ++ | 72 |
| 484 | +++ | 96 | ++ | 77 |
| 485 | +++ | 93 | ++ | 82 |
| 486 | +++ | 95 | + | 70 |
| 487 | ++++ | 100 | +++ | 95 |
| 488 | ++++ | 101 | +++ | 91 |
| 489 | +++ | 97 | ++ | 76 |
| 490 | +++ | 93 | + | 40 |
| 491 | +++ | 98 | ++ | 76 |
| 492 | ++++ | 99 | +++ | 91 |
| 493 | +++ | 96 | ++ | 68 |
| 494 | ++++ | 99 | +++ | 90 |
| 495 | ++++ | 99 | +++ | 88 |
| 496 | ++++ | 100 | ++++ | 92 |
| 497 | ++++ | 100 | ++++ | 97 |
| 498 | +++ | 98 | +++ | 83 |
| 499 | +++ | 85 | + | 29 |
| 500 | ++++ | 95 | ++ | 83 |
| 501 | +++ | 85 | + | 47 |
| 502 | +++ | 89 | ++ | 70 |
| 503 | +++ | 94 | + | 72 |
| 504 | +++ | 84 | + | 44 |
| 505 | +++ | 96 | ++ | 72 |
| 506 | ++++ | 97 | +++ | 96 |
| 507 | ++++ | 97 | +++ | 77 |
| 508 | +++ | 90 | + | 46 |
| 509 | +++ | 93 | ++ | 54 |
| 510 | +++ | 89 | + | 64 |
| 511 | ++++ | 96 | ++ | 79 |
| 512 | ++++ | 97 | ++ | 80 |
| 513 | +++ | 87 | + | 36 |
| 514 | +++ | 95 | + | 59 |
| 515 | +++ | 90 | ++ | 68 |
| 516 | +++ | 96 | ++ | 77 |
| 517 | ++++ | 98 | +++ | 88 |
| 518 | +++ | 96 | ++ | 72 |
| 519 | ++++ | 96 | +++ | 90 |
| 520 | +++ | 88 | ++ | 53 |
| 521 | +++ | 85 | + | 34 |
| 522 | +++ | 97 | ++ | 83 |
| 523 | +++ | 90 | ++ | 58 |
| 524 | +++ | 95 | + | 50 |
| 525 | +++ | 97 | ++ | 69 |
| | | | | |

| | | | | |
|---|---|---|---|---|
| "+" indicates inhibitory effect of > 1 µM; "++" indicates inhibitory effect of 0.5-1 µM; "+++" indicates inhibitory effect of 0.1-0.5 µM, "++++" indicates inhibitory effect of < 0.1 µM | | | | |

### Example 39. Synthesis of Compound A

BRG1/BRM Inhibitor compound A has the structure:

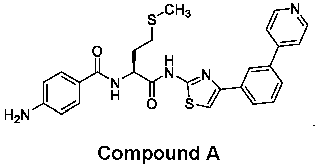

Compound A was synthesized as shown in Scheme 1 below.

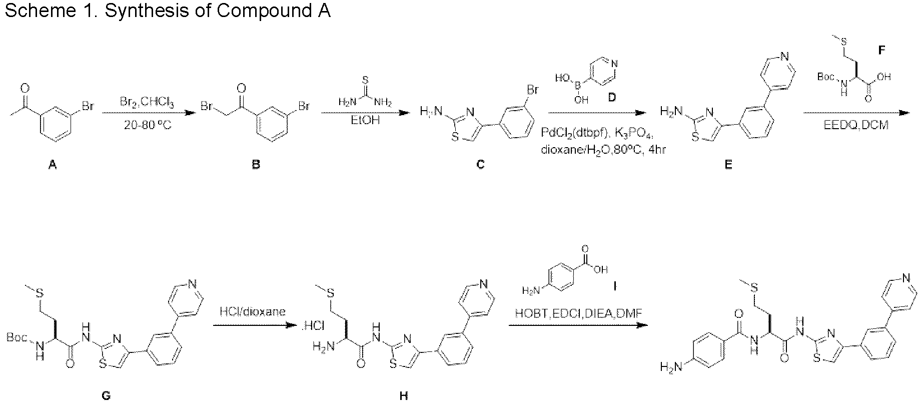

The ATPase catalytic activity of BRM or BRG-1 in the presence of Compound A was measured by the in vitro biochemical assay using ADP-Glo^{™} (Promega, V9102) described above. Compound A was found to have an IC₅₀ of 10.4 nM against BRM and 19.3 nM against BRG1 in the assay.

### Example 40. Effects of BRG1/BRM ATPase Inhibition on the Growth of Uveal Melanoma and Hematological Cancer Cell Lines

Procedure: Uveal melanoma cell lines (92-1, MP41, MP38, MP46), prostate cancer cell lines (LNCAP), lung cancer cell lines (NCI-H1299), and immortalized embryonic kidney lines (HEK293T) were plated into 96 well plates with growth media (see Table 5). BRG1/BRM ATPase inhibitor, Compound A, was dissolved in DMSO and added to the cells in a concentration gradient from 0 to 10 micromolar at the time of plating. Cells were incubated at 37 degrees Celsius for 3 days. After three days of treatment, the media was removed from the cells and 30 microliters of TrypLE (Gibco) was added to cells for 10 minutes. Cells were detached from the plates and resuspended with the addition of 170 microliters of growth media. Cells from two DMSO-treated control wells were counted, and the initial number of cells plated at the start of the experiment, were re-plated into fresh-compound containing plates for an additional four days at 37 degrees Celsius. At day 7, cells were harvested as described above. On day 3 and day 7, relative cell growth was measured by the addition of Cell-titer glo (Promega) and luminescence was measured on an Envision plate reader (Perkin Elmer). The concentration of compound at which each cell line's growth was inhibited by 50% (GI₅₀), was calculated using Graphpad Prism, and is plotted below. For multiple myeloma cell lines (OPM2, MM1S, LP1), ALL cell lines (TALL1, JURKAT, RS411), DLBCL cell lines (SUDHL6, SUDHL4, DB, WSUDLCL2, PFEIFFER), AML cell lines (OCIAML5), MDS cell lines (SKM1), ovarian cancer cell lines (OV7, TYKNU), esophageal cancer cell lines (KYSE150), rhabdoid tumor lines (RD, G402, G401, HS729, A204), liver cancer cell lines (HLF, HLE, PLCRPF5), and lung cancer cell lines (SW1573, NCIH2444), the above methods were performed with the following modifications: Cells were plated in 96 well plates, and the next day, BRG1/BRM ATPase inhibitor, Compound A, was dissolved in DMSO and added to the cells in a concentration gradient from 0 to 10 micromolar. At the time of cell splitting on days 3 and 7, cells were split into new 96 well plates, and fresh compound was added four hours after re-plating.

Table 26 lists the tested cell lines and growth media used.

**Table 26. Cell Lines and Growth Media**

| **Cell Line** | **Source** | **Growth Media** |
|---|---|---|
| 92-1 | SIGMA | RPMI1640+ 20% FBS |
| A204 | ATCC | McCoy's 5A + 10% FBS |
| DB | ATCC | RPMI1640+ 10% FBS |
| G401 | ATCC | McCoy's 5A +10% FBS |
| G402 | ATCC | McCoy's 5A +10% FBS |
| HEK293T | ATCC | DMEM + 10% FBS |
| HLE | JCRB | DMEM + 10% FBS |
| HLF | JCRB | DMEM + 10% FBS |
| HS729 | ATCC | DMEM + 10% FBS |
| JURKAT | ATCC | RPMI1640+ 10% FBS |
| KYSE150 | DSMZ | RPMI1640/Ham's F12 + 10% FBS |
| LNCAP | ATCC | RPMI1640+ 10% FBS |
| LP1 | DSMZ | IMDM + 20% FBS |
| MM1S | ATCC | RPMI1640+ 10% FBS |
| MP38 | ATCC | RPMI1640 + 20% FBS |
| MP41 | ATCC | RPMI1640 + 20% FBS |
| MP46 | ATCC | RPMI1640 + 20% FBS |
| NCIH1299 | ATCC | RPMI1640+ 10% FBS |
| NCIH2444 | ATCC | RPMI1640 + 20% FBS |
| OCIAML5 | DSMZ | alpha-MEM + 20% FBS +10ng/ml GM-CSF |
| OPM2 | DSMZ | RPMI1640+ 10% FBS |
| OV7 | ECACC | DMEM/Ham's F12 (1:1) + 2mM Glutamine + 10% FBS + 0.5 ug/ml hydrocortisone + 10ug/ml insulin |
| PFEIFFER | ATCC | RPMI1640+ 10% FBS |
| PLCPRF5 | ATCC | EMEM + 10% FBS |
| RD | ATCC | DMEM + 10% FBS |
| RS411 | ATCC | RPMI1640 + 10% FBS |
| SKM1 | JCRB | RPMI1640+ 10% FBS |
| SUDHL4 | DSMZ | RPMI1640+ 10% FBS |
| SUDHL6 | ATCC | RPMI1640 + 20% FBS |
| SW1573 | ATCC | DMEM + 10% FBS |
| TALL1 | JCRB | RPMI1640+ 10% FBS |
| TYKNU | JCRB | EMEM + 20% FBS |
| WSUDLCL2 | DSMZ | RPMI1640+ 10% FBS |

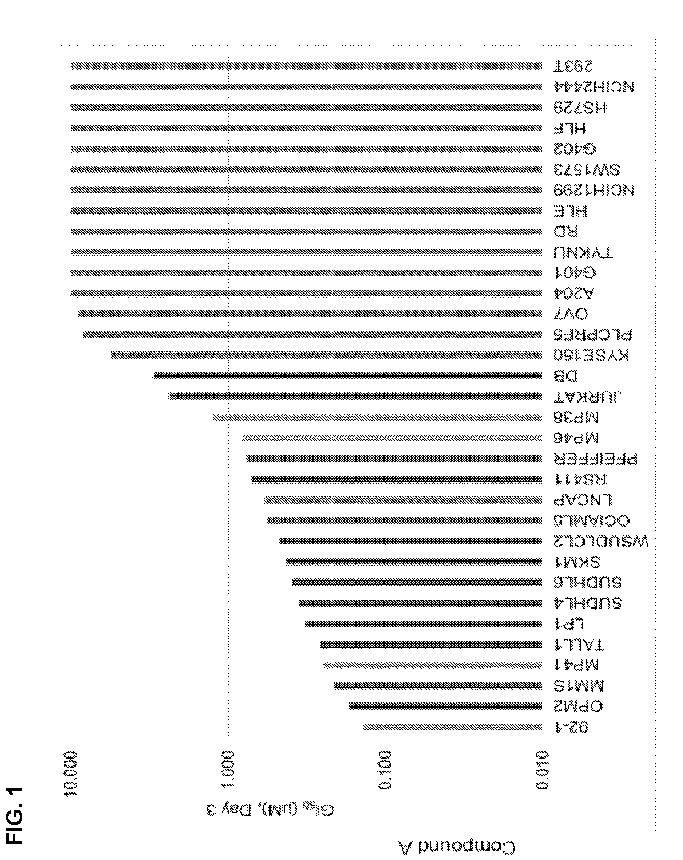

Results: As shown in FIG. 1, the uveal melanoma and hematologic cancer cell lines were more sensitive to BRG1/BRM inhibition than the other tested cell lines. Inhibition of the uveal melanoma and hematologic cancer cell lines was maintained through day 7.

### Example 41. Comparison of BRG1/BRM Inhibitors to clinical PKC and MEK inhibitors in uveal melanoma cell lines

Procedure: Uveal melanoma cell lines, 92-1 or MP41, were plated in 96 well plates in the presence of growth media (see Table 26). BAF ATPase inhibitors (Compound A), PKC inhibitor (LXS196; MedChemExpress), or MEK inhibitor (Selumetinib; Selleck Chemicals) were dissolved in DMSO and added to the cells in a concentration gradient from 0 to 10 micromolar at the time of plating. Cells were incubated at 37 degrees Celsius for 3 days. After three days of treatment, cell growth was measured with Cell-titer glow (Promega), and luminescence was read on an Envision plate reader (Perkin Elmer).

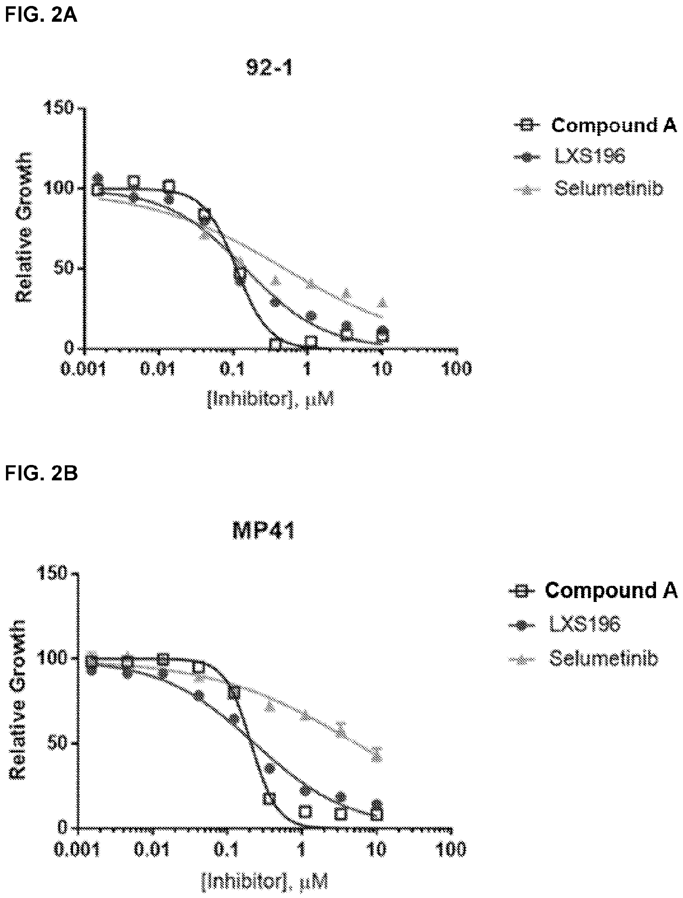

Results: As shown in FIG. 2A and FIG. 2B, Compound A showed comparable growth inhibition of uveal melanoma cells as the clinical PKC and MEK inhibitors. Further, compound A was found to result in a faster onset of inhibition than the clinical PKC and MEK inhibitors.

### Example 42. Synthesis of Compound B

BRG1/BRM Inhibitor Compound B has the structure:

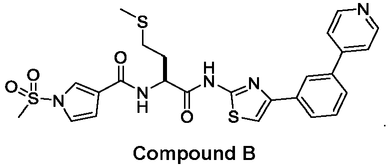

Compound B was synthesized as shown in Scheme 2 below.
Scheme 2. Synthesis of Compound B

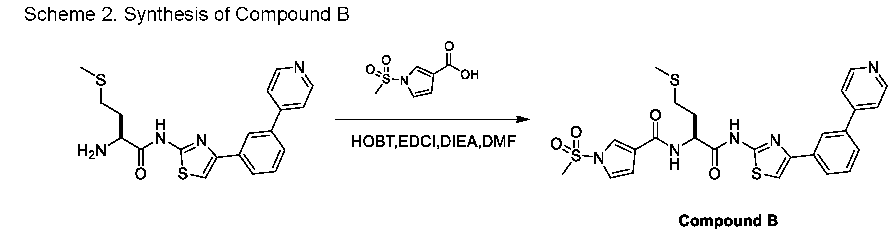

### Step 1: Preparation of (S)-1-(methylsulfonyl)-N-(4-(methylthio)-1-oxo-1-((4-(3-(pyridin-4-yl)phenyl)thiazol-2-yl)amino)butan-2-yl)-1H-pyrrole-3-carboxamide (Compound B)

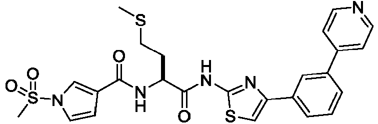

To a mixture of (2S)-2-amino-4-methylsulfanyl-N-[4-[3-(4-pyridyl)phenyl]thiazol-2-yl]butanamide (2 g, 4.75 mmol, HCl salt) and 1-methylsulfonylpyrrole-3-carboxylic acid (898.81 mg, 4.75 mmol) in DMF (20 mL) was added EDCI (1.37 g, 7.13 mmol), HOBt (962.92 mg, 7.13 mmol), and DIEA (2.46 g, 19.00 mmol, 3.31 mL) and the mixture was stirred at 25 °C for 3 hours. The mixture was poured into H₂O (100 mL) and the precipitate was collected by filtration. The solid was triturated in MeOH (20 mL) and the precipitate was collected by filtration. The solid was dissolved in DMSO (10 mL) and then the mixture was poured into MeOH (50 mL) and the formed precipitate was collected by filtration and lyophilized to give Compound B (2.05 g, 3.66 mmol, 77.01% yield) as a white solid. LCMS (ESI) m/z [M+H]⁺=555.9. ¹H NMR (400 MHz, DMSO) δ 12.49 (s, 1H), 8.68-8.66 (m, 2H), 8.46 (d, J=7.2 Hz, 1H), 8.31-8.30 (m, 1H), 8.02-8.00 (m, 1H), 7.94-7.96 (m, 1H), 7.83 (s, 1H), 7.73-7.74 (m, 3H), 7.61-7.57 (m, 1H), 7.31-7.29 (m, 1H), 6.79-6.77 (m, 1H), 4.74-4.69 (m, 1H), 3.57 (s, 3H), 2.67-2.53 (m, 2H), 2.13-2.01 (m, 5H). SFC: AS-3-MeOH (DEA)-40-3mL-35T.Icm, t = 0.932 min, ee%=100%.

### Example 43. Effects of BRG1/BRM ATPase inhibition on the growth of uveal melanoma, hematological cancer, prostate cancer, breast cancer, and Ewing's sarcoma cell lines

Procedure: All cell lines described above in Example 28 were also tested as described above with Compound B. In addition, the following cell lines were also tested as follows. Briefly, for Ewing's sarcoma cell lines (CADOES1, RDES, SKES1), retinoblastoma cell lines (WERIRB1), ALL cell lines (REH), AML cell lines (KASUMI1), prostate cancer cell lines (PC3, DU145, 22RV1), melanoma cell lines (SH4, SKMEL28, WM115, COLO829, SKMEL3, A375), breast cancer cell lines (MDAMB415, CAMA1, MCF7, BT474, HCC1419, DU4475, BT549), B-ALL cell lines (SUPB15), CML cell lines (K562, MEG01), Burkitt's lymphoma cell lines (RAMOS2G64C10, DAUDI), mantle cell lymphoma cell lines (JEKO1, REC1), bladder cancer cell lines (HT1197), and lung cancer cell lines (SBC5), the above methods were performed with the following modifications: Cells were plated in 96 well plates, and the next day, BRG1/BRM ATPase inhibitor, Compound B, was dissolved in DMSO and added to the cells in a concentration gradient from 0 to 10 micromolar. At the time of cell splitting on days 3 and 7, cells were split into new 96 well plates, and fresh compound was added four hours after re-plating.

Table 27 lists the tested cell lines and growth media used.

**Table 27. Cell Lines And Growth Media**

| **Cell Line** | **Source** | **Growth Media** |
|---|---|---|
| 22RV1 | ATCC | RPMI1640 + 10% FBS |
| A375 | ATCC | DMEM + 10% FBS |
| BT474 | ATCC | Hybricare medium + 1.5 g/L sodium bicarbonate + 10% FBS |
| BT549 | ATCC | RPMI1640 + 0.023 IU/ml insulin + 10% FBS |
| CADOES1 | DSMZ | RPMI1640 + 10% FBS |
| CAMA1 | ATCC | EMEM + 10% FBS |
| COLO829 | ATCC | RPMI1640 + 10% FBS |
| DAUDI | ATCC | RPMI1640 + 10% FBS |
| DU145 | ATCC | EMEM + 10% FBS |
| DU4475 | ATCC | RPMI1640 + 10% FBS |
| HCC1419 | ATCC | RPMI1640 + 10% FBS |
| HT1197 | ATCC | EMEM + 10% FBS |
| JEKO1 | ATCC | RPMI1640 + 20% FBS |
| K562 | ATCC | IMDM + 10% FBS |
| KASUMI1 | ATCC | RPMI1640 + 10% FBS |
| MCF7 | ATCC | EMEM +0.01 mg/ml bovine insulin+ 10% FBS |
| MDAMB415 | ATCC | Leibovitz's L-15 + 2mM L-glutamine + 10 mcg/ml insulin + 10 mcg/ml glutathione + 15% FBS |
| MEG01 | ATCC | RPMI1640 + 10% FBS |
| PC3 | ATCC | F-12K + 10% FBS |
| RAMOS2G64C10 | ATCC | RPMI1640 + 10% FBS |
| RDES | ATCC | RPMI1640 + 15% FBS |
| REC1 | ATCC | RPMI1640 + 10% FBS |
| REH | ATCC | RPMI1640 + 10% FBS |
| SBCS | JCRB | EMEM + 10% FBS |
| SH4 | ATCC | DMEM + 10% FBS |
| SKES1 | ATCC | McCoy's SA + 15% FBS |
| SKMEL28 | ATCC | EMEM + 10% FBS |
| SKMEL3 | ATCC | McCoy's 5A + 15% FBS |
| SUPB15 | ATCC | IMDM + 4 mM L-glutamine + 1.5 g/L sodium bicarbonate + 0.05 mM 2-mercaptoethanol + 20% FBS |
| WERIRB1 | ATCC | RPMI1640 + 10% FBS |
| WM115 | ATCC | EMEM + 10% FBS |

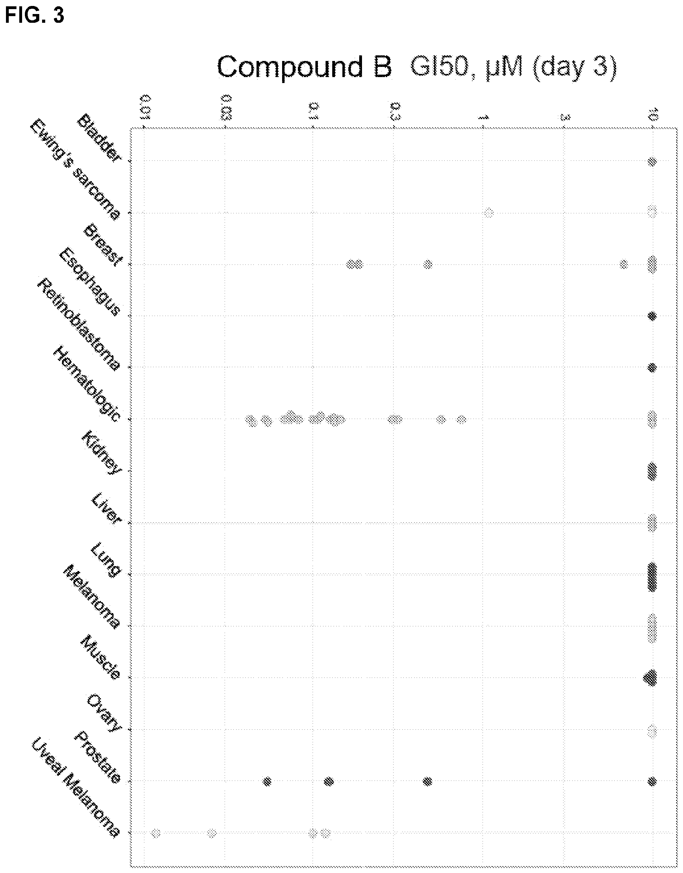

Results: As shown in FIG. 3, the uveal melanoma, hematologic cancer, prostate cancer, breast cancer, and Ewing's sarcoma cell lines were more sensitive to BRG1/BRM inhibition than the other tested cell lines. Inhibition of the uveal melanoma, hematologic cancer, prostate cancer, breast cancer, and Ewing's sarcoma cell lines was maintained through day 7.

### Example 44. Effects of BRG1/BRM ATPase inhibition on the growth of cancer cell lines.

**Procedure:** A pooled cell viability assay was performed using PRISM (Profiling Relative Inhibition Simultaneously in Mixtures) as previously described ("High-throughput identification of genotype-specific cancer vulnerabilities in mixtures of barcoded tumor cell lines", Yu et al, Nature Biotechnology 34, 419-423, 2016), with the following modifications. Cell lines were obtained from the Cancer Cell Line Encyclopedia (CCLE) collection and adapted to RPMI-1640 medium without phenol red, supplemented with 10% heat-inactivated fetal bovine serum (FBS), in order to apply a unique infection and pooling protocol to such a big compendium of cell lines. A lentiviral spin-infection protocol was executed to introduce a 24 nucleotide-barcode in each cell line, with an estimated multiplicity of infection (MOI) of 1 for all cell lines, using blasticidin as selection marker. Over 750 PRISM cancer cell lines stably barcoded were then pooled together according to doubling time in pools of 25. For the screen execution, instead of plating a pool of 25 cell lines in each well as previously described (Yu et al.), all the adherent or all the suspension cell line pools were plated together using T25 flasks (100,000 cells/flask) or 6-well plates (50,000 cells/well), respectively. Cells were treated with either DMSO or compound in a 8-point 3-fold dose response in triplicate, starting from a top concentration of 10 µM. As control for assay robustness, cells were treated in parallel with two previously validated compounds, the pan-Raf inhibitor AZ-628, and the proteasome inhibitor bortezomib, using a top concentration of 2.5 µM and 0.039 µM, respectively.

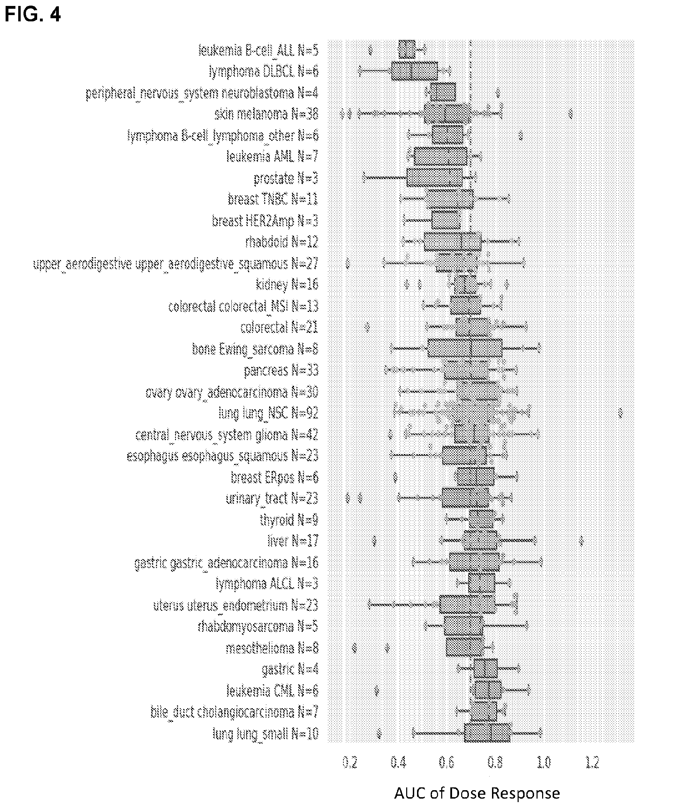

Following 3 days of treatment with compounds, cells were lysed, genomic DNA was extracted, barcodes were amplified by PCR and detected with Next-Generation Sequencing. Cell viability was determined by comparing the counts of cell-line specific barcodes in treated samples to those in the DMSO-control and Day 0 control. Dose-response curves were fit for each cell line and corresponding area under the curves (AUCs) were calculated and compared to the median AUC of all cell lines (FIG. 4). Cell lines with AUCs less than the median were considered most sensitive.

### Example 45. Effects of BRG1/BRM ATPase inhibitors on the growth of uveal melanoma cell lines.

**Procedure:** Uveal melanoma cell lines (92-1, MP41, MP38, MP46) and Non-small cell lung cancer cells (NCIH1299) were plated into 96 well plates with growth media (see Table 27). BRG1/BRM ATPase inhibitor, compound 67, was dissolved in DMSO and added to the cells in a concentration ^{g}radient from 0 to 10 micromolar at the time of plating. Cells were incubated at 37 °C for 3 days. After three days of treatment, cell growth was measured with Cell-titer glow (Promega), and luminescence was read on an Envision plate reader (Perkin Elmer).

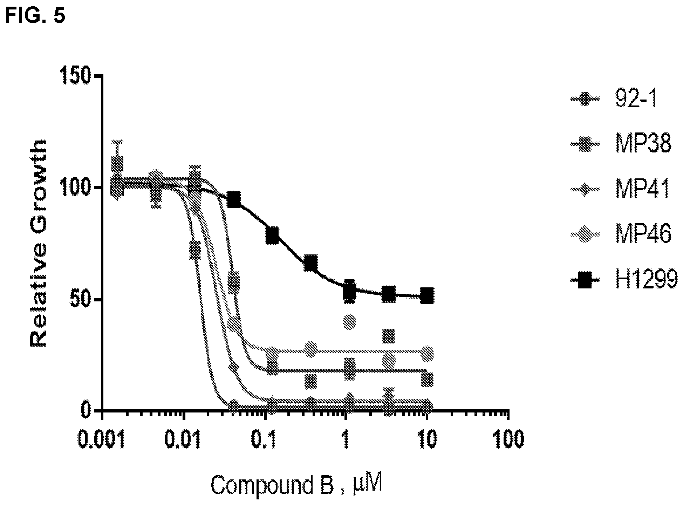

**Results:** As shown in FIG. 5, Compound B resulted in potent growth inhibition in the uveal melanoma cell lines.

### Example 46. Comparison of BRG1/BRM Inhibitors to clinical PKC and MEK inhibitors in uveal melanoma cell lines

**Procedure:** Uveal melanoma cell lines, 92-1 or MP41, were plated in 96 well plates in the presence of growth media (see Table 27). BAF ATPase inhibitor (Compound B), PKC inhibitor (LXS196; MedChemExpress), and MEK inhibitor (Selumetinib; Selleck Chemicals) were dissolved in DMSO and added to the cells in a concentration gradient from 0 to 10 micromolar at the time of plating. Cells were incubated at 37 °C for 3 days. After three days of treatment, cell growth was measured with Cell-titer glow (Promega), and luminescence was read on an Envision plate reader (Perkin Elmer).

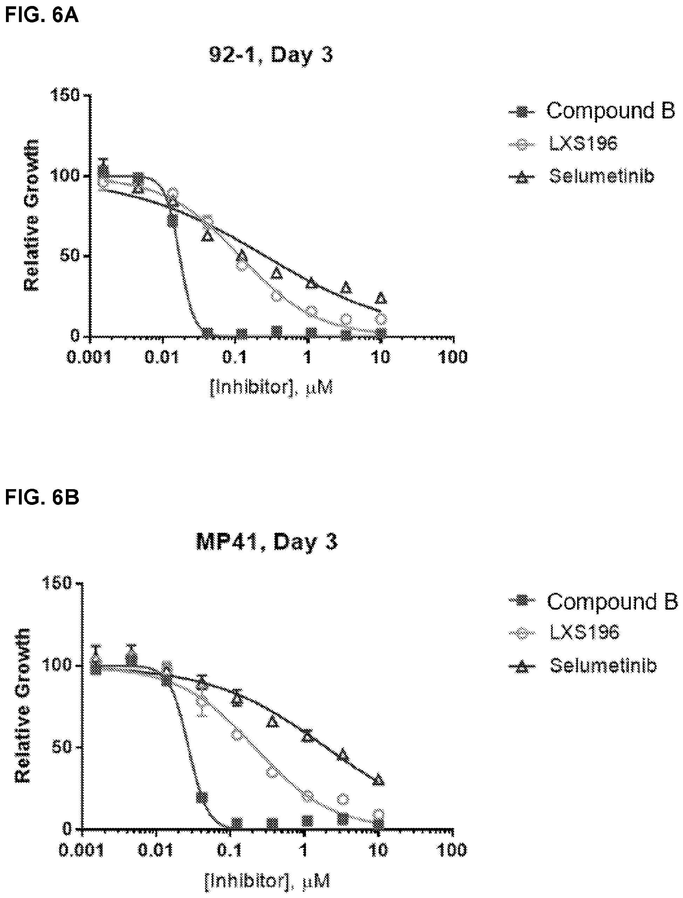

**Results:** As shown in FIG. 6A and FIG. 6B, compound B showed more potent effects on growth inhibition of uveal melanoma cells as compared to the clinical PKC and MEK inhibitors. Further, Compound B was found to result in a faster onset of growth inhibition than the clinical PKC and MEK inhibitors.

### Example 47. BRG1/BRM ATPase inhibitors are effective at inhibiting the growth of PKC inhibitor-resistant cells.

**Procedure:** MP41 uveal melanoma cells were made resistant to the PKC inhibitor (LXS196; MedChemExpress), by long-term culture in growth media (see Table 27) containing increasing concentrations of the compound, up to 1 micromolar. After 3 months, sensitivity of the parental MP41 cells and the PKC inhibitor (PKCi)-resistant cells to the PKC inhibitor (LXS196) or the BRG1/BRM ATPase inhibitor (Compound B) was tested in a 7-day growth inhibition assay as described above in Example 9.

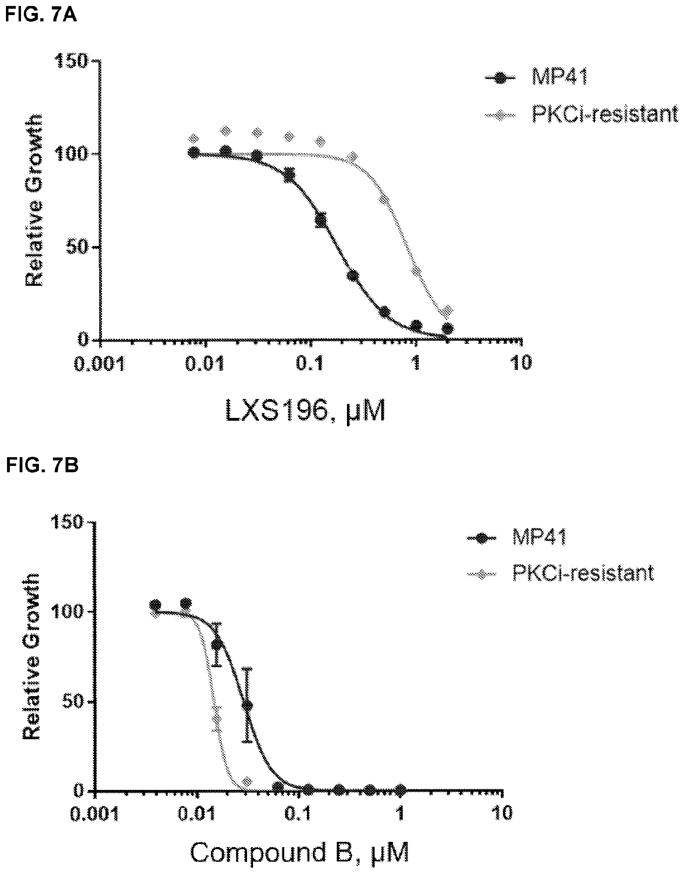

**Results:** While the PKCi-resistant cells could tolerate growth at higher concentrations of LXS196 than could the parental MP41 cell line (FIG. 7A), the BRG1/BRM ATPase inhibitor (Compound B) still resulted in strong growth inhibition of both the PKCi-resistant and parental cell lines (FIG. 7B). The PKCi-resistant cells were more sensitive to Compound B than were the parental MP41 cells (FIG. 7B).

### Example 48. Synthesis of Compound C

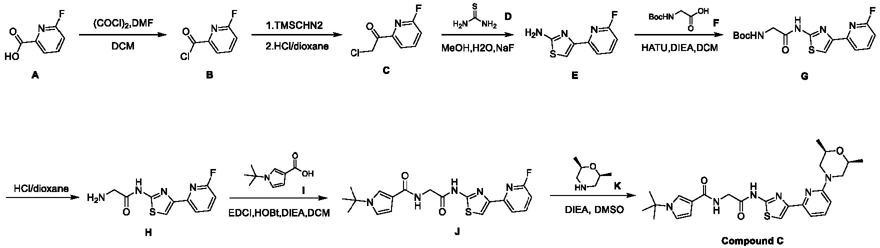

### Step 1: Preparation of 6-fluoropyridine-2-carbonyl chloride (Intermediate B)

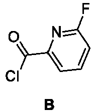

To a cooled (0 °C) solution of 6-fluoropyridine-2-carboxylic acid (50.00 g, 354.36 mmol) in dichloromethane (500 mL) and N,N-dimethylformamide (0.26 mL, 3.54 mmol) was added oxalyl chloride (155.10 mL, 1.77 mol). After complete addition of oxalyl chloride, the reaction mixture was warmed to room temperature and stirred for an additional 0.5 h. The mixture was concentrated under vacuum to give ***Intermediate** B* (56.50 g) as a white solid, which was used to next step without further purification.

### Step 2: Preparation of 2-chloro-1-(6-fluoro-2-pyridyl)ethenone (Intermediate C)

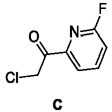

To a cooled (0 °C) mixture of ***Intermediate** B* (56.00 g, 351.00 mmol) in 1,4-dioxane (800 mL) was added in a dropwise manner a solution of 2 M trimethylsilyl diazomethane in hexanes (351 mL). The resulting reaction mixture was stirred at 25 °C for 10 h. The reaction mixture was subsequently quenched with a solution of 4 M HCl in 1,4-dioxane (500 mL). After stirring for 2 h, the reaction solution was concentrated under vacuum to give an oil. The residue was diluted with saturated aqueous NaHCO₃ (500 mL) and extracted with ethyl acetate (200 mL x 3). The combined organic layers were washed with brine (300 mL x 2), dried over Na₂SO₄, filtered, and concentrated under reduced pressure to give ***Intermediate*** C (35.50 g) as a white solid, which was used to next step directly. LCMS (ESI) m/z: [M+H]⁺ = 173.8.

### Step 3: Preparation of 4-(6-fluoro-2-pyridyl)thiazol-2-amine (Intermediate E)

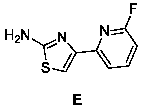

To a solution of ***Intermediate C*** (35.50 g, 204.53 mmol) and thiourea (14.01 g, 184.07 mmol) in a mixture of MeOH (250 mL) and H₂O (250 mL) at room temperature was added NaF (3.56 g, 84.82 mmol). After stirring for 0.5 h, the reaction mixture was partially concentrated under vacuum to remove MeOH, and the resulting solution was acidified to pH ~3 with aqueous 2 M HCl. After 15 min, the solution was extracted with ethyl acetate (200 mL x 3), the organic layers were discarded and the aqueous phase was alkalized with NaHCO₃ (500 mL) and stirred for 30 min, then extracted with ethyl acetate (325 mL*3), the combined organic layers were washed with brine (225 mL * 3), dried over Na₂SO₄, filtered, and concentrated under reduced pressure. The residue was triturated with petroleum ether (300 mL) and stirred at 25 °C for 10 min and filtered. The resultant solids were dried under vacuum to give ***Intermediate E*** (28.00 g, 143.43 mmol, 70.13% yield, 100% purity) as a white solid. LCMS (ESI) m/z: [M+H]⁺ = 195.8.; ¹H NMR (400 MHz, DMSO-d6) δ 8.00-7.96 (m, 1H), 7.72 (d, J=7.2 Hz, 1H), 7.24 (s, 1H), 7.16 (s, 2H), 7.02 (d, J= 8.0 Hz, 1H).

### Step 4: Preparation of tert-butyl N-[2-[[4-(6-fluoro-2-pyridyl)thiazol-2-yl]amino]-2-oxoethyl]carbamate (Intermediate G)

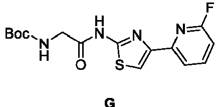

To a solution of N-Boc-glycine (5.92 g, 33.81 mmol), HATU (12.86 g, 33.81 mmol), and DIEA (15.89 g, 122.94 mmol, 21.41 mL) in dichloromethane (100 mL) was added ***Intermediate E*** (6.00 g, 30.74 mmol). After stirring for 2 h, the reaction mixture was concentrated and subsequently diluted with water (100 mL) and extracted with ethyl acetate (60 mL x 4). The combined organic layers were washed with brine (100 mL x 2), dried over Na₂SO₄, filtered, and concentrated under reduced pressure to give a residue. The residue was triturated with a 1:1 mixture of petroleum ether and MeOH (40mL). After stirring at 25 °C for 20 min, the suspension was filtered, the filter cake was washed with MTBE (20 mL) and dried in vacuo to give ***Intermediate* G** (7.7 g, 21.63 mmol, 70.4% yield, 99.0% purity) as a white solid. LCMS (ESI) m/z: [M+H]⁺ = 353.1.

### Step 5: Preparation of 2-((4-(6-fluoropyridin-2-yl)thiazol-2-yl)amino)-2-oxoethan-1-aminium chloride (Intermediate H)

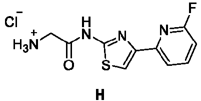

A solution of ***Intermediate* G** (5.40 g, 15.32 mmol) in 4 M HCl in 1,4-dioxane (35 mL) was stirred at 25 °C for 1.5 h. The mixture was concentrated under vacuum to give ***Intermediate H*** (4.42 g) as a white solid, which was used to next step directly without further purification. LCMS (ESI) m/z: [M+H]⁺ = 252.9.

### Step 6: Preparation of 1-tert-butyl-N-[2-[[4-(6-fluoro-2-pyridyl)thiazol-2-yl]amino]-2-oxoethyl]pyrrole-3-carboxamide (Intermediate J)

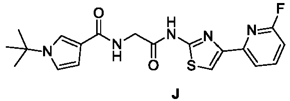

To a solution of ***Intermediate H*** (3.00 g, 10.39 mmol), 1-tert-butylpyrrole-3-carboxylic acid (1.74 g, 10.39 mmol), and DIEA (6.71 g, 51.95 mmol, 9.05 mL) in dichloromethane (40 mL) was sequentially added HOBt (1.68 g, 12.47 mmol) and EDCI (2.39 g, 12.47 mmol). After stirring for 4 h, the mixture was concentrated under vacuum. The residue was diluted with water (250 mL) and extracted with ethyl acetate (200 mL x 3). The combined organic layers were washed with brine (300 mL x 3), dried over Na₂SO₄, filtered, and concentrated under reduced pressure. The resulting solids were triturated with a 1:1 mixture of MTBE/ethyl acetate (400 mL) and after 30 min, the suspension was filtered. The solids were washed with MTBE (85 mL x 3) and then dried under vacuum to give ***Intermediate* J** (3.10 g, 7.64 mmol, 73.6% yield, 99.0% purity) as a white solid.
LCMS (ESI) m/z: [M+H]⁺ = 402.3.
¹H NMR (400 MHz, DMSO-d6) δ 12.40 (s, 1H), 8.18 - 8.15 (m, 1H), 8.09-8.08 (m, 1H), 7.87-7.83 (m, 2H), 7.52 (s, 1H), 7.11 (d, J=8.0 Hz, 1H), 6.97 (m, 1H), 6.47 (s, 1H), 4.10 (d, J=5.6 Hz, 2H), 1.49 (s, 9H).

### Step 7: Preparation of 1-(tert-butyl)-N-(2-((4-(6-(cis-2,6-dimethylmorpholino)pyridin-2-yl)thiazol-2-yl)amino)-2-oxoethyl)-1H-pyrrole-3-carboxamide (Compound C)

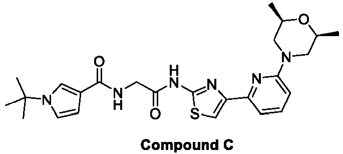

To a solution of ***Intermediate J*** (0.100 g, 0.249 mmol) in DMSO (1 mL) was added DIEA (0.130 mL, 0.747 mmol) and cis-2,6-dimethylmorpholine (0.057 g, 0.498 mmol) and the mixture was stirred at 120 °C. After 12 h, the solution was cooled to room temperature and reaction mixture was diluted with MeOH (3 mL). The residue was purified by prep-HPLC (0.1% TFA; column: Luna C18 150*25 5u; mobile phase: [water (0.075% TFA) - ACN]; B%: 30%-60%, 2min). The appropriate fractions were collected and lyophilized to give Compound C (0.079 g, 0.129 mmol, 51.94% yield, 100% purity) as a white solid. LCMS (ESI) m/z: [M+H]⁺ = 497.5.

¹H NMR (400 MHz, DMSO-d6) δ 12.27 (s, 1H), 8.17 - 8.14 (m, 1H), 7.75 (s, 1H), 7.63 - 7.59 (m, 1H), 7.51 (s, 1H),7.25 (d, J = 7.2 Hz, 1H), 6.96 (s, 1H), 6.79 (d, J = 8.8 Hz, 1H), 6.47 (s, 1H), 4.24 (d, J = 12.4 Hz, 2H), 4.08 (d, J =5.6 Hz, 2H), 3.64 - 3.61 (m, 2H), 2.44 - 2.38 (m, 2H), 1.49 (s, 9H), 1.18 (d, J = 5.6 Hz, 6H).

### Example 49. BRG1/BRM ATPase inhibitors cause uveal melanoma tumor growth inhibition in vivo.

**Procedure:** Nude mice (Envigo) were engrafted subcutaneously in the axillary region with 5x10⁶ 92-1 uveal melanoma cells in 50 % Matrigel. Tumors were grown to a mean of ~200 mm³, at which point mice were grouped and dosing was initiated. Mice were dosed once daily by oral gavage with vehicle (20% 2-Hydroxypropyl-β-Cyclodextrin) or increasing doses of Compound C. Tumor volumes and body weights were measured over the course of 3 weeks, and doses were adjusted by body weight to achieve the proper dose in terms of mg/kg. At this time, animals were sacrificed, and tumors were dissected and imaged.

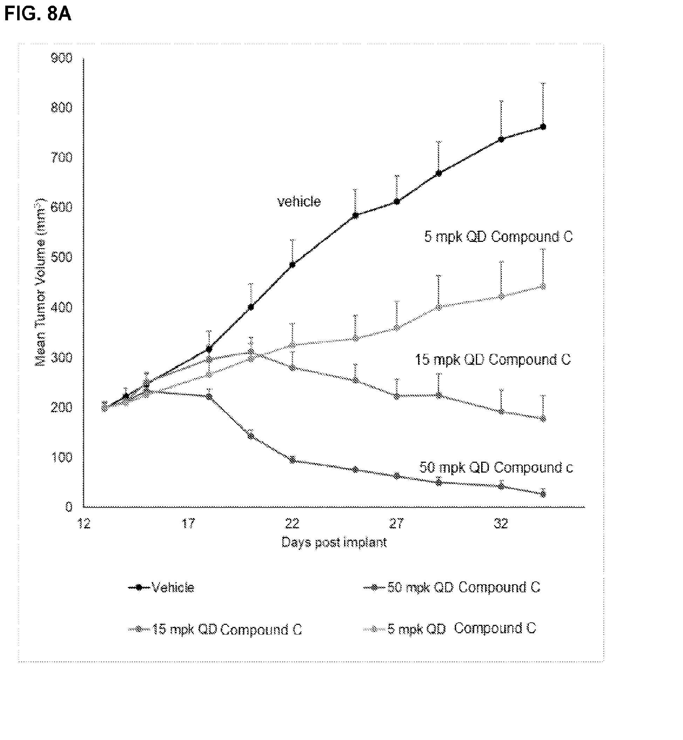

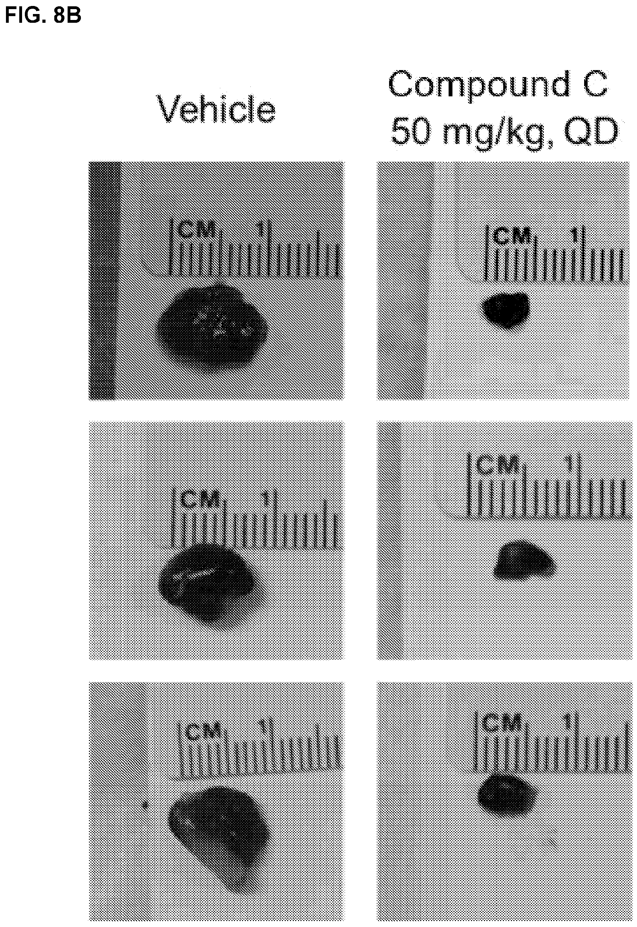

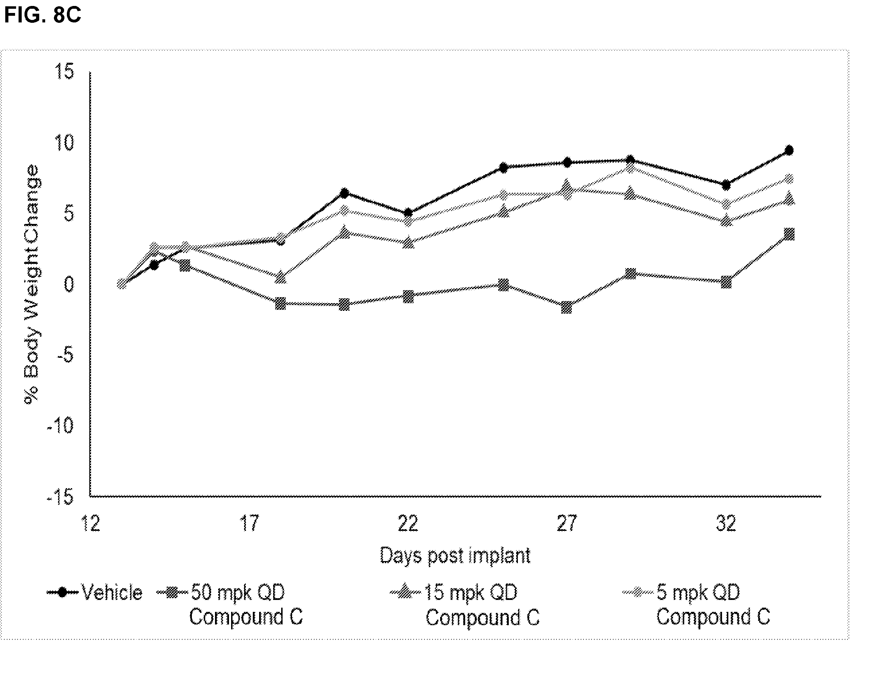

**Results:** Treatment with Compound C led to tumor growth inhibition in a dose-dependent manner with tumor regression observed at the highest (50 mg/kg) dose. (FIG. 8A and FIG. 8B). All treatments were well tolerated with no body weight loss observed (FIG. 8C).

## Claims

1. A compound having the structure: 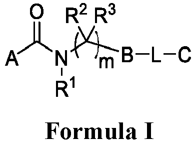 wherein
A is 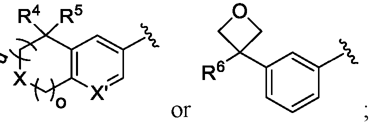
m is 0, 1, 2, or 3;
n is 1 or 2;
o is 0 or 1;
X is O, CH₂, or NR⁷;
X' is N, CH, or CR^{X}, wherein R^{X} is a halogen;
B is an optionally substituted 6-membered monocyclic heteroarylene or an optionally substituted 9- or 10-membered bicyclic heteroarylene;
L is a covalent bond, optionally substituted C₁-C₃ alkylene, C₂ alkynylene, optionally substituted C₂ alkenylene, optionally substituted C₂-C₃ heteroalkylene, optionally substituted C₃-C₅ cycloalkylene, or optionally substituted 4- to 10-membered heterocyclylene;
C is optionally substituted 3- to 10-membered cycloalkyl; optionally substituted 6- to 10-membered aryl; optionally substituted 5- to 10-membered heteroaryl; or optionally substituted 5- to 10-membered heterocyclyl;
R¹ and R⁷ are, independently, hydrogen or optionally substituted C₁-C₆ alkyl;
each R² and R³ is independently, hydrogen, optionally substituted C₁-C₆ alkyl; or optionally substituted C₁-C₆ heteroalkyl;
R⁴ is cyano, fluoro, hydroxy, or -CH₂OH;
R⁵ is C₁-C₃ alkyl optionally substituted with one, two, three, four, five, six, or seven fluoro groups; and
R⁶ is C₁-C₃ alkyl optionally substituted with one, two, three, four, five, six, or seven fluoro groups,
or a pharmaceutically acceptable salt thereof.

2. The compound of claim 1, wherein R¹ is hydrogen.

3. The compound of claim 1 or 2, wherein m is 1.

4. The compound of any one of claims 1 to 3, wherein R³ is hydrogen.

5. The compound of any one of claims 1 to 4, wherein R² is hydrogen.

6. The compound of any one of claims 1 to 4, wherein R² is optionally substituted C₁-C₆ alkyl.

7. The compound of any one of claims 1 to 4, wherein R² is optionally substituted C₁-C₆ heteroalkyl.

8. The compound of any one of claims 1 to 7, wherein A is

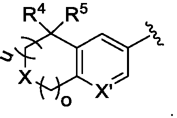

9. The compound of claim 8, wherein *R⁵* is methyl or -CHF₂.

10. The compound of claim 8 or 9, wherein X is O.

11. The compound of any one of claims 8 to 10, wherein R⁴ is cyano.

12. The compound of claim 8, wherein A is

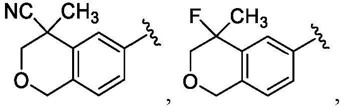

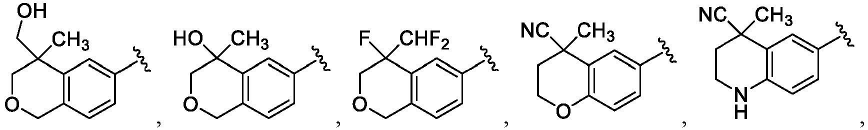

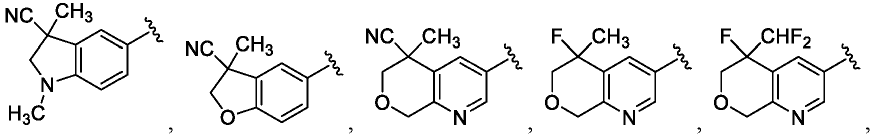

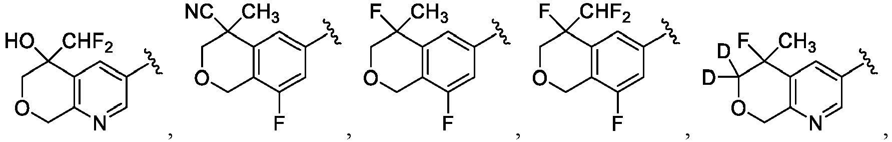

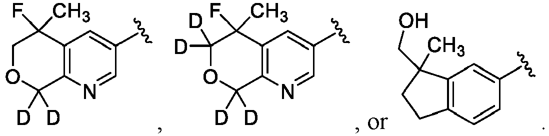

13. The compound of any one of claims 1 to 12, wherein C is optionally substituted 3- to 10-membered cycloalkyl.

14. The compound of any one of claims 1 to 12, wherein C is optionally substituted 6- to 10-membered aryl.

15. The compound of claim 14, wherein C is phenyl, 3-difluoromethyl-phenyl, 4-dilfluoromethylphenyl, 2-methoxy-phenyl, 3-methoxy-phenyl, 4-methoxy-phenyl, 3,5-difluorophenyl, 3-difluoromethylphenyl, 2-methoxy-5-methyl-phenyl, 2-methoxy-4-chlorophenyl, 3-cyano-phenyl, 3-fluoro-5-methoxy-phenyl, 3-fluoro-5-azitidinyl-phenyl, 3-azetidyl-phenyl, 4-azetidyl-2-methoxy-phenyl, 4-morpholin-4-yl-2-methoxy-phenyl, 4-tert-butyl-2-methoxy-phenyl, 4-azetidyl-phenyl, 2-methoxy-4-oxetan-3-yl-phenyl, 2-azetidin-2-one-1-yl-phenyl, 3-(3-difluoromethyloxetan-3-yl)-phenyl, 3-(3-methoxyoxetan-3-yl)-phenyl, 3-(N-methyl-N-acetamido)-phenyl, 3-cyclopropylphenyl, 3-(N-methylazetidin-3-yl)-phenyl, 3-(N-methyl-N'-piperazinyl)-phenyl, 3-fluoro-5-azetidyl-phenyl, 3-methyl ester-phenyl,

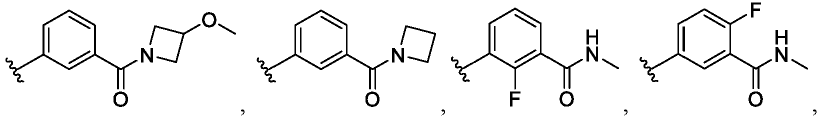

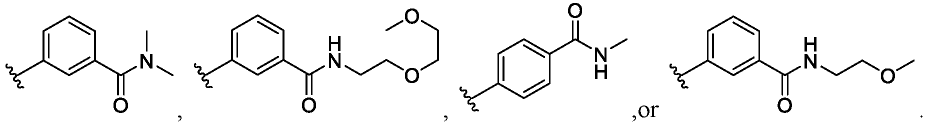

16. The compound of any one of claims 1 to 12, wherein C is optionally substituted 5- to 10-membered heteroaryl.

17. The compound of claim 16, wherein C is

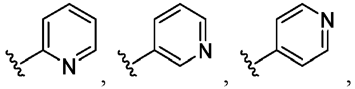

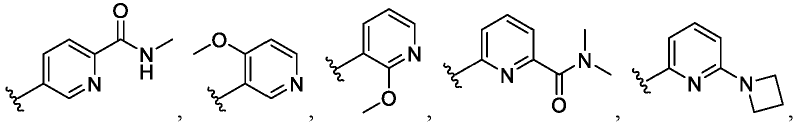

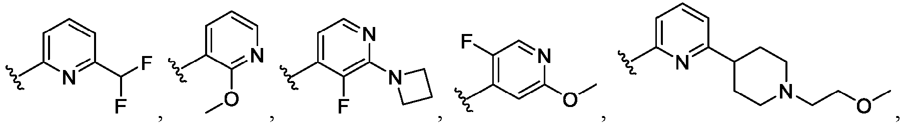

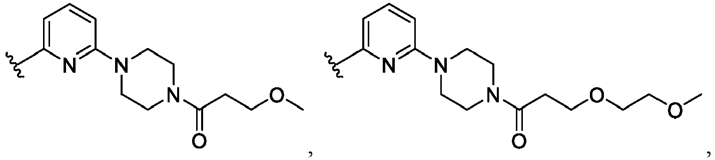

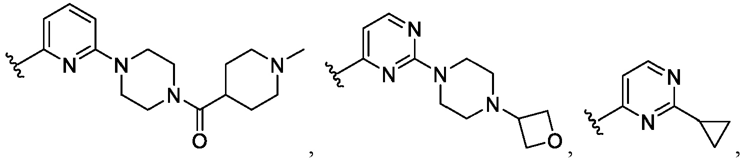

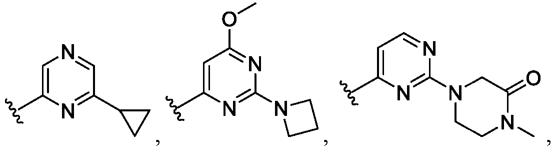

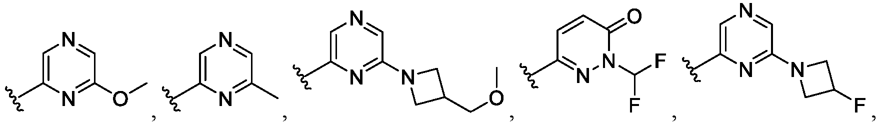

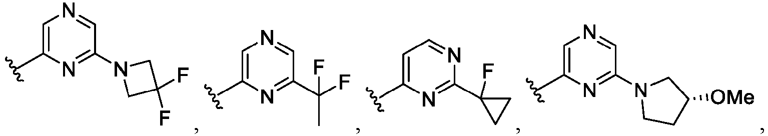

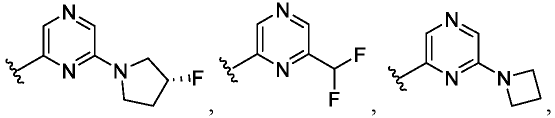

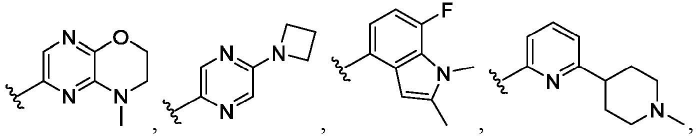

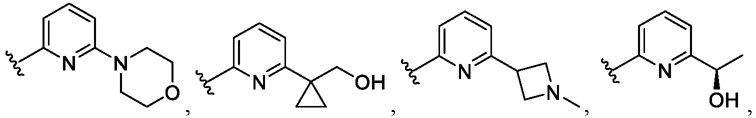

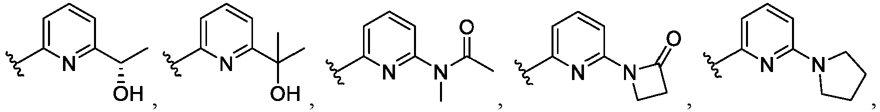

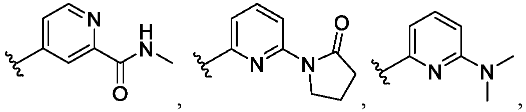

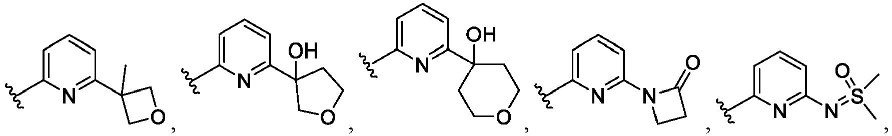

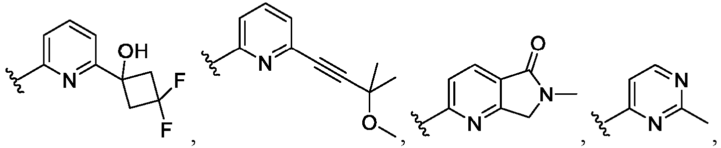

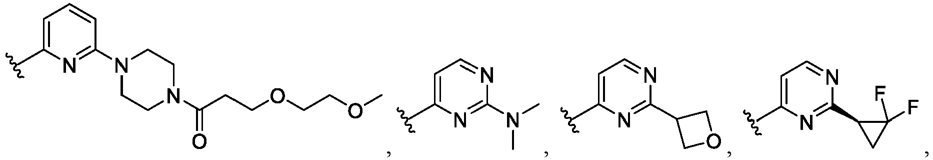

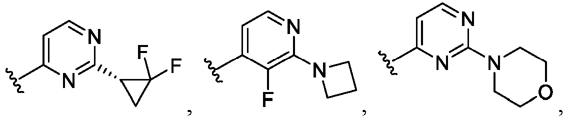

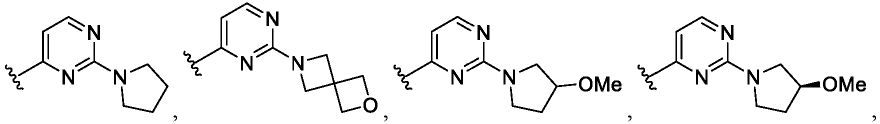

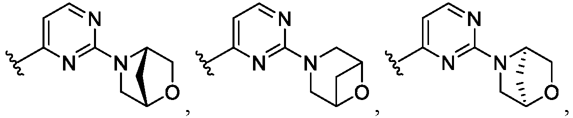

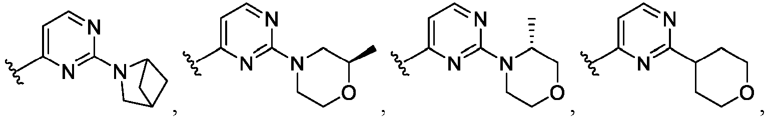

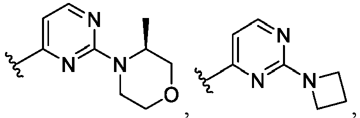

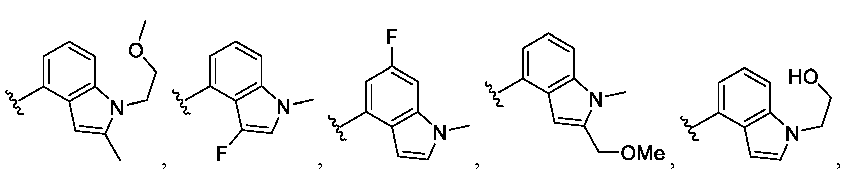

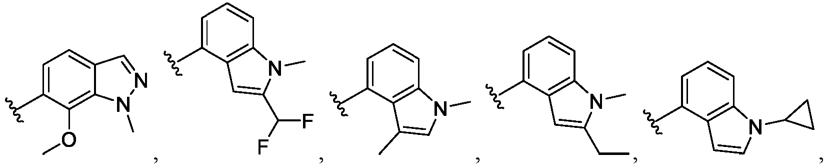

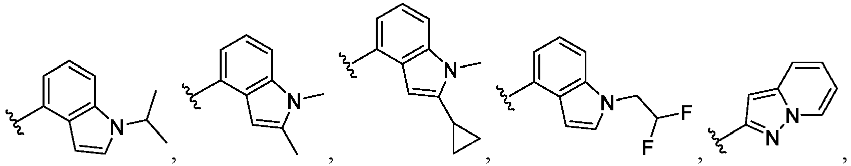

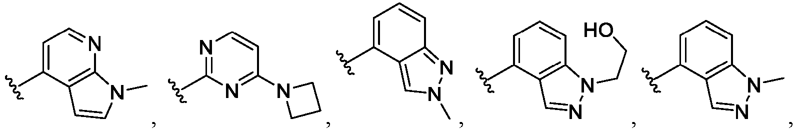

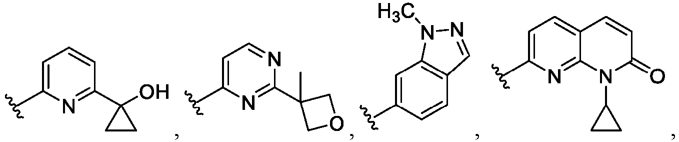

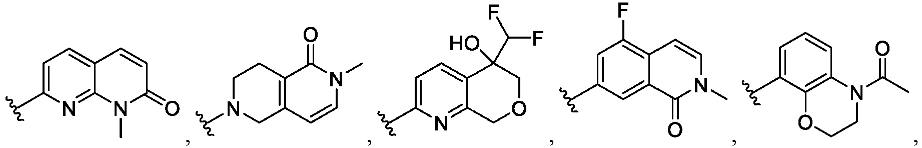

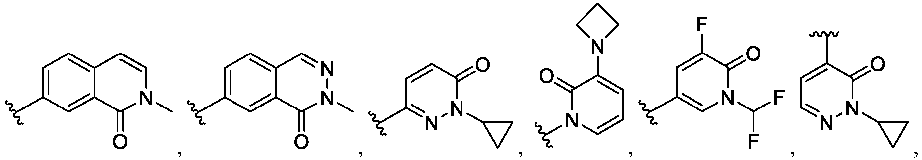

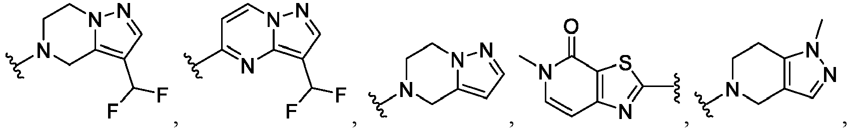

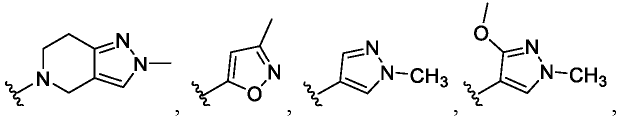

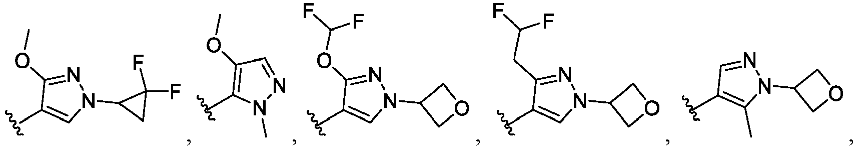

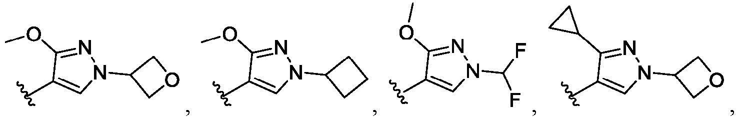

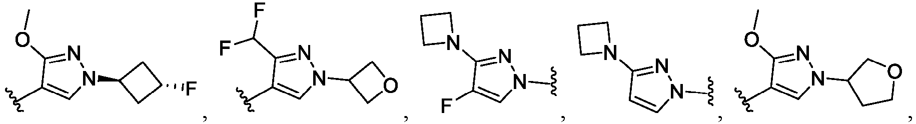

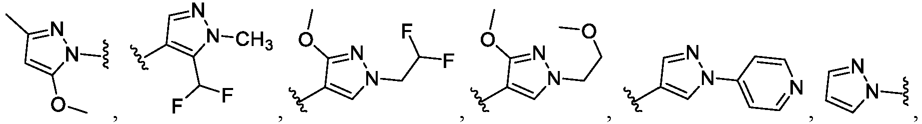

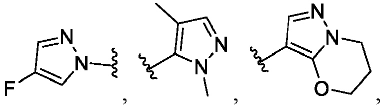

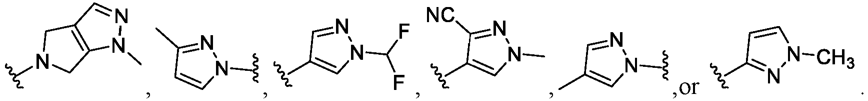

18. The compound of claim 16, wherein C is

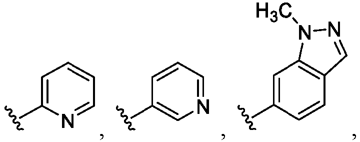

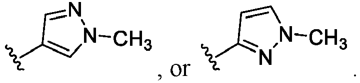

19. The compound of any one of claims 1 to 12, wherein C is optionally substituted 5- to 10-membered heterocyclyl.

20. The compound of claim 19, wherein C is 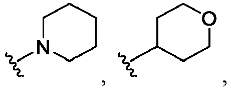 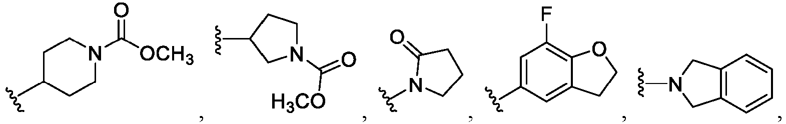 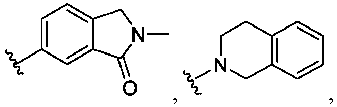 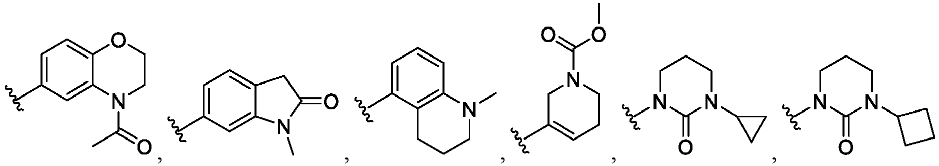 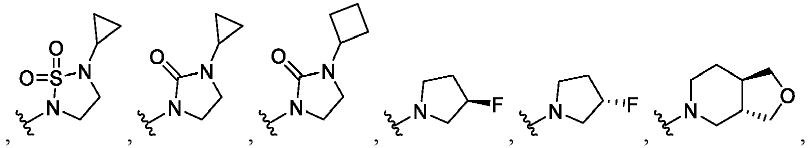 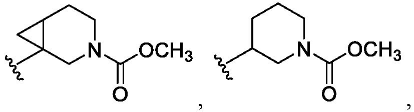 or N-pyrrolidinyl.

21. The compound of claim 19, wherein C is 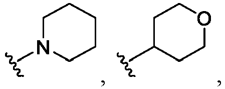 or

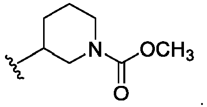

22. The compound of any one of claims 1 to 21, wherein B is an optionally substituted 6-membered monocyclic heteroarylene.

23. The compound of claim 22, wherein B has the structure: 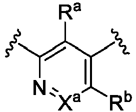
wherein R^{a} is hydrogen or fluoro;
R^{b} is hydrogen, fluoro, or C₁-C₃ alkyl; and
X^{a} is N or CH.

24. The compound of claim 23, wherein B is

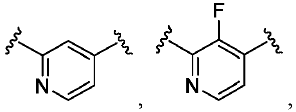

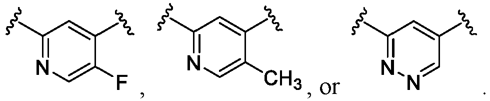

25. The compound of any one of claims 1 to 21, wherein B is an optionally substituted 9- or 10-membered bicyclic heteroarylene.

26. The compound of claim 25, wherein B has the structure: 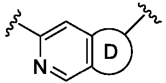 wherein D is an optionally substituted 5- or 6-membered heteroaryl or optionally substituted 5- or 6-membered heterocyclyl.

27. The compound of claim 26, wherein B is

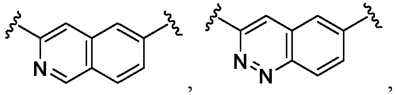

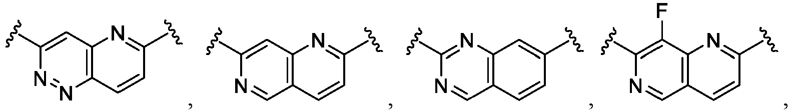

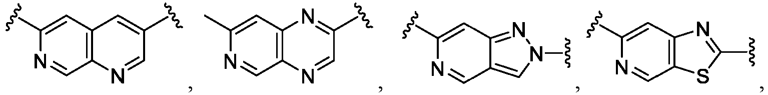

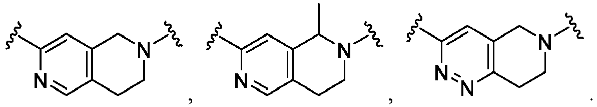

28. The compound of any one of claims 1 to 27, wherein L is optionally substituted C₁-C₃ alkylene.

29. The compound of any one of claims 1 to 27, wherein L is optionally substituted C₂ alkenylene.

30. The compound of claim 29, wherein L is 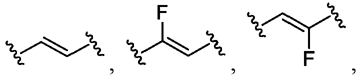 or

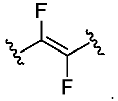

31. The compound of any one of claims 1 to 27, wherein L is optionally substituted C₂-C₃ heteroalkylene.

32. The compound of any one of claims 1 to 27, wherein L is optionally substituted C₃-C₅ cycloalkylene.

33. The compound of claim 32, wherein L is

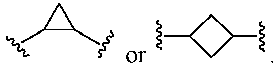

34. The compound of any one of claims 1 to 27, wherein L is optionally substituted 4- to 10-membered heterocyclylene.

35. The compound of claim 34, wherein L is

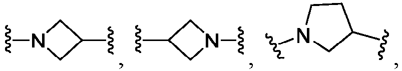

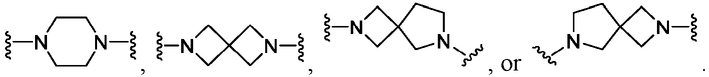

36. A pharmaceutical composition comprising a compound of any one of claims 1 to 35 and a pharmaceutically acceptable excipient.

37. A compound of any one of claims 1 to 35 or a pharmaceutical composition of claim 36 for use in treating cancer.

38. The compound for use according to claim 37, wherein the cancer is non-small cell lung cancer, colorectal cancer, bladder cancer, cancer of unknown primary, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, small-cell lung cancer, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer, or penile cancer.

39. The compound for use according to claim 38, wherein the cancer is non-small cell lung cancer, colorectal cancer, bladder cancer, cancer of unknown primary, glioma, breast cancer, melanoma, non-melanoma skin cancer, endometrial cancer, soft tissue sarcoma, or penile cancer.

40. The compound for use according to claim 39, wherein the cancer is non-small cell lung cancer.

41. The compound for use according to claim 39, wherein the cancer is soft tissue sarcoma.

## Patentansprüche

1. Verbindung, die die Struktur aufweist: 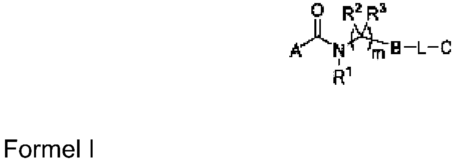 wobei
A ist 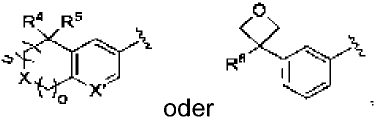
m 0, 1, 2 oder 3 ist;
n 1 oder 2 ist;
o 0 oder 1 ist;
X O, CH₂ oder NR⁷ ist;
X' N, CH oder CR^{X} ist, wobei R^{X} ein Halogen ist;
B ein optional substituiertes 6-gliedriges monocyclisches Heteroarylen oder ein optional substituiertes 9- oder 10-gliedriges bicyclisches Heteroarylen ist;
L eine kovalente Bindung, optional substituiertes C₁-C₃-Alkylen, C₂-Alkinylen, optional substituiertes C₂-Alkenylen, optional substituiertes C₂-C₃-Heteroalkylen, optional substituiertes C₃-C₅-Cycloalkylen oder optional substituiertes 4- bis 10-gliedriges Heterocyclylen ist;
C optional substituiertes 3- bis 10-gliedriges Cycloalkyl; optional substituiertes 6- bis 10-gliedriges Aryl; optional substituiertes 5- bis 10-gliedriges Heteroaryl; oder optional substituiertes 5- bis 10-gliedriges Heterocyclyl ist;
R¹ und R⁷ unabhängig Wasserstoff oder optional substituiertes C₁-C₆-Alkyl sind;
jedes R² und R³ unabhängig Wasserstoff, optional substituiertes C₁-C₆-Alkyl; oder optional substituiertes C₁-C₆-Heteroalkyl ist;
R⁴ Cyan, Fluor, Hydroxy oder -CH₂OH ist;
R⁵ C₁-C₃-Alkyl ist, das optional mit einer, zwei, drei, vier, fünf, sechs oder sieben Fluorgruppen substituiert ist; und
R⁶ C₁-C₃-Alkyl ist, das optional mit einer, zwei, drei, vier, fünf, sechs oder sieben Fluorgruppen substituiert ist,
oder ein pharmazeutisch verträgliches Salz davon.

2. Verbindung nach Anspruch 1, wobei R¹ Hydrogen ist.

3. Verbindung nach Anspruch 1 oder 2, wobei m 1 ist.

4. Verbindung nach einem der Ansprüche 1 bis 3, wobei R³ Hydrogen ist.

5. Verbindung nach einem der Ansprüche 1 bis 4, wobei R² Hydrogen ist.

6. Verbindung nach einem der Ansprüche 1 bis 4, wobei R² ein optional substituiertes C₁-C₆-Alkyl ist.

7. Verbindung nach einem der Ansprüche 1 bis 4, wobei R² ein optional substituiertes C₁-C₆-Heteroalkyl ist.

8. Verbindung nach einem der Ansprüche 1 bis 7, wobei A ist

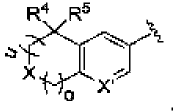

9. Verbindung nach Anspruch 8, wobei R⁵ Methyl oder -CHF₂ ist.

10. Verbindung nach Anspruch 8 oder 9, wobei X O ist.

11. Verbindung nach einem der Ansprüche 8 bis 10, wobei R⁴ Cyan ist.

12. Verbindung nach Anspruch 8, wobei A ist

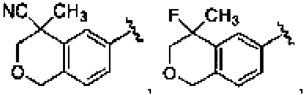

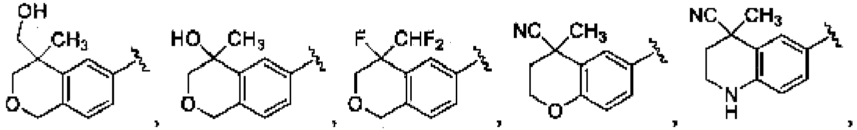

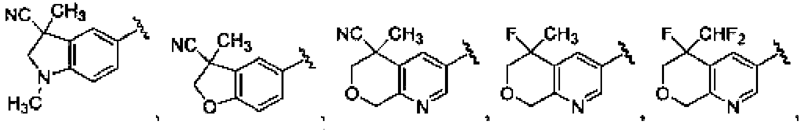

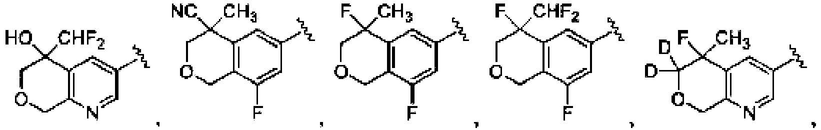

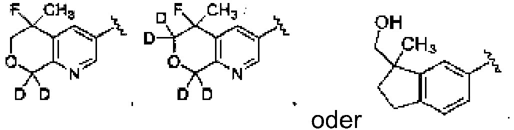

13. Verbindung nach einem der Ansprüche 1 bis 12, wobei C ein optional substituiertes 3- bis 10-gliedriges Cycloalkyl ist.

14. Verbindung nach einem der Ansprüche 1 bis 12, wobei C ein optional substituiertes 6- bis 10-gliedriges Aryl ist.

15. Verbindung nach Anspruch 14, wobei C Phenyl, 3-Difluormethyl-phenyl, 4-Difluormethylphenyl, 2-Methoxy-phenyl, 3-Methoxy-phenyl, 4-Methoxy-phenyl, 3,5-Difluorphenyl, 3-Difluormethylphenyl, 2-Methoxy-5-methyl-phenyl, 2-Methoxy-4-chlor-phenyl, 3-Cyano-phenyl, 3-Fluor-5-methoxy-phenyl, 3-Fluor-5-azitidinyl-phenyl, 3-Azetidyl-phenyl, 4-Azetidyl-2-methoxy-phenyl, 4-Morpholin-4-yl-2-methoxy-phenyl, 4-tert-Butyl-2-methoxy-phenyl, 4-Azetidyl-phenyl, 2-Methoxy-4-oxetan-3-yl-phenyl, 2-Azetidin-2-on-1-yl-phenyl, 3-(3-Difluormethyloxetan-3-yl)-phenyl, 3-(3-Methoxyoxetan-3-yl)-phenyl, 3-(N-Methyl-N-acetamido)-phenyl, 3-Cyclopropylphenyl, 3-(N-Methylazetidin-3-yl)-phenyl, 3-(N-Methyl-N'-piperazinyl)-phenyl, 3-Fluor-5-azetidyl-phenyl, 3-Methylester-phenyl ist, 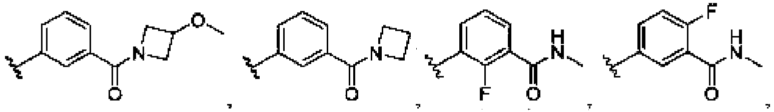 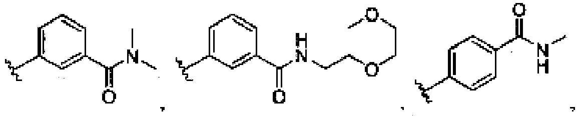 oder

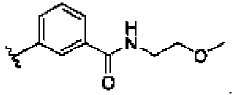

16. Verbindung nach einem der Ansprüche 1 bis 12, wobei C ein optional substituiertes 5- bis 10-gliedriges Heteroaryl ist.

17. Verbindung nach Anspruch 16, wobei C ist

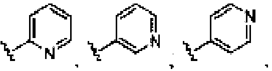

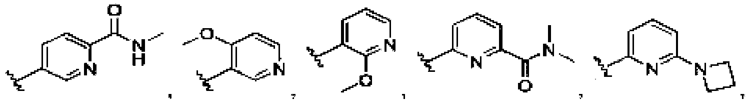

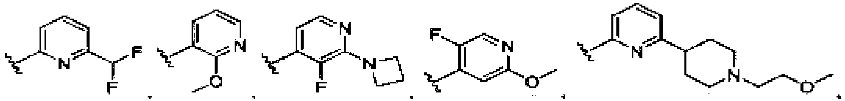

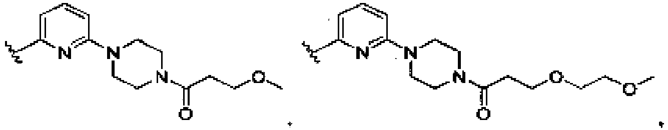

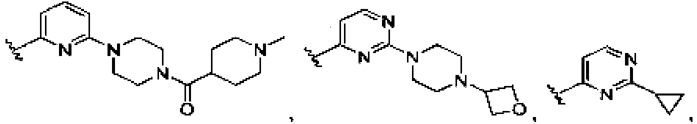

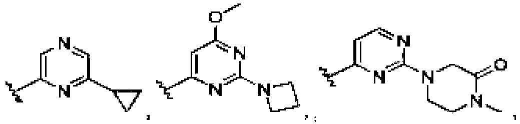

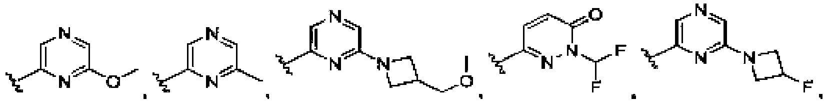

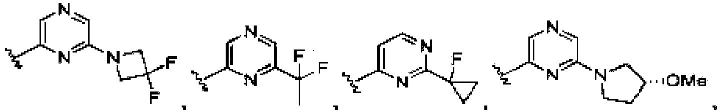

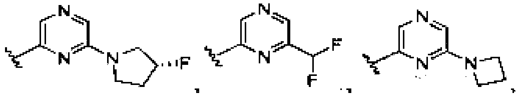

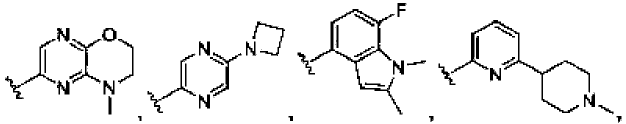

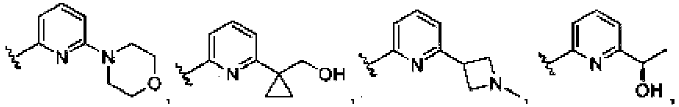

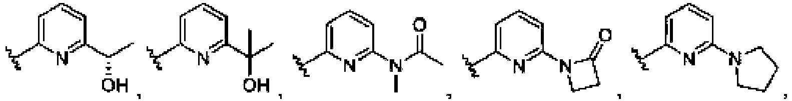

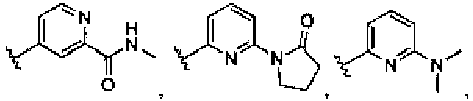

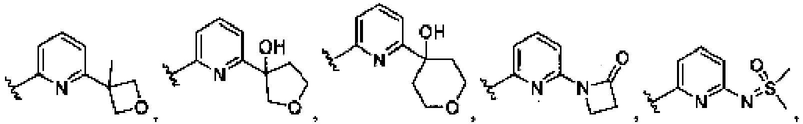

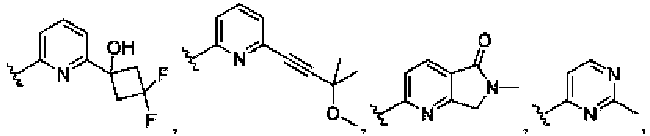

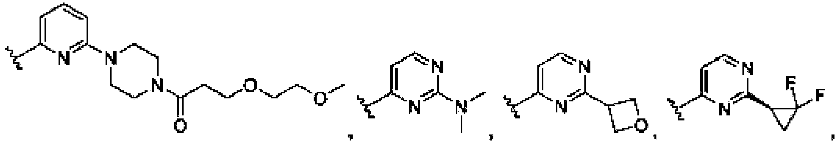

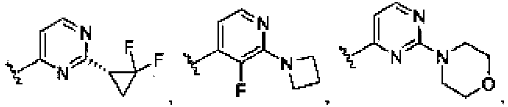

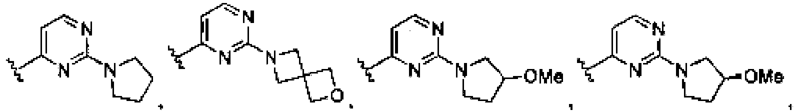

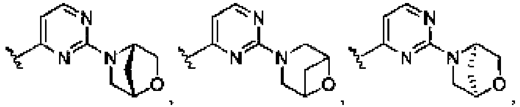

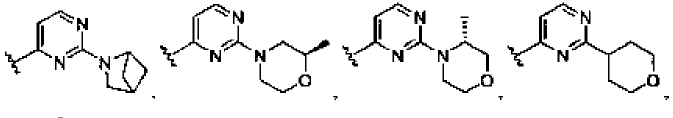

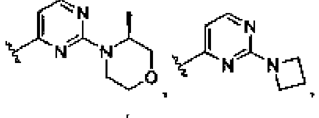

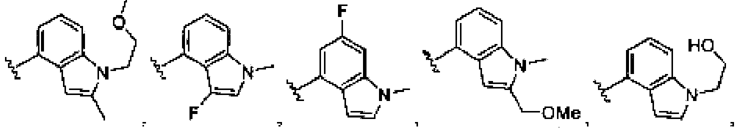

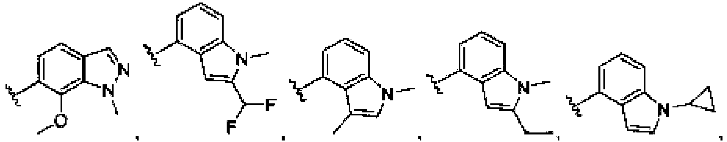

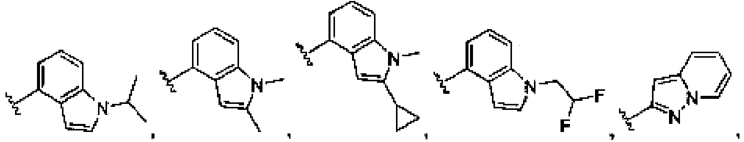

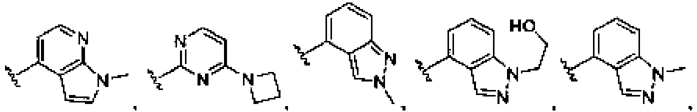

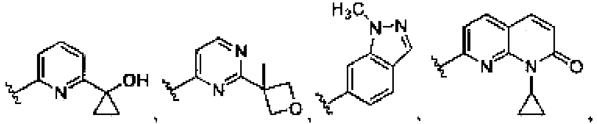

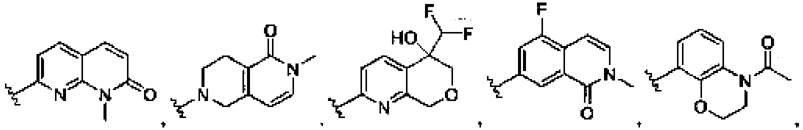

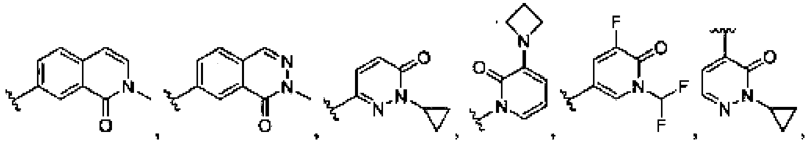

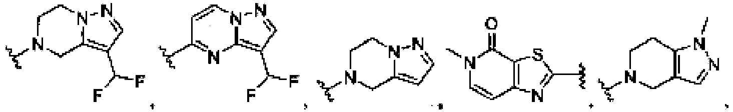

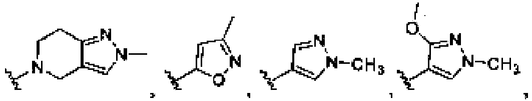

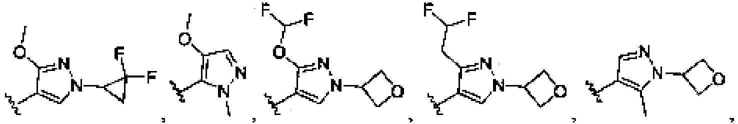

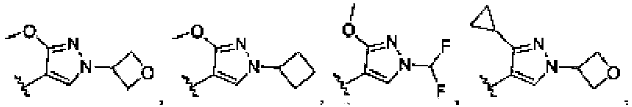

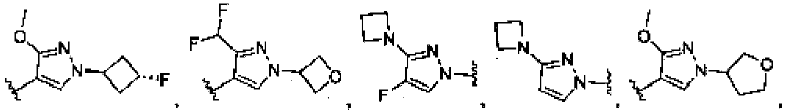

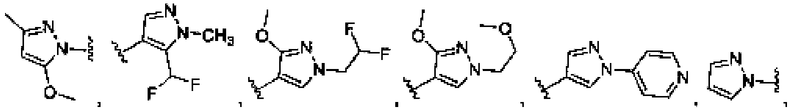

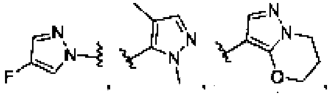

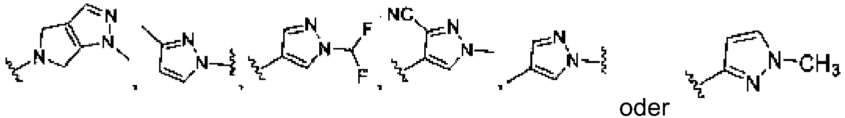

18. Verbindung nach Anspruch 16, wobei C ist

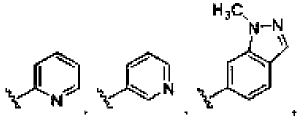

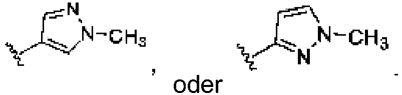

19. Verbindung nach einem der Ansprüche 1 bis 12, wobei C ein optional substituiertes 5- bis 10-gliedriges Heterocyclyl ist.

20. Verbindung nach Anspruch 19, wobei C 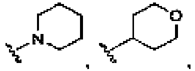 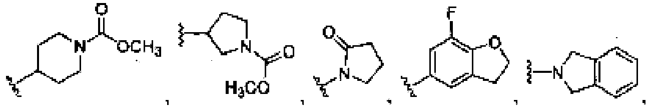 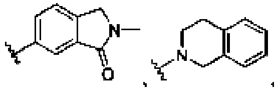 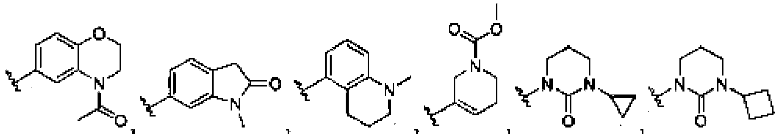 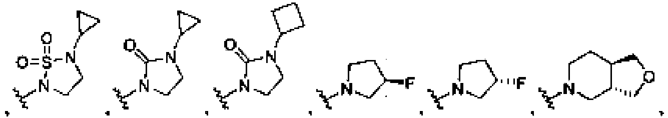 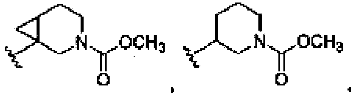 oder N-Pyrrolidinyl ist.

21. Verbindung nach Anspruch 19, wobei C ist 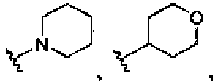 oder

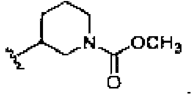

22. Verbindung nach einem der Ansprüche 1 bis 21, wobei B ein optional substituiertes 6-gliedriges monocyclisches Heteroarylen ist.

23. Verbindung nach Anspruch 22, wobei B die Struktur aufweist: 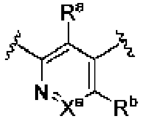
wobei R^{a} Wasserstoff oder Fluor ist;
R^{b} Hydrogen, Fluor oder C₁-C₃-Alkyl ist; und
X^{a} N oder CH ist.

24. Verbindung nach Anspruch 23, wobei B ist

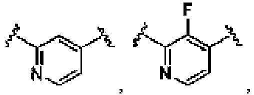

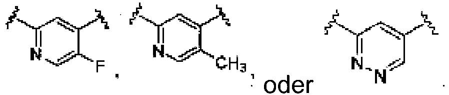

25. Verbindung nach einem der Ansprüche 1 bis 21, wobei B ein optional substituiertes 9- oder 10-gliedriges bicyclisches Heteroarylen ist.

26. Verbindung nach Anspruch 25, wobei B die Struktur aufweist: 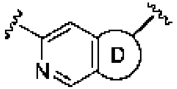 wobei D ein optional substituiertes 5- oder 6-gliedriges Heteroaryl oder ein optional substituiertes 5- oder 6-gliedriges Heterocyclyl ist.

27. Verbindung nach Anspruch 26, wobei B ist

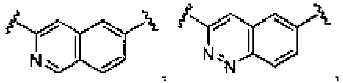

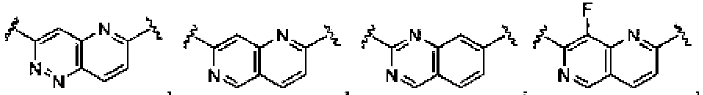

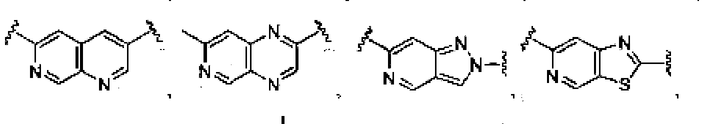

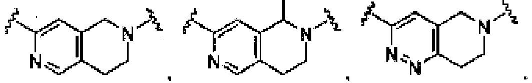

28. Verbindung nach einem der Ansprüche 1 bis 27, wobei L ein optional substituiertes C₁-C₃-Alkylen ist.

29. Verbindung nach einem der Ansprüche 1 bis 27, wobei L ein optional substituiertes C₂-Alkenylen ist.

30. Verbindung nach Anspruch 29, wobei L ist 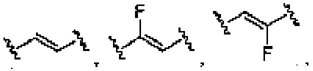 oder

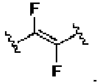

31. Verbindung nach einem der Ansprüche 1 bis 27, wobei L ein optional substituiertes C₂-C₃-Heteroalkylen ist.

32. Verbindung nach einem der Ansprüche 1 bis 27, wobei L ein optional substituiertes C₃-C₅-Cycloalkylen ist.

33. Verbindung nach Anspruch 32, wobei L ist 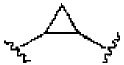 oder

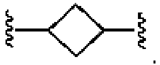

34. Verbindung nach einem der Ansprüche 1 bis 27, wobei L ein optional substituiertes 4- bis 10-gliedriges Heterocyclylen ist.

35. Verbindung nach Anspruch 34, wobei L ist 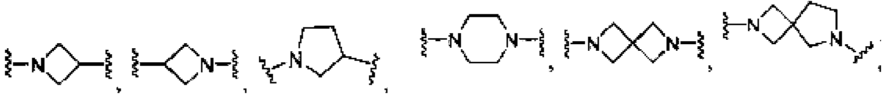 oder

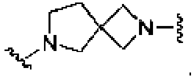

36. Pharmazeutische Zusammensetzung, umfassend eine Verbindung nach einem der Ansprüche 1 bis 35 und einen pharmazeutisch verträglichen Trägerstoff.

37. Verbindung nach einem der Ansprüche 1 bis 35 oder pharmazeutische Zusammensetzung nach Anspruch 36 zur Verwendung bei einem Behandeln von Krebs.

38. Verbindung zur Verwendung nach Anspruch 37, wobei der Krebs nichtkleinzelliger Lungenkrebs, kolorektaler Krebs, Blasenkrebs, Krebs unbekannten Primärtumors, Gliom, Brustkrebs, Melanom, Nicht-Melanom-Hautkrebs, Endometriumkrebs, ösophagogastrischer Krebs, Pankreaskrebs, hepatobiliärer Krebs, Weichgewebesarkom, Eierstockkrebs, Kopf- und Halskrebs, Nierenzellkarzinom, Knochenkrebs, Non-Hodgkin-Lymphom, kleinzelliger Lungenkrebs, Prostatakrebs, embryonaler Tumor, Keimzelltumor, Gebärmutterhalskrebs, Schilddrüsenkrebs, Speicheldrüsenkrebs, gastrointestinaler neuroendokriner Tumor, Uterussarkom, gastrointestinaler Stromatumor, ZNS-Krebs, Thymustumor, Nebennierenrindenkarzinom, Appendixkrebs, Dünndarmkrebs oder Peniskrebs ist.

39. Verbindung zur Verwendung nach Anspruch 38, wobei der Krebs nichtkleinzelliger Lungenkrebs, kolorektaler Krebs, Blasenkrebs, Krebs unbekannten Primärtumors, Gliom, Brustkrebs, Melanom, Nicht-Melanom-Hautkrebs, Endometriumkrebs, Weichgewebesarkom oder Peniskrebs ist.

40. Verbindung zur Verwendung nach Anspruch 39, wobei der Krebs nichtkleinzelliger Lungenkrebs ist.

41. Verbindung zur Verwendung nach Anspruch 39, wobei der Krebs ein Weichgewebesarkom ist.

## Revendications

1. Composé ayant la structure : 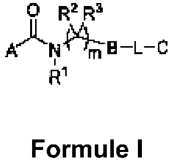 dans lequel
A est 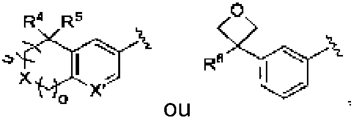
m vaut 0, 1, 2, ou 3 ;
n vaut 1 ou 2 ;
o vaut 0 ou 1 ;
X est O, CH₂, ou NR⁷ ;
X' est N, CH, ou CR^{X}, dans lequel R^{X} est un halogène ;
B est un hétéroarylène monocyclique à 6 chaînons facultativement substitué ou un hétéroarylène bicyclique à 9 ou 10 chaînons facultativement substitué ;
L est une liaison covalente, alkylène en C₁-C₃ facultativement substitué, alcynylène en C₂, alcénylène en C₂ facultativement substitué, hétéroalkylène en C₂-C₃ facultativement substitué, cycloalkylène en C₃-C₅ facultativement substitué, ou hétérocyclylène à 4 à 10 chaînons facultativement substitué ;
C est cycloalkyle à 3 à 10 chaînons facultativement substitué ; aryle à 6 à 10 chaînons facultativement substitué ; hétéroaryle à 5 à 10 chaînons facultativement substitué ; ou hétérocyclyle à 5 à 10 chaînons facultativement substitué ;
R¹ et R⁷ sont, indépendamment, hydrogène ou alkyle en C₁-C₆ facultativement substitué ;
chaque R² et R³ est indépendamment hydrogène, alkyle en C₁-C₆ facultativement substitué ; ou hétéroalkyle en C₁-C₆ facultativement substitué ;
R⁴ est cyano, fluoro, hydroxy, ou -CH₂OH ;
R⁵ est alkyle en C₁-C₃ facultativement substitué par un, deux, trois, quatre, cinq, six, ou sept groupes fluoro ; et
R⁶ est alkyle en C₁-C₃ facultativement substitué par un, deux, trois, quatre, cinq, six, ou sept groupes fluoro,
ou un sel pharmaceutiquement acceptable de celui-ci.

2. Composé selon la revendication 1, dans lequel R¹ est hydrogène.

3. Composé selon la revendication 1 ou 2, dans lequel m vaut 1.

4. Composé selon l'une quelconque des revendications 1 à 3, dans lequel R³ est hydrogène.

5. Composé selon l'une quelconque des revendications 1 à 4, dans lequel R² est hydrogène.

6. Composé selon l'une quelconque des revendications 1 à 4, dans lequel R² est alkyle en C₁-C₆ facultativement substitué.

7. Composé selon l'une quelconque des revendications 1 à 4, dans lequel R² est hétéroalkyle en C₁-C₆ facultativement substitué.

8. Composé selon l'une quelconque des revendications 1 à 7, dans lequel 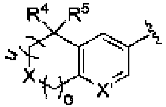 A est

9. Composé selon la revendication 8, dans lequel R⁵ est méthyle ou -CHF₂.

10. Composé selon la revendication 8 ou 9, dans lequel X est O.

11. Composé selon l'une quelconque des revendications 8 à 10, dans lequel R⁴ est cyano.

12. Composé selon la revendication 8, dans lequel A est

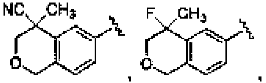

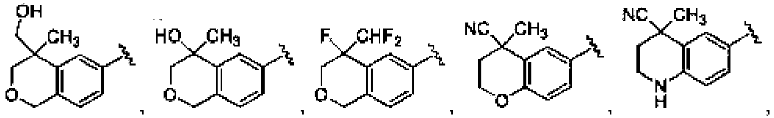

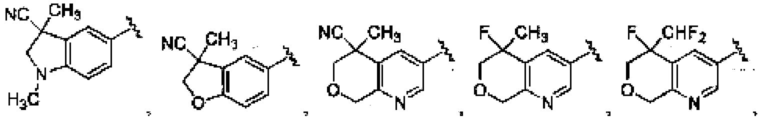

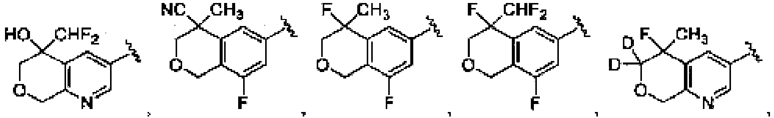

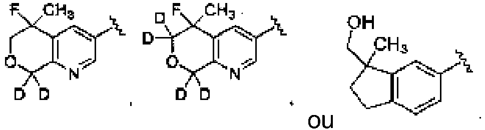

13. Composé selon l'une quelconque des revendications 1 à 12, dans lequel C est cycloalkyle à 3 à 10 chaînons facultativement substitué.

14. Composé selon l'une quelconque des revendications 1 à 12, dans lequel C est aryle à 6 à 10 chaînons facultativement substitué.

15. Composé selon la revendication 14, dans lequel C est phényle, 3-difluorométhyl-phényle, 4-dilfluorométhylphényle, 2-méthoxy-phényle, 3-méthoxy-phényle, 4-méthoxy-phényle, 3,5-difluorophényle, 3-difluorométhylphényle, 2-méthoxy-5-méthyl-phényle, 2-méthoxy-4-chloro-phényle, 3-cyano-phényle, 3-fluoro-5-méthoxy-phényle, 3-fluoro-5-azitidinyl-phényle, 3-azétidyl-phényle, 4-azétidyl-2-méthoxy-phényle, 4-morpholin-4-yl-2-méthoxy-phényle, 4-tert-butyl-2-méthoxy-phényle, 4-azétidyl-phényle, 2-méthoxy-4-oxétan-3-yl-phényle, 2-azétidin-2-one-1-yl-phényle, 3-(3-difluorométhyloxétan-3-yl)-phényle, 3-(3-méthoxyoxétan-3-yl)-phényle, 3-(N-méthyl-N-acétamido)-phényle, 3-cyclopropylphényle, 3-(N-méthylazétidin-3-yl)-phényle, 3-(N-méthyl-N'-pipérazinyl)-phényle, 3-fluoro-5-azétidyl-phényle, 3-méthylester-phényle,

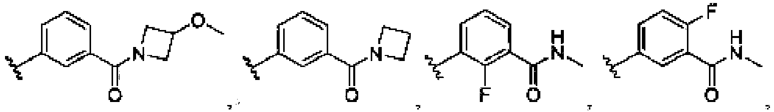

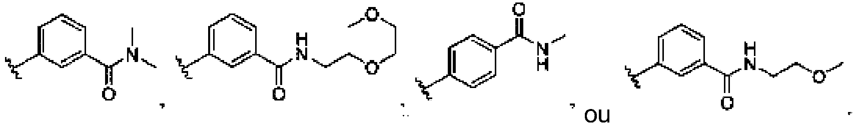

16. Composé selon l'une quelconque des revendications 1 à 12, dans lequel C est hétéroaryle à 5 à 10 chaînons facultativement substitué.

17. Composé selon la revendication 16, dans lequel C est

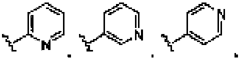

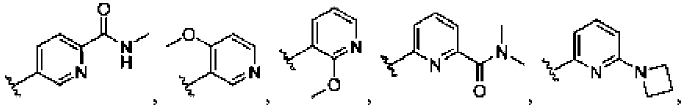

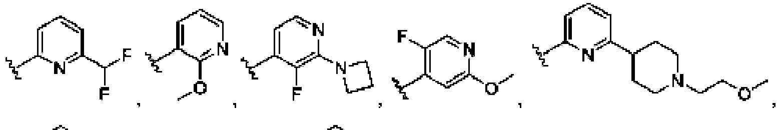

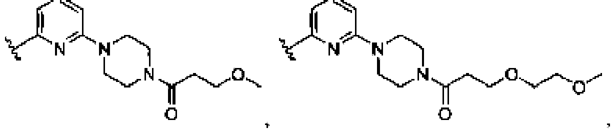

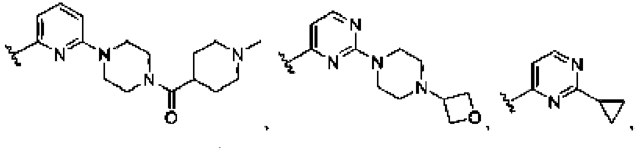

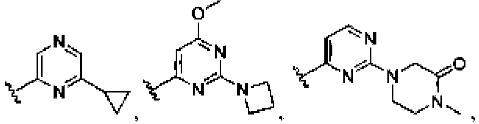

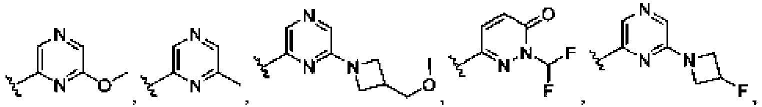

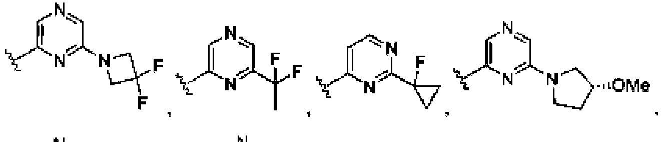

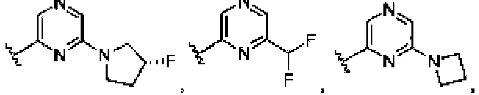

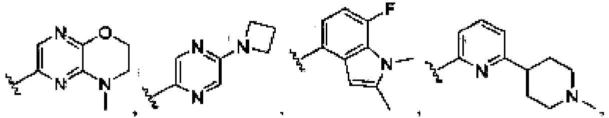

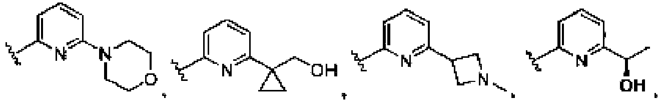

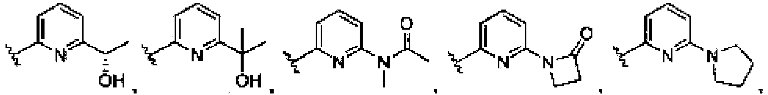

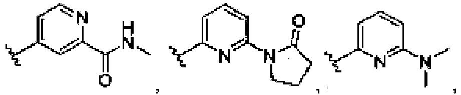

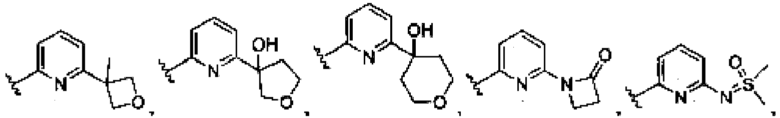

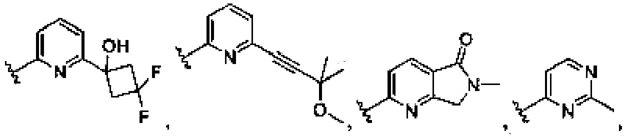

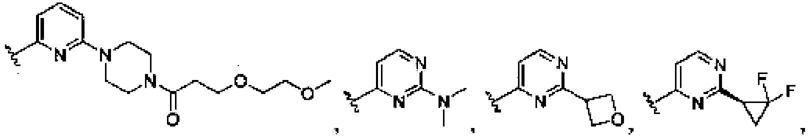

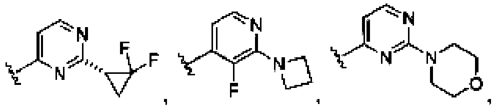

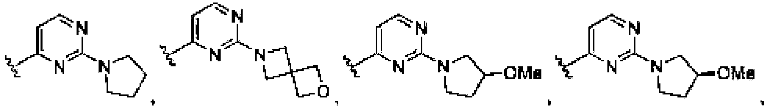

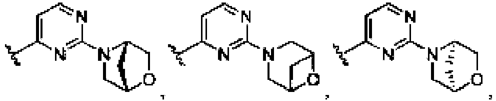

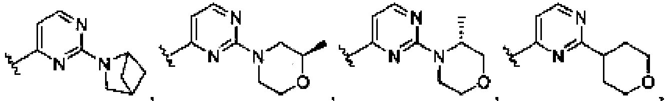

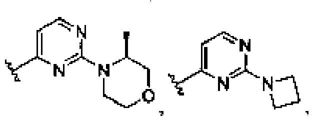

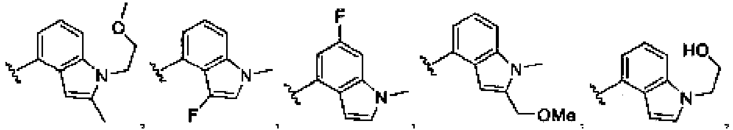

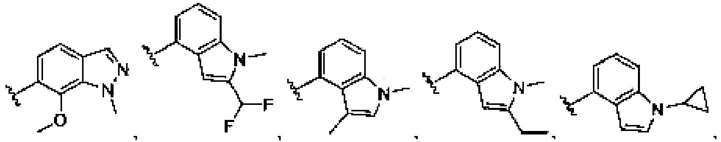

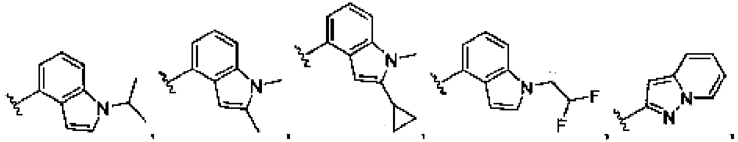

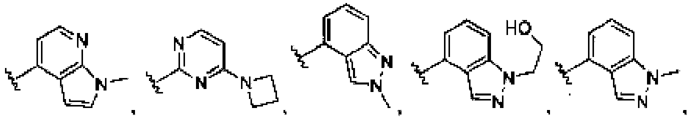

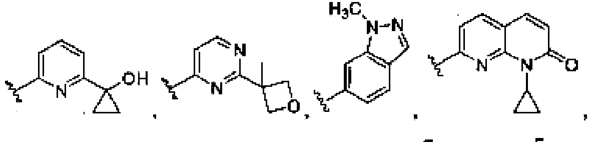

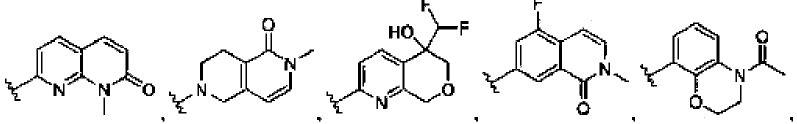

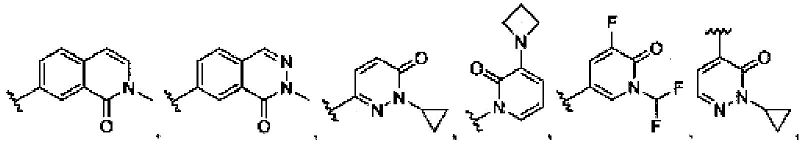

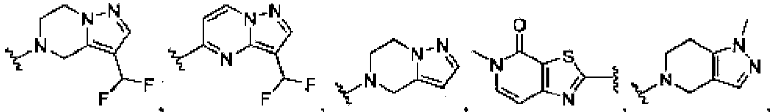

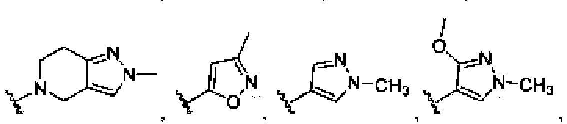

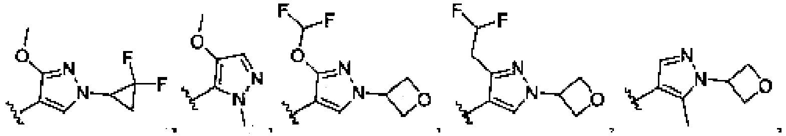

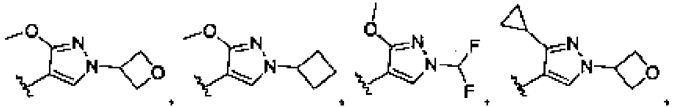

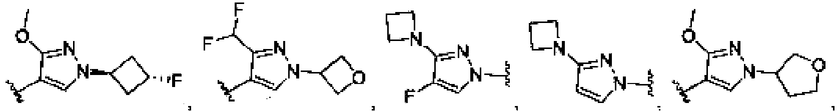

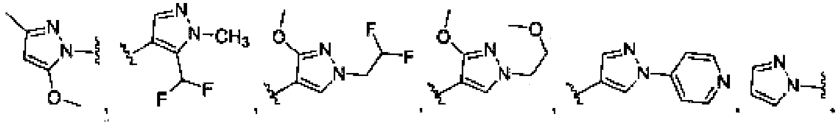

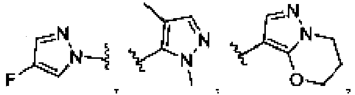

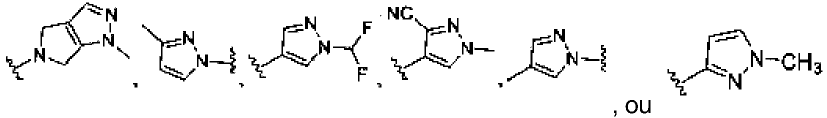

18. Composé selon la revendication 16, dans lequel C est

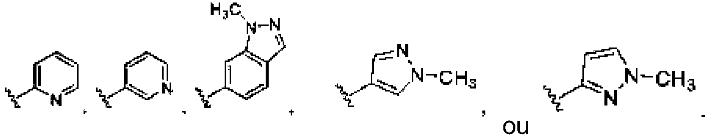

19. Composé selon l'une quelconque des revendications 1 à 12, dans lequel C est hétéroaryle à 5 à 10 chaînons facultativement substitué.

20. Composé selon la revendication 19, dans lequel C est 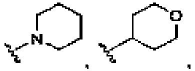 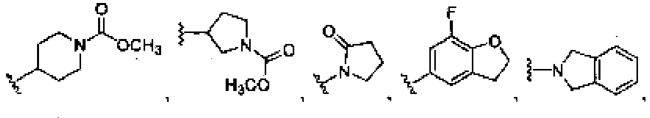 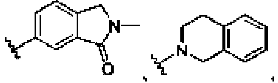 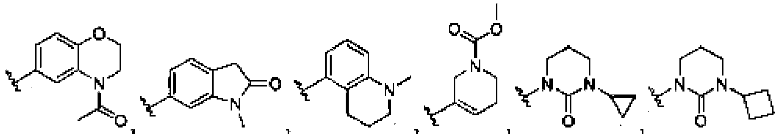 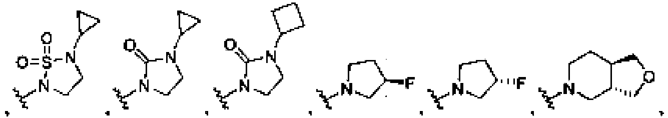 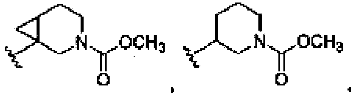 ou N-pyrrolidinyle.

21. Composé selon la revendication 19, dans lequel C est 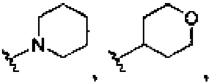 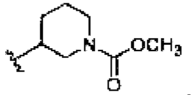 ou

22. Composé selon l'une quelconque des revendications 1 à 21, dans lequel B est un hétéroarylène monocyclique à 6 chaînons facultativement substitué.

23. Composé selon la revendication 22, dans lequel B a la structure :
dans lequel R^{a} est hydrogène ou fluoro ; 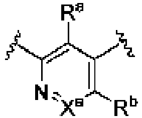
R^{b} représente hydrogène, fluoro, ou alkyle en C₁-C₃ ; et
X^{a} est N ou CH.

24. Composé selon la revendication 23, dans lequel B est

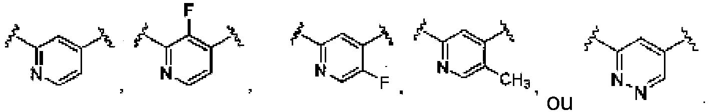

25. Composé selon l'une quelconque des revendications 1 à 21, dans lequel B est un hétéroarylène bicyclique à 9 ou 10 chaînons facultativement substitué.

26. Composé selon la revendication 25, dans lequel B a la structure : 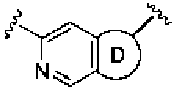 dans lequel D est un hétéroaryle à 5 ou 6 chaînons facultativement substitué ou un hétérocyclyle à 5 ou 6 chaînons facultativement substitué.

27. Composé selon la revendication 26, dans lequel B est

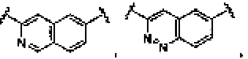

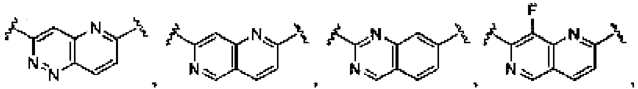

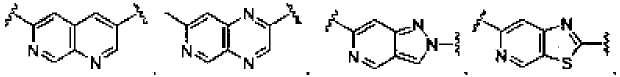

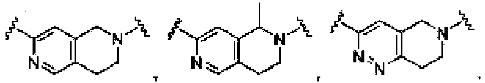

28. Composé selon l'une quelconque des revendications 1 à 27, dans lequel L est alkylène en C₁-C₃ facultativement substitué.

29. Composé selon l'une quelconque des revendications 1 à 27, dans lequel L est alcénylène en C₂ facultativement substitué.

30. Composé selon la revendication 29, dans lequel L est

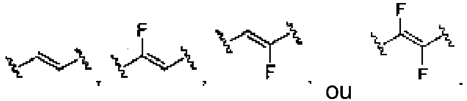

31. Composé selon l'une quelconque des revendications 1 à 27, dans lequel L est hétéroalkylène en C₂-C₃ facultativement substitué.

32. Composé selon l'une quelconque des revendications 1 à 27, dans lequel L est cycloalkylène en C₃-C₅ facultativement substitué.

33. Composé selon la revendication 32, dans lequel L est 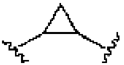 ou

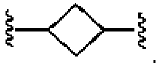

34. Composé selon l'une quelconque des revendications 1 à 27, dans lequel Z est hétérocyclylène à 4 à 10 chaînons facultativement substitué.

35. Composé selon la revendication 34, dans lequel L est 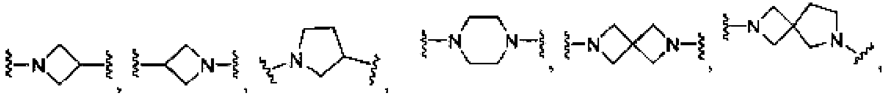 ou

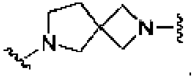

36. Composition pharmaceutique comprenant un composé selon l'une quelconque des revendications 1 à 35 et un excipient pharmaceutiquement acceptable.

37. Composé selon l'une quelconque des revendications 1 à 35 ou composition pharmaceutique selon la revendication 36 pour une utilisation dans le traitement du cancer.

38. Composé pour une utilisation selon la revendication 37, le cancer étant un cancer du poumon non à petites cellules, un cancer colorectal, un cancer de la vessie, un cancer de primitif inconnu, un gliome, un cancer du sein, un mélanome, un cancer de la peau autre que mélanome, un cancer de l'endomètre, un cancer œsophagogastrique, un cancer du pancréas, un cancer hépatobiliaire, un sarcome des tissus mous, un cancer de l'ovaire, un cancer de la tête et du cou, un carcinome à cellules rénales, un cancer des os, un lymphome non hodgkinien, un cancer du poumon à petites cellules, un cancer de la prostate, une tumeur embryonnaire, une tumeur germinale, un cancer du col de l'utérus, un cancer de la thyroïde, un cancer des glandes salivaires, une tumeur neuroendocrine gastro-intestinale, un sarcome utérin, une tumeur stromale gastro-intestinale, un cancer du SNC, une tumeur thymique, un carcinome corticosurrénal, un cancer de l'appendice, un cancer de l'intestin grêle, ou un cancer du pénis.

39. Composé pour une utilisation selon la revendication 38, le cancer étant un cancer du poumon non à petites cellules, un cancer colorectal, un cancer de la vessie, un cancer de primitif inconnu, un gliome, un cancer du sein, un mélanome, un cancer de la peau autre que mélanome, un cancer de l'endomètre, un sarcome des tissus mous, ou un cancer du pénis.

40. Composé pour une utilisation selon la revendication 39, le cancer étant un cancer du poumon non à petites cellules.

41. Composé pour une utilisation selon la revendication 39, le cancer étant un sarcome des tissus mous.
